(19) Europäisches Patentamt
European Patent Office
Office européen des brevets

(11) EP 1 772 521 A1

# (12) EUROPEAN PATENT APPLICATION

(43) Date of publication:
**11.04.2007 Bulletin 2007/15**

(51) Int Cl.:
***C12Q 1/68*** *(2006.01)*

(21) Application number: **05021625.8**

(22) Date of filing: **04.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **OncoScore AG**
**4125 Riehen (CH)**

(72) Inventors:
- **Urban, Patrick**
  **4142 Münchenstein (CH)**
- **Vuaroqueaux, Vincent**
  **4057 Basel (CH)**
- **Labuhn, Martin**
  **4057 Basel (CH)**
- **Eppenberger, Urs**
  **4125 Riehen (CH)**
- **Eppenberger-Castori, Serenella**
  **4125 Riehen (CH)**

(74) Representative: **Hoechst, Bruno Werner et al**
**A. Braun Braun Héritier Eschmann AG**
**Patentanwälte**
**Holbeinstrasse 36-38**
**4051 Basel (CH)**

## (54) Methods for the prognosis of cancer patients

(57) A method used for assessing the probability of survival of a cancer patient by assaying one or more ex-vivo samples from that patient, wherein the (SEQ ID NO. 37, 38) ERBB2 status of that patient is determined to be positive or negative; characterized in that in (SEQ ID NO. 37, 38) ERBB2-positive patients, at least one of a first set of cancer related genes is analysed in the sample or in one of the samples from said patient, and in (SEQ ID NO. 37, 38) ERBB2-negative patients, at least one of a second set of cancer related genes is analysed in the sample or in one of the samples from said patient.

EP 1 772 521 A1

## Description

The present invention relates to methods of cancer prognosis and kits for cancer prognosis as well as the use of specific genes in cancer prognosis.

The first task of a clinician in assessing a patient suspected of having cancer because of the presence of a suspicious lesion (e.g. a node in the breast), is to investigate to what entity the lesion belongs (cyst, tumor, inflammation, etc.) and to what dignity the lesion belongs (malignant, benign). Traditionally, this is done clinically using imaging methods including ultrasound, (digital) mammography, MRI, etc. and histo-pathologically after tissue sampling or tissue removal.

Once a lesion has been diagnosed as cancer, further classification is important with respect to prognosis (patient outcome) and selection of treatment (therapy prediction). Currently classification is achieved based largely on histo-pathological tumor features (grade, tumor size, nodal involvement), as well as patient age. For example, in breast cancer, the St. Gallen and NIH consensus criteria are currently the most widely used classification systems. These guidelines classify the majority of primary breast cancers into intermediate or high-risk subgroups. This leads to the recommended administration of adjuvant systemic therapy for nearly 90% of all newly diagnosed breast cancer patients. However, these classification systems do not assess individual biological changes on a molecular level in a systematic way.

Gene expression profiling using microarrays can be used to assess such changes by measuring hundreds of genes simultaneously. However this method has the major disadvantage that the cost, complexity and interpretation of DNA micro-arrays make them currently unsuitable for routine testing used in standard clinical settings.

Other workers have focused on individual genes as biomarkers involved in cancer, for example (SEQ ID NO. 37, 38) ERBB2 (also known as HER2: Human Epidermal Growth Factor Receptor 2 and *neu*: neuro/glioblastoma derived oncogene homolog (avian)). (SEQ ID NO. 37, 38) ERBB2 has been mechanistically linked with a variety of malignant processes including lymph node and distant metastasis. Amplification and/or overexpression of (SEQ ID NO. 37, 38) ERBB2 is observed in 15 to 30% of all newly diagnosed breast cancers, as well as in 15 to 30% of ovarian carcinomas. It has also been found to be overexpressed in lung and prostate cancer. It is associated with poor prognosis and serves as a predictor of clinical responsiveness to the anti-(SEQ ID NO. 37, 38) ERBB2 therapeutics such as trastuzumab (Herceptin®) as well as chemo- and endocrine therapy.

In the most recent ($9^{th}$) St. Gallen consensus panel determination, all (SEQ ID NO. 37, 38) ERBB2-positive breast cancer cases are to be considered high-risk (Goldhirsch et *al,* Annals of Oncology, 2005, doi:10.1093/annonc/mdi326).

This is unsatisfactory, since (SEQ ID NO. 37, 38) ERBB2-positive breast cancers display varying amounts of clinical aggressiveness i.e. not all (SEQ ID NO. 37, 38) ERBB2-positive patients develop metastasis, and thus not all are at high risk. Thus some patients are unnecessarily subjected to aggressive chemotherapy and unnecessary suffering as a consequence.

In addition not all (SEQ ID NO. 37, 38) ERBB2-positive patients respond to anti-(SEQ ID NO. 37, 38) ERBB2 therapy. In fact only up to 30% of (SEQ ID NO. 37, 38) ERBB2-positive patients will respond to trastuzumab therapy. This implies that other factors may also be influential in determining responsiveness to trastuzumab therapy.

Therefore, additional biomarkers are needed to improve classification of (SEQ ID NO. 37, 38) ERBB2-positive patients with respect to prognosis and therapeutic responsiveness in order to optimize the clinical management of these patients. Similarly additional biomarkers are needed to improve classification of (SEQ ID NO. 37, 38) ERBB2-negative patients with respect to prognosis and therapeutic responsiveness.

Thus the present invention provides a method used for assessing the probability of survival of a cancer patient by assaying one or more ex-vivo samples from that patient, wherein the (SEQ ID NO. 37, 38) ERBB2 status of that patient is determined to be positive or negative; characterized in that

a) in (SEQ ID NO. 37, 38) ERBB2-positive patients, at least one of a first set of cancer related genes is analysed in the sample or in one of the samples from said patient
and
b) in (SEQ ID NO. 37, 38) ERBB2-negative patients, at least one of a second set of cancer related genes is analysed in the sample or in one of the samples from said patient.

Preferably the probability of survival of the cancer patient is assessed in terms of metastasis free survival (MFS).

Alternatively, the probability of survival of the cancer patient is assessed in terms of relapse free survival (RFS).

The term cancer related genes relates to genes particularly the following (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 93, 94) TIMP3, (SEQ ID NO. 41, 42) ERBB3, (SEQ ID NO. 33, 34) ECGF1, (SEQ ID NO. 81, 82) SERPINE1, (SEQ ID NO. 53, 54) MMP2, (SEQ ID NO. 9, 10) BIRC5, (SEQ ID NO. 29, 30) E2F1, (SEQ ID NO. 105, 106) TOP2A, (SEQ ID NO. 101, 102) TK1, (SEQ ID NO. 109, 110) TYMS, (SEQ ID NO. 113, 114) VEGF, (SEQ ID NO. 85, 86) SRD5A1, (SEQ ID NO. 13, 14) CCND1, (SEQ ID NO. 25, 26) CTSD, (SEQ ID NO. 5, 6) BAX, (SEQ ID NO. 21, 22) CTSB, (SEQ ID NO. 17, 18) CTNNB1, (SEQ ID NO. 1, 2) ADM, (SEQ ID NO. 61, 62) MMP9, (SEQ ID NO. 89, 90) STK11 and (SEQ ID NO. 97, 98) TIMP4.

In a preferred embodiment said first set of genes comprises (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 93, 94) TIMP3, (SEQ ID NO. 41, 42) ERBB3, (SEQ ID NO. 33, 34) ECGF1, (SEQ ID NO. 81, 82) SERPINE1, and (SEQ ID NO. 53, 54) MMP2.

In another preferred embodiment of the invention, said second set of genes comprises (SEQ ID NO. 9, 10) BIRC5, (SEQ ID NO. 29, 30) E2F1, (SEQ ID NO. 105, 106) TOP2A, (SEQ ID NO. 101, 102) TK1, (SEQ ID NO. 109, 110) TYMS, (SEQ ID NO. 113, 114) VEGF, (SEQ ID NO. 85, 86) SRD5A1, (SEQ ID NO. 13, 14) CCND1, (SEQ ID NO. 25, 26) CTSD, (SEQ ID NO. 5, 6) BAX, (SEQ ID NO. 21, 22) CTSB, (SEQ ID NO. 17, 18) CTNNB1, (SEQ ID NO. 1, 2) ADM, (SEQ ID NO. 61, 62) MMP9, (SEQ ID NO. 89, 90) STK11 and (SEQ ID NO. 97, 98) TIMP4.

Preferably, in (SEQ ID NO. 37, 38) ERBB2-positive patients the combination of genes of (SEQ ID NO. 73, 74) PLAU, (SEQ ID NO. 77, 78) PLAUR, (SEQ ID NO. 65, 66) MMP11 and (SEQ ID NO. 49, 50) MMP1 is analysed.

In one embodiment of the invention, in (SEQ ID NO. 37, 38) ERBB2-positive patients the combination of genes of (SEQ ID NO. 73, 74) PLAU, (SEQ ID NO. 77, 78) PLAUR and (SEQ ID NO. 65, 66) MMP11 is analysed.

In another embodiment of the invention, in (SEQ ID NO. 37, 38) ERBB2-positive patients the combination of genes of (SEQ ID NO. 77, 78) PLAUR, (SEQ ID NO. 65, 66) MMP11 and (SEQ ID NO. 49, 50) MMP1 is analysed.

In yet another embodiment of the invention, in (SEQ ID NO. 37, 38) ERBB2-positive patients in addition to the aforementioned combinations of genes, the gene (SEQ ID NO. 57, 58) MMP3 is analysed, namely the combination of (SEQ ID NO. 73, 74) PLAU, (SEQ ID NO. 77, 78) PLAUR, (SEQ ID NO. 65, 66) MMP11, (SEQ ID NO. 49, 50) MMP1 and (SEQ ID NO. 57, 58) MMP3 or the combination (SEQ ID NO. 73, 74) PLAU, (SEQ ID NO. 77, 78) PLAUR, (SEQ ID NO. 65, 66) MMP11 and (SEQ ID NO. 57, 58) MMP3 or the combination (SEQ ID NO. 77, 78) PLAUR, (SEQ ID NO. 65, 66) MMP11, (SEQ ID NO. 49, 50) MMP1 and (SEQ ID NO. 57, 58) MMP3.

In a preferred embodiment of the invention, in (SEQ ID NO. 37, 38) ERBB2-positive patients in addition to the aforementioned combinations, the gene (SEQ ID NO. 93, 94) TIMP3 is analysed, such as for example the combination of (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3 and (SEQ ID NO. 93, 94) TIMP3.

In yet a further embodiment of the invention, in (SEQ ID NO. 37, 38) ERBB2-positive patients in addition to the aforementioned combinations the gene (SEQ ID NO. 53, 54) MMP2 is analysed, such as for example the combination of (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 93, 94) TIMP3 and (SEQ ID NO. 53, 54) MMP2.

In a still yet further embodiment of the invention, in (SEQ ID NO. 37, 38) ERBB2-positive patients in addition to the aforementioned combinations the gene (SEQ ID NO. 81, 82) SERPINE1 is analysed, such as for example the combination of (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 93, 94) TIMP3, (SEQ ID NO. 53, 54) MMP2 and (SEQ ID NO. 81, 82) SERPINE1.

Preferably in (SEQ ID NO. 37, 38) ERBB2-negative patients, the combination of genes of (SEQ ID NO. 9, 10) BIRC5, (SEQ ID NO. 29, 30) E2F1, (SEQ ID NO. 105, 106) TOP2A and (SEQ ID NO. 101, 102) TK1 is analysed.

In a preferred embodiment of the invention, in (SEQ ID NO. 37, 38) ERBB2-negative patients the combination of genes of (SEQ ID NO. 9, 10) BIRC5, (SEQ ID NO. 29, 30) E2F1 and (SEQ ID NO. 105, 106) TOP2A is analysed.

In another embodiment of the invention, in (SEQ ID NO. 37, 38) ERBB2-negative patients the combination of genes of (SEQ ID NO. 9, 10) BIRC5, (SEQ ID NO. 29, 30) E2F1 and (SEQ ID NO. 101, 102) TK1 is analysed.

In yet another embodiment of the invention, in (SEQ ID NO. 37, 38) ERBB2-negative patients the combination of genes of (SEQ ID NO. 9, 10) BIRC5, (SEQ ID NO. 29, 30) E2F1 and (SEQ ID NO. 109, 110) TYMS is analysed.

In still yet another embodiment of the invention, in (SEQ ID NO. 37, 38) ERBB2-negative patients the combination of genes of (SEQ ID NO. 9, 10) BIRC5, (SEQ ID NO. 29, 30) E2F1, (SEQ ID NO. 101, 102) TK1 and (SEQ ID NO. 109, 110) TYMS is analysed.

In a further embodiment of the invention, in (SEQ ID NO. 37, 38) ERBB2-negative patients in addition to the aforementioned combinations, the gene (SEQ ID NO. 21, 22) CTSB is analysed, such as for example the combination of (SEQ ID NO. 9, 10) BIRC5, (SEQ ID NO. 29, 30) E2F1, (SEQ ID NO. 105, 106) TOP2A, (SEQ ID NO. 101, 102) TK1 and (SEQ ID NO. 21, 22) CTSB.

In a yet further embodiment of the invention, in (SEQ ID NO. 37, 38) ERBB2-negative patients in addition to the aforementioned combinations, the gene (SEQ ID NO. 25, 26) CTSD is analysed, such as for example the combination of (SEQ ID NO. 9, 10) BIRC5, (SEQ ID NO. 29, 30) E2F1, (SEQ ID NO. 105, 106) TOP2A, (SEQ ID NO. 101, 102) TK1, (SEQ ID NO. 21, 22) CTSB and (SEQ ID NO. 25, 26) CTSD.

Suitable methods known in the art may be used to measure the expression levels of the genes. In a preferred embodiment of the method of the invention the analysis of the genes involves measuring the expression levels of the genes at the mRNA level.

Preferably also the (SEQ ID NO. 37, 38) ERBB2 status is determined at the mRNA level by qRT-PCR by measuring the cycle threshold value for (SEQ ID NO. 37, 38) ERBB2 and for 18S rRNA, subtracting the cycle threshold value for (SEQ ID NO. 37, 38) ERBB2 from the cycle threshold value for 18S rRNA. A value of ≥ -15.8 is determined to be positive (SEQ ID NO. 37, 38) ERBB2 status.

Most preferably in the method of the invention, in the first set of genes each gene is statistically weighted according to its association with survival in a population of cancer patients and within the second set of genes each gene is statistically weighted according to its association with survival in a population of cancer patients.

Preferably also the statistical weighting of the genes in the first set of genes and the statistical weighting of the genes in the second set of genes is expressed in terms of the Cox-coefficient. (Cox DR: Regression Models and Lifetables J.R. Soc. 34: 187-220, 1972) Another aspect of the invention is a kit for assessing the probability of survival of a patient of whom the (SEQ ID NO. 37, 38) ERBB2 status is known to be positive; comprising a set of oligonucleotide primers (SEQ ID NO. 75, 76, 79, 80, 67, 68, 51, 52), said primer set allowing PCR to be performed on mRNA transcribed from the following genes: (SEQ ID NO. 73, 74) PLAU, (SEQ ID NO. 77, 78) PLAUR, (SEQ ID NO. 65, 66) MMP11 and (SEQ ID NO. 49, 50) MMP1.

A further aspect of the invention is a kit for assessing the probability of survival of a patient of whom the (SEQ ID NO. 37, 38) ERBB2 status is known to be negative; comprising a set of oligonucleotide primers (SEQ ID NO. 11, 12, 31, 32, 107, 108, 103, 104), said primer set allowing PCR to be performed on mRNA transcribed from the following genes: (SEQ ID NO. 9, 10) BIRC5, (SEQ ID NO. 29, 30) E2F1, (SEQ ID NO. 105, 106) TOP2A and (SEQ ID NO. 101, 102) TK1.

Preferably, there is provided a kit comprising a set of oligonucleotide primers (SEQ ID NO. 75, 76, 79, 80, 67, 68, 51, 52, 11, 12, 31, 32, 107, 108, 103, 104), said primer set allowing PCR to be performed on mRNA transcribed from the following genes: (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 9, 10) BIRC5, (SEQ ID NO. 29, 30) E2F1, (SEQ ID NO. 105, 106) TOP2A and (SEQ ID NO. 101, 102) TK1.

More preferably there is provided a kit with all of the primers in Table 2 (SEQ ID NO. 3, 7, 11, 15, 19, 23, 27, 31, 35, 39, 43, 47, 51, 55, 59, 63, 67, 71, 75, 79, 83, 87, 91, 95, 99, 103, 107, 111, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207), and table 3 (SEQ ID NO. 4, 8, 12, 16, 20, 24, 28, 32, 36, 40, 44, 48, 52, 56, 60, 64, 68, 72, 76, 80, 84, 88, 92, 96, 100, 104, 108, 112, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208).

The aforementioned kits may also contain suitable reagents (other than primers) to perform PCR.

Another aspect of the invention is use of any of the genes or any combination of the genes disclosed herein in cancer prognosis.

Preferably the invention relates to the use of the genes (SEQ ID NO. 73, 74) PLAU, (SEQ ID NO. 77, 78) PLAUR, (SEQ ID NO. 65, 66) MMP11 and (SEQ ID NO. 49, 50) MMP1 in cancer prognosis.

A further aspect of the invention is use of the genes (SEQ ID NO. 9, 10) BIRC5, (SEQ ID NO. 29, 30) E2F1, (SEQ ID NO. 105, 106) TOP2A and (SEQ ID NO. 101, 102) TK1 in cancer prognosis.

## Brief Description of the Figures

For a more detailed description of the terms used in the figure legends see the detailed description section and the examples.

Figure 1: Distribution of (SEQ ID NO. 37, 38) ERBB2 mRNA expression levels in breast cancer biopsies from 575 patients. X: axis: (SEQ ID NO. 37, 38) ERBB2 expression level (delta cT), Y: axis: density (probability distribution)

Figure 2: ROC (Receiver operator characteristics) analysis of (SEQ ID NO. 37, 38) ERBB2 mRNA expression levels measured by PCR (n=100) (SEQ ID NO. 37, 38) ERBB2 amplification measured by FISH was used as reference. X: axis 1-specificity, Y: axis: sensitivity

Figure 3: Ranked (SEQ ID NO. 37, 38) ERBB2 mRNA expression levels (n=100). Black circles: FISH positive, empty circles: FISH negative samples, Broken line: cut off value for (SEQ ID NO. 37, 38) ERBB2 status by PCR (-15.8) X:axis: samples ranked according to (SEQ ID NO. 37, 38) ERBB2 expression level (ordered samples) Y:axis: (SEQ ID NO. 37, 38) ERBB2 expression (delta cT)

Figure 4: Distribution of mRNA expression levels in 317 breast cancer biopsies. X-axis: (SEQ ID NO. 45, 46) ESR1 expression (delta cT). Y-axis: density (probability distribution)

Figure 5: ROC (Receiver operator characteristics) analysis of ER mRNA expression levels measured by PCR (n=317). (SEQ ID NO. 45, 46) ESR1 protein cut off of 20 fmol/mg protein measured by ELISA was used as reference. X: axis 1-specificity, Y: axis: sensitivity

Figure 6: Correlation between (SEQ ID NO. 45, 46) ESR1 levels detected by PCR and ELISA. Solid line represents the regression line. Horizontal broken line shows the cut off value (20 fmol/mg protein) and vertical broken line shows the corresponding PCR cut off value (delta cT value of -19. X-axis: (SEQ ID NO. 45, 46) ESR1 expression measured by PCR (delta cT). Y-axis: (SEQ ID NO. 45, 46) ESR1 Expression levels measured by ELISA (log transformed)

Figure 7: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, (SEQ ID NO. 49, 50) MMP1 and (SEQ ID NO. 65, 66) MMP11. Shown is the probability of 5-years metastasis-free survival (Y-axis) as a function of different cut-offs of the prognostic score in percentiles (X-axis).

Figure 8: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, (SEQ ID NO. 49, 50) MMP1 and (SEQ ID NO. 65, 66) MMP11. Shown is the probability of 5-years relapse-free survival (Y-axis) as a function of different cut-offs of the prognostic score in percentiles (X-axis).

Figure 9: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, and (SEQ ID NO. 65, 66) MMP11. Shown is the probability of 5-years metastasis-free survival (Y-axis) as a function of different cut-offs of the prognostic score in percentiles (X-axis).

Figure 10: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, and (SEQ ID NO. 65, 66) MMP11. Shown is the probability of 5-years relapse-free survival (Y-axis) as a function of different cut-offs of the prognostic score in percentiles (X-axis).

Figure 11: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 81, 82) SERPINE1 and (SEQ ID NO. 57, 58) MMP3. Shown is the probability of 5-years metastasis-free survival (Y-axis) as a function of different cut-offs of the prognostic score in percentiles (X-axis).

Figure 12: Prognosis graph for (SEQ ID NO. 37, 38) ERBB2-positive patients determined by analyzing the gene combination (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 81, 82) SERPINE1 and (SEQ ID NO. 57, 58) MMP3. Shown is the probability of 5-years relapse-free survival (Y-axis) as a function of different cut-offs of the prognostic score in percentiles (X-axis).

Figure 13: Prognosis graph for (SEQ ID NO. 37, 38) ERBB2-positive patients determined by analyzing the gene (SEQ ID NO. 73, 74) PLAU. Shown is the probability of 5-years metastasis-free survival (Y-axis) as a function of different cut-offs of the prognostic score in percentiles (X-axis).

Figure 14: Prognosis graph for (SEQ ID NO. 37, 38) ERBB-positive patients determined by analyzing the gene (SEQ ID NO. 73, 74) PLAU. Shown is the probability of 5-years relapse-free survival (Y-axis) as a function of different cut-offs of the prognostic score in percentiles (X-axis).

Figure 15: Prognosis graph for (SEQ ID NO. 37, 38) ERBB2-positive patients determined by analyzing the gene (SEQ ID NO. 77, 78) PLAUR. Shown is the probability of 5-years metastasis-free survival (Y-axis) as a function of different cut-offs of the prognostic score in percentiles (X-axis).

Figure 16: Prognosis graph for (SEQ ID NO. 37, 38) ERBB2-positive patients determined by analyzing the gene (SEQ ID NO. 77, 78) PLAUR. Shown is the probability of 5-years relapse-free survival (Y-axis) as a function of different cut-offs of the prognostic score in percentiles (X-axis).

Figure 17: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, (SEQ ID NO. 105, 106) TOP2A and (SEQ ID NO. 101, 102) TK1. Shown is the probability of 5-years metastasis-free survival (Y-axis) as a function of different cut-offs of the prognostic score in percentiles (X-axis).

Figure 18: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, (SEQ ID NO. 105, 106) TOP2A and (SEQ ID NO. 101, 102) TK1. Shown is the probability of 5-years relapse-free survival (Y-axis) as a function of different cut-offs of the prognostic score in percentiles (X-axis).

Figure 19: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, and (SEQ ID NO. 105, 106) TOP2A. Shown is the probability of 5-years metastasis-free survival (Y-axis) as a function of different cut-offs of the prognostic score in percentiles (X-axis).

Figure 20: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, and (SEQ ID NO. 105, 106) TOP2A. Shown is the probability of 5-years relapse-free survival (Y-axis) as a function of different cut-offs of the prognostic score in percentiles (X-axis).

Figure 21: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, (SEQ ID NO. 105, 106) TOP2A, (SEQ ID NO. 21, 22) CTSB and (SEQ ID NO. 25, 26) CTSD. Shown is the probability of 5-years metastasis-free survival (Y-axis) as a function of different cut-offs of the prognostic score in percentiles (X-axis).

Figure 22: Prognosis graph for (SEQ ID NO. 37, 38) ERBB2-negative patients determined by analyzing the gene combination (SEQ ID NO. 9, 10) BIRC5, (SEQ ID NO. 29, 30) E2F1, (SEQ ID NO. 105, 106) TOP2A, (SEQ ID NO. 21, 22) CTSB and (SEQ ID NO. 25, 26) CTSD. Shown is the probability of 5-years relapse-free survival (Y-axis) as a function of different cut-offs of the prognostic score in percentiles (X-axis).

Figure 23: Prognosis graph for (SEQ ID NO. 37, 38) ERBB2-negative patients determined by analyzing the gene (SEQ ID NO. 9, 10) BIRC5. Shown is the probability of 5-years metastasis-free survival (Y-axis) as a function of different cut-offs of the prognostic score in percentiles (X-axis).

Figure 24: Prognosis graph for (SEQ ID NO. 37, 38) ERBB2-negative patients determined by analyzing the gene (SEQ ID NO. 9, 10) BIRC5. Shown is the probability of 5-years relapse-free survival (Y-axis) as a function of different cut-offs of the prognostic score in percentiles (X-axis).

Figure 25: Prognosis graph for (SEQ ID NO. 37, 38) ERBB2-negative patients determined by analyzing the gene (SEQ ID NO. 29, 30) E2F1. Shown is the probability of 5-years metastasis-free survival (Y-axis) as a function of different cut-offs of the prognostic score in percentiles (X-axis).

Figure 26: Prognosis graph for (SEQ ID NO. 37, 38) ERBB2-negative patients determined by analyzing the gene (SEQ ID NO. 29, 30) E2F1. Shown is the probability of 5-years relapse-free survival (Y-axis) as a function of different cut-offs of the prognostic score in percentiles (X-axis).

Detailed description

This invention provides a method of assessing the probability of survival of a cancer patient, which may be assessed in terms of metastasis free survival, relapse free survival or overall survival.

The term metastasis-free survival (MFS) refers to distant metastasis-free survival MFS (DMFS) i.e. it refers to living without the occurrence of metastasis at a site distant from the primary tumour.

The term relapse-free survival (RFS) refers to living without the recurrence of cancer at any site, except as second primary cancer.

The term overall survival as used herein refers to disease-specific overall survival, i.e. it refers to living rather than dying from a specific disease.

The survival prediction is made based on the analysis of one or more ex-vivo samples from a patient. Ex-vivo samples include tissue samples, stool samples, cell aspirates, ductal lavages, serum samples, urine and blood samples.

The (SEQ ID NO. 37, 38) ERBB2 status of the patient is determined to be positive or negative. The following methods may be used to determine the (SEQ ID NO. 37, 38) ERBB2 status and are routinely used in the field. They are intended to be illustrative of the techniques available, however measurement of the (SEQ ID NO. 37, 38) ERBB2 status is not intended to be restricted to the assays described below. The methods may also be used in combination to determine the (SEQ ID NO. 37, 38) ERBB2 status.

The (SEQ ID NO. 37, 38) ERBB2 status may be determined at the protein level for example by IHC (immunohistochemistry), ELISA (enzyme linked immunosorption assay) or western blot. IHC based testing is possible through use of the HercepTest (DAKO) kit, which has received US FDA approval for clinical use. The kit contains standardized reagents and control slides. A scoring system is employed in which a score ranging in whole numbers from 0 to 3 is given which reflects the staining intensity and pattern in 10% or more of tumor cells. A score of 3+ is considered as positive. Scores of 2+ require additional confirmation by another method such as FISH (see below) to determine positivity. (Hanna, W. Testing for HER2 Status., Oncology 2001; 61 (suppl.2) 22-30; Leo. A et al., Current Status of HER2 Testing; Oncology 2002, 63 (suppl. 1)25-32)).

The (SEQ ID NO. 37, 38) ERBB2 status may be determined at the DNA level for example by FISH (fluorescence in situ hybridization), CISH (chromogenic in situ hybridization), PCR (polymerase chain reaction) or southern blot. With FISH the number of (SEQ ID NO. 37, 38) ERBB2 genes in the cell nucleus can be counted to measure (SEQ ID NO. 37, 38) ERBB2 amplification. Some FISH assays measure (SEQ ID NO. 37, 38) ERBB2 amplification as an absolute number of gene copies, while others involve the ratio between (SEQ ID NO. 37, 38) ERBB2 gene copies and chromosome 17 copies (the (SEQ ID NO. 37, 38) ERBB2 gene resides on chromosome 17). Standardized kits for use for FISH are currently available, which have been approved for use by the FDA. The Inform (SEQ ID NO. 37, 38) ERBB2 kit (ONCOR/Ventana) is an example of a FISH assay which measures the absolute number of (SEQ ID NO. 37, 38) ERBB2 gene copies. The PathVysion® Kit (Vysis) on the other hand uses two DNA probes, one which specifically recognizes the (SEQ ID NO. 37, 38) ERBB2 gene and the other the chromosome 17 centromere. (SEQ ID NO. 37, 38) ERBB2 and centromere signals are measured in 60 nuclei and then the (SEQ ID NO. 37, 38) ERBB2 gene: chromosome 17 centromere ratio is calculated. A ratio of ≥ 2 indicates (SEQ ID NO. 37, 38) ERBB2 positivity. (Hanna, W. Testing for HER2 Status., Oncology 2001; 61 (suppl.2) 22-30; Leo. A et al., Current Status of HER2 Testing; Oncology 2002, 63 (suppl. 1)25-32)).

(SEQ ID NO. 37, 38) ERBB2 status may also determined using a gene expression based signature based on the expression levels of other marker genes.

Importantly also, the (SEQ ID NO. 37, 38) ERBB2 status may be determined at the RNA level using PCR, microarray analysis or northern blot.
PCR includes competitive PCR and quantitative real-time PCR (qRT-PCR). QRT-PCR measurement of RNA expression is a highly sensitive, reproducible, quantitative and cost effective method. A description of the use of qRT-PCR to determine (SEQ ID NO. 37, 38) ERBB2 status is to be found in Examples 1, 2 and 3.

In addition to (SEQ ID NO. 37, 38) ERBB2 status additional genes are analysed in accordance with this invention. If (SEQ ID NO. 37, 38) ERBB2 status is determined to be positive, then at least one of a first set of cancer related genes is analysed in the or one of the samples from the patient and in (SEQ ID NO. 37, 38) ERBB2-negative patients, at least one of a second set of cancer related genes is analysed in the or one of the samples from the patient.

Genes analysed according to the invention are related to full-length nucleic acid sequences that code for the production of a protein or peptide. A skilled person in the art will recognize that portions of these sequences or ESTs can be used to assess gene expression for the corresponding gene. A skilled person in the art will also recognize that complements of the sequences disclosed therein can be measured according to the invention.

It is possible to measure the expression at the protein level by well known methods in the art such as IHC. It is also possible to measure gene amplification at the DNA level by well known methods in the art such as FISH.

Preferably the genes are analysed by measuring the expression levels of those genes at the mRNA level. Suitable methods for such measurement are PCR, microarray analysis or northern blot. PCR includes competitive PCR and quantitative real-time PCR (qRT-PCR). Most preferably the method of the invention employs the use of qrt-PCR to measure the mRNA expression levels of the genes in the first set and the second set.

Most preferably the analysis of the genes involves measuring expression levels of the genes and then a determination of the probability of survival. The (SEQ ID NO. 37, 38) ERBB2 status may be determined before, after or simultaneously as the measurement of the genes in either the first set and/or the second set of cancer related genes.

In a preferred method of the invention, after the expression levels of the genes are measured from a specific patient, the analysis of those genes further involves the calculation of a prognostic score and then comparison of that score value with a patient population data set. This then allows a determination of what percentile in the population that patient to be analysed falls within. From this a prediction regarding the survival probability for that patient can be given. The probability of survival of the cancer patient can be expressed in terms of MFS, RFS or OS by using patient population data concerning MFS, RFS and OS respectively.

The prognostic score (risk index) is based on univariate Cox regression models. The score is calculated as follows:

Prognostic Score:

$$PS_j = \sum_{i=1}^{n} \beta_i x_{i,j} \quad \text{Formula I}$$

where $\beta_i$ is the Cox-coefficient (weighting factor) for gene i, $x_{i,j}$ the expression level of gene i in sample j, and n the number of genes in the score (n≥1).

The cox-coefficient is determined for each gene based on analysis of samples from cancer patient populations.

The invention is further illustrated by the following nonlimiting examples.

Example 1: Tissue processing, RNA extraction and cDNA preparation

A patient population of 575 patients diagnosed with primary breast cancer were studied. Breast tissue samples, which included core needle and excisional biopsies, were taken from the patient and immediately stored in either ammonium sulfate, liquid nitrogen, or dry ice to preserve tissue quality and prevent (RNA) degradation. Samples were either used immediately or stored at -80°C until further processing (i.e. gene expression profiling using qrt-PCR).

The following steps were performed to process the sample:

a) 5 to 10 μm thick frozen sections were prepared from each biopsy using a cryostat (cut at -25 to -20°C). Subsequently, tissues were stained using Hematoxylin & Eosin (staining kit from Richard & Allan International) according to the manufacturers instructions. For each sample, amount of tumors cells, stromal component, fatty tissue, leukocyte infiltration and necrosis was quantified.

*b*) Ten consecutive sections of 5 μm which were directly put into a lysis buffer (Qiazol, RNeasy Kit, Qiagen) and subjected to nucleic acid extraction.

*c*) After the ten sections taken for nucleic acid extraction, another consecutive 5μm section was taken for a second control at the end of the tissue opposite to the end from which the sections in step a) were taken. Histological control of this tissue was performed to determine the tumor cells content, stromal component and lymphocytic infiltration.

Total RNA was extracted from the sections taken in step b) using the Lipid Tissues RNeasy Kit (Qiagen) according to the manufacturer's recommendations.

The amount of RNA and RNA quality was assessed with a Bioanalyzer 2100 (Agilent Technologies) and RNA 6000 Nano LabChip-Kit (Agilent Technologies) following the manufacturer's recommendations.

The degree of rRNA degradation in the sample was used as an estimate of overall RNA quality. The 28S and 18S rRNA species were electrophoretically separated in the Bioananalyzer 2100, and the sharpness of the two RNA bands on the gel was assessed. Furthermore the ratio of 28S to 18S rRNA provided an estimate of the RNA quality. Since the amplicons generated in the following PCR reactions were short, even samples showing partial rRNA degradation were used. Samples having a 28S/18S rRNA ratio 0.5 or more were used in the following examples.

Reverse transcription

100ng to 1μg total RNA of the target sample was reverse transcribed to cDNA in a final volume of 20μl using a MMLV reverse transcription kit (Invitrogen Corp., Carlsbad, Calif) according to the manufacturer's recommendations. After reverse transcription, the 20 μl of cDNA was diluted by adding 180μl of molecular biology grade water (Eppendorf).

Example 2: qRT-PCR

A total of 75 genes were analysed, selected because of their involvement in cancer.

Table 1: genes studied

Gene Name, Gene Symbol and Genbank accession numbers

| Gene Name | Official Symbol | Reference Sequence |
|---|---|---|
| Cathepsin B | CTSB | NM_001908 |

(continued)

| Gene Name | Official Symbol | Reference Sequence |
|---|---|---|
| Cathepsin D | CTSD | NM_001909 |
| Plasminogen activator, urokinase | PLAU | NM_002658 |
| Plasminogen activator, urokinase receptor | PLAUR | NM_002659 |
| Metalloprotease 1 | MMP1 | NM_002421 |
| Metalloprotease 2 | MMP2 | NM_004530 |
| Metalloprotease 3 | MMP3 | NM_002422 |
| Metalloprotease 7 | MMP7 | NM_002423 |
| Metalloprotease 9 | MMP9 | NM_004994 |
| Metalloprotease 11 | MMP11 | NM_005940 |
| Tissue inhibitor of Metalloproteases 1 Metalloproteases 1 | TIMP1 | NM_003254 |
| Tissue inhibitor of Metalloproteases 2 | TIMP2 | NM_003255 |
| Tissue inhibitor of Metalloproteases 3 | TIMP3 | NM_000362 |
| Tissue inhibitor of Metalloproteases 4 | TIMP4 | NM_003256 |
| Plasminogen Activator Inhibitor-1 | SERPINE1 | NM_000602 |
| Thymidilate synthase | TYMS | NM_001071 |
| Thymidine kinase 1 | TK1 | NM_003258 |
| Topoisomerase II alpha | TOP2A | NM_001067 |
| Thymidine Phosphorylase | ECGF1 | NM_001953 |
| Dihydropyrimidine deshydrogenase | DPYD | NM_000110 |
| Multidrug restistance 1 | ABCB1 | NM_000927 |
| Transforming Growth factor, alpha | TGFA | NM_003236 |
| Insulin Growth Factor 1 | IGF1 | NM_000618 |
| Insulin Growth Factor 2 | IGF2 | NM_000612 |
| Transforming Growth Factor, beta 1 | TGFB1 | NM_000660 |
| Amphiregulin | AREG | NM_001657 |
| Epidermal Growth Factor | EGF | NM_001963 |
| Vascular Endothelial Growth Factor A | VEGF | NM_003376 |
| Vascular Endothelial Growth Factor B | VEGFB | NM_003377 |
| Vascular Endothelial Growth Factor C | VEGFC | NM_005429 |
| Vascular Endothelial Growth Factor D | FIGF | NM_004469 |
| Epidermal Growth Factor receptor 1 | EGFR | NM_005228 |
| Epidermal Growth Factor receptor 2 | ERBB2 | NM_004448 |
| Epidermal Growth Factor receptor 3 | ERBB3 | NM_001982 |
| Epidermal Growth Factor receptor 4 | ERBB4 | NM_005235 |
| Insulin Growth Factor 1 receptor | IGF1R | NM_000875 |
| Insulin Growth Factor 2 receptor | IGF2R | NM_000876 |
| Vascular Endothelial Growth Factor receptor 1/Flt1 | FLT1 | NM_002019 |
| Vascular Endothelial Growth Factor receptor 2/KDR | KDR | NM_002253 |

(continued)

| Gene Name | Official Symbol | Reference Sequence |
|---|---|---|
| Vascular Endothelial Growth Factor receptor 3/Flt4 | FLT4 | NM_002020 |
| Fibroblast growth factor receptor 1 | FGFR1 | NM_000604 |
| Estrogen receptor alpha | ESR1 | NM_000125 |
| Progesterone receptor | PGR | NM_000926 |
| Androgen receptor | AR | NM_000044 |
| Hydroxy (17-beta) Steroid Deshydrogenase 1 | HSD17B1 | NM_000413 |
| Aromatase | CYP19A1 | NM_000103 |
| Steroid 5-alpha-reductase | SRD5A1 | NM_001047 |
| Cyclooxygenase 2b | PTGS2 | NM_000963 |
| Peptidylglycine alpha-amidating Monooxygenase | PAM | NM_000919 |
| BCL2-associated protein X | BAX | NM_138761 |
| B-cell CLL/lymphoma 2 | BCL2 | NM_000633 |
| Survivin | BIRC5 | NM_001168 |
| Keratin 19 | KRT19 | NM_002276 |
| Keratin 7 | KRT7 | NM_005556 |
| Glutathione S-transferase pi | GSTP1 | NM_000852 |
| Cyclin D1 | CCND1 | NM_053056 |
| P21/Cip | CDKN1A | NM_078467 |
| P27/Kip | CDKN1B | NM_004064 |
| MDM2 | MDM2 | NM_002392 |
| Retinoblastoma 1 | RB1 | NM_000321 |
| Adrenomedullin | ADM | NM_001124 |
| Transcription factor E2F1 | E2F1 | NM_005225 |
| Hypoxia inducible factor 1, alpha | HIF1A | NM_001530 |
| Transcription Factor 4 | TCF4 | NM_003199 |
| Catentin, beta 1 | CTNNB1 | NM_001904 |
| Amplified in Breast cancer1 (AIB1) | NCOA3 | NM_006534 |
| NCOR1 | NCOR1 | NM_006311 |
| P53 | TP53 | NM_000546 |
| c-Myc | MYC | NM_002467 |
| v-ETS erythroblastosis virus E26 oncogene homolog 1 | ETS1 | NM_005238 |
| Secreted frizzled-related protein 1 | SFRP1 | NM_003012 |
| Mammaglobin 1 | SCGB2A2 | NM_002411 |
| Mammaglobin 2 | SCGB2A1 | NM_002407 |
| 18S ribosomal RNA pseudogene | LOC359724 | NC_000024 |
| LKB1 | STK11 | NM_000455 |

Primer selection:

Primers were designed using the Primer Express™ v2.0 software (Applied Biosystems, Forster City, CA). All primer sets were selected to anneal at the same conditions to the target sequence (AT=60°C) and to be cDNA specific. Primers were further designed to be compatible with (gene-specific) fluorescent detection probes which were designed in parallel. In addition, primers were tested on the LightCycler Probe design software to validate their design. To check primer specificity all primer sequences were also blasted against the dbEST and nr databases.

Table 2: Forward primers

| Official Symbol | Forward Primer | Seq. ID |
|---|---|---|
| CTSB | GGAGAATGGCACACCCTACTG | 23 |
| CTSD | TGATTCAGGGCGAGTACATGAT | 27 |
| PLAU | GGAAAACCTCATCCTACACAAGGA | 75 |
| PLAUR | CCGAGGTTGTGTGTGGGTTAG | 79 |
| MMP1 | ACACCTCTGACATTCACCAAGGT | 51 |
| MMP2 | CCTGAGCTCCCGGAAAAGA | 55 |
| MMP3 | AAAGGATACAACAGGGACCAATTTA | 59 |
| MMP7 | GGATGGTAGCAGTCTAGGGATTAACTT | 117 |
| MMP9 | CAGTACCGAGAGAAAGCCTATTTCTG | 63 |
| MMP11 | AAGACGGACCTCACCTACAGGAT | 67 |
| TIMP1 | CCAGCGCCCAGAGAGACA | 119 |
| TIMP2 | CACCCAGAAGAAGAGCCTGAA | 121 |
| TIMP3 | CTGCTGACAGGTCGCGTCTA | 95 |
| TIMP4 | CACCCTCAGCAGCACATCTG | 99 |
| SERPINE1 | GGCTGACTTCACGAGTCTTTCAG | 83 |
| TYMS | GAGTGATTGACACCATCAAAACCA | 111 |
| TK1 | ATTCTCGGGCCGATGTTCT | 103 |
| TOP2A | GATTCATTGAAGACGCTTCGTTATG | 107 |
| ECGF1 | CCTGCGGACGGAATCCTATA | 35 |
| DPYD | AAAGTGGTCTTCAGTTTCTCCATAGTG | 123 |
| ABCB1 | CCTAATGCCGAACACAT | 125 |
| TGFA | CCTGGCTGTCCTTATCATCACA | 127 |
| IGF1 | TGTATTGCGCACCCCTCAA | 129 |
| IGF2 | CCGTGCTTCCGGACAACTT | 131 |
| TGFB1 | AAATTGAGGGCTTTCGCCTTA | 133 |
| AREG | CGGCTCAGGCCATTATGC | 135 |
| EGF | TGCAGCTTCAGGACCACAAC | 137 |
| VEGF | GGGCAGAATCATCACGAAGTG | 115 |
| VEGFB | GACGATGGCCTGGAGTGTGT | 139 |
| VEGFC | TACCTCAGCAAGACGTTATTTGAAA | 141 |
| FIGF | GAGGAAAATCCACTTGCTGGAA | 143 |
| EGFR | GGACTATGTCCGGGAACACAA | 145 |

(continued)

| Official Symbol | Forward Primer | Seq. ID |
|---|---|---|
| ERBB2 | CTGAACTGGTGTATGCAGATTGC | 39 |
| ERBB3 | AATAAAAGGGCTATGAGGCGATACT | 43 |
| ERBB4 | GTCCAGATAGCTAAGGGAATGATGTAC | 147 |
| IGF1R | GCATACCTCAACGCCAATAAGTTC | 149 |
| IGF2R | GCAGAAGCTGGGTGTCATAGGT | 151 |
| FLT1 | GCTAAAAATCTTGACCCACATTGG | 153 |
| KDR | CTGAAACGGCGCTTGGA | 155 |
| FLT4 | GGAAAGAATAAGACTGTGAGCAAGCT | 157 |
| FGFR1 | GAGGCTACAAGGTCCGTTATGC | 159 |
| ESR1 | CTTGCTCTTGGACAGGAACCA | 47 |
| PGR | TGTCGAGCTCACAGCGTTTC | 71 |
| AR | CCGCTGAAGGGAAACAGAAG | 161 |
| HSD17B1 | TGCCTTTCAATGACGTTTATTGC | 163 |
| CYP19A1 | TGCTCCTCACTGGCCTTTTT | 165 |
| SRD5A1 | CCACTGTTGGCATGTACAATGG | 87 |
| PTGS2 | GAATCATTCACCAGGCAAATTG | 167 |
| PAM | ACCATTTAGGTAAGGTAGTAAGTGGATACAG | 169 |
| BAX | TGGAGCTGCAGAGGATGATTG | 7 |
| BCL2 | CCTGTGGATGACTGAGTACCTGAA | 171 |
| BIRC5 | GACGACCCCATAGAGGAACATAAA | 11 |
| KRT19 | TGCGGGACAAGATTCTTGGT | 173 |
| KRT7 | ATGCTGCCTACATGAGCAAGGT | 175 |
| GSTP1 | GAGACCCTGCTGTCCCAGAAC | 177 |
| CCND1 | CTGGAGGTCTGCGAGGAACA | 15 |
| CDKN1A | GCAGACCAGCATGACAGATTTC | 179 |
| CDKN1B | CCTGCAACCGACGATTCTTC | 181 |
| MDM2 | AAAGAGCACAGGAAAATATATACCATGA | 183 |
| RB1 | CGGATTCCTGGAGGGAACA | 185 |
| ADM | CCGTCGCCCTGATGTACCT | 3 |
| E2F1 | GAGCAGATGGTTATGGTGATCAAAG | 31 |
| HIF1A | TGCCCCAGATTCAGGATCAG | 187 |
| TCF4 | CTCCAGGTTTGCCATCTTCAGT | 189 |
| CTNNB1 | ATAAAGGCTACTGTTGGATTGATTCG | 19 |
| NCOA3 | CAGCCCCAGCAGGGTTTT | 191 |
| NCOR1 | TGAAACACCTAGCGATGCTATTGA | 193 |
| TP53 | GATGGAGAATATTTCACCCTTCAGAT | 195 |
| MYC | CAGCTGCTTAGACGCTGGATTT | 197 |
| ETS1 | TATACCTCGGATTACTTCATTAGCTATGGTA | 199 |

(continued)

| Official Symbol | Forward Primer | Seq. ID |
|---|---|---|
| SFRP1 | ACGTCTGCATCGCCATGAC | 201 |
| SCGB2A2 | TTCTTAACCAAACGGATGAAACTCT | 203 |
| SCGB2A1 | GGGAAATTCAAGCAGTGTTTCC | 205 |
| LOC359724 | CTACCACATCCAAGGAAGGCA | 207 |
| STK11 | GGGAGGCCAACGTGAAGA | 91 |

Table 3: Reverse primers

| Official Symbol | Reverse Primer | Seq. ID |
|---|---|---|
| CTSB | CTGAGTATTTTAAAGAAGCCATTGTCA | 24 |
| CTSD | GGGACAGCTTGTAGCCTTTGC | 28 |
| PLAU | ACGGATCTTCAGCAAGGCAAT | 76 |
| PLAUR | GGCTTCGGGAATAGGTGACA | 80 |
| MMP1 | CCGATGATCTCCCCTGACAA | 52 |
| MMP2 | ATTCATTCCCTGCAAAGAACACA | 56 |
| MMP3 | CAGTGTTGGCTGAGTGAAAGAGA | 60 |
| MMP7 | GGAATGTCCCATACCCAAAGAA | 118 |
| MMP9 | TAGGTCACGTAGCCCACTTGGT | 64 |
| MMP11 | GGCGTCACATCGCTCCAT | 68 |
| TIMP1 | AGCAACAACAGGATGCCAGAA | 120 |
| TIMP2 | GGCAGCGCGTGATCTTG | 122 |
| TIMP3 | TTACAACCCAGGTGATACCGATAGT | 96 |
| TIMP4 | TGGCCGGAACTACCTTCTCA | 100 |
| SERPINE1 | CGTTCACCTCGATCTTCACTTT | 84 |
| TYMS | GCGCCATCAGAGGAAGATCTC | 112 |
| TK1 | GCACTTGTACTGAGCAATCTGGAA | 104 |
| TOP2A | GAAGAGAGGGCCAGTTGTGATG | 108 |
| ECGF1 | TTACTGAGAATGGAGGCTGTGATG | 36 |
| DPYD | CTTCGATCACAGTGAAATCCAGAT | 124 |
| ABCB1 | TCCAGGCTCAGTCCCTGAAG | 126 |
| TGFA | GGGCGCTGGGCTTCTC | 128 |
| IGF1 | CCTCTACTTGCGTTCTTCAAATGTAC | 130 |
| IGF2 | GTGGACTGCTTCCAGGTGTCA | 132 |
| TGFB1 | CCGGTAGTGAACCCGTTGAT | 134 |
| AREG | GGTCCCCAGAAAATGGTTCAC | 136 |
| EGF | TTCCATAGTCTGTTCCATCAAAATG | 138 |
| VEGF | GGTCTCGATTGGATGGCAGTA | 116 |
| VEGFB | GGTACCGGATCATGAGGATCTG | 140 |
| VEGFC | AAGTGTGATTGGCAAAACTGATTGT | 142 |

(continued)

| Official Symbol | Reverse Primer | Seq. ID |
|---|---|---|
| FIGF | ATCATGTGTGGCCCACAGAGA | 144 |
| EGFR | CCAAGTAGTTCATGCCCTTTGC | 146 |
| ERBB2 | TTCCGAGCGGCCAAGTC | 40 |
| ERBB3 | AGCTTCCTTAGCTCTGTCTCTTTGA | 44 |
| ERBB4 | CTAGCCCAAAATCTGTGATTTTCAC | 148 |
| IGF1R | CGTCATACCAAAATCTCCGATTTT | 150 |
| IGF2R | CACGGAGGATGCGGTCTTATT | 152 |
| FLT1 | AGTCACGTTTGCTCTTGAGGTAGTT | 154 |
| KDR | CAGATCTTCAGGAGCTTCCTCTTC | 156 |
| FLT4 | CATAGAAGTAGATGAGCCGCTCATC | 158 |
| FGFR1 | CTGCCGTACTCATTCTCCACAA | 160 |
| ESR1 | CAAACTCCTCTCCCTGCAGATT | 48 |
| PGR | TACAGATGAAGTTGTTTGACAAGATCA | 72 |
| AR | TCATAACATTTCCGAAGACGACAA | 162 |
| HSD17B1 | GGCTCAAGTGGACCCCAAA | 164 |
| CYP19A1 | ATGCAGTAGCCAGGAC | 166 |
| SRD5A1 | GTTAACCACAAGCCAAAACCTATTAGA | 88 |
| PTGS2 | TCTGTACTGCGGGTGGAACA | 168 |
| PAM | GCAGCCAGTAGGTCACCAAAA | 170 |
| BAX | GCTGCCACTCGGAAAAAGAC | 8 |
| BCL2 | CAGAGACAGCCAGGAGAAATCA | 172 |
| BIRC5 | CACCAAGGGTTAATTCTTCAAACTG | 12 |
| KRT19 | GCAGCCAGACGGGCATT | 174 |
| KRT7 | GCTCTGTCAACTCCGTCTCATTG | 176 |
| GSTP1 | AGGTTGTAGTCAGCGAAGGAGATCT | 178 |
| CCND1 | TGCAGGCGGCTCTTTTTC | 16 |
| CDKN1A | GCGGATTAGGGCTTCCTCTT | 180 |
| CDKN1B | GGGCGTCTGCTCCACAGA | 182 |
| MDM2 | GGTGACACCTGTTCTCACTCACA | 184 |
| RB1 | CAATTGATACTAAGATTCTTGATCTTGGA | 186 |
| ADM | CCCACTTATTCCACTTCTTTCGAA | 4 |
| E2F1 | GGAGATCTGAAAGTTCTCCGAAGA | 32 |
| HIF1A | TGGGACTATTAGGCTCAGGTGAAC | 188 |
| TCF4 | GCCTGGCGAGTCCCTATTG | 190 |
| CTNNB1 | CTCACGCAAAGGTGCATGATT | 20 |
| NCOA3 | CACCCTCTGTTGTCGGAAGTG | 192 |
| NCOR1 | GGTAGGATCATTTTCCGCTTGA | 194 |
| TP53 | CCTCATTCAGCTCTCGGAACA | 196 |

(continued)

| Official Symbol | Reverse Primer | Seq. ID |
|---|---|---|
| MYC | TTCCTGTTGGTGAAGCTAACGTT | 198 |
| ETS1 | CCGAGCTGATGGGATGGA | 200 |
| SFRP1 | ATGGCCTCAGATTTCAACTCGTT | 202 |
| SCGB2A2 | CCAAAGGTCTTGCAGAAAGTTAAAA | 204 |
| SCGB2A1 | CCAGTCACATAGAACTCGAAAAACTTTGGAC TGATG | 206 |
| LOC359724 | TTTTTCGTCACTACCTCCCCG | 208 |
| STK11 | CATCACCATATACATTTTCTGCTTCTC | 92 |

The primers were designed to yield small amplicon sizes, ranging from 65 to 146 nucleotides, the mean being around 90 nucleotides. This was because short amplicon sizes are relatively insensitive to RNA degradation compared to longer ones and are expected to have better sensitivity.

Primers were then manufactured by GeneScan Europe (Freiburg, Germany). Primer sets were tested in qRT-PCR reactions with universal human reference RNA (Stratagene, La Jolla, CA) and/or samples that express the target gene. After qrt-PCR all amplicons were submitted to a temperature ramping to be analyzed for their melting points. In addition, amplicons were each individually sequenced. Finally, amplicon sizes were checked using the Bioanalyzer 2100 DNA 500 Lab Chip Kit (Agilent).

PCR plate preparation

For each gene a mixture was prepared containing 1 μM each of reverse and forward primers in molecular biology grade water. 5 μl of each primer mixture were distributed per well on a standard 96 well PCR plate (Applied Biosystems). The PCR plate was closed using adhesive covers (Applied Biosystems) shortly centrifuged and then stored at -20°C until use.

Preparation of PCR mixtures

200μl cDNA target sample prepared as described in Example 1, was diluted in a mixture containing 395 μl molecular biology grade water (Eppendorf) and 988 μl of 2X SYBR® GREEN PCR Master Mix (Applied Biosystems), which contained enzyme, dye (SYBR GREEN), dNTP's, MgCl. This is referred to later as the PCR reaction mixture.

Also an inter-assay PCR control was prepared. 1μg of pre-aliquoted universal human reference RNA (Stratagene, La Jolla, CA)) was reverse transcribed using a MMLV reverse transcription kit (Invitrogen Corp., Carlsbad, Calif) according to the manufacturer's recommendations in a final volume of 20μl. After reverse transcription 180μl of molecular biology grade water was added and aliquoted in tubes with 15μl each. Subsequently, 1 aliquot (15μl) was diluted into a mixture containing 7.5 μl molecular biology grade water (Eppendorf/) and 37.5 μl of 2X SYBR® GREEN PCR Master Mix (Applied Biosystems).

Mixture plating:

A PCR plate prepared as described above was defrosted 5 minute before use. 20 μl of the PCR reaction mixture containing target sample were distributed per well into wells of the PCR plate. 20 μl of interassay PCR control was distributed into 2 wells which did not contain target sample PCR reaction mixture. The PCR plate was closed using adhesive covers, vortexed and quickly centrifuged.

Performing a PCR run using ABI Prism 7000 Taqman instrument: The PCR plate was inserted into an ABI Prism 7000 Taqman instrument (Applied Biosystems) and a PCR run was performed according to the instructions of the manufacturer. PCR cycling was carried out as follow: 1) plateau at 95°C for 10 minutes 2) 40 cycles with 20 seconds at 95°C and for 1 minute at 60°C, followed by dissociation protocols (melting point analysis).

At the end of the run, the relative gene quantities were obtained after normalization to the 18S rRNA according to the following formula

$$\text{Delta cT} = \text{cT(18s rRNA)} - \text{cT (gene of interest)}$$

The cT (cycle threshold) value specifies after how many PCR cycles a gene is detected at a specified threshold baseline (fluorescence level). Background fluorescence (noise) calculation was performed between cycle 6 to 15 for all genes except 18SrRNA. A threshold baseline was fixed at 0.25 for all genes and cT values calculated. The delta cT value corresponds to a normalised cT value for individual genes by subtracting the cT value of a gene of interest from the cT-value of the reference gene (18S rRNA).

Example 3: Quantitative real-time PCR assay to determine (SEQ ID NO. 37, 38) ERBB2 status

After evaluation of 575 breast cancer biopsies using PCR (methodology and conditions are as described in Example 1 and 2), (SEQ ID NO. 37, 38) ERBB2 RNA expression levels revealed a clear bi-modal distribution (Figure 1).

Results suggest that FISH is more accurate than IHC in predicting patient outcome and response to trastuzumab (Herceptin®) (Duffy MJ. Predictive markers in breast and other cancers: a review. Clin Chem 2005;51(3):494-503.). Therefore, (SEQ ID NO. 37, 38) ERBB2 DNA amplification measured by FISH (n=100 cases) was used to define a cutoff value for (SEQ ID NO. 37, 38) ERBB2 mRNA status measured by PCR. Importantly, all 100 biopsies analyzed by PCR were obtained from a single pathological institute (Institute of Pathology, University of Basel) which also performed all FISH analysis, and which is a reference center for FISH analysis. Receiver operator characteristics (ROC) analysis ( Zweig M et al., Receiver operating characteristic (ROC) plots: A fundamental evaluation tool in clinical medicine, Clin Chem. 1993 Apr 39(4)561-77) was applied to assess sensitivity and specificity of the PCR method compared to FISH. There was very good concordance between the two methods (Figure 2).

The sensitivity of a test is the probability that the test is positive when the true test result is positive.

$$\text{Sensitivity} = TP / (TP + FN)$$

where TP and FN are the number of true positive and false negative results, respectively.

The specificity of a test is the probability that the test will be negative when the true test result is negative.

$$\text{Specificity} = TN / (TN + FP)$$

where TN and FP and the number of true negative and false positive results, respectively.

Subsequently, a cutoff value for (SEQ ID NO. 37, 38) ERBB2 mRNA status by PCR was calculated setting the sensitivity to be 95% and using (SEQ ID NO. 37, 38) ERBB2 amplification by FISH as the reference. The resulting specificity and accuracy were 94% each. This corresponded to a delta-CT value for (SEQ ID NO. 37, 38) ERBB2 mRNA expression level of -15.8 (cT[18S]-cT[(SEQ ID NO. 37, 38) ERBB2]) (Figure 3) or a "raw" cT value of 22.3 (cT[(SEQ ID NO. 37, 38) ERBB2]) per 12.5ng total RNA. Thus, a patient would be considered as (SEQ ID NO. 37, 38) ERBB2-if:
delta-cT (cT[18S]-cT[ERBB2]) > -15.8 (cycles)
or cT[ERBB2] < 22.3 cT/12.5 ng total RNA

Example 4: Quantitative real-time PCR assay to determine (SEQ ID NO. 45, 46) ESR1 Status

Estrogen receptor (SEQ ID NO. 45, 46) ESR1, also commonly referred to as ER) status was also determined based on mRNA expression levels. Methodology and conditions used to extract RNA and perform PCR are described in detail in Example 1 and 2.

(SEQ ID NO. 45, 46) ESR1 mRNA expression levels measured by PCR showed a bi-modal distribution (Figure 4). (SEQ ID NO. 45, 46) ESR1 mRNA expression levels by PCR were compared to (SEQ ID NO. 45, 46) ESR1 protein expression levels detected by ELISA in 317 primary breast cancer biopsies. ROC analysis showed high concordance between the two methods (Figure 5). Cutoff value for (SEQ ID NO. 45, 46) ESR1 mRNA expression levels was determined using regression analysis and set to be equivalent to a protein cutoff (ELISA) of 20 fmol/mg protein (Figure 6). This corresponds to a delta-CT value of -19 (cT[18S]-cT[(SEQ ID NO. 45, 46) ESR1]) or a "raw" cT value of 25.5 (cT[(SEQ ID NO. 45, 46) ESR1]) per 12.5 ng total RNA. The sensitivity was 94%, specificity 82% and accuracy 91%. In summary, a patient would be considered as (SEQ ID NO. 45, 46) ESR1 positive if:
delta-cT(cT[18S]-cT[ESR1]) > -19 (cycles)
or cT[ESR1] < 25.5 cT/12.5 ng total RNA

Therapy decisions can be made based on the method according to the invention in combination with (SEQ ID NO. 45, 46) ESR1 status determination.

Example 5: Identification and Ranking of Prognostic Genes

317 breast cancer biopsy samples were analysed. All patients underwent primary surgery between 1992 and 1996. Median age at diagnosis was 60 years (range 27 to 88). 90 (30%) patients relapsed and 57 (18%) patients developed distant metastasis. Median relapse-free survival and median metastasis-free survival time was 44 months each. 143 (53%) patients were node-positive, 231 (73%) (SEQ ID NO. 45, 46) ESR1-positive (>20 fmol/mg protein), and 82 (26%) (SEQ ID NO. 37, 38) ERBB2-positive (> -15.8 cycles by PCR). Systemic adjuvant hormone therapy was administrated to 135 (43%), chemotherapy to 72 (22%) and combination therapy to 50 (16%) patients. None of the patients received neoadjuvant therapy or (SEQ ID NO. 37, 38) ERBB2-targeted therapy.

The samples were prepared and the gene expression levels measured using the methodology and conditions as described in Examples 1 and 2. The genes studied were those listed in Table 1. The (SEQ ID NO. 37, 38) ERBB2 status and (SEQ ID NO. 45, 46) ESR1 status were determined according to the cut off values determined in Examples 3 and 4 ie
(SEQ ID NO. 37, 38) ERBB2 positive if:
delta-cT (cT[18S]-cT[(ERBB2]) > -15.8 (cycles)
or cT[ERBB2] < 22.3 cT/12.5 ng total RNA
(SEQ ID NO. 45, 46) ESR1 positive if:
delta-cT (cT[18S]-cT[ESR1]) > -19 (cycles)
or cT[ESR1] < 25.5 cT/12.5 ng total RNA

The prognostic value of genes was assessed by univariate Cox regression against relapse-free (RFS), distant metastasis-free (MFS) and overall survival (OS). A description of Cox-regression is to be found in the publication Cox DR: Regression Models and Lifetables J.R. Soc. 34: 187-220, 1972.

Analysis was performed in (SEQ ID NO. 37, 38) ERBB2-positive and (SEQ ID NO. 37, 38) ERBB2-negative patients separately, and with and without stratification by (SEQ ID NO. 45, 46) ESR1 status and treatment group. Genes were ranked according to their p-value in each group ((SEQ ID NO. 37, 38) ERBB2 positive and (SEQ ID NO. 37, 38) ERBB2 negative). The results revealed that the prognostic value of the 75 genes surprisingly differed substantially according to (SEQ ID NO. 37, 38) ERBB2 status. Those with a p-value less than 0.1 are shown for MFS in Tables 4 and 5.

The tables 4 and 5 also include the Cox-coefficient, hazard ratio (HR) and level of significance (p-value) for each gene. Cox-coefficients were used as weights in the prognostic score (see below).

Table 4. Univariate Cox, MFS, (SEQ ID NO. 37, 38) ERBB2-positive samples.

| Gene | Cox Coefficient | Hazard Ratio | p-Value |
|---|---|---|---|
| (SEQ ID NO. 73, 74) PLAU | 0.519404159 | 1.681025727 | 0.010035908 |
| (SEQ ID NO. 77, 78) PLAUR | 0.513453392 | 1.671052039 | 0.021442621 |
| (SEQ ID NO. 65, 66) MMP11 | 0.329722687 | 1.390582448 | 0.022349904 |
| (SEQ ID NO. 49, 50) MMP1 | 0.178773462 | 1.195749831 | 0.02493824 |
| (SEQ ID NO. 57, 58) MMP3 | 0.286152341 | 1.331295251 | 0.036556477 |
| (SEQ ID NO. 93, 94) TIMP3 | 0.314571596 | 1.369672412 | 0.05542383 |
| (SEQ ID NO. 41, 42) ERBB3 | -0.33623338 | 0.714456344 | 0.056972358 |
| (SEQ ID NO. 33, 34) ECGF1 | 0.422523747 | 1.525807452 | 0.076088667 |
| (SEQ ID NO. 81, 82) SERPINE1 | 0.275693224 | 1.317443642 | 0.076627596 |
| (SEQ ID NO. 53, 54) | | | |

(continued)

| Gene | Cox Coefficient | Hazard Ratio | p-Value |
|---|---|---|---|
| MMP2 | 0.285592031 | 1.330549522 | 0.098816859 |

Table 5. Univariate Cox, MFS, (SEQ ID NO. 37, 38) ERBB2-negative samples.

| Gene | Cox Coefficient | Hazard Ratio | p-Value |
|---|---|---|---|
| (SEQ ID NO. 9, 10) BIRC5 | 0.315945286 | 1.37155521 | 0.000431973 |
| (SEQ ID NO. 29, 30) E2F1 | 0.375411367 | 1.455590073 | 0.000811719 |
| (SEQ ID NO. 105, 106) TOP2A | 0.269379935 | 1.309152439 | 0.00318208 |
| (SEQ ID NO. 101, 102) TK1 | 0.3453796 | 1.412526013 | 0.003898848 |
| (SEQ ID NO. 109, 110) TYMS | 0.313585409 | 1.368322325 | 0.006069729 |
| (SEQ ID NO. 113, 114) VEGF | 0.270465209 | 1.310574 | 0.013399219 |
| (SEQ ID NO. 85, 86) SRD5A1 | 0.202704459 | 1.224710463 | 0.018686172 |
| (SEQ ID NO. 13, 14) CCND1 | 0.250139642 | 1.284204733 | 0.023188033 |
| (SEQ ID NO. 25, 26) CTSD | 0.347293242 | 1.41523167 | 0.032034051 |
| (SEQ ID NO. 5, 6) BAX | 0.513971316 | 1.671917742 | 0.038030789 |
| (SEQ ID NO. 21, 22) CTSB | 0.267234911 | 1.306347286 | 0.042570705 |
| (SEQ ID NO. 17, 18) CTNNB1 | 0.373064118 | 1.452177447 | 0.053195743 |
| (SEQ ID NO. 1, 2) ADM | 0.177200297 | 1.193870198 | 0.054674004 |
| (SEQ ID NO. 61, 62) MMP9 | 0.153711516 | 1.166154422 | 0.054743765 |
| (SEQ ID NO. 89, 90) STK11 | 0.26430051 | 1.302519558 | 0.070879025 |
| (SEQ ID NO. 97, 98) TIMP4 | -0.19485774 | 0.822951723 | 0.078932524 |

Therefore this analysis revealed that a specific subset of genes is important in the prognosis of (SEQ ID NO. 37, 38) ERBB2-positive patients (Table 4), and another second subset is important in the prognosis of (SEQ ID NO. 37, 38) ERBB2-negative patients (Table 5).

Table 6. Genes important in prognosis of cancer

| Official Symbol | Name | Ref. Seq. | cDNA SEQ ID NO | DNA SEQ ID NO |
|---|---|---|---|---|
| ERBB2 | Epidermal Growth Factor receptor 2 | NM_004448 | 37 | 38 |
| PLAU | Plasminogen activator, urokinase | NM_002658 | 73 | 74 |
| PLAUR | Plasminogen activator, urokinase receptor | NM_002659 | 77 | 78 |
| MMP11 | Metalloprotease 11 | NM_005940 | 65 | 66 |

(continued)

| Official Symbol | Name | Ref. Seq. | cDNA SEQ ID NO | DNA SEQ ID NO |
|---|---|---|---|---|
| MMP1 | Metalloprotease 1 | NM_002421 | 49 | 50 |
| MMP3 | Metalloprotease 3 | NM_002422 | 57 | 58 |
| TIMP3 | Tissue inhibitor of Metalloproteases 3 | NM_000362 | 93 | 94 |
| ERBB3 | Epidermal Growth Factor receptor 3 | NM_001982 | 41 | 42 |
| ECGF1 | Thymidine Phosphorylase | NM_001953 | 33 | 34 |
| SERPINE1 | Plasminogen Activator Inhibitor 1 | NM_000602 | 81 | 82 |
| MMP2 | Metalloprotease 2 | NM_004530 | 53 | 58 |
| BIRC5 | Survivin | NM_001168 | 9 | 10 |
| E2F1 | Transcription factor E2F1 | NM_005225 | 29 | 30 |
| TOP2A | Topoisomerase II alpha | NM_001067 | 105 | 106 |
| TK1 | Thymidine kinase 1 | NM_003258 | 101 | 102 |
| TYMS | Thymidilate synthase | NM_001071 | 109 | 110 |
| VEGF | Vascular Endothelial Growth Factor A | NM_003376 | 113 | 114 |
| SRDSA1 | Steroid 5-alpha-reductase | NM_001047 | 85 | 86 |
| CCND1 | Cyclin D1 | NM_053056 | 13 | 14 |
| ( CTSD | Cathepsin D | NM_001909 | 25 | 26 |
| BAX | BCL2-associated protein X | NM_138761 | 5 | 6 |
| CTSB | Cathepsin B | NM_001908 | 21 | 22 |
| CTNNB1 | Catenin, beta 1 | NM_001904 | 17 | 18 |
| ADM | Adrenomedullin | NM_001124 | 1 | 2 |
| MMP9 | Metalloprotease 9 | NM_004994 | 63 | 64 |
| STK11 | LKB1 | NM_000455 | 89 | 90 |
| TIMP4 | Tissue inhibitor of Metalloproteases 4 | NM_003256 | 97 | 98 |
| ERBB2 | Epidermal Growth Factor receptor 2 | NM_004448 | 37 | 38 |
| ESR1 | Estrogen receptor alpha | NM_000125 | 45 | 46 |

Listed above are the sequence ID numbers for each gene in table 6. Accompanying this application is the DNA sequence for each gene and a corresponding mRNA sequence for each gene, taken from the GenBank database. The GenBank accession numbers listed above in Table 6 (with the prefix NM_) are for the corresponding mRNA sequence. As the mRNA sequence is listed in the GenBank database as the corresponding cDNA sequence, this has been indicated in the accompanying sequence listings as DNA and referred to with the sequence ID name gene X cDNA. However it is intended with this to disclose the mRNA sequence. These mRNA sequences were used to design the corresponding primers listed in Tables 2 and 3. The corresponding primers in Tables 2 and 3 are also listed in the accompanying sequence listings. Thus, for example, for the gene ADM, the DNA sequence is SEQ ID NO. 1, the cDNA (corresponding to mRNA) sequence is SEQ ID NO 2, the forward primer is SEQ ID NO. 3 and the reverse primer is SEQ ID NO. 4. The information recorded in computer readable form is identical to the written sequence listing.

Example 6: <u>Evaluation and Performance Assessment of the Prognostic score</u>

317 breast cancer samples were used as described in example 5 and gene expression levels were measured using the methodology and conditions as described in Examples 1 and 2. The genes studied were those listed in Table 1. The (SEQ ID NO. 37, 38) ERBB2 status was determined according to the cut off value determined in Examples 3.

To assess the performance of the prognostic score as described in formula I and the ability to predict new data, a cross-validation procedure was used where a rule was created on one set of data (the training set) and then was applied to an independent set of data (the validation set). The test set emulates the set of future samples for which class labels or the outcome variable is to be predicted. Consequently, the test samples are not used for the development of the prediction model (R Simon, British Journal of Cancer 2003 (89), p1599).

Therefore, samples were randomly rearranged and split into a training set (two thirds of the samples) and a test set (remaining third). Univariate Cox-coefficients were assessed for each gene using only the training set samples. Prognostic scores were calculated for both the training set and test set samples using the coefficients established in the training set.

In order to estimate the outcome as a function of the prognostic score (e.g. the 5-year survival or hazard ratio for a given prognostic score), a cutoff-value (in percentiles) was determined for the distribution of prognostic scores in training set and applied to dichotomize the test set samples according to their prognostic scores. Subsequently, the probability of 5-year survival (RFS, MFS or OS) was determined for each of the dichotomized groups in the test set (e.g. the samples above the cutoff value and the samples below the the training set cutoff value). The entire procedure was repeated 1000 times for each individual cutoff starting typically with the $10^{th}$ percentile up to the $90^{th}$ percentile of the distribution of prognostic scores determined in the training set. 5-year survival probabilities were summarized in graphs and plotted as a function of the cutoff (in percentiles). Survival probabilities were calculated according to the Kaplan-Meier method (M Tableman et al , Survival Analysis Using S, 2004, Chapman & Hall/CRC). Coefficients (and hazard ratios) were assessed by univariate Cox regression analysis. The results of this procedure are shown in figures 7 to 26 and summarized in tables 7 and 8.

Each percentile cutoff has two corresponding data points: the clear circle represents the probability of 5-year survival (e.g. being metastasis-free or relapse-free after 5 years) given the PS of the test set (test sample) is lower than the x-th percentile (below cutoff) of the PS in the training set. Accordingly, the black, filled-in circles correspond to the probability of 5-year survival (e.g. MFS, or RFS) given the PS of the test set (test sample) is higher than the x-th percentile (above cutoff) of the PS in the training set. Each data point itself summarises the result of 1000 random training/test sets for each cutoff. These graphs are the basis for tables 7 and 8, and to estimate the outcome of a new sample as described in example 7.

Analogous procedure was used to assess hazard ratios as a function of the prognostic score and to estimate the performance depending on the number of genes used in the prognostic score, thereby using a specific cutoff value for the prognostic score (e.g. the $30^{th}$ and $70^{th}$ percentile) and increasing the number of genes in the score. It was found that the performance (hazard ratio) increases with the number of genes used in the prognostic score and that at least three genes, more preferably four or more genes should be used.

Table 7. Expected prognosis (5-year MFS and RFS) for different genes and gene combinations (prognostic scores) depending on different cutoffs, in (SEQ ID NO. 37, 38) ERBB2-positives.

| Gene/ Combination | Cutoff MFS (Percentil e) | Prognosis 5year-MFS | Cutoff RFS (Percentil e) | Prognosis 5year-RFS |
|---|---|---|---|---|
| (SEQ ID NO. 73, 74) PLAU | <40<br>>50-80<br>>80 | 80-80%<br>50-60%<br><50 | <50<br>>50 | 80-90%<br>50-60% |
| (SEQ ID NO. 77, 78) PLAUR | <15<br><15-30<br><30-50<br>>50-75<br>>75 | >90%<br>80-90%<br>70-80%<br>50-60%<br><50% | <15<br><15-30<br><30-50<br>>50<br>>65 | >90%<br>80-90%<br>60-80%<br><50%<br><40% |
| (SEQ ID NO. 65, 66) MMP11 | <15<br><15-30<br>>50-75<br>>75 | >90%<br>80-90%<br>50-60%<br><50% | <15<br><15-50<br>>50<br>>60 | >90%<br>70-80%<br><50%<br><40% |
| (SEQ ID NO. 73, 74) PLAU, (SEQ ID NO. 77, 78) PLAUR, (SEQ ID NO. 65, 66) MMP11 | <15<br><15-40<br>>50-70<br>>70 | >90%<br>80-90%<br>50-60%<br><50% | <15<br><15-25<br><25-50<br>>50<br>>70 | >90%<br>80-90%<br>60-80%<br><50%<br><40% |

(continued)

| Gene/ Combination | Cutoff MFS (Percentil e) | Prognosis 5year-MFS | Cutoff RFS (Percentil e) | Prognosis 5year-RFS |
|---|---|---|---|---|
| (SEQ ID NO. 77, 78) PLAUR, (SEQ ID NO. 65, 66) MMP11, (SEQ ID NO. 49, 50) MMP1 | <15<br><15-30<br><30-50<br>>50-70<br>>70 | >90%<br>80-90%<br>70-80%<br>50-60%<br><50% | <15<br><15-25<br><25-50<br>>50<br>>70 | >90%<br>80-90%<br>60-80%<br><50%<br><40% |
| (SEQ ID NO. 73, 74) PLAU, (SEQ ID NO. 77, 78) PLAUR, (SEQ ID NO. 65, 66) MMP11, (SEQ ID NO. 49, 50) MMP1 | <15<br><15-30<br><30-50<br>>50-70<br>>70 | >90%<br>80-90%<br>70-80%<br>50-60%<br><50% | <15<br><15-25<br><25-50<br>>50<br>>65 | >90%<br>80-90%<br>60-80%<br><50%<br><40% |
| (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 81, 82) SERPINE1 | <20<br><20-30<br><30-50<br>>50-70<br>>70 | >90%<br>80-90%<br>70-80%<br>50-60%<br><50% | <15<br><15-25<br><25-50<br>>50<br>>65 | >90%<br>80-90%<br>60-80%<br><50%<br><40% |

Table 8. Expected prognosis (5-year MFS and RFS) for different genes and gene combinations (prognostic scores) depending on different cutoffs, in (SEQ ID NO. 37, 38) ERBB2-negatives.

| | | | | |
|---|---|---|---|---|
| (SEQ ID NO. 29, 30) E2F1 | <20<br><20-70<br>>15 | >90%<br>80-90%<br>60-80% | <20<br><20-70<br>>15 | >90%<br>70-80%<br>60-80% |
| (SEQ ID NO. 9, 10) BIRC5 | <50<br>>50-85<br>>85 | 80-90%<br>60-70%<br><60% | <50<br>>50-85<br>>85 | >90%<br>70-80%<br>60-80% |
| (SEQ ID NO. 105, 106) TOP2A | <50<br>>50-75<br>>75 | 80-90%<br>70-80%<br><70% | <15<br><15-60<br>>15 | 80-90%<br>60-70%<br><60% |
| (SEQ ID NO. 29, 30) E2F1, (SEQ ID NO. 9, 10) BIRC5, (SEQ ID NO. 105, 106) TOP2A | <20<br><20-50<br>>50 | >90%<br>80-90%<br>60-70% | <15<br><15-25<br><25-50<br>>50 | 80-90%<br>70-80%<br>60-70 |

(continued)

| | | | | |
|---|---|---|---|---|
| (SEQ ID NO. 29, 30) E2F1, (SEQ ID NO. 9, 10) BIRC5, (SEQ ID NO. 101, 102) TK1 | <25<br><25-50<br>>50 | >90%<br>80-90%<br>60-70% | <15<br><15-30<br><30-50<br>>50 | >90%<br>80-90%<br>70-80%<br>60-70% |
| (SEQ ID NO. 29, 30) E2F1, (SEQ ID NO. 9, 10) BIRC5, (SEQ ID NO. 109, 110) TYMS | <20<br><20-50<br>>50 | >90%<br>80-90%<br>60-70% | <15<br><15-25<br><25-50<br>>50 | >90%<br>80-90%<br>70-80%<br>60-70% |
| (SEQ ID NO. 29, 30) E2F1, (SEQ ID NO. 9, 10) BIRC5, (SEQ ID NO. 105, 106) TOP2A, (SEQ ID NO. 101, 102) TK1 | <20<br><20-50<br>>50 | >90%<br>80-90%<br>60-70% | <15<br><15-30<br><30-50<br>>50 | >90%<br>80-90%<br>70-80%<br>60-70% |
| (SEQ ID NO. 29, 30) E2F1, (SEQ ID NO. 9, 10) BIRC5, (SEQ ID NO. 105, 106) TOP2A, (SEQ ID NO. 101, 102) TK1, (SEQ ID NO. 21, 22) CTSB, (SEQ ID NO. 25, 26) CTSD | <20<br><20-50<br>>50 | >90%<br>80-90%<br>60-70% | <15<br><15-30<br><30-50<br>>50 | >90%<br>80-90%<br>70-80%<br>60-70% |

Example 7: Analysis and Classification of a New Sample

The following example illustrates use of the method of the invention. A core biopsy is taken from patient X diagnosed with breast cancer. This core biopsy is analyzed using the tissue preparation, RNA extraction and qRT-PCR methods as described in examples 1 and 2. (SEQ ID NO. 37, 38) ERBB2 status is determined as described example 3. The delta CT value for (SEQ ID NO. 37, 38) ERBB2 is -14.5, and thus the sample is classified (SEQ ID NO. 37, 38) ERBB2 positive.

Subsequently, a prognostic score is calculated using a gene combination of 4 genes ((SEQ ID NO. 73, 74) PLAU, (SEQ ID NO. 77, 78) PLAUR, (SEQ ID NO. 65, 66) MMP11, (SEQ ID NO. 49, 50) MMP1) by applying the prognostic score formula I and the weights (coefficients) from table 4. The established score of patient X is -29. Compared with the distribution of prognostic scores in a reference population (e.g. 317 breast cancer samples as described in example 5) determined with the same 4 genes and the formula I, the score of patient X corresponds to the $18^{th}$ percentile. According to figure 7 and table 7 patient X has an expected 5-year MFS of 80 to 90%. Figure 7 and table 7 were established using the methods described in example 6.

Although patient X is (SEQ ID NO. 37, 38) ERBB2-positive, according to his low prognostic score ($18^{th}$ percentile) patient X is expected to have a good prognosis (estimated 5-year MFS of 80 to 90%). This is in contrast to the latest St.

Gallen consensus meeting recommendations which classifies all (SEQ ID NO. 37, 38) ERBB2-positive patients in the highest risk category.

## Claims

**1.** A method used for assessing the probability of survival of a cancer patient by assaying one or more ex-vivo samples from that patient, wherein the (SEQ ID NO. 37, 38) ERBB2 status of that patient is determined to be positive or negative; **characterized in that**

a) in (SEQ ID NO. 37, 38) ERBB2-positive patients, at least one of a first set of cancer related genes is analysed in the sample or in one of the samples from said patient
and

b) in (SEQ ID NO. 37, 38) ERBB2-negative patients, at least one of a second set of cancer related genes is analysed in the sample or in one of the samples from said patient.

2. A method according to claim 1, wherein the probability of survival of the cancer patient is assessed in terms of metastasis free survival (MFS).

3. A method according to claim 1, wherein the probability of survival of the cancer patient is assessed in terms of relapse free survival (RFS).

4. A method according to any one of claims 1 to 3, wherein the first set of cancer related genes comprises (SEQ ID NO. 73, 74) PLAU, (SEQ ID NO. 77, 78) PLAUR, (SEQ ID NO. 65, 66) MMP11, (SEQ ID NO. 49, 50) MMP1, (SEQ ID NO. 57, 58) MMP3, (SEQ ID NO. 93, 94) TIMP3, (SEQ ID NO. 41, 42) ERBB3, (SEQ ID NO. 33, 34) ECGF1, (SEQ ID NO. 81, 82) SERPINE1, and (SEQ ID NO. 53, 54) MMP2.

5. A method according to any one of claims 1 to 4, wherein the second set of genes comprises (SEQ ID NO. 9, 10) BIRC5, (SEQ ID NO. 29, 30) E2F1, (SEQ ID NO. 105, 106) TOP2A, (SEQ ID NO. 101, 102) TK1, (SEQ ID NO. 109, 110) TYMS, (SEQ ID NO. 113, 114) VEGF, (SEQ ID NO. 85, 86) SRD5A1, (SEQ ID NO. 13, 14) CCND1, (SEQ ID NO. 25, 26) CTSD, (SEQ ID NO. 5, 6) BAX, (SEQ ID NO. 21, 22) CTSB, (SEQ ID NO. 17, 18) CTNNB1, (SEQ ID NO. 1, 2) ADM, (SEQ ID NO. 61, 62) MMP9, (SEQ ID NO. 89, 90) STK11 and (SEQ ID NO. 97, 98) TIMP4.

6. A method according to claim 4 or 5, wherein in (SEQ ID NO. 37, 38) ERBB2 positive patients the gene combination of (SEQ ID NO. 73, 74) PLAU, (SEQ ID NO. 77, 78) PLAUR, (SEQ ID NO. 65, 66) MMP11, and (SEQ ID NO. 49, 50) MMP1 is analysed.

7. A method according to claim 4 or 5, wherein in (SEQ ID NO. 37, 38) ERBB2-positive patients the gene combination of (SEQ ID NO. 73, 74) PLAU, (SEQ ID NO. 77, 78) PLAUR and (SEQ ID NO. 65, 66) MMP11 is analysed.

8. A method according to claim 4 or 5, wherein in (SEQ ID NO. 37, 38) ERBB2-positive patients the gene combination of (SEQ ID NO. 77, 78) PLAUR, (SEQ 10 NO. 65, 66) MMP11 and (SEQ ID NO. 49, 50) MMP1 is analysed.

9. A method according to any one of claims 6 to 8, wherein in (SEQ ID NO. 37, 38) ERBB2-positive patients additionally (SEQ ID NO. 57, 58) MMP3 is analysed.

10. A method according to any one of claims 6 to 9, wherein in (SEQ ID NO. 37, 38) ERBB2-positive patients additionally one or both of (SEQ ID NO. 93, 94) TIMP3 and (SEQ ID NO. 53, 54) MMP2 are analysed.

11. A method according to any one of claims 6 to 10, wherein in (SEQ ID NO. 37, 38) ERBB2-positive patients additionally (SEQ ID NO. 81, 82) SERPINE1 is analysed.

12. A method according to any one of claims 5 to 11, wherein in (SEQ ID NO. 37, 38) ERBB2-negative patients the combination of (SEQ ID NO. 9, 10) BIRC5, (SEQ ID NO. 29, 30) E2F1, (SEQ ID NO. 105, 106) TOP2A and (SEQ ID NO. 101, 102) TK1 is analysed.

13. A method according to any one of claims 5 to 11, wherein in (SEQ ID NO. 37, 38) ERBB2-negative patients the combination (SEQ ID NO. 9, 10) BIRC5, (SEQ ID NO. 29, 30) E2F1 and (SEQ ID NO. 105, 106) TOP2A is analysed.

14. A method according to any one of claims 5 to 11, wherein in (SEQ ID NO. 37, 38) ERBB2-negative patients the combination (SEQ ID NO. 9, 10) BIRC5, (SEQ ID NO. 29, 30) E2F1 and (SEQ ID NO. 101, 102) TK1 is analysed.

15. A method according to any one of claims 5 to 11, wherein in (SEQ ID NO. 37, 38) ERBB2-negative patients the combination (SEQ ID NO. 9, 10) BIRC5, (SEQ ID NO. 29, 30) E2F1 and (SEQ ID NO. 109, 110) TYMS is analysed.

16. A method according to any one of claims 5 to 11, wherein in (SEQ ID NO. 37, 38) ERBB2-negative patients the combination (SEQ ID NO. 9, 10) BIRC5, (SEQ ID NO. 29, 30) E2F1, (SEQ ID NO. 101, 102) TK1 and (SEQ ID NO. 109, 110) TYMS is analysed.

17. A method according to any one of claims 12 to 15, wherein in (SEQ ID NO. 37, 38) ERBB2-negative patients additionally one or both of (SEQ ID NO. 21, 22) CTSB or (SEQ ID NO. 25, 26) CTSD is analysed.

**18.** A method according to any one of claims 1 to 17, wherein the analysis of the genes involves measuring the expression levels of the genes at the mRNA level.

**19.** A method according to any one of claims 1 to 18, wherein the (SEQ ID NO. 37, 38) ERBB2 status is determined at the mRNA level by qRT-PCR by measuring the cycle threshold value for (SEQ ID NO. 37, 38) ERBB2 and for 18S rRNA, subtracting the cycle threshold value for (SEQ ID NO. 37, 38) ERBB2 from the cycle threshold value for 18S rRNA and wherein a value of ≥ -15.8 is determined to be positive (SEQ ID NO. 37, 38) ERBB2 status.

**20.** A method according to any one of claims 1 to 19, wherein within the first set of genes each gene is statistically weighted according to its association with survival in a population of cancer patients and within the second set of genes each gene is statistically weighted according to its association with survival in a population of cancer patients.

**21.** A method according to claim 20, wherein the statistical weighting of the genes in the first set of genes and the statistical weighting of the genes in the second set of genes is expressed in terms of the Cox-coefficient.

**22.** A kit for assessing the probability of survival of a patient of whom the (SEQ ID NO. 37, 38) ERBB2 status is known to be positive; comprising a set of oligonucleotide primers (SEQ ID NO. 75, 76, 79, 80, 67, 68, 51, 52), said primer set allowing PCR to be performed on mRNA transcribed from the following genes: (SEQ ID NO. 73, 74) PLAU, (SEQ ID NO. 77, 78) PLAUR, (SEQ ID NO. 65, 66) MMP11 and (SEQ ID NO. 49, 50) MMP1.

**23.** A kit for assessing the probability of survival of a patient of whom the (SEQ ID NO. 37, 38) ERBB2 status is known to be negative; comprising a set of oligonucleotide primers (SEQ ID NO. 11, 12, 31, 32, 107, 108, 103, 104), said primer set allowing PCR to be performed on mRNA transcribed from the following genes: (SEQ ID NO. 9, 10) BIRC5, (SEQ ID NO. 29, 30) E2F1, (SEQ ID NO. 105, 106) TOP2A and (SEQ ID NO. 101, 102) TK1.

**24.** A kit comprising the kit of claim 22 and the kit of claim 23.

**25.** A kit for assessing the probability of survival of a patient comprising the oligonucleotide primers in table 2 (SEQ ID NO. 3, 7, 11, 15, 19, 23, 27, 31, 35, 39, 43, 47, 51, 55, 59, 63, 67, 71, 75, 79, 83, 87, 91, 95, 99, 103, 107, 111, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207), and table 3 (SEQ ID NO. 4, 8, 12, 16, 20, 24, 28, 32, 36, 40, 44, 48, 52, 56, 60, 64, 68, 72, 76, 80, 84, 88, 92, 96, 100, 104, 108, 112, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208).

**26.** Use of the genes (SEQ ID NO. 73, 74) PLAU, (SEQ ID NO. 77, 78) PLAUR, (SEQ ID NO. 65, 66) MMP11 and (SEQ ID NO. 49, 50) MMP1 in cancer prognosis.

**27.** Use of the genes (SEQ ID NO. 9, 10) BIRC5, (SEQ ID NO. 29, 30) E2F1, (SEQ ID NO. 105, 106) TOP2A and (SEQ ID NO. 101, 102) TK1 in cancer prognosis.

Figure 1

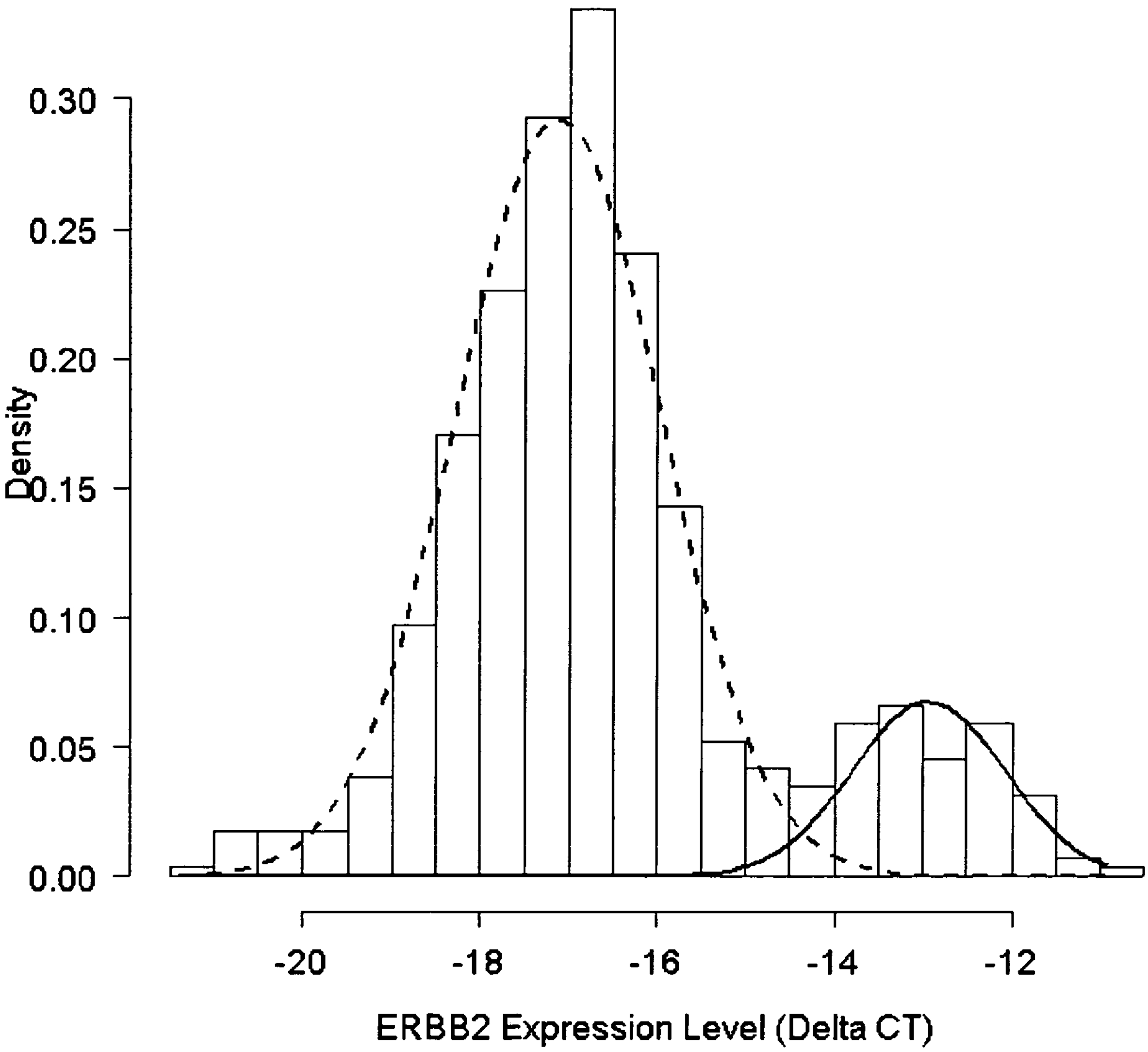
0.30
0.25
0.20
0.15
0.10
0.05
0.00
Density
-20
-18
-16
-14
-12
ERBB2 Expression Level (Delta CT)

# Figure 2

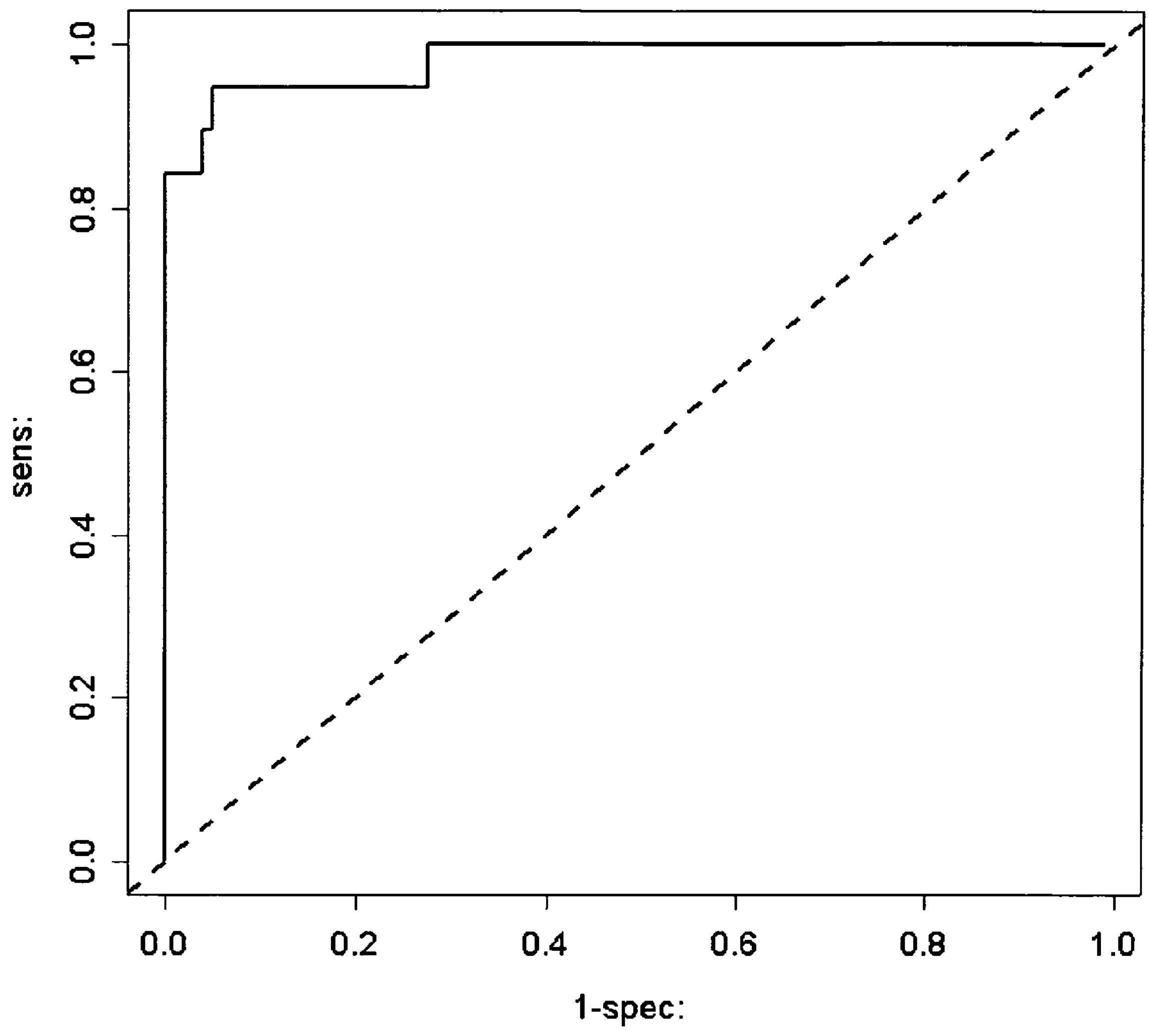

Figure 3

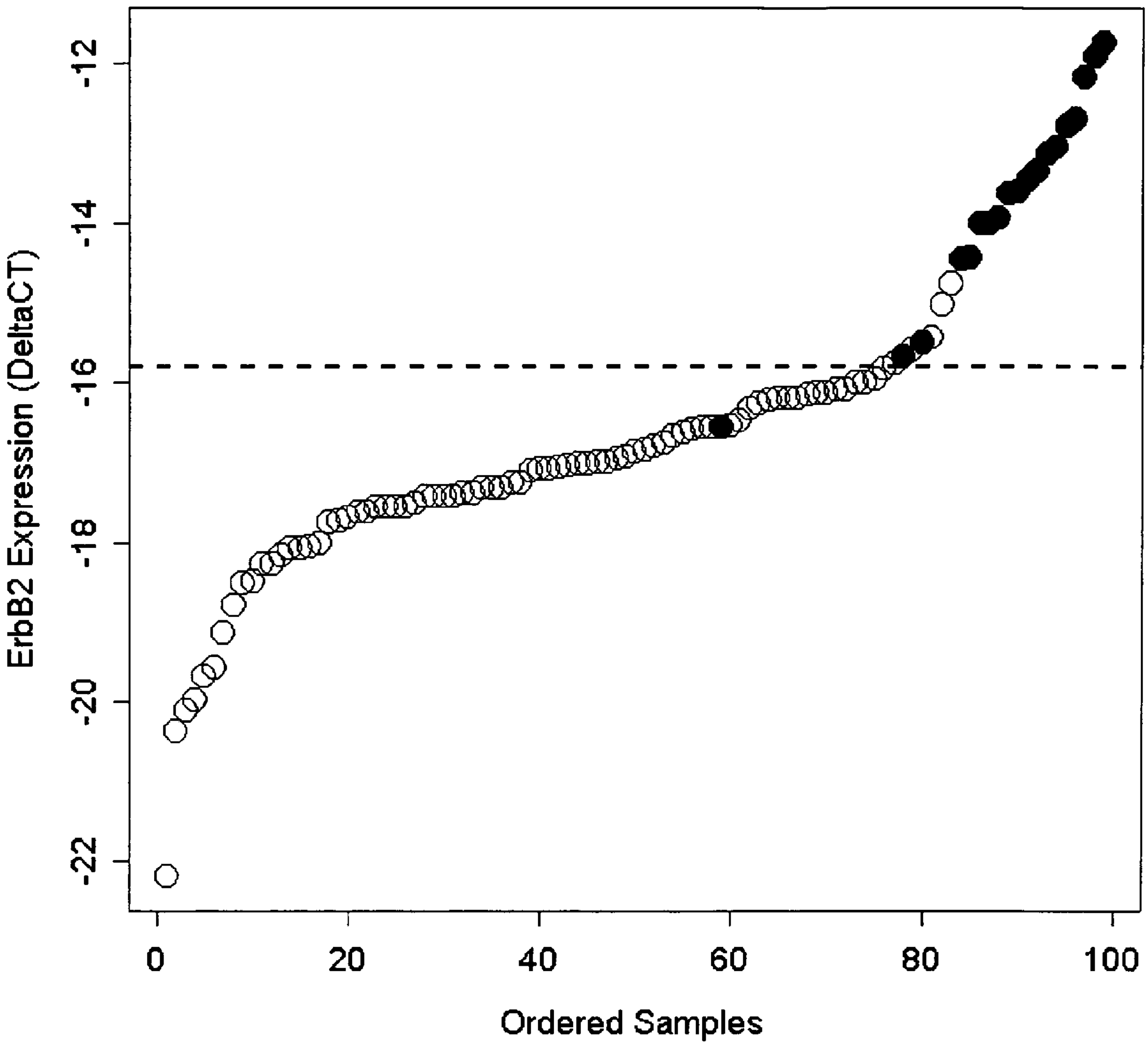
ErbB2 Expression (DeltaCT)
-12
-14
-16
-18
-20
-22
0
20
40
60
80
100
Ordered Samples

**Figure 4**

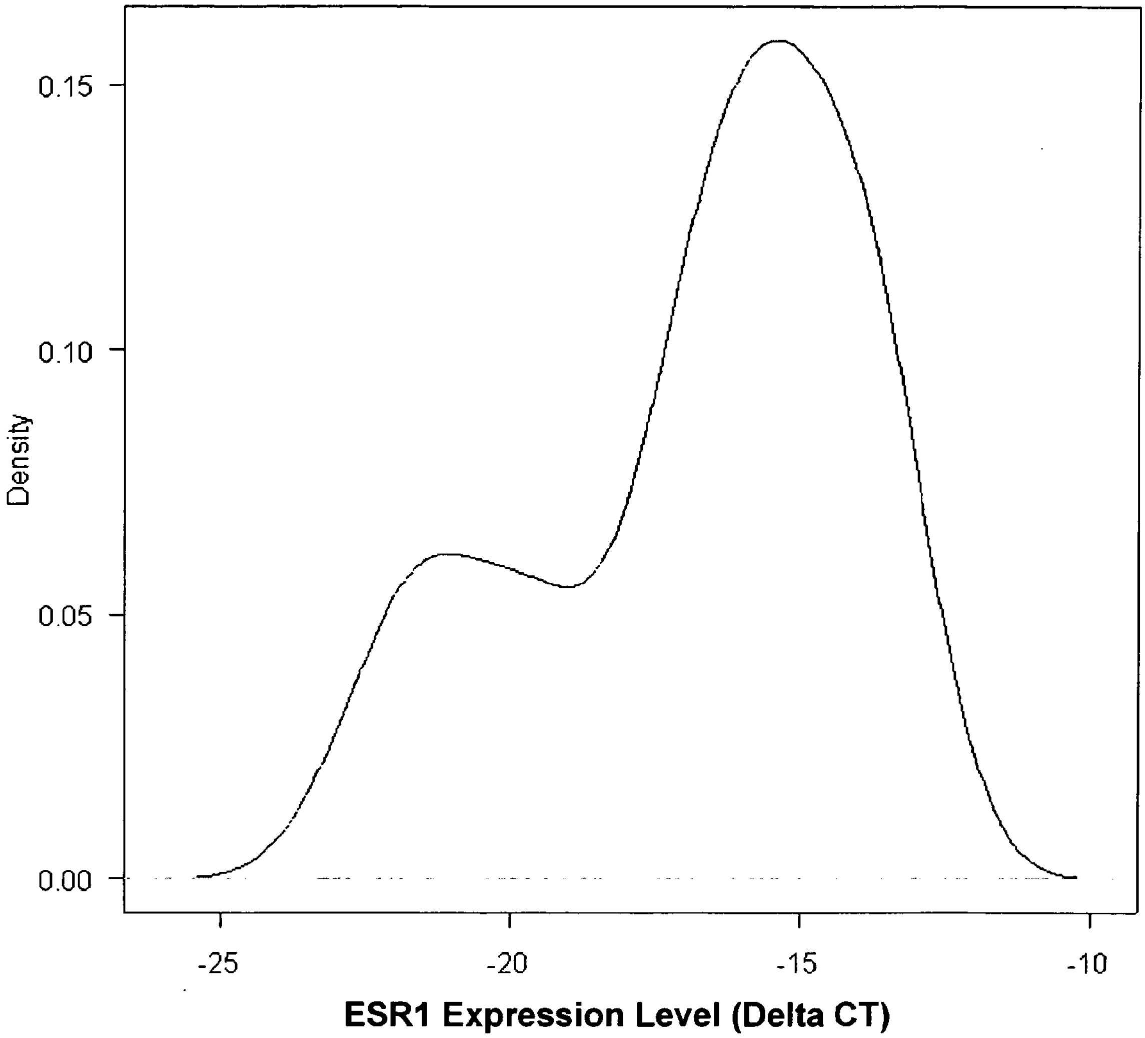
0.15
0.10
0.05
0.00
Density
-25
-20
-15
-10
ESR1 Expression Level (Delta CT)

Figure 5

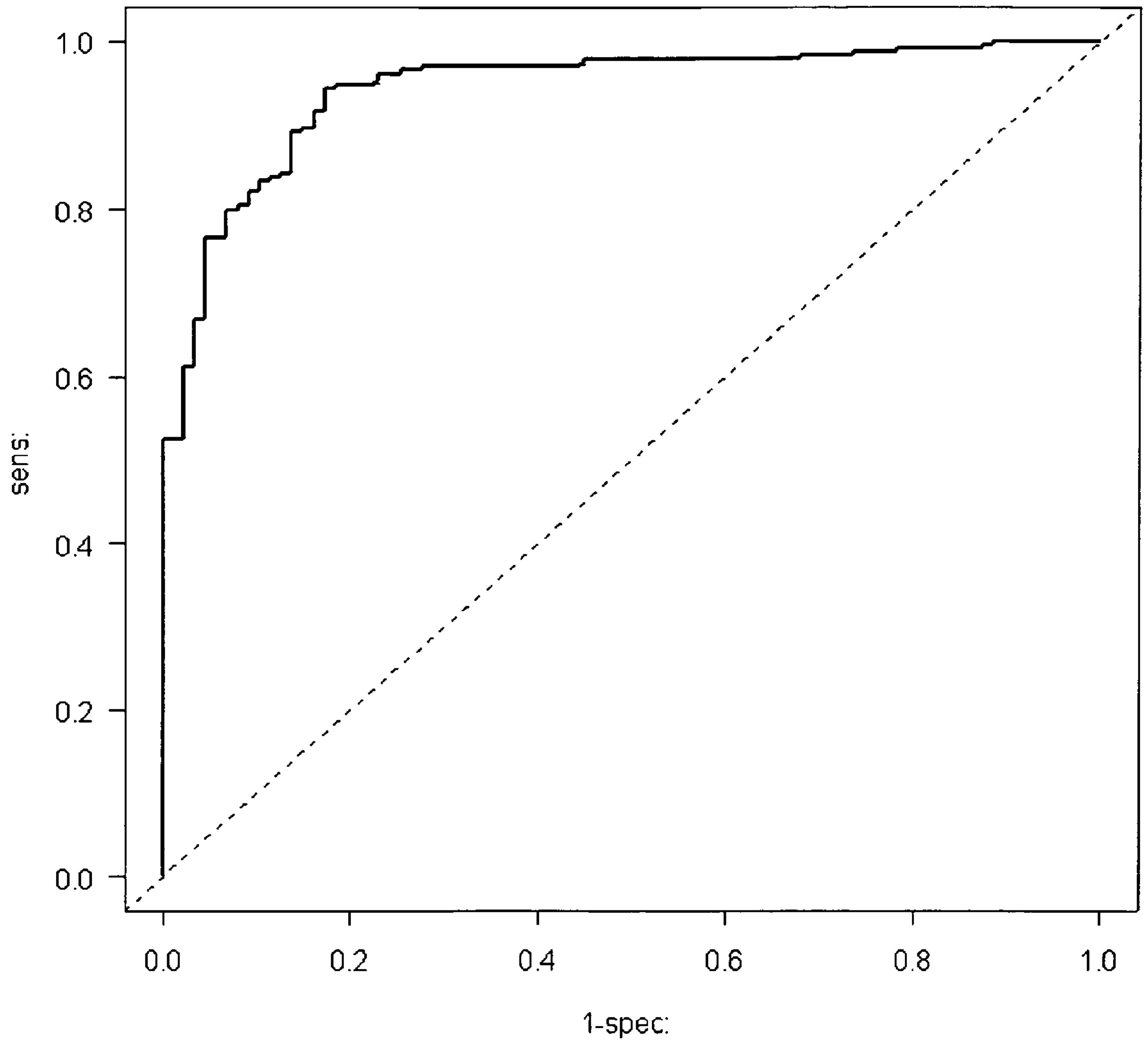

# Figure 6

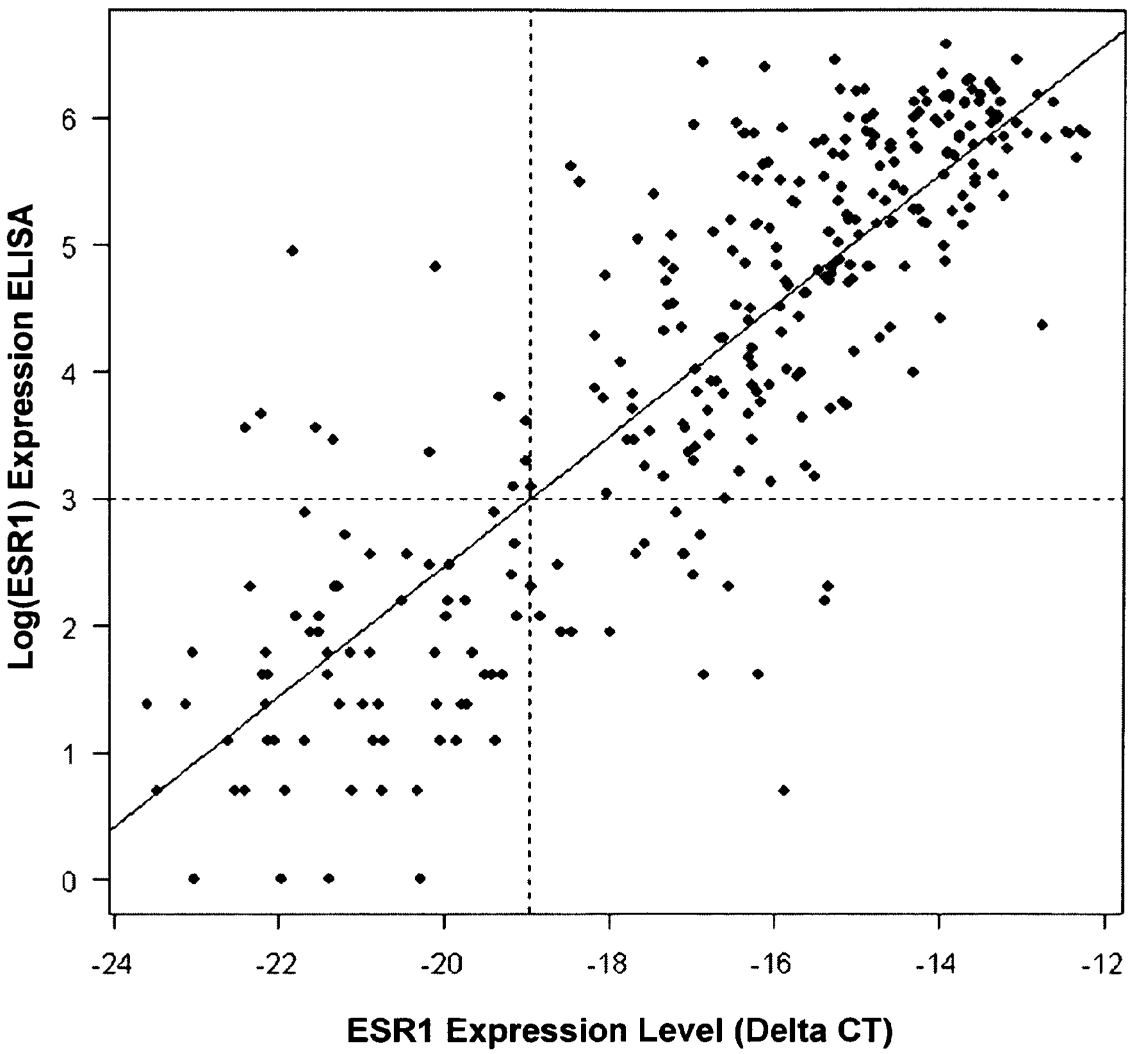
Log(ESR1) Expression ELISA
0
1
2
3
4
5
6
-24
-22
-20
-18
-16
-14
-12
ESR1 Expression Level (Delta CT)

Figure 7

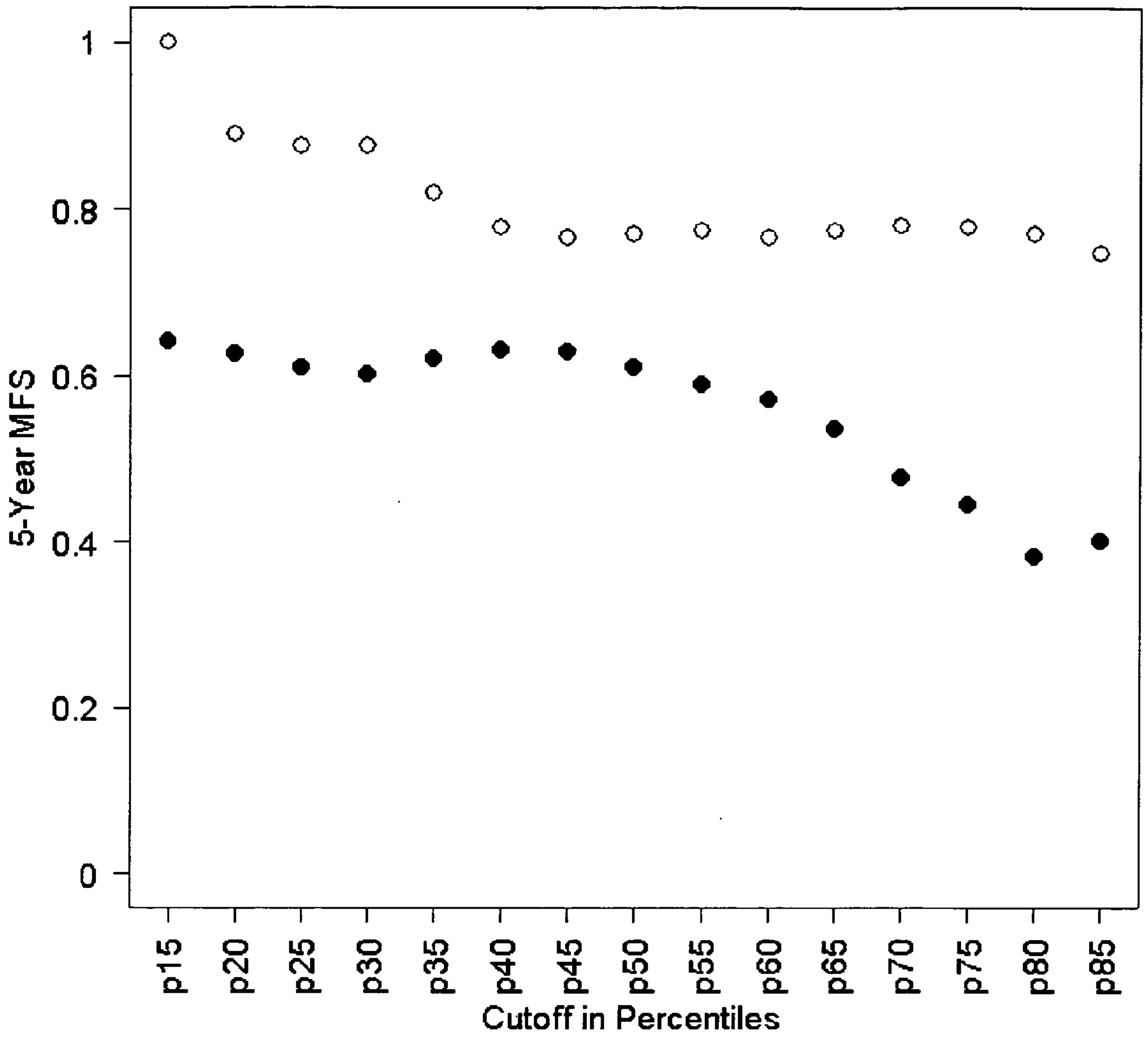
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 8

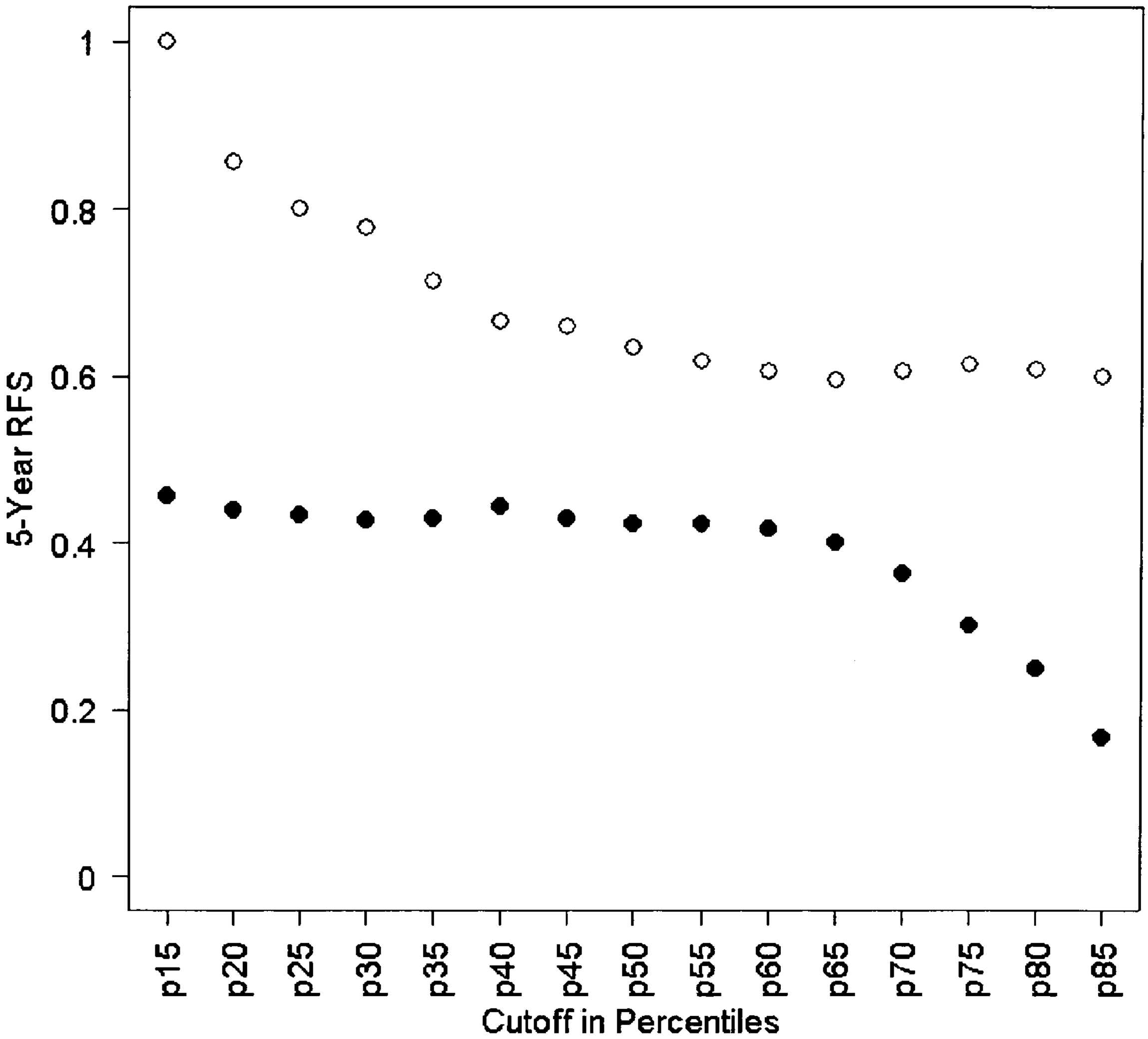
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 9

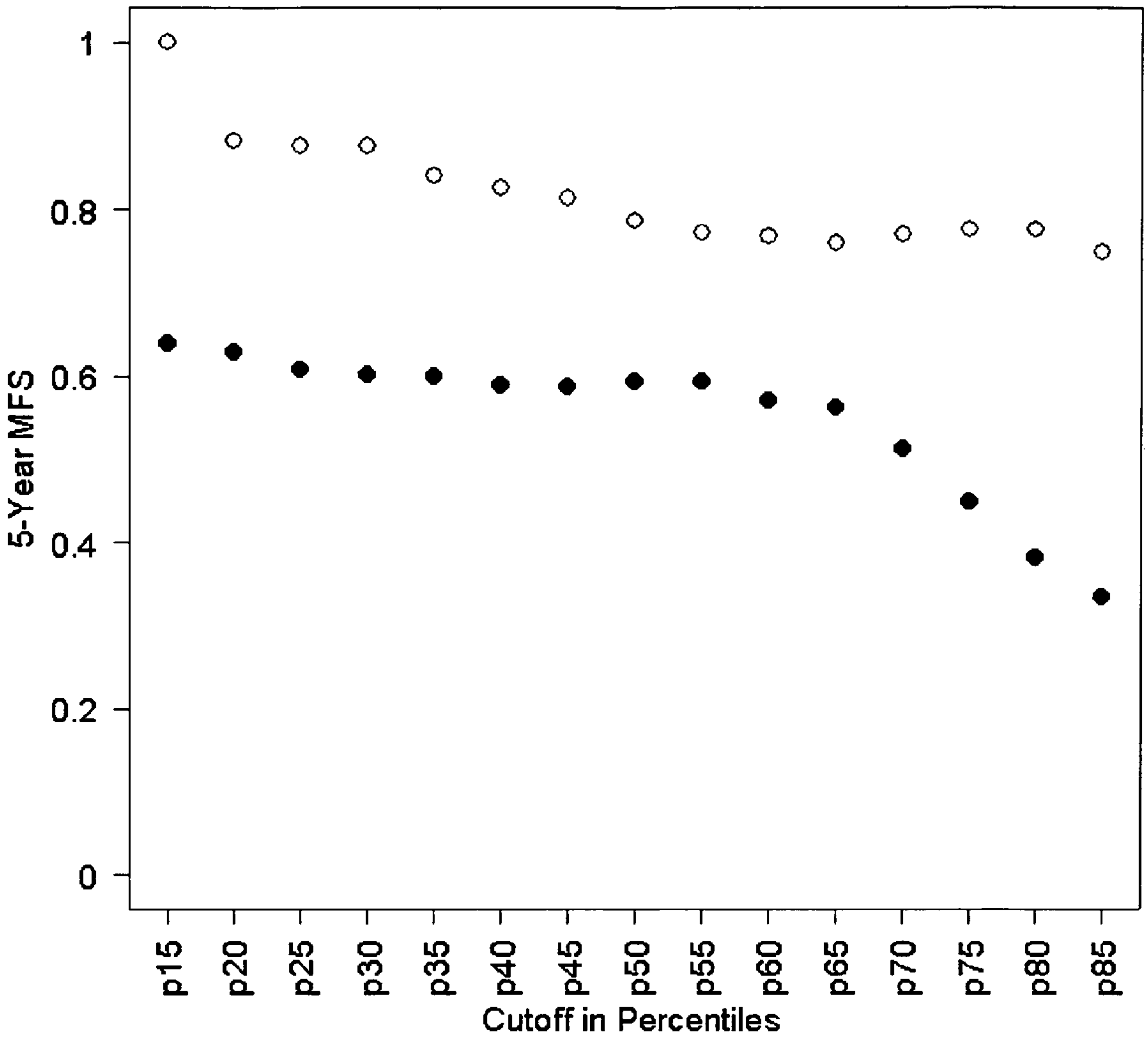
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 10

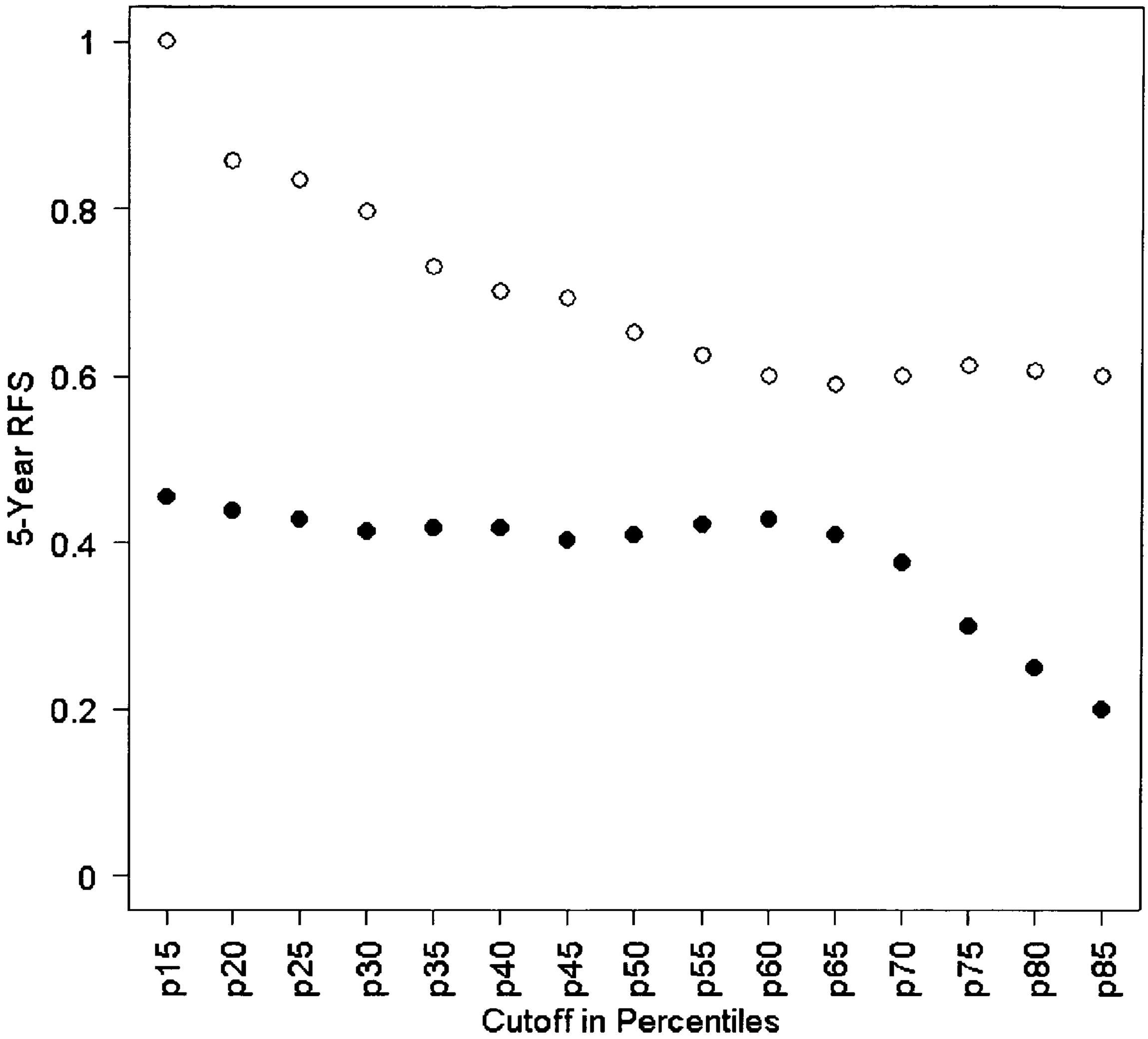
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 11

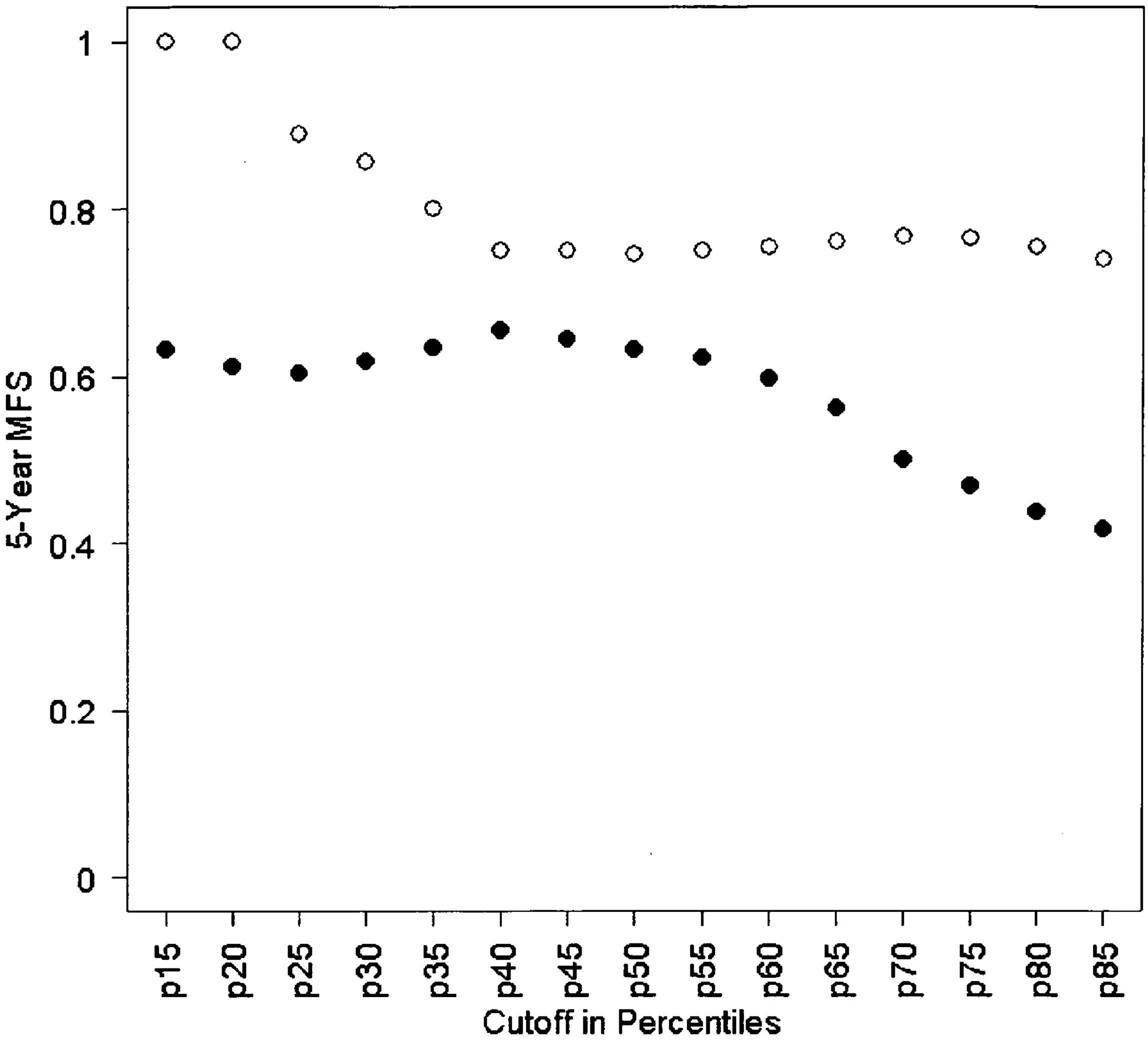
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 12

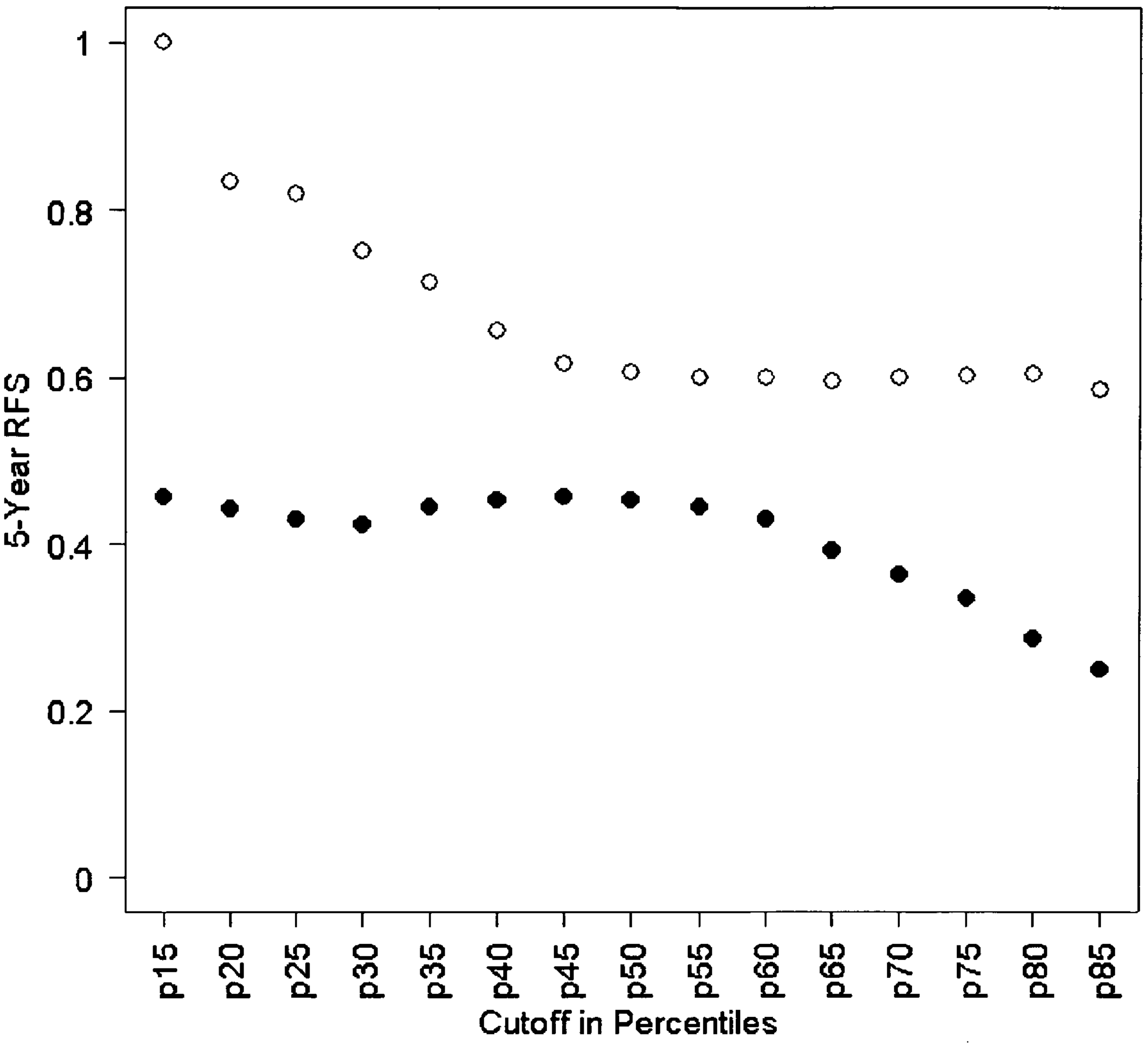
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 13

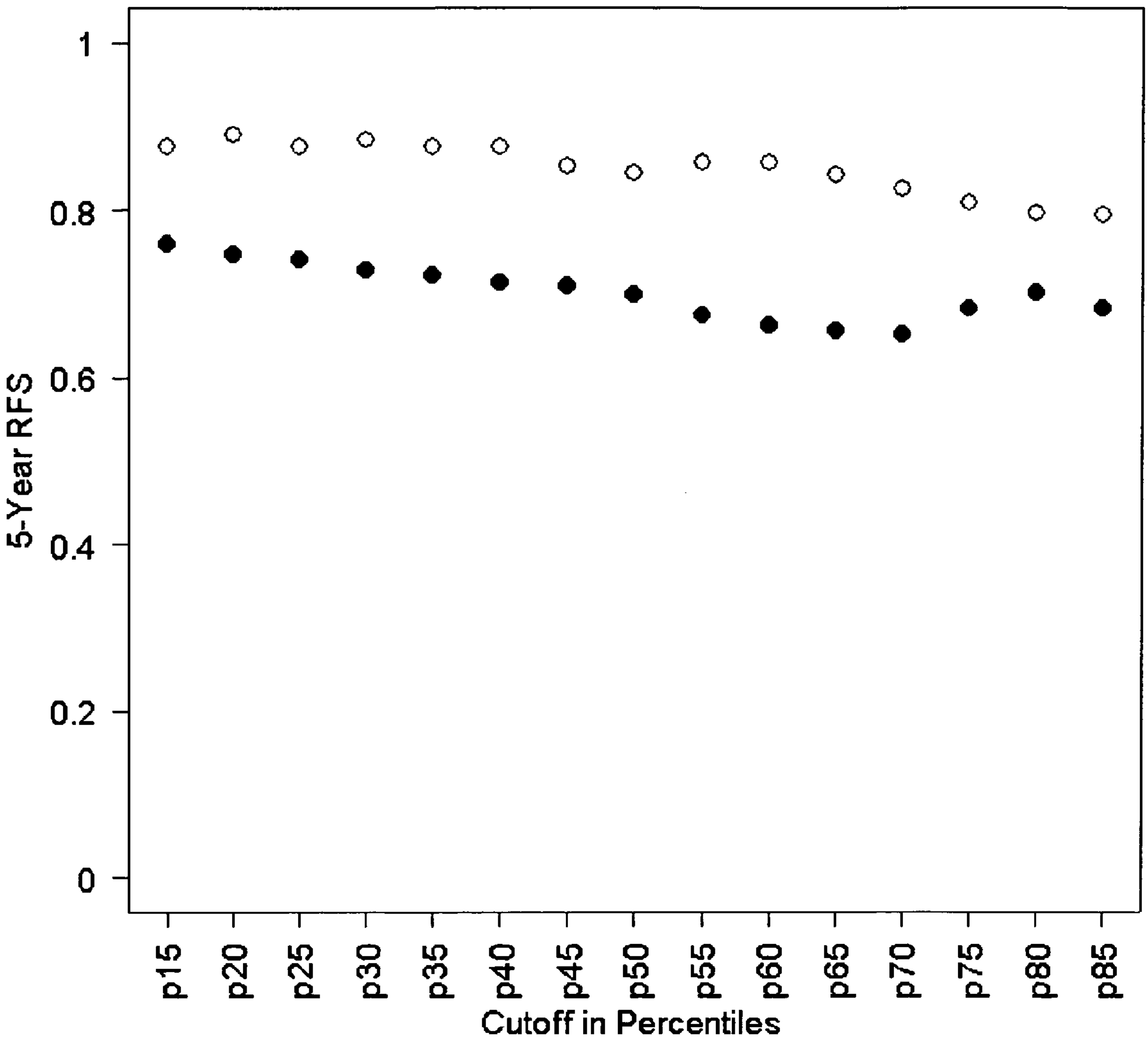
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 14

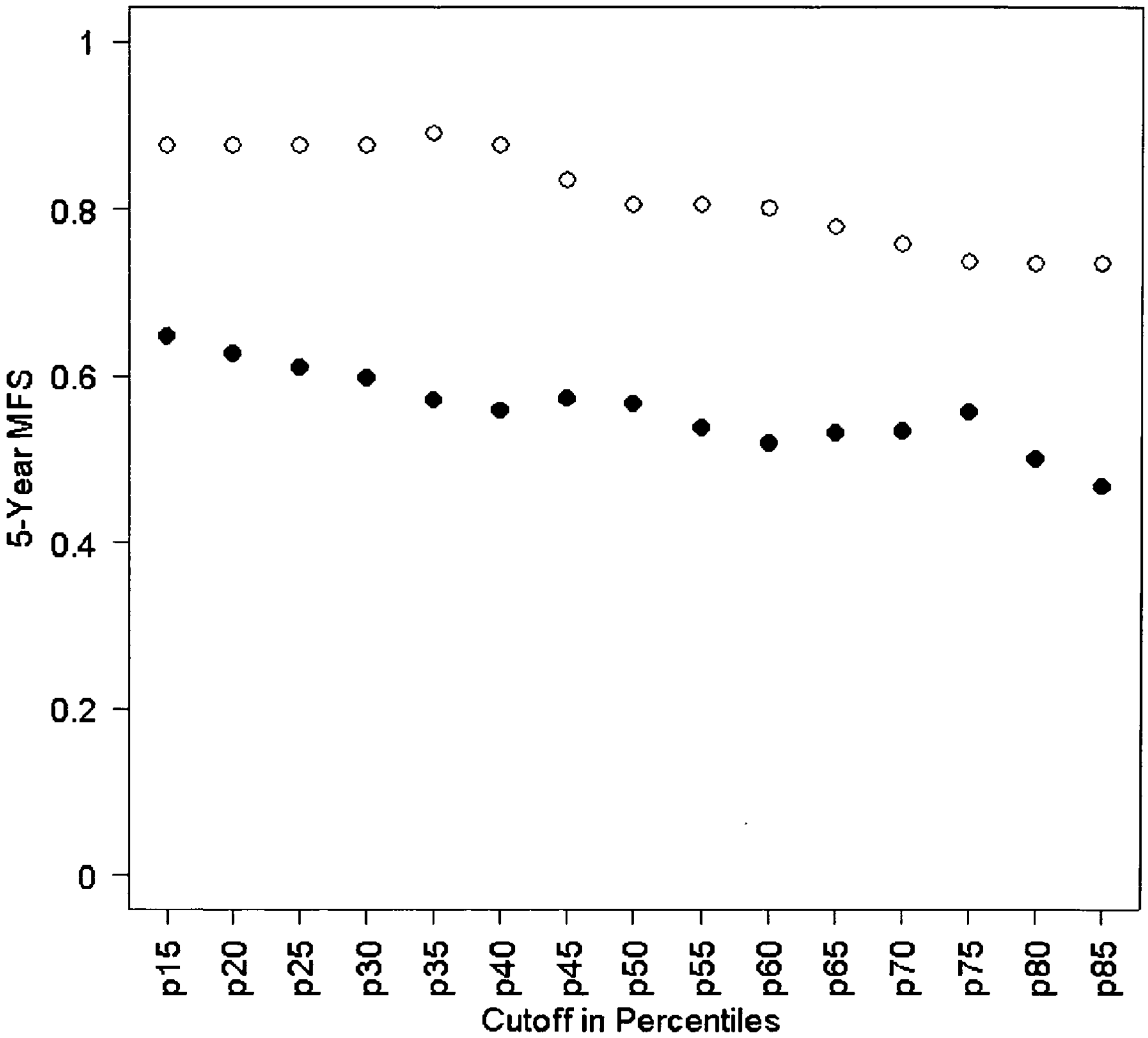
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 15

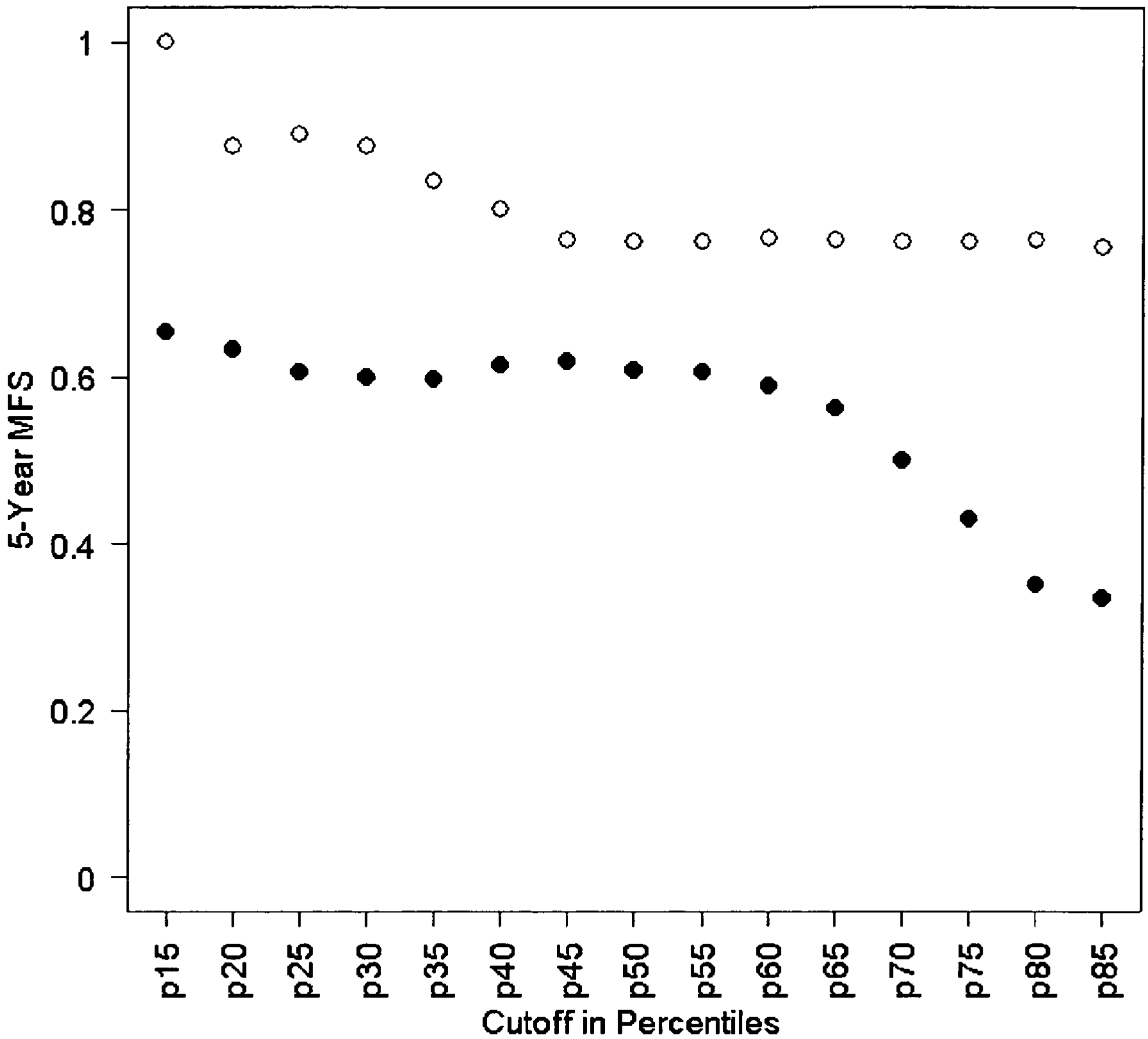
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 16

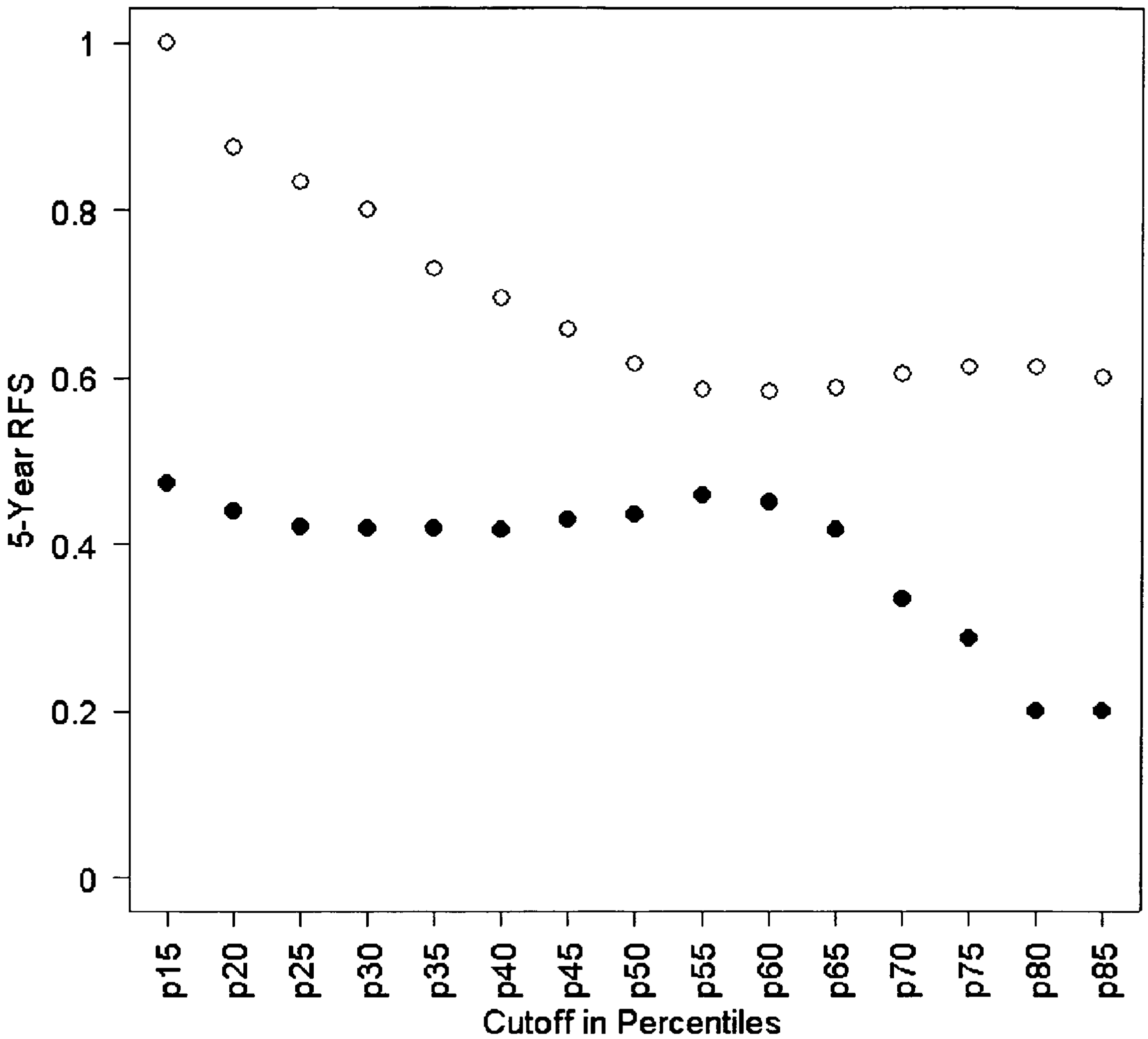
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 17

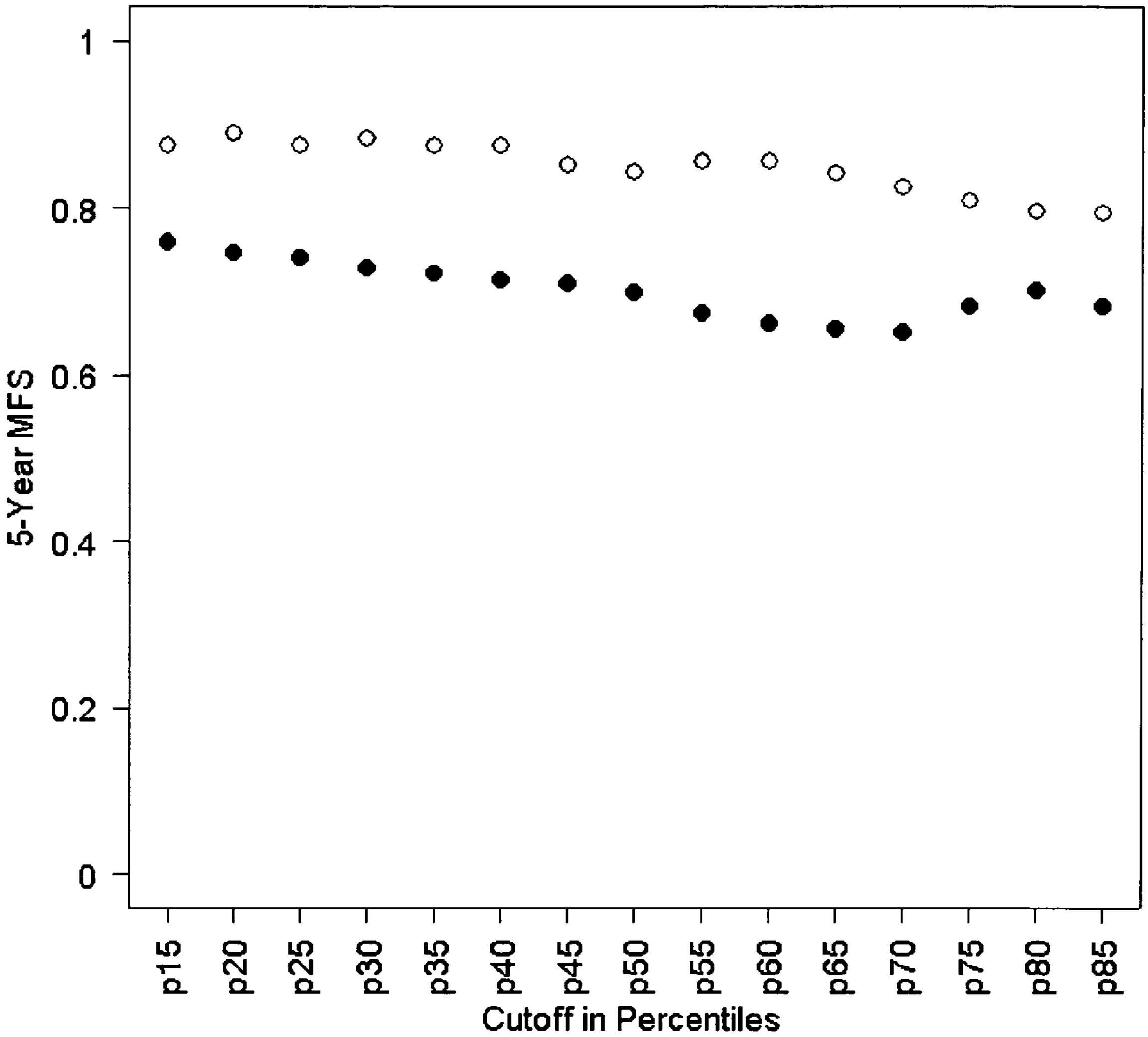
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 18

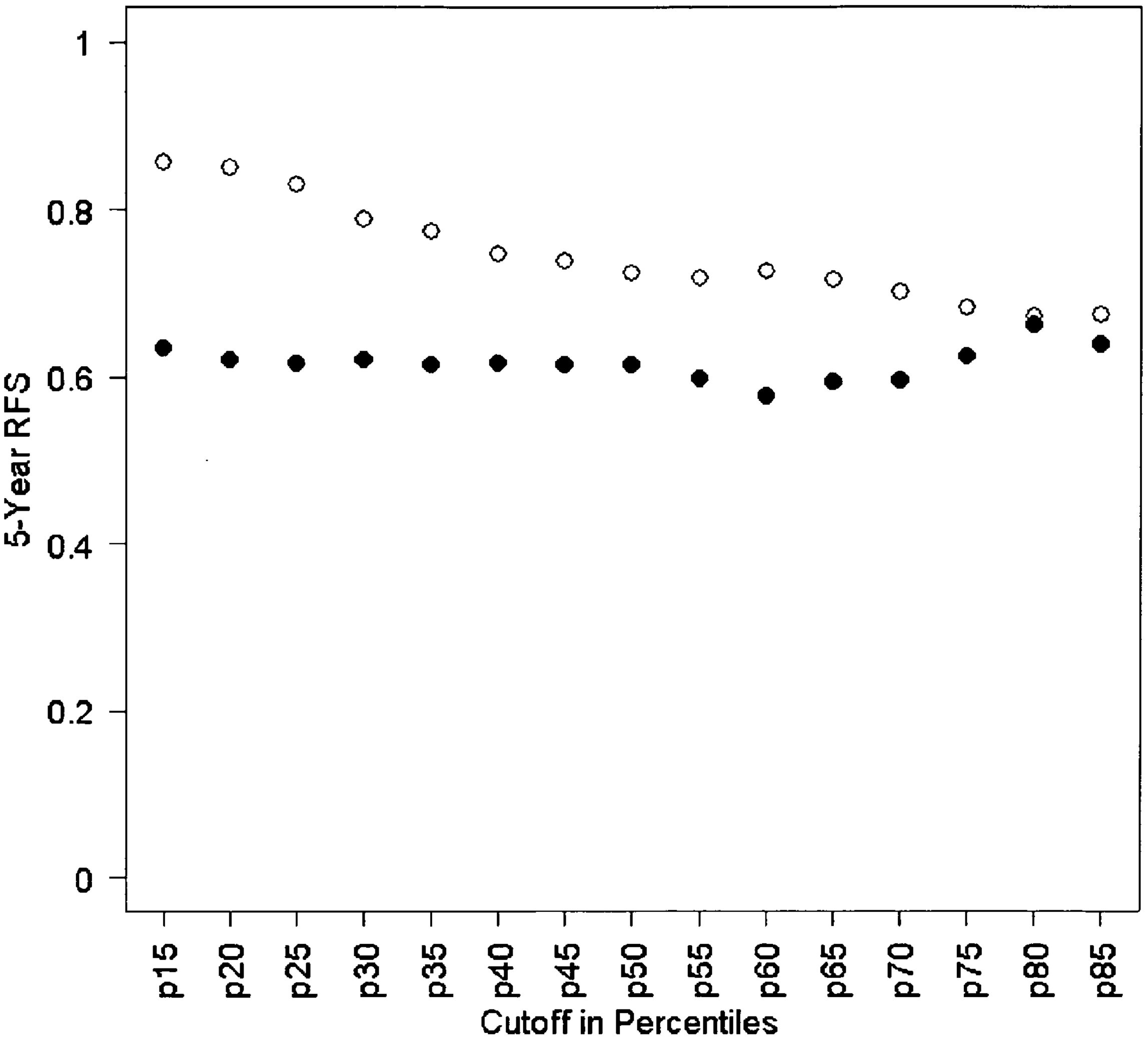
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 19

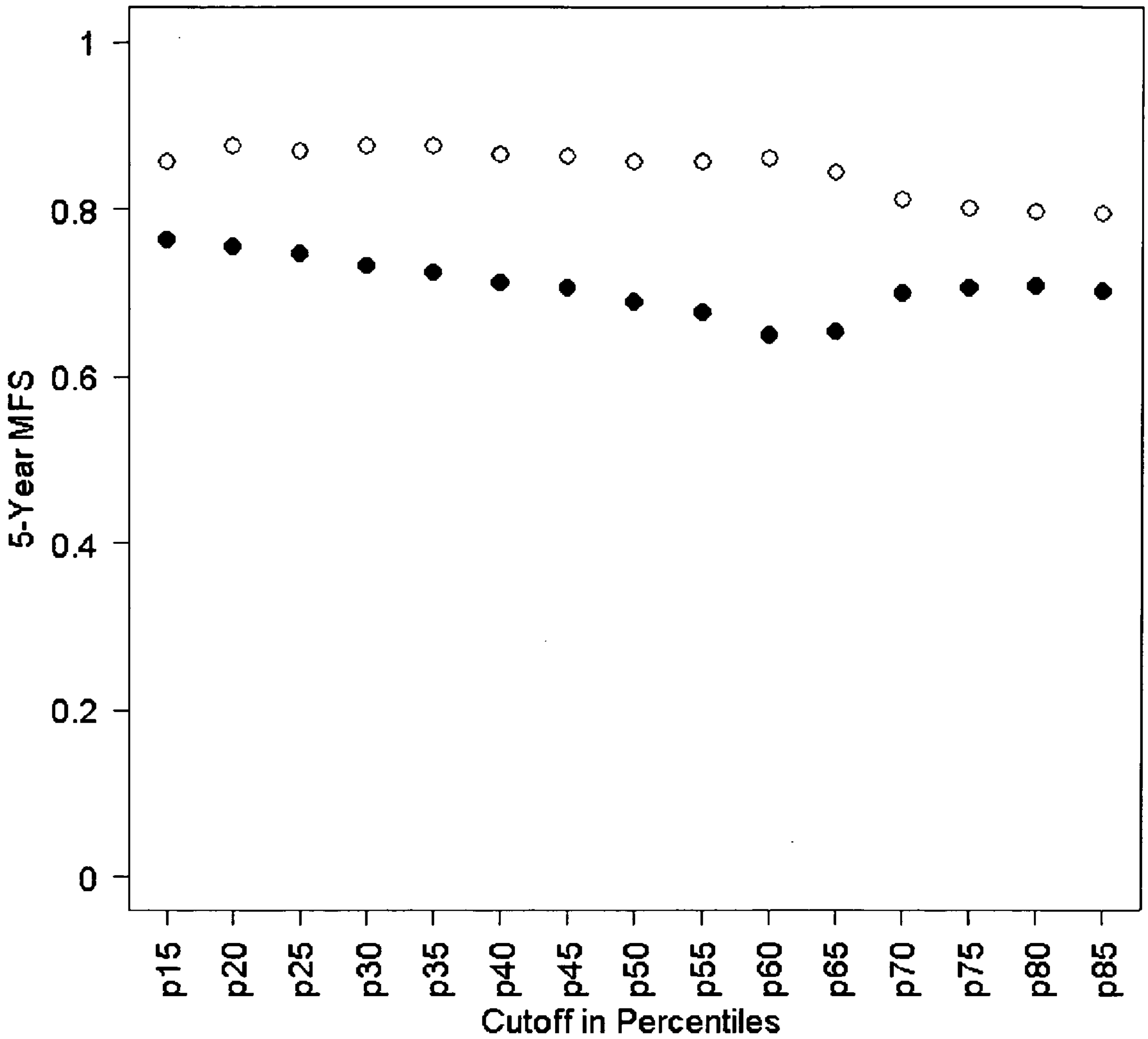
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 20

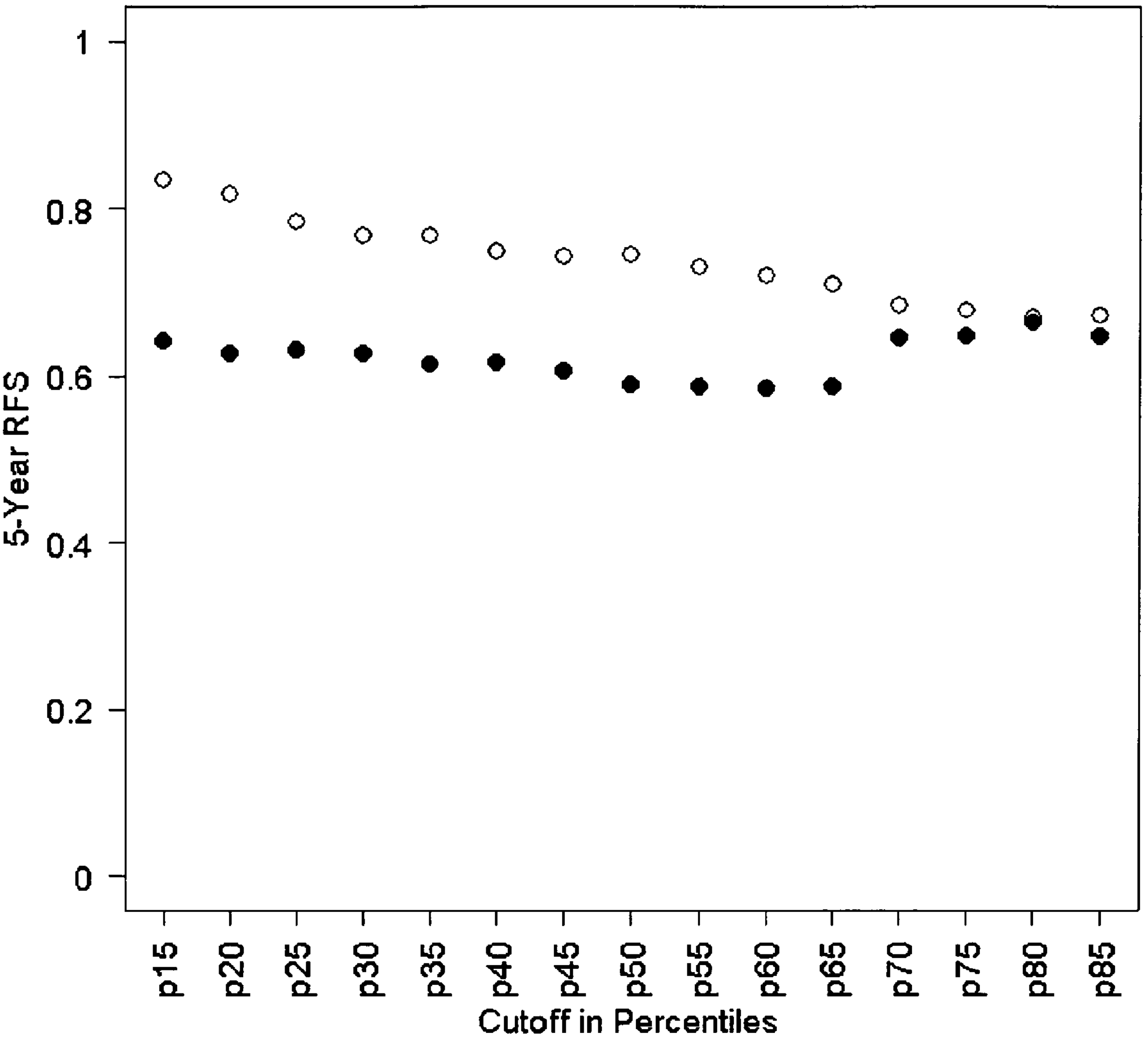
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 21

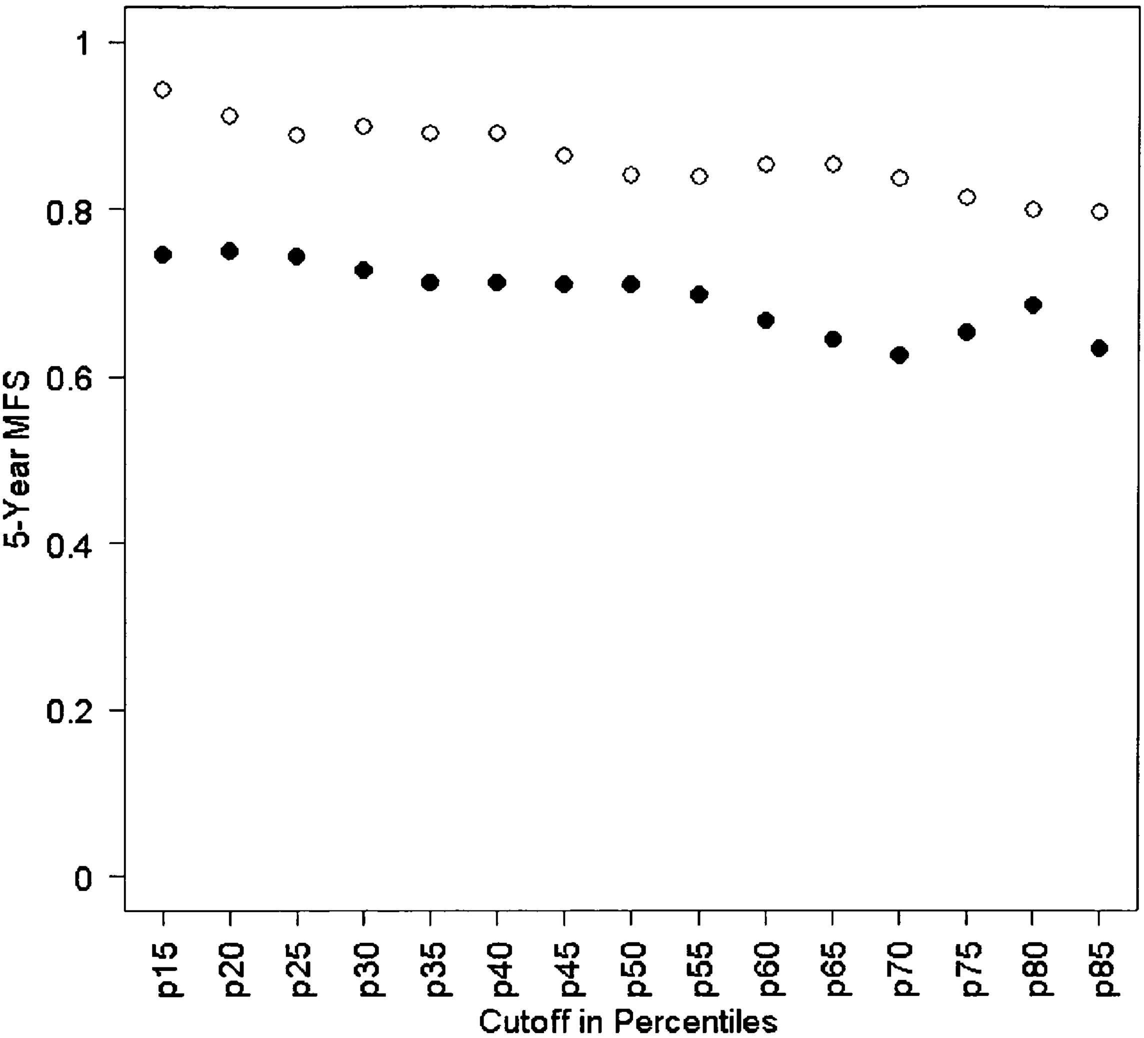
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 22

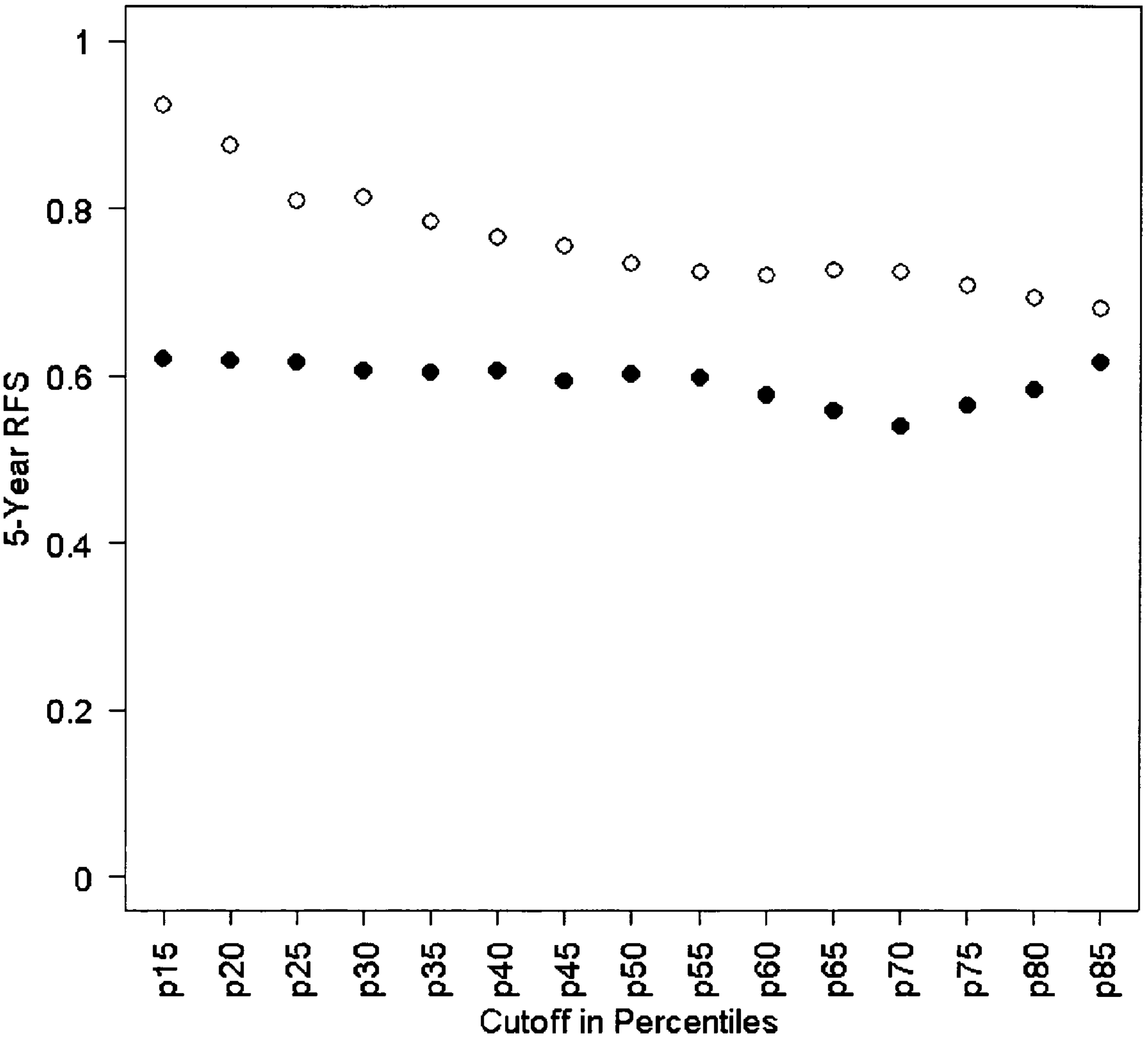
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 23

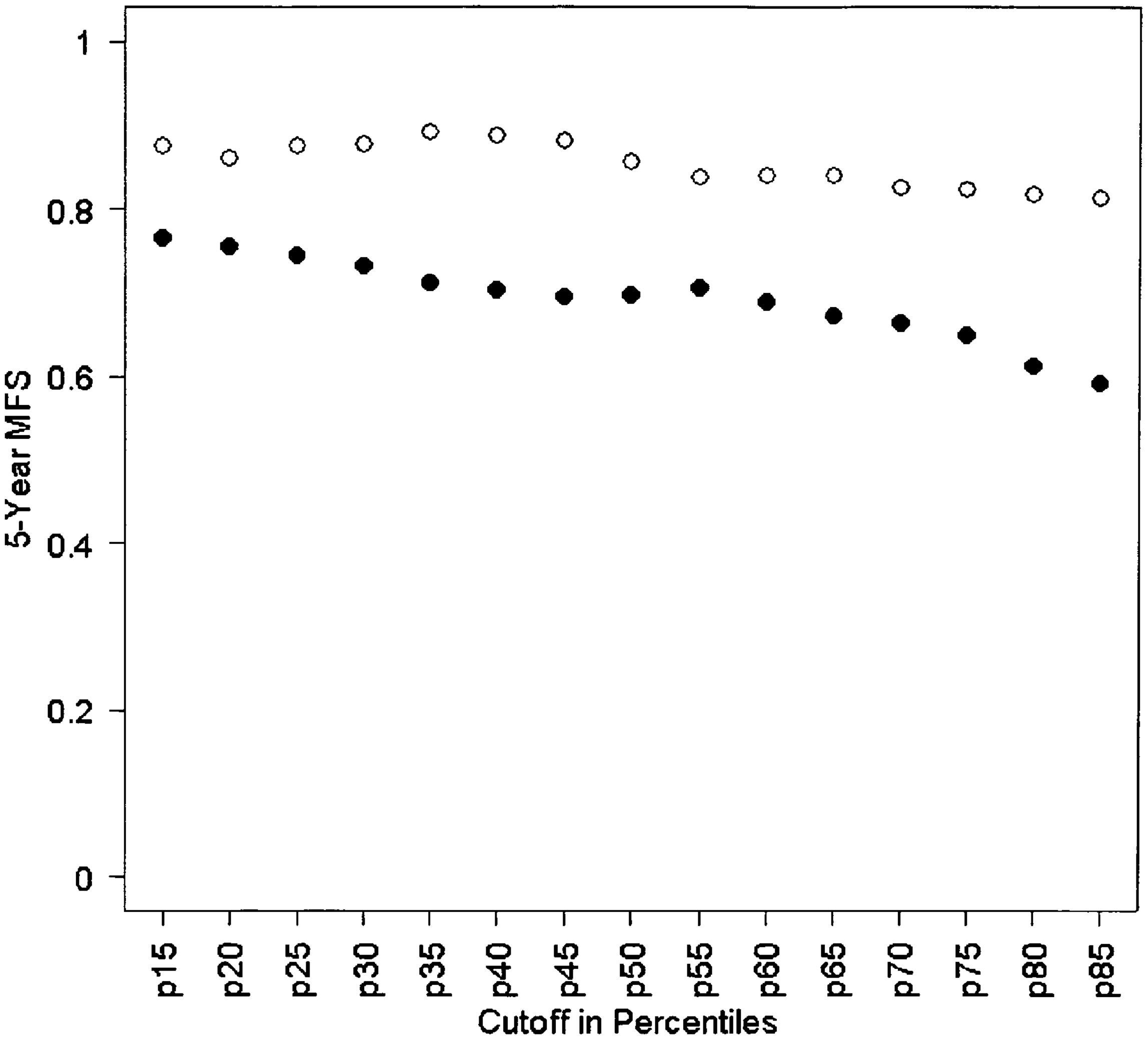
5-Year MFS
1
0.8
0.6
0.4
0.2
0
p15
p20
p25
p30
p35
p40
p45
p50
p55
p60
p65
p70
p75
p80
p85
Cutoff in Percentiles

Figure 24

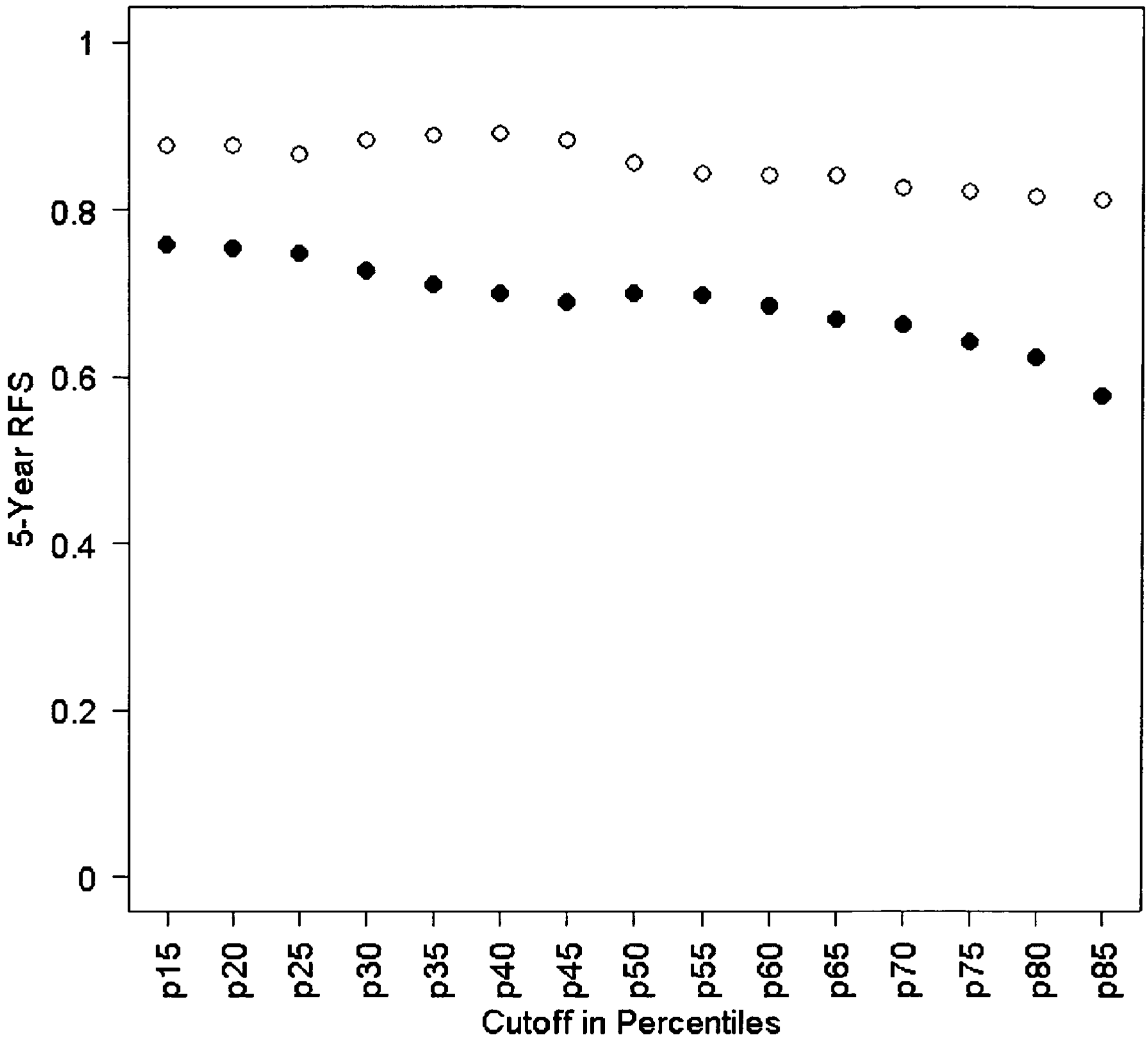
1
0.8
0.6
0.4
0.2
0
5-Year RFS
p15
p20
p25
p30
p35
p40
p45
p50
p55
p60
p65
p70
p75
p80
p85
Cutoff in Percentiles

Figure 25

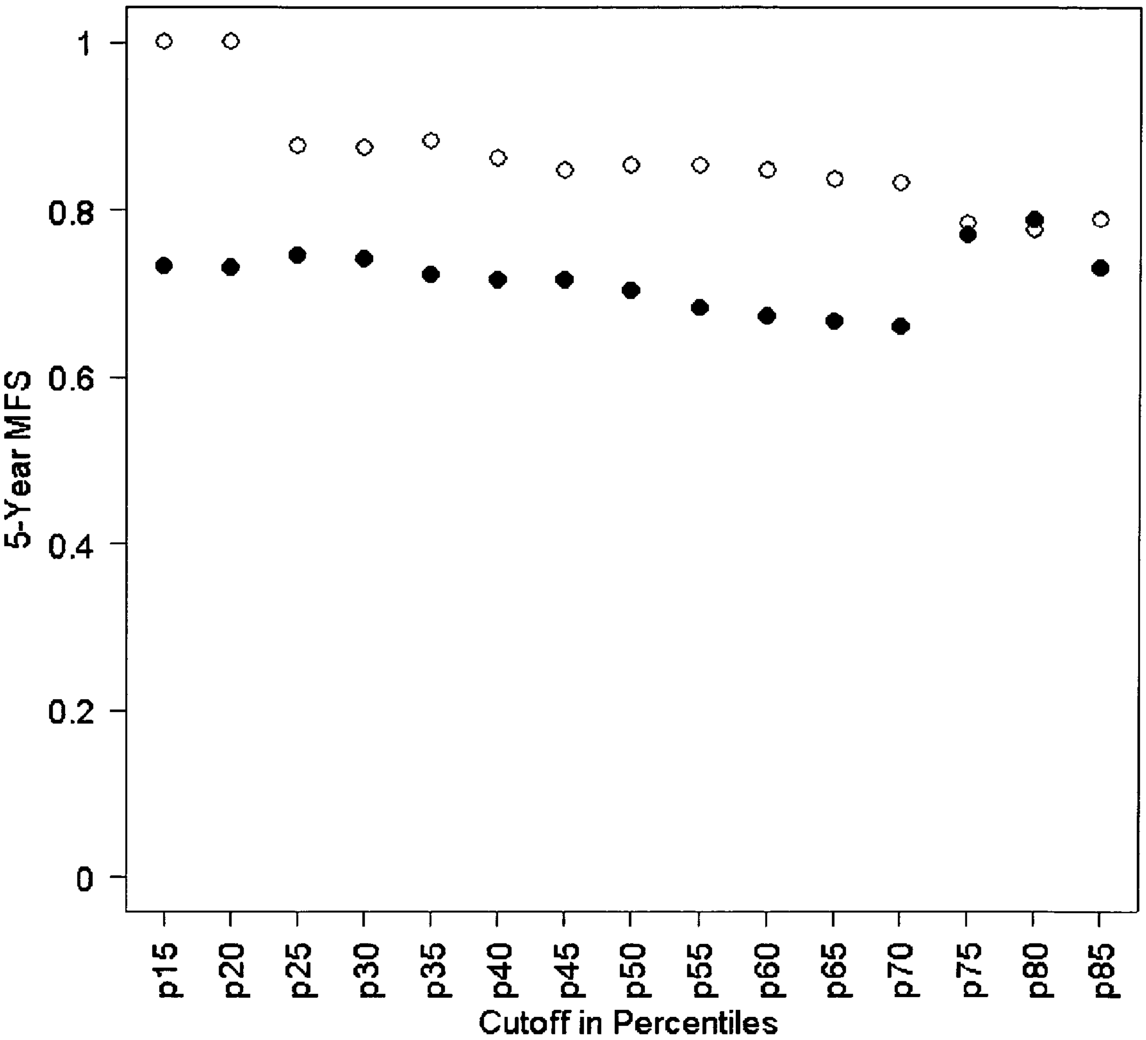
1
0.8
0.6
0.4
0.2
0
5-Year MFS
p15
p20
p25
p30
p35
p40
p45
p50
p55
p60
p65
p70
p75
p80
p85
Cutoff in Percentiles

Figure 26

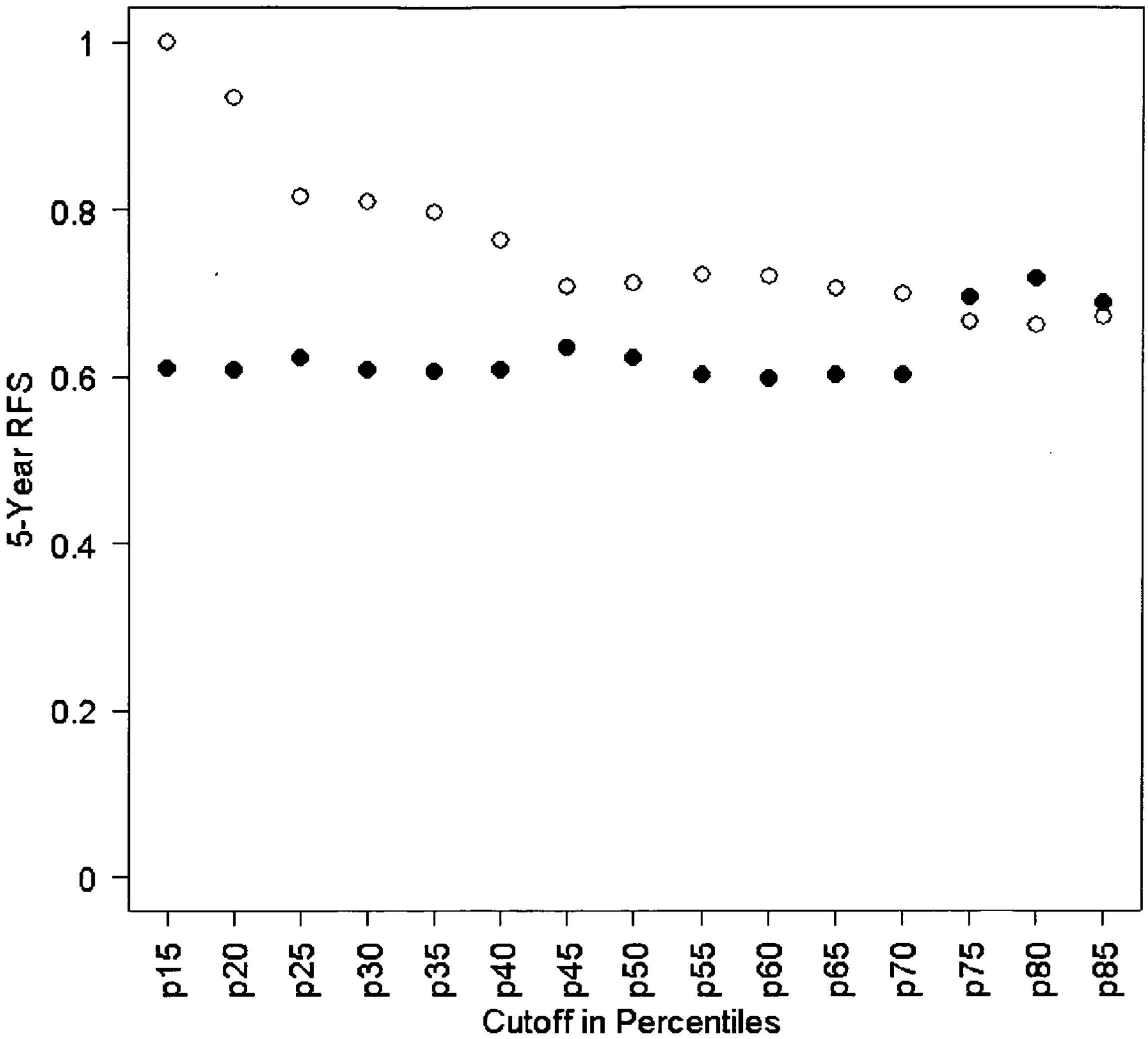
1
0.8
0.6
0.4
0.2
0
5-Year RFS
p15
p20
p25
p30
p35
p40
p45
p50
p55
p60
p65
p70
p75
p80
p85
Cutoff in Percentiles

European Patent Office

# PARTIAL EUROPEAN SEARCH REPORT

which under Rule 45 of the European Patent Convention shall be considered, for the purposes of subsequent proceedings, as the European search report

Application Number EP 05 02 1625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/005601 A (THE REGENTS OF THE UNIVERSITY OF MICHIGAN; CLARKE, MICHAEL, F; LIU, RU) 20 January 2005 (2005-01-20) | 1,4-18, 22-25,27 | C12Q1/68 |
| Y | * tables 4-7 * | 2,3 | |
| | * claims 1,3 * | | |
| | ----- | | |
| X | EP 1 561 821 A (EPIGENOMICS AG) 10 August 2005 (2005-08-10) | 26 | |
| Y | * claim 1 * | 2,3 | |
| | * tables 1,2 * | | |
| | * paragraphs [0030], [0051], [0085] * | | |
| | ----- | | |
| X | US 2004/191783 A1 (LECLERCQ GUY ET AL) 30 September 2004 (2004-09-30) | 26 | |
| | * table II * | | |
| | ----- | | |
| | -/-- | | |

TECHNICAL FIELDS SEARCHED (IPC)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Munich | 10 February 2006 | Helliot, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 05 02 1625

| Category | Citation of document with indication, where appropriate, of relevant passages | Relevant to claim | CLASSIFICATION OF THE APPLICATION (IPC) |
|---|---|---|---|
| A | CICENAS J. ET AL.: "INCREASED LEVEL OF PHOSPHORYLATED AKT MEASURED BY CHEMILUMINESCENCE-LINKED IMMUNOSORBENT ASSAY IS A PREDICTOR OF POOR PROGNOSIS IN PRIMARY BREAST CANCER OVEREXPRESSING ERBB-2." BREAST CANCER RESEARCH, vol. 7, no. 4, 24 March 2005 (2005-03-24), pages R394-R401, XP002366710 Retrieved from the Internet: URL:http://breast-cancer-research.com/content/7/4/R394> [retrieved on 2006-02-07] * the whole document * ----- | 1-27 | |
| | | | TECHNICAL FIELDS SEARCHED (IPC) |

2

EPO FORM 1503 03.82 (P04C10)

European Patent Office

# INCOMPLETE SEARCH SHEET C

Application Number
EP 05 02 1625

Claim(s) searched incompletely:
1

Reason for the limitation of the search:

The present claim 1 relates to a method used for assessing the probability of survival of a cancer patient characterized in that in erb-B2 positive patients, at least one of a first set of cancer related genes is analysed and in erb-B2-negative patients, at least one of a second set of cancer related genes is analysed.
However, in the absence of any indication as to the technical feature relating to the first and second set of cancer related genes, a lack of clarity within the meaning of Art. 84 EPC arise to such an extent that the skilled person cannot understand the actual scope of said claim. Consequently, the search has been carried out for those parts of the claim 1 which refer to the first set of cancer related genes as disclosed in claim 4, namely a first set of cancer related genes consisting of PLAU, PLAUR, MMP11, MMP1, MMP3, TIMP3, ERBB3, ECGF1, SERPINE1 and MMP2, and to the second set of cancer related genes as disclosed in claim 5, namely a second set of cancer related genes consisting of BIRC5, E2F1, TOP2A, TK1, TYMS, VEGF, SRD5A1, CCND1, CTSD, BAX, CTSB, CTNNB1, ADM, MMP9, STK11 and TIMP4.

However, even restricted as such, the claim contains so many options of gene combination that a lack of clarity still arises within the meaning of Article 84 EPC to such an extent as to render a meaningful search of the claim impossible. Consequently, the search has been carried out for those parts of the claim 1 which refer to the genes PLAU, PLAUR or MMP11, individually, or to the combinations of (PLAU, PLAUR and MMP11), or (PLAUR, MMP11 and MMP1), or (PLAU, PLAUR, MMP11 and MMP1) or (PLAU, PLAUR, MMP11, MMP1, MMP3 and SERPINE1), as disclosed in Tab. 7 of the present application and to the genes E2F1, BIRC5 or TOP2A, individually, or to the combinations of (E2F1, BIRC5 and TOP2A), or (E2F1, BIRC5 and TK1), or (E2F1, BIRC5 and TYMS), or (E2F1, BIRC5, TOP2A and TK1) or (E2F1, BIRC5, TOP2A, TK1, CTSB and CTSD), as disclosed in Tab. 8 of the present application.

## ANNEX TO THE EUROPEAN SEARCH REPORT ON EUROPEAN PATENT APPLICATION NO. EP 05 02 1625

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2006

| Patent document cited in search report | | Publication date | Patent family member(s) | | Publication date |
|---|---|---|---|---|---|
| WO 2005005601 | A | 20-01-2005 | US | 2006019256 A1 | 26-01-2006 |
| EP 1561821 | A | 10-08-2005 | NONE | | |
| US 2004191783 | A1 | 30-09-2004 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **COX DR.** *Regression Models and Lifetables J.R. Soc.,* 1972, vol. 34, 187-220
- **DUFFY MJ.** Predictive markers in breast and other cancers: a review. *Clin Chem,* 2005, vol. 51 (3), 494-503
- **ZWEIG M et al.** Receiver operating characteristic (ROC) plots: A fundamental evaluation tool in clinical medicine. *Clin Chem.,* 03 April 1993, vol. 9 (4), 561-77
- **M TABLEMAN et al.** Survival Analysis Using S. Chapman & Hall/CRC, 2004

SEQUENCE LISTING

<110> OncoScore AG

<120> Method for the Prognosis of Cancer Patients

<130> TM - P35016EP00

<160> 208

<170> PatentIn version 3.3

<210> 1
<211> 1449
<212> DNA
<213> homo sapiens

<400> 1
```
ctggatagaa cagctcaagc cttgccactt cgggcttctc actgcagctg ggcttggact      60

tcggagtttt gccattgcca gtgggacgtc tgagactttc tccttcaagt acttggcaga     120

tcactctctt agcagggtct gcgcttcgca gccgggatga agctggtttc cgtcgccctg     180

atgtacctgg gttcgctcgc cttcctaggc gctgacaccg ctcggttgga tgtcgcgtcg     240

gagtttcgaa agaagtggaa taagtgggct ctgagtcgtg ggaagaggga actgcggatg     300

tccagcagct accccaccgg gctcgctgac gtgaaggccg ggcctgccca gacccttatt     360

cggccccagg acatgaaggg tgcctctcga agccccgaag acagcagtcc ggatgccgcc     420

cgcatccgag tcaagcgcta ccgccagagc atgaacaact tccagggcct ccggagcttt     480

ggctgccgct tcgggacgtg cacggtgcag aagctggcac accagatcta ccagttcaca     540

gataaggaca aggacaacgt cgcccccagg agcaagatca gcccccaggg ctacggccgc     600

cggcgccggc gctccctgcc cgaggccggc ccgggtcgga ctctggtgtc ttctaagcca     660

caagcacacg gggctccagc ccccccgagt ggaagtgctc cccactttct ttaggattta     720

ggcgcccatg gtacaaggaa tagtcgcgca agcatcccgc tggtgcctcc cgggacgaag     780

gacttcccga gcggtgtggg gaccgggctc tgacagccct gcggagaccc tgagtccggg     840

aggcaccgtc cggcggcgag ctctggcttt gcaagggccc ctccttctgg gggcttcgct     900

tccttagcct tgctcaggtg caagtgcccc agggggcggg gtgcagaaga atccgagtgt     960

ttgccaggct taaggagagg agaaactgag aaatgaatgc tgagaccccc ggagcagggg    1020

tctgagccac agccgtgctc gcccacaaac tgatttctca cggcgtgtca ccccaccagg    1080

gcgcaagcct cactattact tgaactttcc aaaacctaaa gaggaaaagt gcaatgcgtg    1140

ttgtacatac agaggtaact atcaatattt aagtttgttg ctgtcaagat tttttttgta    1200

acttcaaata tagagatatt tttgtacgtt atatattgta ttaagggcat tttaaaagca    1260

attatattgt cctcccctat tttaagacgt gaatgtctca gcgaggtgta aagttgttcg    1320

ccgcgtggaa tgtgagtgtg tttgtgtgca tgaaagagaa agactgatta cctcctgtgt    1380
```

ggaagaagga aacaccgagt ctctgtataa tctatttaca taaaatgggt gatatgcgaa 1440

cagcaaacc 1449

<210> 2
<211> 2282
<212> DNA
<213> homo sapiens

<400> 2
ctggatagaa cagctcaagc cttgccactt cgggcttctc actgcagctg ggcttggact 60

tcggagtttt gccattgcca gtgggacgtc tgagactttc tccttcaagt acttggcaga 120

tcactctctt agcaggtagg tgccgcagac cctgcgggtt aagaggtggg gtgggggca 180

gtgcttgcca aggccctaaa ctgggagcgc tgggtgaggg gaacaaccca ctttggaggg 240

ttctctgaga gatagataca ccccatatcc tgggcccagc tcgtgcacac agctggaggt 300

ccagagaccc agtcccctct gctccgtcag ccaagttcca agaagttgag cagagaccct 360

ctgggagcct ggcggggtgc agcggcctcc cctgcggggc ctgtcacccg gccggcgcgt 420

gcaaacgcct ctggcgcctc tctgcgcgga gggagataag cgtctgagcc agggaaagcg 480

cgggctaaac ccgcctcgcc ggggcccctg cccgccctcc gtgccccgcc cgggcggtgc 540

agctggcccg ggtgctcacg ctcgactctc tttcttcttt tccagggtct gcgcttcgca 600

gccgggatga agctggtttc cgtcgccctg atgtacctgg gttcgctcgc cttcctaggc 660

gctgacaccg ctcggttgga tgtcgcgtcg gagtttcgaa agaagtgagt ccgggcagcg 720

ccttcccct tgctggtacc tggcaggcaa ggggaactga ccgttggtcc cgaaggtcta 780

gaagtgaatg ggagcaggga caggcctggg cgtcacctga acgcacgcga atcgggtctg 840

cttgtgtttt ccaggtggaa taagtgggct ctgagtcgtg ggaagaggga actgcggatg 900

tccagcagct accccaccgg gctcgctgac gtgaaggccg ggcctgccca gacccttatt 960

cggccccagg acatgaaggg tgcctctcga agccccgaag acaggtaact acgccctgtg 1020

ctgtccaggg acgggaggga aggaaggtgt gcgggaggag ttctctgtct ccactcccct 1080

ggcccggggg atcgtcgggg ctggaccgca gctcagatgg cgcgagcagt ttccagctcc 1140

ctctggctct agaatggctc ccgttcccgg tgttggggcc aaagctctgc ttgatggggt 1200

ctcaagttgc ctttcttccc cctcccccg cccgcagcag tccggatgcc gcccgcatcc 1260

gagtcaagcg ctaccgccag agcatgaaca acttccaggg cctccggagc tttggctgcc 1320

gcttcgggac gtgcacggtg cagaagctgg cacaccagat ctaccagttc acagataagg 1380

acaaggacaa cgtcgccccc aggagcaaga tcagccccca gggctacggc cgccggcgcc 1440

ggcgctccct gcccgaggcc ggcccgggtc ggactctggt gtcttctaag ccacaagcac 1500

acggggctcc agcccccccg agtggaagtg ctccccactt tctttaggat ttaggcgccc 1560

atggtacaag gaatagtcgc gcaagcatcc cgctggtgcc tcccgggacg aaggacttcc 1620

cgagcggtgt ggggaccggg ctctgacagc cctgcggaga ccctgagtcc gggaggcacc 1680

gtccggcggc gagctctggc tttgcaaggg cccctccttc tgggggcttc gcttccttag 1740

ccttgctcag gtgcaagtgc cccagggggc ggggtgcaga agaatccgag tgtttgccag 1800

gcttaaggag aggagaaact gagaaatgaa tgctgagacc cccggagcag ggtctgagc 1860

cacagccgtg ctcgcccaca aactgatttc tcacggcgtg tcaccccacc agggcgcaag 1920

cctcactatt acttgaactt tccaaaacct aaagaggaaa agtgcaatgc gtgttgtaca 1980

tacagaggta actatcaata tttaagtttg ttgctgtcaa gatttttttt gtaacttcaa 2040

atatagagat atttttgtac gttatatatt gtattaaggg cattttaaaa gcaattatat 2100

tgtcctcccc tattttaaga cgtgaatgtc tcagcgaggt gtaaagttgt tcgccgcgtg 2160

gaatgtgagt gtgtttgtgt gcatgaaaga gaaagactga ttacctcctg tgtggaagaa 2220

ggaaacaccg agtctctgta taatctattt acataaaatg ggtgatatgc gaacagcaaa 2280

cc 2282

<210> 3
<211> 19
<212> DNA
<213> Artificial

<220>
<223> Synthesis

<220>
<221> misc_signal
<222> (1)..(19)

<400> 3
ccgtcgccct gatgtacct 19

<210> 4
<211> 24
<212> DNA
<213> Artificial

<220>
<223> Synthesis

<400> 4
cccacttatt ccacttcttt cgaa 24

<210> 5
<211> 888
<212> DNA
<213> homo sapiens

<400> 5

tcacgtgacc cgggcgcgct gcggccgccc gcgcggaccc ggcgagaggc ggcggcggga 60

gcggcggtga tggacgggtc cggggagcag cccagaggcg gggggcccac cagctctgag 120

cagatcatga agacaggggc ccttttgctt cagggtttca tccaggatcg agcagggcga 180

atgggggggg aggcacccga gctggccctg gacccggtgc ctcaggatgc gtccaccaag 240

aagctgagcg agtgtctcaa gcgcatcggg gacgaactgg acagtaacat ggagctgcag 300

aggatgattg ccgccgtgga cacagactcc ccccgagagg tctttttccg agtggcagct 360

gacatgtttt ctgacggcaa cttcaactgg ggccgggttg tcgccctttt ctactttgcc 420

agcaaactgg tgctcaaggc cctgtgcacc aaggtgccgg aactgatcag aaccatcatg 480

ggctggacat tggacttcct ccgggagcgg ctgttgggct ggatccaaga ccagggtggt 540

tgggacggcc tcctctccta ctttgggacg cccacgtggc agaccgtgac catctttgtg 600

gcgggagtgc tcaccgcctc actcaccatc tggaagaaga tgggctgagg cccccagctg 660

ccttggactg tgtttttcct ccataaatta tggcattttt ctgggagggg tggggattgg 720

gggacatggg catttttctt acttttgtaa ttattggggg gtgtggggaa gagtggtctt 780

gagggggtaa taaacctcct tcgggacaca aaaaaaaaaa aaaaaaaaaa aaaaaaaaaa 840

aaaaaaaaaa aaaaaaaaaa aaaaaaaaaa aaaaaaaaaa aaaaaaaa 888

<210> 6
<211> 6939
<212> DNA
<213> homo sapiens

<400> 6
tcacgtgacc cgggcgcgct gcggccgccc gcgcggaccc ggcgagaggc ggcggcggga 60

gcggcggtga tggacgggtc cggggagcag cccagaggcg ggggtgaggc gggaggcaga 120

cgggcgggag gagggcgagc cccctcgccg gcccgtccgg gatccttcct accggcctgg 180

ggctgtgcga tctccaagca ctgaggggca gaaactcccg gatcgggcgc tgccagcctc 240

cagtcccctc cgtcctcgga ggttcctggc tctctgatcc ccgtgtcccg atccctgcct 300

ctctggcgct ctcggaccct cgagaaccag gggatctcgg aagccaagcc cccgggcagg 360

cccgggcttg tcgcccgcac cacttcctgc ctctggcact ggtgggaggg gcgggtctcg 420

cctctgcccc ctcaggccag gggtctggat gcatatagcg ttcccctagc ctctttcccc 480

ggggagaatg taggatacag gcccagcctc ctggcctttc tccatcaggg actcagttgt 540

ctgggccccc ccgtcacttt atctgctagg gtcccagaag tccagggtcc ccagctctgt 600

cctccctcag ggccgtgag tctccacagt ctcctgatcc cctagaaccc aagagtccag 660

gtacctcttc ccttcctttc tcctctaggg cccaccagct ctgagcagat catgaagaca 720

ggggcccttt tgcttcaggg gtgagtttga ggtctgatta ttgtggcaca gatttgagga 780

gtgacacccc gttctgattc tgcaccctca ctccatcccc actctagttt catccaggat 840
cgagcagggc gaatgggggg ggaggcaccc gagctggccc tggacccggt gcctcaggat 900
gcgtccacca agaagctgag cgagtgtctc aagcgcatcg gggacgaact ggacagtaac 960
atggagctgc agaggtgtgg gcccctgagg acccagaagt ccagccactg ggctccttca 1020
ggacacagga ctctcagccc cgcattctcc tcctccccta agaactagga gtctgggccc 1080
cacaactcag cgcaaacatt ccggactccc agccctcctc tctgccagga tcttaaaacc 1140
ctccttcagg gagtcatttt tcccaccttc ctaaatgtct gtcttgtccc cttcccttgt 1200
cccccgttgg cctgttgctt ttcatttcag cctggcttgg ggctcagtct ccttatcttt 1260
agtgtgcggt ggatgcggga attttccacc atcagcctga tgcctgctcc ccggcactgg 1320
ttctcctctc tcctgcagga tgattgccgc cgtggacaca gactcccccc gagaggtctt 1380
tttccgagtg gcagctgaca tgttttctga cggcaacttc aactggggcc gggttgtcgc 1440
ccttttctac tttgccagca aactggtgct caaggtgggc agctgcaggg cagtgagccc 1500
agggatgctc cccctcagat ctgtgaggac ctggggatcg tggtatcaac cccctgcagt 1560
ggcccagtga ccacagaggg catggagaga gatggctgtg cactgggtgt ctgctccttc 1620
ttttattcat tcaacaagca tttactggac ctgctatgtg ccaggcctat acctggcacc 1680
tgggacacag cactgtacaa agcaggctac atccctgctc tcagggagtt cacgtgcagg 1740
ggtgaagtaa agtgggcagt gtgatttagc agagtggtca ggaaagattt ctattttttt 1800
tttttttttt tttgagatgg agttttgctc ttgttgccca ggcttgagtg caatggcatg 1860
atcttggttc actgcaacct ctgcctccca ggttcaagcg attctcctgc ctcagcttcc 1920
tgagtagctg ggattatggg tgtgcaccat tatccctggc tttttttttt tttttttttt 1980
tttttgtatt tttagtagag acgggtttca ccatgttggt caggctggtc ttgaactcct 2040
gacctcaagt gatccacctg ccttggcctc ccaaagtgct gggattacag gcatgagcca 2100
ccgcaacaag ccaggaaaga cttctaaggg caggtgacat caaagagcag gtgacattaa 2160
agccaaagct agaatgataa gaagcatgca gtgatctata gtgatcgggg ggaagaggca 2220
tctggtagag ggaacagcaa gtgcaaaggc cctgaggtag gaccaagcct catcttttga 2280
cagtagggag gaggccagtg ctgttggaac agagtgaact ggggagaggg tgggaaagga 2340
aggcacagtt gggcaggggc agattgtgtg gagtttttcg ggctgctgga aagacttttt 2400
cttttctttt tttttttttt tttttttttt tttgagacag agtctcactt tgttgcccag 2460
gctggagtgc aatggcgcca tctcggctca ctgcaacctc tgcctcccgg gttcaagtga 2520
ctctcatgct ttagcctccc aagtagctag aattacaggc acacaccacc atgcctggct 2580
agtttttgta tttttagtag agacggagtt tcgccatgtt ggccaggctg gtctcaaact 2640

cctggcctca agtgatccgc ccaccttggc ctcccaaagt gctgggatta caggcgtgaa 2700

ccactgcacc cagcctggaa agactttaac tttactctga gtgtgatggg agtgattggc 2760

tggttttaaa tacggaagcg acaagacctg atttataact ttaagagatt attctagcaa 2820

gtatagatgc cccttgactt gcaatggtgt tacatcctag taatcccatc ataaattgaa 2880

aataacataa gttgaaaatg cattcaatgc cccgataaac ccatagtaag gtcaaaattg 2940

taagtcaaac cgtcataagt tggggactgt ctgtatagca aaatgttaat tgtagaatct 3000

aattataggt ggcggctata tggcttttaa ctgtgaaatt cttttaactt ttcattatga 3060

agattctcat aagaaaatgt ggtgggggtg gtggtgaaat gctcctggct gttgttggcc 3120

aaaagagatc atgaggcaca agggcagaag cgaggatcct ggagaggtgg cacctgtcat 3180

agtcttgctg aaagatgaca agccctggtg gtagcagcag aggtgcgggg ggggcatttt 3240

ttttttaag aggtagggtc ttgctctgtt ggccaggctg gagtgcagta gtgatcatag 3300

ctcactgcag cctcaaaccg ctggggtcaa gcagttctct cgcctcagtc tccagagtag 3360

ctgagactac aggagcatgc caccacgccc cactaactgt tgcattagtc tttttctata 3420

gagacggggt ctcgctatgt tgtccaggct ggtctccaac agggagggat atttcttttt 3480

ggaagatttt tttttttttt tttttttttt tttgatacag ggtctcgctc tgttaccaag 3540

gctggaatgc agtggcatga ccttggctca ctgcagtctc cacctcctgg gttcaagcaa 3600

ttctacctca gcctcctgag aagctgggat tacaggctct ccccaccaca ccagcaaatg 3660

ggagggatat ttctttcttt ctttcttttt tttttttcct gagacggagt ctctctcggt 3720

tgcacccagg ctggagtgca gtggcgcgat ctcagctcac tgcaacctcc acctcctggg 3780

tttaagcgat tctcttgcct cagtctcccg agtagcttgg gattacaggc gtccgccacc 3840

acacccagct aatttttgca tttttagtag agatggggtt tcaccatgtt ggccaggctg 3900

gtctcgaact ccttacctca ggtgatccgc ctgcctcggt ctcccaaagt gctgggatta 3960

caggcgtgag ccaccgtgct cagctgagag ggatatttct tgatgtgttt tcaagattga 4020

gctgatgggg cctgaacgtc cgagatgagg ggaatagcag tgcaggtgat ggtggcacca 4080

cctacagagc ggggatgata gaatagaagt ggccagataa aggctgcagg agaagtcttg 4140

ggagttggga gagcaggtcg gggtccatgg tcaggggttg atcttctctg gtccagaaaa 4200

gtcctctctg gctgggcgtg gtggctcacg cctgtaatcc cagcactttg ggaggccgag 4260

gcaggggat catgaggtca ggagatcgag accatcctgg ctaacatggt gaaaccccgt 4320

ctctactaaa aatacaaaaa attagccggg catggtggtg ggcacttgta gtcccagcta 4380

ctcgggaggc tgaggcagaa gaatggcgtg aactgggagg cggagtttgc agtgagctga 4440

gattgcacca ctgcactcca gcctggagcg acagagtgag actccgtctc aaaaaaaaaa 4500

aaaaaaaaaa aagaaaagtc ctctctgggg aagtgacatt tgccctgaga gctgaagggt 4560

aagaatttgc ttcgtcgagg cctgtttagg cggaaacagg agtacatgaa gaagtcccga 4620
ggcaggaaga atttgatggg aatttaaaaa attaaaaaaa aaaaaaaaaa tggggctggg 4680
catggtggct cacacctgta atcccggcac tttgggaggc tgaggcatgc ggatcaccta 4740
aggccaggag tttgagacca gcctgaccaa catggtgaaa ccttgtctgc actaaaaatt 4800
caaaaaaaaa aaaaaaaata gcccggtgtg gtggcggatg cctgtaatac cagctgctta 4860
ggaggctcag gcaggaggat cacttaaatc tgggaggcgg aggttgcaat gagccgagac 4920
tgcactattg tactccagcc tgggcaacaa gatcaaaact ctatctcaaa aaaaaaaaaa 4980
aaaattagcc aggcatgttg gcaggcacct gtaatcccaa ccctttggga ggctggggca 5040
ggagaattgc ttgaacccag aaggcaaagc tttcagtgag ccgagattgc gccactgcac 5100
tccatcctgg gtgtcagagc aagactccat cttaaaaaaa aaaaatggaa gcagctcagc 5160
gaggcacaaa acaggaagtg gaaaggtgga gtgaggtcag gccaaagcct gcacacaggg 5220
cttgtgggct gcactgtgcc ttcgggtctt catcctgagg gtactgggga gccacagaag 5280
gctcaggggt ggggcagttg agagtaacat tatcttgttt acaattttat ttttttttatt 5340
tatttatttt ttgagacgga ttcttgctct attgtccagg ctggcgtgaa atggcgtgat 5400
ctgggctcac tgcaacctct gcctcctggg ttcaagcgat tcacctgcct cagcatccca 5460
aggagctggg attacaggtg cctgccacca cacccagcta atttttgtat ttatttattt 5520
tgagatggag ttttgctctt gttgcccagg ctggagtgca atggcgcaac ctcggctcac 5580
tgcaacctcc gcctcccggg ttcaagcaat tctcctgcct cagactccca agtagctggg 5640
attacaggca tgtgccacca cgcccggcta attttgtatt tttagtagag atggcattac 5700
tccgtattgg tcaggctggt cttgaactcc cgacctcaag tgatccgcct gccttggcct 5760
cccaaagtgc tgggattaca ggcatgagcc gccgcacctg gccatgttta caatttttga 5820
agccgacttc aattgtgggt ggcagaaatc tttgagggga ggcaaagaat tgacaaagga 5880
ggtttggggc cactatctcc aggcagtggg gacaaggttc agtccctaac gcccactcca 5940
ctccccacag gccctgtgca ccaaggtgcc ggaactgatc agaaccatca tgggctggac 6000
attggacttc ctccgggagc ggctgttggg ctggatccaa gaccagggtg gttgggtgag 6060
actcctcaag cctcctcacc cccaccaccg cgccctcacc accgcccctg ccccaccgtc 6120
cctgcccccc gccactcctc tgggaccctg ggccttctgg agcaggtcac agtggtgccc 6180
tctccccatc ttcagatcat cagatgtggt ctataatgcg ttttccttac gtgtctgatc 6240
aatccccgat tcatctaccc tgctgacctc ccagtgaccc ctgacctcac tgtgaccttg 6300
acttgattag tgccttctgc cctccctgga gcctccactg cctctggaat tgctcaagtt 6360
cattgatgac cctctgaccc tagctctttc cttttttttt ttttccccac tgagaagggg 6420

tctcgctatg ttgcccaggt tggtctcgaa ctcctggcct caagcgatcc tcccgcctca 6480

gcctctcaaa gtgctgggat tacaggtgtg agccaccatg cctggcctga gtccagctct 6540

ttaatgcccg ttcatctcag tcccctgccc gcaatcctgc cttctggcct cctccgtccc 6600

tgatcccgcc tctgcctgcc caggggctgc ccctggccga gtcactgaag cgactgatgt 6660

ccctgtctcc aggacggcct cctctcctac tttgggacgc ccacgtggca gaccgtgacc 6720

atctttgtgg cgggagtgct caccgcctca ctcaccatct ggaagaagat gggctgaggc 6780

ccccagctgc cttggactgt gtttttcctc cataaattat ggcatttttc tgggaggggt 6840

ggggattggg ggacgtgggc atttttctta cttttgtaat tattgggggg tgtggggaag 6900

agtggtcttg agggggtaat aaacctcctt cgggacaca 6939

<210> 7
<211> 21
<212> DNA
<213> Artificial

<220>
<223> Synthesis

<220>
<221> misc_signal
<222> (1)..(21)

<400> 7
tggagctgca gaggatgatt g 21

<210> 8
<211> 20
<212> DNA
<213> Artificial

<220>
<223> Synthesis

<220>
<221> misc_signal
<222> (1)..(20)

<400> 8
gctgccactc ggaaaaagac 20

<210> 9

<211> 2655
<212> DNA
<213> homo sapiens

<400> 9
cccagaaggc cgcggggggt ggaccgccta agagggcgtg cgctcccgac atgccccgcg 60

gcgcgccatt aaccgccaga tttgaatcgc gggacccgtt ggcagaggtg gcggcggcgg 120

catgggtgcc ccgacgttgc cccctgcctg gcagcccttt ctcaaggacc accgcatctc 180
tacattcaag aactggccct tcttggaggg ctgcgcctgc accccggagc ggatggccga 240
ggctggcttc atccactgcc ccactgagaa cgagccagac ttggcccagt gtttcttctg 300
cttcaaggag ctggaaggct gggagccaga tgacgacccc atagaggaac ataaaaagca 360
ttcgtccggt tgcgctttcc tttctgtcaa gaagcagttt gaagaattaa cccttggtga 420
atttttgaaa ctggacagag aaagagccaa gaacaaaatt gcaaaggaaa ccaacaataa 480
gaagaaagaa tttgaggaaa ctgcggagaa agtgcgccgt gccatcgagc agctggctgc 540
catggattga ggcctctggc cggagctgcc tggtcccaga gtggctgcac cacttccagg 600
gtttattccc tggtgccacc agccttcctg tgggcccctt agcaatgtct taggaaagga 660
gatcaacatt ttcaaattag atgtttcaac tgtgctcttg ttttgtcttg aaagtggcac 720
cagaggtgct tctgcctgtg cagcgggtgc tgctggtaac agtggctgct tctctctctc 780
tctctctttt ttgggggctc atttttgctg ttttgattcc cgggcttacc aggtgagaag 840
tgagggagga agaaggcagt gtcccttttg ctagagctga cagctttgtt cgcgtgggca 900
gagccttcca cagtgaatgt gtctggacct catgttgttg aggctgtcac agtcctgagt 960
gtggacttgg caggtgcctg ttgaatctga gctgcaggtt ccttatctgt cacacctgtg 1020
cctcctcaga ggacagtttt tttgttgttg tgtttttttg tttttttttt tttggtagat 1080
gcatgacttg tgtgtgatga gagaatggag acagagtccc tggctcctct actgtttaac 1140
aacatggctt tcttattttg tttgaattgt taattcacag aatagcacaa actacaatta 1200
aaactaagca caaagccatt ctaagtcatt ggggaaacgg ggtgaacttc aggtggatga 1260
ggagacagaa tagagtgata ggaagcgtct ggcagatact ccttttgcca ctgctgtgtg 1320
attagacagg cccagtgagc cgcggggcac atgctggccg ctcctccctc agaaaaaggc 1380
agtggcctaa atcctttta aatgacttgg ctcgatgctg tgggggactg gctgggctgc 1440
tgcaggccgt gtgtctgtca gcccaacctt cacatctgtc acgttctcca cacgggggag 1500
agacgcagtc cgcccaggtc cccgctttct ttggaggcag cagctcccgc agggctgaag 1560
tctggcgtaa gatgatggat ttgattcgcc ctcctccctg tcatagagct gcagggtgga 1620
ttgttacagc ttcgctggaa acctctggag gtcatctcgg ctgttcctga gaaataaaaa 1680
gcctgtcatt tcaaacactg ctgtggaccc tactgggttt ttaaaatatt gtcagttttt 1740
catcgtcgtc cctagcctgc caacagccat ctgcccagac agccgcagtg aggatgagcg 1800
tcctggcaga gacgcagttg tctctgggcg cttgccagag ccacgaaccc cagacctgtt 1860
tgtatcatcc gggctccttc cgggcagaaa caactgaaaa tgcacttcag acccacttat 1920
ttctgccaca tctgagtcgg cctgagatag acttttccct ctaaactggg agaatatcac 1980

agtggttttt gttagcagaa aatgcactcc agcctctgta ctcatctaag ctgcttattt 2040

ttgatatttg tgtcagtctg taaatggata cttcacttta ataactgttg cttagtaatt 2100

ggctttgtag agaagctgga aaaaaatggt tttgtcttca actcctttgc atgccaggcg 2160

gtgatgtgga tctcggcttc tgtgagcctg tgctgtgggc agggctgagc tggagccgcc 2220

cctctcagcc cgcctgccac ggcctttcct taaaggccat ccttaaaacc agaccctcat 2280

ggctaccagc acctgaaagc ttcctcgaca tctgttaata aagccgtagg cccttgtcta 2340

agtgcaaccg cctagacttt ctttcagata catgtccaca tgtccatttt tcaggttctc 2400

taagttggag tggagtctgg gaagggttgt gaatgaggct tctgggctat gggtgaggtt 2460

ccaatggcag gttagagccc ctcgggccaa ctgccatcct ggaaagtaga gacagcagtg 2520

cccgctgccc agaagagacc agcaagccaa actggagccc ccattgcagg ctgtcgccat 2580

gtggaaagag taactcacaa ttgccaataa agtctcatgt ggttttatct aaaaaaaaaa 2640

aaaaaaaaaa aaaaa 2655

<210> 10
<211> 10429
<212> DNA
<213> homo sapiens

<400> 10
ccgccagatt tgaatcgcgg gacccgttgg cagaggtggc ggcggcggca tgggtgcccc 60

gacgttgccc cctgcctggc agccctttct caaggaccac cgcatctcta cattcaagaa 120

ctggcccttc ttggagggct gcgcctgcac cccggagcgg gtgagactgc ccggcctcct 180

ggggtccccc acgcccgcct tgccctgtcc ctagcgaggc cactgtgact gggcctcggg 240

ggtacaagcc gccctcccct ccccgtcctg tccccagcga ggccactgtg gctgggcccc 300

ttgggtccag gccggcctcc cctccctgct ttgtccccat cgaggccttt gtggctgggc 360

ctcggggttc cgggctgcca cgtccactca cgagctgtgc tgtcccttgc agatggccga 420

ggctggcttc atccactgcc ccactgagaa cgagccagac ttggcccagt gtttcttctg 480

cttcaaggag ctggaaggct gggagccaga tgacgacccc atgtaagtct tctctggcca 540

gcctcgatgg gctttgtttt gaactgagtt gtcaaaagat ttgagttgca aagacactta 600

gtatgggagg gttgctttcc accctcattg cttcttaaac agctgttgtg aacggatacc 660

tctctatatg ctggtgcctt ggtgatgctt acaacctaat taaatctcat ttgaccaaaa 720

tgccttgggg tggacgtaag atgcctgatg cctttcatgt tcaacagaat acatcagcag 780

accctgttgt tgtgaactcc caggaacgtc caagtgcttt ttttgagatt ttttaaaaaa 840

cagtttaatt gaaatataac ctacacagca caaaaattac cctttgaaag tgtgcacttc 900

acactttcgg aggctgaggc gggcggatca cctgaggtca ggagttcaag acctgcctgg 960

ccaacttggc gaaaccccgt ctctactaaa aatacaaaaa ttagccgggc atggtagcgc 1020

acgcccgtaa tcccagctac tcgggaggct aaggcaggag aatcgcttga acctgggagg 1080

cggaggttgc agtgagccga gattgtgcca atgcactcca gcctcggcga cagagcgaga 1140

ctccgtcata aaaataaaaa attgaaaaaa aaaaaagaaa gaaagcatat acttcagtgt 1200

tgttctggat tttttcttc aagatgccta gttaatgaca atgaaattct gtactcggat 1260

ggtatctgtc tttccacact gtaatgccat attcttttct cacctttttt tctgtcggat 1320

tcagttgctt ccacagcttt aatttttttc ccctggagaa tcaccccagt tgtttttctt 1380

tttggccaga agagagtagc tgtttttttt cttagtatgt ttgctatggt ggttatactg 1440

catccccgta atcactggga aaagatcagt ggtattcttc ttgaaaatga ataagtgtta 1500

tgatattttc agattagagt tacaactggc tgtctttttg gactttgtgt ggccatgttt 1560

tcattgtaat gcagttctgg taacggtgat agtcagttat acagggagac tcccctagca 1620

gaaaatgaga gtgtgagcta gggggtccct tggggaaccc ggggcaataa tgcccttctc 1680

tgcccttaat ccttacagtg ggccgggcac ggtggcttac gcctgtaata ccagcacttt 1740

gggaggccga ggcgggcgga tcacgaggtc aggagatcga gaccatcttg gctaatacgg 1800

tgaaaccccg tctccactaa aaatacaaaa aattagccgg gcgtggtggt gggcgcctgt 1860

agtcccagct actcgggagg ctgaggcagg agaatggcgt gaacccagga ggcggagctt 1920

gcagtgagcc gagattgcac cactgcactc cagcctgggc gacagaatga gactccgtct 1980

caaaaaaaaa aaaaaaagaa aaaaatcttt acagtggatt acataacaat tccagtgaaa 2040

tgaaattact tcaaacagtt ccttgagaat gttggaggga tttgacatgt aattcctttg 2100

gacatatacc atgtaacact tttccaacta attgctaagg aagtccagat aaaatagata 2160

cattagccac acagatgtgg ggggagatgt ccacagggag agagaaggtg ctaagaggtg 2220

ccatatggga atgtggcttg ggcaaagcac tgatgccatc aacttcagac ttgacgtctt 2280

actcctgagg cagagcaggg tgtgcctgtg gagggcgtgg ggaggtggcc cgtggggagt 2340

ggactgccgc tttaatccct tcagctgcct ttccgctgtt gttttgattt ttctagagag 2400

gaacataaaa agcattcgtc cggttgcgct ttcctttctg tcaagaagca gtttgaagaa 2460

ttaacccttg gtgaattttt gaaactggac agagaaagag ccaagaacaa aattgtatgt 2520

attgggaata agaactgctc aaaccctgtt caatgtcttt agcactaaac tacctagtcc 2580

ctcaaaggga ctctgtgttt tcctcaggaa gcattttttt ttttttctg agatagagtt 2640

tcactcttgt tgcccaggct ggagtgcaat ggtgcaatct tggctcactg caacctctgc 2700

ctctcgggtt caagtgattc tcctgcctca gcctcccaag taactgggat tacagggaag 2760

tgccaccaca cccagctaat ttttgtattt ttagtagaga tggggtttca ccacattgcc 2820

caggctggtc ttgaactcct gacctcgtga ttcgcccacc ttggcctccc aaagtgctgg 2880

gattacaggc gtgaaccacc acgcctggct tttttttttt tgttctgaga cacagtttca 2940
ctctgttacc caggctggag tggggtggcc tgatctcgga tcactgcaac ctccgcctcc 3000
tgggctcaag tgatttgcct gcttcagcct cccaagtagc cgagattaca ggcatgtgcc 3060
accacaccca ggtaattttt gtatttttgg tagagacgag gtttcaccat gttggccagg 3120
ctggtcttga actcctgacc tcaggtgatc cacccgcctc agcctcccaa agtgctgaga 3180
ttataggtgt gagccaccac acctggcctc aggaagtatt tttattttta aatttattta 3240
tttatttgag atggagtctt gctctgtcgc ccaggctaga gtgcagcgac gggatctcgg 3300
ctcactgcaa gctccgcccc ccaggttcaa gccattctcc tgcctcagcc tcccgagtag 3360
ctgggactac aggcgcccgc caccacaccc ggctaatttt tttgtatttt tagtagagac 3420
gggttttcac cgtgttagcc aggagggtct cgatctcctg acctcgtgat ctgcctgcct 3480
cggcctccca aagtgctggg attacaggtg tgagccacca cacccggcta tttttatttt 3540
tttgagacag ggactcactc tgtcacctgg gctgcagtgc agtggtacac catagctcac 3600
tgcagcctcg aactcctgag ctcaagtgat cctcccacct catcctccca agtaattggg 3660
actacaggcg caccccacca tgcccacctt atttatttat ttatttattt atttatttat 3720
tttcatagag atgagggttc cctgtgttgt ccaggctggt cttgaactcc tgagctcaag 3780
ggatcctttt gcctgggcct cccaaagtgc tgagattaca ggcatgagcc accgtgccca 3840
gctaggaatc atttttaaag cccctaggat gtctgtgtga ttttaaagct cctggagtgt 3900
ggccggtata agtatatacc ggtataagta aatcccacat tttgtgtcag tatttactag 3960
aaacttagtc atttatctga agttgaaatg taactgggct ttatttattt atttatttat 4020
ttatttattt ttaatttttt tttttgagac gagtctcact ttgtcaccca ggctggagtg 4080
cagtggcacg atctcggctc actgcaacct ctgcctcccg ggtcaagcg attctcctgc 4140
cttagcctcc cgagtagctg ggactacagg cacgcaccac catgcctggc taatttttgt 4200
atttttagta gacggggttt caccatgctg gccaagctgg tctcaaactc ctgaccttgt 4260
gatctgcccg ctttagcctc ccagagtgct gggattacag gcatgagcca ccatgcgtgg 4320
tctttttaaa attttttgat tttttttttt tttgagacag agccttgctc tgtcgcccag 4380
gctggagtgc agtggcacga tctcagctca ctacaagctc cgcctcccgg gttcacgcca 4440
ttcttctgcc tcagcctcct gagtagctgg gactacaggt gcccaccacc acgcctggct 4500
aatttttttt ggtattttta ttagagacaa ggtttcatca tgttggccag gctggtctca 4560
aactcctgac ctcaagtgat ctgcctgcct cggcctccca aagcgctgag attacaggtg 4620
tgatctactg caccaggcct gggcgtcata tattcttatt tgctaagtct ggcagcccca 4680
cacagaataa gtactggggg attccatatc cttgtagcaa agccctgggt ggagagtcag 4740

gagatgttgt agttctgtct ctgccacttg cagactttga gtttaagcca gtcgtgctca 4800
tgctttcctt gctaaataga ggttagaccc cctatcccat ggtttctcag gttgcttttc 4860
agcttgaaaa ttgtattcct ttgtagagat cagcgtaaaa taattctgtc cttatatgtg 4920
gctttatttt aatttgagac agagtgtcac tcagtcgccc aggctggagt gtggtggtgc 4980
gatcttggct cactgcgacc tccacctccc aggttcaagc gattctcgtg cctcaggctc 5040
ccaagtagct gagattatag gtgtgtgcca ccaggcccag ctaacttttg tatttttagt 5100
agagacaggg ttttgccatg ttggctaagc tggtctcgaa ctcctggcct caagtgatct 5160
gcccgccttg gcatcccaaa gtgctgggat tacaggtgtg aaccaccaca cctggcctca 5220
atatagtggc ttttaagtgc taaggactga gattgtgttt tgtcaggaag aggccagttg 5280
tgggtgaagc atgctgtgag agagcttgtc acctggttga ggttgtggga gctgcagcgt 5340
gggaactgga aagtgggctg gggatcatct ttttccaggt caggggtcag ccagcttttc 5400
tgcagcgtgc catagaccat ctcttagccc tcgtgggtca gagtctctgt tgcatattgt 5460
cttttgttgt ttttcacaac cttttagaaa cataaaaagc attcttagcc cgtgggctgg 5520
acaaaaaaag gccatgacgg gctgtatgga tttggcccag caggcccttg cttgccaagc 5580
cctgttttag acaaggagca gcttgtgtgc ctggaaccat catgggcaca ggggaggagc 5640
agagtggatg tggaggtgtg agctggaaac caggtcccag agcgctgaga aagacagagg 5700
gtttttgccc ttgcaaatag agcaactgaa atctgacacc atccagttcc agaaagccct 5760
gaagtgctgg tggacgctgc ggggtgctcc gctctagggt tacagggatg aagatgcagt 5820
ctggtagggg gagtccactc acctgttgga agatgtgatt aagaaaagta gactttcagg 5880
gccgggcatg gtggctcacg cctgtaatcc cagcactttg ggaggccgag gcgggtggat 5940
cacgaggtca ggagatcgag accatcctgg ctaacatggt gaaaccccgt ctttactaaa 6000
aatacaaaaa attagctggg cgtggtggcg ggcgcctgta gtcccagcta ctcgggaggc 6060
tgaggcagga gaatggcgtg aacctgggag gtggagcttg cagtgagccg agatcgcgcc 6120
actgcactcc agcctgggcg acagagcgag actccgtctc aaaaaaaaaa aaaaaaagta 6180
ggctttcatg atgtgtgagc tgaaggcgca gtaggcagaa gtagaggcct cagtccctgc 6240
aggagacctc tcggtctcta tctcctgata gtcagaccca gccacactgg aaagagggga 6300
gacattacag cctgcaagaa aagtagggag atttaaaaac tgcttggctt ttattttgaa 6360
ctgttttttt tgtttgtttg ttttccccaa ttcagaatac agaatacttt tatggatttg 6420
tttttattac tttaattttg aaacaatata atcttttttt tgttgttttt ttgagacggg 6480
gtcttactct gtcacccagg ctgagtgcag tggtgtgatc ttggctcacc tcagcctcga 6540
ccccctgggc tcaaatgatt ctcccacctc agcttcccaa gtagctggga ccacaggtgc 6600
gtgtgttgcg ctatacaaat cctgaagaca aggatgctgt tgctggtgat gctggggatt 6660

```
cccaagatcc cagatttgat ggcaggatgc ccctgtctgc tgccttgcca gggtgccagg    6720
agggcgctgc tgtggaagct gaggcccggc catccagggc gatgcattgg gcgctgattc    6780
ttgttcctgc tgctgcctcg gtgcttagct tttgaaacaa tgaaataaat tagaaccagt    6840
gtgaaaatcg atcaggaaat aaatttaatg tggaaataaa ctgaacaact tagttcttca    6900
taagagttta cttggtaaat acttgtgatg aggacaaaac gaagcactag aaggagaggc    6960
gagttgtaga cctgggtggc aggagtgttt tgtttgtttt ctttggcagg gtcttgctct    7020
gttgctcagg ctggagtaca gtggcgcaat cacagctcac tatagcctcg acctcctgga    7080
ctcaagcaat cctcctgcct cagcctccca gtagctggga ctacaggcgc atgccaccat    7140
gcctggctaa ttttaaattt tttttttct cttttttgag atggaatctc actctgtcgc    7200
ccaggctgga gtgcagtggc gtgatctcgg ctgacggcaa gctccgcctc ccaggttcac    7260
tccattcgcc tgcctcagcc tcccaagtag ctgggactac aggcgctggg attacaaacc    7320
caaacccaaa gtgctgggat tacaggcgtg agccaccgca cccggcctgt tttgtctttc    7380
aatagcaaga gttgtgtttg cttcgcccct acctttagtg gaaaaatgta taaaatggag    7440
atattgacct ccacattggg gtggttaaat tatagcatgt atgcaaagga gcttcgctaa    7500
tttaaggctt ttttgaaaga gaagaaactg aataatccat gtgtgtatat atattttaaa    7560
agccatggtc atctttccat atcagtaaag ctgaggctcc ctgggactgc agagttgtcc    7620
atcacagtcc attataagtg cgctgctggg ccaggtgcag tggcttgtgc ctgaatccca    7680
gcactttggg aggccaaggc aggaggattc attgagccca ggagttttga ggcgagcctg    7740
ggcaatgtgg ccagacctca tctcttcaaa aaatacacaa aaaattagcc aggcatggtg    7800
gcacgtgcct gtagtctcag ctactcagga ggctgaggtg ggaggatcac tttgagcctt    7860
gcaggtcaaa gctgcagtaa gccatgatct tgccactgca ttccagcctg gatgacagag    7920
cgagaccctg tctctaaaaa aaaaaaaacc aaacggtgca ctgttttctt ttttcttatc    7980
aatttattat ttttaaatta aattttcttt taataattta taaattataa atttatatta    8040
aaaaatgaca aatttttatt acttatacat gaggtaaaac ttaggatata taaagtacat    8100
attgaaaagt aatttttttgg ctggcacagt ggctcacacc tgtaatccca gcactttggg    8160
aggccgtggc gggcagatca catgagatca tgagttcgag accaacctga ccaacatgga    8220
gagaccccat ctctactaaa aatacaaaat tagccggggt ggtggcgcat gcctgtaatc    8280
ccagctactc gggaggctga ggcaggagaa tctcttgaac ccgggaggca gaggttgcgg    8340
tgagccaaga tcgtgccttt gcacaccagc caaggcaaca agagcgaaag tccgtctcaa    8400
aaaaaaagta atttttttta agttaacctc tgtcagcaaa caaatttaac ccaataaagg    8460
tctttgtttt ttaatgtagt agaggagtta gggtttataa aaaatatggt agggaagggg    8520
```

gtccctggat ttgctaatgt gattgtcatt tgccccttag gagagagctc tgttagcaga 8580
atgaaaaaat tggaagccag attcagggag ggactggaag caaaagaatt tctgttcgag 8640
gaagagcctg atgtttgcca gggtctgttt aactggacat gaagaggaag gctctggact 8700
ttcctccagg agtttcagga gaaaggtagg gcagtggtta agagcagagc tctgcctaga 8760
ctagctgggg tgcctagact agctggggtg cccagactag ctggggtgcc tagactagct 8820
gggtactttg agtggctcct tcagcctgga cctcggtttc ctcacctgta tagtagagat 8880
atgggagcac ccagcgcagg atcactgtga acataaatca gttaatggag gaagcaggta 8940
gagtggtgct gggtgcatac caagcactcc gtcagtgttt cctgttattc gatgattagg 9000
aggcagctta aactagaggg agttgagctg aatcaggatg tttgtcccag gtagctggga 9060
atctgcctag cccagtgccc agtttattta ggtgctctct cagtgttccc tgattgtttt 9120
ttcctttgtc atcttatcta caggatgtga ctgggaagct ctggtttcag tgtcatgtgt 9180
ctattcttta tttccaggca aaggaaacca acaataagaa gaaagaattt gaggaaactg 9240
cggagaaagt gcgccgtgcc atcgagcagc tggctgccat ggattgaggc ctctggccgg 9300
agctgcctgg tcccagagtg gctgcaccac ttccagggtt tattccctgg tgccaccagc 9360
cttcctgtgg gccccttagc aatgtcttag gaaaggagat caacattttc aaattagatg 9420
tttcaactgt gctcttgttt tgtcttgaaa gtggcaccag aggtgcttct gcctgtgcag 9480
cgggtgctgc tggtaacagt ggctgcttct ctctctctct ctcttttttg ggggctcatt 9540
tttgctgttt tgattcccgg gcttaccagg tgagaagtga gggaggaaga aggcagtgtc 9600
ccttttgcta gagctgacag ctttgttcgc gtgggcagag ccttccacag tgaatgtgtc 9660
tggacctcat gttgttgagg ctgtcacagt cctgagtgtg gacttggcag gtgcctgttg 9720
aatctgagct gcaggttcct tatctgtcac acctgtgcct cctcagagga cagttttttt 9780
gttgttgtgt ttttttgttt ttttttttt ggtagatgca tgacttgtgt gtgatgagag 9840
aatggagaca gagtccctgg ctcctctact gtttaacaac atggctttct tattttgttt 9900
gaattgttaa ttcacagaat agcacaaact acaattaaaa ctaagcacaa agccattcta 9960
agtcattggg gaaacggggt gaacttcagg tggatgagga gacagaatag agtgatagga 10020
agcgtctggc agatactcct tttgccactg ctgtgtgatt agacaggccc agtgagccgc 10080
ggggcacatg ctggccgctc ctccctcaga aaaaggcagt ggcctaaatc ctttttaaat 10140
gacttggctc gatgctgtgg gggactggct gggctgctgc aggccgtgtg tctgtcagcc 10200
caaccttcac atctgtcacg ttctccacac gggggagaga cgcagtccgc ccaggtcccc 10260
gctttctttg gaggcagcag ctcccgcagg gctgaagtct ggcgtaagat gatggatttg 10320
attcgccctc ctccctgtca tagagctgca gggtggattg ttacagcttc gctggaaacc 10380
tctggaggtc atctcggctg ttcctgagaa ataaaaagcc tgtcatttc 10429

<210> 11
<211> 24
<212> DNA
<213> Artificial

<220>
<223> Synthesis

<220>
<221> misc_signal
<222> (1)..(24)

<400> 11
gacgacccca tagaggaaca taaa 24

<210> 12
<211> 25
<212> DNA
<213> Artificial

<220>
<223> Synthesis

<220>
<221> misc_signal
<222> (1)..(25)

<400> 12
caccaagggt taattcttca aactg 25

<210> 13
<211> 4306
<212> DNA
<213> homo sapiens

<400> 13
cacacggact acaggggagt tttgttgaag ttgcaaagtc ctggagcctc cagagggctg 60

tcggcgcagt agcagcgagc agcagagtcc gcacgctccg gcgaggggca gaagagcgcg 120

agggagcgcg gggcagcaga agcgagagcc gagcgcggac ccagccagga cccacagccc 180

tccccagctg cccaggaaga gccccagcca tggaacacca gctcctgtgc tgcgaagtgg 240

aaaccatccg ccgcgcgtac cccgatgcca acctcctcaa cgaccgggtg ctgcgggcca 300

tgctgaaggc ggaggagacc tgcgcgccct cggtgtccta cttcaaatgt gtgcagaagg 360

aggtcctgcc gtccatgcgg aagatcgtcg ccacctggat gctggaggtc tgcgaggaac 420

agaagtgcga ggaggaggtc ttcccgctgg ccatgaacta cctggaccgc ttcctgtcgc 480

tggagcccgt gaaaaagagc cgcctgcagc tgctgggggc cacttgcatg ttcgtggcct 540

ctaagatgaa ggagaccatc cccctgacgg ccgagaagct gtgcatctac accgacaact 600

ccatccggcc cgaggagctg ctgcaaatgg agctgctcct ggtgaacaag ctcaagtgga 660

acctggccgc aatgaccccg cacgatttca ttgaacactt cctctccaaa atgccagagg 720
cggaggagaa caaacagatc atccgcaaac acgcgcagac cttcgttgcc ctctgtgcca 780
cagatgtgaa gttcatttcc aatccgccct ccatggtggc agcggggagc gtggtggccg 840
cagtgcaagg cctgaacctg aggagcccca acaacttcct gtcctactac cgcctcacac 900
gcttcctctc cagagtgatc aagtgtgacc cagactgcct ccgggcctgc caggagcaga 960
tcgaagccct gctggagtca agcctgcgcc aggcccagca gaacatggac cccaaggccg 1020
ccgaggagga ggaagaggag gaggaggagg tggacctggc ttgcacaccc accgacgtgc 1080
gggacgtgga catctgaggg cgccaggcag gcgggcgcca ccgccacccg cagcgagggc 1140
ggagccggcc ccaggtgctc ccctgacagt ccctcctctc cggagcattt tgataccaga 1200
agggaaagct tcattctcct tgttgttggt tgtttttttcc tttgctcttt ccccccttcca 1260
tctctgactt aagcaaaaga aaaagattac ccaaaaactg tctttaaaag agagagagag 1320
aaaaaaaaaa tagtatttgc ataaccctga gcggtggggg aggagggttg tgctacagat 1380
gatagaggat tttatacccc aataatcaac tcgtttttat attaatgtac ttgtttctct 1440
gttgtaagaa taggcattaa cacaaaggag gcgtctcggg agaggattag gttccatcct 1500
ttacgtgttt aaaaaaaagc ataaaaacat tttaaaaaca tagaaaaatt cagcaaacca 1560
tttttaaagt agaagagggt tttaggtaga aaaacatatt cttgtgcttt tcctgataaa 1620
gcacagctgt agtggggttc taggcatctc tgtactttgc ttgctcatat gcatgtagtc 1680
actttataag tcattgtatg ttattatatt ccgtaggtag atgtgtaacc tcttcacctt 1740
attcatggct gaagtcacct cttcgttaca gtagcgtagc gtggccgtgt gcatgtcctt 1800
tgcgcctgtg accaccaccc caacaaacca tccagtgaca aaccatccag tggaggtttg 1860
tcgggcacca gccagcgtag cagggtcggg aaaggccacc tgtcccactc ctacgatacg 1920
ctactataaa gagaagacga aatagtgaca taatatattc tatttttata ctcttcctat 1980
ttttgtagtg acctgtttat gagatgctgg ttttctaccc aacggccctg cagccagctc 2040
acgtccaggt tcaacccaca gctacttggt ttgtgttctt cttcatattc taaaaccatt 2100
ccatttccaa gcactttcag tccaataggt gtaggaaata gcgctgtttt tgttgtgtgt 2160
gcagggaggg cagttttcta atggaatggt ttgggaatat ccatgtactt gtttgcaagc 2220
aggactttga ggcaagtgtg ggccactgtg gtggcagtgg aggtggggtg tttgggaggc 2280
tgcgtgccag tcaagaagaa aaaggtttgc attctcacat tgccaggatg ataagttcct 2340
ttccttttct ttaaagaagt tgaagtttag gaatcctttg gtgccaactg gtgtttgaaa 2400
gtagggacct cagaggttta cctagagaac aggtggtttt taagggttat cttagatgtt 2460
tcacaccgga aggtttttaa acactaaaat atataattta tagttaaggc taaaaagtat 2520

atttattgca gaggatgttc ataaggccag tatgatttat aaatgcaatc tccccttgat 2580

ttaaacacac agatacacac acacacacac acacacacac aaaccttctg cctttgatgt 2640

tacagattta atacagttta tttttaaaga tagatccttt tataggtgag aaaaaaacaa 2700

tctggaagaa aaaaaccaca caaagacatt gattcagcct gtttggcgtt tcccagagtc 2760

atctgattgg acaggcatgg gtgcaaggaa aattagggta ctcaacctaa gttcggttcc 2820

gatgaattct tatcccctgc cccttccttt aaaaaactta gtgacaaaat agacaatttg 2880

cacatcttgg ctatgtaatt cttgtaattt ttatttagga agtgttgaag ggaggtggca 2940

agagtgtgga ggctgacgtg tgagggagga caggcgggag gaggtgtgag gaggaggctc 3000

ccgaggggaa ggggcggtgc ccacaccggg gacaggccgc agctccattt tcttattgcg 3060

ctgctaccgt tgacttccag gcacggtttg gaaatattca catcgcttct gtgtatctct 3120

ttcacattgt ttgctgctat tggaggatca gtttttttgtt ttacaatgtc atatactgcc 3180

atgtactagt tttagttttc tcttagaaca ttgtattaca gatgcctttt ttgtagtttt 3240

tttttttttt atgtgatcaa ttttgactta atgtgattac tgctctattc caaaaaggtt 3300

gctgtttcac aatacctcat gcttcactta gccatggtgg acccagcggg caggttctgc 3360

ctgctttggc gggcagacac gcgggcgcga tcccacacag gctggcgggg gccggccccg 3420

aggccgcgtg cgtgagaacc gcgccggtgt ccccagagac caggctgtgt ccctcttctc 3480

ttccctgcgc ctgtgatgct gggcacttca tctgatcggg ggcgtagcat catagtagtt 3540

tttacagctg tgttattctt tgcgtgtagc tatggaagtt gcataattat tattattatt 3600

attataacaa gtgtgtctta cgtgccacca cggcgttgta cctgtaggac tctcattcgg 3660

gatgattgga atagcttctg gaatttgttc aagttttggg tatgtttaat ctgttatgta 3720

ctagtgttct gtttgttatt gttttgttaa ttacaccata atgctaattt aaagagactc 3780

caaatctcaa tgaagccagc tcacagtgct gtgtgccccg gtcacctagc aagctgccga 3840

accaaaagaa tttgcacccc gctgcgggcc cacgtggttg ggccctgcc ctggcagggt 3900

catcctgtgc tcggaggcca tctcgggcac aggcccaccc cgccccaccc ctccagaaca 3960

cggctcacgc ttacctcaac catcctggct gcggcgtctg tctgaaccac gcgggggcct 4020

tgagggacgc tttgtctgtc gtgatggggc aagggcacaa gtcctggatg ttgtgtgtat 4080

cgagaggcca aaggctggtg gcaagtgcac ggggcacagc ggagtctgtc ctgtgacgcg 4140

caagtctgag ggtctgggcg gcgggcggct ggtctgtgc atttctggtt gcaccgcggc 4200

gcttcccagc accaacatgt aaccggcatg tttccagcag aagacaaaaa gacaaacatg 4260

aaagtctaga aataaaactg gtaaaacccc aaaaaaaaaa aaaaaa 4306

<210> 14
<211> 13370

<212> DNA
<213> homo sapiens

<400> 14
cacacggact acaggggagt tttgttgaag ttgcaaagtc ctggagcctc cagagggctg 60

tcggcgcagt agcagcgagc agcagagtcc gcacgctccg gcgaggggca gaagagcgcg 120

agggagcgcg gggcagcaga agcgagagcc gagcgcggac ccagccagga cccacagccc 180

tccccagctg cccaggaaga gccccagcca tggaacacca gctcctgtgc tgcgaagtgg 240

aaaccatccg ccgcgcgtac cccgatgcca acctcctcaa cgaccgggtg ctgcgggcca 300

tgctgaaggc ggaggagacc tgcgcgccct cggtgtccta cttcaaatgt gtgcagaagg 360

aggtcctgcc gtccatgcgg aagatcgtcg ccacctggat gctggaggtg cggggcttcg 420

ggcggctctc ttaagacttc cctgcaactt gttgcccaga cccacgtttc tttgctactc 480

acccccctcc cttctctccc gctagaactt tgaagtttgc cgtggtgttt ctagggatcc 540

gtattttcaa aataaaaatt gcgggtattt tctgaaggag gaaggggtgg gggtgggggt 600

gctagaagta gcgtttcgtg ggaggggaga aggggggtccg ggaggggtgc cttcgggaga 660

agccagtgcc aggggcaccc caatgggccc gagggtgcgg gctggcaggc tgggtgcgct 720

ttgtgtcccc cgcctgcgcc ccagcccggc tgcgcctcag cggccgggag ccgccaactc 780

cggggggagg gggcatagat ttgattttta aattaatatc catggacacg tatgcaaggg 840

ccgctcgtgc cagtattatg cgccatcttt gctcttttat tgcaaagcaa aagtgtttat 900

taataattgg gggcagggtg ggggcgggga gcggccgccg ggcgctgggg ccgcagctaa 960

gggccgcgcg gctgccggga gcccgcggga ggggcgcagg gacgcggcat gggtagtttt 1020

gggggggacgc cgctagggaa ggggggggcct ttgttcaagc agcgagtccc ggggcgcccc 1080

gaacgggcag cctgggccgg agagcacggc gagctgcaag gtcgcgtggc cccaagacg 1140

ccagggcttg atccccgtct gcagggatat cggcttggag gaccttctcc gagcgagccg 1200

ggggcctggg agcacatttt cagaccttcg gtgggcgcct gaggggcccg caagtatttt 1260

aaaataattt ttgaaagtgc ggcgtggtgc ccttgcgaga gggaaacgcc gcccgcgccc 1320

agggggaagg gggggccccg gagtttgaat tcctggggct ccccccggag cctgtaacga 1380

actcccaacc cccggcctgg gtaaagggtc gcccgagggt cattttcagg gtttttttat 1440

gcacttagtt attttttttaa tatttttaaa tattttttga aaagatgacg tctggggaaa 1500

tgcggcgcgg cggcctggga cgccaccttt gtgtctcgca ggcgcggcgc ccaaccccgc 1560

ggcccgttcc gcggccccgc accccagttg gtgtcgaccc ccagtcagag ggaccacgga 1620

gctccagggc gggccagggt cccgggggcc ggcagcccgc gccgccgcgc acgccgccca 1680

gctgtgcccg ctcccgcccc caccgtgcca gcctcgcggg gactttccct ttcagtttcg 1740

gggagggtgg gtactgggga cgcgcggggg agggggcgca tcacgggaag ctcctgccgc 1800

ccccagcccc gacccctcgg cgccctccag acctggcggc cctgccaagc gcgatggggg 1860

gtgcgggggc gtgcgggggg gcggcgcgac ctggcggcgg cggtcacggg ccccgtgcct 1920

ccgtaggtct gcgaggaaca gaagtgcgag gaggaggtct tcccgctggc catgaactac 1980

ctggaccgct tcctgtcgct ggagcccgtg aaaaagagcc gcctgcagct gctgggggcc 2040

acttgcatgt tcgtggcctc taagatgaag gagaccatcc ccctgacggc cgagaagctg 2100

tgcatctaca ccgacaactc catccggccc gaggagctgc tggtaaccac tggaccccgc 2160

cgcccccgc cccccgcgag ccgcacgcag gaccacgggg ccggggaagg tgcaggcggt 2220

ggcggccggc ccgcctctga catatctgct cctccgaggg agggcggccc cgccgccggg 2280

cgtccctgtc cggggagcgg gcgggatcct agccgccctc gtcccgccgc cctgtgtgcg 2340

cttgcctgcg actcccaccg cgttcgcgcc ccgcggtgtg gccgaaaagt gggcggcgcg 2400

cgccctccag cggctgcacg aggagcgccg cgctcggcgc tgagcctcca gttccaggtg 2460

gtgggaggtc tttttgtttc cacttgcaga gtcttttcac gcggcgggcg ccttttctgt 2520

tttgatctgg gattgcgtgt tgccccagct cccttgagtc cccagcattc gccagccctc 2580

ccctccaaca tccaggaccg cacgagacgc aggggccagt gctctgagcc ggaggtgcgg 2640

cgtggcccgg cccccgtgct gccggcttcc ccgcgccccc gggctggccc gcacctcccc 2700

tgatggccgc tcaccctgtg ttcgcagcaa atggagctgc tcctggtgaa caagctcaag 2760

tggaacctgg ccgcaatgac cccgcacgat ttcattgaac acttcctctc caaaatgcca 2820

gaggcggagg agaacaaaca gatcatccgc aaacacgcgc agaccttcgt tgccctctgt 2880

gccacaggta gggcaggccc ggcagccccc ggcctcccct tgagagccgg ctccttaggt 2940

gaccctggcc ggcttcttgc tctccacctg ggtgctgtct gggaagatgt ccccagaccc 3000

cctcctgcgc tggagagcgc tcttccagct ctggtgagca gaggccctgg attgtttgtc 3060

gcgctggatg gagggagatt tgctccctca cggccaccat gcagtacctt gggcattggt 3120

gtggacggct cagcctgcct gtgtcccgtt actctggcct cgtccttcag gccaggcagc 3180

ctgtggccac tccatgctga aaggggttta ccttggccac agggccgcct cctttctcca 3240

cccacctcca gcccttcttg tgtccttaag gagcctgagc tgcagaggcc ccctcctggc 3300

ctctcccagg ctgggccacc tgccagaggc gcctccaggg gcggggagag ctgtcggcct 3360

gcctgcacca cgtgctctgg gcagccgagt gcaggggtgt ccagcagagg agctcggctg 3420

cctgaggccc tgccaggggt gccggcagcc agccgggctc agctgagccc tgaggggcg 3480

cttcagagca ctctcagctt gggccgccac cgtgggcagc agaagcaccc agtcctcact 3540

tcccctggca tggccccaga ggcccctccc tgacatggcc ttggccccag aacccagtgg 3600

ggacagactc gcacatacac agggtgccgc ctcctgctgt ccccagccct gcctctgacc 3660

cccctgtgac cgcctccttc cctggcccag gaggcctggt taccttcatg ggggagcatg 3720
gccccatccc acccagctct gctgtggccc acctttggtc aagcctcagt tgtcacatct 3780
gtttgggggc tcactctggg tgacctaggc cacaaggccc acggggcatc aaagaggcag 3840
tagcatcttc tcccctcccc agagggcaga gccccccaag cctacttcag agctcccttc 3900
tgacaccggt agcccgcagc cggtattcca gaatgggttc tggtttaggc gtgaggcctc 3960
ccccacctcc tccacctgct tggggcatga acccctcccc cacgtttcca agcgagtccc 4020
caaggtgggc agatgaagat gccaaggatg tcgaccagtc tggatgggtc tggggtgggg 4080
gggcatgcgg cagacaggga ggcattctct ggctggtgct cctcagagga gagaggcctc 4140
cggagactcc agacagcctt ttatggagct gaaagtggct tcagagaaat gcaaagtttc 4200
ctggagagaa cgtggggcgt ggttcttgca cagcctccct acagggtggc tccagcagtg 4260
gagctcccct cccaggaccc ctgggtgcta gtgggaggca gtgggcaggt gcagattctc 4320
gtccttccca ctactgcaca ccctttgtct gcgaaggcgc ccccagcggt gggtgaagga 4380
ggagggacac ttggggaccc agctgtgcac gtgctctcag tgactgtgga gtccactcca 4440
gggtgggtcc cgagggaggg gcaggagacc aggggaccca cccctgcaaa gtgctccggg 4500
tcctgacccg tggccacccc atggaacgta actgagcagc cagtgccttg ttcctgctgg 4560
acatctgtgg agacaagagt gacttacggc tgcttaaagt cagaaacagg ttgaaggagg 4620
tggaggcgtg ggaaagagtc taggaaggtg tttttgccct ccacgtggca aaggttacat 4680
ttaaaggtga tgctgggtgt tctccctgca ctaggcattc ctggccccag gtccccagca 4740
ggtgtgcaca tgctgcatac actcacgcat gggggtttca gggcaggtgc gcccttggct 4800
ccgtgggagg ccaggtgagg aacgtccagt gccaaggagc ttccgggaca gctgtcactt 4860
ccctttacaa ccaggcagcg gatagggtca aatcctggag ctttggtgtc taattctggg 4920
tggctcctaa tctaagcaca gacagcacca cacactgggg tgggggcacg agcttctgaa 4980
acaacgtggc cccagtgact ccacgctgtg tgtgcccctg gagacggggg ggtgcacaag 5040
gtgcggagcc agctagaacc tgtcgctccc tgcagaagcg gtttctgtgt gcggttctga 5100
tttgcctcaa tgagaaggtt ttcattcatg gctcccggct ctcagactgg gtggaactgc 5160
tcccatttaa aggggaaaag aggtggctcg gctcgttaag gatttctttt tctaagttgt 5220
tacggcgccc agcagccggc tttgtctccc cttcagggtg gctgcctttc ttcccggccc 5280
ctcgccggcg gccctctctt taacaaggcc gaagttgttt attctctcgg gatgaagtct 5340
cggatgggcc gccacacccc tggcggcccg tgggggcccc tctccctttg tgcctgggtc 5400
ggctcccatt cagctccccc gaccccccctt gttcccgggc gctcagtggc gcgagatgag 5460
gcgatggggc cgacaaagat gccacactca tccctgccga cgtccggctc ccagcccagg 5520
gcccctggtt cctgtgcaga attcctcgtg ggtgtgacaa aaggctgccc ccaggctccg 5580

ctggggtggg ggccaggcca agaggcacat cccacactgg cccacctgtc cacggtaggc 5640
gcatgactgc cctgaggagg ggaggccggc attccccgcc acaaaccagg acgtaattgg 5700
tggcagggct ctctgtggaa agagccagtc tgctgtttgt ctaggaggtc agtcacagag 5760
gccccgagac gcccactact gcagcctggc aggcggatga gcccagtatc tggcagtgac 5820
cagagggagt tttgtgcaga ccacaaaggc tgatgggccg ccctagattg gtgtccctct 5880
tggaagtggg cccagatgtg cgggacagtc cccaggaagc cccaggtgag ggcactggtg 5940
ccctcttggg aaagctgctc cctcctgggg cccggctccc ggcccagtcc tccaggggtg 6000
tcccatggtg actggtgcta ggaaccccac acctcttccc ttacttggga agtcactgga 6060
attgttgggc tacatcagac ggcccagaaa agtgtttttg tcatcggcca gaaataggag 6120
agttgtgagt agagggcccg ggtggagttg gggtgtactt ggtctgtgct ctgaaggtca 6180
ctgtgacagt catggtccca tggtaagggg catgggttgc tggaagagct cttccttccc 6240
gagtgagcca agccgggctc tcctggcgcc agggcctgag ccgcagccac accacagccg 6300
ccctgaaggc tgccggccag ggcttacccc tcaagggaca cggaatggct tcatcagtac 6360
cctgcagccc cgtggcctgg cccgggtgga ggcctaggct tcagccatgc gatgtccctt 6420
cagaatatga cttgtctgca atccctgctg ctgggggtg gcaggtactt ggggtgaggg 6480
ttagggtcat agaagcgaca tctctacgtc ctcatatttg cgtcatctaa ttttgttttt 6540
gtgaatacgt gataacattc acaaggctca agatgctaaa aggatgagaa ggcagtgatg 6600
tccccatcac ctgtcctgtg tcttcccgtg gctttctctt tccttggtta tgtttgagtc 6660
aacagtgggg ctgacgttcc aggagggtcc gtgggccagg ctcttgctct ccgagtgccc 6720
agggatggct ggaggctgag gagggcctgg atgtggagcc tcagataccg agtgcttccc 6780
ttcaggccgg gccgcttgct cagagccagc acacagggat gcccggatca cgggggccct 6840
gagagggtcc cctgctcaca gcctccttcc ctctctcctt ctgcctcaga tgtgaagttc 6900
atttccaatc cgccctccat ggtggcagcg gggagcgtgg tggccgcagt gcaaggcctg 6960
aacctgagga gccccaacaa cttcctgtcc tactaccgcc tcacacgctt cctctccaga 7020
gtgatcaagt gtgacccggt aagtgagggt gatgtcccag gcagccttgc cggggcttac 7080
agggggagac acctagtgcc acggaaatgc cgaggctggt gccaaggccc ccaagggtga 7140
caaggttggg gctggggctg ggcccctcgg accccaggcc acagactgac agggcaccgg 7200
cttcttccac tgctcctaga acttactgac tggctgggag gtcctcacag ccttctcacg 7260
tcccctgggg cttccaggag ccgtagagtt tctgggcgaa gcgtccggga cggaggcccc 7320
aggcggcccc agccaatggt ctgtgtggtg atggtgtgtg gggttaggcc caggcgagct 7380
ttgtttgggc cacaatgtgc gtggccaata aatagatgct tgaaaagggc tcctgtgagg 7440

tccgagacac cggacaacgg gcggatagag acagccttgt tgtttacggc ctctttgaga 7500
ggctgctgct gttaaaccct gggatgactg tgtctttctt cttaaaaatg ccattgtttt 7560
attcccgagt cttttcttaa agaaagaatt aaaatgacaa tcaaaagggt ttgtggcatt 7620
taccaaatta gaccagagag gtggccgggt cagccgccgg ccccgcggtg tgtgagggag 7680
tgaccgcctg accccagctt ggggctgggt gggcctgcaa gacccgtttt ggctctggcc 7740
tgggccgcct cttggtggtc tgccctcgag cctcccgggg actccgcacg ggtctcagca 7800
gatgctatct agggtccacc tgcctgtccc ctgcctagtg gtgcctctgt cccggggaca 7860
ctgggagtag cggctgccca gcccatgtgt gtctcggaag aggaagaagc ttttttgccg 7920
tgggacaccg aagttggcag ggcctccct tctgtgttct cggccatggc ctcccttgca 7980
ccctgccccg tgttatcctt tgggggtggt gaggtgtcct cacccgctgt agggtggagg 8040
ccagcagccc gcagctctct caggaaaatg gctcagaaac accatcgagg cctccagaag 8100
cccagcaaag agaaagcccc tccatcaaaa tgaaactcgc gtctgcactt ttcatttcga 8160
actccacgcc ctgagtgaaa accgcttccc cgccaggggt gactgccctg ggatgttgct 8220
gtcttcgggc agttgtggga agttgggcgc tggcccttat ttgagtagag accatcttaa 8280
ctagattgga ggcacacgtc tcacagctga cagacacacg gggtgaagtt acccgaggcg 8340
gagtccactc tgcctgatca gctagtgacc aacgtagctg agcccagact cagaaaaacc 8400
gtccacagca gaggcccctg cattttctag ggcgtgttct agaattttct ttggtgggtg 8460
gaatgtccat ctgtgcaaat cgggtgcgca gtgccacaca ccagtgactt ttcgcggagg 8520
agcgtgctgc ctttttggag cttctggctg tgggagaaca gctttgtcca ccggggtagc 8580
cttgcaggca gctgtggggc cagaggaatg aaggaaggtc ctggagtcta gctgcatgtg 8640
tgaccctgga gtgggtcatg ggcgagggac gggccgcagg tgaagaatcc ctggatggag 8700
ctgccaggcc cctggggctg agaattgaag ctggctggtg ttttaggttg aacgtcagga 8760
gtcttgtatc tcaccccagg cctctggcct cagtttcccc atctgtacag tgggactgtt 8820
tgtgcagcca gcccggccag cttcatttgc catgatgaga atttatctga ggggcgggag 8880
aggaaagccc tccctataaa ggtacaggcg ctaaaatgtc gtgacctcag tggtccacct 8940
aaaagtcgtt ctggcctggg tcatcgcctg tcgtgctatg cctttgtcca gccccttctg 9000
gttgggagtt aagtggcacc tgtgcggcac gtggtggggc tgtggcccag ccctgctcct 9060
tgtggaaggt ctgtttcctg ggctgcctag agacttggct tgaagcccta gcgtggcttc 9120
ctggcagttg ggacacacac agccccaaca catggagccg gttctccatc cagaagcccc 9180
cgggcagtaa gcagccactt caggctgcgt gggacttgcc cgtggtggag cctaggagag 9240
gcccctggct gggcgtggcg ttccagattt cacggctgct ctttcccact gacagtgtgg 9300
tgtggacgct gccaagggag tctggagccc cagagggtgg aggtgcagga cttccaggag 9360

cgtccgtcgc actccacccg agggcgagca cctcagtggc cgcagtgggt ggatgcatgc 9420
tgtgccaggc tgatggctgg ccccggggca caggcctgag cgggagagga tggaggggag 9480
ggatcaatgg tccaggtccc cctggccacc cagcattcat cctcagtcat gcacggccca 9540
aggcttcgac agccattgat catggaaggc caggttcacc tcaagggctg ccacatggag 9600
aggttaagtc tgaaaaggct gaaaaggcag ggttcaaagg gcctcctgtc cagatcagat 9660
ggcactgaat tccccaggga gctggcacgg ccagtgggaa caggcggtga aggcgctgtt 9720
ggacatgggg acgggcaggg ggtgtgcagg gtgggcgggc aagcatctgg tgtcttgtgg 9780
ctccagagac caggtgggag gtggaggcat ttggtcctga gtgtcctgac aggtgatggc 9840
agctcccaca tctcgctcag gttcagagga ggcagcatgg gccgagggac agtttttggc 9900
ttagtcttgc tcttataaag gcttccgggt catggcacct gggaaggggc cctcgctgca 9960
ggccccttct aaggaccccc tcttcccacc tctccccacc ctctctctct caggactgcc 10020
tccgggcctg ccaggagcag atcgaagccc tgctggagtc aagcctgcgc caggcccagc 10080
agaacatgga ccccaaggcc gccgaggagg aggaagagga ggaggaggag gtggacctgg 10140
cttgcacacc caccgacgtg cgggacgtgg acatctgagg gcgccaggca ggcgggcgcc 10200
accgccaccc gcagcgaggg cggagccggc cccaggtgct cccctgacag tccctcctct 10260
ccggagcatt ttgataccag aagggaaagc ttcattctcc ttgttgttgg ttgtttttc 10320
ctttgctctt tccccccttcc atctctgact taagcaaaag aaaaagatta cccaaaaact 10380
gtctttaaaa gagagagaga gaaaaaaaaa atagtatttg cataaccctg agcggtgggg 10440
gaggagggtt gtgctacaga tgatagagga ttttataccc caataatcaa ctcgttttta 10500
tattaatgta cttgtttctc tgttgtaaga ataggcatta acacaaagga ggcgtctcgg 10560
gagaggatta ggttccatcc tttacgtgtt taaaaaaaag cataaaaaca ttttaaaaac 10620
atagaaaaat tcagcaaacc atttttaaag tagaagaggg ttttaggtag aaaaacatat 10680
tcttgtgctt ttcctgataa agcacagctg tagtggggtt ctaggcatct ctgtactttg 10740
cttgctcata tgcatgtagt cactttataa gtcattgtat gttattatat tccgtaggta 10800
gatgtgtaac ctcttcacct tattcatggc tgaagtcacc tcttggttac agtagcgtag 10860
cgtgcccgtg tgcatgtcct ttgcgcctgt gaccaccacc ccaacaaacc atccagtgac 10920
aaaccatcca gtggaggttt gtcgggcacc agccagcgta gcagggtcgg gaaaggccac 10980
ctgtcccact cctacgatac gctactataa agagaagacg aaatagtgac ataatatatt 11040
ctatttttat actcttccta tttttgtagt gacctgttta tgagatgctg gttttctacc 11100
caacggccct gcagccagct cacgtccagg ttcaacccac agctacttgg tttgtgttct 11160
tcttcatatt ctaaaaccat tccatttcca agcactttca gtccaatagg tgtaggaaat 11220

agcgctgttt ttgttgtgtg tgcagggagg gcagttttct aatggaatgg tttgggaata 11280
tccatgtact tgtttgcaag caggactttg aggcaagtgt gggccactgt ggtggcagtg 11340
gaggtggggt gtttgggagg ctgcgtgcca gtcaagaaga aaaaggtttg cattctcaca 11400
ttgccaggat gataagttcc tttccttttc tttaaagaag ttgaagttta ggaatccttt 11460
ggtgccaact ggtgtttgaa agtagggacc tcagaggttt acctagagaa caggtggttt 11520
ttaagggtta tcttagatgt ttcacaccgg aaggttttta aacactaaaa tatataattt 11580
atagttaagg ctaaaaagta tatttattgc agaggatgtt cataaggcca gtatgattta 11640
taaatgcaat ctccccttga tttaaacaca cagatacaca cacacacaca cacacacaca 11700
aaccttctgc ctttgatgtt acagatttaa tacagtttat ttttaaagat agatcctttt 11760
ataggtgaga aaaaaacaat ctggaagaaa aaaaccacac aaagacattg attcagcctg 11820
tttggcgttt cccagagtca tctgattgga caggcatggg tgcaaggaaa attagggtac 11880
tcaacctaag ttcggttccg atgaattctt atcccctgcc ccttccttta aaaaacttag 11940
tgacaaaata gacaatttgc acatcttggc tatgtaattc ttgtaatttt tatttaggaa 12000
gtgttgaagg gaggtggcaa gagtgtggag gctgacgtgt gagggaggac aggcgggagg 12060
aggtgtgagg aggaggctcc cgaggggaag gggcggtgcc cacaccgggg acaggccgca 12120
gctccatttt cttattgcgc tgctaccgtt gacttccagg cacggtttgg aaatattcac 12180
atcgcttctg tgtatctctt tcacattgtt tgctgctatt ggaggatcag tttttttgttt 12240
tacaatgtca tatactgcca tgtactagtt ttagttttct cttagaacat tgtattacag 12300
atgccttttt tgtagttttt ttttttttta tgtgatcaat tttgacttaa tgtgattact 12360
gctctattcc aaaaaggttg ctgtttcaca atacctcatg cttcacttag ccatggtgga 12420
cccagcgggc aggttctgcc tgctttggcg ggcagacacg cgggcgcgat cccacacagg 12480
ctggcggggg ccggccccga ggccgcgtgc gtgagaaccg cgccggtgtc cccagagacc 12540
aggctgtgtc cctcttctct tccctgcgcc tgtgatgctg ggcacttcat ctgatcgggg 12600
gcgtagcatc atagtagttt ttacagctgt gttattcttt gcgtgtagct atggaagttg 12660
cataattatt attattatta ttataacaag tgtgtcttac gtgccaccac ggcgttgtac 12720
ctgtaggact ctcattcggg atgattggaa tagcttctgg aatttgttca agttttgggt 12780
atgtttaatc tgttatgtac tagtgttctg tttgttattg ttttgttaat tacaccataa 12840
tgctaattta aagagactcc aaatctcaat gaagccagct cacagtgctg tgtgccccgg 12900
tcacctagca agctgccgaa ccaaaagaat ttgcaccccg ctgcgggccc acgtggttgg 12960
ggccctgccc tggcagggtc atcctgtgct cggaggccat ctcgggcaca ggcccacccc 13020
gccccacccc tccagaacac ggctcacgct tacctcaacc atcctggctg cggcgtctgt 13080
ctgaaccacg cgggggcctt gagggacgct ttgtctgtcg tgatggggca agggcacaag 13140

tcctggatgt tgtgtgtatc gagaggccaa aggctggtgg caagtgcacg gggcacagcg 13200

gagtctgtcc tgtgacgcgc aagtctgagg gtctgggcgg cgggcggctg ggtctgtgca 13260

tttctggttg caccgcggcg cttcccagca ccaacatgta accggcatgt ttccagcaga 13320

agacaaaaag acaaacatga aagtctagaa ataaaactgg taaaacccca 13370

<210> 15
<211> 20
<212> DNA
<213> Artificial

<220>
<223> Synthesis

<220>
<221> misc_signal
<222> (1)..(20)

<400> 15
ctggaggtct gcgaggaaca 20

<210> 16
<211> 18
<212> DNA
<213> Artificial

<220>
<223> Synthesis

<220>
<221> misc_signal
<222> (1)..(18)

<400> 16
tgcaggcggc tctttttc 18

<210> 17
<211> 3697
<212> DNA
<213> homo sapiens

<400> 17
cccacgcgtc cgggcagcag cgttggcccg gccccgggag cggagagcga ggggaggcgg 60

agacggagga aggtctgagg agcagcttca gtccccgccg agccgccacc gcaggtcgag 120

gacggtcgga ctcccgcggc gggaggagcc tgttcccctg agggtatttg aagtatacca 180

tacaactgtt ttgaaaatcc agcgtggaca atggctactc aagctgattt gatggagttg 240

gacatggcca tggaaccaga cagaaaagcg gctgttagtc actggcagca acagtcttac 300

ctggactctg gaatccattc tggtgccact accacagctc cttctctgag tggtaaaggc 360

aatcctgagg aagaggatgt ggatacctcc caagtcctgt atgagtggga acagggattt 420

tctcagtcct tcactcaaga acaagtagct gatattgatg gacagtatgc aatgactcga 480
gctcagaggg tacgagctgc tatgttccct gagacattag atgagggcat gcagatccca 540
tctacacagt ttgatgctgc tcatcccact aatgtccagc gtttggctga accatcacag 600
atgctgaaac atgcagttgt aaacttgatt aactatcaag atgatgcaga acttgccaca 660
cgtgcaatcc ctgaactgac aaaactgcta aatgacgagg accaggtggt ggttaataag 720
gctgcagtta tggtccatca gctttctaaa aaggaagctt ccagacacgc tatcatgcgt 780
tctcctcaga tggtgtctgc tattgtacgt accatgcaga atacaaatga tgtagaaaca 840
gctcgttgta ccgctgggac cttgcataac ctttcccatc atcgtgaggg cttactggcc 900
atctttaagt ctggaggcat tcctgccctg gtgaaaatgc ttggttcacc agtggattct 960
gtgttgtttt atgccattac aactctccac aaccttttat tacatcaaga aggagctaaa 1020
atggcagtgc gtttagctgg tgggctgcag aaaatggttg ccttgctcaa caaaacaaat 1080
gttaaattct tggctattac gacagactgc cttcaaattt tagcttatgg caaccaagaa 1140
agcaagctca tcatactggc tagtggtgga ccccaagctt tagtaaatat aatgaggacc 1200
tatacttacg aaaaactact gtggaccaca agcagagtgc tgaaggtgct atctgtctgc 1260
tctagtaata agccggctat tgtagaagct ggtggaatgc aagctttagg acttcacctg 1320
acagatccaa gtcaacgtct tgttcagaac tgtctttgga ctctcaggaa tctttcagat 1380
gctgcaacta aacaggaagg gatggaaggt ctccttggga ctcttgttca gcttctgggt 1440
tcagatgata taaatgtggt cacctgtgca gctggaattc tttctaacct cacttgcaat 1500
aattataaga acaagatgat ggtctgccaa gtgggtggta tagaggctct tgtgcgtact 1560
gtccttcggg ctggtgacag ggaagacatc actgagcctg ccatctgtgc tcttcgtcat 1620
ctgaccagcc gacaccaaga agcagagatg gcccagaatg cagttcgcct tcactatgga 1680
ctaccagttg tggttaagct cttacaccca ccatcccact ggcctctgat aaaggctact 1740
gttggattga ttcgaaatct tgccctttgt cccgcaaatc atgcaccttt gcgtgagcag 1800
ggtgccattc cacgactagt tcagttgctt gttcgtgcac atcaggatac ccagcgccgt 1860
acgtccatgg gtgggacaca gcagcaattt gtggaggggg tccgcatgga agaaatagtt 1920
gaaggttgta ccggagccct tcacatccta gctcgggatg ttcacaaccg aattgttatc 1980
agaggactaa ataccattcc attgtttgtg cagctgcttt attctcccat tgaaaacatc 2040
caaagagtag ctgcaggggt cctctgtgaa cttgctcagg acaaggaagc tgcagaagct 2100
attgaagctg agggagccac agctcctctg acagagttac ttcactctag gaatgaaggt 2160
gtggcgacat atgcagctgc tgttttgttc cgaatgtctg aggacaagcc acaagattac 2220
aagaaacggc tttcagttga gctgaccagc tctctcttca gaacagagcc aatggcttgg 2280

aatgagactg ctgatcttgg acttgatatt ggtgcccagg gagaacccct tggatatcgc 2340

caggatgatc ctagctatcg ttcttttcac tctggtggat atggccagga tgccttgggt 2400

atggacccca tgatggaaca tgagatgggt ggccaccacc ctggtgctga ctatccagtt 2460

gatgggctgc cagatctggg gcatgcccag gacctcatgg atgggctgcc tccaggtgac 2520

agcaatcagc tggcctggtt tgatactgac ctgtaaatca tcctttaggt aagaagtttt 2580

aaaaagccag tttgggtaaa atacttttac tctgcctaca gaacttcaga aagacttggt 2640

tggtagggtg ggagtggttt aggctatttg taaatctgcc acaaaaacag gtatatactt 2700

tgaaaggaga tgtcttggaa cattggaatg ttctcagatt tctggttgtt atgtgatcat 2760

gtgtggaagt tattaacttt aatgtttttt gccacagctt ttgcaactta atactcaaat 2820

gagtaacatt tgctgtttta aacattaata gcagcctttc tctctttata cagctgtatt 2880

gtctgaactt gcattgtgat tggcctgtag agttgctgag agggctcgag gggtgggctg 2940

gtatctcaga aagtgcctga cacactaacc aagctgagtt tcctatggga acaattgaag 3000

taaacttttt gttctggtcc tttttggtcg aggagtaaca atacaaatgg attttgggag 3060

tgactcaaga agtgaagaat gcacaagaat ggatcacaag atggaattta gcaaacccta 3120

gccttgcttg ttaaaatttt tttttttttt ttttaagaat atctgtaatg gtactgactt 3180

tgcttgcttt gaagtagctc tttttttttt tttttttttt ttttttttgc agtaactgtt 3240

ttttaagtct ctcgtagtgt taagttatag tgaatactgc tacagcaatt tctaattttt 3300

aagaattgag taatggtgta gaacactaat taattcataa tcactctaat taattgtaat 3360

ctgaataaag tgtaacaatt gtgtagcctt tttgtataaa atagacaaat agaaaatggt 3420

ccaattagtt tcctttttaa tatgcttaaa ataagcaggt ggatctattt catgtttttg 3480

atcaaaaact atttgggata tgtatgggta gggtaaatca gtaagaggtg ttatttggaa 3540

ccttgttttg gacagtttac cagttgcctt ttatcccaaa gttgttgtaa cctgctgtga 3600

tacgatgctt caagagaaaa tgcggttata aaaaatggtt cagaattaaa cttttaattc 3660

attcaaaaaa aaaaaaaaaa aaaaaaaaaa aaaaaaa 3697

<210> 18
<211> 40935
<212> DNA
<213> homo sapiens

<400> 18
ctctcggtct gtggcagcag cgttggcccg gccccgggag cggagagcga ggggaggcgg 60

agacggagga aggtctgagg agcagcttca gtccccgccg agccgccacc gcaggtcgag 120

gacggtcgga ctcccgcggc gggaggagcc tgttcccctg aggtgcttgg gcgctccttt 180

ccttatcctt ccggggctgc tcccgcttcc tctcggagcc aaacttcgta gcaggcgcgc 240

ggtccgggcg gcgggctggg cgcagccggg aggcctgggg ttgggagcgg ggagctcagg 300
tgggggacgg tgagggtggg ccgcgcccgg ggcgcggagg gcggcggccg ggcccgggtt 360
ccggtcgcgc tgcctctctg gggccctggg ggcatcgctt gcggggaggg ggcgccgcgg 420
gggcgcgtac aggagcccgg atggcaggcg gggtgggggt gggggtgggg gtctgtggtt 480
tccgtccggg gctctggcct tggccgagtt tgggggaggg acccggtgcc tcgggatgcg 540
ccgggccctg ggtgggggc ggggtgggga cggggggctc cgccttctca gctcttgcgg 600
cgagttgggg ttcgggcgct gaggcagaga cgccacccta agtcccatca gtcctgggga 660
tcggaccagt ggactttctc ttaagatttc ctctttcatt cttaagaata gaagtgttat 720
tattttttt aatgccctgg ctatgtgagt ttgaatcgaa gcaactttaa accttagagc 780
aactaaactc taagtgcagc gggtgcgatg cgtcagtagg gtgagcacat aaaaaatcca 840
tgtcttgcac ctgtatttta gcgtactatg caggtgagtg aaagcagtgg ataatgtact 900
gggagtctta tggatttatg gtagtgggta tgagaccctg gtgaaataag ggggtggagg 960
aaggcgaagg tgatggctta ctgtttctta ccaagtgaac tgcaggattc agcctctgac 1020
tcagaccgct tcgagaattt tgttcgtaga aataatttaa atttattcaa atagtttgat 1080
ggcagctaaa attgaattat agagcacgtt ttcttttcag cggagtgaat ttttccttcg 1140
ctccaaagct ggccaaatgg aattcaagca ttgcaacttc tttcagtgtt ttgtctggag 1200
agaggacttt gaaccgagac ttttcgaagt taagttccta tagcctgctt ctgaatctgc 1260
caagcttgaa agctttggca gttgggtgta tgtagttgtt gccttcgttc tcttcccttt 1320
tggagggagc gttgtctcct actttgtatc ttccagacat ctgtggtctt ccccccaccc 1380
ctcgagtttg tgagtggtga atgaagaaag actaggctgc tggtatgcag aggtcggcaa 1440
aaggaaatcg aggagtggtt ttagtgaaat gagagctttg tatcatgaat aatggtggct 1500
taggctagac atcaacttga agagacggca gcatttcctt tcataaagtc taggctaatg 1560
tttttcagat cgctaagttg tagtttgtct ggaatttagg aagccatttc agtatttgtc 1620
acttggtgaa cgaacattca ataccttcag atgtcttcgt gttgacttgt attcatccta 1680
agaaatagta aatatagtct caagtgttat ttatgttata ctgctggttt attctctgct 1740
taaattattg acataaattt ctactttgga ggcttttcgt ttgaactaag gctgtgcgga 1800
atttatttta cttttatatt taaatctttg aaaaatctct gattaaaaaa aaagtaccct 1860
taaaggtttg aggatgtcct ttcacaccag acaaaatttg gttaatttgc gcccaatatt 1920
cattactttg acctaacctt tgttctgaag gccgtgtaca aggacaaggc cctgagatta 1980
ttgcaacagt aacttgaaaa actttcagaa gtctattctg taggattaaa ggaatgctga 2040
gactattcaa gtttgaagtc ctgggggtgg ggaaaaataa aaaacctgtg ctagaaagct 2100
tagtatagca tgtaacttta gagtcctgtg gagtcctgag tctcccacag accagaacag 2160

tcatttaaaa gttttcagga aaaaccaact taaaaaaaaa taaggtggct aattaaaaaa 2220

aaatgaagca tttaacagtg ttcaggtttc agagtatgga agaggggttt tttaaactgt 2280

tatctgatta tttcttttac caacatgata tagaaaagtg tatttccagt attaaaattt 2340

atcagactga gcttactgtt cctgttaatg actggaataa aaattggcat aaatgagggt 2400

ctgtatgctt gttttaataa caccaccacc aagatagaaa acgaggaggc aagtttctcc 2460

aagggtattt tgaaatgtgt tagcaaaact attgcagata ctcgtttttg ttatagggtg 2520

aggtggggag aggcgcatgc taagtattgt tgaaactagg gatgtagaga attaaaagtt 2580

tgaatataat tattttgtag ttataagtag cagtgaaatt aaatctcctg caatagacta 2640

tagaagtata tttagccaaa tgaaacttca gtgttattga aatgaaataa tacatctgtc 2700

ctgttacaag attattttta tttctcttgt ggtttcctag cttctgataa tcaataattg 2760

tagatgagta ggtggtaagt tttaagtttg tactttgagc ttagtcggaa gcatgcttga 2820

ctgccaaccc ggggcacaaa ggatgaaggc ttttagaact ggacaaactt ctaacaaaag 2880

gtatttgcaa ctcttttgta gtgtgtcatg ttgatttgtg acattgtttt tgaaaatatg 2940

tgttaactta gttttcttgt agccctcttt ttattggaac tgtggtatct attgttgaaa 3000

ctgcttgact gagaacattt ttataccata aaagtaaata gtaaacatag cccaggagcg 3060

gcttctggtt tgtccatcgt atgtagccat tgcctccttg tactctcatt gagaagatac 3120

tgatttgcag attcagttgt ccttctctaa cagactattt atgtaatatt gcagttgtga 3180

ttgtgatagg taagtggacc agtcggttaa aataaatact caggtttcac aaaaggaaaa 3240

taatatgatt tgtgttgatc taaatgagta taggagttaa ctcctatagt ttttcatcac 3300

ttaaactcag ggaaagttc tttatttcct ctgtttactt aagaatgctg cttttgtgtt 3360

tcatgcaaga ctgagcttga ctcagtttga aacctaggct catctgttga ggcctgaacc 3420

ctgctgtcct tgaagtatgc atataatttg cttccttcct aaggaaaaat aagctcttga 3480

aagataaagt caatcacatt aggaacccat ttttagggtt tagccacttt tttttttttt 3540

ttttttaac tcatgggcat ctcttctgtt aagagacatt ccccactctc caagtttccc 3600

tcaagcctga agcagcagag tgagtagtgt tggagcatgt tttcattgca tgcttgggtc 3660

atgttgagtg ccctccagtg gatatagtat aatgcttgtg attttttttt ttttaattcc 3720

aaacaagttt atgtgggata tatttaggaa tagttctgat gagggagaat caactaagaa 3780

acctttgatt tctaaaataa ttaatatcat tactgctaat taaaatacag gcttgagaaa 3840

atgtcttctc agccaatatt tgcagtagaa aagtcgggag gtttttaag gtcactttga 3900

gtaggcagtt ctgcttaaat atatcataat gataaaccag aatctcagta tagtacttta 3960

ggaggtaaaa gatcataata ttcagttata ttgatgaatt acagcaactg aaattctcag 4020

aaaaaaatta atgaaaatgt gaattgtcaa tttgtctaaa atcattcaca gagtaaaaca 4080
taagtgctca acttgattat attaggaaat agatagaaat aaaggtaatt gagccagtgt 4140
atgtgaccta aaatataatg cccttagtga ccatagggtt ggtctcattt gtacatagtg 4200
gtgggccatg atgaactgtg ttttgccctt tgaatttttc cttaaaaagc tttctctagg 4260
ctcctatgtt catggttttt ctgttagtaa tattattttc tgaaaatcca tgtttcaaat 4320
cagaatctaa ttagcaacag gaatgaagct tattctaaat tagtttttgg aagttaaacg 4380
gtcagcatat ggaaattttt cagggtttag atttttaaaa atttgttttt cagaatatgt 4440
tgctggaatg aaaacgttag cgtagggacg gaaaatgaca cttaccagtg attgctttac 4500
tttgcctgtg gaattcagtg taattttgtg gaaacattgg tatatgattt tttactactt 4560
aagaaatgta ttgctatagt tagggttttt ttttttttaa aggcaagaat gcctcaagtg 4620
ctttatgtga atgattattt caggatggat taaatattcc tccatcaagg accatacttg 4680
taaatcagtg atttccaagt tggtgcttag tatttacagc atttactgtc tataagcttc 4740
tgttctgatt tttcaagagt tttctgagaa atgagagtag gcttaaaagt tctttgaaaa 4800
attatgtaca tacaacttac tgaaaaaaat tgctaccggg gacttaattt gtctcttgaa 4860
atgggctact tgccttcatt aatgtagcat actacaattt gatgttcaag atatgttact 4920
aagaataaga tcgctttcag aagccttata taggattggt cttactacat tgtagtggga 4980
atggctactc aaatgtctcc agggccagtt aggtattggg taaatgggac catgcagact 5040
attaaaaatt gaagtgcaca tgaagcagcc agtcataagc agctccagcc actgtgtggg 5100
aatatagttt atgttgccag atcatctgat ttctttcccc taagtgggaa atccagatca 5160
atgtacatct cttgatttgc aagtgttggt gaacaaaatt catattttaa gatgctgtat 5220
tcagcacaaa ttaaatacac ttatttgctg aatactgcca gtttgtccct ctgcagtagt 5280
accatttgaa gtacagtgtt ttcataatga ttctgtgaaa tgactggttc tgtgaatgta 5340
cataatttag cagataacat tgttaaatta ttaggtttgt atttatttag gcacttggga 5400
aatgccttgt gtcaattgat tatagattag gagcttaaaa gcaagattta tattatcaac 5460
ttatttgtga agactgggaa acccacattt ttaaagttag gaattaagat ggccaggttc 5520
aaggaaaagg gggagaagta actttcttat tactcaacca tcttaaatag agttctttaa 5580
gtgtattttt aagaggtctc aaaacttaat ctgaagggac gtcaaatgct ggacaaattc 5640
tgtgtataca actcaagtca gcccccaatt ttactggtct ttaaatcatg tcctttttac 5700
cagaagtttg catttctaag ctaaactatt actgttagac tagatccaaa acttaaaaac 5760
agtttaggta attaaaaatt aattgaatat aaacgtttta cttaaattaa tggcaaatgg 5820
ctttttggcc aatttaagtt tatgtaggca gttaaatcga ttttggttaa atcttttgct 5880
gctaacaagg tatttccaga ttttgaaaag tggggtggcc tggtgcctgt agtaccagca 5940

ctttgggagg ctggggaggg tggatcacct gaggtcagga gttcgagact agcctggccg 6000
acgtggtgaa tacaaaaatt agccaggcat ggtggcaggt gcctgtgatc ccagctgctt 6060
ggaagtctga agcatgagaa ttgcttgaac ctgggaagcg gaggttgcag tgagctgaga 6120
tcacgccact gcactccagc tggggcaaca gagcgagact ccatctcaag aaagaaaagt 6180
ggggtgttta gtcttcaaac tccgtgttta agtgactgga gtgaaaatgt aaatcatagg 6240
ccggtgttgg tttaaaaagc atcatctgaa aataatgctg tagtctgcaa ttatttttat 6300
tacgatacga tggtgtaaaa tacaagcaga tcagtgaacc attcatgaaa cattaatcct 6360
aaaggcgtct caccccaagt ctatcccaca atctccatga gacttcgtgg aaccactgta 6420
aagtttcttg tgtaatatcc cagaagtttc ctacctctgg tatcttttga acttgttgaa 6480
aaggcttttc cacccccctct ttatgatggt ttgaagagtg tgaacatctg aatgatgctg 6540
gggtgaaact gcttcataac acttccattt tctcccctat ttatttccat atttttattt 6600
tttcactaat atccccacgg ttttacttct gttttagtaa ttcacatgtt gctggactaa 6660
ttctttttaa ctgacttgta acagatatgt taaaccgttt aaaacttggg gggtattttt 6720
aacctacttt aagttagttc aagttaatca gtctacatgg catataaacc ttatgattaa 6780
taaatcttaa atgctggtag ctgagttgga agccaaagac gtacaaaaaa gctgaagtgt 6840
taggtttagt gtgataagct tctcttacta acagggtttt gtaatagcag aaatagatat 6900
atgcatatat atgtgcatat atatagcata ccttattgga tgtccatata aaaatgtgta 6960
agaagttaaa tttactgcaa aatttcttgg gagtgcaatt tgaagatgat cttaagtggt 7020
gatagtagtt tgctacactg gggatagtt gttgcaaact gctcctaatt ttcctttact 7080
gtgaagtaaa ctgaacagct gtaataggga ttaggaactg tactccctct ctctcttttt 7140
taagtataat taagtggttt tggggtaagg gtgtagggag tgagtgtctt tgaagttttg 7200
catatactag atgaatgcca catgtataag ggaggaacaa gggattcttg gaaatatttt 7260
tcaatccaag taactttgga ggcttccaag tggagttcat tcccctgtgt aggaaagtgc 7320
tggggtagac ccttaaattc ctttctgagc cattgaaaga atgtcctcaa acttcgctta 7380
tactttatag ttcatttaga tacaaaagtt acaaactgaa tgctatttag gaaacgtaat 7440
acactgacat accgctcttt aaatagatta taaatttagt atatcaattt tctggcattt 7500
tgctgaattt tattgtttag ttttcaagcc caactatctt gttactttgt atatcgtagt 7560
tgtcccccgt tgatcactgt ttcctgctta attgtgctgt cgtttttcct gggtcctgat 7620
tcagagtgtc agcattctgt tccccataga ataagaagag gctagaaagt ttacagatga 7680
gatatctagg aatgccagaa gatcaggggt caccgttgag gcagagtaat taattatggt 7740
taaaatggtg ttgctgataa gtgggtgctg ggaaataatt aaaatttgat tttttagaag 7800

aatacttctc atgcttgaag agcgccctca ttatatgcta aagggcctca ggtttttcct 7860
tattgccatt atgctgcaga ttctattaca tttgtctgaa aagatctaag acagaagggc 7920
tgtttaatac cttccctttt ctcctgaact tcccctctcc tctcccccat caggagctaa 7980
gtaggaaccc cttcaccttg ttaccatcag atttcatcaa tggtctgtct ttacaatgaa 8040
ggaagtagta ctgcattctg ggcagaggcc agtcctgagg catgcctttt caaggacatt 8100
gttactttag ttacactggc tcttctgttt taactcttat cccccagact ctaatcctgt 8160
tgcttttttt ggtccccatc tcccaccttt catcatctga aatccattca ttgtaacttc 8220
tggaactcag tcgttagaaa atcctttata ttctcaatct tgtgaatgtt cctttctttc 8280
ttattccagc tgtaacctag ccttctcccc aagaatgcta cttcccttgc agctctctca 8340
agtggtgaat ttttcccttc ttgcacacct tataacactg aactaggagg tgtgtggact 8400
aaatgtctgc ttttgttcct tattgtcact tcttgacctt tattttccaa aacttcaagc 8460
tttgactttc atgtgatcaa attataccac ccactgcctg tctttatttc aagcacctgc 8520
aaaccttcct gggtcattca catccttctt tgttcacttc attagctctt ggctcattgt 8580
cactgtctct tatttctgtc ataattcttg gtgacatcag tatctatgta gagcaatact 8640
agtgaagatg tggtctggta actgttacct gtatgaatta agataaggag ttatgccaga 8700
atataagtca cctgtgtcac taagtttact gtttagctta ctttttttgt agcaagattt 8760
tgatgaagga cgcaatatgt tgatttacag tctggtacaa attttgatgt agaagatgct 8820
tccaatatcc tggtctctta gttccttgat ttcttctcca gtgatcttat tttctaccct 8880
aactcaacta catattccca ttgtcatatc ctagaatatt ttgtctttta tctgtaactc 8940
tgctctcttc ccccaatctc atttcaagca tcccactttc taattcctct agtaaatacg 9000
tcagttccaa cagcccatca atcccattgg gacctacagt ttatctatcc aagcttttcc 9060
ctgttcctca ccctcacttc tatacagctg aagtttcata ctgaattata atcactttct 9120
cgtatacacg tttaacaatc ttgtccctcc ctggcttcat gcccagtgat ctcttgtatc 9180
tatgaccatg tcctttatct tctcctctgt cactggatga actgtagcct tccaagataa 9240
ggccactcag ttcatttgta cagcagattc catcccctct tgctctcaag aatattactg 9300
tggtatctct cttttcttgt ctctactggc tctttccatg agcaaacatg gtattatccc 9360
attacaaaaa aaatttttc tccgtctctc cttccactca ccacctcagt ctctgcttct 9420
ctttcccgca aaataacctt gaaaaattgc tttatgtact cccgttttct tttgaacccc 9480
tgccagtgac caccacgtta taaatttgta gttgtcatct cacttaatct gttagtagta 9540
tttggcacca ttgctacagt tgcttgaaat gccttttcat tggtttccag gccaccatgt 9600
ctgttagcag cttttcctct tacttcacta gcatttcctt ctttgttttt tctgttatct 9660
ttctgacctc tgttggagtg gctgaaggtt tagtccttga atcttttttt gttgtgcata 9720

tttactccag tatcatagct ttatacagat ggtatttaca tctgtttgct aacgatttcc 9780
aaattggtat ccttaaactg gtatccagct attttttggt cagcattttg gatgtctaag 9840
aagcttctca aactaaactg acctcccggt tttccccaaa gctgcatctt agtcttttcc 9900
gaaatgcaat tctgtctttc cagttaccta gcttaaaagc ttgcagttct tgactcatct 9960
ttctctcata ccacgtatct gaattctctc tgcaaaaaat tgtctgttct cccttcagaa 10020
taaagtcacg tgtcatttta tgatggggat acattcagaa atgcgtcatt aggagataat 10080
catggttgtg tgaacatcag agtatacata gacaaaccta gatggtatag cctactacac 10140
atctaggcta tatggtgtgg ccaattacta tgatgaatac tgtaggtaat tgtaacataa 10200
aggtaggtat ttttatctaa acgtattgaa acatagaaaa agtacagtaa aaaatatggt 10260
atcaaaaata aaaaatggta caactgtata aggcagttgt gatgaatgga gcttgcagga 10320
tatgttgctc tgggtgagtc agcgagtgac gattgaggga acgtgaaaga tgtgggacat 10380
cactgtacac tactgtagac tttataaaca ctgtacactt gggctacact acatttttgt 10440
aaggttttaa aagacttttt tctataataa accttaaatt actgtcactt ttttacttta 10500
tgaattctta atttttttaaa cgttttcact cttgtaataa cacgtagctt aaaacataca 10560
ttgtacagct gtacaaaaat tttctttata tctttataag ctttttttata tttttaaaat 10620
tactttttac cttttagctt ttttgttgaa aaactaagac atgggccagg cgcggtggct 10680
cacgcctgta atcccagcac tttgggaggc tgaggcaggc ggatcacgag gtcaggagat 10740
aagagaccat cctggctaac atggtgaaac cccgtctcta ctaaaaatac aaaaaattag 10800
ccgggcgtgg tggcgggcac ctgtagtccg agctacttgg gaggctgagg caggagaatg 10860
gcgtgaaccc aggaggcgga gtttgcagtg agccgagata gcgccactgc actccagtct 10920
gggcgacaga gcggaaactc cgtctcaaaa aaaaacaaac aaaaaactaa gacatgaaca 10980
cattagccta ggcctacaga gggtcaggat catcagtatc actgtatttc catctccaca 11040
tcttgtcctt ctggaatgtc ttcagaggca gtaaacataa atggagctgc cacctcctgt 11100
gataacagtg ccttctggaa tacctcttga aggacctacc tgtggctgtt ttatagttaa 11160
cttttttttt ttaagaagta acagaaggag tacactctaa tgataaaaag tatagtaagt 11220
acataaacct gtaacaatca ttatcattat caagtgtcat gtactggaca taactgtata 11280
tgctatactt tttttttttg agatggcatc tcactctgtc acccaggctg gagtgcagtg 11340
gtgcgaggat agctcactgt aacctcagac tcctgggctc aagtgatcct cctacctcag 11400
cctcccaagt agctgggact acaccaggca ccccaccatg cctggctaat taaaaaaaat 11460
tttttgtaga gacagggtct cactctgttg ccagggctgg ccttgaattc ctggcatcaa 11520
gtaatcctcc cactttggcc tcacaaagtg cgaggattac aggtaagagc caccatgtct 11580

ggcccactgt acttttatac aactgaagca cagtaaacct actgtggttt cgtttacacc 11640
agcatcacca caaacaccat gagtagaaca ttgtgctgcg acgttaacga tggctacaac 11700
atcactaggt gataggaatt tttcagctcc attataatct tatgagacca ctgttgtatg 11760
tgcagttcat catccactga aatgtcctta tgtgatgcat gtcttcatat ccaaaaatat 11820
taatcatttc tcactgaagc catgccatgc catgccatct tttgcctgta ttattatttt 11880
tcagctttta ttttagattc agggtgtaca tgtgcaggtt tgttagaaag agtatatcgt 11940
atgatgctga agtttgggat acagttgaac cagtcaccca ggtagtgagc atagtactca 12000
atagataacg ttctaacatt actcctcctt ccctccctgt tcttgtctct gtctattgta 12060
tctttatgtc catgtgtacc aaatgtttag ctcattcttg tgagaacatg tggcatttga 12120
ttttgtttct gtgttaattt gcttacaata aatagtctcc agctgcatcc acattgctac 12180
aaaggacatg attttgttct tttttatagg ctgcatcata ttccatggtg tataggtacc 12240
acattttctt gatccagtct accgttcatg ggcatttggg ttgattgtat ctttgctatt 12300
atggatggct tttgcctata ttattggaaa ggccttctaa ctggtgtccc tgcttacacc 12360
gttttccccc ttaaatgtgt tttcaacatg gtagccagag taaccctttt tataacaata 12420
aatcgtgtaa ctttttttgtt cagaaactta cagggcttac catttcattc agtaaaagct 12480
caagctcctg tatagtcaga ccatatcctt catcacctgt tacttttctc ctctgactct 12540
tcagcctttt tgtttttcct caaactgatg aagccttcat ggctgatgtc agatgttttg 12600
cccattgaga tcttccttgt tgactcagtt gcacttggtc atatgatttt catttatttg 12660
gggtatctaa tcataatctg aaagttggct acttattttt accccttttga gggtccttgc 12720
cctgtttttg tatccctgat agcgggacag ccagatatct ggaacttaca ggtgttcaat 12780
aaagttttgt tgaatgaata ttctggaatc acccaacctt ttttttcccc tccacttatt 12840
tttcttctcc ctttcacggc ctgaaagatg tcctatgtat atggttccac ttatcactct 12900
catcccagtt tgtgatatac tattccatta tattactatt attaatacaa ttccattgaa 12960
cttgctcttg ctgacttcac cactggacct acatgttggc caaatggata ctttataatt 13020
ttagtcttga cccctgcctt tggcacattt cttacctcta gcacagcact gtccagtaat 13080
ccacactttc tgagacagtg gaaatgttca gtatctgtgc tgttcagttg gtagcaacca 13140
gctacccatg cctattaaac atttgaaatg tggctgtgtg actagtggca attatgttgg 13200
agagtacagt tttagaaact cctgtttttc ttacatggca ctacatttag tatcacaatc 13260
taattgtgca agccagatag gtaggagtca tctttattcc tgttatttaa tttttctcat 13320
ctactatatc cagttcatca catcaacagc gcctgttgtt tctacctcct aaatatttct 13380
ttagtctaac tactacttgt ccctagtgcc accaccatct atcagctgga atattgctat 13440
agctgcctta caggtttccc ttctttcctg ttctcttcta gtttttgaa ttttagtcag 13500

cacgagattt taaaaactca aataagattg tgttattcac ctgcttaaaa cctttcatga 13560
ctttcagtgt cacgtagaac agaaaacact tttcttacca aaggctagag agctctacgt 13620
gatctggcta tttttaacgt ttcattgcac tcaccctttt cctctataat caaactactc 13680
tgatctcaag ggttagttct tgaaagatga tcatgttctt taatgacttt aggtttttgt 13740
gtgttatttt ctatttctgg gatgtttatt ctctgttcct tacatgctgg cccttttgca 13800
tccttcttca ggtctcagct tacatgttac cttcaagaag cctttgacca ctctaagtgg 13860
gcccttcctt ccacttctgc tgtgtaatcc cactcccttc tcccacttgt taattagtta 13920
catacttttt tgtaattgtt tatttggttg ctgtctccct ctcaagaatg cagggaccat 13980
gtctgcattc tgcagtaatc actactgcac acccagaatc tattacagat cctggcatgt 14040
agctgatgca taaatatttg ttgaatgaaa gtctgtacat tgtatttatg ctattggtat 14100
tgctatgacc tgaaactaaa aggagttgtg gaaaagattt cttatggaac agaaatatcc 14160
cttttgatta atatcacaat ctcgtaaatt gagaaaacaa aaaaatatat actactggag 14220
cattcatgta tagttggaga ttatgactca tttattggtg tgtttttgga ctcagaacaa 14280
agatgaggga atattcctta aagctctgta ttgaaataac gaaaagcagt cacattttaa 14340
taatagaagc ttcctagctt actctttctg taatcttctt ttcctaaatg taagagagcc 14400
tcataattat gaggcttatt actagagtaa ggctgtcaaa ggcagcaaaa tgtctttctg 14460
tttggaagaa taacataaac ttgacatgta tggtggggga cagaaggttt caaaagttta 14520
agaatctgtg ttgtcttaac aaatagatgc ttctcaagga gcttacgcta gtggttactc 14580
tgtccagtca gggtttttc ttctttaact tgggttcatt tcctgatggc acacatgaag 14640
tttggatcat atggtttgac tttagctatg gtccttagct atggggagca gcatcagcga 14700
cctgtgacat gtaaattaaa aatacaatgc cagggccctt ccccagcccc tctgatagag 14760
aacctcttgg ccatctgtat ttttagatgt tccaggttag tctgattaac acccttggtt 14820
aagaaccatt gggaggatct gattgccagt ttaaggggac cttcaagcct gtaggtcttt 14880
atagttaaaa aaaaaaaaag attttaaaaa tcatgcatat gttgtggctg aattctggtt 14940
tagcacatac tgcttttaat ggcctgaaat gtttttccca aataaattgt cttgttatag 15000
ctttcatgtg tgatttggtc cagcttcttg ttttgaagat acttacgggg gggaacactt 15060
tgtgatttct cttagtaaca tattaaccca cttaaaaacc ctttctatta caggtcttca 15120
catttaggct taatgtgctt aattcaaatg taaaaataca cctgcctttg ttctcagtga 15180
aagtatgtaa taaataaatg aggggttggc aaactactgc ccaccatctg tttttttatg 15240
gcctatgaac taagaatcgt tttggatagc taaaaaaaaa aatcaaaagg ataattattt 15300
tgtgacgtga aaattatatg aaattcaaat ttcagtttct gtgaatgaag ttttaatgga 15360

acacagccat ccatgcttat gtaagtgtgc atattctctg gctgttttca ctgcaatagc 15420
agagttgagt agttgtgaca aagagtttat ggcccacaaa acctaaaata tttactttct 15480
gatgctttac agaaaaagtt tcctgaacct tattctagct atatgttgtt cataaatgaa 15540
tctttcgtgg ttctgaaggc atttaagaat ctcttaggtt ataaattggc tgggcgcagt 15600
ggctcacgcc tgtaatccca gcactttggg aggccgaggc tggtggatca cgagggcagg 15660
agttcaagat cagcctagcc aagatggtga aaccctgtct ccattaaaaa aaaaaaaaaa 15720
aaaaaaaaaa tagctggggt tggtggtggg cagtaatccc agctactcgg gaggctgagg 15780
cagagaattg cttaaaccca ggaggcggag gatgcagtga gccaagatcg cgccactgca 15840
ctccagcctg ggcaacagag tgaaacacca tctcaaaaaa aaaaaaaaaa aaaaaacact 15900
cttaggttat aaataattgt tgttagctct ccaagcctcc atattacatt ttgtgtgttc 15960
tcctgttcac attttgagca ttttattttt tattagcaca ttcagttcat caggtattta 16020
agagcttaat atatgccaaa gcatatatta agcgagaagc tgtttctaaa tgtactgtct 16080
cagccctcac agagttcact tcattaggct ctttaaaatt tctttcttta aaaggtcagc 16140
gtgctggtat agtggggaag ggaaactctt acaacacgtc gagtagagga aggttatcat 16200
tatgggatat aatttggaag tcattgagta cctgccatta attctgcctg tagtctgaat 16260
gtagagatta acatgtagaa actttttga aataaaatct tcaatttctt tggcatatct 16320
agtactgtct agctaggcat atagtcaaag tatggtgtat atttcaagta ttaaaagttt 16380
ttttgggctg tagtcactgt tgaaaggata tagttcttta ctattacatg tgataccttt 16440
atataaaatt ggctaacccc tgtctttcat ttatctgcaa cactgactgt taccagttgt 16500
ctctaacttt ggtatggggg gtggaaatat gattagattg aaagggtaca tgactgagcc 16560
acaagcagac ctggatttga attttaactg aacggtttat tagctattct tacattaata 16620
ctgctaatca gttttcttgt gatatgagga atgatgtctt ctttatgagg ttgctaggaa 16680
gattcaatga gataacatac taggctcaga actgaagttg ctaggaattt aattatgcta 16740
ccttgttaaa gtatgtcaaa ggcagaattc agtgtttagc tgataccaca aggcagtatc 16800
ctaaaattat gctgtaaaag atataaagat gctgtaagtg actcagaaac ctagtgactt 16860
tgtaatgcag ttgattctta gaatactgtc actttaacag aataggagct aggaatgaag 16920
aaatagttat taaattacta aaatagaaaa tttattgaca catgtaaagt gacatttgct 16980
taaatattga aaaatttgta gtactatttc cttgctttag aaaacattgg ttaccacttt 17040
ttttatttat agcagtttgt ttttgccttg aggcaagatg gttgactgag tagttgccac 17100
atttcttttg tacaaagtcc atttcatagg ccatctagct tttatgctta gaaacatttc 17160
cttaacgtta tatttcagta tttggctaac ctatataggg ttaaattata taggctaact 17220
tctcggacag atatttctaa taatttatgt atttggttct gcaaatgtat gcaaaaatat 17280

atgtacaaag gtatgcagat gccttgcata cttgatatat gttaaatttt ttttaatgta 17340
gaccttttc gttctcttta atgactatat ggtattccac catccccgc tcacctggac 17400
aactacagta acctcctaaa tggtgtttct actttgctat tgcccttat tgtcttttt 17460
cccctttata gctgctggag tgaattttag aaagcctaag tcatacatca cattgcttca 17520
tgggcatccc agtacacttt ggattttatt ttacatcctt actgatctga ttctcatctc 17580
tgtctcttca tggttctctg ccttctagtt acactggtga cctttcaaaa cctttaccac 17640
attgagttca ttccttactt ttcactcttt ctctgcctgg agtgttctgc cccatcttta 17700
cgtggccagc tgctcctcct ctgatgaaat gtctcttcct cacaggcctt ccctgaccac 17760
ccactagagt agcacatctt ctacctcata aacttgttta ttagtatttc ttactctaaa 17820
ttttctttta aattgcttaa ttccctaaca gtagaatata agcttcactg tatgtatgat 17880
cttgttgact ctcttactca ttgttattgt aataccagta acaaagggtg tttaaaattt 17940
gttcagtggg tgaatatatg ttccatttaa tggataaatt atttttattt cagtctcctg 18000
ttgatggaca tttgaataat ttccatcttt ttctctatga atgcctcact tggcatgctt 18060
ctgacagtat tgccacagaa tacatttctg ttataaaaat tgaattttta agtcaaaggg 18120
tagttacact ttaatggata gtggcagctt actatcaaaa gtttctgcta gtttcaccat 18180
atccttatta gcagtagata ttatcaatct tttcaatctt tgccaatctg ataagcaaaa 18240
agtaaatggg tttaaacatc ctttgtatat attcattgct cactttatgt ttttcctttg 18300
aaatgttatt tcttgttctt tccctgcagt atgattcttt cttttttga cttgttccca 18360
gtttttgtg tactatggat attagccttt aattatgtta cggatgttct agtatgttat 18420
tttttgaatt acttcaaatg tgatttgttg ctcagatttt aaaaactaca tacacaaatt 18480
atctcatgtt tcccttttg gtttcaattt cgactcatgc ttaatcagtt catcgattgg 18540
gcatggtttt attcttaata tatacccgta ttttatctca ttttattttt ttacgtgtaa 18600
atatttggtg aatataggtt taattttaat gtaaaataag gatgaaaaat gatagttgga 18660
attacaagcc catttctcct aatactttta atcaagtaat ccactaattg aaatattacc 18720
ttcttcattt atgaaattgc cacattatat ctgggtgttt ttctgcctac tacagtctct 18780
tacccatttc tttcctaata atacaatact tgaattgctg tggttgttga tttataatgt 18840
tatcttaatg ataacattat aaatgtgatg gaactggttc ctccttatag ttcttcttaa 18900
atcaagaaca agacatatct tcccatttac tctcgtatgt atctcatttt actgttatga 18960
atgaaatctg tcctatttgt gtataggaaa atagtttttg tatgtaattg tgatatggcc 19020
agttttatta aaaatttggt taaactaaga gttgttttct gttcagcctt atcatactat 19080
aaaatccaca taaaatgggt ataaaagtgt cgcaggacac tgggctcaga tgattctccc 19140

acctcagctt cccaagtagc tgggactaca gcggcatatg ccaccacacc cagccaattt 19200
ttaaataagt tttaaaaata gtatttttag tagagacagg gtttcaccat gttgcccagg 19260
ctggtcttga actcctggac tcagacaatc cacctgcctt ggcttcccaa agtgttggga 19320
ttacaggtgt gagccaccac accttgccga attgcagcca tatttaatac tttttttccat 19380
cctattccct ttgctgcccc caggcctcct gtattgatag cccgctatta agaagctagt 19440
gtatattctt tgcatacttt tacttcataa actatatgaa gcattgttct gtttttttaac 19500
ttaattggta taaaattata ttttggaaat tcagtatatt ctgtgaaaat tatttagaaa 19560
atgtgcctct gagataaagc ctattcagga tgtatcttaa aggagatagc tgtgctttaa 19620
cattatcagt ctttttggct gcttatgtta atataagttg gagaaaaaca gtctgctttt 19680
tgtgataata tgttcttgga gatggagtga aagattgttt aaaaacattg tcttttttttt 19740
cccctgaagt accagtattt attttaggat tatgttactg atcaaagatg ctgtgtggag 19800
ttactcattg gtgagactaa caataaatca cacatgcaaa ggatgttacc ataatctaat 19860
tattttaaac agtaaaatta tattctaaga catccagttg gcctatatgt gctatatcaa 19920
tgactatcaa ggggcttttt atgtatactg tatacatgta cttcacaaaa atataaaagg 19980
atgacatcaa aaatctggca agccaaaagc ctacattaca tgtagcaaat aaataagcat 20040
atgaacttat tggaatttaa aaccctgtag gatgggcggg tgatggtatg tatgttagat 20100
gtgtggacat atctattaaa agttgtgtca gataacagct ggtgctgaca agcccttggt 20160
aagatggcag catgttcaat atgttctgtg aaaattatct cagtttatga tctgtcagta 20220
ttgtggagct atgcatgaaa ggacttaaaa ttcttaccct taaactcagt aacagtgttt 20280
ctagaacttc tggtgatatg ggaaattaag agaattattt atatgcaaag gtgtttattg 20340
cagcattgtt ggaataatag acaaaatggg gaagaacaag ctcagaatgg aggaggtagc 20400
ttatagtata gacatacgat acaatccaga tgataatatt ttataatagt cttcacaagg 20460
aattttatat ttttattttt aaaaatacat agcagtgagt ttaatatacc aaacatacca 20520
aaatgtcatc atttactgtg tggtggactc atatgatgga gatgataaat aaaaatatta 20580
atttatttga ggcatatatt tatggctgag gaaggaagac agttatgaag aacagctcat 20640
tctggaaaca tactaatttt tcccagccat aaagagattt cctatttctt tttttttttcc 20700
atttaccttc tgtttcctac ctgagaagat ttcatacttc taataaccat ttgtgtacct 20760
atttaaagac agtaccaaag gcatacattt tagtgtttgg aggaccaagg gtcatttgat 20820
gtttgatgct tattgactat tcgaggatga caagacacct tgagaacaca cacacccaca 20880
cccacaccca caccctcacc cacccacccc accccctcc ccgaagaaag ctgtgaagga 20940
agaaagcaga aaagaacctg gagtgagttg taacttaaaa tgttagtgtt gcatgaagtg 21000
tgttaaaaca ggaagatttg aggaaattgc atacattttc tagatggcaa agtattactg 21060

gtgacagtta atgaaaatgc atatgcatgt gtttttagat ttacaaattt tactaagaac 21120

tttttaaaaa tccctgaagg tgtatcaaaa gtttatcatg cttatgaaat agagtagcac 21180

tttctaactt taaaacgggg aataattctt tggatcttga ttattggaaa agtgaattat 21240

gaattgctag tataaaactg tggttttaaa atatgtctgc tttatatttt tatgtagcag 21300

atttactcct agttaataat actcaaactt actgaaaact aaggtaatta agataattct 21360

gtcctgatgg gaagaggaaa aataacttca gtgtgaaatc tattatatat tagttgtggc 21420

aagatttctc ccattgactt tgactggaga catttatagg gttaaaatcg gaaatagcac 21480

ggtgaatttt gaagtatcct tgtagttgga aagagtatta tgttcatatt gccaaaaaaa 21540

agatgcatgg atgcattaga ctggatggaa aatacatgag aagttggcta gccccctctt 21600

tgtcaaaaca tcacttggtg gtgataaagc tgttggaaaa cacagcattc taatgtagtc 21660

tgtagtttaa tgataatctg tgtcttgaaa catttagcgt agtacttata caaacctaga 21720

tggcatagtg tactgcatgc ctagcctata tagtatagcc tgttgcttct agggtgtaaa 21780

gctgtatagc gtgttactat aggcagttga aacagtggta tttatgtatc cttttttttt 21840

tttttaaatt cttttaagag acagggtctt gctctgttgc ccaggctgga tgcattggtg 21900

tgatcatagc tcactataac cttgaactcc taagtgatcc tctttgcctc agcctcccca 21960

gtggctagga ctacaggcac atactaccac acctggctaa tttttaacat ttttttgtag 22020

agatggaatt tcgctgtgtt gtccaggctg gtcttggaac tcttgtgctg cagcaatcca 22080

cccgcctccc aaagtgttag aattacaagc cacttcgcct ggcttgttta cctaaacata 22140

gaaaagatcc agtaaaaata cagaattaaa atcttgtggg gccactgtag catatgtagt 22200

ccatcttgac tgaaatgtcc ttatgcagtg catgattgta cttcataatt tttaagcact 22260

cctccctctt gattggtact tagtggattt tatcattttt gtttcttcat aattctttct 22320

gaaatgtcta ctggttggac ctttgatctc ctgaattgat cgtgatttct tctgttgtat 22380

tttttgtctt tgtcattttt ttgtactcta ggcagttttc tcaattttag tttctattca 22440

actttttgtt tttatttatt ctctccagta tttatggaga tactaaattg aagtgttctg 22500

tttctctctc caccctatcc ctagtttcaa gttttatctc agtttctatg gagtcagttt 22560

tttcgttgct ttaaaaaaaa attttcctga agtgattggt aagttttggc taattgggag 22620

cactagaatt gggcccttaa tggttggcag ggtgtggtgg aggagagaca gcccttagtc 22680

caaaggctca ggccagaaaa agaaagagga aggctttcct tttcctttcc ggagcagggt 22740

tctgccctag gtcttgcttg gcagtctatt tgatttcttt agcagttaat gctcagtttt 22800

ttggcatatg tggatctgcc tccagagcag gtacaaggtg agtgagtcta tgctgttacc 22860

taattagatc cccatttcta ccctttgttt ttacttctct atctactgat aggtttttac 22920

cctccttcac ctcatagggt tgcagtgaag agcaagatga atttttattt atgttgcata 22980
aattttaaaa gctaaaaaat atatatgtaa tgttgggaag tcccagtgta caaatggcta 23040
ttgtaaattt ggaacatgaa cttgcttttt tccattgtaa aaatgaaatc attataaatt 23100
gcggtcaagt tactaggtca gcccacacag agtttaccca gtaatatgcg taaatgtttt 23160
gcctttgcat caacaacaag gaaaaacagt actataaaaa aatgttcctg gaagccggat 23220
gtatcaaagc acttctgaaa tagctatata gcctatagac atgaccagtt ggtttctgag 23280
tctgttgaca ttggccaaag gagaagctca gtgtagaaca tgtttggagt ctccttttgc 23340
agaaatacat tggaggctgg agtggggaac caatttttca gaaaggtggt gaagtagtta 23400
catagccact cttttaaaga cagtcaaaag atagaaacta aggccaggtg ttggctcaca 23460
tctgagatag gaaaatcact tgaacctggg aggcggaggt tgcagtgagc ccagtatgca 23520
cctctgcact ccagcctggt ttggcaagag accaaaactc tgtctcaaaa aaaaacaaaa 23580
catagttcac acttaaatat tttattccat atctttacat acccaatatg ttaatttata 23640
gttcaagatg aacttgtttg ggacagattt tgtaataaag gaaatcgtgt tattagaaat 23700
atctagaggc catgagccct taaactgttc taatttgcaa gtagttccct gtgtgatgca 23760
gtttttttca atattgcaca ataaaggcaa aatacggaca aattagatga taagatttat 23820
ataaattttt aaaatattga tcaaaatatg tatccatatt ggtaatattt gtatttataa 23880
taaatcattg ctgtaaattt gaacttagaa aaattttact aataaaggtg cttttgtgtt 23940
gcaaactttc atttgaaaag taatttttct ttgtaccaaa aaatctaaaa ttcgctattc 24000
tagtcaccaa aatttgcttt atgaaaaata atttttgatg gcactatatc agaaaacaac 24060
ttgttaaaga aaatgtggag tttttaaaat cccactgtac ctctgttatc caaaggggat 24120
ctgtgaattt ttctgtgaaa ggttaaaaaa ggagagacct ttaggaattc agagagcagc 24180
tgatttttga atagtgtttt cccctccctg gcttttatta ttacaactct gtgctttttc 24240
atcaccatcc tgaatatcta taattaatat ttatactatt aataaaaaga catttttggt 24300
aaggaggagt tttcactgaa gttcagcagt gatggagctg tggttgaggt gtctggagga 24360
gaccatgagg tctgcgtttc actaacctgg taaaagagga tatgggtttt ttttgtgggt 24420
gtaatagtga catttaacag gtatcccagt gacttaggag tattaatcaa gctaaattta 24480
aatcctaatg acttttgatt aacttttttt agggtatttg aagtatacca tacaactgtt 24540
ttgaaaatcc agcgtggaca atggctactc aaggtttgtg tcattaaatc tttagttact 24600
gaattggggc tctgcttcgt tgccattaag ccagtctggc tgagatcccc ctgctttcct 24660
ctctccctgc ttacttgtca ggctaccttt tgctccattt tctgctcact cctcctaatg 24720
gcttggtgaa atagcaaaca agccaccagc aggaatctag tctggatgac tgcttctgga 24780
gcctggatgc agtaccattc ttccactgat tcagtgagta actgttaggt ggttccctaa 24840

gggattaggt atttcatcac tgagctaacc ctggctatca ttctgctttt cttggctgtc 24900
tttcagattt gactttattt ctaaaaatat ttcaatgggt catatcacag attctttttt 24960
tttaaattaa agtaacattt ccaatctact aatgctaata ctgtttcgta tttatagctg 25020
atttgatgga gttggacatg gccatggaac cagacagaaa agcggctgtt agtcactggc 25080
agcaacagtc ttacctggac tctggaatcc attctggtgc cactaccaca gctccttctc 25140
tgagtggtaa aggcaatcct gaggaagagg atgtggatac ctcccaagtc ctgtatgagt 25200
gggaacaggg attttctcag tccttcactc aagaacaagt agctggtaag agtattattt 25260
ttcattgcct tactgaaagt cagaatgcag ttttgagaac taaaaagtta gtgtataata 25320
gtttaaataa aatgttgtgg tgaagaaaag agagtaatag caatgtcact tttaccattt 25380
aggatagcaa atacttaggt aaatgctgaa ctgtggatag tgagtgttga attaaccttt 25440
tccagatatt gatggacagt atgcaatgac tcgagctcag agggtacgag ctgctatgtt 25500
ccctgagaca ttagatgagg gcatgcagat cccatctaca cagtttgatg ctgctcatcc 25560
cactaatgtc cagcgtttgg ctgaaccatc acagatgctg aaacatgcag ttgtaaactt 25620
gattaactat caagatgatg cagaacttgc cacacgtgca atccctgaac tgacaaaact 25680
gctaaatgac gaggaccagg taagcaatga catagctagc tttttagtct gctttgaagt 25740
aaatgctcaa ggggagtagt ttcagaatgt ctacccaata ccagtacttg aaaactaacg 25800
atgtttctga attcctgtat tacaggtggt ggttaataag gctgcagtta tggtccatca 25860
gctttctaaa aaggaagctt ccagacacgc tatcatgcgt tctcctcaga tggtgtctgc 25920
tattgtacgt accatgcaga atacaaatga tgtagaaaca gctcgttgta ccgctgggac 25980
cttgcataac ctttcccatc atcgtgaggg cttactggcc atctttaagt ctggaggcat 26040
tcctgccctg gtgaaaatgc ttgggtaaga aaacatgtca gaatgcttga agctaaaaag 26100
tagaagagta tactcacaat atttctgatg aggctttttt cttcttccca gttcaccagt 26160
ggattctgtg ttgttttatg ccattacaac tctccacaac cttttattac atcaagaagg 26220
agctaaaatg gcagtgcgtt tagctggtgg gctgcagaaa atggttgcct tgctcaacaa 26280
aacaaatgtt aaattcttgg ctattacgac agactgcctt caaattttag cttatggcaa 26340
ccaagaaagc aaggtaagag aattattctt tatgtggttt tcatggagca ttggacacct 26400
ccagtgtcat gtcattccat gcagtgttcc taaccttttt ggcaccaggg accagtttcg 26460
tggaaaacag tttttccatg aatgggttgt gggaatggtt tctggatgac accattccac 26520
ctcagataat caggcattag attctcatag ggagcgtgca gcctagatcc ctcgcatgtg 26580
cagtccacac tagggtttct actcctatga gactctcatg gtgcagttga tctgacagga 26640
ggtagagctc aagccaggta atgctcgctc acctgccact tacctcctgc tgtgcagccc 26700

agttcatttc tgttctttta aatttttgag tttccatatg taaagcacta tgcgaagtag 26760
tagggatatg gtaggcaagc ttctcttcac acttttgttc ttaggtggga tgtagatgtt 26820
gggaataata acctaatatt taatttgtgt agtgggaaga agtggggcta tgagggcaca 26880
taacacaagt tgaaactgac tctttttgag ggttcaagga gacctcttgg aggaagtgat 26940
agttgagttc agtgttcaag gatgagaagg gattcactag gtgaaggtta ggtgagaaaa 27000
caacatcttt gaaacgaagg aaggagatgg aaagttttgg gaatttaaga aatactaata 27060
gtaaggagga agaaaggttt gaggtgaggc tattgagata gacttagcag atctcatagg 27120
gctttgtaga gcatgtttaa aagcacaatg ggaaatttca gcagaagcct gaaatgatga 27180
aatttgtttt tagaaaattg gggcagtgtt gaaagggaag atatacaggg aatgaaagga 27240
caagcatgaa tgatcatttt atggtatctg tttttaaggt ggatataatt aggaaaatta 27300
aagggccaaa tgatgaggag ttaagtgcca gttctggttc aaattttcag tgaatcagtt 27360
ttgatataac tttcatctta gggcattact cttgcctacc aacatagttt ctaaattttt 27420
ttcttttggt gtgatcactg tgggaagaag gaaattgggc ccaaactgat acattgtttg 27480
gaggactggg atgtctgaat ttgagtggaa tgctttaaaa ggacaagttg gatagggccc 27540
cagtatgggg gtctgagtga tggggtccag gaatacattt aggtccaatg gcaagctggc 27600
tgaaattctt gtataataaa ataggttggt aatatggctc ttctcagaca tgtgatcaag 27660
attccttgac taacaagata tatatatata tctttctagc tcatcatact ggctagtggt 27720
ggaccccaag ctttagtaaa tataatgagg acctatactt acgaaaaact actgtggacc 27780
acaagcagag tgctgaaggt gctatctgtc tgctctagta ataagccggc tattgtagaa 27840
gctggtaagt atatgtatct attctgagtc ttgtgtatag catctgcagt tctaattaga 27900
ttacttttct taggaaaagg tggtagaact ttaactactg aaaataaatg gtcctattca 27960
gtttgcagcc aagatttaca ttcagagtac ctgtcatctg gattgtagct aaatatttaa 28020
ggctagttta ggtagagttc ttattatcca tcaaaaatga tggcatatgt tttgcttaat 28080
aaaatttgtt tgtaatttca gttttgagta aacctaagat ttgctaacag agctgtgaat 28140
ttataggaga aaagacaaat tctaatatag tacagtttta tgtaaagtga ttgctttatt 28200
agtagatgct catgagcagt ttttgttttg ttttaacttt taggttccgg gtaatgtgca 28260
ggcttgttat ataggtaaat tgcatgtcac aggggtttcg tgtgcagatt attttgtcac 28320
ccaggcagta agtattgtac ccaataggta gtttttcagt tctttacctc ccacccgtaa 28380
gtaggcccca gtgtctgttg ttcccttctt tgtgcccgtg tgtactcagt gtttacctcc 28440
cacttataag tgagaacatg tggtatttgg ttttctattc ctatgttagt ttgcttagga 28500
taatggcctc cagctccatc catgttgctg aggaagacat cttggtattt ttttatggct 28560
gcttagtatt ccatagtata tatgtaccac attttcttta tctagtctac cattgatggg 28620

catttaggtt aattccatat ctttgctatt gtgaataatg ctgcagtgaa catatgcatg 28680
catgtgtctt tatggtaaaa agatttcttt ttctttgggc atatacctaa taataggatt 28740
gctggattga atggtaattc tgtcaggttt tttgagaaat caccaaattg ctttccacaa 28800
tggctgaact aatttacttt cccaccagca gtgtataagc attctctttt ctcagcaacc 28860
tcaccagcat ctgtcatttt ttgacttttt attagtagcc attctaactg gtgtgagacg 28920
gtatctcatt gtggttttga tttgcatttc tctaatgatc agtgatgtcg agcttttctt 28980
catatgtttc ttggccactt gtatgtcttc ttttgaaaag tgtctgttca tgtcctttgc 29040
ccactttttta atggggttgt tcttttttgc ttgttaattt aagtttattg taaactctgg 29100
atattagacc tttgtcagat gcatagtttg ccagtacttt ctcccatgcc agtactttct 29160
cccattctgt aggttgtctg tttactctgt tgatttcttt tgctgcgcag aagctcttta 29220
tactgtccca tttgtcagtt tttgtttttg ttgcaacttc tcttggcatc ttcgtcatga 29280
aatctttgcc aggtcttatg tccagaatgg tatttcctag gttatcttgc agagttttta 29340
cagttttaag ttttatattt aagtctttaa tccattctga gttgattttt gtacatcatg 29400
taaggatggg gtgcagtttc aatcttggat gtggctagcc agttatccca gcaccattta 29460
ttgaataggg agtcctttcc ccattgcttg tttttgttta cttgttaggt gtgcggccta 29520
acttctgggc tttcttttct gttccattgg tctctgtgtc tgtttgtata ccagtaccat 29580
gctgtgattg taaccttgta ttaacagtat agcttgaagt tgggtaaagt gattcctcca 29640
gttttgttct ttttgcttag gattgccttg gctattcagg ctcttttttg ggttcatatg 29700
aatttttaaa tagttttttt ttaattatgt gaagaatgcc attggtagtt tggtaggaat 29760
agcattgaat ctgtgaattg ctttgggctg tatggccatt ttaacaatat tgattcttcc 29820
tgccatgaaa tagaatgttt tttcatttgt tggtgtcatc tctgatttct ttgagcagtg 29880
ttttttgtaa ttctcattgt agagatcttt cacctccctg gttagttgta ttcctaggta 29940
ttttattctt tttgtggctt tggtaaatgg gattgcattc ttgatttggc ttgcagcttg 30000
gatgttgttg gtgtctagaa atgcttctga cttttgtaca ttgattttta tatcctgaaa 30060
ctttgctgaa gtttattgga tcaaggagct tttgggcaga gattatgggg ttttctaggt 30120
atagaatcat attgtttgca aacagacttc ctatttggat gcattttctt tctcttgcct 30180
gattatgagc agtgttttgc cctgatattc tgtattctca gtgaatagat gtcgtctaag 30240
tatgagaaac aatttttttc tattctgagt atttttaaga aggcaactta tatgtggtac 30300
tttgtatatt gtgtatgttg gcaattgggg aaaagaatag atggtttgta ctagggcctc 30360
ttgggttctg tgtgtgtgtg tgtgtgtgtg tgtgtgtgtc atgaaaacag ttacttttta 30420
gctaccaagc atttttctc ctttcagtaa cccacctaac aacatttact cagaatttca 30480

aagcaagctt caaatcagta ttgaaagaag gaaaaatata aaggcattta atggaagaaa 30540
atgttgggaa taaagtatag ggctggcaac acttactttt ctcacttatt gagagtaatt 30600
ttacttggga atttatgaga gagaaagaca ttatgattgc tccaggtaac tactggcaga 30660
ggaaccatag tcttggggat agacaaatgt ggctgagttc atatagaatg aggggatggg 30720
atgtaaattc tgtcagctgt tccagcagta acctgtaatg taggctaaaa atacagattt 30780
tgagatttat ttaatcagaa tccctggagt gttaattttt atatcaagat ctcatagtgt 30840
tttatttgaa gtgacaggga ggtctgtaga tagctggaca tgtatgggac tggaagctta 30900
ggaatcttta agttcttcca ggttattctt atgttcattt gtttattctg aaaatagcat 30960
ctaatgtatt ttaagaaatg gaataggcac atagtataca ttgggtaaca caacagatag 31020
ggtccccgtg cttaattctt agtcttgtga aggtgacaaa aatacttaaa aatatgtgat 31080
cctaaattag aatgagtgtt atgggagaaa tgacagcaaa tagtgatgag aattaatggg 31140
gaggggaatt gtctagatga gagggaaaag gtctccttga aaaggggatg ttaagtggga 31200
ctgcaggatg agagggaacc gtctcttgtc tatatgagaa gtgagggtta aacgttttcc 31260
aggtagagaa aaggaacacc atgtgctatg tcttagaacc agggatatcc agtcttttgg 31320
cttccctggg ccacattgga agaagaataa ttgtcttggg ctacacacca aatacactaa 31380
tgatagctga tgagctaaaa caaaaaaaaa ttgcaaaaga atctcataat gtttaagaaa 31440
gtttacgaat ttgtgttggg ctacattcag agctgtccta ggccatgtgg cccatgggct 31500
gcaggttgga caagcttgcc ttagaaggaa agagattggt caggcacggt ggctcacgcc 31560
tgtaattcca gcactttggg aggctgaggt gggcggatca tgaggtcagg agatcgagac 31620
cagcctggct aacacagtga aaccccatct ctactaaaaa tacaaaaagt tagccgggcg 31680
tggtggcagg cgcctgtagt cccagctact tgggaggctg aggcaggaga atggtgtgaa 31740
cccgggaggc ggagcttgca gtgagctgag atagcgccac tgcacttcag cctgggcgac 31800
agagtgagac tctatctcaa aaaaaaaaaa aagggaaaga gattgtggag atccaggtgc 31860
tgaagagaag gtctgcataa acagaactta gtaatgaggt ggatggcctg gtatgaggtt 31920
gaggttaggt aagcagagcc ataacatgca ggactttcta ggttcctata agatagttac 31980
tactcatgga gtttattcat gctttattcc agctttggag ccatagatac agaatacttt 32040
ggtcagtttg gaaggctagg tgggatccaa attctaaacg gttcctcagg gttatactaa 32100
agtatttcta ttatcttaaa aggatgctga gacactttcg atggttgttt atcaatagca 32160
aagcatcaca gtggtgtgtt taaaatatta ataatagcat tgtatagatt aacagtttga 32220
atgaccaaaa gctagaagac cagactactg agatgttaca ggcttttagg aatgaaatag 32280
tttgctttta gaactcaata gcaaagggca gatgtctgag atgcctgaaa gaatcataga 32340
atgtaataat ataggagcta agggagcaac caaaaacggt ttgtggaggg gacaacattg 32400

gtaccatgaa gataaatgga accctcagaa ggcatcctta atttttgaac ataataattt 32460
aagaagctga cttaaagtga cttaaaaggt cagtaggtag ctggaaatgt atgatactag 32520
aatgcaagag aggcaggcta gagatttgga agtttccctc ttagtatata ggggtaaggg 32580
cagcagggaa ggggaggtag aggtgccaca gagtcatctg tatgggactt tttttttac 32640
cctagaactg ctgaatcaga atgtgtgtgt tttaaagtct ctgtaggcca ttctgatgga 32700
catctggggt taaaatccat tctcttagag ttaatagtta tgtaaaggga gggaatgaag 32760
tcttaaagag gggaaagaag gtagtcattt cacaaatact gagcatcctg atcatcagtc 32820
ttacgcagat cattctatta gtagctggag ctactatgaa aaaggaaccc aacagaggtg 32880
atctttgtct tgtagggaaa gtggagtaac ttacactatg aaggagaagt gcagggtacc 32940
ataagaatta cagcagatag acctcatctg aggaaataaa acagacccga aagatgaagg 33000
agacaaggaa aagtatctct tactgcattc agaagtgatt taagttgaag atggatgagc 33060
gaagttaatc tactatgtgg gcattgggct tccatttata ctcctttgcc agagtaaatg 33120
tcccccattt aagggtccta aaggatggaa gattgtaaac cttggaacac atgttttgta 33180
gtcagtgaat tgtataaagt ccctgacagt aagtgttttc atgccgtctt tctggattgt 33240
tcttacccca ggaatttacc tagcttcttt aggtctttag tcagatgtca ccttcacagt 33300
gaggtgacct aattatctat ttaaaatcgc agccccactc cattattttt ctccatagcc 33360
ctttaatatc atctgacata ctgtatggtt ttagtttatt gtatattttt ctgcctcttc 33420
caactagatc ataaattctg agggtaggaa cttctgaata tttttgttca ctggtctatc 33480
tgcagctcag aacaggacct ggtactgaat aaatattttt gaaatgattg aatggatgaa 33540
aagaaatgag taataagaat attacctaag ggggacagtg gagataacaa aggctttttc 33600
ggcttaggaa aggaacagta gctatttgag agtttgtcac tagtgaggtg aactggcaaa 33660
gtgaaggaaa ctgagcaaca ttctagaaaa tgagaggaaa tcaaatactt aggtgaaagg 33720
aagtaaactc tggaaataca gaaggacacc tcctaaggct agaacagata tttaggattg 33780
ataggcactt ctagctaatg actagggcct tatatccttt ttaattttct aggtggaatg 33840
caagctttag gacttcacct gacagatcca agtcaacgtc ttgttcagaa ctgtctttgg 33900
actctcagga atctttcaga tgctgcaact aaacaggtaa attctgagta aactggtgcc 33960
atgggaatag agtcaagatg agtatgtgct tgtactgacc atctgttttt atctccatag 34020
gaagggatgg aaggtctcct tgggactctt gttcagcttc tgggttcaga tgatataaat 34080
gtggtcacct gtgcagctgg aattctttct aacctcactt gcaataatta taagaacaag 34140
atgatggtct gccaagtggg tggtatagag gctcttgtgc gtactgtcct tcgggctggt 34200
gacagggaag acatcactga gcctgccatc tgtgctcttc gtcatctgac cagccgacac 34260

caagaagcag agatggccca gaatgcagtt cgccttcact atggactacc agttgtggtt 34320
aagctcttac acccaccatc ccactggcct ctgataaagg taaattgtca aagtagaatt 34380
tacctttgtt gcagaattga aaatgaagca tctctagctg ttggatggct gtctaagcat 34440
agtgatcaat aagtaggaat tgtattcctt agtaagtagg aagtatggct gcgatagggg 34500
taagattctg aaatgtttgt gtagtcagaa ctacttttag ttgataccaa tagatttagt 34560
gtggtgggaa ttttagggta agaaaatgat tttgttgagt tgtatgccag ttcttccttc 34620
tgtttttcag gctactgttg gattgattcg aaatcttgcc ctttgtcccg caaatcatgc 34680
acctttgcgt gagcagggtg ccattccacg actagttcag ttgcttgttc gtgcacatca 34740
ggatacccag cgccgtacgt ccatgggtgg gacacagcag caatttgtgg taggtaaatt 34800
cttacagtga tacctggcta tctaaaagga atgcataaat ccaaaggatc ctgaacttct 34860
ttctttggtc attggttccc cccatccgtc ttcctgaaga gctaatgaca aagtaaataa 34920
ataaataatt acacatttct atggctgcag agaaaataag gcatagtgtg gccccagtga 34980
tatttccttg gacacgtcct tcacatggtc agtcttacaa aggttgggtt aggtgtttca 35040
taaagtgttc tcatttaatt tacacaaagg cccacttcct taggaagagg tagagtcata 35100
atttgagatc aaatctgtgt aatttcagag cctcttaccc ttgcctcatc atgcattttg 35160
actataaata tttagcagtc cgttttatta tcttttctgt gagttaaact tttttcatgg 35220
acctaagaat attcagaaat aagtagtagc atttctgtac tcttaaccac aaaaatctca 35280
acctgaagct ttgatacaaa gtttgtgtct taaaagtagc ttcattaaaa gtatagtcta 35340
atgacatttc tgatttctca gactttaaga ccttattagg ttagtttaga aaacaaagat 35400
ggagcctacc agaacagatg ttaggaatct cattttgctg gttgctttgt gtatgtactc 35460
atattggggc tttggctttc ttcatttatt actgttggta ttggcccatc tccatgaggt 35520
gacttaatag aacgttgagg gcacctttta ttttaaatct cttttctagg aagaagagag 35580
tttttgtgtc cttgtaagaa tcaagttatt tataaaagct gctaaatgta gcagaataat 35640
aacccctttt aaaactcaaa tccagaaaca ggagaaacag atggtactta catattgcaa 35700
aagctatctt ccttctatac atgaggctgt cagctgaata gtcttggaag agtgaggagt 35760
gaatttttct gctggcaact cggttagttt tagcagttgg tgctaaaact tggcaaagtt 35820
ttcaccaaat acatggaaga tatacaaaaa tagagggggc atgtaaaaga aaaacgttga 35880
catagtctga gcattacttt ctcatcttct ctttttatat accttttacc cagaatgatt 35940
ggtgccctta ctgtaggaaa gttgtctttg ggattcagcg ctgtatggaa gctctgttgc 36000
actgtgtatg ggggaggggt gctgctttga attagtgctg ccaggaggcc tcttttcagt 36060
gacattcaag ttaatggaat ccttcttcct tcctgaacta attgcaagtt acggggaact 36120
tcgggtatat aatgtaaata attacagtct aataattgtt cctcaaactt tacagaggag 36180

aatgccctgt ttgttaacca tgtttctttt ggcaggaggg ggtccgcatg gaagaaatag 36240
ttgaaggttg taccggagcc cttcacatcc tagctcggga tgttcacaac cgaattgtta 36300
tcagaggact aaataccatt ccattgtttg tgcaggtatg ttttaagtga agtgttctag 36360
gttttatgtc cataaaattt ccagattgta atgactaata acatttcaga aaattaggga 36420
ccataatagg gttaccaaca tttaatttta tgaaaattcc ctacattttt tggtcagtaa 36480
gagaaacatt gagacttgag aagagggagg agatttcaca tttcactttt atgggtgcct 36540
agaggggaga gctgacctgg gctgccagag gcagggcata gaccccccaac caattctggg 36600
ttttccaaat cttagatcag ttagagctgc ctctgaagaa agggtttata gctaaaaaat 36660
attatggaaa tccagtgctc cagagcatta aacaccccaa gacataaaat tcagagaata 36720
ttatttacta cagtgtgaat gcctcttgca ctctgaattg ggaatgtttg caccacagtg 36780
gggggcttgc catgttttag ctttagattt aattaggttt tgtttgtgtt ttctccttag 36840
ctgctttatt ctcccattga aaacatccaa agagtagctg caggggtcct ctgtgaactt 36900
gctcaggaca aggaagctgc agaagctatt gaagctgagg gagccacagc tcctctgaca 36960
gagttacttc actctaggaa tgaaggtgtg ggtaagtaaa aaggaaccaa agcctttagc 37020
agatgtgtac attgaagtct cagtttttcc tcaagggcct ttttctcctt gtctcttagc 37080
gacatatgca gctgctgttt tgttccgaat gtctgaggac aagccacaag attacaagaa 37140
acggctttca gttgagctga ccagctctct cttcagaaca gagccaatgg cttggaatga 37200
ggtagggaaa tgtgagcagt tatttatctg gtagtttcct agagcaggta tggcagcttg 37260
ttctttcctc tcaaaacact tagtacacat tcatttgcat tgatgtttcc ctggcttgag 37320
tatttcttct ttatgctgtc tagcaactgc tctgaggaag aactataata caagctttaa 37380
agagtctgtt cagaatcatt acaaataagt tgtgttattt aaaattataa ttcataaggg 37440
agaaagatga aaaatgttac cagattaaag aagatttttc aaaaggatgt aaggaaagag 37500
gcagtgttaa acactgttaa gaggacagtt tatcagtatt ttttactaaa ctttaataaa 37560
acttttctat ttgaatttct gctatgaatt tttcttcagc atttgtcctc agtacaggtg 37620
gttccttgaa acattgtttc taataaaact agaacatcct gatattttat ccattctata 37680
gagatcattg atggtacaca gacatacagt ggattatgtt tgttgagtga atggaaagag 37740
agattgttag gtttacaacg atgcagctct tgagaccgga gtttaagatc agcctgggca 37800
acatagtgaa accccatctt tagctgggca tggagatgga tgcctatagt cctagctact 37860
ggggagacgg gggcaggagg attgcttgaa cccaggagtt aacagactgc actcagtgac 37920
agagccagac tccaacacaa aaaaaaaaaa aaaaaaaaag caaattacca gtgagtagtg 37980
tgttacttgg gtttttaata ggcatcttat taacatgttc caacttgagc ccttaacttt 38040

ctccacctac ccccttccac aaacctgttt tcactgtctt ctctgtctta gttaatgtca 38100
gctttgtctg tccagctgct caggctaaaa cttttctttc atataacaca tcctatcagc 38160
agctcctgtt tgtgggtagg cattttgcct tttttttttt tttttttttt aaactgctat 38220
atctctagca tgtagaacag tgcctggcag cacataatag gtgcttaata taatatttgt 38280
tgaaagaaca agtcagtgag tatttttaat gtgaggtgca aagagaaaaa aaaatgtatc 38340
tttgaggtgt ggagttttga agaacttcca ttttctaagc atttgtgtaa tgttggagtt 38400
acttgttcct tttgtaatct gaaagtatgc tttaaaaaaa attagtgtac ttttgagaat 38460
tttcattttg ctttctattc ttccttgctt tgtgcatgtt tatctagact gctgatcttg 38520
gacttgatat tggtgcccag ggagaacccc ttggatatcg ccaggatggt atgtgtctca 38580
tatttctcga ttaactccag atcaagctaa agttctaaaa cttttatcag aagagccggt 38640
ttgctcatct gggaaaccag tgttggcaga aaagtagtgg cttcaattaa aagcagttct 38700
taaattccag tcagcaacag tatctttaat ggagcacagg gaattcagag ccacacaatg 38760
agtagcagta ggattacacc accaacaaat acatgctact gctaggcctc tgcagtgcag 38820
gatgttacaa tttacctggc tttttattct ctttttggcc agaggactca taatacctttt 38880
gtctacaagc tacccaagga agataggaaa actcctgttt ctaggctcag atctcgggtg 38940
ggtttttaca tagttgcatt atcatcaggg ttttcttgaa aagctaattt aaatctgggt 39000
aatgaacatg gaggatggca tagaccacta acaattataa ctgtcttaca tttataaccg 39060
catctgcttc tacctaatta tgaaaccact aaagcgcaga ttcttactgt gagaaataac 39120
atgtcaaccc taagataaaa tatgttgagg tttcatggaa atagtgcctt tccttagtac 39180
ttttgtgggt gtcacttggc ctttttgtca agatagatta cacctgccag acctcattat 39240
tgtcttaatc ctccttccca tgacttctca ctgcctaggt ggtcacacag tagattcctg 39300
cttcttctcc tcgggaaccc caagtctctt gacaggggta aatgcagagt gttcagggtt 39360
agactaatga tgtgactagg ccctgctggt gtgcctgtct gatggaaata gatgttattt 39420
gtgtagtctc atgggtggcc tggcactgag taattacttg gctaaagaaa gctggaggtt 39480
gaagaggcta gaaagcgttg ttttctgaca agtttgctgc tgaactttgg atgccctaac 39540
ctcagtgtta acgtctatgt ctgcttctct cctctctctt ttgccttcct tcttgcctat 39600
tttgttgaca ccctgactct tctagatcct agctatcgtt cttttcactc tggtggatat 39660
ggccaggatg ccttgggtat ggaccccatg atggaacatg agatgggtgg ccaccaccct 39720
ggtgctgact atccagttga tgggctgcca gatctggggc atgcccagga cctcatggat 39780
gggctgcctc caggtgacag caatcagctg gcctggtttg atactgacct gtaaatcatc 39840
ctttaggtaa gaagttttaa aaagccagtt tgggtaaaat acttttactc tgcctacaga 39900
acttcagaaa gacttggttg gtagggtggg agtggtttag gctatttgta aatctgccac 39960

aaaaacaggt atatactttg aaaggagatg tcttggaaca ttggaatgtt ctcagatttc 40020

tggttgttat gtgatcatgt gtggaagtta ttaactttaa tgtttttgc cacagctttt 40080

gcaacttaat actcaaatga gtaacatttg ctgttttaaa cattaatagc agcctttctc 40140

tctttataca gctgtattgt ctgaacttgc attgtgattg gcctgtagag ttgctgagag 40200

ggctcgaggg gtgggctggt atctcagaaa gtgcctgaca cactaaccaa gctgagtttc 40260

ctatgggaac aattgaagta aactttttgt tctggtcctt tttggtcgag gagtaacaat 40320

acaaatggat tttgggagtg actcaagaag tgaagaatgc acaagaatgg atcacaagat 40380

ggaatttatc aaaccctagc cttgcttgtt aaattttttt tttttttttt ttaagaatat 40440

ctgtaatggt actgactttg cttgctttga agtagctctt tttttttttt tttttttttt 40500

tttgcagtaa ctgtttttta agtctctcgt agtgttaagt tatagtgaat actgctacag 40560

caatttctaa tttttaagaa ttgagtaatg gtgtagaaca ctaattcata atcactctaa 40620

ttaattgtaa tctgaataaa gtgtaacaat tgtgtagcct ttttgtataa aatagacaaa 40680

tagaaaatgg tccaattagt ttccttttta atatgcttaa aataagcagg tggatctatt 40740

tcatgttttt gatcaaaaac tatttgggat atgtatgggt agggtaaatc agtaagaggt 40800

gttatttgga accttgtttt ggacagttta ccagttgcct tttatcccaa agttgttgta 40860

acctgctgtg atacgatgct tcaagagaaa atgcggttat aaaaaatggt tcagaattaa 40920

acttttaatt cattc 40935

<210> 19
<211> 26
<212> DNA
<213> Artificial

<220>
<223> Synthesis

<220>
<221> misc_signal
<222> (1)..(26)

<400> 19
ataaaggcta ctgttggatt gattcg 26

<210> 20
<211> 21
<212> DNA
<213> Artificial

<220>
<223> Synthesis

<220>

<221> misc_signal
<222> (1)..(21)

<400> 20
ctcacgcaaa ggtgcatgat t 21

<210> 21
<211> 3783
<212> DNA
<213> homo sapiens

<400> 21
ggggcggggc cgggagggta cttagggccg gggctggccc aggctacggc ggctgcaggg 60

ctccggcaac cgctccggca acgccaaccg ctccgctgcg cgcaggctgg gctgcaggct 120

ctcggctgca gcgctgggtg gatctaggat ccggcttcca acatgtggca gctctgggcc 180

tccctctgct gcctgctggt gttggccaat gcccggagca ggccctcttt ccatcccctg 240

tcggatgagc tggtcaacta tgtcaacaaa cggaatacca cgtggcaggc cgggcacaac 300

ttctacaacg tggacatgag ctacttgaag aggctatgtg gtaccttcct gggtgggccc 360

aagccacccc agagagttat gtttaccgag gacctgaagc tgcctgcaag cttcgatgca 420

cgggaacaat ggccacagtg tcccaccatc aaagagatca gagaccaggg ctcctgtggc 480

tcctgctggg ccttcggggc tgtggaagcc atctctgacc ggatctgcat ccacaccaat 540

gcgcacgtca gcgtggaggt gtcggcggag gacctgctca catgctgtgg cagcatgtgt 600

ggggacggct gtaatggtgg ctatcctgct gaagcttgga acttctggac aagaaaaggc 660

ctggtttctg gtggcctcta tgaatcccat gtagggtgca gaccgtactc catccctccc 720

tgtgagcacc acgtcaacgg ctcccggccc ccatgcacgg gggagggaga tacccccaag 780

tgtagcaaga tctgtgagcc tggctacagc ccgacctaca aacaggacaa gcactacgga 840

tacaattcct acagcgtctc caatagcgag aaggacatca tggccgagat ctacaaaaac 900

ggccccgtgg agggagcttt ctctgtgtat tcggacttcc tgctctacaa gtcaggagtg 960

taccaacacg tcaccggaga gatgatgggt ggccatgcca tccgcatcct gggctgggga 1020

gtggagaatg gcacacccta ctggctggtt gccaactcct ggaacactga ctggggtgac 1080

aatggcttct ttaaaatact cagaggacag gatcactgtg gaatcgaatc agaagtggtg 1140

gctggaattc cacgcaccga tcagtactgg gaaaagatct aatctgccgt gggcctgtcg 1200

tgccagtcct gggggcgaga tcggggtaga aatgcatttt attctttaag ttcacgtaag 1260

atacaagttt cagacagggt ctgaaggact ggattggcca aacatcagac ctgtcttcca 1320

aggagaccaa gtcctggcta catcccagcc tgtggttaca gtgcagacag gccatgtgag 1380

ccaccgctgc cagcacagag cgtccttccc cctgtagact agtgccgtag ggagtacctg 1440

ctgccccagc tgactgtggc cccctccgtg atccatccat ctccagggag caagacagag 1500

```
acgcaggaat ggaaagcgga gttcctaaca ggatgaaagt tcccccatca gttcccccag   1560
tacctccaag caagtagctt tccacatttg tcacagaaat cagaggagag acggtgttgg   1620
gagccctttg gagaacgcca gtctcccagg cccccctgcat ctatcgagtt tgcaatgtca  1680
caacctctct gatcttgtgc tcagcatgat tctttaatag aagttttatt ttttcgtgca   1740
ctctgctaat catgtgggtg agccagtgga acagcgggag acctgtgcta gttttacaga   1800
ttgcctcctt atgacgcggc tcaaaaggaa accaagtggt caggagttgt ttctgaccca   1860
ctgatctcta ctaccacaag gaaaatagtt taggagaaac cagcttttac tgtttttgaa   1920
aaattacagc ttcaccctgt caagttaaca aggaatgcct gtgccaataa aagttttctc   1980
caacttgaag tctactctga tgggatctca gatcctttgt cactgcctat agacttgtag   2040
ctgctgtctc tctttgtccc tgcagagaat cacgtcctgg aactgcatgt tcttgcgact   2100
cttgggactt catcttaact tctcgctgcc ccagccatgt tttcaaccat ggcatccctc   2160
ccccaattag ttccctgtca tcctcgtcaa ccttctctgt aagtgcctgg taagcttgcc   2220
cttgcttaag aactcaaaac atagctgtgc tctatttttt tgttgttgtt gtgactgaca   2280
gagtgagatt ccgtctccca ggctggagtg cagtggcgcc ttctcagctc actgcaacct   2340
gcagcctcct agattcaagc gattctcctg cttcagcctt ccgagtagct gggatgacag   2400
gcactcacca atatgcctgg gtaatttttg tatttttaag tacatacagg atttcaccat   2460
gttggccagg ctagtttcaa actcccggcc tcaggtggtc tgcctgcctc agcctcccaa   2520
agtgttggga ttacaggcgt gagccactgg gccctgcctg tattttttat cagccacaaa   2580
tccagcaaca agctgaggat tcagctcata aaacaggctt ggtgtcttgg tgatctcaca   2640
taaccaagat gctaccccgt ggggaaccac atcccccctgg atgccctcca gccttggttt  2700
gggctggagt cagggcctgt atacagtatt ttgaatttgt atgccactgg tttgcattgc   2760
tggtcaggaa ctctagtgct ttgcatagcc ctggtttaga aacatgttat agcagttctt   2820
ggtatagagc aaactagaag aaccagcaat cattccactg tcctgccaag gtacacctca   2880
gtactcccct tcccaactga agtggtatga ggctagctct ttccaaaagc attcaagttt   2940
ggcttctgat gtgactcaga atttaggaac cagatgctag atcaaataag ctctgaaaat   3000
ctgaggaaca ttgtaggaaa ggtttgttaa gcatctctta agtgccatga tgagcataac   3060
agccggccgt cgtggctcac gcctgtaatc ccagcacttt gggaggccaa ggtgggagga   3120
tgacaaggtc aggagttcaa gaccagcctg gccaacatgc tgaaacctca cctctactaa   3180
aaatacaaaa attagctggg catggtggca catgcctgta atcccagcta cttgggaggc   3240
tgaggcagga gaatcgcttg aacccgggag gcggaggttg cagtgagcca agacagtgcc   3300
agtgcactcc agcctcggtg acagcgcaag gctccgtctc aataattaaa aaaaaaaaaa   3360
aaaaaaaaaa ggccgggcgc agtggctcaa gcctgtaatc ccagcacttt gggaggctga   3420
```

ggcgggcaga tcacctgagg tcaggagttt tgagatcagc cttggcaaca cggtgaaacc 3480

ccatctctac taaaaataca aaattagcca agcatgctgg cacatgcctg taatcccagc 3540

tactcgggag gctgaggtac gagaatcgct tgaacctggg aggcagagga tgcagtgagc 3600

cgagatcacg ccattgcact ccagcctggg ggacaagagt gaatctgtgt ctcaccaaaa 3660

aaaaaaagaa aaagaaagat gcttaacaaa ggttaccata agccacaaat tcataaccac 3720

ttatccttcc agtttcaagt agaatatatt cataacctca ataaagttct ccctgctccc 3780

aaa 3783

<210> 22
<211> 23809
<212> DNA
<213> homo sapiens

<400> 22
gggagggtac ttagggccgg ggctggccca ggctacggcg gctgcagggc tccggcaacc 60

gctccggcaa cgccaaccgc tccgctgcgc gcaggctggg ctgcaggctc tcggctgcag 120

cgctgggtga gtgctgggga cccggggcca ccgcagcgta agtgaccttg gcggggacgg 180

tgctacccgg ccgccgagac gggttcctct gcgccctcag tcgggcccag gcgcggcccc 240

gcggcgtccc tgggggccgg cggggagccg ggaccctcgg gactgtccct gacgggcggg 300

ctggggtggg agtccgcgcg ctccgaagcg tcggcgagaa aagcagaaaa cagctccgcc 360

cgccagccct ctgccctccg ctcccctccc cgggctgtgc gccggacccc ggccctcgga 420

gcggggacgc ggccaggacc gccgagggag gcgcctgcga ggaagagctc ggccgggtcc 480

ggagactgct gcctgggacc gcgctcccag cgcctgggcc tcggtgtctc cgggccaaac 540

tgccgacata atcgcatctg ccggcatcta ttttcggttt atttcccccct cattgcgaag 600

gatttgcctg gccaactttc tgcgcaagat cccacgcaat tcctgggacc ccagaagaca 660

ggtcctgttg aagaacagga atctggcact gggtgggctg gggaggaagc cgcacggtgt 720

taaatccata aacaggaaga gaaaccagac agcgaaacca agaggcgaat gggcgattgg 780

atgccggtgg ggagaaggcc gggggcgcac cctgctcctg gactccagta aagggaggcc 840

gggcagagtc cctggggcgc cacctccccc tcggttagta gccctggagg ccggggggag 900

ttggcctctg gggagcagtg ggtgctgggt gtggggcgtt gcaggcaggc tggggtgggc 960

gacccaggtg gaagtgaatt gcacttggct tcctggtggg cctctgtcac cccctcccca 1020

ggcgctgaga aagccagcag gctggcaaag aaaaggaccc tagcgcaggc cccacactcc 1080

tcctcctaac ggacgagaga ccccccaaac ccactggaga agtgacgctg tggggttcaa 1140

atgcagacct ggcacctttt tgtagcctgg aaaaacattc ccactgcctg ctgccggagg 1200

agaggatagc tgagatgcac tctctttgaa tccaaacgtt caggaacgta aggcgaagag 1260

gcctaagagg gcgttggctg gctctgtctc tcaggctgga gcacagtggc gcgatctcgg 1320
ctcactacaa cttccgcctc ccaaattcaa gctattctcc tgcctcagcc tcccgagtag 1380
ctgggattac aggtgcccgc caccacgccc agctaatttt tgtattttta gtagagatgg 1440
ggtttcacca tgttgaccag gcagatcttg acctcctgac ctcaggtgat ccgcctgtct 1500
cggcctcccg gtgagtcacg gtgcctggcc aagaactgtt tcttgttggc tctggtgctg 1560
gtgacttaga acccgccagc tcctggagaa aggggctggg ccgcccaccc tgtgtagctt 1620
tcccaaagac agagtcaaac gtctcctgga gaacagaggc ttcccttcgt ctttggtcat 1680
ttgtcctcta gctgggggta ccccctggtg gaaaggcaca ggtcccttgc tccccaggtg 1740
gcaacgcagg ccagacacgg ccctggcaca gctctcctgg gtgttggctc aggacagccc 1800
tgtttccaac tggttaggcg gtgaggggtg gtggcccttt ggttccaggt tgaaactgcc 1860
catgtggtgc tgatttagca gactggggag gctcttttg taggcaggtt cttttctttc 1920
cccagctgct ggacctggga gttggaagag aagttgcacc cattttaggg gtaacagata 1980
ttttctgttg ctcttggttg gattgggaag tgaattgaag ggaggtcacg tttcaggggt 2040
gccttgggat gtctgtcagt gattttcttt tctttcttaa tttcttttc tttcttttt 2100
ttttttttt gagacacact ccctctatcg ctcaggctgg agtgcagtgg tgcgatctcg 2160
gttcactgcc acctccgcct ctcatgttga agcaattctc ctgtctcagc ctccctccca 2220
agtagctggg attgccagtg cccatcacca cacctggctt ttttttttt ttttgtattt 2280
ttagtagaga cgggctttca ccatgttagc caggctggtt ttcgaactcc tgatctcaag 2340
tgatccgcct cagcctccca aagtggtagg attacaggca tgagccaccg cgcggtggag 2400
gggtaatttt cttaaatctg gtaatgagtt gtggttgtgt agagtaacat accgtccttt 2460
cgagatatgg actgaaacat tgagagggag gagttacagg tatgtcgatt cttcttttct 2520
ctctctcctt ttttttttg aggtggagtc tgactctctc acccaggctg gagtgcagtg 2580
gcaagatctc agctcactgc aactccgctt cctgtgttca agccattctc ctgcctcagt 2640
ctctcaacta gctgcgatta caggcatgtg cctccacact cagctaattt ttttattttt 2700
agtagagatt ttttgtctct cctaaaaaaa tccaatgtaa aaaaatccca atgtgggggt 2760
tttgccacgt tggccaggct ggtctcgaac tcctgacctc gtgatccgcc cacctcggcc 2820
tcccaaagcg ctgggattac aggtatgagc cactgcgccc agtctgctgc ctcttttcaa 2880
tggtctggcc taaggaaatt attggaaaca tgtgcggttg agtgatattt actgggcact 2940
tccacatggt ccatgtaaag ggagatggtt ggggtgacag gcagttgagt ctaggggagg 3000
catgtacaga tgtgctgtgc ctctgggata tcagggtggc aggcagcagt ctctacgtct 3060
ggtcccaggc tgcctgagaa agagcatgtg ggaggcaaac cttgcgccct ggcatggttg 3120

ttaatgttta tatttaccct agcttgtgtg gggtaggagg tttagggatc aaattccact 3180
ctgtgtttag acattttttt ctttcttttt ttttttgaga cagtttcact ctgtcaccca 3240
ggctggaatg ccgtggcaca atctcagctc actgcaacct cacctcccag gttcaagcaa 3300
tgttcctgcc tcagcctccg gagtagctgg gattagaggc atgcaccacc atgcctggct 3360
aatttttgta tttttagtag agacctcaaa tgatctgccc gcctcgcctc ccaaagtgtt 3420
gggattacag gtgtgagtca ctgtgcccag ccatgtgttt agacttttaa ctaatctctt 3480
ttttagtttc aagccttatc gtccgcctct gtagaccact cctgtgcctg tttcctgatc 3540
cttccaaggg ccattgtatt ccctgtctgc tgcccctctt ttggattctt ctgcacattt 3600
ttttgttcat gcattcattc atttattgtt tgattaatga cagggtcttg ctttgtctcc 3660
caggctggtg tgcagtggtg cgaccacggc tcacggcagc ctcagccacc cagatgtaag 3720
cgatctggtt cccacctcag cctcccgagt agtaagtagc tgggaccaca ggcggtgccc 3780
aggttttttt tttttttttt tttttttcg ttggtagaga cagggtctca ctgttgccag 3840
gactgctctg aaactcctgg tttcaagtga tccccttgcc tcctcccact taggccttcc 3900
aaatgctggg attacaaggc atgagtcacc tccaggcctt tttgtacttt taaaactctg 3960
catcagtgta taaacaatgt tattaaagtt tatatgactt cagttacact acatggatcc 4020
tttttcactc acggttgtga gatttatttc tgttgctaca tccagttcta gtccatttgt 4080
gttaagtgcc cagtgtgttt atctactgag ggacagttat gttatttcgt gttcactatt 4140
atcccatgct acaataaata tcctgtgtct cccagatact tagaagagtt tctgcagggc 4200
acatgtggga gagtttgttt ctgggtcatg aggtgtgttc atcttccatc ttgctagatg 4260
ctggcaaagg gttctccagt gtggttgcat caatttactc gcccagcagt gtgcagagtt 4320
cctgttcctc acatttacca acactagata gtaccagact ttgatttttg ccaatctgat 4380
gggtttgaag tggtacccct gttttaattt catgaccaga aattcaaatt taatctttgc 4440
tgtaggacaa cagtaaccca cttatgccta gtgttccatt attagaacgc taagcatgtg 4500
ggagttttta catcatactg ctcaaggtca tcgccaaggt ctgatgtttt tactcgtgca 4560
aaaatttaaa aaattgcaac ctctggcata aatgggttga gtgacacttt tcctgttttt 4620
attgttggtc agtgatggca tatttgctgg gtttttttgt tttttttttg aaacggagtc 4680
tcactgtgtc gccaggctgg agtgcagtgg tgtaatctcg gctcactgta acctccgcct 4740
cccgggttca agtgattctc ctgcctcagc cccctgagta gctgggatta caggcgtgtg 4800
ccaccacgcc cagctaattt ttgtattttt agtagagacg ggatgtcact atgtttgtcg 4860
ggctggtgtt gaactcctga gctcatgatc tgcctgtctt ggcttcccta agtgctgaga 4920
ttacaggcct gagccaccgc tagcctattt attttttatt ttaaatttta attttctata 4980
tagagacgag gtcactatcc tgcccaggct ggtcttaatc tcctgggttc agtcaatctt 5040

cccaccttgg cctcccgaag tgtcagaatt atagatgtga gccactgtgc tcagcccaga 5100
actgatgttt tctaaatgct gggtgctgag aaggatgtgt ggctggcagt cttgactgtg 5160
ttatctgtct ttaccaggcc agtaacttct ttggtctggt catcaagata atctagcatc 5220
accagcaagc atgcatggag aaggatgggc ccaatgtggc caagatggta acgggaccag 5280
tagagagccc tgtagaagac atctagatat tctgccctaa gagcccggag ggccgggctg 5340
tctcatgacc ctctgacgtg ctgacctgga ctctggcaga atgtgcacac acacagtcac 5400
acagcttcct ggcttgcgca agtcccagga gggcggtgcc agccacaggc ttttcccatt 5460
cgagggttgg aagcgtatca tcaaaccaca tcagagtgct gggggccacc tgccacccat 5520
tcccaaccca ctcagccttc ctggtgtttg ggacatgctt tgctttggca gtcaagacag 5580
cagaacaaat caacttttaa ggccttgtca ctgatagtac aatttccatt atttttcatc 5640
caaattagga tacttctgaa aatagaaatg atgactctgg gatgcaaacg ttggctgtcc 5700
tatgtataag gagatggctt ttcacgctcc cagtgactga ggaagtttct cccagatggc 5760
gctgctctga gcctggtgca gggtaggcac tttcaaaaga gtgtctcctt gtatcttcca 5820
tcagccttgc gagatgggta tctgttccca gggccccaag ggaggaaaac aggacctagc 5880
tggatccaag agctaggcct ttcttttttt tttttttttt gagatggagt ctgactctgt 5940
cgcccaggct ggagtgcagt ggcgtgatct cggctcactg caaactccgc ctcccgggtt 6000
cacgccattc tcctgcctca gcctcccgag tagctgggac tacaggcgcc tgccaccatg 6060
cctggctatt tttttgtatt tttagtagaa acggggtttc accgtgttat ccaggatggt 6120
ctcgatctcc tgacctcgtg attcgcccac ctcagcctcc caaagtactg ggattacagg 6180
cgtgagccag catgcccggc ccagagctag gcctttctgt ggctggcctt cgcgtcagcc 6240
tcaactaccc tggtgtaatc tcgcctgcgg ttgaattagg gaaccgccgt gttctgcaag 6300
ctggagaggc agaacactaa tgagcagaac actaatctca ttgcaatctc aaaggatctc 6360
taaaagcttt tataaagcag gcccaaggtc ctttggtatc cgatgcagac gtggtgaatg 6420
cattggctct gtcagcatct gagcaagtca gtaacagaaa tggggagtaa aagctttcag 6480
aactttccag aatattgact aaattgtctt gtttacaacc aacaacgaca acaaaaaata 6540
actgctgagg gccttcgtag tgtctgctgt ttcaagtgta cagtagtcat tttgtctgca 6600
ggatgtgggg ttgctgtggc tgaccttgta caatattcca ctcataggtg tcttcaggcc 6660
tatggagagc agcttgcgtg ggctgggcct gcagtacctg gtttgcatag atgattggca 6720
ggtgggcagc acggggaagg acctgtgagt ggccaacctg gttcaggtga gggaggtgga 6780
gtggggcttc tctgcttccc ctggttccct ggaagcctcc aaggctggtg agcatcactg 6840
ctgcctctgc acacctgtgt gctgggtggg ttttctgaca ggttttcagt tgcttcgggg 6900

ctacagctgc agggagcctg ctccatggga cagatgggcc tctggtgccc gttcatcagg 6960
ggactgatga gaccgaggcc tgagagccct ttggattttg tttttgtcct taatttaatc 7020
ataagccaag aatctactaa acacagttcc attaggggca aagacgtaac acatcagagg 7080
ccacagcaag gctgtgattc atactcaaaa aggaaaggtc tctgggtcac aacagagcat 7140
agttgaggtc agcacactcc cacccagtgc agggctgctc cagcattgag gtgtgtctgg 7200
caggttgaag taggggaaga tgaaactcgc cgaagtcttg ttttgtggtt gcacttaagt 7260
ggtcaaaact tcaggagcaa ctgccgttat tagcggtgag tgccaagact agtttttata 7320
gaagagaaag aaacaaagta ctctgggaag gtcttactga gccttcacag tctccccacc 7380
tttccactgt tcccgtgctc ttagccgctc tgctggccta taaggcacag tcttcatttg 7440
tggcttctgg caaaatgtaa gcacttgact tttgtttttg ttttgttttg ttttgttttt 7500
ttgagacgga gtttccctct tgttgcccaa ggtggagtgc aatggtgcga tctcagccca 7560
ctgcagcctc cacctcctgg gttcaagcaa ttgtcctgct tcagcctccc gagtagttgg 7620
gattataggt gcacaaccac cacgcctggc taattttttg tatttttagt agaaatggga 7680
tttcaccatg ttagccaggc tggtctcgaa ctcctgacct caggtgatcc acctccttgg 7740
cctcccaaag tgctgggatt acaggtgtgt gccactgtgc ccggccaact ttcaattctt 7800
tagagctgac tatgagagga gccagcagta tagccacagc accaacgaat gaggaagagc 7860
aaaatactgc atgacagctt tgctaagaat tctttcactt tttttgtcta tcagccagga 7920
gctagcaact tggcttattt ggaaatttta agtgtacata tcctgtctcc ttaaatcctt 7980
tacagattta aagtgcagtc tacctgaggg ctctgtgacc atgtaagaaa gctttttctt 8040
tctttttttt tctctgagac agagtgttgc tctgtcgccc aggctggagt gcagtggtgt 8100
gatcttggct cactgcaacc tctgcctcct gggttcaagc aattttcctg cctcagcttc 8160
ctgagtagct gggactacag gcagcaccac catgcccggc tgagttttgt atttttagta 8220
gagacagggt ttcaccatgt tggccaggct ggtcttgaac tcctgacctc gtgatccgcc 8280
tgcctcagcc tcccaaagtg ctgggattac atgcgtgagc cattgtgtcc ggcctttttt 8340
tttttttttt tttttttttg agacagagtc tcgctctgtt gcccaggctg gagtgcagtg 8400
gtgtgacctc agcttactgc aacctccgct tcctggattc aagtgattct cctgcctcag 8460
cctcccaagt aggtgggatt acaggcaccc accaccgtgc ctggctaatt tttgtatttt 8520
tagtagagac aggagtttca ccttgtttag tagagacagg ctggtctcga actcctgacc 8580
tcaggtgatc cgcctgcctt gacctcccaa agcgctggga ttacaggcat gagccactgt 8640
gcctggccag aaagccttct ttattgagct tggtggcagc ccaaaactga ttctttaagg 8700
gtgtcaggac ttaacacctc ctgtgactta gccgcacctc ctctcctttg actttcattc 8760
cacctccttc caggatcgca aggtccctat ttgtcctgga aacggcttca aggtagtcta 8820

gggtgccgtt tgccggggga ggaaggtgct ctggttgata gagtcgcctg gccgcacact 8880
ctttttggca cataacaacg ttctacagag ccggggtgga gcgtgctttc tcataagtgc 8940
tctgcaggtt tggagagaga ggatatgagg agcacccttt tctgtttttt ttaacccaaa 9000
gattagcttg gaaaaggggc agaggggtgc actggaactc aggtctgcct aagcagcaca 9060
gcagaccaag gtctagagat gacatctgct cgcagctgtt cttccaccag cccgcatcct 9120
ggaaaggggt cttgtggcac acaagagttc acatccttcc ctcgtgaaat aaggactttg 9180
tgttcatcat ctcttgtaag aagcagagca gaaagcacag aattaagaaa taaaagggaa 9240
gtgggtgcct atataaaggg aagtgaaaat gggttgctgt cccatgcaaa gaccctggaa 9300
agctgttaac agctcagctt gtcactttca ccatctgcat ttgtccagag tgattgagat 9360
ttgcgttgtt gtggagagaa aggcgcctgt tgcacaatgg agtgagattg ccactgctgt 9420
caggacctct gtgtttggct tgacactttt tgagttctca gcagtctcgg gaccctcaag 9480
agtggaagca tttttggatg ttaaatgctg gggttaattg aagttaagag cttgttttac 9540
tgggcatggt ggctcacacc tataatcccg acactttggg aggccaaggc gggcagatca 9600
cttgagtcca ggagtttgag accagcctgg gcaacatagc aaaaccccat ctctacaaaa 9660
aatacaaagc tgggcgtagt ggtgtatgcc tgtagtccca gctccttagg aggctgaggg 9720
gagcagatct cttgagccca ggaggcagag tttgcagtga gccatgatcg tgccactgca 9780
ctccagcctg ggcaacagag tgagatcctg tcttaaaaca aacaaaaaaa acaaacttgt 9840
tttcatttag actcttcctg gcgttgggga cctattggaa taggtttagt gtgaactgag 9900
agctagaagt gttagaggag agagggaggg aacagagccc gctggagcga gtgcccttcc 9960
taccttatca ctgcatgcca ggcatgtgcc ggcgctttgg tcctcctcat ttcattcttg 10020
actgccacct gagacacgat ggttactagc tccattttat aggtggtgaa actgaggctt 10080
ggggaaggtc agaccccaag ggtgccattt agtcagtggc agagccagat ccaaatgcag 10140
gtctcctgac tccaagtgca gggctcattt tatcgtccgg ttgcagcacg ctggcggccc 10200
cttgagcccc aacctggata ccatagggga ggagcagaga agccaggaac accacagccc 10260
tgggccaagg tgcggggctg aaagaacttc ccagcgctca gcctgggact agtggaatgg 10320
gctgggccct ggggctggca gcggtggccc cggggagcct gggaatgagt agggagcaca 10380
gggaggtgtg ggagggcctg ggaaccatga aaaggagggc gggtgcaggg aagtcgcctg 10440
ctagtgaagt ggcgaggggg ccctgtggga ctccagggaa tggccacggc aggttgtcct 10500
ccaggagttg agagccactg gacatggcag ctgcctgtgt tctcagccac cacagtaacc 10560
aaagaaatct tggttttaaa attcaagttg ccatggaaac gctcccatcc tcgacttggc 10620
ttattattta aaataacatc tctacagcac aaagcccccg ggtacatcca aggacactgc 10680

tgtctgccca cgagacatgc taacctcaca gtgtggaggc tgtgtgggtc actgacatgc 10740

atggccacgt gagacgctgc ctacccacga gtcacggaaa aggggaagat tattaagaaa 10800

gtcactaggg gccaggtcca gtggctcatg cctgtaatcc tagcactttg ggaagctgag 10860

gcagttagat cccttgaagc caggagttca agaccagcct ggccaacata acgaaacacg 10920

gactatacta caaaaattag ccaaacgtgg tagcacaggc ctgtaatccc agctactcag 10980

gaggctgagg cacaagaatc acttgaacct gggaggtaga ggtttcagtg agccaagatt 11040

gcgccactgt actccagcat gtgccacaga gcgagactcc catctcaaag tcactaggga 11100

ggaagcctca ttggtgggaa ggaagaccaa attggaaatg ctctgaggaa tcattaaaac 11160

aaatgtcctt ttatcagttt ggtggctcag ggcctttagt aatactgcca actatttttc 11220

ctagaagaaa caaaactgaa ataataggaa catactcact tttttttttt tcttaaaagt 11280

aagggtatgt tgtgaaaaaa agtctcccca ccgtagtgac cgactgccgt gcatcttcct 11340

tggcattttg catgtagtgg caggagtgtt cctacatgtg tagattgctg agagggtcag 11400

atgcttatgg tcctcagtca cccacagctt gctttttccc cacttaacat tgggacttgg 11460

ggcattttta ctctgttaat acaatagaat tcacttcaac tagttggttt tttactttta 11520

ttttattatt attattttta gatgaagtct caatctgtcg tccaggctgg agtgcagcct 11580

ctgcctcctg ggttcaagtg attctactgc ctcagcctcc caagtagctg ggattacagg 11640

catgcgccac cacgcctggc taattttttg tatttagtag atacagggtt tcaccacctt 11700

agtcaggctg gtctctaact cctgacctca ggtgatccaa ccgcctcggc tacaggcatg 11760

cgccaccgtg ccccaccaac taattgcttt tttaatggtt gcttcatatt ctatttaacc 11820

acttaccgta atttaacagt tactctgtta ttggatactt gattcatttc caggacatgc 11880

agatttagaa actggtgggc gttgctggtt gatgtctcgg tggttgtgcc cacccacccc 11940

aggcactcat tatcatgcct tccgtttccc cacttcctaa gccctgcaag aagtgccaaa 12000

ctgtccagcc cttgccaatc tgatacatgc tgaatacctc cttgatttta tctgcacctc 12060

cctgaggttg aacttatttt actttatttt attttatttt tgaggcagag tctcactgtc 12120

acccaggcag gaatgcagtg gtgaaatctt ggctcactgc aatctccacc tcctgggttc 12180

aagtgagttg aagcaattct cctgtctcag cctcccaagt agctgcgatt acaggcacct 12240

gccaccacac ctgggtaatt tttgtatttt tagtggagac ggggtttcac catgttggcc 12300

aggctggtct caaactcctg acctcaggcg atccacctgc ctcagcctcc caaagtgctg 12360

agattacaag cttgagccac catgccggtt gaacttattt ttatctgtgt tggccatttg 12420

tagtttttct attatgctgg ttccattttt ctgactttga agagcctttt gtggaaacga 12480

agcctaaagt atatgtgagt actgctttgt ttcgtcagtt tttgttttaa agagactttt 12540

cagtgtaatg caagcatttt cccttgaagg ctgtgtttcc tgtcaatcct aagctttcct 12600

ccagcattac ctttttaaaa aactttattt tctatattga tggggtctca caatgttgtc 12660
caggctggtg ttgaacacct ggcctcaagt gatccacttg ccttggcctc ccaaagtact 12720
gggattatag gcatcagcca ccgcacccag cctgtttttc aaagggcatt gatttttttc 12780
ataaaacttt ttaaattaag atctgtgggc ctggtgcggt gcctcacgcc tgtcatcgca 12840
gcactttggg aggctgagtc aggtggatca cgaggtcagg cgttcgagac cagcctggcc 12900
aaaatggtga aaccctgtct ttactaaaaa tatgaaaatt agctgggcat gatggcacat 12960
gcctgtaatc ccgctgctcg ggaagctgag gtaggagaat agcttgaacc caggaggcag 13020
aggttgcagt gagccgagat catgccattg cactccagcc tgggggacag agtaagactc 13080
cgtctcaaaa aaacaaaaca attctgtgtt gcatgtggta ctttttgtgt gtgaggagtc 13140
cagtgtgaag attcagactt caggcagcca cttgtacaag cactgtcctg tttcctctct 13200
ggcctcacct aggtaacgct gattcctcca cggaggatgt gcttctgagt ggtccgttgg 13260
gtgctgtgct gatgagcatc acccagcatt ttacgacaca tgtgctgccc cagagggctg 13320
ggctcccgtc agagctcttt tccactggct gggtgcggtg gctcacacct gtaatcccag 13380
cactttggga ggctgaggcc agtggatcac ctgaggtcag gagttcgaga ccagcctggc 13440
taacatggta aaaccccatc tctactaaaa atacagaaat taggtgcgtg tggtggcgca 13500
cacctgtaat cccagctact tgggaggcta agaacctggg aggtggaggc tgcagtgagc 13560
caagattgtg ccactgtacc ccagactggg tgacatggcc cacagaccaa gaccctgtct 13620
caaaaaaaaa aaaaaagctt ttttccagag ccatcttgct gtcctcatat atttattctc 13680
ctagatgaat tgttgcttaa gcaacaatga agtttaaatt gttcttcaga tagaacccct 13740
aggtgccagg cagtttgtta agcactgaca ccgcagtcct gtgactttca tgacgacctg 13800
tgacctgtgg gaggtagaac acctcaccca cccgtgggag cccctggagt gactgacagg 13860
aacccctgcc tcacccagct ccccgcgccg cggctctccc cagccctgga tgcaggcgcc 13920
caggagacat gtattgcttt tgttgagctg ctcacttagg agggtgactc agagttcaag 13980
ctggaaagca ctggcttgtg actcatgagc cagtgcaaga tcaaacggct ggggcatcga 14040
ggtgagagtt ttccttctca gaagcctcat ctccgcagcc ggaagcagag cccttggctg 14100
actggaaaaa ccagagaggc cccgggagtg ggtggatggc cagcccagcc ccacctctca 14160
gcctcaacct ccaccagccc acaccgagct tgtttgtctg tgtcctgtcg aaactaggac 14220
tcctggattg taacttttct tacatttccc tgtcccctgg gtcctccact tagggtgtaa 14280
tcacacagac aggctcttaa ctgtttcata tcactcactt gggaaagtgt ctcaagctgt 14340
tctacaaatc catgcaaagg ccgtttaaaa atagcagcga aggccctgga ctcggtctcg 14400
tccagcacag ccccttggct ctctctctgg gctctggccg cctggccccc ggggacccac 14460

acgaggtcat ggcgtgcttc gggcaggggg gcggggatcc catagacacc tcagctcctt 14520

aagagttctc cgcctgggcc aggacgagca tgggggtccc cactgatgcc cgagacggtg 14580

cccctgtgtg tgtgagccct cgacccacat aacagagagg tgtcctgatg ccctctgtcc 14640

tctccaggtg gatctaggat ccggcttcca acatgtggca gctctgggcc tccctctgct 14700

gcctgctggt gttggccaat gcccggagca ggccctcttt ccatcccctg tcggatgagc 14760

tggtcaacta tgtcaacaaa cggaatacca cgtggcaggt aggctgtggg gctgcgtcct 14820

atgtatgtcc cctcccaggt cggtctgtgc acactgactc caggaatggg aagccagccc 14880

tcactgtggc ccacagaaca ttgtcctgga ctgttgaaaa atggccctga ccctgagtca 14940

tgtcggctct ggaccacgcc tccctccttt gtcccatacc ccacgtgtgt gcatgagtgt 15000

gtgtgcatgt gtatgaatat atggttgtgt gcacgtggag gtgtgtgggg gagtaagtga 15060

gcttcggaag tgttcaaaac cagctagccc ctgtgacgcc cgccgtggca cagtggcagt 15120

ttatcagtcc cgcccctgat tccctttgtc tctgcccagc cctgaccctc ggactgagaa 15180

ccaagaatga ggagtgagcc atgttgaggg ctggagaggg tctctgattg tcagcaaatg 15240

ggagcagatc aggggagaca cccactctgc ccgtgtgtca ctctgggctg ttccccagtg 15300

ccccccaacc cttgggccct tgaattgggt agggtctctg gttttccctg ggttgggctt 15360

cgtgtgtggg cagcgtgccc atccaccgcc atcccgggag acccttcagt cagtgggtga 15420

ctctcttcca ggccgggcac aacttctaca acgtggacat gagctacttg aagaggctat 15480

gtggtacctt cctgggtggg cccaagccac cccagaggtg agtgcctgct cctctgcacc 15540

gctgtaatgt gagtggcagg cgttggtttg gggcagtggg aagtgggaga gtgaaggcct 15600

ctctggcgtc cgcagggctc atgctgccca gggctgccga caccgcttga ggtacagtac 15660

ttgtttcttt tcatttttat attactttct ctttttattt tattttttcc ttctcaagtt 15720

attagtagag aagatgctgg tgcttttttt tggtttttga gagagtctca ctctgtcacc 15780

caggctggag tgcagtggca cgatcttggc tcactgcaaa ctctgtctcc cgggttcaag 15840

cgattctcat gccacagcct cctgagtagc tggggccaca ggcttgcacc accgtgcacg 15900

gctaatattt gtatttttag tagagactgg gtttcgccat actggccagg ctcgtctcga 15960

actcttggcc tcagcaagtc atctgcccaa ctcggcatcc cagagtgctg ggattaccgg 16020

catgagccac ccccagtgtt ttctattgtg cttctccaag gtgtctcagc cgcagatgtg 16080

acagacaagg tctgggtccc cactatgtgg gcagactcaa tcttgagtaa tttaagggaa 16140

atctaagaaa agccagatga ggccaagtat ggtggctcac gcctgtaatc ccagcacttt 16200

gggatgccaa ggcaagcgga tcacctgagg tcaggagttc gagaccagtc tgaccaacat 16260

gatgaaaccc cgtctctact aaaaataaaa aactttgcca ggcgtggtgg cgggcgcctg 16320

taatcccagc tactcgggag gctgaggcta gagaatcgca tgaacccggg aggcagaggt 16380

tgcggtgagc tgagatgatg ccacttccca cgtgaggatg gggtagttaa gttgcacgac 16440
actgccttct gcacaatggg gatgatatga gaaatgatga gaggatcctg ggcagaggtt 16500
gaggcaggag agcagggcaa gaaacactat ctggactgaa taaacttatt taaaaatgag 16560
tttataattt gtggataaat tcatcttaaa aaatccatta cataagccta gaaagaagcg 16620
tacaatatcg caagtcaggc caggtgtggt ggatcatgcc tgtaatccca gcactttggg 16680
aggccaaggc aggcagatca tttgaagcca ggagtttaag accagcctgg ccaacatggt 16740
gaaaccctgt ctttactaaa aatacaaaaa ttagccgggt gtggtggtgc atgcctgtaa 16800
tcccagctac tcaggaggct gaggcacgag aatcgattga actcaggagg cagaggttgc 16860
agtgggcaga gatctcgcca ctgcactcca gcctggatga cacagtgaaa ttctgtccac 16920
tcccaccctc ccgtcccacc caaaaaaaaa atcaaaaggt gcattgagca acagaatttc 16980
cttttgtttt atgaaattac caaggacacc tgtctgcagc cttcttcggc atgtcttgtg 17040
ctcatttcct tgttagggga gtgtggccca gccaaagtgg cctttgcagg gattggggag 17100
ggagttgtgg gatcagagct tgtaatgaag accttccttt atccagagtt atgtttaccg 17160
aggacctgaa gctgcctgca agcttcgatg cacgggaaca atggccacag tgtcccacca 17220
tcaaagagat cagagaccag ggctcctgtg gctcctgctg ggtaaggccc tgctggctgg 17280
cggggaagcg ctggagagaa agtgggagca acactggaga gtcttggggg attcggggtg 17340
gggacaactc tgacaaggca agttatagaa actttctgag tcccagtttc catcagtaca 17400
aaaatcacaa tccctctggc catgaatgat ggcgaggatt aggtggagtg gcgggcagag 17460
catccagcag attgcaagtc cacgtgtaca ggtggcgaag cagctccctt tccctgacat 17520
gctggcccgt ccgcaaatac caggagctct cactgctact ctgcttcaag aaagcatccc 17580
tttagtgtca gtgagctgtc ttaattttgt catttaattg tggtaaaata cacgtaacag 17640
aaatgtaata atcttagcaa tcttcttttg ttttcttttt ctttcttttt tttttttttt 17700
ttttttgga gatggagtct tgctctgtca cccaggctgg agtgcagtgg tgggatattg 17760
gctcactgca acctctgcct cctgggttca agcaattctc ctgcctcagt ctccccagta 17820
gctaagacta caggcatgtg ccaccacgcc cagctaattt ttgtattttt agtagagatg 17880
gggttttgcc atgttggaca gcctggtctc aaacgcctca cctgttgatc tgccttcctc 17940
ggcctcgcaa aatgctggga ttacaggcgt gagccaccgt gcccagcaac tattttcaag 18000
tgtacagttc tgtagcatta agtacattca cagtgttgct cagccaccac caccatctgt 18060
cccccgaact ctttttcagc tcgcaagaca gaaactctgt ccccattaac accaatattg 18120
tagcccctgg taagccccac tctactttct gtctctatga atttgactcc tagggacctc 18180
atacaagtgg atcacaacag tatttatttt ctgggtgagc tgtgtttttt gttaagaaaa 18240

aaacacagcc aggtacggta gctcacgcct gtaatctcaa cactttggga ggctgaggca 18300

ggcggatcac ctgaggtcag gagtttgaga ccaacctggc caacatggtg aaaccctgtc 18360

tctactaaaa agacaaaact tagctgggcc tggtggcagg cacctataat cccagctact 18420

atagaggctg aggcaggaga attgcttgaa cccaggaggc gtaggttgca gtgagctgag 18480

attgcgccac tgcattccag cctgggcaac aaaagtgaaa ctccgcctcc aggaaaaaaa 18540

aaaaaaacca cacacacaca tacaaactaa tactacacat tttgcagatt tcagaaatga 18600

acccagtttc caggcagagg ttcacggggg tgcttctctt gctttaaaag ctgagttggg 18660

caaatcgttg aggcaggaga gtggggcaag tggctcatca cgcctctaat cccagcactt 18720

tgggaggcca aggtgggcaa atcacttgaa cccgggagtt caagaccagt ctggccaacg 18780

tagcaagacc ccatttctac caaagaaaat aaaagctgag tttgagcccc aggagcgtcc 18840

cctggtgttg agagatcagt tgcctacaag gtctgaggtg cccctgtggc cctttgaggg 18900

gactgctgca gagggcccag cccggacatg gcagcctcac ccggtggggc tcgttcctgc 18960

aggccttcgg ggctgtggaa gccatctctg accggatctg catccacacc aatgcgcacg 19020

tcagcgtgga ggtgtcggcg gaggacctgc tcacatgctg tggcagcatg tgtggggacg 19080

ggtgagtcag gctgtgcttc cacagcgggt ttagtgctga gagaccctgg gccccagctt 19140

ctcagtggag gggactttga ggacttcctg ggactgctgc gagtcagaag tgtttcgggg 19200

agactccgag agtctggcag gcagggcctg gcaaggctgc tgcttcctgg gaggtgcctg 19260

aaccaaggct ggcccggcag agctgtctga ggggtggcat cccagcaaaa cagtcagttt 19320

cagaaaatgt ggtggaatgt ctggcccctg gtctggtttg tgtcatcgca tgttcctttt 19380

tcctgcttgg ggaaccagtt tggggcattt ccttatgtgt agttagcggg gatggctcca 19440

ccttaacaac aaggtggtgg cacagaactt tctgctccag ctgcctgcag cctcgccttg 19500

gttccgcagt agcggtgttc actggcggct gcagatccga aatgcctgag ggcttaaaaa 19560

aatgcatggg cttctcccct aactagttaa atgggaatca ctggcgattc tcatttcaga 19620

gaactggtgg tgtttttaag taccttaggg aagtttagcg tctgctcagt tgagaatcag 19680

tgttctaccc ggaagggcac tcgtagcctg gtctggtatg gacatgaaca ggagagcctc 19740

ctgtcttctc ccggatcttt gtgggcaggg gtggggctgg cggttattcc ctgcaagctg 19800

tgcttatcta gggagcgtcc cttggagggt ttggggtctg ggaggtctgc tcgcaccact 19860

tgctgcagct gggggtgggt ccacgagtgg cctcgggcac ttgggtagca cacagtggtc 19920

tggagagctg gtggtgcttc tctcagaggt tttcattaga ggctgtcttt tcagctgtaa 19980

tggtggctat cctgctgaag cttggaactt ctggacaaga aaaggcctgg tttctggtgg 20040

cctctatgaa tcccatgtag gtaagtgtgt ccccttggcc actttctggc cagatggatt 20100

gtttgagcaa ttaaccatca tggctttatt tgcctttata aactgggggt tgagacagag 20160

gggctgctga gaggtgctag ccaggtgcac aggcctctgg caggacctgc ctggcgtcca 20220
tgctgcaggc acgaggcttg ccttgcccca ggtcttctcc gtgggggctg taggttgact 20280
ccgctttctc ccgcgtccca tcagggtgca gaccgtactc catccctccc tgtgagcacc 20340
acgtcaacgg ctcccggccc ccatgcacgg gggagggaga tacccccaag tgtagcaaga 20400
tctgtgagcc tggctacagc ccgacctaca aacaggacaa gcactacggt aaggggcctg 20460
gggcctggcc acggcgcacg tggaggctgg ggagctgctg catccctcct cacgctgcag 20520
cgaagaggtc agggctgagg agccttgggg tcccggaact ctaggataga ggagggggag 20580
tgatgccctc ttgccaggag aagcagcaca ctctccactt tctgcctgtt ccaccctgaa 20640
ctcagcctca gcccttccaa actggaaggg accaaagccc tccttttaca agggagtggc 20700
gtgtcctgga gtcttgaggt atcctggcct cttccggggc ctcgtgctca ttgctccgtt 20760
ctcccatttc tgagcttcca gccccagccc tgtccttagt tcttcagggg agccttcctt 20820
gagctcccct ggcagggcaa gtccccttag atgcagtctt cccctggagg ccaccagaaa 20880
tcagtgactg gctgaccgtg gcgtgccacg aggcacggtc actgccctcc ccaaccccga 20940
gctcggttca ttttccagga tacaattcct acagcgtctc caatagcgag aaggacatca 21000
tggccgagat ctacaaaaac ggccccgtgg agggagcttt ctctgtgtat tcggacttcc 21060
tgctctacaa gtcaggtgcg tgctgatggc tgatggcaat agagggtggg ggtcgggagg 21120
ggagcctggg tgccaggatg gttcatgttg accaataggg ctggattggg caggcaggtg 21180
aggggctggg gaagagggct gtgtggggcc ttccatatag gctcactcct ctgtggacca 21240
gcctggcacc tgactgtctt tgtccaggtg ttgtgccaac acacacaatt ccagaggcct 21300
ctcccagagc ctctgggcta gttccttccc atccattagc gtccagctca tacatgttct 21360
ctgtgggaac gccccccctcc ctggtggctg gccacgcctg ccctcgcctt cctggctttg 21420
cattgcttta ccagcatgtt tctgttataa tggcaggccg ttcgtgtttg cactggctga 21480
cacattttct tcatctttct ctttgcaaag gcctagatcg tgcctcacac cagtggatga 21540
acacacaggg aggaccttgc cctgggtagt gccataggga ctccaaccaa ggaatctgga 21600
cagtccccat ccccaagtcc tgtcttagaa atcccttgct ccagatgaac agtctttgct 21660
gactgcatct atcccattaa tattttgcta gattggaaac ttgtaaaaca acagtttgac 21720
aaaggaagat gattctggca agaaaaagtt tctgtgaaga tggcacttct tagcatagtc 21780
ctcgccctta ttattaaaag atgcatttcc actttgacag tgatttcccc acccgagagc 21840
ctggttgggc agccctgagc cccttagcca gtatctttcc catcagctag cattagctgc 21900
caccagtcct cccttctcct ccctcacctc ttcagggtgt acctggagca gctattaaaa 21960
ctctttaagc cgtgtaacgc ttttgacaaa cccttactta catggaatcc cagtataaag 22020

aacagatgaa tcagtcatct agcattaaat gttttatcaa ttagaatgta ttagaaattc 22080
tatttttaaa gccaacaagc acattttgta ggaatctatt tcaatattta ctcaacaact 22140
ttgagtgtgt tgggccttca aaaatatatt taattcccca gtattgtggg acccccccctg 22200
gagcctcctg gtggagcagc aggttccctc tggaagctgt ttctccttcc cggagcatga 22260
ccagctgagt gtggggggtg ctgtggggcg tgggacagtg gcccggtctc ggtctcagcc 22320
agttcttccc ttttcaggag tgtaccaaca cgtcaccgga gagatgatgg gtggccatgc 22380
catccgcatc ctgggctggg gagtggagaa tggcacaccc tactggctgg ttgccaactc 22440
ctggaacact gactggggtg acaatggtga gtggctgccc ccttcctgcc aagaacagtg 22500
aattgtgagc cacaccccgt ggccatctcg gctttctctg ttctacccac cgcacagcct 22560
taaaccctgg acctacggcc aggctgtgag ctcctcctaa gtgccaggca gtcaggagtt 22620
ccctttttgct gtgagggcag actctgagca gcttcagagc caacgcctgc aacagttccc 22680
acagcatcgc gccagatcct gtgatgggaa gggtgaccgg gcaggggggct tgcccgtgga 22740
ggtgtgcccc acggctccag aagccttgtg gtgttgagga ctgtgctagt gggccaacag 22800
caggagtggc cagggatgag tgacttaagg tcttttaagg atgagtctga ctatattggt 22860
tgacccttgt cacactttaa aagcacctta ctttttattc ccaggcttct ttaaaatact 22920
cagaggacag gatcactgtg gaatcgaatc agaagtggtg gctgtaattc cacgcaccga 22980
tcagtactgg gaaaagatct aatctgccgt gggcctgtcg tgccagtcct gggggcgaga 23040
tcggggtaga aatgcatttt attctttaag ttcacgtaag atacaagttt cagacagggt 23100
ctgaaggact ggattggcca aacatcagac ctgtcttcca aggagaccaa gtcctggcta 23160
catcccagcc tgtggttaca gtgcagacag gccatgtgag ccaccgctgc cagcacagag 23220
cgtccttccc cctgtagact agtgccgtag ggagtacctg ctgccccagc tgactgtggc 23280
cccctccgtg atccatccat ctccagggag caagacagag acgcaggaat ggaaagcgga 23340
gttcctaaca ggatgaaagt tccccccatca gttcccccag tacctccaag caagtagctt 23400
tccacatttg tcacagaaat cagaggagag acggtgttgg gagccctttg gagaacgcca 23460
gtctcccagg ccccctgcat ctatcgagtt tgcaatgtca caacctctct gatcttgtgc 23520
tcagcatgat tctttaatag aagttttatt ttttcgtgca ctctgctaat catgtgggtg 23580
agccagtgga acagcgggag acctgtgcta gttttacaga ttgcctcctt atgacgcggc 23640
tcaaaaggaa accaagtggt caggagttgt ttctgaccca ctgatctcta ctaccacaag 23700
gaaaatagtt taggagaaac cagcttttac tgtttttgaa aaattacagc ttcaccctgt 23760
caagttaaca aggaatgcct gtgccaataa aagttttctc caacttgaa 23809

<210> 23
<211> 21

<212> DNA
<213> Artificial

<220>
<223> Synthesis

<220>
<221> misc_signal
<222> (1)..(21)

<400> 23
ggagaatggc acaccctact g 21

<210> 24
<211> 27
<212> DNA
<213> Artificial

<220>
<223> Synthesis

<220>
<221> misc_signal
<222> (1)..(27)

<400> 24
ctgagtattt taaagaagcc attgtca 27

<210> 25
<211> 2205
<212> DNA
<213> homo sapiens

<400> 25
gcgcacgccg gccgcgccca cgtgaccggt ccgggtgcaa acacgcgggt cagctgatcc 60

ggcccaactg cggcgtcatc ccggctataa gcgcacggcc tcggcgaccc tctccgaccc 120

ggccgccgcc gccatgcagc cctccagcct tctgccgctc gccctctgcc tgctggctgc 180

acccgcctcc gcgctcgtca ggatcccgct gcacaagttc acgtccatcc gccggaccat 240

gtcggaggtt gggggctctg tggaggacct gattgccaaa ggcccccgtct caaagtactc 300

ccaggcggtg ccagccgtga ccgaggggcc cattcccgag gtgctcaaga actacatgga 360

cgcccagtac tacggggaga ttggcatcgg gacgcccccc cagtgcttca cagtcgtctt 420

cgacacgggc tcctccaacc tgtgggtccc ctccatccac tgcaaactgc tggacatcgc 480

ttgctggatc caccacaagt acaacagcga caagtccagc acctacgtga agaatggtac 540

ctcgtttgac atccactatg gctcgggcag cctctccggg tacctgagcc aggacactgt 600

gtcggtgccc tgccagtcag cgtcgtcagc ctctgccctg ggcggtgtca aagtggagag 660

gcaggtcttt ggggaggcca ccaagcagcc aggcatcacc ttcatcgcag ccaagttcga 720

tggcatcctg ggcatggcct acccccgcat ctccgtcaac aacgtgctgc ccgtcttcga 780

caacctgatg cagcagaagc tggtggacca gaacatcttc tccttctacc tgagcaggga 840

cccagatgcg cagcctgggg gtgagctgat gctgggtggc acagactcca agtattacaa 900

gggttctctg tcctacctga atgtcacccg caaggcctac tggcaggtcc acctggacca 960

ggtggaggtg gccagcgggc tgaccctgtg caaggagggc tgtgaggcca ttgtggacac 1020

aggcacttcc ctcatggtgg gcccggtgga tgaggtgcgc gagctgcaga aggccatcgg 1080

ggccgtgccg ctgattcagg gcgagtacat gatcccctgt gagaaggtgt ccaccctgcc 1140

cgcgatcaca ctgaagctgg gaggcaaagg ctacaagctg tccccagagg actacacgct 1200

caaggtgtcg caggccggga agaccctctg cctgagcggc ttcatgggca tggacatccc 1260

gccacccagc gggccactct ggatcctggg cgacgtcttc atcggccgct actacactgt 1320

gtttgaccgt gacaacaaca gggtgggctt cgccgaggct gcccgcctct agttcccaag 1380

gcgtccgcgc gccagcacag aaacagagga gagtcccaga gcaggaggcc cctggcccag 1440

cggcccctcc cacacacacc cacacactcg cccgcccact gtcctgggcg ccctggaagc 1500

cggcggccca agcccgactt gctgttttgt tctgtggttt tcccctccct gggttcagaa 1560

atgctgcctg cctgtctgtc tctccatctg tttggtgggg gtagagctga tccagagcac 1620

agatctgttt cgtgcattgg aagaccccac ccaagcttgg cagccgagct cgtgtatcct 1680

ggggctccct tcatctccag ggagtcccct ccccggccct accagcgccc gctgggctga 1740

gcccctaccc cacaccaggc cgtcctcccg ggccctccct tggaaacctg ccctgcctga 1800

gggcccctct gcccagcttg ggcccagctg ggctctgcca ccctacctgt tcagtgtccc 1860

gggcccgttg aggatgaggc cgctagaggc ctgaggatga gctggaagga gtgagagggg 1920

acaaaaccca ccttgttgga gcctgcaggg tggtgctggg actgagccag tcccaggggc 1980

atgtattggc ctggaggtgg ggttgggatt gggggctggt gccagccttc ctctgcagct 2040

gacctctgtt gtcctcccct tgggcggctg agagccccag ctgacatgga aatacagttg 2100

ttggcctccg gcctcccctc aaaaaaaaaa aaaaaaaaaa aaaaaaaaaa aaaaaaaaaa 2160

aaaaaaaaaa aaaaaaaaaa aaaaaaaaaa aaaaaaaaaa aaaaa 2205

<210> 26
<211> 11238
<212> DNA
<213> homo sapiens

<400> 26
gcgcacgccg gccgcgccca cgtgaccggt ccgggtgcaa acacgcgggt cagctgatcc 60

ggcccaactg cggcgtcatc ccggctataa gcgcacggcc tcggcgaccc tctccgaccc 120

ggccgccgcc gccatgcagc cctccagcct tctgccgctc gccctctgcc tgctggctgc 180

acccgcctcc gcgctcgtca ggtgaagcct caggggccgg ggctcaggga cgggcagggg 240

tcgcggcgcc gaggtcccgg ggcctgtggt gactttcgcg ctcccctgtg gcccccacga 300
gccccttgcg ccccccgcgc tggaatgcac ctgtgccgcc ctgcgcggcc tcctgcacgg 360
accacccgcc tacggggcgc cgggctccgg aggtgcaggg gacccggggc agaggcgcca 420
gatgcctctc ccccatatgc caccctgggt tgtaccttga ggactgcaga ctgaccgcag 480
cctccctgga gacggggcgg ggcgggggga ggtagtgctc attcggggca ggtggaattg 540
gggtctgtac tgagcgccct tgttgctgga gacctaggtc aggcctcaga gcccccgagt 600
ctgggcgagt ccatttcctt agggacccct ttaccacctg tgaactgggg gctttaaaag 660
tttgctccag cggctcttat cacaggccct gggctgggag acccctcgag accctaggag 720
ttcccatgtc cctgagagag gaggaggcat ggggagtggg tcggctcacc caccccgggc 780
ctggggttgt gctgtagtga ggcccacacg ctcctcaggc cgatcccctg tgccaggtga 840
ggccaccgat tgggcctgga tgggatgggg cccggccatg cctgaccagc tgggcagagg 900
agggccatgc tgcagtctgc tttcttgacc ccctccccag cccttgcaag gcagcccgca 960
ttcccaggag gggtatgctg acccatccca ttgggcacct gccccaccct tgctctgggc 1020
ctttgtggga gacctgggat ctgcgatggg tccactgcct tttggcaggt gggtgaggtc 1080
agaaggctgc aggggctgga gctggctggg ccagctgggt aggactgagc ctcaccaaag 1140
gctgtgggga atggcccggg ggcgggtagc cccaattaaa gtcgttgtgg gggagtagcc 1200
acaagcctga gcctgccttg accttgccag cctatccaca ggcctcccct ctccaaggag 1260
gacagacaca gcagagggga aacgatcctg gggcttcttg gagggaaggg tagctgaatc 1320
caagccctca cccgattcca gctcttgtgc gactgatact attacacctg cttcctggtc 1380
cctggagggc gtgtccctcc cccaggacaa aacctggagc tcttccagcc caccagctct 1440
taggcaataa tctcatcttc cgggatcacg cccctgacaa gccaggaaaa gccagctatg 1500
accttgtact ctcaagtccc tggggcaggg aagaggtttt atttaagtga ttaaaagccc 1560
aggggagctt ccttggaaca aggagtgggt tcacaccaag gggaaggcca gtggccctgg 1620
gggaggagca gggacccctc tctctcttac tcgcttcctg ggtttagaac tcaggacccc 1680
gatctcagtc tggagctccc tcctgcaccc tggctggcgg tgtgctgggt gacaggactc 1740
tggaggggta ccctgagtgc agctgtcgga ggaggcaggg cggtgggggg ggcagcacag 1800
aagcctctaa ggccccaggt gcagtcctgg acctcgtgga gccgcatgga gtgaggagag 1860
gtgcggatgc ccagaaacag atgtgggatg agggcactgg gcagccacag ggtccatgtg 1920
gaggaggaca ggtagtcaag gagggcttct ggaggtggtg tggagggccc atctgatggc 1980
cagaggaggc caggcagagc tgccagtgcc agcctggagg tggggccacc ttcgtgcagg 2040
tgtctggggg tggagagcag gtgtgatggg ggctgggtac agtgggctgc ctcagagcac 2100

tttgggcagg agtgacaggt acccgccagc accctgagca gccatgctgg ccaccatcct 2160
ggaagagacc agcgcaggtg cagagggagg acgggagacc cttgggggct ttggagcctc 2220
cagaatggct gaggagagga gggttgggca cactggcccc caggttgggt gtgtggggct 2280
gaggtgggtg gcgggtgacc acttcttagg actgtggcct gtgcaacctg gcgggggca 2340
atgggttgcc attcactgac ttgggggagc tgggccagca gtttccaggt gacaggcagg 2400
agtttggttt tggctgtggc gactctgaga ttccccaggg gcctccaggt ggatgtgcag 2460
cattggcagc gtcggccggc aggcgggagg gcctccctga tatgccccga cccgtggttg 2520
acaggatccc gctgcacaag ttcacgtcca tccgccggac catgtcggag gttgggggct 2580
ctgtggagga cctgattgcc aaaggccccg tctcaaagta ctcccaggcg gtgccagccg 2640
tgaccgaggg gcccattccc gaggtgctca agaactacat ggacgtgagt atgaggtctt 2700
agccctgctg caagcggagc cactgtcagg agagctccgt ggcagtatgg ggaacattcc 2760
cacctcctgt tctcagcagc gcagtttgag tggctcacct ggccacggtg gcgtcccggc 2820
tcagccacac tcctttcttc cccattctgg aacctcctgc cactggcccc tgtcattgca 2880
gggtcttggt cctgctaggg cctgggaggg tgattgggag tggcctcggg ccttggtgcc 2940
agctcgcctg agggggcggt ccttgggcct gcctgagtgt ggccggctga cctggagcct 3000
ttcactgctg tcctgctggg aggcctgtgg tgactcgtgg cctccccagc ccctccccat 3060
cttcttcctc tcgcaacagc ccctcctgtg cccactactc cttcaggggg aagcaggatc 3120
caaggtggag cactctggaa gccacctagc aagctggggc ttggtcagcc tggtcccagc 3180
tctatggggt cagttcgagg ccagggccta ctgtccagcc tcggggctct ggcccactgt 3240
gggggagcct tgccctctgt cctgcttggc ccgagtcctg gctgtgacag gaagcccaag 3300
actcacaggc atgtgactgg gccagggggg ccctgggggg aaccaggcgc cgtgtgcctg 3360
gtcccgactg gccagttcct gattgtccta gcgcgcgagc aagcagatgc acgcatgcgc 3420
acacatgcac acacacacat ggaaatttgc tgagtgtccc cctgccagtg gtcacttctg 3480
tggggccatg agtcagcagc tgctgctgct ccgtgaagcc aggcggttgg agaaacattg 3540
ggctgggctg gtgccaggaa tctggtggct gacggtggcc caggctccaa tcctggggga 3600
agcgggcgcc caggcgaccc caaactccag gaccctctta ctctctgcct cctgagaggc 3660
tcggcggcat gggacccctt gtacttgcct gatccctgag tcagcacccc cacctgcgcc 3720
tgctatccct gtgtacacac ggggaaactg aggccctggg ccattaaggc agggttgctg 3780
ggcaggcagt gattgtaagg cttaccttct ccccttgacc agctgaaccc ctctgcctgg 3840
aagccctcca agcctggggt tcaccctgga gggcagggca ggcactgaga cacccagaat 3900
cagaccttga catggcccca acctggggag gaagtcactt cctttctcag accttggact 3960
gcgggccacc aggggagtct tccaggccgg gctttcctgc acccggggct caggctgagg 4020

gccacgtctg tccccaccct ggcttgacct ctggccttgt cttttccgtg gggaagtcct 4080
cagcctcacc atgtattgag caggggtagg tgacagaagc caggggtcta gagaccccaa 4140
gtacccgtgg gcattaggac cgagggctgg ggacctggcc catcttccct gcaggctgag 4200
caggtgggag tgggtggctg ttggcagctg tgggccccgt gatgcccctc cctccagtat 4260
gggccttggc tctggggaca gccgggcctt ctgaggccca gtggggagat ggggccccct 4320
ctcccatccc tgacggaccc tgtcccctgc caggcccagt actacgggga gattggcatc 4380
gggacgcccc cccagtgctt cacagtcgtc ttcgacacgg gctcctccaa cctgtgggtc 4440
ccctccatcc actgcaaact gctggacatc gcttgctgtg agtcacgaac cctggccccg 4500
tcgcccaggt cctgcccttc cgggatgtcg ctgcagggct gcctcaggaa tcacttgggc 4560
aacgcaattc tcctgcctct tggccccgtg agccaggcca gccctccacc ctgctccagc 4620
cactgacttt ctgggtgaac caccagctgt ggtcttgctc tcagcagggc tggggctggg 4680
gtggccacag agggagccgg ctgtggctgg gagggaggcc cggggtcaca gcccacagtc 4740
ccggggctct ggcatgatgg tgggcctcag atcccctcca aatcccaccc tggggaggca 4800
gcttgggggg tgcgggctcc atggtagatt gtagggggat gggagggcta aggccgtggc 4860
gtgggctgcg ggatgaccgg cgggccccct tgtcgcccgg ggcagggatc caccacaagt 4920
acaacagcga caagtccagc acctacgtga agaatggtac ctcgtttgac atccactatg 4980
gctcgggcag cctctccggg tacctgagcc aggacactgt gtcggtgagt ccctctgggg 5040
cctttcccag gactcgaggg tgccagggtg tggggttcac ccaccgtggg tatgtggttg 5100
aaagggaggg ctcggtgatc ccagcccaac cccagccctc aggtggccgg ggcagtcagc 5160
agggtgagga ggggtctggg aatgggcctg gttgtgtgca ggctggaggg acagcctcaa 5220
acccaggggg tacaggggca ggggtccccg gagtcaggcc acaatgagtg ggagggagga 5280
cagggcagat cgatcgggct ctttttggca cattgggttt gaggtgccag caggtgttga 5340
gggctggacc tggggctgca cagggtccct gcagcaggcc gagggcttgg ggaggcttgg 5400
ggttgggagg atgtaccagg aacccgctgt gggggctgct ggcggagagt caccggcagg 5460
agcctgggag ggcaagggag ggggagcagc agtggctgtt gggggccccg cctgcatcca 5520
ccatcctcag ggccctgtgc agagcaactc cctgccctag gagagggtga gctggccctt 5580
gtcaccctgc ctgggcctca gtgagttctc acctcggagc tgtctgctgg gggtggacca 5640
ggccacaagg ggttcaggag ttacgggatg gtgacacagc cccagctct ggagcgggcc 5700
aggagggcag cagagccccc ctgcaggccc agggacccgg gaagagggcc ccttcctcca 5760
gctgaagctg ctcctaacag ttccctgctg ggctggagtc cagtctgtac tgggggctcc 5820
tcagagccct cctgtctgga ccctgctcag cctgaacagg aaattgcccc gtctgccctc 5880

ttccgccctc ttctgccccc tccgccccgt gtcagcctca agctgtcgtt cccttcccac 5940
atcctgctct ggccgtgttc tctctctgca gcctcatcca gggctggggg aggggacagg 6000
cagagaaggg gaagggggca gtatggctgt gagcttggga tggggtcggc aggttcccca 6060
tctctcgggc tcctggccca ggctgtgtct tgttcccagc gctgagggca ggagcaggac 6120
ctgcctgtca ttggtggtgg gagtaagagg tcggagcagg gagcggagca aggggcctgc 6180
ccgtctgtgc tcctgcctgg ttcccatctc gtgtaaaccg agccctgatg acttccacga 6240
agagggcccc gccatacccc gtgtccccat ccccagcggt gttcagctca gggtttccag 6300
ccctttcttc tgggccctct gggccccatc gtgtgtggga tgggcatcca ttgaactggg 6360
ttttgtagcc tcatgctcag ggagtggtgt agggctcagc ctgtctgctg cccactgact 6420
ctgccctggc ctgcaggtgc cctgccagtc agcgtcgtca gcctctgccc tgggcggtgt 6480
caaagtggag aggcaggtct ttggggaggc caccaagcag ccaggcatca ccttcatcgc 6540
agccaagttc gatggcatcc tgggcatggc ctacccccgc atctccgtca acaacgtgct 6600
gcccgtcttc gacaacctga tgcagcagaa gctggtggac cagaacatct tctccttcta 6660
cctgagcagg tgggcgtgtg ggttccctct cgctccgcgt tgctgggagg cagggcgggg 6720
ctggacgggg agccttctag gcaccccctc tcagtgctgc cccctccctg ctgctgtgcc 6780
agagctcctg acctctgacc tcagggcatc cgggaggcgg gggttggccg cccttctgca 6840
gaggagtaag cggcagcaca gagaaagctg tttggccggg gtctcccagt gggaggggct 6900
cgggccaggc tgtgggtcct gggaccttgg cagctgggcc tccctcctat gagaatggac 6960
ccaggtcagg ggtcgtggca gttcctcttg gttcagtcgg cccgtcggtc cccagacctg 7020
gtgatgggca catgtggtcc tggtcccggg gttgctgatg gtggagaggg tcatggtgcc 7080
caggggaccg gggatccccg gggaggtgac cttgggtgcg tatgggtccc cggcaccacg 7140
ctgcgagcag ctctgtgggc gtccccagca ggtgccggct gccctcgagg aggaacatag 7200
ggggcccttc cttctgggaa aagggttctc ccccagggcc cccacctgca gccgctgctc 7260
cagcttgggt gacccatggt caggtgacct tgggaagggg acgtcggact cagttagttt 7320
cctcttctcg gggcagacct gagtggaggg ttccacataa gaggggtgac tgggggttgg 7380
gggcttttgt tttgggggtg ggcagcgtgt cagccggcgg cctcctgccc tgagctgcgc 7440
ctggtcactg ccccacactc tccagggctg acaggggcag gtttacctca cgtggctcac 7500
ttggcacttg gagtctctgg gcctgacccc taaccttgga tcgcttccgg cagaattccg 7560
gctgtagggc tggctgggcc tctttgctct gccccttgcc tgggcagtga atactcctta 7620
gcaacaccca gggctaagct gctcactaga ggccaggaca caggtgaacc ggctggccca 7680
tttccccagg agccttgggg cacaggggag gcagccaggt gaaaaggggt cccctgaggg 7740
caagagggca tgcaggcatg agtgcccaca tggggagggg gcacacagcc tgagtaccca 7800

ggtcagggag ggggacacag gcatgaatgc ccatgtgggg gaggggcaca cagcatgagt 7860
gcccatgtgg gggaggggca cacggtatga gtacccaggt cgggggaggc atacacaggc 7920
atgagtgcca ggtggaagga ggggcacaca gagtgagtgc ccaggtgggg gaggtgcaca 7980
tggttgtgag tgcccaggtg tagtctcaag gcagcagctt gggcaggaag tggagccagg 8040
caggagggag ggtctgaggg cttctgaaag catgtttggt gaggagagga ggggtgggag 8100
gcgctgatca ggtttctaca cttgggattg cagaggtgtt gacaagaggc aaaggcggag 8160
gaggctggag gagggcggag gccccaatcg gtgttgggag gactgggtca ggcctggcac 8220
tgcctcgagt gacaggcagt gggatggtgg ccagcttagc tgcagacgct ctggcgggat 8280
ggcagaaccg ccccagacac agagagcttc tctaccagga ccggcaggat ttgctgcgtt 8340
gaaagctgta cttgagcaat gtttagaaac aaacccgggc gacatgggtt gcaggtccta 8400
ggaagtgcag tgcgctcctg cccaggagca ccttggctgg ccatcagtgg tctggatgag 8460
ggggagatga gcggacgtgg ctcggggatg caggtggagg gtgttcccag gagcagccag 8520
tgcagaggcc ctgcggccag aaccagccat cccaacttcc cagattgtgc catcactccc 8580
tcctggaagc cttctttggt tttttcttcc agacagacag acagggtcct ccccagtgga 8640
gctcctggca ctcacttcct tgtctgccgc tagccttgac cctgatgtct ggctgaacct 8700
tggctcctga gcagaccagg agaacctgag ggttgaggaa aacctcttct gggccaggct 8760
gggcccacgc agagcagccc taccccggga agaagggagt ctgtccctcc tcctgccttc 8820
tgggcctttc catccctgca gtttcagaaa ggcccctcct tcaggaagct ctccctgatt 8880
gccacattca ccctcttact cctgacctgg cactcttgtg cttgggggt ggcgtggcag 8940
ctgactcctc ccttttcctc ctagggaccc agatgcgcag cctgggggtg agctgatgct 9000
gggtggcaca gactccaagt attacaaggg ttctctgtcc tacctgaatg tcacccgcaa 9060
ggcctactgg caggtccacc tggaccagtg agtagtggct gcagtcggct cccctgggtt 9120
ctgtgggcgg gggcggtgtg cggagaccct ggaggacccc ggttctgcag gtgggggttg 9180
catgtgggga gtagtgggag ctgggcaaga aagagatggg gtcagaccag ccctccatgc 9240
ccctccttgc ccctccatgc tccccatcac ctccatcccc tctattccct ctatccctcc 9300
atccctccat ttcctccatg cctctgtgac tctccatgac ccaccatccc ttctgtccat 9360
ccctccatgc cctccatccc ctccatcccc tcatccctct gtgactcttc atgaccctcc 9420
atctcctcca tccctccatc ccctccatcc ctccatccct ccatcccctc catccctcca 9480
tccctccatc cccacatccc tctgtgactc tccatgaccc tccatcccct ccatctgtct 9540
atccctccat ccctccatcc ctccatgccc ctccatccct tcatcccctc catcccctcc 9600
atccctctat gcccctccat cccctccatc cctccatgcc cctcaccacc acctgagggt 9660

ctcccacccc ctctaccact ctgtgtctcc tctcccaccc tcttccctgg agggcttaca 9720
gccggctgtg cttccaggag ccctgagggg aggagagtgc agcccagcca ggggaggggc 9780
tcccagggag gggcactggg cccccagggc acactccagt cccggcaggg gcttcacgcc 9840
ctgactcccc gcagggtgga ggtggccagc gggctgaccc tgtgcaagga gggctgtgag 9900
gccattgtgg acacaggcac ttccctcatg gtgggcccgg tggatgaggt gcgcgagctg 9960
cagaaggcca tcggggccgt gccgctgatt cagggcgagg tgagcgccgg gggctggggc 10020
tggggctggg gctggcaggg ggagccccaa ggccaccact accaccctga cactgctgtg 10080
acccctctta gtacatgatc cctgtgagaa aggtgtccac cctgcccgcg atcacactga 10140
agctgggagg caaaggctac aagctgtccc cagaggacta cacgctcaag gtgagcgggc 10200
aatggggtgc cgcacgcccc aggtgagcgg gcggtgaggg ggcgcacgct ccaggtgagc 10260
gggcaacagg tggggggcg ggtggtgcta ggcctgggta ctgaccacca gggccgtccc 10320
aggtgtcgca ggccgggaag accctctgcc tgagcggctt catgggcatg gacatcccgc 10380
cacccagcgg gccactctgg atcctgggcg acgtcttcat cggccgctac tacactgtgt 10440
ttgaccgtga caacaacagg gtgggcttcg ccgaggctgc ccgcctctag ttcccaaggc 10500
gtccgcgcgc cagcacagaa acagaggaga gtcccagagc aggaggcccc tggcccagcg 10560
gcccctccca cacacaccca cacactcgcc cgcccactgt cctgggcgcc ctggaagccg 10620
gcggcccaag cccgacttgc tgttttgttc tgtggttttc ccctccctgg gttcagaaat 10680
gctgcctgcc tgtctgtctc tccatctgtt tggtgggggt agagctgatc cagagcacag 10740
atctgtttcg tgcattggaa gaccccaccc aagcttggca gccgagctcg tgtatcctgg 10800
ggctcccttc atctccaggg agtcccctcc ccggccctac cagcgcccgc tgggctgagc 10860
ccctacccca caccaggccg tcctcccggg ccctcccttg gaaacctgcc ctgcctgagg 10920
gcccctctgc ccagcttggg cccagctggg ctctgccacc ctacctgttc agtgtcccgg 10980
gcccgttgag gatgaggccg ctagaggcct gaggatgagc tggaaggagt gagaggggac 11040
aaaacccacc ttgttggagc ctgcagggtg gtgctgggac tgagccagtc ccaggggcat 11100
gtattggcct ggaggtgggg ttgggattgg gggctggtgc cagccttcct ctgcagctga 11160
cctctgttgt cctccccttg ggcggctgag agccccagct gacatggaaa tacagttgtt 11220
ggcctccggc ctcccctc 11238

<210> 27
<211> 22
<212> DNA
<213> Artificial

<220>
<223> Synthesis

<220>
<221> misc_signal
<222> (1)..(22)

<400> 27
tgattcaggg cgagtacatg at 22

<210> 28
<211> 21
<212> DNA
<213> Artificial

<220>
<223> Synthesis

<220>
<221> misc_signal
<222> (1)..(21)

<400> 28
gggacagctt gtagcctttg c 21

<210> 29
<211> 2486
<212> DNA
<213> homo sapiens

<400> 29
ccgggacttt gcaggcagcg gcggccgggg gcggagcggg atcgagccct cgccgaggcc 60

tgccgccatg ggcccgcgcc gccgccgccg cctgtcaccc gggccgcgcg ggccgtgagc 120

gtcatggcct tggccggggc ccctgcgggc ggcccatgcg cgccggcgct ggaggccctg 180

ctcggggccg gcgcgctgcg gctgctcgac tcctcgcaga tcgtcatcat ctccgccgcg 240

caggacgcca gcgccccgcc ggctcccacc ggccccgcgg cgcccgccgc cggcccctgc 300

gaccctgacc tgctgctctt cgccacaccg caggcgcccc ggcccacacc cagtgcgccg 360

cggcccgcgc tcggccgccc gccggtgaag cggaggctgg acctggaaac tgaccatcag 420

tacctggccg agagcagtgg gccagctcgg ggcagaggcc gccatccagg aaaaggtgtg 480

aaatccccgg gggagaagtc acgctatgag acctcactga atctgaccac caagcgcttc 540

ctggagctgc tgagccactc ggctgacggt gtcgtcgacc tgaactgggc tgccgaggtg 600

ctgaaggtgc agaagcggcg catctatgac atcaccaacg tccttgaggg catccagctc 660

attgccaaga agtccaagaa ccacatccag tggctgggca gccacaccac agtgggcgtc 720

ggcggacggc ttgaggggtt gacccaggac ctccgacagc tgcaggagag cgagcagcag 780

ctggaccacc tgatgaatat ctgtactacg cagctgcgcc tgctctccga ggacactgac 840

agccagcgcc tggcctacgt gacgtgtcag gaccttcgta gcattgcaga ccctgcagag 900

cagatggtta tggtgatcaa agcccctcct gagacccagc tccaagccgt ggactcttcg 960

gagaactttc agatctccct taagagcaaa caaggcccga tcgatgtttt cctgtgccct 1020

gaggagaccg taggtgggat cagccctggg aagaccccat cccaggaggt cacttctgag 1080

gaggagaaca gggccactga ctctgccacc atagtgtcac caccaccatc atctcccccc 1140

tcatccctca ccacagatcc cagccagtct ctactcagcc tggagcaaga accgctgttg 1200

tcccggatgg gcagcctgcg ggctcccgtg gacgaggacc gcctgtcccc gctggtggcg 1260

gccgactcgc tcctggagca tgtgcgggag gacttctccg gcctcctccc tgaggagttc 1320

atcagccttt ccccacccca cgaggccctc gactaccact tcggcctcga ggagggcgag 1380

ggcatcagag acctcttcga ctgtgacttt ggggacctca cccccctgga tttctgacag 1440

ggcttggagg gaccagggtt tccagagtag ctcaccttgt ctctgcagcc ctggagcccc 1500

ctgtccctgg ccgtcctccc agcctgtttg gaaacattta atttataccc ctctcctctg 1560

tctccagaag cttctagctc tggggtctgg ctaccgctag gaggctgagc aagccaggaa 1620

gggaaggagt ctgtgtggtg tgtatgtgca tgcagcctac acccacacgt gtgtaccggg 1680

ggtgaatgtg tgtgagcatg tgtgtgtgca tgtaccgggg aatgaaggtg aacatacacc 1740

tctgtgtgtg cactgcagac acgccccagt gtgtccacat gtgtgtgcat gagtccatct 1800

ctgcgcgtgg gggggctcta actgcacttt cggccctttt gctcgtgggg tcccacaagg 1860

cccagggcag tgcctgctcc cagaatctgg tgctctgacc aggccaggtg gggaggcttt 1920

ggctggctgg gcgtgtagga cggtgagagc acttctgtct taaaggtttt ttctgattga 1980

agctttaatg gagcgttatt tatttatcga ggcctctttg gtgagcctgg ggaatcagca 2040

aaaggggagg aggggtgtgg ggttgatacc ccaactccct ctacccttga gcaagggcag 2100

gggtccctga gctgttcttc tgccccatac tgaaggaact gaggcctggg tgatttattt 2160

attgggaaag tgagggaggg agacagactg actgacagcc atgggtggtc agatggtggg 2220

gtgggccctc tccagggggc cagttcaggg cccagctgcc ccccaggatg gatatgagat 2280

gggagaggtg agtgggggac cttcactgat gtgggcagga ggggtggtga aggcctcccc 2340

cagcccagac cctgtggtcc ctcctgcagt gtctgaagcg cctgcctccc cactgctctg 2400

ccccaccctc caatctgcac tttgatttgc ttcctaacag ctctgttccc tcctgctttg 2460

gttttaataa atattttgat gacgtt 2486

<210> 30
<211> 10705
<212> DNA
<213> homo sapiens

<400> 30
ccgggacttt gcaggcagcg gcggccgggg gcggagcggg atcgagccct cgccgaggcc 60

tgccgccatg ggcccgcgcc gccgccgccg cctgtcaccc gggccgcgcg ggccgtgagc 120

gtcatggcct tggccggggc ccctgcgggc ggcccatgcg cgccggcgct ggaggccctg 180
ctcggggccg gcgcgctgcg gctgctcgac tcctcgcaga tcgtcatcat ctccgccgcg 240
caggacgcca gcgccccgcc ggctcccacc ggccccgcgg cgcccgccgc cggcccctgc 300
gaccctgacc tgctgctctt cgccacaccg caggcgcccc ggcccacacc cagtgcgccg 360
cggcccgcgc tcggccgccc gccggtacgg accccaggga cgccgcgccg acagcgccgc 420
ctgtgccccc cgcgcagacc cgggagggcg ccgtgtttgg cctggaggcg gcaggggcg 480
ggggaggggt tgactgaggc gcccaggctg ggcggtgcag agccggggtt gggcctccgg 540
gccccgccct ggggtgcggg gtgacctcgg gccggtccct gtttgctgct ctgtgctctg 600
cgcctcagct tcctcaccgg agcctcccca gcgtcggact cagtgataat aatagcccac 660
gtgtattcaa ctggcgttta ctgcatgcca ggcctttggg cgacttcaag ccagctaaaa 720
ctcacaaccc cgctgtgagc tgtgttgtca ctgtgcccat ctgacggagg aggaactgag 780
gcccagagag ggaaagtgac ttgcccctgg tcacacggct gcaggtggtg ggctgggat 840
ttgaactgag gcctgctggc ttcagctcag aatttgcatc tcggtagtga gcacagctgc 900
taggggaagt gaaggcgggg cggaggaaag tcctggggcc taaccgttgg tgagccattg 960
aggagaccct ccttcctgcc tcgatgctga ctgccaggtg caggcctggc cggccaggcc 1020
tctggggaca gccctcctgg ctgggaggcc ttctctgcag ggcggggca gctggaccag 1080
ctgctgatcc cctccctctg cctgtccttc catctgccac cctccaccct cccaggccct 1140
ggttgccatg gaacctcatt aatcaggcag gccgacccct gagtctctgt agaaggacta 1200
ggtgggctgg gcctctggga gctggagagg aggcccccag tgccaggaga gaaaatgtct 1260
cagggagatc ctttaataaa gccctccctg atcgctgagc tcaagacgaa ttcagactgg 1320
agggcccaga agaggcggct gaattccagt gggagaaggg attggaggcg tagaagcctg 1380
gagctgggaa ctgtggggca cagggcaggg tgtggagacc agtttgactg gcgcagccta 1440
tgggtgaagg tggggctctg gtgtgcaggc ggggtctggc caggcaggat ggggtctcag 1500
cagggaactt ggagcaagtg ggaggggtga ggcaggactc aggacaagga ggtagaagct 1560
gcctaactgc tgatgggggt gtgggtggca gctggtccct gaggcatcct gaggaggctc 1620
ccccaacccc cttggcccca tgcccagtgc tgccttccgg aatcaccaca atgctaataa 1680
taataacaat agcagccacc attgttttga ggatgtcttg tgtgccaggc tctgtgccaa 1740
gcgctttact taattaactc atttaacccc atcagcagca gtcccttttt acagattggg 1800
aagctgaggc ctagagatgt tagaatcttc ccatgtcatt ctgctcttga gtggggagag 1860
cagtctgcgt ttcctctgag gttgacctct gggatgcctt gctttctggg gatgctgata 1920
caggttgaag aaagggcaag aagggctttt aggggccctc cctcttaatg agtgggtggg 1980

aggggggttgt accaggggtg ggccaggtgg cctgtctcct gtactccagg ggcctgttcc 2040
tgtgggtacc acttgggacc tgggcaagca tcttccattc agctgtgtga caagcattat 2100
agaaggctca ggtctgcgga aatctaacaa cctgagggtc ttgtcctcaa agatctgaac 2160
aggattatgg gttctaggat caaagggagg gacaggggca aagaaaaatg ccctccactc 2220
cacctgtcag gggcaggctc attggcctgg gcattccatt cattcaacag ttgttgagtt 2280
agtgaatgcc caggaggcac taggcccaga gacaaggagc cctggccccc agtgtactta 2340
agggccagga ggctggcacc attctggaac tgaagtccag aaagtccaca ggtttggctt 2400
ttggcctctc tgcactggca tttgtctggc acagtccttg aaataagcta caggcttagc 2460
ttctatacca gcaggcattc tgtatacaag gctctgcaat tcagccattc ctcttggccc 2520
tgcctgtcca cttccacaaa ctcaagtatt ataactataa tacatgttta atgtagaaaa 2580
cttaactttt ttatttattt atttattttt tttttggaga cagggtctca ttctgttacc 2640
caggctggag tgcagtggcg tgattgcggc tcactgcaac ctctgcctcc caagttcaag 2700
ctattctgtg cctcagcctc ccgagtagct gggattacag gcgtgtacca ccatgcccag 2760
ccaatttttg tatttttagt agagagaggg tcttgccatg ttgcccaggc tggtcttgaa 2820
ctcctgagtt cagacaatct gactgtctca gcctctcaaa gtgctgggat tacaggtgtg 2880
agtcaccatg cccagctgaa aacttaactc ttaactcttt tttttttttt tttttttgag 2940
atgaagtctc gctctgtcac ctaggttgga gtacagtggc gccatctcgg ctcaccgcaa 3000
cctctacctc ctgggttcaa gcgattcttc tgcctcagcc tcccgagtag ctgggactac 3060
agacgtgcac caccataccc ggctaatttt tgtatttttg gtagagatga ggtttcacca 3120
tattggtcag gctggtcttg aactcctgac ctcaagtgat ctgcccgcct cggcctccca 3180
aagtgccagg attacaggcg tgagccactg cgcctggcca aaaacttaac tcttaagaaa 3240
ctagaaagtt gacggcagga tcccagccct attcattttt ttcctttttt tccctaaggc 3300
tatttttgtt tgtggtggtt gtgacagttt ttgtttttta aagttttaaa aattgtagtt 3360
tttcctgtat cattcacacc attcagatat ggcactggtc tcccacagtc cttccagagg 3420
ctgccccgaa atgtattcta taataatata aactgaacca gagaaaagtt tcatgagctt 3480
tttaatccaa aagggatcat ataaatatat cgccctgtga cagtgttttg cttgtctgta 3540
tgtgtgaggg gattcttgtc aacctgcctc ggtccttgac agctgcgtgg tgttcaaggg 3600
gctaaacaaa gcacagcttt cgtgaccccc ttcatggatg ggtgttcgag tcatccagat 3660
tttttcagca gtgaatgtct tcacccctgc gtttcggggc ccctctgcca gtgtttatct 3720
agggagaagt ggcagaaatg gcatccactc aagcctttgt tttagggcgt ggcactgacc 3780
tgccttcagg ggtggacaag ggtggggatc ccagggagaa ggcaaggggt gcagatggca 3840
gaagcatgct agggtttggg ccccaggggc gagtatggag ggggatacat tctggctggg 3900

ttctggggag gtggggcacg tggcgggtat gagtgtgggc tctgtaccag ccaactaggg 3960
tgcaagtcct gggtccacct ctcactgtgt ggcctctctc aggtggaggc catcatagca 4020
cctgccaaaa ggtggccgga gggttgcagg ggaagcagtt gagtacacag tgcttagagc 4080
acaacagctc ttctttcccc tgggtgcctt caaaagtcag tgaatgtgca caagagggag 4140
tggttcatca gcatgggggg ggttcattca gcaaacattt gttgatgcca ggctccatgg 4200
cagacactat agattctcaa gtgattcaga cacctctgag ggtgagatag atacatctgc 4260
agaaatgtgt agggagagac agaatgtggc aaaaggttgg gtttgaggca gttcagaggg 4320
agaagaggtg acacctccta gctgatgcgt aaggtttctg cccacagagg tagagggagc 4380
aggcgtgaag tggggagggt gggagggatg caagggcagt ctagatttct gggaaaacgt 4440
gactcgggtt cctctagaga agcagtggca gatgagagta caaaggcaat ggggagctgg 4500
aggaaggcct taagcagggg cggcggcatg gtaaggtttg taggaggact ggctgcagca 4560
gaggcaggga gaccagtgtg gagtctgctc agcagcccac tgggaaggtg gtgatcgccg 4620
tggtgatgag cagttcttgg tagctgcatg tgaggagggt gacaggtcag gaactctagc 4680
tcaggaaacc ctgtggatgg tggaggggaa gatcagtctg gttttggtcc gggtactttt 4740
gagcagcctg tgagacctcc aggacagtcc caatcctgga gtgtctgaac taagccagag 4800
tgcttagagc ttgggttctt cctcagaccg tcttgggtac aaatcctgac tctaccactt 4860
cctgggcctc cctgggcttt agttgcctta tctggaaaat ggggccattt ttatacagcc 4920
agtccgttga atggaatgaa ctaatggatg caaggaactt gatgagcacc agctgctacc 4980
atgcgtatag attagtcaaa aaccagagag aaggtgattt agccaaggga cctatgtaat 5040
cagtggctta gctgggcatg gtggctgact agtcccagca cgactacagt gaaggctgag 5100
atgggagaat tgcttgagcc taggagtttg agaccagtct gggcaacata gggagacccc 5160
atctctacga gaaatagaaa agttagctgg tcttggaggg atgcgcctgt ggtcccagct 5220
gtttggaagg ctgaggtggg aggattgctt gagcccagga agtctaggct gcagagagtt 5280
gtgatcatac cactgcactc cagcatagtc aacagagcga taccctgtct aaaaaagaaa 5340
aaaaaatagt gccttaaaac aacaaacatt tattatatag tttttgtggg tcaggaattc 5400
agggaagact tatccagctc tggcttgggg tctctggaga ttcagccagt gtttgggttc 5460
tggctgctaa cagaagttgt gactaagtgt tgctgttggg aggaggccat ggtttcttgc 5520
ccatcattag ggctacttga gtgcccatat gatatggcag agtaagtgac ccaatacaga 5580
gcaaggagga agctccagcg cctctgaccc agtctcaaaa gtgacaggcc atcccttcca 5640
ccacattcca ttcattcatc cccagaagca agttaccagg tctggctcat aaggggagaa 5700
ttaggctata tcttttgaag gagaaggatc agaaagtttg cggatgtgtt ttaaatccac 5760

cgctagaccc cagagaagtg gtgaaaatgg aatgtcctgt gtggtaggac tgtgactctc 5820
aggctatgtc ccctctgatc tccctcagtc acacactcag gcatcctgct agagaaactg 5880
aggcgagggt agcccagagg cacccagcct taagccaggt ctcttctggc ctcactcctg 5940
gttactgggc atcctcccgt tttcgccaca ggtgaagcgg aggctggacc tggaaactga 6000
ccatcagtac ctggccgaga gcagtgggcc agctcggggc agaggccgcc atccaggaaa 6060
aggtacctat ggagcttggg ctgggcagac cccctgtgac caggcccagg ttagggatgt 6120
gaacagacaa acatgcttgc aagctaacgt ctggcttgtc gggcttgagc caggcctctg 6180
aggaccctga tgggcagttg gagtggccac ctggcttggt ttctccaggc cctgaggggt 6240
gaggagttcc ccagccagaa gaagaatgct gtggttggcc tcgccaaggg gaaggagctg 6300
gctgtccccc agcccccatc atcctgctgc cctgcactgc cagagtggca ggcctggctg 6360
gaggtggggg tggatgctga agggccctgt cttgcctgac cacacccccca caggtgtgaa 6420
atccccgggg gagaagtcac gctatgagac ctcactgaat ctgaccacca agcgcttcct 6480
ggagctgctg agccactcgg ctgacggtgt cgtcgacctg aactgggctg ccgaggtgct 6540
gaaggtgcag aagcggcgca tctatgacat caccaacgtc cttgagggca tccagctcat 6600
tgccaagaag tccaagaacc acatccagtg gctgtaggta ccggccacac aggagggcag 6660
gcacacctgc ccatgccagc ctggaggagc tggtagtaat acccactgtg gctgcctgca 6720
tccccatctt atagctgggg aaacgggctc agagaggaga ggtagcttcc ctgagatgat 6780
agatggtaaa aagcacacaa atcttacata tccacctcag ttgcagcttt actttttct 6840
tttttagatg gagtttcact ctgtcaccca ggctggagtg cagtggtgta atctcagctc 6900
actgtaacct ctacctccca ggttcaggtg cttctcctgc ctcagcctcc tgaatagctg 6960
gggatacagg cgcctgccac catgcccagc tactttttgt atttgtagta gaaatggggt 7020
ttcaccatgt tggccagtct ggtctcgaac tcctgatctc aggtgatctg cctgcctcag 7080
cctcccaaag tgctgggatt acaggcatga gccactgcgc cccgcgcagc tttatatatt 7140
tattcatctg ggttgccacc attcagatca agataagagc attttcaaca ccctagaagg 7200
ttctcttgtg ccccctttcca gtcattgcca acccctcaca ccgccaggtc accactatcc 7260
tggcttctca tgccctcggt gagatctgtc tggcatagat attttattct tgttttattt 7320
tccccctctc gccttcgcgc cagtcatcat gctactaccc tttctttgat acacctgccg 7380
tttcattttt tggtagatcg ttccattctg catcttttgt ggttttttgg gtggaggggg 7440
gtggtttgag acagggtgtt gctctgtcgc ccaggctgga gtatggaggc acaaatacag 7500
ctcactgcag cctcaacctc ctgggctcaa gctccgtacc tgtagctagg actacaagta 7560
catgccacca tgtctggcta atgtctttga ttttgtgtag aggcagagtc tcactgtgtt 7620
gcccaggctg gtctcaaact cctgaggctc aagcaatcct ctggcctcag cccccccaaag 7680

tgctgggatt ataggtataa gccaccaagc ccagcctttt tttttttttc atttgacagc 7740
atgtcttgga aatgcatgtg tgtgtgcatg tgtaatattt cgtggcatgt cagtcccatc 7800
atttgtttat cccgccctgg ttgagggaca tgtaggtgtt tatattcggg ccagacttca 7860
agcccatgac actctgattt ggagcccagc tgaggagcca gacccagaca gaagcaaggg 7920
cttgcctagg cctcacagaa cagaggcaac agcagactct gtcctgaggc ccagggctcc 7980
tggccagagg ggccaatcgc tgttaaccct accctccctg gtgcctcccc acaggggcag 8040
ccacaccaca gtgggcgtcg gcggacggct tgaggggttg acccaggacc tccgacagct 8100
gcaggagagc gagcagcagc tggaccacct gatgaatatc tgtactacgc agctgcgcct 8160
gctctccgag gacactgaca gccagcgata tccttggatt ggccgtaggg tgtggaaggc 8220
aggcttagca ggcaggggct ccacccaagt gggactagag aggcttggat ttaagagaga 8280
gctctgattt tggttgcaag tcctggctca gcattttctc actgttgtga cactggataa 8340
attgcttaac ctcttggcct cagttttatc tataaaaggg gaataataat atccaattct 8400
gaaattgtac agattaaaaa agaaaagtgg ctaggtgcgg tggttcatgc ctgtaatcct 8460
agtgcttcaa gaggctgagg tgggaggatc acttgagccc aggagtttga ggctgcagtg 8520
agctctggtt gagccacatc actccagcct gggtgacata gcgagaccct gcctctaaaa 8580
aataaattcc cagcaccagg cctggagttg tgttgaacat tttacatgga atcgttcccg 8640
gtagccatta ttaccttggg attcaatggg tagagtgatt tcattataca gatgtggaac 8700
ttgaaactca ggcgtgaggt aggttttgag tgggtaggag gttgggagga caggagtgac 8760
taggcaggtg tgagccctgc cttgtgagct gttggagtga gtgacccccct agaagtcaaa 8820
ggtcatgtgg tccttgactc tgccaaccct ggcctacgtg acgtgtcagg accttcgtag 8880
cattgcagac cctgcagagc agatggttat ggtgatcaaa gcccctcctg agacccagct 8940
ccaagccgtg gactcttcgg aggtgagatc tgggaactcc gggcccaact gggctgggct 9000
gggctgggct ggtggtcacc tgggcctcag tttaccctgc ctgctgcttc cacccagaac 9060
tttcagatct cccttaagag caaacaaggc ccgatcgatg ttttcctgtg ccctgaggag 9120
accgtaggtg ggatcagccc tgggaagacc ccatcccagg aggtcacttc tgaggaggag 9180
aacagggcca ctgactctgc caccatagtg tcaccaccac catcatctcc cccctcatcc 9240
ctcaccacag atcccagcca gtctctactc agcctggagc aaggtgggtg atgggtaggt 9300
gggtggggtg gggcagggcc cctcttctgg ggggtgggca ggcacgacag cccctgcctt 9360
cctccctgct ggggcatccc cggcctgtga tgctccccgt ctccccagaa ccgctgttgt 9420
cccggatggg cagcctgcgg gctcccgtgg acgaggaccg cctgtccccg ctggtggcgg 9480
ccgactcgct cctggagcat gtgcgggagg acttctccgg cctcctccct gaggagttca 9540

tcagcctttc cccaccccac gaggccctcg actaccactt cggcctcgag gagggcgagg 9600

gcatcagaga cctcttcgac tgtgactttg gggacctcac ccccctggat ttctgacagg 9660

gcttggaggg accagggttt ccagagatgc tcaccttgtc tctgcagccc tggagccccc 9720

tgtccctggc cgtcctccca gcctgtttgg aaacatttaa tttatacccc tctcctctgt 9780

ctccagaagc ttctagctct ggggtctggc taccgctagg aggctgagca agccaggaag 9840

ggaaggagtc tgtgtggtgt gtatgtgcat gcagcctaca cccacacgtg tgtaccgggg 9900

gtgaatgtgt gtgagcatgt gtgtgtgcat gtaccgggga atgaaggtga acatacacct 9960

ctgtgtgtgc actgcagaca cgccccagtg tgtccacatg tgtgtgcatg agtccatgtg 10020

tgcgcgtggg ggggctctaa ctgcactttc ggcccttttg ctctgggggt cccacaaggc 10080

ccagggcagt gcctgctccc agaatctggt gctctgacca ggccaggtgg ggaggctttg 10140

gctggctggg cgtgtaggac ggtgagagca cttctgtctt aaaggttttt tctgattgaa 10200

gctttaatgg agcgttattt atttatcgag gcctctttgg tgagcctggg gaatcagcaa 10260

aggggaggag gggtgtgggg ttgatacccc aactccctct acccttgagc aagggcaggg 10320

gtccctgagc tgttcttctg ccccatactg aaggaactga ggcctgggtg atttatttat 10380

tgggaaagtg agggagggag acagactgac tgacagccat gggtggtcag atggtggggt 10440

gggccctctc caggggccca gttcagggcc ccagctgccc cccaggatgg atatgagatg 10500

ggagaggtga gtgggggacc ttcactgatg tgggcaggag gggtggtgaa ggcctccccc 10560

agcccagacc ctgtggtccc tcctgcagtg tctgaagcgc ctgcctcccc actgctctgc 10620

cccaccctcc aatctgcact ttgatttgct tcctaacagc tctgttccct cctgctttgg 10680

ttttaataaa tattttgatg acgtt 10705

<210> 31
<211> 25
<212> DNA
<213> Artificial

<220>
<223> Synthesis

<220>
<221> misc_signal
<222> (1)..(25)

<400> 31
gagcagatgg ttatggtgat caaag 25

<210> 32
<211> 24
<212> DNA
<213> Artificial

<220>
<223> Synthesis

<220>
<221> misc_signal
<222> (1)..(24)

<400> 32
ggagatctga aagttctccg aaga 24

<210> 33
<211> 1600
<212> DNA
<213> homo sapiens

<400> 33
gccccgccgc cggcagtgga ccgctgtgcg cgaaccctga accctacggt cccgacccgc 60

gggcgaggcc gggtacctgg gctgggatcc ggagcaagcg ggcgagggca gcgccctaag 120

caggcccgga gcgatggcag ccttgatgac cccgggaacc ggggccccac ccgcgcctgg 180

tgacttctcc ggggaaggga gccagggact tcccgaccct tcgccagagc ccaagcagct 240

cccggagctg atccgcatga agcgagacgg aggccgcctg agcgaagcgg acatcagggg 300

cttcgtggcc gctgtggtga atgggagcgc gcagggcgca cagatcgggg ccatgctgat 360

ggccatccga cttcggggca tggatctgga ggagacctcg gtgctgaccc aggccctggc 420

tcagtcggga cagcagctgg agtggccaga ggcctggcgc cagcagcttg tggacaagca 480

ttccacaggg ggtgtgggtg acaaggtcag cctggtcctc gcacctgccc tggcggcatg 540

tggctgcaag gtgccaatga tcagcggacg tggtctgggg cacacaggag gcaccttgga 600

taagctggag tctattcctg gattcaatgt catccagagc ccagagcaga tgcaagtgct 660

gctggaccag gcgggctgct gtatcgtggg tcagagtgag cagctggttc ctgcggacgg 720

aatcctatat gcagccagag atgtgacagc caccgtggac agcctgccac tcatcacagc 780

ctccattctc agtaagaaac tcgtggaggg gctgtccgct ctggtggtgg acgttaagtt 840

cggaggggcc gccgtcttcc ccaaccagga gcaggcccgg gagctggcaa agacgctggt 900

tggcgtggga gccagcctag ggcttcgggt cgcggcagcg ctgaccgcca tggacaagcc 960

cctgggtcgc tgcgtgggcc acgccctgga ggtggaggag gcgctgctct gcatggacgg 1020

cgcaggcccg ccagacttaa gggacctggt caccacgctc gggggcgccc tgctctggct 1080

cagcggacac gcggggactc aggctcaggg cgctgcccgg gtggccgcgg cgctggacga 1140

cggctcggcc cttggccgct tcgagcggat gctggcggcg cagggcgtgg atcccggtct 1200

ggcccgagcc ctgtgctcgg gaagtcccgc acaacgccgg cagctgctgc ctcgcgcccg 1260

ggagcaggag gagctgctgg cgcccgcaga tggcaccgtg gagctggtcc gggcgctgcc 1320

gctggcgctg gtgctgcacg agctcggggc cgggcgcagc cgcgctgggg agccgctccg 1380

cctgggggtg ggcgcagagc tgctggtcga cgtgggtcag aggctgcgcc gtgggacccc 1440

ctggctccgc gtgcaccggg acggccccgc gctcagcggc ccgcagagcc gcgccctgca 1500

ggaggcgctc gtactctccg accgcgcgcc attcgccgcc ccctcgccct tcgcagagct 1560

cgttctgccg ccgcagcaat aaagctcctt tgccgcgaaa 1600

<210> 34
<211> 4275
<212> DNA
<213> homo sapiens

<400> 34
gccccgccgc cggcagtgga ccgctgtgcg cgaaccctga accctacggt cccgacccgc 60

gggcgaggcc gggtacctgg gctgggatcc ggagcaagcg ggcgagggca gcgccctaag 120

caggtacggg cggggctcaa gtcgcgaggc ggggaagcgg gaggcagaca cggacgaggg 180

cgacacagac acgggaccga ggggcggaca ccggagagac acgggaaagg ggtcgggaca 240

ggagcacgtg gctcagacac cgacgccggg aggccgcaga ccccggacgt gtcaggcatc 300

cccgcaggcc cggagcgatg gcagccttga tgaccccggg aaccggggcc ccacccgcgc 360

ctggtgactt ctccggggaa gggagccagg gacttcccga cccttcgcca gagcccaagc 420

agctcccgga gctgatccgc atgaagcgag acggaggccg cctgagcgaa gcggacatca 480

ggggcttcgt ggccgctgtg gtgaatggga gcgcgcaggg cgcacagatc ggtgcgtggg 540

gagggttggg cgttcctgac cccgactggg aggtcagccc gagagacttt gggtccctgg 600

gggtgcgacg gtgccccact accagcaccg gccccagggt gccccaccgc tgtgggctgc 660

caccctcacg cgtacccccca cataccaggg gccatgctga tggccatccg acttcggggc 720

atggatctgg aggagacctc ggtgctgacc caggccctgg ctcagtcggg acagcagctg 780

gagtggccag aggcctggcg ccagcagctt gtggacaagc attccacagg gggtgtgggt 840

gacaaggtca gcctggtcct cgcacctgcc ctggcggcat gtggctgcaa ggttagaaac 900

cacctccttt ccagacggga gcctataccg cacatgcagc aaccagtcca tccacaggca 960

gctcccaacc tcaagcctgg cccaaagcct ccaagaccct accaaggctt ctccccaccc 1020

tgctccccag cacagttctc cccaccccgt tccccagcac agcgcttggg gcccctctgg 1080

ctccagacca ggccccttgg agcaggaaaa agatccactg atggaattca gaccccttc 1140

cccttgggtc cccagacagc tcccccaagg gaggagctga ggactttcct ccctctgccc 1200

caagccttgt ttccccaagg acaggtacca acctcctccc ctactgacac ttctcaacca 1260

agaaaacttc ctttccattc cctcaccagc tgggcacccc tatagctgct taaatacttt 1320

ccaaatccag ctgcactcct agccagggaa ggtgaaggga tgcacagagg tgggggaggg 1380

gtactgtgca gggtactcag catccctgac caccaggtgc caatgatcag cggacgtggt 1440

ctggggcaca caggaggcac cttggataag ctggagtcta ttcctggatt caatgtcatc 1500
cagagcccag agcaggtacg gggcgccacg gatcagtcat taatccaggt tgatgatgga 1560
gaccctggcc agaatcacta aaagatcact ggtggatcat tagggtcact aatgagaaca 1620
ctggtcaagg ttactcatga gtcactgggc ctgggccgaa atcatcagtg gaactttgat 1680
taggatcata aaatgggaag ttggtcaaaa tcacagatgg ctggcggggc acggtggctc 1740
acacctgtag tcctagcact tggggaggcc gaagagggca gatcccttga acccaggagt 1800
tcaaaaccag cctggataac acggcaaaac cccatctcta caaaatagtt cgctgcgtgt 1860
ggtggtgcac gcatgtggtt ccagctactc aggaggctga ggcaggagga tcacttgagc 1920
ctgggaggtc taggctgcag tgagccggga cgatgccact gcactccagc ctgggcaaca 1980
gagtgagacc ctgtcccagc actctgggag gcagaggagc ccagttggag atcagcctgg 2040
gtaatatagt gaaacttgat ctctacaaaa aaaagaagaa aaaaaaaagc cgcgtgtggt 2100
ggtgcgcacc tgtagtccca gctactggga agctgaggtg ggaggatcac ttaagcccag 2160
gaggcagagg tcacaatgag ccgaaattgt gccaactgca ctccagcctg ggcaacagag 2220
gaagactctt cacagaaaaa aaaaaaaaaa aaaaaaaagc tgctaagtca tttaccataa 2280
gtcactgaga acaggggatg tctgaccaga tgcaagtgct gctggaccag gcgggctgct 2340
gtatcgtggg tcagagtgag cagctggttc ctgcggacgg aatcctatat gcagccagag 2400
atgtgacagc caccgtggac agcctgccac tcatcacagg tgacctgact ccatggcctg 2460
cttctgcatg ttcacaggct cctgacctcc aaactcaagt caagggcctc tcgttaggag 2520
ttacccgtca cctgaccgtg tgccccccta cccccatcac aagatgcctg accaccacca 2580
tgtggggtgg cctgatactc aacccaccag gtgctgccac ccccataata agggacttga 2640
ccctcaatgc tcagggcccc tgaccccaaa gtcggcatcc ccgaactctc ccaagaagct 2700
ccaggttctc cattgtctcc aacctcctct gcctccccca aagcctccat tctcagtaag 2760
aaactcgtgg aggggctgtc cgctctggtg gtggacgtta agttcggagg ggccgccgtc 2820
ttccccaacc aggagcaggc ccgggagctg gcaaagacgc tggtgagcgg tgtggccttt 2880
ccctgggcaa gcgtcttgat gcgggcccag cctacccttc acccctcccg tccccactgc 2940
ctccctccac tcagcagtcc tgcctaaccc cagtcccacc ctcttctgcc cgaagtccct 3000
ccctccttca cggcttccta acctgctgtg actttagagg tcaaggctgg cccggcctgg 3060
acctggggaa gccctctgtg gcgttcctgc cccagaccaa gtacaagttc ctcctggccc 3120
catggcgagg tgtcgcactt cactcgtgtc tcttccccac cccaatcctt ccctgacttc 3180
atgctggggg gctggcaacc cagggtgcag caggggctgg agttcgacca agaaccggct 3240
gcagaaggcc ccgccatggg gggtccacgc tgagcctcct ctccgcaggt tggcgtggga 3300

gccagcctag ggcttcgggt cgcggcagcg ctgaccgcca tggacaagcc cctgggtcgc 3360

tgcgtgggcc acgccctgga ggtggaggag gcgctgctct gcatggacgg cgcaggcccg 3420

ccagacttaa gggacctggt caccacgctc ggtgaggggg acggggtgta ggggagcgga 3480

ggcggcgggg ggtgcttccc gctggggccg ccccgacccg gccgcgccta agacccgtcc 3540

ccgcccgcag gggcgccct gctctggctc agcggacacg cggggactca ggcccagggc 3600

gctgcccggg tggccgcggc gctggacgac ggctcggccc ttggccgctt cgagcggatg 3660

ctggcggcgc agggcgtgga tcccggtctg gcccgagccc tgtgctcggg aagtcccgca 3720

gaacgccggc agctgctgcc tcgcgcccgg gagcaggagg agctgctggc gcccgcagat 3780

ggtgagcgtc gggggagtcc ccgtccttcc gcctccgcca tccccttccc ttcccgaggc 3840

cccgcccctt cccgagcccg cgcctctcag cccctctccc cgcaggcacc gtggagctgg 3900

tccgggcgct gccgctggcg ctggtgctgc acgagctcgg ggccgggcgc agccgcgctg 3960

gggagccgct ccgcctgggg gtgggcgcag agctgctggt cgacgtgggt cagaggctgc 4020

gccgtggtga gcgccgcccc cgccctgctg gccccgcacc cccgcccagc tccggccgcg 4080

cggcctctaa cagcccctcg ctctgcaggg acccccctggc tccgcgtgca ccgggacggc 4140

cccgcgctca gcggcccgca gagccgcgcc ctgcaggagg cgctcgtact ctccgaccgc 4200

gcgccattcg ccgcccccctc gcccttcgca gagctcgttc tgccgccgca gcaataaagc 4260

tcctttgccg cgaaa 4275

<210> 35
<211> 20
<212> DNA
<213> Artificial

<220>
<223> Synthesis

<220>
<221> misc_signal
<222> (1)..(20)

<400> 35
cctgcggacg gaatcctata 20

<210> 36
<211> 20
<212> DNA
<213> Artificial

<220>
<223> Synthesis

<220>
<221> misc_signal

<222> (1)..(20)

<400> 36
cctgcggacg gaatcctata 20

<210> 37
<211> 4624
<212> DNA
<213> homo sapiens

<400> 37
ggaggaggtg gaggaggagg gctgcttgag gaagtataag aatgaagttg tgaagctgag 60

attcccctcc attgggaccg gagaaaccag gggagccccc cgggcagccg cgcgcccctt 120

cccacggggc cctttactgc gccgcgcgcc cggcccccac ccctcgcagc accccgcgcc 180

ccgcgccctc ccagccgggt ccagccggag ccatggggcc ggagccgcag tgagcaccat 240

ggagctggcg gccttgtgcc gctgggggct cctcctcgcc ctcttgcccc ccggagccgc 300

gagcacccaa gtgtgcaccg gcacagacat gaagctgcgg ctccctgcca gtcccgagac 360

ccacctggac atgctccgcc acctctacca gggctgccag gtggtgcagg gaaacctgga 420

actcacctac ctgcccacca atgccagcct gtccttcctg caggatatcc aggaggtgca 480

gggctacgtg ctcatcgctc acaaccaagt gaggcaggtc ccactgcaga ggctgcggat 540

tgtgcgaggc acccagctct ttgaggacaa ctatgccctg gccgtgctag acaatggaga 600

cccgctgaac aataccaccc ctgtcacagg ggcctcccca ggaggcctgc gggagctgca 660

gcttcgaagc ctcacagaga tcttgaaagg aggggtcttg atccagcgga acccccagct 720

ctgctaccag gacacgattt tgtggaagga catcttccac aagaacaacc agctggctct 780

cacactgata gacaccaacc gctctcgggc ctgccacccc tgttctccga tgtgtaaggg 840

ctcccgctgc tggggagaga gttctgagga ttgtcagagc ctgacgcgca ctgtctgtgc 900

cggtggctgt gcccgctgca aggggccact gcccactgac tgctgccatg agcagtgtgc 960

tgccggctgc acgggcccca agcactctga ctgcctggcc tgcctccact tcaaccacag 1020

tggcatctgt gagctgcact gcccagccct ggtcacctac aacacagaca cgtttgagtc 1080

catgcccaat cccgagggcc ggtatacatt cggcgccagc tgtgtgactg cctgtcccta 1140

caactacctt tctacggacg tgggatcctg caccctcgtc tgcccctgc acaaccaaga 1200

ggtgacagca gaggatggaa cacagcggtg tgagaagtgc agcaagccct gtgcccgagt 1260

gtgctatggt ctgggcatgg agcacttgcg agaggtgagg gcagttacca gtgccaatat 1320

ccaggagttt gctggctgca agaagatctt tgggagcctg gcatttctgc cggagagctt 1380

tgatggggac ccagcctcca acactgcccc gctccagcca gagcagctcc aagtgtttga 1440

gactctggaa gagatcacag gttacctata catctcagca tggccggaca gcctgcctga 1500

cctcagcgtc ttccagaacc tgcaagtaat ccggggacga attctgcaca atggcgccta 1560

ctcgctgacc ctgcaagggc tgggcatcag ctggctgggg ctgcgctcac tgagggaact 1620
gggcagtgga ctggccctca tccaccataa cacccacctc tgcttcgtgc acacggtgcc 1680
ctgggaccag ctctttcgga acccgcacca agctctgctc cacactgcca accggccaga 1740
ggacgagtgt gtgggcgagg gcctggcctg ccaccagctg tgcgcccgag ggcactgctg 1800
gggtccaggg cccacccagt gtgtcaactg cagccagttc cttcggggcc aggagtgcgt 1860
ggaggaatgc cgagtactgc aggggctccc cagggagtat gtgaatgcca ggcactgttt 1920
gccgtgccac cctgagtgtc agccccagaa tggctcagtg acctgttttg gaccggaggc 1980
tgaccagtgt gtggcctgtg cccactataa ggaccctccc ttctgcgtgg cccgctgccc 2040
cagcggtgtg aaacctgacc tctcctacat gcccatctgg aagtttccag atgaggaggg 2100
cgcatgccag ccttgcccca tcaactgcac ccactcctgt gtggacctgg atgacaaggg 2160
ctgccccgcc gagcagagag ccagccctct gacgtccatc atctctgcgg tggttggcat 2220
tctgctggtc gtggtcttgg gggtggtctt tgggatcctc atcaagcgac ggcagcagaa 2280
gatccggaag tacacgatgc ggagactgct gcaggaaacg gagctggtgg agccgctgac 2340
acctagcgga gcgatgccca accaggcgca gatgcggatc ctgaaagaga cggagctgag 2400
gaaggtgaag gtgcttggat ctggcgcttt tggcacagtc tacaagggca tctggatccc 2460
tgatggggag aatgtgaaaa ttccagtggc catcaaagtg ttgagggaaa acacatcccc 2520
caaagccaac aaagaaatct tagacgaagc atacgtgatg gctggtgtgg gctccccata 2580
tgtctcccgc cttctgggca tctgcctgac atccacggtg cagctggtga cacagcttat 2640
gccctatggc tgcctcttag accatgtccg ggaaaaccgc ggacgcctgg gctcccagga 2700
cctgctgaac tggtgtatgc agattgccaa ggggatgagc tacctggagg atgtgcggct 2760
cgtacacagg gacttggccg ctcggaacgt gctggtcaag agtcccaacc atgtcaaaat 2820
tacagacttc gggctggctc ggctgctgga cattgacgag acagagtacc atgcagatgg 2880
gggcaaggtg cccatcaagt ggatggcgct ggagtccatt ctccgccggc ggttcaccca 2940
ccagagtgat gtgtggagtt atggtgtgac tgtgtgggag ctgatgactt ttggggccaa 3000
accttacgat gggatcccag cccgggagat ccctgacctg ctggaaaagg gggagcggct 3060
gccccagccc cccatctgca ccattgatgt ctacatgatc atggtcaaat gttggatgat 3120
tgactctgaa tgtcggccaa gattccggga gttggtgtct gaattctccc gcatggccag 3180
ggacccccag cgctttgtgg tcatccagaa tgaggacttg ggcccagcca gtcccttgga 3240
cagcaccttc taccgctcac tgctggagga cgatgacatg ggggacctgg tggatgctga 3300
ggagtatctg gtaccccagc agggcttctt ctgtccagac cctgccccgg gcgctggggg 3360
catggtccac cacaggcacc gcagctcatc taccaggagt ggcggtgggg acctgacact 3420

agggctggag ccctctgaag aggaggcccc caggtctcca ctggcaccct ccgaaggggc 3480

tggctccgat gtatttgatg gtgacctggg aatgggggca gccaaggggc tgcaaagcct 3540

ccccacacat gaccccagcc ctctacagcg gtacagtgag gaccccacag taccccctgcc 3600

ctctgagact gatggctacg ttgcccccct gacctgcagc cccccagcctg aatatgtgaa 3660

ccagccagat gttcggcccc agccccccttc gccccgagag ggccctctgc ctgctgcccg 3720

acctgctggt gccactctgg aaaggcccaa gactctctcc ccagggaaga atggggtcgt 3780

caaagacgtt tttgcctttg ggggtgccgt ggagaacccc gagtacttga caccccaggg 3840

aggagctgcc cctcagcccc accctcctcc tgccttcagc ccagccttcg acaacctcta 3900

ttactgggac caggacccac cagagcgggg ggctccaccc agcaccttca aagggacacc 3960

tacggcagag aacccagagt acctgggtct ggacgtgcca gtgtgaacca gaaggccaag 4020

tccgcagaag ccctgatgtg tcctcaggga gcagggaagg cctgacttct gctggcatca 4080

agaggtggga gggccctccg accacttcca ggggaacctg ccatgccagg aacctgtcct 4140

aaggaacctt ccttcctgct tgagttccca gatggctgga aggggtccag cctcgttgga 4200

agaggaacag cactggggag tctttgtgga ttctgaggcc ctgcccaatg agactctagg 4260

gtccagtgga tgccacagcc cagcttggcc ctttccttcc agatcctggg tactgaaagc 4320

cttagggaag ctggcctgag aggggaagcg gccctaaggg agtgtctaag aacaaaagcg 4380

acccattcag agactgtccc tgaaacctag tactgccccc catgaggaag gaacagcaat 4440

ggtgtcagta tccaggcttt gtacagagtg cttttctgtt tagtttttac tttttttgtt 4500

ttgttttttt aaagatgaaa taaagaccca gggggagaat gggtgttgta tggggaggca 4560

agtgtggggg gtccttctcc acacccactt tgtccatttg caaatatatt ttggaaaaca 4620

gcta 4624

<210> 38
<211> 28515
<212> DNA
<213> homo sapiens

<400> 38
cgcgcgcccg gcccccaccc ctcgcagcac cccgcgcccc gcgccctccc agccgggtcc 60

agccggagcc atggggccgg agccgcagtg agcaccatgg agctggcggc cttgtgccgc 120

tgggggctcc tcctcgccct cttgcccccc ggagccgcga gcacccaagg tgggtctggt 180

gtggggaggg gacggagcag cggcgggacc ctgccctgtg gatgccccgc cgaggtcccg 240

cggccggcgg ggccagaggg gcccggacga gctctcctat cccgaagttg tggacagtcg 300

agacgctcag ggcagccggg ccctggggcc ctcgggcggg agggggcagt tacacggcag 360

cggctcgaga tggcccatcc aagagactgg cgctttccag gctccgaggg gctccgggaa 420

cttgtcaaag aagttctctg aaattgttca gaaagttttc ccgcaaaggg tgtattgcgt 480
agagcgcgcg cgcgcgtttc ccccttctt gagccccctc aagctttctc aaagcctttc 540
cagttggcag cctccgcctc cggactggcc tgggctggat tccttggggg ggtcctctgc 600
cctgcccctc ctccagcccc tccccgctcc ccttcagacg attttggttt ggttgctcct 660
gcttctggcg gggtcgggtg tgtgtgtgtg tggtggagtg gagggtggca tagcaacctg 720
tcccaaccag agccggggag gaaagggtgg cccggagggt ggcctcttgc tggggtctgg 780
gttgggggcg ggggagacgt ttgctttgaa cagattcttg ggccagctt agggactgtg 840
ctctgtgact tttggagcgc gtggaccatg gaggggtggg ggtgggtttc ttggggtgta 900
aagtgggaga gttcccagag aaggaagcta agaaataagg ccagatggga gcctagggag 960
ggctgcgttg ttctgctgcc ttttccttgg tgctgtgcgt ggggaagggt gagtgggggc 1020
agtgtgtatc ctgacccatc tgtccacctg tgtgcattaa tcataaaagc taacatatag 1080
cctgggccag gtatactctg ccaggaactg tttgtggtgt tttgcatgca ttctccttta 1140
atcctagaac acccctatag tggaagttct gccagcattc tggactgagt agcagtccag 1200
aggttgagta gcagctagta agtggtgggg tcaagatggg accccaggca gtgcgacccc 1260
caaccatgca ttcgaaatcg ctatatggat gagtgcacct ggagcaatga gggacactgc 1320
tccctgagtc actgggctgc aggggagaca aaatgaaagt gttctgggag tcgtgggtgg 1380
tctccatagg tcagagggtc tggggaggga gtgggtgtca tcgtggctgt gtgttgcccg 1440
aggggccctc tgtgagtgag tgcatggccg tgttatctct gcaggtctac gccagggtgt 1500
tcctcagttg tgtggtcttt gtatttgtgt gtctgggctt tgtgttgcca aacagcagtc 1560
tctctgctga cttggggaca caggctgaac tctgtcctct gcaggaactc ccttaaggtg 1620
ctgggccaga tctgccataa acagagggag gtagccttct atggccacgc cttcttgctg 1680
aggaagaagg ttcctctctt ccagggagta catccttgcc ctccctgttt cccagacaag 1740
catcttcacc tctcatcttc tgatgagaag ggtgaggcca tactgagctg tcaggctgag 1800
ctgctgccct tcctcacctt gggctgggag ttgatcaggg aatggcagtt gctgcagagc 1860
tggatttgag ggctgggttc tctggatggg gcctcctcat gtcctcaccc ctcaacctgc 1920
actattgatt gtgttgtgca ggagttagtt aaaaagtcat tgcacagcct gggcaacaag 1980
gcaaaactct gtacaaaaaa tacaaaaatt agttggatgt gattacacgt gcctgtagtc 2040
ccagctactc cggaggctga ggcaggagga tcacctgagc ccaggaagtt gaggcttgca 2100
gtgagctgtg attgcaaatg ctctccagcc tgggtgacag tgtgagactc cgtttcagaa 2160
aaaaagtata ccacccagct gcctccagca cccagatttt acccaagggg tgaggtctgg 2220
ggcaggaatg tgggggaagg ggaggcctag ggggagcccc agaggggtca ggatttttct 2280
gaaatccttt cttagaggta tgggttttac aaattgcagc aaatacatcc ttttaatctt 2340

gcagaactcc ttcatatttt aattccagta tgattcttcc aacagcctcc tctctttact 2400
atacttgggg aaagtactca ttttatttgt caagaaaaaa acaattgaaa agatagggat 2460
caaatgtaaa aagaaaaaat acgtggcatt ccaaagtcaa acacaaagca tgtttaattt 2520
tctcgtggtt tgggattacc catattcctg ctgtatgaac ctgtcttgtc ttaactttta 2580
agaaatgtac ggtgtacttc ctatatgcta ggtttttatc catgctttca tttaatctct 2640
gtgacagtcc tgtgaagtag gtgcacagat gagaaaatgg aagttcagag aaatgaagca 2700
acttatccaa ggctcccagc tacccagtaa tgtccaggga atttttggac tctgaagagg 2760
aggcattaag aggtggttag agtcttattc cagccaacaa taatgggttg aacaaagcct 2820
taggggcagg caggtggcca gatgggagga gaagcgctcc tcttgttcag gcgaatgacc 2880
tttccatcca cttctctagg ctgtagaaag tggagctgag ctgggggccc tgaggttccc 2940
tcttgacttc agagtcctct cccttcctgt ccagccaatg cctgtcttcc ttttgggccc 3000
taccagcatg acagggggct gcgggcagga ggggacagag gccacgttga cacacagggc 3060
tgtgggtgag agagacagct gaagtgtcag cgtgaggggc cagtgtgggg ctgcggctgg 3120
gagggctggg gtggggccca gggtagttgt gcctgtcctt gggtgatgga atgatctgga 3180
aagagattcc ttccctgccc tccacctgtg agaagcccct ctagagtgac atctccatct 3240
tatgtttggc cacccatcct ccccctggga agagagccga ggtggggtaa gggatgtgta 3300
ctctttcaag gagtgggaga attattctag cgaatgtttg tgttgtccca gttctgttta 3360
caaagcctcg tcatgtttac agatggctgc gcaattcatt acctcattta actctcatgt 3420
acctcctctg agggagtaag agctgttaca gccaagttta ggtcagtaaa tattcaccaa 3480
gttgcaggta ctgcagggca tagagatgaa tccgatttag cttctgccct ggaggtctgg 3540
gaacttgctc aagatcactc agtgagcagc tgagctaggg ttctcaacta aagaccctgg 3600
gcccaggccc tggtctgatg tcaggcctga tacaccaggt gtttgtggtc ggggaatccc 3660
agtgtcactt gaatgggctg tgacattatg ggtctgggag agctgagctt tggggacaca 3720
ggtcatttta ctgtagtatt catggaaacc aagggaagta ttggcttttc tgctgtgagc 3780
aagaggagca gctggggctg caagctggtg gggaggagag aacccacctg agagaaacct 3840
caggactggg gtcaagtcct gaccaccaga gtccagagag acatgaagga ctgtgaccag 3900
ctctgagcag agagatggat tccatgacct caactggtcc cttttgttcg gagactcgtg 3960
actggacttc attcatccac tcattcattc attcactcag cagacactta tctagcgctc 4020
cctgtggctg gtcctgcctc atactgtctt tgctctggag aattggaggt tggggttcct 4080
gaggggcagg gtcctggaga caaggacact cctgggtaga attaggacct accccccagg 4140
aaatcaacgg ggaccaggtg ccgtggctca cacctgtaat cccagcactt tgggaggccg 4200

agacgggcgg atcacaaggt cagcagttca ggaccagcct ggccaacatg gtgaaacccg 4260
cctcaactaa aaatacaaaa attagccagg tgtggtgtca ggcacccgta atcccagcta 4320
ctgaggaggc tgaggcagga gaattgcttg aacccgggag gcagaggttg cagtgagccg 4380
agattgcgcc actgcactcc agcctggcga cagggcgaga ctccatctca aaaaaagaaa 4440
accaatggga cagggcagat atggggacaa tggtaaggag atgggagagt gggagggagg 4500
tgtcaggaag accttcttga cttcatgtag gctggtgggg gtgttagcca gcaagcctcc 4560
agttccctgg gaaccgttct cagggtacca atttaccac ctgtctgcaa acactttaag 4620
attcttaatc agactcaaat tggccacaaa tcaggtaaac aaactcacta gtggggtggg 4680
gctaccaccc gttctgaccc tccagcccaa cccagcccag ccaccctgcc ctccgtagag 4740
cctgtggtgt ttatcggtgg cattgggaga attagtgtgt atttatgttg gcgtggggtg 4800
tggggtggat ttgtgtgtgt gcagttaggc ctagtggaag gaatgtggga tctgaaggca 4860
ggccagcctg agttccagtc ctgcctgttg ctcacaagct ttatgaggcg agagctaacc 4920
cctgccagcc tcagttgtct tctttgcaag atggaggttg cagccccagt ctctggagca 4980
tgttatgcag atccaccgag agtgcctgcc aggcacacag taggtgctca gctcagttac 5040
tgtggcggcc cccactcccc attgttgttg ttttcctatt gcctggcggc cacagctggt 5100
atcccttgaa aagggctaca gggggtggag tcggaccctg ccccagccct gtggagaccc 5160
tgggcttggg ccagggcctg gggtctgggc ctgcagacag ctgtgtctat aaagcagctg 5220
aagggctgag gccgggggag gtcctggcag cagggcgtta ttttgggcct ggcctgccac 5280
ccccagctcc tgtttctctt gggagtctgt tgggggagga agtgtgggga agaggagggg 5340
gtgcaagtgg gtgaggcatg gagtggggag gcctccctca gggacatgga cccttgagtt 5400
ctatttctgt tcctccctcc tgttcctccc tctttgtcct tatctgccta gagaggtggg 5460
aatagaggcc attctgagta tcactaggag accaccagtt tgtggccact ggccactggc 5520
ccaggcaggg aacctggggg cttgccctac cagcctctcc cagcaatctg aaggcagggg 5580
gtacctcgta ttacccccta ggatttgacc ttaggctcca acttgctggg agagcagtgc 5640
ctctggtgtc agaccccaag ccagcccttg tgctgtccct gaatctgcat gtagcctgtg 5700
ggaggcggag cagtgaccgg caggaattct gggcagctca ggcacctgtg ggcctgaggg 5760
tgccctctgc ccccaccctt ccgatctcct gggcaagaca cgccaggtga ttcatctcac 5820
cagagcagaa aaacaagttc aactgggcac tttaatctcc cctcactggc aggcctggtg 5880
tgagctgcta ccccggcgcc cctcaccagg ggtgctttac ctcctctagt attcctgacc 5940
ttagtgggca tttctggtct cagggatacc aggctggggt ccaagtgggc caggtgtggc 6000
agttcagccc tatgccccat ggctgatggc tcgcgctggg caggtatgca gggctgacgt 6060
agtgcctttg tggcagcagt ttcgtggcac acattctgcc agctggttct ggagtcttgc 6120

cctgaggagg tggccagggt gagggtgcca gcgcaggaac ctttggcgca tgcttcaccc 6180

tggcctggga tctgcagcct gggtccagat gcccacaact ggaatctgac gctccttttc 6240

tcttcatggg ggactcccag aggtctctgc aatgaccaga gccccggttg tcccatgcct 6300

cagctgcaac tccagctgac cctccttccc cactctctgg gtggcattac gggggtgtgg 6360

atcccttgcc aagaggttgg catgtgggtg tgctggaatg gcatagggag aatgcaccga 6420

gtttgtttgc ttgggagagg ggcagggggt atccagaaga ttcatgattc gtcatcgcct 6480

ctcttggggg atttttaccc ctttgccctg agttgtgcct ttgggacaaa ggaagccttt 6540

ctttgccagc caacaccctg tactggcggg cgagctcccc agggctggca cgctggggca 6600

gcctctgaat gcacagggtg ggcctagtca gaagaagcct ttcccctgaa atccctctac 6660

ttcccaagca cgcaagcttt ctcctgctgt taaacctgca gtgtgcaagg gacatgggcg 6720

gaggggtcct tcagtcaggc ttctccctgt ctgaggtggc atgacttgga gtgagtttgg 6780

atggggtggc caggtctgag aaggtccccc gccagtgtcc tctgacccat ctgctctctc 6840

ctgccagtgt gcaccggcac agacatgaag ctgcggctcc ctgccagtcc cgagacccac 6900

ctggacatgc tccgccacct ctaccagggc tgccaggtgg tgcagggaaa cctggaactc 6960

acctacctgc ccaccaatgc cagcctgtcc ttcctgcagg tgaggcccgt gggcaaccca 7020

gccaggccct gcctccagct gggctgagcc ctctgtttac aggtgggtgg cagaagaagg 7080

tgccctgccc ttctgtttcc tctcttgttg tggtttctca accaggaagt cctttctaac 7140

atctaacccc cattcatttt actgcagaat cagttgactc tctctataac gtggctggcc 7200

gaggtcatgt ctggatggga tgcgtctgtg tttccgctaa atcttgtgct ctcttgccag 7260

catgatcatg tcccctgtcc acctgctcca gccactatcc ctctcccact tacagcagaa 7320

gaaagggctg gtgagaaagg tggattacag gcccacttct gccactgacg agccctatga 7380

atgtggccta cacccccttta gcttcactgg gtctcagttt ccctatctgt atattgggag 7440

cagttgtgaa gctcagaaga gaaatgtctg tgaaaaggtt atgaacagga gggagagtgg 7500

aaaccaacct gctggatcgt gtccacagac cctggaatgg ggccacatgc ttggtttgtc 7560

aaattgcaga cgccggccgg gtgcgatggc tcatgcctgt aatcccagca ctttgggagg 7620

ccgaggcgga cagatcactt gaggtcggga gttcgagacc agcctgacca acatggagaa 7680

accccgtctc tactgaaaat acaaaattag ccaggcatgg tggcacatgc ctataatccc 7740

agctacttgg gaaggctgag gcaggagaat cacttgaacc tgggagacgg aggttgtggt 7800

gagcctagat cgtgccattg tactccagcc tgggcaacaa gagtgaaact ccgtctcaaa 7860

aaaaaaaaat ttgcagacgc catcccatcc aggcctttgc tttcactgat gaagaaactg 7920

agatacagag agggcagggc acctgttcgg agtttatgaa atgccccccc accattatct 7980

ttcttgatca tataagaatc tggtgaggca aggtagggcg tgatctttat ctctatttta 8040

tcgttttatt taagcgggaa caggactgct cagtggctgg gggccttgcc caagatctcc 8100

aagtactggg gaaccccagg gaggccctgg ggggtggcag tgttcctatt tcagccccac 8160

tctgcttccc cctcccagga tatccaggag gtgcagggct acgtgctcat cgctcacaac 8220

caagtgaggc aggtcccact gcagaggctg cggattgtgc gaggcaccca gctctttgag 8280

gacaactatg ccctggccgt gctagacaat ggagacccgc tgaacaatac cacccctgtc 8340

acaggggcct ccccaggagg cctgcgggag ctgcagcttc gaagcctcac aggtggcctt 8400

caccgtcatt gaaaccttct cttggttatt cagagctgac cagggccact gctaaccagg 8460

gggaggcttt gtgtgcatta gaaatggtgt cccttctggg cagacgcagg cagagcccgg 8520

gaagacgccc tcagaagatt ggaaaaagat tccccttctt cctgggaagt tgtagcttgc 8580

gtcagcacat ataattcaat cgtgagaatg caggctgggt ttttgccccc acttggctga 8640

gtgaagtgta cagtgaacaa cctatgtaac tatttgctgg ccctggagcc gactctgccc 8700

cagagtctgg gtgccaggtg ctttgcccgc atggcccatt tcagtcacgc tgcagtcctg 8760

tcaggaaaaa atcagtgtta ttctcattct acatatgaga aaactgaggc ttgcagatat 8820

aagggccaaa agttacacag ctagtgagtg atggggctga gtttcagact ccacagtctc 8880

ttaaccacca agcagcatgc ccagagtaga gctgagaagg aaggagagag ctgcggtcca 8940

catgagcatc tggacctagc atggacaact cactcctccc tggctctcgc tttgttcttg 9000

ttgcgggtgt ggtggtggtg ggactcaaag acggtaaaga tagctttctc tcctccctgg 9060

ggaatctggg ggttgtttaa aaggcctgct cctcttttag aaggcaggag ggccccaagg 9120

gaagcagaag gtgacagaag gggaaagggt cctctgatca ttgctcaccc cacagagatc 9180

ttgaaaggag gggtcttgat ccagcggaac ccccagctct gctaccagga cacgattttg 9240

tggaaggaca tcttccacaa gaacaaccag ctggctctca cactgataga caccaaccgc 9300

tctcgggcct gtaagccatg cccctccctg ctgcctcttc tctcagacag cctgacccca 9360

gccgcaaact cccaacttac aacccagtgc ctgcccgcca ctgccccagc cgcctacacc 9420

acccatttcc tccctctctg tccctcctgc catctccctg tgcctcttca tctctggggt 9480

tctctgtctt gtctccctct gcttataggt tgtgcctctg gtttgggggc ctctcagcct 9540

gtctgggtcc ctcccttgct gtgcagttgg cctcgtggcc tctgctgctg tttgtgcctc 9600

tctctgttac taacccgtcc tctcgctgtt agacatctct ctcactgcct gtctctggtt 9660

ctgtcctcag gccacccctg ttctccgatg tgtaagggct cccgctgctg gggagagagt 9720

tctgaggatt gtcagagccg tgagtctcag ggaggcctgg agtcagggaa ggggagggct 9780

ggggccgggt ggaatgcagg tgtcatacag gtgacatggg aggggtggga taacaggctt 9840

gggatgtctc ccctgggcca ggtagtctcc ctagaaggtg atgctgatga gggtctggtg 9900

cccagggcgc cactcagccc tcatcctgcc ctttgcccaa cagtgacgcg cactgtctgt 9960
gccggtggct gtgcccgctg caaggggcca ctgcccactg actgctgcca tgagcagtgt 10020
gctgccggct gcacgggccc caagcactct gactgcctgg tatgtgcctc tgctttgtgc 10080
ccaatgtgct ctacccccca ggatgcaagg ggtgggcacc ctgcctggta ctgccctatt 10140
gcccctggca caccagggca aaacagcaca gtgaaagcca gccacctgtc cccccaggcc 10200
tgcctccact tcaaccacag tggcatctgt gagctgcact gcccagccct ggtcacctac 10260
aacacagaca cgtttgagtc catgcccaat cccgagggcc ggtatacatt cggcgccagc 10320
tgtgtgactg cctgtccctg tgagtgccag ggagaaacac agttttctca ttttggtggg 10380
gaggtttgtt tctgtaaatg ggagcatatg gggagcactg tctgcatctt gctttgagag 10440
ctggtcatga cagttcctgc cgagctgcct tgttctttca acagctgtgg agcaggtggc 10500
agtaaggaga ggcagctaag agcccagact tgggagccag actgcctggg tttgaaaccc 10560
agctctatca attagtaggc acgtgaccct cttgctgtgc ctcagtttcc tcatcagtaa 10620
aatgggggca agaatagtcc caactgcata agatggttat aacatttgaa agagttaata 10680
tttgtaaagc tcttagaacg gtgcctggta tgtactaagt gctcctaaat gttagctttt 10740
attctatagc ctggtgaggt cagttttacc tttcgttttg tttttgagac cgaatttagt 10800
tagctctatc gcagtggcgc gatctcggct cactgcaacc tccgcctccc aggttcgtgc 10860
tattctcgtg tctcagcctc ctgagtagct gggattacag gcgcccacca ccatgcctcg 10920
ctaaattttg tatttttagt agagacaggg tttcaccacg ttggccagac tggtctcgaa 10980
ctcctgactt caggcgatcc acctgcctcg gcctctgaaa gtgctgggat tacaggcgtg 11040
agccactgca cccggacttt tttttttttg gcagagtctc gctccattgc ccaggctgga 11100
gtgcagtggt gcaattttgg ctcactgcaa cctctgcctt ccgcattcaa gcaattcttg 11160
tgcctcagac tcttgagtag gtggaactac aggcatgcac caccatggct gggtaatttt 11220
tgtattttta gtagagacgg agtttcacta tgttggccaa gctggtctcg aactcctgac 11280
ctcaagtgat ccacccgcct tggtctccca aagtgctggg attacaggca tgagccatcg 11340
tgcctggcct agctcagttt tatttaacag atcacctatt tactgatggg cgtttatgga 11400
ctgggctcag acctggggaa cctctttcct cctctcacag gaacaggagt gggccttcag 11460
atcctggctg actgtgttag ggagaggaca aaatgtagag ccagaccatt tgggttcaaa 11520
tcctcgctcc tccactcact agcacaatga ccttgaataa tttacagaac tctctgcttt 11580
ggtctccctt tttgcaaaat gggaatctca cagtgctgat cccgtctggt tgttgtgagg 11640
ggtaaatgga tgtcaggtgc tgatgcgtgg tagggcattt aagtattggt tgatattatt 11700
cttcttgtgc ctgggcacgg taatgctgct catggtggtg cacgaagggc cagggtatgt 11760

ggctacatgt tcctgatctc cttagacaac tacctttcta cggacgtggg atcctgcacc 11820
ctcgtctgcc ccctgcacaa ccaagaggtg acagcagagg atggaacaca gcggtgtgag 11880
aagtgcagca agccctgtgc ccgaggtacc cactcactgc cccgaggcc agctgcagtt 11940
cctgtccctc tgcgcatgca gcctggccca gcccaccctg tcctatcctt cctcagaccc 12000
tcttgggacc tagtctctgc cttctactct ctacccctgg ccccccctcag ccctacaagt 12060
gtccctatat cccctgtcag tgtggggagg ggcccggacc ctgatgctca tgtggctgtt 12120
gacctgtccc ggtatgaagg ctgagacggc cccttcccca cccacccccca cctcctcagt 12180
gtgctatggt ctgggcatgg agcacttgcg agaggtgagg gcagttacca gtgccaatat 12240
ccaggagttt gctggctgca agaagatctt tgggagcctg gcatttctgc cggagagctt 12300
tgatgggtaa gagtgggcac gatgacctga gacagtgtca gggcagacag agtcctgagg 12360
atccagatgt ggcagcatct cttcgggatg gcaggagaca gaagtggggg gatcaagaat 12420
gcaaagaaag cagatgggag accagaggag cagggccttt ggtgggtggg ggtgattatt 12480
tttgtaaatg acatgctatc cgtgaacaag gacttgtatg gaggtcagac catctagata 12540
aagtaaaatt ccctttgagt tcatagcagc tttattcaaa atatccccaa attggaaata 12600
actcaaatgt gcatcactag gtgaaggaat aaacaagtgg cagtgtatcc atttggtgaa 12660
gttctactta gcaaccaaag gaaatgaact accgatacaa cataaatgaa tctcagaaac 12720
attacattga gcaaaagaag ccagagacaa gattccatac tgtctgatcc cctttatgtg 12780
aggctctgaa ccgaaaaaac cactctgtgg tgggagagat cagaacggtg gttgccccag 12840
ggtggggggc ttcaaaaggg aggcacacaa ggacatttct ggggtaatag aaatgctctg 12900
tatagtgatt ggggtagtgg atacatgagc gaatccattt gtcaaaactc atcaaactgt 12960
gtgataagag tctgtgcatt ttatttattt cattttattt tttgagatag agtctcactc 13020
tgtcagcagg ctggagtgca gtggtacgat cttggctcac tgcaacctct gcctcctgga 13080
ttcaagcaat tctcctgcct cagtctcctg agtagctggg actacaggtg tgtgccacca 13140
tgcccagcta atttttgtat ttttaataga gatggggttt caccatgttg gcaaggatgg 13200
tctcgatctc ttgacgtcgt gatccgccca cctcagcctc ccaaagtgct gggattacag 13260
gcatgagcca ccacacccgg tgcattttat tgtatataag ttatacttca ataagaaatg 13320
aattggggcc aggcacggtg gctcacgcct gtaatcccag cactttggga ggccgaggca 13380
ggcagatcac ttgaggtcag gagttcaaga ccagcctggc caacatggtg aaaccccatc 13440
tctactaaaa aatataaaaa attagccagg cttcctggca tgcgcctatc atcccagcta 13500
cttgggaggc tgaggcagga gaattgcatg aactcgggag gtggaggttg tagtgagctg 13560
agatttcgct attgcactcc agcctgggcg acagagtgag accctgtctc aaaaagaaaa 13620
aaaaaaaaaa gggtcaggcg ccgtggtgca cacctgtaat cccagcactt tgggaggctg 13680

aagcaggaag attgcttgag cccaggaatt caagaacagc gtgggcaaca tagtgagatc 13740
ccatctctac aaaaaaacac aaaaaattag ccgggcatgg tggtacgcac ctgtagtctc 13800
agctactagg gagactgagg tgggagaatc acctgagcct gggaggtgga ggttgcagtg 13860
ggttgaaatc atgtcactgt actccagcct gggtgacaga atgagaccct gtctcaaaaa 13920
aaaaaaaaaa aaaaaaattc cctttcacac ttcctttacc tccactcccc tttccagagg 13980
gggccatggt taacagtgtg tgtgttcacc tagaccgttt atgcatctgt agacacacac 14040
acagtgaagt gtggttttcg tcgttttggt ggggaggttg gtttctgtaa atgggaacat 14100
atagggagca ctgtctgcac cttgctttga gagccggtca tgacagttcc cattgaactg 14160
ccttgttctt tcaatagctg cagagcaggt ggcggcaagg agaggcagct aagagcccag 14220
acttgggagc cagactgcct gggtttgaaa cccggctcta ccacttacta ggcatgtgac 14280
ccttgtgctg tgcctcagtt tcttcatctg taaagtgggg gcaagaacag tcccaacttc 14340
ataagatggt tataccacca tgcctggcca gatgattata aagtttgaat gagttaatat 14400
ttgtaaagct cttagaacag tgcctggcag atactaggtg ctcctaaatg ttggttttta 14460
ttatgtggct gggtggctcg gggttttatt taacagctcc cctatttact aatagacatt 14520
tagatcatgt tccattttca ctcttacaaa cagttccact ttgtgtgtgg ctctgggaac 14580
atgggccagt gtctccctag gccacattcc tagaaataag atttcttttc tttttttttt 14640
ttttttgaga cagagtctcg ctttatcgcc aggctggtgt gcagtagtgt gatctcggct 14700
cactgcaacc tctgcctccc gggttcaagt gattctcctg cctcagcctc tcgagtaact 14760
gggactatag gcgcgcggca ccacacccag ctaatttttg tatttgtagt agagatgggg 14820
tttcaccatg ttggccagga tggtctccat ctcttgactt cgtgatccgc ccgcctcggc 14880
ctcccaaagt gctgggatta caggcgtgag ccactgagcc caggcagaaa taagatttct 14940
agatcaaagg atataaatac tgttttgata gatgttgccg aactaaggcc tgggctttga 15000
agcccaggat gggaacagct gggctcgatg ggcaaagggt ttgagtgaag gcattcatgg 15060
tggggagtgg ctggcatggc cagtgctggg agtgatgtcc accctgttcc tggccctgct 15120
gactcctctc ctgacccctc cagggaccca gcctccaaca ctgccccgct ccagccagag 15180
cagctccaag tgtttgagac tctggaagag atcacaggtg ggctctgtct ctgcatcctg 15240
ttctgcaggg gctgggagtc cttgtcctgt ccccactcct ttaatctcac cctctgcctg 15300
caggttacct atacatctca gcatggccgg acagcctgcc tgacctcagc gtcttccaga 15360
acctgcaagt aatccgggga cgaattctgc acaagtgagc actgagaaag agggggcctg 15420
atggggagga gtcccaggga ggagtccctg tgggaagctt tgggcctgag ggagtactcc 15480
tgtagcagta acctttccat gaaagtctgc agagtgtgct ggggatggag gaagatgaga 15540

atagcctttg ctgaccggga aggggtccgt ggtaaggtgc ccacctttct cccatagtgg 15600

cgcctactcg ctgaccctgc aagggctggg catcagctgg ctggggctgc gctcactgag 15660

ggaactgggc agtggactgg ccctcatcca ccataacacc cacctctgct tcgtgcacac 15720

ggtgccctgg gaccagctct ttcggaaccc gcaccaagct ctgctccaca ctgccaaccg 15780

gccagaggac gagtgtggta agacagggag cccagtgtgc gcactcccca tctgccagca 15840

cacagcagtg cccagggggc cctggcagca gcgttcttgg acttgtgcag actgcccgtc 15900

tctgtgcacc cttcttgact cagcacagct ctggctggct tggcctcttg gcatggcttc 15960

tctagctggg tcctacctgc cttggcatcc ttccctcccc ctctgtttct gaaatctcag 16020

aactcttcct ctccctacat cggccccacc tgtccccacc cctccagccc acagccatgc 16080

ccacagccag ttccctggtt cacttggacc tggggcctcc cctaaaagtc ccctgcggtc 16140

ccttcctcct cactgcagtg ggcgagggcc tggcctgcca ccagctgtgc gcccgagggc 16200

actgctgggg tccagggccc acccagtgtg tcaactgcag ccagttcctt cggggccagg 16260

agtgcgtgga ggaatgccga gtactgcagg ggtatgaggg gcggaggaga gggtggctgg 16320

aggggtgcat ggggctcctc tcagaccccc tcaccactgt cccttctctc aggctcccca 16380

gggagtatgt gaatgccagg cactgtttgc cgtgccaccc tgagtgtcag ccccagaatg 16440

gctcagtgac ctgttttgga ccggtgagct gctggcgggc tcagagctgg gtggaggggg 16500

gcagcgaggg ggattgccag ggacttggca ggatggcgag atgcagtagg gtgtgctatc 16560

tggtaaaata tccctggaga gggctcagcg ctcagacctg aacagcaaca gagtggcaga 16620

aaaggggcct gggggacact ggggcccttc agactatgaa aaggttctaa ggaggtctgt 16680

gttggtggct gtgactgtgg ctgtgctagg gtggtgagcc ctgtgggctc aggcgtcaga 16740

ctacctggat tcagacccag ctcctgcttc caaccttggt tttttattcc taaaatgggt 16800

attgtaataa tacctacctt gctggggtgt ggcaagaatg aaattaaaca gggcttggca 16860

cagtgaagca cgggaaaggc tttctacaga gcagtgactg ttgttactcg ctgttacacc 16920

ttaggtaatg cgttttcctc tctgggtgcc tcccattttc tggctcaagt ccctgcccag 16980

gatcaagctt ggaggagggc cccgagggag gggccacaga gactgggtga agagcaaggg 17040

tgtttgtccc aggagcatgg cgaaaattgc tgctgggtgg ccttgggaag cacaaagggg 17100

acccaactaa gggcctgatc ctactgccct gggggtgtca gtgccagccc cccacaaatc 17160

ttttctgccc cccccaggag gctgaccagt gtgtggcctg tgcccactat aaggaccctc 17220

ccttctgcgt ggcccgctgc cccagcggtg tgaaacctga cctctcctac atgcccatct 17280

ggaagtttcc agatgaggag ggcgcatgcc agccttgccc catcaactgc acccactcgt 17340

gagtccaacg gtcttttctg cagaaaggag gactttcctt tcaggggtct ttctggggct 17400

cttactataa aaggggacca actctccctt tgtcatatct tgtttctgat gacaaaaata 17460

acacattgtt aaaattgtaa aattaaaaca tgaaatataa attaatgccc tagcagttct 17520
atccccactg ttaataattt gaaatatttt tcctctagtt atttttgtct gtgcacattc 17580
taatatgtat atataagtta acatatatta atattattct ccagttattt ttatctgtgc 17640
acattttaac acacacacac acacacacac acacacacat atgtattttt agacggagtt 17700
tcactctgtc gcccaggctg gagtgcagta gtacaatctt ggctcactgc agcctccacc 17760
tcctgggttt aagcaattct cctgcttccg cctcctgagt agctgggatt acgggaacgt 17820
gctaccttgc ctggctaatt tttgtatttt tagtacatag gatttcacca tgttggccag 17880
gctggtctcg aacccctgac ctcaggtgat ctgccagcct cggtccccca aagtgttggg 17940
attacagcgg tgagccacca tgcccagtca tatatttctt tttaacaaat agaatcatag 18000
atcatacata ttgtttgcaa attgcttttt ctcactttcc agaaccttga aatgtttttc 18060
catgttctaa catggtgatc taccttattc ttttaatttt tcttatttag ttgtctttac 18120
acatgaaaca catgaataca tccttgtgat aaacattttc agtaacataa aagtataaat 18180
gttacaaagc caacgtgccc tttcactcaa ctccctgtcc acccagtctc tcctgtctgc 18240
tgggagaacc accgcattga cttgtgtgtt cacccttcca ggctcttttc tgcacactta 18300
tatagacata ctacatttat attaggtcga gtcaaataag attgctgttt gtgtaaacca 18360
aaaagtgtca agagcctggg cgcagtgact cacacctgta atcccagcac tttgggaggc 18420
tgaggcaggc agatcacttg agatcaggag ttcgagacca atctggccaa catagcgaga 18480
ccccgtctct actaaaaata caaaaactag ccaggtgtgg tgatgctgtt ctgcactttg 18540
ctttcccccc gacttgaggt atcctttctt gtgagtacag acggatctac cacctttatt 18600
ttttttttaa ttactcaacc tgtaacatgg atgtaatttc actttgtttt tgagggatat 18660
tgagcttgtt tccctgtttt tgcagtttat tgcaattgag ctccacacac aagtgagccc 18720
tcttttgtat gccccctagt gggaatacag tgctggcaat gtttatcaca aggatatatt 18780
catgcatttc aatttaaaga caactaaatg agaaaaatta aaagaatatg gatccaggct 18840
gggcatggtg gctcacgcct gtaatcccag cactttggga ggccgaggca ggcagatcac 18900
ctgaggtcag gagttcaaga ccagcctggc caacatggca aaaccccgtc tctactaaaa 18960
atacaaaaat tagccaggcg tggtggtggg cgcctgtaat cccagctatt tgagaggttg 19020
agacaggaga attgcttgaa cctgggcagc ggaggttgca gtgagacgag attgcaccag 19080
tgcactccaa cctgggcaac acagtgcaac tccttctcaa gaaaaaaaag aaaaaaaaaa 19140
agaatatggg tccagatcca tatggatcct agatccagat cacggtgtta gaacatggaa 19200
aaacattgca agattctgct aagtgaaaaa agcatttgca aacagtatgt acagtctata 19260
ttcagaggag gaactgctgg gtcatagatg atatttcata ggtattgcca aaccgttctc 19320

tggagaagtg gtatgggttt accctgggat tcttctatgg agggaatagt tgagctcccg 19380

ggcttgctct tctgggtgcc cctccccgct tcctatccac cacaaggagc tgcaggggag 19440

cggggcatgc cggttccttg gctggagaag gagtctcctt gtgaggtggt agaaggagca 19500

ctgacggcct tgagcccagt ttctgccttt gtcaaatggg gataatgacc cagccacacc 19560

cctcccaggg ttgttgtgag gctggaaagg tggttcccaa gagggtggtt cccagaattg 19620

ttgatgagac tgtttctcct gcagctgtgt ggacctggat gacaagggct gccccgccga 19680

gcagagagcc aggttggcct ggaccccagg atgtaccctt cattgccctt cactccccca 19740

ctggatgctg ggtggtcact gctgtaggga ggggaccccc tgacatatgt cccttcccac 19800

ccactcttcc actgtggaac ctcctgtcat tttccacttc accaagtgac agaggacctg 19860

ctcagatgct gaggggaggg gactgcaagg aaagatggct aggaaaccca gtccctccac 19920

accctagagt aacttgatgc cttgtgaggg acacaggcaa agttcaattc cttggaagtc 19980

aagggagact gagaagagta cagctgcagc actgagggag tgatgaattc ttaactgggg 20040

atggtgggag gcttcgagtg ggaggtggca tttgagctag gctttgagag aggagcaggt 20100

attgcacttg catttaggta gaaagcattg gggtgcaagg tgacactgga gggggaggca 20160

tcaggaaatc caggatgtct tcaaagttct ggtgtcgggg gctgttgagt aagcacagga 20220

ataagggggt caagttagag tcagggtggg gtctgacctg gatgccatag gacctgatcc 20280

ccaagccaca gggtgggact tgactgggca gtggggacct ttggaaagga ctttggggag 20340

aaaaacagac tggagtctgt cttaggcgat catcggtccg tgaaatgagc atgtgttaca 20400

ggcttggtat gtaccagacc ctgtgctaag caaggggggta tggagaggag agggtgacaa 20460

gaatattgga tcaacacccg ggagctccat ctatcccagg atgcactatc ttttttttat 20520

tttttgaga cggagtctca ctctgcctgc aggctggagt gcagtggctc catctcggtt 20580

cactgcaacc tctgcctcct gggttcaagc gcttcttgtg cctcagcctc ccaagtagct 20640

gggattacag gcacatgcca ccacacccag ctaatttttg tatttttagt agagacgggg 20700

tttcaccatg ttggccagga tggtctcgat ctcttgacct caagatccgc ccaccttggc 20760

ctcccaaagt gctgggatta cagacatgag ccaccgtgcc cagccagata cgctatcttt 20820

ttattgagtg attgagacag ggtcttgctc tcttgtccag tcttgaatgt ggtggtgtaa 20880

tcacaggctc actgcagcct tgacctcctg ggctcaagtt acccttctgc agtagctggg 20940

actataggag cgtgccacca cgcctgggta atttaaaaaa tttttttgt atagacaggg 21000

tctcactatg ttgcccgagc tggtctcaaa ctcgtgggct caagtgatcc tccagttttg 21060

gcctcccaaa atgttgggat cacaggagtg agccaccact cctggcgatg agccaagtct 21120

ttttttttt ttttttttt tgatatggag tcttgctctg ttgcccaggc tggagtgcaa 21180

tgacacgatc ttggctcact gcaacctctg cctcccaggt tcaagcagtt caagcaatcc 21240

tcctgtctca gccccccagt agctgggatt acaggcatgc gctaccacgt ccggctaatt 21300
tttgtatttt tagtagagat gaggttttgc catgttggcc aggctggtct tgaactgctg 21360
acctcaggtg atccacctgc ctcggcctcc caaagtgctg ggattacagg tgtgagccat 21420
cgtgcctggc ggagccgagt cttaaaagat gaccctgtgg agaaatggtg gtccaggctg 21480
aagggacagc ctatgcaaac actgggaggt gtggaaaatc atgacctgtg ggtggaaatt 21540
ttggctagaa catcaaaatc atcaggtgta cattcctgta cccatgcagc agtcagaatc 21600
tctgggggtg gggccccaaa attgtatgca tacagactgt gtgctgattt gtgatattac 21660
ttaggatttt ttgactttac aatggtggaa aagcaataat atacattcag tataaaccgt 21720
actttgaata cccatacagc cattctgttt ttcactttta tttttattta tttatttatt 21780
tattatttat tttgagatgt cattttgctg ttgttaccca ggctggagtg caatggcgca 21840
gtcttggctc accgcaacct ccacctctca ggttcaaacg attctcctgc ttcagcctcc 21900
agagtggctg ggattacagg caggcaccac cacacccggc taattttgta tttttagtag 21960
agacggggtt tctccatgtt agtcaggctg gtctcgaact cgagagctca ggtgatctgc 22020
ccatctcagc ctcaagccac catgcccagc cctactttca gtattcaata aattacatag 22080
ccaggcaccg tggctcacac ctgtaatccc agcactttag gaggccaagg tgggaggatc 22140
ctttgaggcc agaagctcga gaccagcctg ggcaacatag tgagacccca tttctacaaa 22200
aaataaaaaa actagctgag tgtggtggcg tgtgtctgta gtcccagcta cttgggcagc 22260
tgaggtggaa agactgcttg agcccagagg tcagggctgc agtgggccat gatctcacca 22320
ctgcactcag cctgggcaac acagcaaggc cctgtctcaa aaataaataa ataaataaca 22380
caaacttatt taacagttta ctataaaata ggctttgtgt cagatgattc tgcccaactg 22440
taagctgctg gcagtgtaaa tgttctgagc acgtgtaagc caggctaggt gtcttaaatg 22500
cattttcagt ttcaacttag aattggttta tcaggacgta gccccttggt gttgaggggc 22560
atgtgtatta acagtctcct tagtgacttt tttttttttg agatggagtc ttgcactggc 22620
cgtagtgcag tggcacaatc tcagctcact gcaacctctt gtctcccggg ttcaagcgat 22680
tctcctgcct cagtctccca agtagctggg attacaggca cccacaccac gcccagctaa 22740
tttttgtgtg tgtgtatttt tagtagagac gggggtttca ctatgttggc caggctggtc 22800
tcgaactcct gaccttgtga tctgcccacc tcagactctc aaagtgctag gattccaggc 22860
atgagccacc gcgcccagag tccttagtga tttttacacc atgaattgtt gaagccctaa 22920
gccagagcca agggcaagag tatagagaat ctggagatgc ggagagggtt ctgattgcct 22980
acaaggagtt tggactttat tgtggaggca gcggggagcc aaggcaggtt ttagagtagg 23040
agagggtcca agcctgtggg tcacccttcc gacttccctt tccgaatgcc aaacaccttc 23100

atgtcccccg tgggccccct ttgtccctcc caccccaaac tagccctcaa tccctgaccc 23160
tggcttccgc ccccagccct ctgacgtcca tcatctctgc ggtggttggc attctgctgg 23220
tcgtggtctt gggggtggtc tttgggatcc tcatcaagcg acggcagcag aagatccgga 23280
agtacacgat gcggagactg ctgcaggaaa cggaggtgag gcggggtgaa gtcctcccag 23340
cccgcgtggg gtctgcaccg gcccccggca ctgacccacc accccctcac cccagctggt 23400
ggagccgctg acacctagcg gagcgatgcc caaccaggcg cagatgcgga tcctgaaaga 23460
gacggagctg aggaaggtga aggtgcttgg atctggcgct tttggcacag tctacaaggt 23520
cagggccagg tcctggggtg ggcggcccca gaggatgggg gcggtgcctg gaggggtgtg 23580
gtcggcagtt ctgatgggag gggcaagagc tggaggcagt gtttggggga gggcagttac 23640
agcggagaag ggagcggggc caagccctag ggtggtgaag gatgtttgga ggacaagtaa 23700
tgatctcctg gaaggcaggt aggatccagc ccacgctctt ctcactcata tcctcctctt 23760
tctgcccagg gcatctggat ccctgatggg gagaatgtga aaattccagt ggccatcaaa 23820
gtgttgaggg aaaacacatc cccaaagcc aacaaagaaa tcttagacgt aagcccctcc 23880
accctctcct gctaggagga caggaaggac cccatggctg caggtctggg ctctggtctc 23940
tcttcattgg ggtttgggga gatatgactc ccgcaaacct agactatttt tttggagacg 24000
gagtcttgct ctgtcaccca ggctggagtg cagtggcgtt atctcggctc actgcaacct 24060
ccacctcctg gactcaagcg attttcatgc ctcaggctcc tgagtagctg ggattacaag 24120
cgcccgctaa tttttttttt tttttgaga cagagtctcg ctctgtcacc caggctagag 24180
tgaaatggtg cggtctcagc tcagcctccc aggttaaagc gattcttctc cctcagtctc 24240
ctgagtagct gggattacag gcgcgagcca ccacgcccgg ctaatttttg tatttttagt 24300
agagatggga tttcaccatg ttggccaggt tggtgtcaaa ctcctgacct catgatccgc 24360
ccgcctcggc ctcccaaagt gctgggatta caggtgtgag ccaccgtgcc cggcctaatc 24420
tttgtatttt tagtagagac agggtttcac catgttgtcc aggctggtac tttgagcctt 24480
cacaggctgt gggccatggc tgtggtttgt gatggttggg aggctgtgtg gtgtttgggg 24540
gtgtgtggtc tcccataccc tctcagcgta cccttgtccc caggaagcat acgtgatggc 24600
tggtgtgggc tccccatatg tctcccgcct tctgggcatc tgcctgacat ccacggtgca 24660
gctggtgaca cagcttatgc cctatggctg cctcttagac catgtccggg aaaaccgcgg 24720
acgcctgggc tcccaggacc tgctgaactg gtgtatgcag attgccaagg tatgcacctg 24780
ggctctttgc aggtctctcc ggagcaaacc cctatgtcca caaggggcta ggatggggac 24840
tcttgctggg catgtggcca ggcccaggcc ctcccagaag gtctacatgg gtgcttccca 24900
ttccagggga tgagctacct ggaggatgtg cggctcgtac acagggactt ggccgctcgg 24960
aacgtgctgg tcaagagtcc caaccatgtc aaaattacag acttcgggct ggctcggctg 25020

ctggacattg acgagacaga gtaccatgca gatgggggca aggttaggtg aaggaccaag 25080
gagcagagga ggctgggtgg agtggtgtct agcccatggg agaactctga gtggccacct 25140
ccccacaaca cacagttgga ggacttcctc ttctgccctc ccaggtgccc atcaagtgga 25200
tggcgctgga gtccattctc cgccggcggt tcacccacca gagtgatgtg tggagttatg 25260
gtgtgtgatg gggggtgttg ggaggggtgg gtgaggagcc atggctggag ggaggatgag 25320
agctgggatg gggagaatta cggggccacc tcagcatgtg aagggaggga aggggctgcc 25380
tgtgccccac cttgcagggt ctgtgcactt cccaggatta gggaaagacc gggtagggtc 25440
tgtctcctgg catcacatct ccccctgcta cctgccatga tgctagactc ctgagcagaa 25500
cctctggctc agtacactaa agctccctct ggccctccca ctcctgaccc tgtctctgcc 25560
ttaggtgtga ctgtgtggga gctgatgact tttggggcca aaccttacga tgggatccca 25620
gcccgggaga tccctgacct gctggaaaag gggggagcggc tgccccagcc ccccatctgc 25680
accattgatg tctacatgat catggtcaaa tgtgcgtggc tgagctgtgc tggctgcctg 25740
gaggagggtg ggaggtcctg ggtggaggag cccacaaggg gcatgaaagg ggaccaggat 25800
gtatgtagac ccaggagccc tagtatgtta ggagcctcaa aaccttcttg tatccctttt 25860
acagtcaaag tccaaagcca ctcttgagga acactcttgt acaaaattaa gctgggcaca 25920
gtggctcatg cctgtaatcc cagtactttt ggaggctgag gtgggaggat cccttgaagc 25980
caggagttca agaccagcct gggcaacata gtgagatcct atctctacaa aaaataaaaa 26040
aattatctgg gtgtggtggt gtgtgccagt agtcccagct actcaggaga ggctgaggca 26100
ggaagatcac ttgagcctag tttaaggttg cagtaagcta tgattgcacc actgaaatcc 26160
agcctgggtg acagagcgaa acctcatctc aaaaaaataa aaaagcaaac aaaaagaaaa 26220
aaaaaattaa aagggaaact agaagagatg ccaaaggttc tggctgaaga ccccagagtc 26280
tggtgctact tctctaccac ctgagggctt tgggctgtcc cttgggactg tctagaccag 26340
actggagggg gagtgggagg ggagaggcag caagcacaca gggcctggga ctagcatgct 26400
gacctccctc ctgccccagg ttggatgatt gactctgaat gtcggccaag attccgggag 26460
ttggtgtctg aattctcccg catggccagg gacccccagc gctttgtggt catccaggta 26520
ctgggcctct gtgccccatc cctgcctgtg gctaagagca ccctcctgca gagggtggga 26580
aggagagatg agtccagtat gccaggcccc tcacggaagg ctgcatgctg ggctggggag 26640
gggccaccat cctgcctctc cttcctccac agaatgagga cttgggccca gccagtccct 26700
tggacagcac cttctaccgc tcactgctgg aggacgatga catgggggac ctggtggatg 26760
ctgaggagta tctggtaccc cagcagggct tcttctgtcc agaccctgcc ccgggcgctg 26820
ggggcatggt ccaccacagg caccgcagct catctaccag ggtcagtgcc ctcggtcaca 26880

ctgtgtggct gtctgcttac ctcccccaac cccggtggac tagggtccct ttctctgatg 26940

ttccctcaac tgtcacctct caaggaaacc ccattatccc tacaaaaaat tcttactgcc 27000

ttccaacccc tgtgacccca ttctctccac ggtgactgtg tcataccccca aaggtgacct 27060

ctgtttttct cctgtgaccc tgtcaccttc catggagtcc ccatcccaga tccgtgagtg 27120

acccccatca tgactttctt tcttgtcccc agagtggcgg tggggacctg acactagggc 27180

tggagccctc tgaagaggag gcccccaggt ctccactggc accctccgaa ggggctggct 27240

ccgatgtatt tgatggtgac ctgggaatgg gggcagccaa ggggctgcaa agcctcccca 27300

cacatgaccc cagccctcta cagcggtaca gtgaggaccc cacagtaccc ctgccctctg 27360

agactgatgg ctacgttgcc cccctgacct gcagccccca gcctggtatg gagtccagtc 27420

taagcagaga gactgatggg caggggaggt gggaccttca gcccagggtc cactgtgggg 27480

gcagagggag tggcagagac accggggttc cttcccctaa tgggtcacct tctcttgacc 27540

tttcagaata tgtgaaccag ccagatgttc ggccccagcc cccttcgccc cgagagggcc 27600

ctctgcctgc tgcccgacct gctggtgcca ctctggaaag gcccaagact ctctccccag 27660

ggaagaatgg ggtcgtcaaa gacgtttttg cctttggggg tgccgtggag aaccccgagt 27720

acttgacacc ccagggagga gctgcccctc agccccaccc tcctcctgcc ttcagcccag 27780

ccttcgacaa cctctattac tgggaccagg acccaccaga gcggggggct ccacccagca 27840

ccttcaaagg gacacctacg gcagagaacc cagagtacct gggtctggac gtgccagtgt 27900

gaaccagaag gccaagtccg cagaagccct gatgtgtcct cagggagcag ggaaggcctg 27960

acttctgctg gcatcaagag gtgggagggc cctccgacca cttccagggg aacctgccat 28020

gccaggaacc tgtcctaagg aaccttcctt cctgcttgag ttcccagatg gctggaaggg 28080

gtccagcctc gttggaagag gaacagcact ggggagtctt tgtggattct gaggccctgc 28140

ccaatgagac tctagggtcc agtggatgcc acagcccagc ttggcccttt ccttccagat 28200

cctgggtact gaaagcctta gggaagctgg cctgagaggg gaagcggccc taagggagtg 28260

tctaagaaca aaagcgaccc attcagagac tgtccctgaa acctagtact gccccccatg 28320

aggaaggaac agcaatggtg tcagtatcca ggctttgtac agagtgcttt tctgtttagt 28380

ttttactttt tttgttttgt ttttttaaag atgaaataaa gacccagggg gagaatgggt 28440

gttgtatggg gaggcaagtg tggggggtcc ttctccacac ccactttgtc catttgcaaa 28500

tatattttgg aaaac 28515

<210> 39
<211> 23
<212> DNA
<213> Artificial

<220>

<223> Synthesis

<220>
<221> misc_signal
<222> (1)..(23)

<400> 39
ctgaactggt gtatgcagat tgc 23

<210> 40
<211> 17
<212> DNA
<213> Artificial

<220>
<223> Synthesis

<220>
<221> misc_signal
<222> (1)..(17)

<400> 40
ttccgagcgg ccaagtc 17

<210> 41
<211> 5511
<212> DNA
<213> homo sapiens

<400> 41
acacacacac acccctcccc tgccatccct ccccggactc cggctccggc tccgattgca 60

atttgcaacc tccgctgccg tcgccgcagc agccaccaat tcgccagcgg ttcaggtggc 120

tcttgcctcg atgtcctagc ctaggggccc ccgggccgga cttggctggg ctcccttcac 180

cctctgcgga gtcatgaggg cgaacgacgc tctgcaggtg ctgggcttgc ttttcagcct 240

ggcccggggc tccgaggtgg gcaactctca ggcagtgtgt cctgggactc tgaatggcct 300

gagtgtgacc ggcgatgctg agaaccaata ccagacactg tacaagctct acgagaggtg 360

tgaggtggtg atggggaacc ttgagattgt gctcacggga cacaatgccg acctctcctt 420

cctgcagtgg attcgagaag tgacaggcta tgtcctcgtg gccatgaatg aattctctac 480

tctaccattg cccaacctcc gcgtggtgcg agggacccag gtctacgatg ggaagtttgc 540

catcttcgtc atgttgaact ataacaccaa ctccagccac gctctgcgcc agctccgctt 600

gactcagctc accgagattc tgtcaggggg tgtttatatt gagaagaacg ataagctttg 660

tcacatggac acaattgact ggagggacat cgtgagggac cgagatgctg agatagtggt 720

gaaggacaat ggcagaagct gtccccccctg tcatgaggtt tgcaaggggc gatgctgggg 780

tcctggatca gaagactgcc agacattgac caagaccatc tgtgctcctc agtgtaatgg 840

tcactgcttt gggcccaacc ccaaccagtg ctgccatgat gagtgtgccg gggctgctc 900

aggccctcag gacacagact gctttgcctg ccggcacttc aatgacagtg gagcctgtgt 960
acctcgctgt ccacagcctc ttgtctacaa caagctaact ttccagctgg aacccaatcc 1020
ccacaccaag tatcagtatg gaggagtttg tgtagccagc tgtccccata actttgtggt 1080
ggatcaaaca tcctgtgtca gggcctgtcc tcctgacaag atggaagtag ataaaaatgg 1140
gctcaagatg tgtgagcctt gtgggggact atgtcccaaa gcctgtgagg gaacaggctc 1200
tgggagccgc ttccagactg tggactcgag caacattgat ggatttgtga actgcaccaa 1260
gatcctgggc aacctggact ttctgatcac cggcctcaat ggagacccct ggcacaagat 1320
ccctgccctg gacccagaga agctcaatgt cttccggaca gtacgggaga tcacaggtta 1380
cctgaacatc cagtcctggc cgccccacat gcacaacttc agtgtttttt ccaatttgac 1440
aaccattgga ggcagaagcc tctacaaccg gggcttctca ttgttgatca tgaagaactt 1500
gaatgtcaca tctctgggct tccgatccct gaaggaaatt agtgctgggc gtatctatat 1560
aagtgccaat aggcagctct gctaccacca ctctttgaac tggaccaagg tgcttcgggg 1620
gcctacggaa gagcgactag acatcaagca taatcggccg cgcagagact gcgtggcaga 1680
gggcaaagtg tgtgacccac tgtgctcctc tgggggatgc tggggcccag gccctggtca 1740
gtgcttgtcc tgtcgaaatt atagccgagg aggtgtctgt gtgacccact gcaactttct 1800
gaatggggag cctcgagaat ttgcccatga ggccgaatgc ttctcctgcc acccggaatg 1860
ccaacccatg gagggcactg ccacatgcaa tggctcgggc tctgatactt gtgctcaatg 1920
tgcccatttt cgagatgggc cccactgtgt gagcagctgc cccccatggag tcctaggtgc 1980
caagggccca atctacaagt acccagatgt tcagaatgaa tgtcggccct gccatgagaa 2040
ctgcacccag ggtgtaaag gaccagagct tcaagactgt ttaggacaaa cactggtgct 2100
gatcggcaaa acccatctga caatggcttt gacagtgata gcaggattgg tagtgatttt 2160
catgatgctg ggcggcactt ttctctactg gcgtgggcgc cggattcaga ataaaagggc 2220
tatgaggcga tacttggaac ggggtgagag catagagcct ctggacccca gtgagaaggc 2280
taacaaagtc ttggccagaa tcttcaaaga gacagagcta aggaagctta aagtgcttgg 2340
ctcgggtgtc tttggaactg tgcacaaagg agtgtggatc cctgagggtg aatcaatcaa 2400
gattccagtc tgcattaaag tcattgagga caagagtgga cggcagagtt ttcaagctgt 2460
gacagatcat atgctggcca ttggcagcct ggaccatgcc cacattgtaa ggctgctggg 2520
actatgccca gggtcatctc tgcagcttgt cactcaatat ttgcctctgg gttctctgct 2580
ggatcatgtg agacaacacc gggggcact ggggccacag ctgctgctca actggggagt 2640
acaaattgcc aagggaatgt actaccttga ggaacatggt atggtgcata gaaacctggc 2700
tgcccgaaac gtgctactca agtcacccag tcaggttcag gtggcagatt ttggtgtggc 2760

tgacctgctg cctcctgatg ataagcagct gctatacagt gaggccaaga ctccaattaa 2820

gtggatggcc cttgagagta tccactttgg gaaatacaca caccagagtg atgtctggag 2880

ctatggtgtg acagtttggg agttgatgac cttcggggca gagccctatg cagggctacg 2940

attggctgaa gtaccagacc tgctagagaa gggggagcgg ttggcacagc cccagatctg 3000

cacaattgat gtctacatgg tgatggtcaa gtgttggatg attgatgaga acattcgccc 3060

aacctttaaa gaactagcca atgagttcac caggatggcc cgagacccac cacggtatct 3120

ggtcataaag agagagagtg ggcctggaat agcccctggg ccagagcccc atggtctgac 3180

aaacaagaag ctagaggaag tagagctgga gccagaacta gacctagacc tagacttgga 3240

agcagaggag gacaacctgg caaccaccac actgggctcc gccctcagcc taccagttgg 3300

aacacttaat cggccacgtg ggagccagag ccttttaagt ccatcatctg gatacatgcc 3360

catgaaccag ggtaatcttg gggagtcttg ccaggagtct gcagtttctg ggagcagtga 3420

acggtgcccc cgtccagtct ctctacaccc aatgccacgg ggatgcctgg catcagagtc 3480

atcagagggg catgtaacag gctctgaggc tgagctccag gagaaagtgt caatgtgtag 3540

gagccggagc aggagccgga gcccacggcc acgcggagat agcgcctacc attcccagcg 3600

ccacagtctg ctgactcctg ttaccccact ctccccaccc gggttagagg aagaggatgt 3660

caacggttat gtcatgccag atacacacct caaaggtact ccctcctccc gggaaggcac 3720

cctttcttca gtgggtctca gttctgtcct gggtactgaa gaagaagatg aagatgagga 3780

gtatgaatac atgaaccgga ggagaaggca cagtccacct catcccccta ggccaagttc 3840

ccttgaggag ctgggttatg agtacatgga tgtggggtca gacctcagtg cctctctggg 3900

cagcacacag agttgcccac tccaccctgt acccatcatg cccactgcag gcacaactcc 3960

agatgaagac tatgaatata tgaatcggca acgagatgga ggtggtcctg gggggtgatta 4020

tgcagccatg gggggcctgcc cagcatctga gcaagggtat gaagagatga gagcttttca 4080

ggggcctgga catcaggccc cccatgtcca ttatgcccgc ctaaaaactc tacgtagctt 4140

agaggctaca gactctgcct ttgataaccc tgattactgg catagcaggc ttttccccaa 4200

ggctaatgcc cagagaacgt aactcctgct ccctgtggca ctcagggagc atttaatggc 4260

agctagtgcc tttagagggt accgtcttct ccctattccc tctctctccc aggtcccagc 4320

ccctttttccc cagtcccaga caattccatt caatctttgg aggcttttaa acattttgac 4380

acaaaattct tatggtatgt agccagctgt gcactttctt ctctttccca accccaggaa 4440

aggttttcct tattttgtgt gctttcccag tcccattcct cagcttcttc acaggcactc 4500

ctggagatat gaaggattac tctccatatc ccttcctctc aggctcttga ctacttggaa 4560

ctaggctctt atgtgtgcct ttgtttccca tcagactgtc aagaagagga aagggaggaa 4620

acctagcaga ggaaagtgta attttggttt atgactctta accccctaga aagacagaag 4680

cttaaaatct gtgaagaaag aggttaggag tagatattga ttactatcat aattcagcac 4740

ttaactatga gccaggcatc atactaaact tcacctacat tatctcactt agtcctttat 4800

catccttaaa acaattctgt gacatacata ttatctcatt ttacacaaag ggaagtcggg 4860

catggtggct catgcctgta atctcagcac tttgggaggc tgaggcagaa ggattacctg 4920

aggcaaggag tttgagacca gcttagccaa catagtaaga cccccatctc tttaaaaaaa 4980

aaaaaaaaaa aaaaaaaaaa actttagaac tgggtgcagt ggctcatgcc tgtaatccca 5040

gccagcactt tgggaggctg agatgggaag atcacttgag cccagaatta gagataagcc 5100

tatggaaaca tagcaagaca ctgtctctac aggggaaaaa aaaaaaagaa actgagcctt 5160

aaagagatga aataaattaa gcagtagatc caggatgcaa aatcctccca attcctgtgc 5220

atgtgctctt attgtaaggt gccaagaaaa actgatttaa gttacagccc ttgtttaagg 5280

ggcactgttt cttgtttttg cactgaatca agtctaaccc caacagccac atcctcctat 5340

acctagacat ctcatctcag gaagtggtgg tgggggtagt cagaaggaaa aataactgga 5400

catctttgtg taaaccataa tccacatgtg ccgtaaatga tcttcactcc ttatccgagg 5460

gcaaattcac aaggatcccc aagatccact tttagaagcc attctcatcc a 5511

<210> 42
<211> 22701
<212> DNA
<213> homo sapiens

<400> 42
ctctcacaca cacacacccc tcccctgcca tccctccccg gactccggct ccggctccga 60

ttgcaatttg caacctccgc tgccgtcgcc gcagcagcca ccaattcgcc agcggttcag 120

gtggctcttg cctcgatgtc ctagcctagg ggcccccggg ccggacttgg ctgggctccc 180

ttcaccctct gcggagtcat gagggcgaac gacgctctgc aggtgctggg cttgcttttc 240

agcctggccc ggggctccga ggtgggcaac tctcaggcag gtaagtggcg cgagagcacc 300

ggcgggctcg gcacctggga gccggaaccc agtgcgcgca gcctcggagg gtatgggcac 360

ggtctcaggc ggcgcggggt tgtgggtgct gccccccggtt tgccaggacc acctgggaga 420

ggggcggtca ggctcgggtt atcggcgtgg tccggccgag ggcggcattc cgggaccctc 480

acgccaccct tctccagagc gtcgccgacc ctctaattgg tctccccaga agaggctgag 540

gccgaaacag tagttcacac ttctgagggg ccctgcaggg aggggagcag ggaacttcat 600

tctgtaaaca ggaggtgctt ggaggtgggg gccttggcgg gaagggtctc ggtttgctcg 660

ccaacccccct gccccccacc cgcgccgatt tcagctaccc ctagtttcgt tgttttgccg 720

acagggcgga gctacagaag gttggagggg gttgttgttc tctggtgttg gaaaaacagg 780

agcggcacct cctcttccgt gagtgagcct gccctgggga ggtctgagat taaccagagg 840

gccaagttca ggtgacatca ggcaggaggc ccaacagagg ctggcgcccc ctttcctcag 900
tatagcagag cttaagcaac atctctttgt caagacccag gtcaacacaa ctcatattta 960
ttgagcatct actatacaca aggccctggg ccaggagctg taagggcaag gatgtccagc 1020
ctctggtctt ttctctcccc aacctgagga tcaagagggc acctctgcta ctttctaagc 1080
ctcctgcctt ggggagtcct tcctgggctc agcttgtgcc tcccgccccc attttgctta 1140
ttgtctgaca ctgtctctag gaccctagac agagccccgg attgctcttc tccagtcctc 1200
ccccgactcc catgctatct gagcccaccc ctttggggtg tctctgggac cgtggacacc 1260
tgaggactga agttctgtgg atctcctccc ctcccctcag atctcagctt ggggtttggc 1320
acagccaggg ccccttcccc agtgtgggag tggaagaaac cacctgtgct tccctcacag 1380
ttgctgggcc taaatttaga tcctgggatt tttagatgtg aacactccca gctggtggag 1440
gggggtggtc tggggactgg cgtgggaggg agcagtggag tgactgtata actgtcccat 1500
ccagactcct gcaatcttca cccagaaacc caggagtacc agagtctggc caccctccct 1560
ggggaggcag gaggcaggca tggttgggtc tctttaccct ttatctgggt cctgcagcct 1620
ctgggcatcc ggccctgtct cagtccttct atagcccctt tgtcctggct gtgatggggg 1680
tgggggatgt tggaggggag gcctctggtt gaggaggggc tggagattct ggctctatcc 1740
cacccctagt gctctctacc aaaggagggc ctgtgacact gcccctccct atgctcccgg 1800
ttcctgggta cagcagggat ttttatgatt cccttcccct gccctgctcc ccaagctgcc 1860
tagctcctcc ccagaggtgt tgtttgtgct cccttcctgc ccaggccctt tgccccctgt 1920
ttgtgtaata tggactttac cctcagggta gcagggaact gggctacctc taacactgta 1980
gccttccaag cacagacaca aagtctgcac aaacacttat gggcacgtgg gatagatggg 2040
gccacctgaa atacttcctg caaggaaacc caactataga ttcctgagcc agcaggacca 2100
atgtgtacgt gttcgtgtgt acattgtgtg tgtatgtgtg tgcccacact actacttccc 2160
tgtgcagaaa gcttggctcc tcccttatct ggggagaagt gttggcacta taacttggga 2220
aaggggggtat cctgccagga aggggattgg ggtggggctg ttccagaaat gactaacctt 2280
cctagtctct ttcatttcaa cccaaggacc ctggagttcc cagctcctct ggaactagct 2340
ctctttgctg ggactagcca accttcatgg ggagtgataa ggagccctcc aaggtcaaga 2400
agtcagacta ggggtgtatg ttataggagg gattggggcc tcaccagtct ccctccctcc 2460
attcccactg ttgcctccca ctgagctacc accgcctcag ggaagggtgg ctggaacagg 2520
tggtatctac cccctactcc ccaccccaca catggtcttt ccctgaacca gaggaaagag 2580
actggggtag ggcttcagag tccaggactt cccatagccc gttgtccacc acatttgcaa 2640
agaagagtga actcccaagg ctgacatgcc atgtacctct agtctaggct ctcccctagt 2700

gtgggtgagg attgccatgg tgaaaggctt tttcatgaac ctcttctaac aatgaaattg 2760
tgtggaggct caatatgggg catctgctac tatctctctc caggttcctc tgtatttgct 2820
gaaaaatact ctagggctgg aaagtgatgc tgaggttgct agagtgtgtt gggatggggg 2880
agagagtgag aaggaaaccc tgagtttagg aaggcgggga ggcaactagc tccttatctt 2940
tcagctttaa agacaaagct cccattgacc ccccctcacc ccagcactgc cagagctccc 3000
ccctctactg aggtcacttg tctgagccca aggcttgagg gtggagggga gtgctgctga 3060
ggacggggtg tctagggaca gggtggggca gccccccctcc tggatagaat cgcctcattg 3120
tgggctggac tgtggcccca ggcactgccc ccaccctctg cccccatccc accctcagta 3180
gacacaatag gggctgtgta ctagtcccaa agagatattt attccaggac ctagagagag 3240
gcaggatgag ggtagagaag tgagtgccct agttggaggg ggagaggagg gtaatcaaag 3300
ttgcggcctt ttcctaactt ctcttttcta gggagagaaa caaattccct gtcttccttc 3360
tcagttaacc ccttagtacc caaaagaagc acagaggggt cccaggttga aaaaggaaat 3420
cttttcacct tcccattcat ggaatggtaa ggggattctg agaagagaga aagctctcag 3480
gccactacag cttctgccta tcgcttgtgg gagggttgga ggcaaatgcc atctgatcct 3540
gtctaatgta actggaagag ggcaaccaag ggggtgatct ttggggatgg cagatgggct 3600
gagaatttgt gtccagccct cagccactct tccctctgct ttgaacagtg tgtcctggga 3660
ctctgaatgg cctgagtgtg accggcgatg ctgagaacca ataccagaca ctgtacaagc 3720
tctacgagag gtgtgaggtg gtgatgggga accttgagat tgtgctcacg ggacacaatg 3780
ccgacctctc cttcctgcag gttagtgagc ccaccctcct tcctcaacct gctcctcttt 3840
attctcccct agaaccctcc ttccttcttc agggctacct tctgctggag ttcacccttc 3900
ctaagactca ggagttccta agattcaaaa ccgtgtattt atggggacag tggctgtcat 3960
ctgggaccta tggtctcact gttgtagcca gggatatata gggggcaggg tcaggggcag 4020
gtggtgttct gtggatagtg caaggtcagc agggactagt gcagagagaa acctgaggac 4080
caagaggtta cctggggaga tgaggaaggg gccctactgg tatgaggcac tttgaggaga 4140
aagctgcctg tcttcactcc cagaagtgac acagcagtgt gacacagtct actccctact 4200
cccaaatagg aattagcaag agttaaggcc aggtgcagtg gctcatgcct gtaatcccag 4260
cactttggga ggccaaggaa ggcagatcac ttgaggtcag gagttcaaga ccagcctggg 4320
caatgtggtg aaacgctgtc tctacaaaaa tacaaaaatt agctgggtat ggtggcatgc 4380
acctgtagtc ccagctactt ggagggctga ggtgggagga ttgcttgagc ccaggagttt 4440
gacgctgcag tgagcgagat tgtgccactc gtaacagagc gagaccctgt ctcaccaaaa 4500
aaaaaaaaaa aggccaagct cggatcacct gaggtcagga gttcgagacc agcctgacca 4560
acatggaaaa accacatctc tactaaaaat acaaaattag ccaggtgtag tggcacatgc 4620

ctgtaatccc agctacttgg gaggctgcgg caggagaatt gcttgaaccc gggaggtgga 4680
ggttgtggtg agccaagatc gcactattgt actccagcct gggcaacaag agcgtaactc 4740
cgtctcaaat tttaaaaaaa agaaaaaaag aaaggaaaga aagaaggaag aattaaaaca 4800
gttaaagagt ctttaatgcc tgaaggagga gaggagattg agattatttt gccctgttgt 4860
ctctctcatt tacataatct gctctgtcac agtggattcg agaagtgaca ggctatgtcc 4920
tcgtggccat gaatgaattc tctactctac cattgcccaa cctccgcgtg gtgcgaggga 4980
cccaggtcta cgatgggaag tttgccatct tcgtcatgtt gaactataac accaactcca 5040
gccacgctct gcgccagctc cgcttgactc agctcaccgg tcagttcccg atggttcctt 5100
ctggcctcac ccctcagcca gcccaagact ggtacctcct tgatgatgac ccaagactgc 5160
tcactctaag tgcctcttcc aaggtgcctg tcaccttggc cgctgtctaa aggtccattg 5220
ctccctaagc aatagagggc ccccagtagg gggagctagg ggcatctgct ccagggaaag 5280
gaaccctgtg tccttgtggg gctggagtca gagctggatc tgttaaccgt ttttctaatt 5340
tcaaagtaca gtgtaccgga ggccaggcct gatggcttac acctgtaatc ccagcatttt 5400
gggaggccaa ggagggcaga tcacttgaga tcaggagttt gagaccagcc tggccaacat 5460
ggcgaaaccc tgtctctact aaaaatacaa aaaaataaaa taaaataaaa aattagctgg 5520
ctatagtggt gcgcacctgt aatcccagct gttcatgagg ctgaggcagg agactcgctt 5580
gaacctggga ggtggaggtt gcagtgagct gagattgcac cactgcactc tagcgtaagt 5640
gacagtgaga ctccgtctca aaaaaaaaaa aaaaaaaaaa aaaaagcctg ggcgcggtgg 5700
ctcacgcctg taatcccagc actttgggag gccaaggcag gtggatcaca aggtcaggag 5760
atcgagacca tcctggctaa cacggtgaaa ccccgtctct actaaaaata caaaaaatta 5820
gccaggtgtg gtggcgggcg cctgtagtcc gagctactcg ggaggctgag gcaggagaat 5880
ggcgtgaacc cgggaggcgg agcttgcagt gagccaagat tgcaccactg cactccagcc 5940
tgggcgacag agcgagactc caaaaaaaaa aaaaaaaaaa aaaaccaaag tacagtatac 6000
ctggatgtcc ctccttccct aggaattcta cctttactct cctaaaccaa acccctatga 6060
gctggaggaa tataggggtt aaaaaccacc tgtccatctt ctgcttctcc atgtcccagt 6120
cagttggaaa acatatgggc agggcttggg ggagggaatg ttgagtcaga aatctctccc 6180
tctctccctt cccctccccc actagctaaa ccggatctgg acaggtgact gaggaggcag 6240
gagtttcttt tggcctgact cctcatctta taaagggagt cttctctgca gcttagattt 6300
aattggacct atctgtctgc ctcattctcc cactcctgag tctcaggtgt ccttttggat 6360
gggtggagag gtaaggaaga ggcgttccgc tgcggccctt aaccctgtca cttctttccc 6420
tacctcagag attctgtcag gggtgttta tattgagaag aacgataagc tttgtcacat 6480

ggacacaatt gactggaggg acatcgtgag ggaccgagat gctgagatag tggtgaagga 6540
caatggcaga agctgtaagt ggccgtgatc aagattgctc cccagtccca ccaaaccaga 6600
gtgactccct tctttccatc atccttacat tcctgatctg aacccgcctc cccagtgaac 6660
aaacacctca ggtccctgac tcagcagccc accagggcag accattccag tctcctggaa 6720
tctaaaccac agaggaggtg tttcaagaaa aggagcaggc cgagcatggt ggctcatgcc 6780
tataatcctg gcactttggg aagccaaggt gggaggatcg cttgagccca ggagtccaag 6840
accagcctga gtaacatagc aaaaaatcta caaaaaatta aaaaaatcag acaggcgtag 6900
tggctcgcac ctgtaatccc ggctactcag gaggctgagg cagaggattg cttgagccca 6960
ggaggctgca gctgcagtga gctgtgattg tgccattgta ctctagcctg ggcaacagag 7020
tgagaccctg tctcaaaaaa aaaaaaaaaa aatccctgag tactaagcag ggaagccaga 7080
tctttagact ccacatctgt tactcgttcc actagaatat actcccattt ccttggagcc 7140
caccttcccc tgaccattca catgcatata ttcctgcata tattcagttt gcccaggagg 7200
aactaagttc ctgggttggg actaggacta aggttggcat ttgccccagt ccctcccctt 7260
cagctgccca gtgggtggtg tggaggcgtg gccgcgcccc ttgttgacag gtccacttga 7320
gcccagccct gctctccaag ggcagggagg gacacagccc tggctttttg cttcccggga 7380
ttgaggtgcc tgtgtactga catcataccc cgttgattaa aacaagcctt tcttagccct 7440
gatggcccct tgtgttgcct tccttcccaa ccaggtcccc cctgtcatga ggtttgcaag 7500
gggcgatgct ggggtcctgg atcagaagac tgccagacat gtgggtttga aattccctcc 7560
aaaaacttca ctcatacgct ttcatatccc ttcctcccca agcctgggtc aacactgtgg 7620
gggaggcatg agcagtggcc tcagaattca gtcctaggag ccctaacagc catgctttct 7680
ctccttccat agtgaccaag accatctgtg ctcctcagtg taatggtcac tgctttgggc 7740
ccaaccccaa ccagtgctgc catgatgagt gtgccggggg ctgctcaggc cctcaggaca 7800
cagactgctt tgtatgtacc ctttccattg cctgggttct gaaattggga tgtggccttt 7860
gaggaggagg taggggtaca cacgtaacat aaatctgatg agcctccttt tttcccaggc 7920
ctgccggcac ttcaatgaca gtggagcctg tgtacctcgc tgtccacagc ctcttgtcta 7980
caacaagcta actttccagc tggaacccaa tccccacacc aagtatcagt atggaggagt 8040
ttgtgtagcc agctgtcccc gtaagtgtct gaggggaagg aacaatgatc aacaatagta 8100
gatccaagat tttagacaaa attgtggaag ggaaaaagaa tccagttggt gataaatagg 8160
gagattggtg aatggttatg atcatctaac cactccagtg agtgaccctt acgtccagtc 8220
ctcccatgac ttcagctatc acccttactt ctgctccttg tagcaacaaa tagtgaagag 8280
acttttgaat ctatagggca gcacttaagg gatctagggt ggcagatggg gacaaatcca 8340
gtgcagagct ggagggagcc taggcccaga gcaagggttc cattggtagc tggtgatgtt 8400

cctccctcat ctctaatggt gtcctcctcc tcttccctag ataactttgt ggtggatcaa 8460
acatcctgtg tcagggcctg tcctcctgac aagatggaag tagataaaaa tgggctcaag 8520
atgtgtgagc cttgtggggg actatgtccc aaaggtgggt aggagatggt aagaagttgt 8580
aaagagacag cctttcctct gagcctgcgc agaccacccc cactgaacct ctcttacatt 8640
tgcagcctgt gagggaacag gctctgggag ccgcttccag actgtggact cgagcaacat 8700
tgatggattt gtgaactgca ccaagatcct gggcaacctg gactttctga tcaccggcct 8760
caatgggtta gagatcctgc cttccctcct tagaccccag cccacgcacc cctcacagtt 8820
catttcattg gccaaaactt tcctatgtgg agctgactag gaatcaaagt cataaaattc 8880
tagcctgtta caaaggacct gaaagaatgc ttaacacatc ctccatccag gccttcggtc 8940
ccctcaggaa catctttgag caattcaata tcgccctgcc aaggaacaag ggacaggaac 9000
aacatatcct ccttcttaaa gttttctttt ttattctttt ttcttttttg agatagggtc 9060
ttgctctgtc acctaggctg gagtgcagtg gcgtgatctc gactcactgt agcctcgacc 9120
tcctgggctc aagtgatccc aagtagctgg gactataggc acacaccatc atacttgact 9180
aatttttttg tatttttttg tagagacagg gtcttgctat gttgcccagg ctgatctcga 9240
actcctgtgc tcaagcaatc ctcccatctt ggcctcccaa agtgctaggg atcacagcac 9300
ccaacctcct tcttaaagtt ttgtaaaagt tcttccttag atttggataa aaatctgtct 9360
ccaggctggg cccggtggct catgcctata atcccagcac tttgggaggc cgaggtgggc 9420
ggattacgag gtcagatcga gaccatcctg gctaacatgg tgaaatgcca tctctactaa 9480
aaacacaaaa attagctggg tgtggtggtg cacatgcctg taatcccagc tactcaggag 9540
gctgaggcac gagaatcact tgaacccagg aggcggaaat tgcagtgaac cgagattaca 9600
ccaccgcact ccagcctggc gacagagcga gactctttct caaaaaaaaa aaagaaaaga 9660
aaagaaaatt ctgtctcccc atgactttta gctgttttca ctcattctgc tccttggagc 9720
aaaaagaaca aagggacttt ctagtctata ggacagcatt taaaatgtgt gtgtgtgtgt 9780
aaaaaaaacc cactatgacc acctgttttt ttttttcctt taattttta ttttgacata 9840
attttagatc tacactaaag ttgcaagaat ggtataaaat tccccatata cttttttttt 9900
ttttttaaga caaagtctca ctctgttccc caggctagag tgcagtggtg caatcttggc 9960
tcactgcaac ctccgcctcc tctgcctccc gggttcaagc aattctcctg cctcagtctc 10020
ctgagtagct ggaattatag gtgtgtgcca tcatgcccgg ctaatttttg tatttttagt 10080
agagacaggg tttcaccatg taggccaggc tggtctcgaa ctcctgacct caagtgatcc 10140
acccgcccca gcttcccaaa gtgttgggat tacaagtgtg agccaccgcg tctggccccc 10200
catacactct tttacccaga tcctccaaat gttaacatac cacatatggc cgggcacagt 10260

ggctcatgcc tataatccca gcactttggg aggctgaggc aggtggatca ctaggtgtgg 10320
atcacgaggt caagagattg agaccatcct ggccaacatg gtgaaacctc atctctacta 10380
aaaatacaaa aattagctgg gcgtggtggt gtgcgcctgt agtcccagtt actcaggagg 10440
ctgaggcagg agaatagctt gaacctggca ggcagaggtt gcagtgagcc gagatcgcgg 10500
cactgcactc cagcctggtg aaagagcgag actctgtccc ccgccaaaaa aaataccaca 10560
tacgctttat cacttctctc tctctctgtc tctctctaca cacacacaca cacacacaaa 10620
acacatgcta ttgtttttct ggaccacgtg agggtaaatt ttcacacatg gttctttctt 10680
acccctgtta tatttcagcg tatattcctt aaaaataata ttttcttaca taaccacagc 10740
atagttgttt gaatcggaaa attaacatta acacaaaata ttatctaagc tacagacctt 10800
attcagattt cactaattgt cctcctaagg tttgggatca tacattacat tcagttatcg 10860
tggcacttca atctccttta taacagctcc tcaggttttg tttatctttc atgatattct 10920
tgatgagtat agattaggta atgggcagca tgttcttcag tttggattag tttgatgtgt 10980
cctcatgatt agattcaagt ttttgtagtt ttttttttgag acaaggtctg gctctattgc 11040
ccaggctgga atacagtggc atgatctcag ctcactgcaa cctctgcctc ccgtgctcaa 11100
gcgagcacct cagccccctg agtagctggg attacaggtg catgccacca tgcttggcta 11160
attttatata tatatatata tatatatata tataaataat atatataaat ataaatatat 11220
atatataaat aatatatata aatatatata aacataaata ttatatacta tttatatatt 11280
tatatatgtg tgtgtatata tatatatatt tttttttttt tttgagacgg agtctcgctc 11340
ttgttgccca ggctggagtg cagtggcgtg atctcagctc acgcaacctc cacctctcag 11400
gttcaagcca ttctctatag agacagggtt gcaccatgtt gtccaggatg gtctcgaact 11460
catgagctca agtgatcctc ctgtctcagc ctcccaaagt gctgggatta taggcatgcg 11520
ccgctgccag gctggagttt gataagaaca ccacagaggc tgtgagctca gggcatccta 11580
ttgaggatgt acgtgatgtt gatttgtccc agcactcaca atgatgtctc tggtcactta 11640
gttaaggtga tatctgtcag gtttttctac tgtaaagtta ctatttttcc attcacaatt 11700
aatgaatgtc ttgggataat tgcctgaatc aattattgtt atgatagttg ccaaatgata 11760
attttctaat tccattattc cttctgcatt tgtttgttgg cattctactg ttaggaagag 11820
tctttccagc tgagcacagt ggcttatgcc tgtaatctca gccctttggg agccagtggg 11880
aaaattgctt gggcccagga gttcaaggtt acagtgagct atgatggcac tactgctctc 11940
cagccagtgc actcactctg cacaacagag tgagaccctg tctcttaaaa aaaaaaaaaa 12000
aaaaaggcca ggtgcagtgg ctcatgcctg taattccagc actttgggag gccgaggcgg 12060
gcggatcaca aggtcaggag ttcaagacca gcctggccag catggtgaaa ccctgtctct 12120
actaaaaata caaaaaatta gccaggcatg gtggtgtgct cctgtattcc cagctactta 12180

ggaagctgag gcaggagaat cacttgaacc caggaggtgg aggttgcagt gagctgagat 12240
tgctgccact gtactccagc ctgggcgata gagcaagact ctgtctcaaa aaaaaaaaaa 12300
aaaaaaaaaa aaaaaaaagg tcttcctttc ctatttactc ttatggatac ttattttatt 12360
ctagtcaatg gttataatcc tttacaatca ttatttattt tagccagccc ctccaagttg 12420
gctcctgtgt tctttgggct gtacccttaa ttctttgagt cttgtcttgt ttgcacaaga 12480
tgctctgggc ttatattata tttcccccat cccagccatt tctccaagga atgtttcctt 12540
ttagtggaga atgatattta gaaaccaaat gctgaggctg ggtgtgctca ttgccattga 12600
gttatacctt taccttattg actggtttct actgttctat tcagagaccc ctggcacaag 12660
atccctgccc tggacccaga gaagctcaat gtcttccgga cagtacggga gatcacaggt 12720
gagtggcaga gagtttgccc tttctagaag aataggtgaa ccactggcat aaattgcggt 12780
ataactactt gagaaaatca cgtcccaagt tataggggag gagccaggag aacccaagaa 12840
agaagaaggc tccctgccca tatgcctctc tccaacccct caggttacct gaacatccag 12900
tcctggccgc cccacatgca caacttcagt gttttttcca atttgacaac cattggaggc 12960
agaagcctct acaagtgagt aaagggtatg gaggaaatgg catcttcagg caatgaagcc 13020
tgtgtcatag gcattcttta gtaaaataca aggcactgtc tcatacagca gtgcctcaaa 13080
accaaagggt ttcagagttt cacgaggaaa aggcaaaagg aggggggattc cctctcagtg 13140
gatctgacta gcactgagca aatttcttga ctaacatgaa tcctttgaat agttaatgtt 13200
cccttagtag tctctcctct catcctgtct ccttattctc agccggggct tctcattgtt 13260
gatcatgaag aacttgaatg tcacatctct ggcttccga tccctgaagg aaattagtgc 13320
tgggcgtatc tatataagtg ccaataggca gctctgctac caccactctt tgaactggac 13380
caaggtgctt cgggggccta cggaagagcg actagacatc aagcataatc ggccgcgcag 13440
agactgcggt gagggaaagg gtctgctagg tggtgagaat agggagtcag ggaggagagg 13500
gctgaaagga ctattctgcc ctagacgtgg gagtagggtt gagggatgga accaaggaga 13560
agggggctgt taggctggaa gcagtaacga ggaagaataa tgaagagagg gcttgctggg 13620
agtcctcaga ctcctctcct aacccacccc ttcctttcca gtggcagagg gcaaagtgtg 13680
tgacccactg tgctcctctg gggatgctg gggcccaggc cctggtcagt gcttgtcctg 13740
tcgaaattat agccgaggag gtgtctgtgt gacccactgc aactttctga atgggtacag 13800
taaggggagc cagtcaagga tgggtggggg tggggccctg caatggaact gttcaggtgg 13860
catacaataa aagtctttag acagctttct gcatgtgcct tggtgggatt gaggtaggag 13920
acctgtggtt gtgagatcgg agcatgaagg tcaggacttg gaagtgaccc ccccctccct 13980
ttattcccca ctacagggag cctcgagaat ttgcccatga ggccgaatgc ttctcctgcc 14040

acccggaatg ccaacccatg gagggcactg ccacatgcaa tggctcggta tactagtagc 14100

accaggatct ccaagggaga cagagaaggg gcaatacttg gagcatctgg ggaatgatat 14160

ggctaaggat agcacagaga ggccagataa tgctagggcc tgcagataga agatcctgaa 14220

tgtctgggtt ggtctttgct gggaggtatg gaattgacct tgggatctga ttcttcctga 14280

ccttctctct tccactcagg gctctgatac ttgtgctcaa tgtgcccatt ttcgagatgg 14340

gccccactgt gtgagcagct gcccccatgg agtcctaggt gccaagggcc caatctacaa 14400

gtacccagat gttcagaatg aatgtcggcc ctgccatgag aactgcaccc aggggtcagt 14460

gatgggataa taaggagagg gggtcaggtg gaagggtagg agcacagaac tagagtgagg 14520

gaagcagaaa gaagagagag gctgtgattc aagaatcact cccagctggc cgggcgcagt 14580

ggctcacacc tgtaatccca gcactgtggg aggccgaggt gggtggatca cctgaggtcg 14640

ggagttcgag accagcctga ccaatatgga gaaaccctgt ctctaccaaa aatttaaaat 14700

tagcccggcg tggtggcgca tgcctgtaat cccagctacg cgggaggctg aggcaggaga 14760

atttcttgaa cccaggaggc agaggttgcg gtgagctgag attgcatcat tgtactccag 14820

cctgggcaac aagagtgaaa ctctgtctca aaaaaaaaaa aagaatcact cccagctgtg 14880

tagcgaagga ttggagaaag ggaaaatcag taacagcaca aaattacacc acagttttgg 14940

gaacctggaa taacctcagt tcaagggagt ttcacagaag aggggcttgg ggagagctag 15000

tgagctggag gtggaggcca tgtcttggga tcagctctgg gctccaggat gggatgccac 15060

ggtaagttct gaaacaagct tttatatgtt aggctgttga aattgagcct ctgctgtcca 15120

agctctcatt taaggtggtg actttcttcc ctaggtgtaa aggaccagag cttcaagact 15180

gtttaggaca aacactggtg ctgatcgggt atgatggggt tggagattct ggaaactggg 15240

gatatttggg agttgggaga gaggtggtta cctggagaga agagggaggc tgtcttcatt 15300

ctggcctttt atgtatgcag tccactatca ctggacactt gggactcaag aatgcaggct 15360

tctggacttc ccttcctaaa attaactttc agtagtctaa gactggtcca gatttaggtt 15420

ggtcccttca gtgcttaagg atatatatgt gaatgttaat ttcttgcccc aggtcagcat 15480

catacettca acacaagtat agttgacatt tgtaaggaag atgcaaaccc aggataatgt 15540

tgggtttcta tatatcccat agcaaaaccc atctgacaat ggctttgaca gtgatagcag 15600

gattggtagt gattttcatg atgctgggcg gcacttttct ctactggcgt gggcgccgga 15660

ttcagaataa aagggctatg aggcgatact tggaacgggg tgaggtgagt acttagctta 15720

cttttgtttt ttcttttctt tttttgcatg tcctggaagt ctctttatag cttaattttg 15780

agtggtaccc tgtgcaccca ggggtcagtg atgggataaa tgtcactccc ctcctctttc 15840

cccagagatt tgatcccttt cttcaaggaa gtagtgtggt cccctagaag aacactggtc 15900

agagaaatgg gaggcatgca ttctagtcct gattttgcca ttaatttgcc acatgacttt 15960

gaagaagtta cttatcttct ctgtgcctcg gtttatgcat ctatacagag gaaataacat 16020
ttgtccttcc aggatggctg taagggtaaa gggggatgat gtatgtgaaa gtgctttgga 16080
aagcacagag cactgtataa aaggtactca aggtggtaat agtactacca actctcccta 16140
gctgtcccct tccccacttt gtgctcctcc atcaaaggga aaacccaacc cctttgattc 16200
ctgatctcat gagcacaaat aacttcctca gttctcaggg tctgtacctc aatatgccta 16260
taatccattc caggactaac ggtgcttcct cttcctgccc tttcagctgt gctgcttttg 16320
gcattcacct atgaggagcg ggttggagtg ggacatggga atggcctttc ctgagtaact 16380
ccttcccatt tgctcctcag agcatagagc ctctggaccc cagtgagaag gctaacaaag 16440
tcttggccag aatcttcaaa gagacagagc taaggaagct taaagtgctt ggctcgggtg 16500
tctttggaac tgtgcacaaa gtgagtgacc cataggaatt ctggagaggt ggggaaggca 16560
tctagggcaa aggggtgaaa gatttttgca taggattgac ctagggagaa tgaccttatg 16620
ccaactcctg ccccaaactt cccagggagt gtggatccct gagggtgaat caatcaagat 16680
tccagtctgc attaaagtca ttgaggacaa gagtggacgg cagagttttc aagctgtgac 16740
agatgtaagt gaaggaaatt ctgtatgccg ctaggagaga ggacaatatt agatacaatc 16800
atgtagaagc agggtcctgt gcttctcagc agctactatg ttagccagaa tgttgggggt 16860
ggggggggcct gggctggctg tgcacatgct gagtgtatgt gaacctgttg gtttcctaga 16920
taatacccttt tgtgtctctt agcatatgct ggccattggc agcctggacc atgcccacat 16980
tgtaaggctg ctgggactat gcccagggtc atctctgcag cttgtcactc aatatttgcc 17040
tctgggttct ctgctggatc atgtgagaca acaccggggg gcactggggc cacagctgct 17100
gctcaactgg ggagtacaaa ttgccaaggt gagagaagcc tggaggaatt ctgtgataag 17160
aactgcttgt ctgggggcca gccaggaaaa agtgagaagg ttgaagttct gagaggtgag 17220
gtccccaacc cccgggctgc agactggtac cagtccatgg cctgttagga accaggccac 17280
agagcatgtg agcggcaggc aagcgagtga agcttcatct gtatttacag tcagtcccca 17340
tcacttgcat taccgcccga gttccgcctc ctgtcagatc aggggcagca ttagattctc 17400
ttaggagctt gacttctatt gtgaactgtg catgtgaagg atctaggttg tgcactcctt 17460
atgagaatct aactaatgcc tgatgatctg aggtggaaaa atttcatccc aaaaccaacc 17520
ctccccttcc cctggaaaaa ctgtcttcca caaaaccagt ccctggtgcc aaaaaaggtt 17580
ggggaccact gctgagaggt accttcaaga tttgggggaa ttccagatct cagtgactga 17640
ttcccccaac cttaagaata ctttcttccc ctatacctac agggaatgta ctaccttgag 17700
gaacatggta tggtgcatag aaacctggct gcccgaaacg tgctactcaa gtcacccagt 17760
caggttcagg tggcagattt tggtgtggct gacctgctgc ctcctgatga taagcagctg 17820

ctatacagtg aggccaaggt gaggagacac aaagggtaag gaggcggggg tggagtgaag 17880
catggggata gggagcagcc agtggtctct tccagaggca agcagatgct tcatggtaag 17940
ttcaaggaga gaaggctgca gatgccagat attttagttc agagggcaac aaataaaata 18000
atgatcaaga acttgggact ggccgggcgc ggtggctcac gcctgtaatc ccaacacttc 18060
gggaggccaa ggcgggtgga tcacaaggtc aggagatcaa gaccatcctg gctagcacgg 18120
tgaaaccccg tctctactaa atatacaaaa aaaaaaaaaa ttagccaggc gtggcggcat 18180
gcatctgtac tcccagctac tcgggaggct gaggcaggag aatggcgtga acccaggagg 18240
cggagcttgc agtgggccga gatcgcacca ctgcactcca gtctgggcga cagagcgaga 18300
ctccgtctca aaaaaaaaaa aaaaagaatt tgggacttgg aaatcctaag aaaatttgtg 18360
gaaataaact tgtgatacct ctatctttaa tccgcagact ccaattaagt ggatggccct 18420
tgagagtatc cactttggga aatacacaca ccagagtgat gtctggagct atggtcagtg 18480
catctggatg ccctctctac catcactggc cccagtttca aatttacctt ttgagacccc 18540
ctcttagaat ctctaagcac ttcagatttt tgtgttagat caggttctgc cttcccttca 18600
cttcatgccc atgtctacta ttttgccagt gactagtcca tgtcttcctg caacaggtgt 18660
gacagtttgg gagttgatga ccttcggggc agagccctat gcagggctac gattggctga 18720
agtaccagac ctgctagaga agggggagcg gttggcacag ccccagatct gcacaattga 18780
tgtctacatg gtgatggtca agtgtgagtt acctgctgag cccaaccatt ttctcttttt 18840
ttcttttttt ttcttttttt tttttttttg agacagagtc tcacaattgt cacccaggct 18900
ggagtgcaat ggtgcaatca atcttggctc actacaacct ccgcctctcg ggttcaagag 18960
attctcctgc ttcagcctcc ggagtagctg ggattacagg cgcccgccac acctggataa 19020
ctgttacact tttagtagag atggggtttc accatgttgg ccaggctggt ctcaaactcc 19080
tgacctcagg tgatccgcct gcctcagctt cccaaagtgc tgggattaca ggtgtgagcc 19140
atcatgctcg gcctgactgc agccattttc tgacttccct ctgtactcct cttatggctc 19200
tattcctttt ttttttatgg agtctcgctc tgttgcccat actggagtgc agtagcgtga 19260
ccttggctca ccgtgacctc cacgttccag gtttaagttc ttctgtctca gcctcccaga 19320
tagctgggac tttaggcgtg caccaccacg cccagctaat tttttttgt ctttttagta 19380
gagatggggt ttcactatgt tggccaggct ggtctcaaag tcccgacctc aggtgatcca 19440
cccgccttgg cctcccaaag tgctgggatt ataggtgtga gccaccgcgc ccggccatgg 19500
aatgtattct cttttatgtc tctacctcct acatcttatc tccaggttgg atgattgatg 19560
agaacattcg cccaaccttt aaagaactag ccaatgagtt caccaggatg gcccgagacc 19620
caccacggta tctggtcata aaggtgagta gggagtagga ggtgctaagg aaatttagaa 19680
aaaggaggag ttggctggaa ccaggattcc ccctaacaat cacctatcga tatagagaga 19740

gagtgggcct ggaatagccc ctgggccaga gccccatggt ctgacaaaca agaagctaga 19800
ggaagtagag ctggagccag aactagacct agacctagac ttggaagcag aggaggacaa 19860
cctggcaacc accacactgg gctccgccct cagcctacca gttggaacac ttaatcggcc 19920
acgtggggta agacaacttc taattaccca acactttgca ccctgagccc tcacaaaccc 19980
tacagatacc cagattaact actcaaaggc ccccatggtg aatgtagatt tctcccttca 20040
tcttaacctt ttccttattt tttcatccta gagccagagc cttttaagtc catcatctgg 20100
atacatgccc atgaaccagg gtaatcttgg ggagtcttgc caggtaagtt ctgttgctga 20160
gaggctgggt tttaggatca gattgatacg agtagtatgg aagacattag aaacctctga 20220
ggtttaatca gtgtcctgca aaaaagaagg cagtgagggc cgggcgagtt ggctcacacc 20280
tgtaatccca gcactttggg aggccagaga gagtggatca cctgaggtta ggagtttgag 20340
accagcctgg ccaacatggt gaaaccccgt ctctacccaa aatacaaaaa ttagctgggt 20400
gtggtggtgc acacctgtaa tcacagctac tcaggaggct gagacaggag aatcgcttga 20460
acccgggagg cagaggttgc agtgagctga gattgtacca ctgcactcca gcctgggtga 20520
cagagcaaga ccctgtctct taaaaaaaaa aaaaaaaggc caggtgcggt ggctcacgcc 20580
tgtaatccta gcactttggg aggccgaggt gggcggatca tgaggtcagg agttcgagac 20640
cagcctgacc aacatggcaa aaccctgtct gtactaaaaa tacaaaaact agctgcacat 20700
gatggcaggt gcctgtaatc ccagctactc gggaggctga ggcaggagaa tcacttgaac 20760
agggaagcag aggctgcagt gagccaagat aatgccactg cactccagcc tgggcgacaa 20820
gaacaagact ccacctcaaa aaaaaaaaaa aaaaaaaaaa aaggcagtga acaacccaat 20880
atccttctaa acaaatctct cttctttcct catcatgtaa atttccttgc attattttct 20940
gtttattttc ttccttagga gtctgcagtt tctgggagca gtgaacggtg cccccgtcca 21000
gtctctctac acccaatgcc acggggatgc ctggcatcag agtcatcaga ggggcatgta 21060
acaggctctg aggctgagct ccaggagaaa gtgtcaatgt gtaggagccg gagcaggagc 21120
cggagcccac ggccacgcgg agatagcgcc taccattccc agcgccacag tctgctgact 21180
cctgttaccc cactctcccc acccgggtta gaggaagagg atgtcaacgg ttatgtcatg 21240
ccagatacac acctcaaagg tgcctgactc ttcctagggc tttcctcaat ttttcctcga 21300
attctttccc cgggctcctc ttttttcttc tctgatcata tgcctctctg tcctattaat 21360
tttttcaaac tttcccctac cctcatgaag ttcttcacat acctagcctt tcttctcaac 21420
ccccaggtac tccctcctcc cgggaaggca ccctttcttc agtgggtctc agttctgtcc 21480
tgggtactga agaagaagat gaagatgagg agtatgaata catgaaccgg aggagaaggc 21540
acagtccacc tcatccccct aggccaagtt cccttgagga gctgggttat gagtacatgg 21600

atgtggggtc agacctcagt gcctctctgg gcagcacaca gagttgccca ctccaccctg 21660

tacccatcat gcccactgca ggcacaactc cagatgaaga ctatgaatat atgaatcggc 21720

aacgagatgg aggtggtcct gggggtgatt atgcagccat gggggcctgc ccagcatctg 21780

agcaagggta tgaagagatg agagcttttc aggggcctgg acatcaggcc ccccatgtcc 21840

attatgcccg cctaaaaact ctacgtagct tagaggctac agactctgcc tttgataacc 21900

ctgattactg gcatagcagg cttttcccca aggctaatgc ccagagaacg taactcctgc 21960

tccctgtggc actcagggag catttaatgg cagctagtgc ctttagaggg taccgtcttc 22020

tccctattcc ctctctctcc caggtcccag ccccttttcc ccagtcccag acaattccat 22080

tcaatctttg gaggctttta aacattttga cacaaaattc ttatggtatg tagccagctg 22140

tgcactttct tctctttccc aaccccagga aaggttttcc ttattttgtg tgctttccca 22200

gtcccattcc tcagcttctt cacaggcact cctggagata tgaaggatta ctctccatat 22260

cccttcctct caggctcttg actacttgga actaggctct tatgtgtgcc tttgtttccc 22320

atcagactgt caagaagagg aaagggagga aacctagcag aggaaagtgt aattttggtt 22380

tatgactctt aaccccctag aaagacagaa gcttaaaatc tgtgaagaaa gaggttagga 22440

gtagatattg attactatca taattcagca cttaactatg agccaggcat catactaaac 22500

ttcacctaca ttatctcact tagtccttta tcatccttaa aacaattctg tgacatacat 22560

attatctcat tttacacaaa gggaagtcgg gcatggtggc tcatgcctgt aatctcagca 22620

ctttgggagg ctgaggcaga aggattacct gaggcaagga gtttgagacc agcttagcca 22680

acatagtaag acccccatct c 22701

<210> 43
<211> 25
<212> DNA
<213> Artificial

<220>
<223> Synthesis

<220>
<221> misc_signal
<222> (1)..(25)

<400> 43
aataaaaggg ctatgaggcg atact 25

<210> 44
<211> 25
<212> DNA
<213> Artificial

<220>
<223> Synthesis

<220>
<221> misc_signal
<222> (1)..(25)

<400> 44
agcttcctta gctctgtctc tttga 25

<210> 45
<211> 6456
<212> DNA
<213> homo sapiens

<400> 45
gagttgtgcc tggagtgatg tttaagccaa tgtcagggca aggcaacagt ccctggccgt 60

cctccagcac ctttgtaatg catatgagct cgggagacca gtacttaaag ttggaggccc 120

gggagcccag gagctggcgg agggcgttcg tcctgggact gcacttgctc ccgtcgggtc 180

gcccggcttc accggacccg caggctcccg gggcagggcc ggggccagag ctcgcgtgtc 240

ggcgggacat gcgctgcgtc gcctctaacc tcgggctgtg ctctttttcc aggtggcccg 300

ccggtttctg agccttctgc cctgcgggga cacggtctgc accctgcccg cggccacgga 360

ccatgaccat gaccctccac accaaagcat ccgggatggc cctactgcat cagatccaag 420

ggaacgagct ggagcccctg aaccgtccgc agctcaagat ccccctggag cggcccctgg 480

gcgaggtgta cctggacagc agcaagcccg ccgtgtacaa ctaccccgag ggcgccgcct 540

acgagttcaa cgccgcggcc gccgccaacg cgcaggtcta cggtcagacc ggcctcccct 600

acggccccgg gtctgaggct gcggcgttcg gctccaacgg cctggggggt ttccccccac 660

tcaacagcgt gtctccgagc ccgctgatgc tactgcaccc gccgccgcag ctgtcgcctt 720

tcctgcagcc ccacggccag caggtgccct actacctgga gaacgagccc agcggctaca 780

cggtgcgcga ggccggcccg ccggcattct acaggccaaa ttcagataat cgacgccagg 840

gtggcagaga aagattggcc agtaccaatg acaagggaag tatggctatg gaatctgcca 900

aggagactcg ctactgtgca gtgtgcaatg actatgcttc aggctaccat tatggagtct 960

ggtcctgtga gggctgcaag gccttcttca agagaagtat tcaaggacat aacgactata 1020

tgtgtccagc caccaaccag tgcaccattg ataaaaacag gaggaagagc tgccaggcct 1080

gccggctccg caaatgctac gaagtgggaa tgatgaaagg tgggatacga aaagaccgaa 1140

gaggagggag aatgttgaaa cacaagcgcc agagagatga tggggagggc aggggtgaag 1200

tggggtctgc tggagacatg agagctgcca acctttggcc aagcccgctc atgatcaaac 1260

gctctaagaa gaacagcctg gccttgtccc tgacggccga ccagatggtc agtgccttgt 1320

tggatgctga gccccccata ctctattccg agtatgatcc taccagaccc ttcagtgaag 1380

cttcgatgat gggcttactg accaacctgg cagacaggga gctggttcac atgatcaact 1440

gggcgaagag ggtgccaggc tttgtggatt tgaccctcca tgatcaggtc caccttctag 1500
aatgtgcctg gctagagatc ctgatgattg gtctcgtctg gcgctccatg gagcacccag 1560
ggaagctact gtttgctcct aacttgctct tggacaggaa ccagggaaaa tgtgtagagg 1620
gcatggtgga gatcttcgac atgctgctgg ctacatcatc tcggttccgc atgatgaatc 1680
tgcagggaga ggagtttgtg tgcctcaaat ctattatttt gcttaattct ggagtgtaca 1740
catttctgtc cagcaccctg aagtctctgg aagagaagga ccatatccac cgagtcctgg 1800
acaagatcac agacactttg atccacctga tggccaaggc aggcctgacc ctgcagcagc 1860
agcaccagcg gctggcccag ctcctcctca tcctctccca catcaggcac atgagtaaca 1920
aaggcatgga gcatctgtac agcatgaagt gcaagaacgt ggtgcccctc tatgacctgc 1980
tgctggagat gctggacgcc caccgcctac atgcgcccac tagccgtgga ggggcatccg 2040
tggaggagac ggaccaaagc cacttggcca ctgcgggctc tacttcatcg cattccttgc 2100
aaaagtatta catcacgggg gaggcagagg gtttccctgc cacggtctga gagctccctg 2160
gctcccacac ggttcagata atccctgctg cattttaccc tcatcatgca ccactttagc 2220
caaattctgt ctcctgcata cactccggca tgcatccaac accaatggct ttctagatga 2280
gtggccattc atttgcttgc tcagttctta gtggcacatc ttctgtcttc tgttgggaac 2340
agccaaaggg attccaaggc taaatctttg taacagctct ctttccccct tgctatgtta 2400
ctaagcgtga ggattcccgt agctcttcac agctgaactc agtctatggg ttggggctca 2460
gataactctg tgcatttaag ctacttgtag agacccaggc ctggagagta gacattttgc 2520
ctctgataag cactttttaa atggctctaa gaataagcca cagcaaagaa tttaaagtgg 2580
ctcctttaat tggtgacttg gagaaagcta ggtcaagggt ttattatagc accctcttgt 2640
attcctatgg caatgcatcc ttttatgaaa gtggtacacc ttaaagcttt tatatgactg 2700
tagcagagta tctggtgatt gtcaattcat tccccctata ggaatacaag ggcacacag 2760
ggaaggcaga tcccctagtt ggcaagacta ttttaacttg atacactgca gattcagatg 2820
tgctgaaagc tctgcctctg gctttccggt catgggttcc agttaattca tgcctcccat 2880
ggacctatgg agagcagcaa gttgatctta gttaagtctc cctatatgag ggataagttc 2940
ctgatttttg tttttatttt tgtgttacaa aagaaagccc tccctccctg aacttgcagt 3000
aaggtcagct tcaggacctg ttccagtggg cactgtactt ggatcttccc ggcgtgtgtg 3060
tgccttacac aggggtgaac tgttcactgt ggtgatgcat gatgagggta aatggtagtt 3120
gaaaggagca ggggccctgg tgttgcattt agccctgggg catggagctg aacagtactt 3180
gtgcaggatt gttgtggcta ctagagaaca agagggaaag tagggcagaa actggataca 3240
gttctgaggc acagccagac ttgctcaggg tggccctgcc acaggctgca gctacctagg 3300

aacattcctt gcagaccccg cattgccctt tgggggtgcc ctgggatccc tggggtagtc 3360
cagctcttct tcatttccca gcgtggccct ggttggaaga agcagctgtc acagctgctg 3420
tagacagctg tgttcctaca attggcccag caccctgggg cacgggagaa gggtggggac 3480
cgttgctgtc actactcagg ctgactgggg cctggtcaga ttacgtatgc ccttggtggt 3540
ttagagataa tccaaaatca gggtttggtt tggggaagaa aatcctcccc cttcctcccc 3600
cgccccgttc cctaccgcct ccactcctgc cagctcattt ccttcaattt cctttgaacc 3660
tataggctaa aaaagaaagg ctcattccag ccacagggca gccttccctg ggcctttgct 3720
tctctagcac aattatgggt tacttccttt ttcttaacaa aaaagaatgt ttgatttcct 3780
ctgggtgacc ttattgtctg taattgaaac cctattgaga ggtgatgtct gtgttagcca 3840
atgacccagg tgagctgctc gggcttctct tggtatgtct tgtttggaaa agtggatttc 3900
attcatttct gattgtccag ttaagtgatc accaaaggac tgagaatctg ggagggcaaa 3960
aaaaaaaaaa aagtttttat gtgcacttaa atttggggac aattttatgt atctgtgtta 4020
aggatatgtt taagaacata attcttttgt tgctgtttgt ttaagaagca ccttagtttg 4080
tttaagaagc accttatata gtataatata tatttttttg aaattacatt gcttgtttat 4140
cagacaattg aatgtagtaa ttctgttctg gatttaattt gactgggtta acatgcaaaa 4200
accaaggaaa aatatttagt tttttttttt ttttttgtat acttttcaag ctaccttgtc 4260
atgtatacag tcatttatgc ctaaagcctg gtgattattc atttaaatga agatcacatt 4320
tcatatcaac ttttgtatcc acagtagaca aaatagcact aatccagatg cctattgttg 4380
gatattgaat gacagacaat cttatgtagc aaagattatg cctgaaaagg aaaattattc 4440
agggcagcta attttgcttt taccaaaata tcagtagtaa tatttttgga cagtagctaa 4500
tgggtcagtg ggttcttttt aatgtttata cttagatttt cttttaaaaa aattaaaata 4560
aaacaaaaaa aaatttctag gactagacga tgtaatacca gctaaagcca aacaattata 4620
cagtggaagg ttttacatta ttcatccaat gtgtttctat tcatgttaag atactactac 4680
atttgaagtg ggcagagaac atcagatgat tgaaatgttc gcccaggggt ctccagcaac 4740
tttggaaatc tctttgtatt tttacttgaa gtgccactaa tggacagcag atattttctg 4800
gctgatgttg gtattgggtg taggaacatg atttaaaaaa aaactcttgc ctctgctttc 4860
ccccactctg aggcaagtta aaatgtaaaa gatgtgattt atctgggggg ctcaggtatg 4920
gtggggaagt ggattcagga atctggggaa tggcaaatat attaagaaga gtattgaaag 4980
tatttggagg aaaatggtta attctgggtg tgcaccaggg ttcagtagag tccacttctg 5040
ccctggagac cacaaatcaa ctagctccat ttacagccat ttctaaaatg gcagcttcag 5100
ttctagagaa gaaagaacaa catcagcagt aaagtccatg gaatagctag tggtctgtgt 5160
ttcttttcgc cattgcctag cttgccgtaa tgattctata atgccatcat gcagcaatta 5220

tgagaggcta ggtcatccaa agagaagacc ctatcaatgt aggttgcaaa atctaacccc 5280

taaggaagtg cagtctttga tttgatttcc ctagtaacct tgcagatatg tttaaccaag 5340

ccatagccca tgccttttga gggctgaaca aataagggac ttactgataa tttacttttg 5400

atcacattaa ggtgttctca ccttgaaatc ttatacactg aaatggccat tgatttaggc 5460

cactggctta gagtactcct tcccctgcat gacactgatt acaaatactt tcctattcat 5520

actttccaat tatgagatgg actgtgggta ctgggagtga tcactaacac catagtaatg 5580

tctaatattc acaggcagat ctgcttgggg aagctagtta tgtgaaaggc aaatagagtc 5640

atacagtagc tcaaaaggca accataattc tctttggtgc aggtcttggg agcgtgatct 5700

agattacact gcaccattcc caagttaatc ccctgaaaac ttactctcaa ctggagcaaa 5760

tgaactttgg tcccaaatat ccatcttttc agtagcgtta attatgctct gtttccaact 5820

gcatttcctt tccaattgaa ttaaagtgtg gcctcgtttt tagtcattta aaattgtttt 5880

ctaagtaatt gctgcctcta ttatggcact tcaattttgc actgtctttt gagattcaag 5940

aaaaatttct attctttttt ttgcatccaa ttgtgcctga acttttaaaa tatgtaaatg 6000

ctgccatgtt ccaaacccat cgtcagtgtg tgtgtttaga gctgtgcacc ctagaaacaa 6060

catattgtcc catgagcagg tgcctgagac acagacccct ttgcattcac agagaggtca 6120

ttggttatag agacttgaat taataagtga cattatgcca gtttctgttc tctcacaggt 6180

gataaacaat gctttttgtg cactacatac tcttcagtgt agagctcttg ttttatggga 6240

aaaggctcaa atgccaaatt gtgtttgatg gattaatatg cccttttgcc gatgcatact 6300

attactgatg tgactcggtt ttgtcgcagc tttgctttgt ttaatgaaac acacttgtaa 6360

acctcttttg cactttgaaa aagaatccag cgggatgctc gagcacctgt aaacaatttt 6420

ctcaacctat ttgatgttca aataaagaat taaact 6456

<210> 46
<211> 118036
<212> DNA
<213> homo sapiens

<400> 46
gagttgtgcc tggagtgatg tttaagccaa tgtcagggca aggcaacagt ccctggccgt 60

cctccagcac ctttgtaatg catatgagct cgggagacca gtacttaaag ttggaggccc 120

gggagcccag gagctggcgg agggcgttcg tcctgggact gcacttgctc ccgtcgggtc 180

gcccggcttc accggacccg caggctcccg gggcagggcc ggggccagag ctcgcgtgtc 240

ggcgggacat gcgctgcgtc gcctctaacc tcgggctgtg ctctttttcc aggtggcccg 300

ccggtttctg agccttctgc cctgcgggga cacggtctgc accctgcccg cggccacgga 360

ccatgaccat gaccctccac accaaagcat ctgggatggc cctactgcat cagatccaag 420

ggaacgagct ggagcccctg aaccgtccgc agctcaagat ccccctggag cggcccctgg 480
gcgaggtgta cctggacagc agcaagcccg ccgtgtacaa ctaccccgag ggcgccgcct 540
acgagttcaa cgccgcggcc gccgccaacg cgcaggtcta cggtcagacc ggcctcccct 600
acggccccgg gtctgaggct gcggcgttcg gctccaacgg cctggggggt ttccccccac 660
tcaacagcgt gtctccgagc ccgctgatgc tactgcaccc gccgccgcag ctgtcgcctt 720
tcctgcagcc ccacggccag caggtgccct actacctgga gaacgagccc agcggctaca 780
cggtgcgcga ggccggcccg ccggcattct acaggtaccc gcgcccgcgc cgcccgtcgg 840
ggtggccgcc gcgcccggca ggagggaggg agggagggag ggagaaggga gagcctaggg 900
agctgcggga gccgcgggac gcgcgacccg agggtgcgcg cagggagccc ggggcgcgcg 960
gcccagcccg gggttctgc gtgcagcccg cgctgcgttc agagtcaagt tctctcgccg 1020
ggcagctgaa aaaaacgtac tctccaccca cttaccgtcc gtgcgagagg cagacccgaa 1080
agcccgggct tcctaacaaa acacacgttg gaaaaccaga caaagcagca gttatttgtg 1140
ggggaaaaca cctccaggca aataaacacg gggcgctttg agtcacttgg gaaggtctcg 1200
ctcttggcat ttaaagttgg gggtgtttgg agttagcaga gctcagcaga gttttattta 1260
tccttttaat gtttttgttt aatgtgctcc ccaaatttcc tttcatctag actatttgat 1320
tggaaatatg tcagctatga tgatgacttt ctgggaagcg attcctgtca cccgctttcc 1380
cctcctcccc accccacgtc ctggggcttt agagagcgat tgggagttga atgggtctga 1440
tttcggagtt agctggctga gtccgcgctg gagcggattg ctggcatgtg acttctgaca 1500
gccggaaatt tgtaggtgtc ccgcgagttt aaaacaagcc atatggaagc acaagtgctt 1560
aaaaataatc tcctgccagc ccagtgacaa gcctgtccca cccggggaga atgccccgga 1620
gtggcgtgcg ggtcagccag ggtctgcgcc tcgcagccac tgtggaagga gcgcggccgg 1680
tccaggacac aggagaccac tttgtgactt caatggcgaa ggttgtgtgt cctcatttta 1740
atttttttcc ctacaagaat tgttctttct ccctctcctc tccctcccat tttctcttgc 1800
ccagtttctc cttttgtttt ttgtttttg ttttcctgat gggcctgcag agggattagg 1860
tgggcgcttc tggtgaacac cttcctaggt ggccacagga caggtgtacc ccggactggg 1920
tttggaagct tcagggcgcc acatggctgg gtcctgaatt aggcatttcc caactgtaca 1980
ctggtatccg gactggtgtc cctatatctt tctgccttgt aagccgtgga ccagtttttg 2040
ttcagtattc tgtttccagg gatatttata gcagaaggaa ggggactaaa gtgcagtttg 2100
gccccagagg atactgaagg gcagattctg gggtattca gtgtgcatct tcagccgcct 2160
tggagaaatt tagagcatcc cacagccacg cagatccaag ctgtctttac tcaaaagaca 2220
aacaatgaac aaaactttta aaggttggca tatttcaaat taattttact tgttttaatt 2280

tagggttaaa acagagaaaa aggatttctt ctgcccacct tttttttttt aaatggaaga 2340
acaaagtaca gcgattaagt ctaattccac acaacattta aaactgcttg atgtgaagga 2400
aggcactggt atgatgtgaa ttccataacc ttatgatgga ctccagaaac cattttcttc 2460
cctatttaat tttcagttct tttattgcaa attaatgctg ctgaatttca atgggcacta 2520
atgagactgc tccttggtag attatttact gccttgctaa taattacaaa gtgaacctgg 2580
tcaaatacag aggggatcgc atcttattca aaattgttca tcatcccagt gataagtggt 2640
atcagtgtaa tatgccctat cttacacttt ctgcattaca tgatattcaa acactcttag 2700
aataataaaa aaagagacaa ggaacttaaa aattaaaaaa aaaacttgca caaatgggac 2760
tctgtgtgga aattcagttt tagaatgatt tttcctgtgt tttatttccc ggattatctt 2820
tcctcttttg ttagaattct gcctgttatt atccagcaag gaaaagaagc atctatgcaa 2880
gttcttcata tggacagata ttatttagta tttttcccct ctcagttttt ctgcttaaat 2940
gactctgggt ataaaggaaa ggattgattg ggctctttta ggaaacttta agtttcttaa 3000
gtagttctca aaagttttgg ggctgaaagc agtgttttca aactgcttgt catgacccag 3060
agggtcatga actcagttta gtgagtctag aatatttttt aaaaggacta aaatggaaag 3120
gaatataata gaaaatatca gagtgcatgg tatttcgtaa ggataagttt tgtttcctga 3180
aaatctgttt taattatatg tgcttctgtg tgctgattgt gatgtaaaat gtatttctta 3240
ctgtggattg aattcaaaga aaaaattaga aagctaatgg cctaaaatat tatatgttca 3300
gtagaaaaca aaaaattcag gcaagtggct ggttgttttt acctatacaa atcaaaaggc 3360
tattttgatt gtcttcattt tccccttata aattaggttg gtgtctttag tcatttaggc 3420
taagttttac tatctgattc ttaacttttc tattgtagaa tggtgctgtc atgtggactg 3480
tcctcccgag tgtcccactg gatgttcaga gaatttatgt gaaggtcacg tcatttagca 3540
ttgagatgct gtggttacct tcttccattt cttccataat atgcagccac atctatgtgt 3600
gaagaaatgt aatagataaa atttctctgg acgcataata atgtgagaaa gattgtcaca 3660
tgtcccagca aattgttatt aatataaatt tgttacttgg caagctgaga ttttgcaaga 3720
tgttactcaa aatttcacaa tgaaggaaac agggagtcat cttatcctgg gttccttttt 3780
tagatttcaa acaacttagg aactttgaat aaaactaaag atgaagctta actatatcaa 3840
ctatcctttt taaagttcta attaggaatt taatgctgca tgcttatttc agttttatta 3900
ctcagtattc ttaaaagtta gacgtctctc acttctccaa aaaacttgct ccccccctacc 3960
cccaccccac gacaggtccc ggagtgtgat gttccccacc ctgtgtccaa ctgttctcat 4020
tgttcaattc ccacctatga gtgagaacat gcggtgtttg gtttttttgtc cttgggatag 4080
tttgctgaga atgatggttt ccagcttcat ccatgtccct acaaagaaca tgaactcatc 4140
ctttttatg gctgcatagt attccatggt gtatatgtgc cacattttct taatccagtc 4200

tatcattgat ggatgtttgg gttggttcca agtctttgtt attgtgtata gtgccacaat 4260
aaacatacat gtgcatgtgt ctttatagca gcatgattta taatcctttg ggtatatacc 4320
cagtaatggg atggctgggt caaatggtat ttctagttct agatccctga ggaatcgcca 4380
cactgacttc cacaatggtt gaactagttt acagtcccac caacagtgta aaagtgttcc 4440
tgtttctcca catcctctcc agcacctgtt gtttcctgac tttttaatga ttgccattct 4500
aactggtgtg agatgatatc tcattgtggt tttgatttgc atttctctga tggccagtga 4560
tgatgagcat tttttcatgt gtctgttggc tgcataaatg tcttcttttc agaagtgtct 4620
gttcatatcc ttcgcccact tgttgatggg gttgttgttt tttttcttgt aaatttgttt 4680
gagttctttg tagattctgg atattagccc tttatcagat gagtagattg caaaaatttt 4740
ctcccatttt gtaggttgcc tgttcactct gacggtagtt tcttttgctg tgcagaagct 4800
ctttcgttta attagatccc atttgtcaat tttggctttt gttgccattg cttttggtgc 4860
tttggacatg aagtccttgc ccatacctat gtcctgaatg gtattgcctg ggttttcttc 4920
tagggttttt atggttttag gtctaacatt taagaagaag gatacttaaa gtataaggga 4980
aaatgttaca atgtatgaag ggaacatgaa gaaatagaat ctggtaaaaa agagttcttg 5040
cttttgggag gccaaggcct cctggctaac atgatgaaac ctcatctcta ctaaaaatac 5100
aaaaaattag ccgggcgtgg tggcacacgc ctgcagtccc agctgcttgg gaggctgagg 5160
caggagaacc acttgaaccc aggaggtgta ggttgcagtg agccaagctt gcaccactgc 5220
actccaggct gggcaacaga gcgagactcc atctcaaaaa aaaaaagaaa aaaaagagtt 5280
cttgctttca aaactatgga ttaggtaact tttgtgaatg agtaagatca tgagtattat 5340
aaaaatagca cctttctttt ttgtcttggg gaaattatct tattttttaa ttggatttca 5400
gaaaagagta tttcagagaa ataaatctct gaaatgcttt ttgaagtgtg aaagatttag 5460
aagacaaaag caaacctcct gtctagataa acattaaaga gatctgccct cccctcctct 5520
acctattcag gttgcaacac tttgggggtg gctgccttgg tagagcttga tcgtgactct 5580
ggtggcttgg gagatggcat gctgcacaag ggattcatgg ttacagcggg cttgtgggac 5640
tggggctctc caatacgtgg ttgggtttgt aaagaaatca gagctatggt gtgaacaaaa 5700
ggatatgcat gggagacagt gagacaagga aatgctccag aaattattgg aatataggtc 5760
agataactaa ctgtacttgt gccattttct gggggaaaat tctctgaagg ctttttggga 5820
aaagaatgga agtgagaatt ctcaggtcct caaaatattt ccttttactc agtcctaacc 5880
tgaggccgtt aaagaattcc cagagtcacg atggaaggca tgtttgggag taagagccag 5940
agtgagggtt agaaatgtgt tgttggccag gtatggtgga tcatgcctgt aatcccagca 6000
ctttgggagg ccaaggcagg tggaccacct gaggtcagga gtttgagacc agcctggcca 6060

aaatggagaa acctcgtctc taccaaaaat acaaaaatta gccaagtgtg gtgacacgtg 6120
cctgtaatcg agctcttcgg gaggctgaga caggagaatc acttggaccc aggaggtgga 6180
ggttgcagtg agccaagatc atgccactgc actccagcct gggtggcaga gcaagactcc 6240
atctcaaaaa aaaaaaaaaa aaaaaagaaa gaaatgtgtt ttccagggtt ctgggtactt 6300
aggaatttgg ttgcttttgc aggtggaagt ggaggtgact aggtaacagc tgagtgattt 6360
tgccccagtt ggacatgagc caggttgagc agaaagccct gggatgcggg gagggggggtg 6420
gcggggaagg aattgaaagt tggttgtgtg gtttggcttt ggcttcatgg catgctcaca 6480
ccttgcttcg catagcatgc ttagactaca gcaggagcat caggaagtgg atttctgagc 6540
tcaatacaaa aagttataaa taccacctat aagggcaata aagatatata gttgattttc 6600
ttctttgcaa ggccaaatct tataggaaca taagagcgaa tgagttacag cctgggaatt 6660
tgagccttat attcagagat tttaggttgc ttctgattcc gctgtctaga caaaaccatg 6720
agaggatagt gtctagaaat gagaggaagc tcttccaatg cagaggctag aatgtgtcag 6780
cctgtgctgc gaggcctggg atagatgttt ctgaaaagta aaagggcagc tttcctactg 6840
gatacttgat cctcaggctc tagaaaactc tgctttatta actttgttga cttcctaggc 6900
accacatggg atccttgttc ttcctccttg taagcagtaa ttgaaatcag tttggcagcc 6960
tggtttacag tgaccatggt ggcttgtctc ccgtgctctt acctcactct gttgatgttg 7020
taaaacctcc agctaacttc atggggtggc tgacccacgt tgctcattta ttcattcaac 7080
acatattcat tgaccatcta ctctatgcca ggtattgtta tcagcactgg gaatagatca 7140
gtgaactatt gatctatttg tctaatggga caaattgaca aattgggaaa gattccatta 7200
cacaggtgac atttaagcaa agtcttgaat aagggaggga atagtaccat gagatatcct 7260
ggtgaaaagc aatttaggct gagggcacag cagggaagag gccctgatgt gggaacatcc 7320
ctggtgtctt gaggtacaga ggccagcatg gctggcacgg agtaagaagt tggaggtgcc 7380
gggcatggtg actcacacct gtaatcccag cactttgggt ggctgaggca gatgggtcac 7440
ctgagcccag gagcttgaga ccagcctggg caacatggtg agaccccatc tctacaaaaa 7500
aatacaaaga aaattagcca gatgtggtag catgcatctg tagtcccaat tgcttgggag 7560
gctgagatgg gaggatcaaa ttacttggga ggctgagatg ggaggatcac ttgagtccag 7620
gaggtggagg ttgcagtgag ctgagatcat gtcagggtga cagagcgaga ccctgtctca 7680
aaaaaaaaaa aaaagaaaaa gaaaaaagaa aaaaaaagaa gttggaggtg agtaaggaga 7740
ggaacgtggg ggacagagtc ctcaggactc tggcttttac tctgagtgag tcgaaaatcc 7800
aattaaaggt ttgaaagaga ggaatgacct gatctgacat tttattgtga acgttttcaa 7860
atctttacag aagtggaaga gcataacgat ccttcatgta cacatcgccc agcttcaact 7920
atgatgtttc atttgtaaat atttccgtct acacttccaa aggatgatga ctatttttaa 7980

aagtccaagc tacaaagaga ccaatgctag ttggtgcaag gaattcaaga atttggactt 8040
aagtctatat aatgatgatt tttttttttt aacttgagtt tcccggttta tcactcccag 8100
aatataggca gaagtttgag atttttatgt gtattttctg gaaaagatag tttcagtgtt 8160
ttttacattc tcaaacaggt ttatgatcca aagaaaaggc agtggtcaca gatacatgaa 8220
acgacaaggt attcaaagga gaacgttgta ctttatgaca gttctttggg cagtggcttg 8280
caggatgagt ttgaggaatg attggaggca ggagagtaat tctagtaatt caaatgtgga 8340
gtattgttga tctctcagac acaaatggaa aaacaaggaa ttcaaagaaa gataggcaga 8400
gtgttttgaa gaaataattg atgaaatttg gtaatgagtt agatgtagga gatatattta 8460
gcaaatattt attaaggact gtattaatct gttatcatgc tgctaataaa gacataccaa 8520
gactgggtaa attataaaga aaaagagatt taatggactc acagtgccac gtggttgggg 8580
aggcctcaca atcatggcat aaagcaaagg aggaacaaag tcacgtctta catggcaata 8640
gagtgtgtgc aagggaactg ccatttataa aaccatcaga tttcatgaga aatattcact 8700
atcatgagaa cagcacagac aaaagcctgc caccatgatt taattacctc ccactgagtt 8760
cccccaggac acatggaatt atggaagcta caattcaaga taagatttag gtggggatac 8820
agccaaacca tatcaaggac ctactgtata tggttaaaat tgggagcaaa tgagacatga 8880
ttcttgcctt cttggagttt actgtttact aggggaacat acacttgtca ataatcaccc 8940
aaatatagga ttggaaattg tggtaagtgc catgaaaaac aagtataggg aattttgagt 9000
gtacatagct ttggggactt gatttgatga gggagcctta tgaagttatt gcactagaac 9060
tgaattaaac cacatttcta ggaagtggac atctatttgt tggttcttta aatttagctt 9120
tacagaaata tttcctttaa aaaccaaggc ttcttaaatt tttaaaactg cttggctaat 9180
caggggaata atgcttttgg atagctggta tcgttattta tggttggaaa aacaacagta 9240
tttgattaca ttgagcttta aacttttcct ttgattaatg aaaattttat tggcccatag 9300
tttttattat gctctgtttt tacttggtcc aagagattct attctctgga cccaatatga 9360
ataccttcag acatccctct tttttttttt ttttttcacc caggctggag tgcactggca 9420
cgatctaggc tcactgcaac ctctgcctcc tgtgttcaag caattctctg cctcagcctc 9480
ccgagtagct gggattacag gcacctgcca ccacacctgg cttatttttg tatttttacc 9540
agagatgggg tttcaccatc tcggccaggc tggtcttgaa ctcctgacct catgattcac 9600
ccaccttggt ctcctaaagt gctgggatta caggcatgag ccaccacacc cagcccagac 9660
atccctctta attatgttga atatgtaata tcggtgattt catttgaaaa tatttagtag 9720
tcgaactaga tcaaggcagt taagcttcct atttccatag atgcagtggt attgtgtctt 9780
ttttatatga tctctcatgc ttctggacat cctttttttct gctattcttc attccttagc 9840

tacacttggt gcttcgtggt tgtaatgcat tgtcatagat gcgttcattt ctcattcgat 9900
cttcagctct atttctttcc agagaatctc tacaggcatc tgttaggttg aaggacatct 9960
aatgtcttaa tgtgtagctt ggtaaaccag tcaactttct atctgagtct taagagaaag 10020
tgtccaagat gagaaacggt acaggtttgg tgacaactca gtgagaaaaa gaagaatttt 10080
acaaggaagg aggtatctta gtaattttgc taaagaagta ggtaaacctt cacttataat 10140
aaagggatag ggctcggtta gggtttgtga agtctcccct taggaaagca aaccctgaaa 10200
tattttgaat cttttaaaga aggaaaataa gagtctttta aataaatttt taaaatttat 10260
tttatatatt ttttatagac aggctctcac tctgtctccc aggctggaat gcagtggtgc 10320
aatcatagct cactgcagcc ttgaatgcct gggctcaagc ggtccttctg tcccagcctc 10380
ctgagtagct gggactgcag gcatgagcca atgtgcccag caagagacat tcattttggt 10440
actgtgatgg tacagaaaaa caaagggcct ttgaggccga aggagcagaa gaaggatgga 10500
cttagacatg gtataggcac tttctactaa agagctgtga agctaaaaat gccaggtcta 10560
tgacaggtgc agtgggccaa ggccaggtag agagcagcag gaagagagga ggtggggacc 10620
tgtacctagg cccatctgct gggactgatc tagccatagg tactcagaga agcccagatt 10680
ggtgcctgac ccacccttat ggcccagaca tggacacctc ccagtctgtt ccttcctgct 10740
gcccatggat gggctgtgtt agtctgtatt ctgaggacac agctctctgt ctagaggaag 10800
ttatgttatc ttgatctgat ggatactcaa cgtgaacatt atttcaacgt gccacagggt 10860
cttggagccc agaggaagac cgctcttgcc ttttagttta tattctttgt ttttttttaa 10920
ataacatttt gacagtcttt atggagtaag tctgggccaa aatgataatt gacaatgtta 10980
tttacatgga tttctaagtt ggctaaaaaa gttcctttat ggttagtgaa tatagcccat 11040
gtagtttccc cgtcttcttt agatgccttc tatttctatg cccaaagtct gcagttgatt 11100
ttcagtaagc tgggggtcat cttagagata aaatgtagat gaatggcatt ttgctgacag 11160
catacatctt tgctatttct gaggaaaatg ggctctcgct attaaatctt ttgtcaatat 11220
ttataaaaat agtatttaca tattctatct atattgtgga aactatacat ttattgattc 11280
agtcatttga tatcaatgtt gttgagtccc tattccaagt gaggcactat gctctaagca 11340
catggcattt taaagatgaa taagacacca agaactttgc agatagtaat ggaaatgaga 11400
attaatcaat tgaagattaa tatagtaagt agcagaagag aaataaaaaa atcttctaga 11460
gagttcagaa cagggatgtt gattcaagtt tatggggatt aggagtggct ggtaagggag 11520
gcattcaggc aaaagacata aaaatgcagt attcccctcg cactcattag gatggctact 11580
atattagaaa aagaagagag taagtgttgg agaggatata gagcaaatag aaaccttgtg 11640
ccttgttcat gagaatgtaa aatggtgcag ccactgtgga aaacactggt gattcctcaa 11700
aaaatcaaaa tagaattatc atatgatcca gtaattctac ttctgggtat atatctaaaa 11760

gaattaaaaa tctgggtctt gaagaaatat ttgtatactc atagttatag caacattatt 11820

cataatagcc aaaaagtaga agcaatccag atgtctatag atggatgaat gggtaaacaa 11880

agtctgtgta gtatatacag acaatggcat attagtcaca tcatggacct tcaggacatt 11940

atcctaagtg aaatatgcta gacacaaaaa gcaaaagtag ggtttcactt aatgaggtat 12000

ctagaattgc cacattcaca gagagtgatt ctcatggact ttctgtgata gctctttcct 12060

gccctgatat gagatgaaag ctgggggatg gtatatagta tttatttttc cttccgttgc 12120

cagtgggact tttttttttt ttttaaaagc tgttcatatc ttaatcgagt agcatgtgag 12180

gtcaacatgg tctattttaa aagcattttc ttcgacacat tgcttttaac atcttttaga 12240

actctgctgt gagacacatg gacttttttg ttggtatttt tatacaatta atgatattct 12300

caatagtaat ctttgtgtgt gtatatatat agaaataaat tctaaatgta agttaatata 12360

tttattattt ttctaaacat atataaatat atatatgcac acaggctatt taattttatt 12420

agatgatgct attttaattc agaaaaaaat gacatttata ttttgattta ggttagtata 12480

agcccttaga ggtgttttga caactctctt aatttgtggt tttactgttt atttgatttt 12540

atataatcta aaataccatt gtttttacca agcatttaat ttggcagtga aagagcgtct 12600

gacagaggta tggttagtag ataggtctaa ctgcacaact ggatggattg agctgagact 12660

gtttcctcat cagtaaaaat gatttgaagc agtggttggc aaagtttttc tgtaaagggc 12720

cagataataa tattttaggc tttacaaggg ccatgcagtc tctgttgcag ctaccgaact 12780

ggattatagc ctgtaaggtg acctgtaaac acatggaagt gattatgtgc taataaaact 12840

ttatttatca gaataggtaa cagatcagcc ctggcccgtg gccgatccct gatttaatgt 12900

ttatttatct gatctaaata cctttattta tggaagggaa taggggattt ttaaatctaa 12960

agttttgatt attcacattt tactgagaac ttactctata cctgattaga tgttccgaga 13020

gaaataaaaa aaaagtgtaa gacataatcc ataataccac aaaatttaaa atgtatttag 13080

gaaatttatt tgaggaagta aatgtacttg ttctcatgat acaatcagaa agtaagtcag 13140

tattgataaa gtgttacctg tatgagaaag ataaggaaaa caatagagag atgtaagaaa 13200

tgaaaatacc agttataaat taaaattatt aagattgaaa gtggaaatga tcttcctccg 13260

agaaacaatg gcaatattct cacaaatttt ttacatcatt tttgttcagc atttaagata 13320

aaattatata aattcccata acatttagta ttgtctctaa gcattaagaa cagaaaaaac 13380

agaaggaaaa tatatttcta aaaatcaacg aatacagtgt gagatgtttc attggtatgg 13440

cattatctca agttcaaaca ttttgaaaaa tgtctgctta ctctttgata gttaaaaaca 13500

agtatctcag ctggcgtggt ggctcaggcc tgtaacccca gcagtttggg aggctgaggc 13560

gagtggatca caaggtcagg agatcgagac catcctggcc aacatggtga aaccccatct 13620

ctactaaaaa tatgaaaatt agctgagcgt ggtggtgcac acctgtagtc ccagctactt 13680
gggaggctga ggcaggataa ttgcttgaac ctgggaggca gaggttgcag tgagctgaga 13740
tcatgccact gccgtccagc ctggtgacag agtgagactc catctcaaaa aacaaacaaa 13800
acaacaccac caccactaac aaaaacctct tatcgccgtc ttgtatacgc agaccagcta 13860
gtagaatttt actgaaacag tagcctataa aaatgcaatt ccacttggtt tcagaaactt 13920
cttgtgtatc atagtgtgaa gtcacttatc ttaggctttt aaaatgggat aaatattgag 13980
tccaaagttc tggaagaagc ctagaaagaa ggcagagtta ttaactttta gatataggga 14040
ggaaccttaa aattattcag ttcttcattc attcacttat tcattgacta gctttactaa 14100
caaagcccta tgcaagaccc tggaaatgca atgatagaaa aacctggtcc ctaccctcac 14160
agaacttgtg aggtaaaggg ggatacagac tgataaacca gcaattagat gatggtgtca 14220
agatagaggt gaaggcagtg tcttatagga tccaaactcc actcagtcct ggtggtggtt 14280
gagtctggct atcagaggtt tcctgattaa atctggaggg tgagtcaagg gagcatggtg 14340
aagaaggagg gaatgcatgt ttagccatgt gaatgagtcc atgagtgaag accaggagga 14400
aaggcagagc gcggggaatt ctatgcgtaa tatttaacaa aattaatgta ctgttaaaca 14460
aagacatttc tgggccatgg atttaatcct agactgtgta aaaaccaagt aattgatttc 14520
ctttatactt taaaagcatt tccatgtatt tgatttgttt gtgtgtataa aagggaaata 14580
ccacaacaag tttaagggtt tctagttctg ctttctcatc atagtcttga taacttggaa 14640
ctaaaaagtt tttgctgaaa ttgtctgtga ctctttataa atcacactgc ccctcaaaca 14700
catttaagga tggtgaaggg tctgacacgt aggtgggaag ttctgaagat gccgcagctc 14760
tccctgtttt ccttgttact taagaagaga gctagaaatg agtgtacatc agattattct 14820
catgttctaa gtgttttggt tgaagaggta aagtgtttgg cttgaaagca tacaaatttt 14880
catccactac ttagtgtaca aacttgatta cttaagagat tgagtaatgg cctccagtga 14940
aacgcattct ttttaaaaag caaagtgaag gatgctattt aagtcagaag gggcaaaatt 15000
ggatatttta tgagtttatt aatcattgca ggcatagaag tagtgttcct taagatgtgt 15060
tttagacaga gtccctggga tgagttatat aagcagatct ggttgtagct tcagcagcca 15120
gatactacct ttgagtatta cttcaaggaa aaaggactcc actgagctca ctgcttctct 15180
ttcattatta tttcagaagg ttgtgtggcg tagagggggc tcaggcctac ctatacacca 15240
ctagctatgt tgccatttta tattatttct ataaggtgcc aacagaagct gctcatcaga 15300
tcagacagac atagcccagg caagtattga tttacagatg atctttggcc aggaagacat 15360
ggtatcaggg tagagtctgg ttatgggtca atgcagtggg gaccttaggt cctacaggta 15420
taactgagag cctgatccac caggccttag aaagcttcag ggtgagacag tccagcaccc 15480
tggatagctc ctttaacagc tgtggccggt aagcaggcac ttatttgcta aagaactcaa 15540

gcccatttag ctggcttcat ctgctttgta gagctctgtt aaaaagagtt cctatttctc 15600
cacatcctct ccagcacctg ttgtttcctg actttttaat gattgccatt ctaactggtg 15660
tgagatgata tctcatagtg gttttgattt gcatttctct gatggccagt gatgatgagc 15720
atttcttcat gtgtttttg gctgcataaa tgtcttcttt tgagaagtgt ctgttcatgt 15780
ccttcgccca ctttttgatg gggttgtttg ttttttctt gtaaatttgt ttgagttcat 15840
tgtagattct ggatattagc cctttgtcag atgagtaggt tgcgaaaatt ttctcccatg 15900
ttgtaggttg cctgttcact ctgatggtag tttcttttgc tgtgcagaag ctctttagtt 15960
taattagatc ccatttgtca attttggctt ttgttgccat tgcttttggt gttttggaca 16020
tgaagtcctt gcccacgcct atgtcctgaa tggtaattcc tagagaaggg aaagaatcaa 16080
agcgtgaaga ccaatttggc tgatattcaa ctagcttaga tgtactaaaa atctgtactt 16140
tttggtattt gtgaaatgga aagaagggga atagaataaa ggatattata atgaaaggat 16200
atacattgct tgaaggtaat taaatatggg ttatccagga gataaaagag ttaaagaggt 16260
tcagacatag actgaatgaa ctgagaaatg aatgacttgg tcaccaagag gaaggccagt 16320
catcaggggg taggataagt tcaattctag acatgctgca tttgagatga tagctggatg 16380
tccagatgga attatccagc agccacagaa acagaatcag ctctctgcgg atattccagg 16440
ggtggggatt tgaattaatt tcctcaatta attttaaaga aacttgatga aaagaatggt 16500
ctgaatactt cttgaaggtt gcacattatt aataatggag aaataactct aaaaccttcc 16560
tcttgatttt cataataata taagcattcc ctgaatctta ccaaaccttg taagaaacac 16620
tcttattata aaaagtgtat gtgcaaagcc cttctaaaca ggaaaatgat aaattagtcc 16680
tacagggcca aatgcagctc tctgggagct tacaattcag aaagaacatc ctgctaccag 16740
cacattaagc tgtacaaata gtaaactgca gaaacaaata taagcatttt tatgatgtcc 16800
aaacaagaac caagcaggtg tttttttttt tttttttgca gattatttat actgtggcag 16860
ttcatagcct ccttttcgga cccagagctt gcataatcct tcccttattt ctacttacgt 16920
gttttactct ccatcatgtg ttaacataca tactgtgcaa cagaaatgac tatggaggct 16980
gagggcagca gagttttagt gtgtacacat atgagctgtc atgtaatttt caagtgaaag 17040
cctttgcaat gaaacttttt aaaagaaagt catggccggg tgcggtggct catgcctata 17100
atccagcact ttgggaggct gaggcaggca gatcatgagg tcaagagatt gagaccatcc 17160
tggccaacat gatgaaaccc cgtctctact aaaaatacaa aaattagcta ggcatggtgg 17220
tgtgcacctg tagtcccagc tactcaggag gtggaggcag gagaatggct tgaactcgag 17280
aggtggaggt tgtagtgagc cgagattgca ccactgcact ccagcctggc gacagagtga 17340
gactcgtctc aaaaaaaaaa aaaaaaaaaa aaaaaaaaag agaaagaaag aaagaaggaa 17400

aaagaaagaa agagaaagaa aggcgggcat taacttcagg tattggtaaa tttgctaggt 17460
gtttggctac tgtttctcat cagagaaata gaaagacaca ccatgaaagt caaggcctga 17520
aaacctcatt ccatgtaaga atgacatccc cagtgttaag tgcttgttag tggttaatgc 17580
gatcctgtga aacttagatg tgtttgtgca cacatgcacg catatatttg aagaactcag 17640
aagagttaaa tcacagcctt tcaacctgtg aaatgacagt agttcttctt ttttcctctc 17700
cctttggcta agtcatcttt atcttggaga taattaagac aaaaatgcct ctgacaaata 17760
aaatcagtat agaacccccta tttcttggca gcttttgtgg acacagctga agctttcaga 17820
ggtcttgaaa aaccatggca acaaatgcct ttgaagggta agcaaaggtt ccaaatgttt 17880
ttaatcgctg gtgtttttttc tgctaccact tcaagcattt ttcttcattt tttgttcatc 17940
tgatcaaaat taaatttcca atttccctac taagtggttc tgtcctggtc atgccattga 18000
ctatttccat taaagtagta gagtttgtgc ccacatatgg ttgttaagct catcaacaat 18060
tccattagaa agctttgttt atcagtggca ataatttccc ataaaaatta tagataggtt 18120
ttaatgggca cattttcaaa aggcatcaac tcgtcctcaa aattatgtgc tgacactgtt 18180
cttacaacca tggttcgtgg cctaattcca ccaaatttct ctctttttca tagagaactg 18240
ttgtcagtag ctttatatcc ttttaaagag aatggtctgt atctctgata ctcattcaga 18300
gaaatgagta ttttagacgt aggttgctaa ttttaagcta tatactacac tatgtcagca 18360
ctatataggt tatcttgtaa cctgcttgcc tggctatttg gctgaaaaat aacaacatgt 18420
aaggaaaatc tatttcataa gtctaacatt tactttgtaa aacttgttct ggctactctg 18480
ttaattttcc acttacgtgt gggttagaga gcagatttga ttttttttta agcgaaagat 18540
atggcttacc tgagaaaaga acatagtggg gaaagcactc ctattatttt cctcatattt 18600
ccattttcct ttagcggaaa taaaaagaca tttcagtttt tcagttgcta agaaatgaag 18660
gaaccaaaga caaaacaact taattattaa attacaattt atttctgtaa taagcactcg 18720
ttctctctgt tttcctgggg aaagagtatg tggactttca attttatcca aataagcatc 18780
atctttctct gattagtgtg gcagtttcaa aatcatgtat taggaagtac agagtgaatg 18840
agtagagaat ttctaaatta gcacccaagg ttgggtggct agattatgtt tataaatatg 18900
aacttttgta ttaagtgcaa tgatttaaaa gaatgcctgc atcactttag ggcatttcat 18960
taagtgctgt gcacaatatt tttccttata catcataaaa gataaattat agttcataaa 19020
atagtataaa ttcctaatta ttttgtgctt ttgacacctc agagttacta ataagggatt 19080
tcgttttaaa atgatattta tttatttatt tagagacgca gtctttctct gttgcccagg 19140
ttggagtgca gtggtgcgat cttggctcat tgcaacctct gcctcccagg ttcaagcgat 19200
tctcctgcct cagcctccag agtagctggg accacaggca tgggccacca cacccaacta 19260
atttttgtat tttggtgga gacggggttt cactgtgttg ggcacactag tctctaactc 19320

ctgacctcaa gtggtcctcc tgccttggcc tcccaaagtg ctgggattac aggcgtgagc 19380
cactgtgcct ggctcgtttt aaaataattt aaaagtatat tttgccacca ctattaacca 19440
gttaagccat gatggtatat tataatcacc atggagatgg tttttctctt tattttattt 19500
tgtttgtttt ctgattgcta gcatgctgat tactcttcct attctacaag tgcctgcagg 19560
ccagccccat ttttgctctc ttcacttcat ttttcatttc tccctgtttc actcttcata 19620
gcacatttgt actctgtcca cacctagaga cctccctgtt gaagttcttc acattctctt 19680
ccctagagag ggttaacttg ttgagctcag agacttaaac ttaaaataaa atgtaacaga 19740
tatgtattga atgactattt tattttagct ctaggagaaa tgcaaagata tatcatccat 19800
agaccctgaa atccagctaa gggttctgct tatacacaag gtgaaaggtt gtgataatgc 19860
acattaaaca atagaagagt taaattcagc actataagtg atgcctcaaa gcagcataat 19920
gggaaaaagg ggtatttcag aaaaaaatgg tatgagttca gaggcaagag aaaacagaat 19980
gctctagatt aaactcaaag actttatgga ggaggtggtg tttcaataga tacctttcca 20040
cagaaccatg aagagagttc tacttaattg taagtggcct gtgatttgtc gttagtcatt 20100
gacaggtgct ctgtaaagaa tcattggcat cattacctct tgttggctct atcccattcc 20160
ctatagatat ctttctgctc tgagactaag cagagaaaat aatttaacat ttaattattg 20220
tgcttctaaa tccactttta cctcttcttt ctttactttt ctctttatta tttttaatta 20280
tttcaaaaat aaaattgtat atatttaagg tgtacaagat gttttgatat acatatacat 20340
aatgaaatga ttactgcagt caagctaatt aacatcaatc tcctcaccta gttatcattt 20400
ttggggggca tggagtgaga gcacctgaaa tctctcttag caaatttcca gtgtataata 20460
cagtattgtt aaacatagtc atcatgttgt acattagatt tctagacttg ttcatgctac 20520
ataactgcaa ctttgtaccc tttgaccaat acctctctat tttctccact attccatgcc 20580
tgaaattgtg ggatataggt atatagtgtg tgtgatatat atagagggag tgatatatgt 20640
atatataatt gtgtgatata tatgtgtgta tatacaaaca cacagacata cacacatgta 20700
catatgtata tacatgcaca catacacaat gggatattat tcagctttca aaaaggacat 20760
cctgccattt gcaacaacat ggatgaacct ggatgacatt atgttaagtg aaacaagctg 20820
gacacaaaaa gaaacatgca ccttacttct tagacacaaa tttctttctc atagccagca 20880
cctgaaaata gggccaagta ttttgcatga tttacaattg actgtataaa tgtagcatag 20940
ccagttatat gatgtgttac catttctgct ataaaatcaa aagaagagat cagaacattt 21000
aaaaacttcc ctgtggattt ctacctctta cctaagcttt gttcttgttt ttttaaacct 21060
catgaacacg aagctttaga tagcggaggt catgtatcct tgggctcaaa tatacagttt 21120
cttagcaatc tttgattgct acattgccct tttttgtgtt tttaactggc cttccctgac 21180

ttaaaatata ctttaaaaaa taacaaaggt ggtatttagt ttgcacagta tggaattaaa 21240
ggtaaacaac tttaaattaa atttggttgg atgtataaat tattaaaata tcctttctct 21300
catggctggg taagtctgca ttttccattc agcctgctat gtctctttcc atgagaacac 21360
tcccgaagaa tggggccctg cttccaaaca agccaagcat tttgtagtaa attttaaaaa 21420
gctaaatttc ataataacct ttataattgc tacctgaaaa tggagtttgg aatggcacca 21480
aattattaat cagtagaagg agaatagatt gcaaaaaggt aattatatca aattgaatgc 21540
ttcttgaaat atattctgtt aaataaaata cagttcctct tccctgttct catttgtaag 21600
caatgaggac aattcaggta gcatgaatca ggaatcagag tacttttctg ttccgcagat 21660
tggcttaatc attttgggct cacttcaaac tgtagatgag cattgtcctt taacctgccg 21720
ggcagcagag gtgaccacgt tcactgcaaa gcccagaagt gagttgttca tctgggttag 21780
ggtctcagga agaggcatgt tatgacttcc ccacattcct ggacagttta gactgtaatt 21840
cttggttgtt taaaaaggtg tttgaatgta ttttgttcag aggcttgagt aaatggctct 21900
tattcttaca cttttttttt tgcctgcttt ttccagcatt agaatagttt cagccctgac 21960
aattagcctt atgctgactg gagtggattt tttgcgtagg accaagtgtt ttgcatgatt 22020
tacaatggac tgcataaatg tagcctaaca agttatatga tgtgaagaaa attacttaag 22080
ggtaaaaatg gattgtttac tgagaaaatg atgttattat aaactggttt gagggtattg 22140
tggtttagat gctgtcctta caaatttgag ctctgttggt ttggagattt aatatctaat 22200
aataatagtt aatgtgtaat gaatacttgc cctgctccag gctctatgtt acgtgtcctg 22260
aatgcatcat ctcatttaat cctccataaa atttgaagca gtcagcattg taattaatct 22320
cacttttcag atgaggaaac caaggtatgt aagaaacaaa accagtgttg aatctgacta 22380
gtacgactgc aaagcctgac tcttaaccat tgtgttctgt tgccttctcc aagggaaagg 22440
agtgcttcca gaaaggccat tggaagtgag gagtgtcacc tgataatagc tggctcctca 22500
tcccactagg ttaacaggca acagatgggg gccacagagt tggctggagt tagtcctggt 22560
tgccaagggt aaatggggct gttaccatgc aatggataga aactaggatg ctcctgtgtg 22620
tctccttata tcctaccagg tgtagcatga aggttaagga caattccgtc ctcttatttg 22680
gagaggagac caaggccctg taggactaaa ggtttgggtg agctgaaagc caagggcaat 22740
tggcccagct tggggaggag agttacaaat acatttgtga ctatcagacc agaaacagac 22800
tgcggtgttc acctgcggtt tgggtacctg ctgcttgttc tttgctgggg tatctgaaga 22860
ctgaaaacac agctcacact tagccttttt cctattctgt gtgaaagaac tgtagtagtt 22920
ttctcttgct taacaaggtt ctagtggtga agtttattgc ttagtctgtg agttgtaggg 22980
aaataaccac aggatgggat ggtagcagaa cgagaaaaga aaagaagatt gtagatgcca 23040
ggctggtgtc ggcttgaaat ttttaaagta tacagttctt ctttatggat tattaaaaaa 23100

aaacagcaaa tgaatacaca agattaaatg ttaagcagta caacattgta catgatgaaa 23160
ggttaaagtc tttctctggt ctcattcaaa aatctacagt ctcttgtagt atagtttgaa 23220
gtcaggtagc gtggtgcctc cagctttgtt cttttggctt aggattgact tggcaatgcg 23280
ggctcttttt tggttccata tgaactttaa aattctgtga tgaaagtcat tggtagcttg 23340
atggggatgg cattgaatct ataaattacc ttgggcagta tggccatttt cacgatattg 23400
attcttccta tccatgagcg tggaatgttc ttccatttgt ctgtgtcctc ttttatttca 23460
ttgagcagtg gttttgtggt tctccttgaa gaggtccttc acatcccttg taaggtggat 23520
tcctaggtat tttattctca ttgaagcaat tgtgaatggg acttcactca tgatttggct 23580
ctctgtttgt ctgttattgg tgtatagaat gcttatgatt tttgcacatt gattttgtat 23640
cctgagactt tgctgaagtt gcttatcagc ttaaggagat tttgggctga gacgatggag 23700
ttttctagat atacaatcat gtcatctgca aacagggaca atttgacttc ctcttttgct 23760
aattgaatac tctttatttc tttctcctgc ctaattgccc tgtccagaac ttccaacact 23820
atgttgaata ggatggtact ggtaccaaaa cagagatata gaccaatgga acagaacaga 23880
gccctcagaa ataataccac acatctacaa ccatctgatc tttgacaaac gtgacaaaaa 23940
caagaaatgg ggaaaggatt ccctatttaa taaatagtgc tgggaaaact ggctagccat 24000
atgtagaaag ctgaaactgg atcacttcct tacaccttat acaaaaatta attcaagatg 24060
gattaaagac ttaaatgtta gaactaaaac cataaaaacc ctagaagaaa acctaggcaa 24120
taccattcag gacataggca tgggcaagga cttcatgtct aaaacaccaa aagcaatggc 24180
aacaaaagcc aaaattgaca aatgggatct aattaaacta aagagcttct gttctttgct 24240
ggggtatctg aagactgaaa acacagcaaa agaaactacc atcagactga acaggcaacc 24300
tacagaatgg gagaaaattt ttgcaatcta ctcatctgac aaagggctaa tatccagaat 24360
ctacaaagaa ctcaaacaaa tttacaagaa aaaaggaacc ccatcaacaa gtgggtgaag 24420
gatatgaaca gacacttctc aaaagaagac atttatgcag ccaacagaca catgaaaaaa 24480
tgctcatcat cattggccat cagagaaatg caaatcaaaa ccacaatgag ataccatctc 24540
acaccagtta gaatggtgat cattagaaag tcaggaaacg acaggtgctg gagaggatgt 24600
ggagaaatag gaacactttt acactgttgg tgggactgta aactggttca accattgtgg 24660
aagacagtgt ggcgattcct cagggatcta caactagaaa taccatttga cccagccatc 24720
ccattactgg gtatataccc aaaggattat aaatcatgct gctataaaga cacatgcaca 24780
cgtatgttta ttgcggcact attcacaata gcaaagactt ggaaccaacc taaatatcca 24840
acaacaatag gctagattaa gaaaatgtgg cacatataca ccatggaata ctatgcagcc 24900
ataaaaaagg atgagttcat atactttgta gggacatgga tgaagctgga aaccatcatt 24960

ctcagcaaac tattgcaagg acaaaaaacc aaacactgca tgttctcact cataggtggg 25020
aattgaacaa taagaacact tggacacagg gtggggaaca ttacacactg gggcctgttg 25080
tggggtgggg ggaggggga gggatagcat taggagatat aactaatgta aatgatgagt 25140
taatgggtgc agcacaccaa catggcacat gtatacatat gtaacaaacc tgcacattgt 25200
gcacatgtac cctagaactt aaagtataat aaaaaatatt taaaaaatct acagtccctt 25260
tctcaatagg taaacactat taacaatttc ttctgaaaat tacatatgta cacacacaca 25320
caccccacac atagatattt tgttaacata cattacctta tgctctacac attattctgt 25380
gcctagcttt tctcatcgaa taatgtgtat tggagatctt tccatataag tacatattgg 25440
tctcactcat tcatttttaa tggctgtata ttagtccata gtatggagta ataatacctt 25500
ttattggcac aaagttttgc agttgacaaa gggtttgcat atattgtttc attgggttgt 25560
atagcagtca tcaacggaca tatcaagaat cccagaaggg taacctgggc ttcacccagg 25620
atcgcatgga gcttgggact caaacagcta acattcccta aacaggccca tattcctgca 25680
atttagtgca gctgctacaa catttaggta aactctttga aagcagtaaa aagatcacca 25740
atctgggaca tcaagaagcc agacgacaag gagacataga cctcaggtga gtgagaaaag 25800
agaataattg aagtttagaa ctgaatgggc actaacgaac aacagataca tctctttcct 25860
ttcaccgatg aggacctggg tgtggcctgt ggatgttaag tagcatgttt aggccacaca 25920
gctggattgt tttctttcct gaggctagct ctggagaggg gaggaaacgg agatgctaga 25980
tttttccggg ctctcttttc tcatcagcta gaatgggatg tggatggaat gcagcttgag 26040
aagccaaaca cccttgagaa atgagaactc tgctttctga tgtggggcgc atcctatctg 26100
agataactct cctgccagcg cagtatgacc attgcgactt gagctgtgtg ggtgcccagg 26160
tggtgtagac cttaaaacca ctcctgcttt tgtttactga atgtctaata ttcacacagt 26220
tctttgtctg agtgcagggc caaagcagca aataacgaag aagctgtgcc caaccataag 26280
aactatgaaa tgctccagat catctacgct agtttgagtt aaatttacca tgcaaataaa 26340
tatagaacat ttcttcttat gcatagattg tatttgttct attaaacaac catcctgtaa 26400
aggttttatt cattcattta tttttatttg ttaggagaaa ttgttattta agctaagaaa 26460
gtagactagt agttatttct tttttttgc ttattatact ttacgtttgg ggatacatgt 26520
gcaggttagt tacgtaggta tacacgtgcc atggtggttt gctgcaccca tcaacctgtc 26580
atctaaatta ggtatttctt ctaatgctat cccttcccta gccccccacc ccctgacagg 26640
ccccagtgtg tgatgttccc ctccctgtgt ccatgtattc tcattgttca gctcccactt 26700
atgagcgaga acatgcagtg tttggttttc tgttcctgtg ttagtttgct gagaatgatg 26760
gtttccagct tcatccatgt ccctataaag gacatgattg catccttttt tatggctgca 26820
tagtattcca tggtgtatat gtgccacatt ttctttatcc agtctatcat tgatgggcat 26880

ttgggttggt ttcaagtctt tgctattgtg aatagtgctg cagtaaacat acgtgtgcgt 26940
gtgtttttag aatagaatga tttataatcc tttgggtata tacccagtaa tgggatggct 27000
gggtcaaata ctagaaggct acagtaacca aaacagcatg gtactggtac caaaacagat 27060
atctagacca ctggaacaga tcagaggcct cagaaataat gctacacatg tacaaccctc 27120
tgatctttga taaacctgac aaaacaagca atggggaaag gattccctat ttaataaatg 27180
gtgttgggaa aactgactag ccatatgcag aaaactgaaa ctggacccct tccttacacc 27240
ttataagaaa attaactcaa gatggattaa agacttaaat gtaagaccta aaaccataaa 27300
aaccctagaa gaaaacctag gcaatacaat tcaggacata ggcatgggca aagagttcat 27360
gactaaaata ccaaaagcaa tggcaacaaa agctataatt gacaaatggg atctaattaa 27420
actaaagaac aactgcacag caaaagaaac tatcatcaga gtgaacaggc aacctacaga 27480
atgggagaaa atttttgcaa tctatccatc taacaaaggg ctaatatcca gaatgtagaa 27540
ggaacttcaa caaatttaca cacacacaca cacacacaca cacacaaaca accccatcaa 27600
aaagtgggtg aaggatatga acagacagtt ctcaaaagaa gacatttaca ctgccaacaa 27660
cataaatatg ctgccaacaa gcatatgaaa aaaagctcat catcactggt caagagaaat 27720
gcaaatcaaa accacaatga catactatct cacaccagtt agaatggcaa tcattaagaa 27780
gtcaggaaac aacagatgct agagaggatg tggggaaata ggaacgtttt tacactgttg 27840
gtgggagtgt aaattagttc aaccattgtg gaagacagtg tggcgattcc tcaaggacct 27900
acaattagta gttatttcta ggtctgacgg gctgtctttt agtttgtttt cttattctga 27960
ggtggactta aaccaatttt aaaaacaggg atgactgaat tcgtgtgtta atcttgtact 28020
acaggaaccc tttgacattg attcagaagc acccaagtgt ttgttcatta atcctttttt 28080
atttttgcat attatttatt tttcaacttt tattttaaaa atcaggggta catatgcagg 28140
tttgttacaa aggtatattg agtgatgctg aggtctggca tatggatgaa tctgtcactc 28200
aggtaatgag catagtaccc aatgatagtt ttttgtaccc aatgatagtt ttttgttttc 28260
tttgtttttt tttgtttgtt tgtttttga gatggagtct cgctctgtca cccaggctag 28320
agtgcaatgg cttgatctcg gctcaatgca acctccgcct cccagattcc agcaattctc 28380
ctgcctcagc ctcccaagta gctgggacta caggcgtaca ccaccatgcc cggctaattt 28440
ttgtattttt agtagagatg gggttttacc atattggcca ggctggtttt gaactcctga 28500
cctcaggtga tctgcccacc tcggcctccc aaagtggtgg gattacaggc gtcagccact 28560
gtgcctggcc ccaatagata gtttttcagt ccttgcccct gttcctcctt cccacttcta 28620
gttatcacca gtgtctattg tttctgtctt tatgttcatg tgtaccaaat atttagctcc 28680
cacttataag tgagaacaca tggtatttgg ttttctgttt ctatgttagt ttgcttagat 28740

agacctccag ctgcatccac gttgctgcaa atgacacgat ttcattcttt tttatggctg 28800

tgtagtattc cgtggtgtag atataccata tattctttat ctaggataac tgatgggcaa 28860

ttgggttgat tccatgtctt tgctattttg agtagtgttc tgatgaccat atgggtgcat 28920

gtgtcttttc aatagaacaa tttattttca ttactcttat caggatacct gggaggaact 28980

tctcctacga agttaatttg gggggactcc tgaagatgag tgaaacccct tattaagcac 29040

ttagagggtc gaaagtgtac aagggaacgt tcaggtgtga cagcttgaga gacatgcata 29100

ttccaccccc acaccagagc ttagtgaacc gtcttttcta ttttctccca aagcaccaaa 29160

atggcccaga aattggaaga tggaaaatgg acttatttat gagtctggga aggcagggca 29220

gtaagcacag tcctgttcag ggttctgagt tttaccctct tgctctgatt gcgagatcat 29280

tttcctcttc cttgcttctt cagcttagga caagaacagt ttggaaattg tttctactac 29340

tttggacacc atagtttaga tttcaaatga gtacaagtgg gaggaaagct tggataattc 29400

tctggaaata atcgaacaga gtgaaggaga ggggtctaca ggtgagctga atgctgcatg 29460

gcattgaagt aatcacacca gtcgtcacaa ttctctcctc tttgatggtt atctgttgtc 29520

ccagaaggaa caatttcaga aagtcctttt ctggactgta gaatagcact tgcttatttg 29580

atgagccctg agaagcatta ctgaaagcgg ttcattgtcc ctgaggtatt acaatgagat 29640

ggtggtcact gatttcatta tgttttcctt tattgcagct gttggtttga tcctttgcca 29700

ggtgcttaaa acaattgtgg ttttgcagat ggtaagttag aggttggaca aaaaaaggga 29760

tcatgtcact gccctggcca aaatttcaac agactggggt ctagtgaggg caaatagata 29820

gaggctttcc tcttcacttt gtgttattta gaaaaagaaa ctttccagga caaatttctt 29880

tcctagaatt cctttttttaa aaattttttt tctttgaaaa tttacttaga tgcaaataat 29940

atatttttct tccttttaaa taataaaagt aagatgtctc ttggaggtgg tggttgtcac 30000

tgacaagatt aactagaact gactagctgt aaaaatataa tttgggatgc attattaagg 30060

catgccattt ttatttgcat gccattgtgt acagatgtgg ttgtgaaata gttcaaatca 30120

tggcacattg aatgtcctca ctggattttt aggaatgtgt tcactgagac agccaaatcc 30180

tatttcattt tctttggctc attgcattgg ctgtaaattg gagatattca ctttaatatg 30240

tgagtcaaaa tttatttcca aacataatac tgcagttgtt ctgtcacaga atataaattt 30300

cttatttatt tccttaatac gttgctttct actttttctt tttttcttta ttttattctg 30360

gagtatgtgg aaaggttttc cagaaagatt tgcatatgcc ataatctact gatgaatact 30420

ttttttgggt tactctttca tattttggga gatataacta tggaagtgtt aggaatcatg 30480

ggttctggaa atagttttat tactgcttct gaaatgccct cccaatgata ccatatagta 30540

attccatcag ggaataatat ttttattata gtttaaaata taacttaata tttaggtgct 30600

cttgttcagt catcgtcaag ttctttttat ttcaccaacc ctaccatggc actcctgaaa 30660

gacttgtgaa tgcgacagac ctggatttaa tcatggcttt gccatctgct agccaagaga 30720
acttgaacaa gtgagtcaac ttcttggagt ctcattttct gcttctgtaa catgggaact 30780
agggtaatct aactcattgc ttgtgatgat tagatgaggc aaaatgctga gttcacctag 30840
cccagcacct ggtccatggg aagcatgtgg gttctgctgc tacccagtcc ttggcccagt 30900
gcatggtgca cagaagggaa tctgaacagg ccaactttat tcctattctt gacccacccc 30960
atgtagatgc ttcctacatc ttcagcttct tcttcttctt cttttttttt ttaaggcagg 31020
gtctcactct gtcccccagg ctgaagtgca gtggcacaac cacagctcag ggcaacctcg 31080
acctcctagg atcaagtgat cctcccacct cagcctcctg agtaactggg atgacaggac 31140
cacactacca cacttggcta atttaaaaac ttttgtagag ctggggtctt gctatgttgc 31200
ccaggctggt ctcaaactcc tggattcaag tgatgccctc acctcagcct cccaaagtgc 31260
ctggaaaaca ggcctccaca cccagccttc aacttcattt taaaaaattg tggtaaacta 31320
tacaattcat ccatgaaagc agaaaccact tgtaccccaa aagctattga aatttaaaaa 31380
tatatatatt aaaaataaaa ataaaattgt gataagatat acataatatg aaatttacta 31440
ctttaatcat ttttaagtgt acggttcagt ggcattgagt acattcacat ttttgtgcaa 31500
ccggaacttt tcatcctccc aaagtgaagc tctgtactta ttttttattt tattttattt 31560
tttgagatgg agtttcactc tagtcgccca ggctggagtg cagtggtgca atctcggctc 31620
actgcaacct ctgcttcctg ggttcaagag attctcctgc ctcagcctcc caagtagctg 31680
ggattacagg tgcccaccac cacacccagc taagtttttg tatttttagt agagacgggg 31740
ttttgctatg ttgggcaggc tggtctctaa ctcctgatct caggtgatct gcccgcctca 31800
gcctcccaaa atgctgggat tacaggcatg agccactgtg cccggccagc tctgtgctca 31860
ttaaacaatg actccaagtt ccccttcccc acatctcctg ctgacctctc ttctactttc 31920
tgtctctgtg agtttaacta ttctaggtac gtcatgtaag tgcatctatg tgatatttgt 31980
ccttttgtgt ctggcttatt tcacttagca taatgtcttc atgattcatt catgttgtag 32040
catgtgtcag agtttccttc ctttttaagg ctgaataata ctccactgta tggatagacc 32100
acactttatt tatccatttg tctgttgatg gacatttgga tgatttccat cttgtgggta 32160
tactgagtaa ttttgctatg aacatgggta taaaaatatc tatttgagtt tctgctttga 32220
tctgtgtgcc tgtggtggga ggtggggagg gagggtcaag tgaggaagga gattgaatct 32280
agaataatgc ccatgtttca ggcttcacgt gcagacattt cttggttcag ctgggaagtg 32340
aataggaaca gactggcgag gaaagaagac agtcgtcagt tctgtctggg gcacatccat 32400
cttgacgagt gtgagagagt caagtcttga tggacagggg caggtggaca ttcaggaaac 32460
tcaggaaaga gatttgatct gaagtgacca ccagaaaata ctgacggaaa agaggtgaca 32520

aaaggagaga cctgcaatat tatattggct ttctctcagg caactgcttg gcctgtcttt 32580
atattccttt tgaatctctg catatgtacg ggccattatt tattttcaca actaacaaat 32640
gcagactttc tgtgtaatga caacaaagcc aaaccagctg ctaccaaagg agggaaaatc 32700
agaagagaag gaaaaagaac aagggaagct tggggaaaag ctgaatgtgg gtccttctgt 32760
tgctgcaggg gctggggtgg gcccggtgat tcctactgag aggcgttttc tctccccgct 32820
tcctgtcttt ctggttccat ctcattcacc tcctgtcccc tccacttcct gccagtcaaa 32880
ccttagattc ctccagaggc tttttatttt tatcttttga tgggcaagaa aatagtgcgg 32940
attatttttc caaaccttca cctgaacatc acatcgtggc tttggcccta tgggcttggt 33000
tcatccgggc ctgcacagaa ggacttttcg ggccagtctg gtcacataca tcgagtcctg 33060
tcttttcagt taaaaaaaaa cacacacaca cacactgcta tgtttcacta agacaactgg 33120
tgtgagttgt tttttagaaa atcaactcta cttcagtaag attttctcaa gcattatctt 33180
gagaagacca gataataaaa tttaaaaaga atttcttctt tttttttat tatactttaa 33240
gttttagggt acatgtgcac attgtgcagg ttagttacat atgtatacat gtgccatgct 33300
ggtgagctgc acccactaac tcgtcatcta gcattaggta tatctcccga tgctatctta 33360
atgcaactta aatcaattgc tttaataaca catattgacc aagttacact cattaaggaa 33420
aaaaaactac tttgttgttt ttcttcttct gacgtgagct gaagacttag aaatagttgt 33480
taatagtgtt tggttaataa gaatttgttt taattgcata tatcaaatat gtatttatta 33540
aacttttctt tttgtcagtt tatcaattct agctttgtca caaaaggttt gccgtatgaa 33600
catgattctg ttgtacatct atttccattt ttgttaagag acgaattcaa ttgtaaaaat 33660
ctagtacctt ttattcatta aacatgttag ttcaggaatt tcacttggtt ctacaaagat 33720
acatatctac agtggatggc cagtgcaaac atgagactca gccaactggt ctctgaccca 33780
attcagttct cctgtcttct tctggcttac aaagtaactg gctctgggga gaaagtgagt 33840
caaagtaata tttggtttga atggttattg actattttct tctgaaactt aatgtatact 33900
aattaatttt ttattttatt cttttttttt tagagatagg gtctcgctct gttgtccatg 33960
ctggagtgca gtggtgctat cacagctcac tgcagccttg acctcctggg ctcaagcgat 34020
tctcctgtct cagcctcccg agtagctagg attacaggca tgtgccatca tgccaggcta 34080
atatttaatt tttttttttt gtagagacaa gatattgtta tgttgcccag gctggtctaa 34140
aactcctggt ctcaaactat cctcccacct cagcttcctg aagtactgga attataggca 34200
tgagctgcca cacctggcca tatactcatt ttttgttaaa agctgaaata tatcagcata 34260
tactgcataa ataccacagg agactaaaca ctgaaagttt ctttagggta tcagaagaat 34320
acactttttg cttgcagtta gcatctgcac agataagttt tgtttctggt tctattactt 34380
cttcagtttg accctattaa taaggacaat tctaaaaata ataactgtgt ctggatatct 34440

gaatccgtgt gtggtctttt actgaagtta caggtttata actctgctga ctagtttgct 34500
ggtttctgtt atgcaataga agagtgcaaa tgttaatttg atctgaagcc cgtgtataca 34560
gtgactttta taatgtatat ttaaagatgg aaagccaagt tttatgaggc cagcatttct 34620
tgtcaagtcc tcactccacc ctcttctaat tgggcctgac ccttaagttg aataaagaac 34680
aaagagctct gtagttaaaa catttcactg catgttgcat cttgccctta gtaaatggaa 34740
aaaaatagag acttaagcag agaatctgaa ctagggtgtg aaatatatat tcagttttgg 34800
ggtgggagaa tgagaaccat gttttacaat agtatacata acttccttag tctaaattca 34860
ggacatttcc ccaagatatg taagaattta gacttatgca gacagacttt ataaaaacat 34920
gccaatattt attagtttgt gaattttaat attctgctcc ctataaacca gatttatttt 34980
gagggataaa gggatggagg tgacttctaa atcttagagc agaaacttcc tgtgggcagc 35040
tggacatatg taccaggagc tcagagaaga gggttgtgtt gggagcgtac attctgagtg 35100
atctgcattt ggtgatgatg gaagccatgg acatgcacta gattgtcttg tgggagaata 35160
tggagtagta agagaagaag aagacctagg attgagccct gagcacctct ggcttaatgt 35220
tggatggagg aaattgaatc tgtaaacaat accgggaggc tgcagcctga gaggcagaag 35280
gaactggggt gtttgggatt atggaagcca agggaaaaag cctgtctcac agcgggaagg 35340
gaggtatcaa cattgtaagc tgcttcagat aggttatgta ggatgtggac tgaaaaatac 35400
ctgtaaaatt tggcaacgcg attcattggt aatcctaggg aagttgcttt tttggggtaa 35460
ctaaggtgga aaacagattg gtgtgggttg agcaatgcaa gagaggtgaa gaaaaggaga 35520
tttcatgtgc agacgtttct gagttcagct gggaacaagg gataagatag aagatggaag 35580
ttagagagca tgtggggcaa gggagactct tttatgggac agtctcgcat gtgatcaaag 35640
ccaatgagta aggagcattt gagggagaga gagtcaatca acaagagaga aaagaaagag 35700
ggttcatcat aaaataacgg taacaacaac gaacatcttt tgagtgtttt ctatatccgg 35760
ggaactatgg taaactccta acctgcattc tcacattcaa ttcttagaat cgttggatgt 35820
ggtgggttcc atgatttcct ctagattagc gaggaggaaa gagagatcta gaaatgtcag 35880
gtagcttgct cagagttctc caggtagtca gtcatggact aatttgtgaa ctgaaggact 35940
gaacttcgtc accacccagc ccaccaagcc ggcttgactt taggtattct gtgctgcatg 36000
tgagtaccga cttaaattat attttaagaa gggctacttt gaaactctct ctctgaaaac 36060
tctatttcta aaagctctac cctcacaaca attttggcaa gcagtcttgg taaaaccaaa 36120
ccaaaccaaa ccaaaaccaa aaaaccttat ctgctgagaa aatataacca cataaaatat 36180
ggtgctacaa aatatagact gtgtgaactg aaggtgactt gcccaaagga ctcctgaagc 36240
aattggctgc tgtagaaatt aagtccacgg gaggtttttt gttctgtttt tttttttttt 36300

tttttgagat ggattctcac tctgttgcct aggctggagt gcagtggcgc aatctcggct 36360
cactgcaacc tccgcctccc gggttcaagc gattctcctg cctcagcttc ctgagtagct 36420
gggattacag gtgtacacca ccatacccag gtttttttg tatttttagt agagacgggg 36480
tttcaccatg ttggtcaggc tggtcttgaa ctcctgacgt cgtgatccac ctgcctcggc 36540
ctcccaaagt tctgggatta caggcatgag ccaccgtgcc cggcccatga gaggttttgt 36600
ttgcacttca agaaggacag aaaaaggcag gcaggctggg gagcaacata gtaaggctga 36660
ggaagtgata ggaaaacagc ctccaaaagg tttccctgta gattctgact ggctaagttt 36720
cctgaaataa tattaattct gtcctcttgc ttttaatagg acataacgac tatatgtgtc 36780
cagccaccaa ccagtgcacc attgataaaa acaggaggaa gagctgccag gcctgccggc 36840
tccgtaaatg ctacgaagtg ggaatgatga aaggtggtag gtacatctct cccaggggcc 36900
cttggggatg gccctggcca ccgcccagtg ctggctctac ccattggaat aacaccatgg 36960
gaattttgtg tttttttctt ttaattgttt tttttctatt cttatttttc tttgcaacaa 37020
aagtattttc ataatccatt ttattttaaa aaggtggaag tgtctggaac tggaaattct 37080
aacatggcat tttgtgtttt ggattttcaa tgtaaataat tatattttaa atcaaaggtg 37140
tgtgggaggc ggtgatggaa ggaaacgaag agtgcttagt aaattattct agaaatattt 37200
ttcagttact gtttatgttg caaatgctag aaaatgatat ctgaggataa actttcccta 37260
aattgagact tgtaaatgtg aaagctgagt agctaattta tagccttcca gtctgttatc 37320
atcccttaag gaatgtgaat ttcaatcaaa aggcagtttt ctcctttaga acctgagtga 37380
accagccact ttcttaacct cagtgtctag catggtgctg gcatagttga ttactgagca 37440
ctacactaac aagtgtcaga gcatgcatgg tttgtgactg tgggtttgtg tttttgtggt 37500
ctttgtggct gtgtgtgtgt gtggttttct gtcttctcat gtatccgatc ttccagtttt 37560
gtcatacagg aatctggaaa ctgaatccca gttctgggaa tattaggagc cccataaatg 37620
tggttgcctg attgacctca ttgtattctt gggagtctca tcttgaggaa cattgcttct 37680
agtcttggat agccttcagt gtagtggaag agaacaagta gaaaatgtgt aattaaaata 37740
aagtcatgag gctgaggtgg gcagatcatc tgaggtcagg agttcgagac cagcttggcc 37800
aacaaggtga aaccctgtct ctattgaaaa tacaaaaatt agctgggtat ggtggcgagt 37860
gccagtaggc ccagctactt gggaggtgga ggcaggagaa tcacttgaac ctggcaggtg 37920
gagggggcag tgaactgaga tggcgccact gcactccagc ctgggtgata gagtgagact 37980
ctgtctcaat aaataaataa ataaataaat aaataaataa ataaataaat aaataaagta 38040
acggattcat ttagtgtttc agaaggatac tgaagggagg gaagggctga tgatggtgct 38100
acctgctgat tgtaataggg aaaagcccgt ttcttttctg aaggaggtga agcttggggt 38160
gatttaagga gaacaaaatt tcaatgaaaa ggaatagtga ttttgcaaat agtgggcagc 38220

taacctttaa atcattctta ctgggacgca ggaggaagga gaggaaagac caagaatgga 38280
gattatgatg aagacaagct tgattgttaa caagcttaca gtgacaggtt tcatagtccc 38340
ggggtcataa atacgccaga caggttgagg tttggaatgc agcatttgga ataaattctc 38400
ctggtgaaga gatgcaatgt tgaatttacg cattctgtga gctgtgacta tgactataca 38460
tcttgaacat ctgaaaaagc agctttaatt caaagggtaa ttaattccaa gaatttaaca 38520
gctacattca gaaaatgaca cttgagtcat atggattaaa tatttaagga attcatcttt 38580
ctttgtactg ttggagaatt agtcaggctt aattaaactt acaaaatgcg tcttaaggta 38640
acttggtaag tgacaggaat ataacagctg cataagaaaa gctttatttg aaacagtgga 38700
gtcgaggttt aatattcttc tttggcgatt atatgtaggt taagcacagg ctcttccata 38760
cattctcctt gaaagctgaa ataatcaagt catgaatatg tccaaaacaa tttttaaaat 38820
gtgaggggta cccatgcatt gaccccattt cataaaaccg attctgtttt tttttttgta 38880
attaatatcc agatacggtc tggctgcatg aatataaatt acgcattctc atttttaatc 38940
taacaaaaat tcatatatgc aaagacataa taaaagtctc cccactgttt tctcctagga 39000
atctagataa tttgactgta cacaacagac tgtggatggc tccatacaca cttgcacatg 39060
tatattgatg actgtaaaat atatatctgt attagttttc tagggctgcc ataatgaaat 39120
accacaggct gggtggctta aacaacggaa attaattttc tcccaattct ggaagctgga 39180
catgcataat caaagtgctg gaatatttgg tttctagtga gccctccctt tttttttttt 39240
gagagatgga gtcttgctct gttggccagg atggagtgca gtggcactat ctcagctcac 39300
tgaaaccttc acctcccagg ttcaggcgat tctcctgctt caacttccca agtagctggg 39360
actacaggtg tgtgccacca tacccagcta atttttgtgt tttttttttt tgtagagacg 39420
gagtttcact ctatgttggc caggctggtc ttgaactctt gacctcaggt gatccgccca 39480
cctcggcttc ctaaagtgct gggattatag gcgtgagcca ccatgcccgg cctgtgaggc 39540
ctctcttcta ggcttgtggc tagccgtctt ctgcctgtgt cctcacatgg cctttcctct 39600
gcgtccgagc actcttggta tctctttctc ttctcacaag ccctgttgga ttaggagtcc 39660
acccttatga cctcatttaa ccttagttac cgccaaaaag gccctatttc caaatatagt 39720
cataccaggg gctagggctt caacatacaa gttttgtggg gacacagttc ataacaggct 39780
gccctcccaa aataccatag actgggtggc ttaagtaacg gaagtcaatt gcctcatagc 39840
tctggaggct gaagtctgag actaaggtgt tggcaggttt gatttttccc gaggcctctc 39900
tccttggctt gaagatggct gccttctctc tgtgtcccca catggccttt tctcggggca 39960
tccacagctt cttcttcttc ttacgaagac accagtcata tcggattagg gccccacaca 40020
caggacctca ttgaatctta atcacctcac ttaaggtaca atttccaaat acagtcacat 40080

tctgaggccc aggggggtaag atttcaacat atgagtttta agggtacaca attcagtatg 40140

tggcaatatc ccatagggggt tactcagtga ttgttcagta tttctttaca tgtaaggaaa 40200

acatgtatct ttctggcagt ttttatatgt acataagttt taaatgaagc tttaaattca 40260

taaaattata gcccgtgaga acatcttatt ttaattgcat tgaatctgta gattgatttg 40320

tgtcatatgg atatttttaa caatattaat tcttccagtc tatgtatggt atatctttcc 40380

atttatttgt gtcttcttca gtttctttta ttgatgtcta tagcttttag catacaattc 40440

tttcacttcc ttggttaaat ttattcccaa atattttgtt ttatttttat agctattata 40500

aatgaaattt cttcttgatt tcttttttttg gatagtttgc tgttagtgta tagaaacact 40560

actgatttct gtatgttgat tttctcttct gaaactttac tgaatttgtt tattagttct 40620

aatagttttt tgtgtggagt ctatacttac atgatctatg tgtaagatca tgtcatcagc 40680

aaacacgggc aatttaactt cttcttttttc aatttgcatg cctttatttt ttttcttgca 40740

taattgctct ggcaggaact ctcagtacta tgttgaatag aagtggtgag agtgggcatc 40800

cttgtcttgt tattgatctt aggggaagac ttttcaaatt ttcaccattg agtatgatgt 40860

tagctgtagg cttgtcatat atggccttta ttgtgttgag gtacactctt tctatattta 40920

atttgttgag agtttttgat atgaaaggat tttgaatttt gtcaaatgca tttttttcctc 40980

tattgagatg attgtatggt ttttgtcctt cattctgtta atgtgtgtac catagttata 41040

gatttgcata tgttgaacca tcgttgcatc cctgggcaaa tcccactcga tcatggtgaa 41100

tgattctttt tcatgtcatg acaatcaatt ttgctagtat tttgttgtgg atttttgcat 41160

ctatattcat cagggatatt ggcatgacat ttaattttct tgtaataccc ttgtctggct 41220

ttagtatcac agtaatgctg gccttgtaaa atgagtttgg aagtattccc tcctcttcaa 41280

tttttgggaa gagtttgagg attggtatta gttctttaaa tatttggtag agttcagcaa 41340

taaagcccat taggtcctgg gcttttcttc catagaggac tttttattgc tgattcagtc 41400

ttctttctag ctattggtct ggtcagactt tctatttctt catgattcag acttgctagg 41460

tttatgtgtc taagacttta tccatttatt ccaagttatc caagttgttg gtgtgtaatt 41520

catcatagta ctcttataat tttttgcatt tctgtgctat cagttgtaat gtctcctctt 41580

ttatttctga ttcagtttat ttgtgtcttc tcactttttt tcttagtcta gttaaaggtt 41640

aatcaatttt gtttatcttt ttaaaaacaa attcgtagtt tcactgatct tttgttttgt 41700

gtttttagtc tcaattttat ttatttttcc tccgatcttt attattttct tccttctact 41760

aacttggggc ttagtttatt tttatttttc tagttatttg tggtataaca ttagattgtt 41820

tgagatcttc ttctttgatg aagacattta ttgctataaa cttccctctt gaaaatactc 41880

ttgctgcatc ccacaaattt tggtatgttg tgtgtccatt ttcatttgtt tcaagatatt 41940

tttcaacttt tctttttatt tcttctttga cccactggtt ggttctgagt atgttgtttt 42000

attttcaagt atttgtgaat tttccaaaat tattcttgtt gatttatagt ttcatattat 42060
ttatggttgg aaaaaatact tgatatggtg tcaatgttct taaatttatt aagacttatt 42120
ttgtgtccta acatatgaac tatactgttt tttttctttt tttttttttg agacagagtc 42180
tcactgtgtt gcccaggctg gagtgcactg gctcaatctt ggttgactgc aacctccgcc 42240
tcctgggttc aagctattct catacctcag cctcccgagc agctgggaat acaggagcac 42300
accgccatgc ctggctaact ttttgtattt tttagtagag atggggtttc accatttggg 42360
tcaggctgct ctcaaactcc tgacttcaaa tgatctgccc accttagcct cccaaagtgc 42420
tgggattaca ggagtgagcc accgtgccag gccatgatct atactgtttt atctaacatt 42480
cagtggataa tgttccatgt gtatttgaga agaatgtgtg ttctgttgct gttggatgaa 42540
atattctgta tgtatctgtt aagtccatct gtgctaaagt atagtttaag tttgaacttt 42600
cattattgat tttctgtccg aataatctgt ccattgctaa aagtggggta ctgagttccc 42660
caatattatt gtattacagt ctatcttccc tttcatatca attaatattt gtttcattca 42720
tttaggtact ccaatgttgg gtgcatatgt atgttcacct gttatatcct cttaatgaat 42780
tgaccctctt gtcattattt aatggccttc tttgtcttat tttatagttt gtgacttaaa 42840
gcctatttta tttgatataa atatagctgt ctctgctttc ttttggcttc tattttcata 42900
gaatgccttt tttggtccct tcgccttcat tctctgtgtt ttcttaacca tgcattgaat 42960
ctcttctaga catcatataa cagggtcttg tttttttaaa tccatttact cactgtatct 43020
ctttcttcct atcttgctgt cttcctttgt gattaggcga ttttctatag tggtatcctt 43080
tgaattctta ctttttgcct tttgtatatt tgctacagga ttttgctttg tgattaccat 43140
aaggcttaca taaaagatct tatagttata ccaggctatg ttcaactgtt tataacataa 43200
ctttgattaa ttttttaata ctcaatttat ttgattgctt atttagagat aattttgatt 43260
cctatcagaa gaaaactgag gtatctttat gtaccttttc aggaatgacc acgtctcacc 43320
ttaccttgaa tgttacaaga aatgctggca gtgactcaag gccaggcaaa aatttcagaa 43380
agggtttatc ttgtttgtac aattttcttt tgcaaaaata aatgattctt gatatgatgt 43440
aaggactttt tccgtagata gaacctataa aatgcagaac tttggggcag taattatatg 43500
aatgaattgc tactttaaaa ttctgtaagt ccccaggaat ttccatatgc tttttttttgt 43560
ttgtttcagt ggttgtgatc aggctaatgg gtagttttta gagctgaaaa gaacttaaag 43620
atcatctagt gcaccttttt gtttttcagt tggattgttc ttgtccaaat cactaagcta 43680
agttaactgc tcagacagga aaccaagtct cctgacccat ggtttaatgc tcctttcatt 43740
gttatgcgtc tgcctggctg acctgttagt aacataaagt gtcatgagag atagacatat 43800
gcatgagtaa gatattaata tcccataaac caaaactgtg acttaggaaa tgttacggtg 43860

ggcacaggat attactcctc ttcaacaaaa acatcaatca tatgttggct tagttgcaca 43920
gcagaagtac caaaataaat ttatatagac ggtgtcacag atactttaaa aagacctact 43980
taagatttaa ttacctaata ttatttaata tttaatataa tttaaaaggg gcatttttat 44040
ctttcagcca aggttttaat tgtttttaga taattctatt tttgaaagtc tgattttgtt 44100
ttaaaatgcc cattctggta tataaaaaca tcattttgtc aaataggagt cccagctaag 44160
ggccaagcgt gcaattaaac ttgactcttt acctactacc taggaacctt gggcagatca 44220
catacttttt cttcactcag tttttacttc tgaaaaatgc cagctgaagt atgtttcaaa 44280
tcttaaattt tatgttttat gatctaagtt cagattaaca atggaatggt tatctggtcc 44340
tggacttttt gtgttgttgg taatttttaa attaccattt caatcttgct gcttgttatt 44400
ggcctgttca ggatttctaa ttcttcctga cttaagctag gaggtttgta tctttccagg 44460
aatttaccta tctcttctag gttttctagt ttatgtgtgt aaaggtgttc atagtagcct 44520
tgaatgatct tttgtatttc tgtggggttg gttgtaatat ctcctgtttg tttctaattg 44580
agcttatttg gaccttctct cttcttttct tggttaatct tgctaatggt ctatcaattt 44640
catttatctt ttcaaataac cagcttcttt ttgtttcatt tatcttttgt attttttttt 44700
caatttcctt tagctctgct ctgatcttgg tattttcttt cttctgctgg ctttgggttt 44760
cgtttgtttt tatttctgta gttccttgat ttgtgacctt agattgtcta tttgtgctct 44820
ttcagagttt ttgagtaggc atttaaggct gtgaactttc ctcttcgcat tgcttttgct 44880
gtatcccaga ggtttggatg gttgtgtcac tattattgtt caattcgaag aattttaaaa 44940
ttttcatctt gatttcattg ttgacccaat gatcattcag gagcaggtta tttcacttcc 45000
atgtatttgc atgttttgga aggttccttt tggagtcgat ttccagtttt attccactgt 45060
ggactgagag agtagttgac atagttttaa ttttcttagt ttttttgaca cttgtttgtg 45120
gcatatcata tggtctatcc tagagaaagt tgcatacgct gatgaataga atgtatattc 45180
tgtggttgtt gggtagaatg ttatgtaaag atctgttaag tccatttgtt ccagggtaca 45240
atttaaatcc attgtttctt tgttgacttt ctgtcttgat gacctgtcta gtattgtcag 45300
tggagtattg aagtccccca ctattattgt attgctgtct gtctcattac ttaggtgtag 45360
tagtaattgt tctatacatt tgggagttcc agtgttaggt gcatatatat ttaggattat 45420
ggtattttcc ttttggacaa ggccttttat cattatataa tgctcttctt tgtctttttt 45480
aactgctgtt gttttaaagt ttgttttgtc tgatataaga atagctactc ctacttgctt 45540
ttggtgtcca tttgcatgga atgtcttttt ccacaccctt accttaagtt tatgtgagtc 45600
cttatgtgtt aggtgagttt cttgaaggca gcagatactt ggttggtgat tgcttatcca 45660
ttctgcaatt ctgtatcttt taagtggagc atttaggcta tttaaattca atgttagtat 45720
tgagatgtga ggtactattc tgttcatcat gccatttgtt gcctgtatac cttggatttt 45780

ttttagtagt atttttgttt tatacctcca atgagattta cacattaagg aggttctgtt 45840
ttgatgtgtt tccagcattt gtttcaagat ttggagctcc ttttagcagt tcgtgtagtc 45900
atgtagtgct ggcttggtag tggcgaattc tcttagcgtt gtttttatct gaaaaagact 45960
gtatctgtcc ttcatttaag aagcttagtt ttgctggata caaaattctt ggctgctaat 46020
tgttttcttt aaagaagctg aagatagggc cccaatccct tctagtttat agggtttctg 46080
ctgagaaatc tgttaacttg atgggttttc ctttataggt tacctggtgc ttttgcctca 46140
cagctcttaa gattattttt cttatcttaa ctttagataa ccttatgaca atgtgcctag 46200
gcaatgatct ttttgtgatg aatttttcag gtgttatttg agcttcttgt atttggatgt 46260
ctaggtctct aataaggcta aggaagtttt cctcaattat ccccccagat atgttttcca 46320
aacttttaga tttctcttct ttctcaggaa caccaattat tcttaggttt ggttgtttaa 46380
ttctgtccca aacttcttgg aggttttgtt catttttttt ttttttctt ttcttttttt 46440
tttttaattg atcattcttg ggtgtttctc gcagaggggg atttggcagg atcacaggac 46500
aatagtggag ggaaggtcag cagataaaca agtgcacaaa ggtctctggt tttcctaggc 46560
agaggaccct gcggccttct gcagtttttg tgtccctggg tacttgagat tagggagtgg 46620
tgatgactct taacgagcat gctgccttca agcatctgtt taacaaagca catcttgcac 46680
cgcccttaat ccattcaacc ctgagtggat acaccacatg tttcagagag cacagggttg 46740
ggggtaaggt cacagatcaa caggatccca aggcagaaga atttttctta gtacagaaca 46800
aaatgaaaag tctcccacgt ctacctcttt ctacacagac acggcaacca tccgatttct 46860
caatcttttc cccacctttc cccccttct attccacaaa actgccattg tcatcatggc 46920
ccgttctcaa tgagctgttg ggcacacctc ccagacgggg tggtggccgg gcagagcggc 46980
tcctcacttc ccagtagggg cggccgggca gaggcgcccc tcacctcccg gacggggcgg 47040
ctggccgggc ggggggctga accccccacct ccctcccgga cggggcggct ggccgggtgg 47100
ggggctgacc cccccacctc cctcccggac ggggcggctg gccgggcggg gggctgaccc 47160
ccccgcctcc ctcccggacg gggcggctgg ccgggcagag gggctcctca cttcccagta 47220
ggggcagctg ggcagaggcg cccctcacct cccggactgg gcagctggcc aggcgggggg 47280
ctgaaccccc acctccctcc cggacggggc ggctggccgg gcggggggct gacccccccca 47340
cctccctccc ggacggggcg gctggccagg cggggggctg accccccccac ctccttcccg 47400
gacggggcgg ctggccgggc agaggggctc ctcacttccc agtaggggcg gctgggcaga 47460
ggcgcccctc acctcccgga ctgggcagct ggccaggcgg ggggctgacc cccccacctc 47520
cctcccggac ggggcggctg gccgggcggg gggctgaccc ccccacctcc ctcccggacg 47580
gggcggctgg ccgggcgggg agctgacccc cccacctccc tcccggacgg ggtggctgcc 47640

gggcggagac gctcctcact tcccagacgg ggtggctgcc gggctgaggg gctcctcact 47700
tctcagacag ggcggttgcc aggcagaggg tctcctcact tctcagacgg ggcggcccgg 47760
cagagacgct cctcacatcc cagacggggc ggcagggcag aggcgctccc cacatctcag 47820
acgatgggcg gcagggcaga gacgctcctc acttcctaga tgggatggcg gccgggaaga 47880
ggcgctcctc acttcctaga tgggatggcg gctgggcaga gacactcctc actttccaga 47940
ctgggcagcc aggcagaggg gctcctcaca tcccagacga tggcggccag gcagagacgc 48000
tcctcacttc ccagacgggg tggccccggg cagaggctgc aatctcggca ctttgggagg 48060
ccaaggcagg ctgctgggag gtggaggttg tagcgagctg agatcacgcc actgcactcc 48120
agcctgggca ccattgagca ctgagtgcgg ttttgttcat tttttaaatt ctttttttctt 48180
tgtctttgtt ggattgagtt aatttgaaaa ccttgtcttt gagctctgaa gttctttctt 48240
atgcttgttt tattctattg ctgagacttt caagaacatt ttgcatttct ctaagtgtgt 48300
ccttcatttc ctgaagttgt gattgttttt tatttatact aactatttca ctgaagattt 48360
ctcccctcaa tatagatatt tatcttggga agttctcagt gtaacatctt tactgtgttt 48420
ttctctttag attatacttt tatgtttatt attttgtttc gtgggggatt caaaaatatg 48480
cattttatgc catacttggg gattccctat aaattattag ttgtaatatg acagttccat 48540
catgaatttt ccaggtattc ttttatgcaa gcagataata gctcattttg gtttttaaca 48600
cattcatgca tattctttct ctctcaataa atatggtttt aatttattaa atgaaagatt 48660
taaaaatgtg ctaagcattt taataataat cgattttgga ttaacttgtt atgtttactc 48720
tagggctgta gttcaccatt tattcagtta agtccttccc caaattcaat attgaacatg 48780
tggaattgat tcgtttcacg tggatgtatc atttactccc aagatgttgg tttttggcat 48840
ttagtactgg taatgggcca ggaaagtgct catctatatt tgttgttatt cactgattgc 48900
atcttgccct tgcacccact gagacgatgg gaaagtagca acaatactag gtgatttctt 48960
tgatttaaac ccaattaaaa gaattagaga gttgtctgat acaaggccaa agaactaaga 49020
acagaaacaa aagcaaaaca acaaacagca gcacaaactc cagtgagata aatttttaaa 49080
acattgggaa tatttaaaaa ataaaaacac tccaatgaac cacccaggtt ttattaaaga 49140
gtagagaact caaacagcag aaggcagagc tggtggagga aactcagcaa ctgctcagaa 49200
atgaaacaac ctaggaagga ggtgcgagtg acctgaaact ccttttaaaa acagaaagga 49260
caaaaagagg tatgggctga caaaaggaaa gtggtagatt actgatgtat tgcattatgc 49320
ttagaatgtc tcagaatgcg agaggcagta aacacaccag aagaggatac aatatccgga 49380
ccatgtgcaa ctgcaaataa atgtttgggt taaaacttgg ttgattctaa attacatgaa 49440
gagctgatga taattgaggc agaactgata gacctaacaa tagaaaaaaa tcgatttaaa 49500
ttcagagaag aggtcattta agaaagtata atgaagccac ttaataaaag gaaatattcc 49560

tctaactaaa gtttgagatt tagggcataa tcctcaaaga caggctaata taatctattt 49620
tttgattaaa aaaaggaact tttggtttaa agtaagatgt tcagctgctc ttagagttta 49680
tttctccata tttgggcata taagaattta aggaaaaata tagaaatata agaaagattt 49740
catgagaatc acaagcatag tttataggca agatagcctt ttctgtttga aagcgaaaaa 49800
tactatttca acatgtaaaa acctaagtca gttttcactg gcatgtccca caatcatgcc 49860
tgaaataatg gttgaagaca gatgtgagac atttcaaggg caatagatta atacatgaaa 49920
cccacttatg cctagcgttc cattattgga acgctaagca tgtgggagtt atttatatcc 49980
tattgctcaa ggtcatctcc aaggtctgag ttttcactca tgcaaaaatt caaaaaattg 50040
caacctcggc gtaaatgggt taacaaaaag ttaatgctgg acagtaaaat aaactactaa 50100
attagacaca ccatattttt taaattataa gagattaaga actatgagat atttaaaaag 50160
ccacccacag aagtagtagg acacgtagag aaggataaat tctaacaatc aactgtatct 50220
ccacccccacc ttgtaaatga caaattagtt tacttagtca attcaggaaa ttaaacgtat 50280
acattttgtg ttaaaacaga agactttttt taaaaaagtg tgttagttaa ggttaatttt 50340
gacctagtta agaaaaagca gtttagagtc ttgaaatgga aatgagaaat acataattac 50400
ccttaaaaat aagttgatag tgaattttat ctaggattct taagacattt ttaatattta 50460
atgaaaatga actacacaat tattaaaaaa tagttgcctg ggtttagaaa aatgatccct 50520
taataagaca tatacaacag cctgaatttt tatttccaaa tactatagca acaaaatacc 50580
ggcaagaaaa atgcacagaa acattggata gtctcttctt actaatcttt tacagatatt 50640
tatatattct gaattctaac ttttaattta ttttatgtat tataaatgtc atctagtatg 50700
cagcttgact ttgctttatg gtatcatctg tttcacagaa aattttaagt tgtggaattt 50760
atcgtatttt ttctttatag tttgtgcttt aagacctgtc taataaatct ttccttccct 50820
ggacgctata aagttatttg tctatattct gttaaaagga ataaaacttt gctttttttc 50880
acattttgtt acttatttca cctgaaagag aattttgttt attatgtgag gtaggaatat 50940
aacttaattt ttttccattg aataaccaaa ttgtcccaga gcaattaagc aatacattct 51000
ttccctgttg atctgtgaag ctacctccat taggcatgaa gttgtcctgt tgaagagaat 51060
ctgattctga gctctcttca ctatttcttt atttgtctat gtctacacta aaagcaattt 51120
aatttctgtg tctttataat tactttgact ttataatcct tgaaagggac tttgttcttc 51180
ttcaaaattg ttttggctat ttatggctac ttttgctttc aaatgagttt ttaaatcaaa 51240
ctttaatgag attgcattaa acttataaat taatctgagg aggattgaca tcttcatgat 51300
atttagtctt cctacccatt aacattatat aactctccat ttattttagg tctttttgaa 51360
tgacttctaa taaagttttt caattttctc caaaatatct tacatattaa aatttactca 51420

tagctgtctc atcttactga tattttaaag acatatcttt ttaaaaatta tattattaaa 51480
ttgtttcagg tatgtctttt tttttgaagt tttacaaatt tgggattata ctgcctcttc 51540
attcaacatt ttcccatgct gttggggatc cttcaagtca aaattattag aggagctgca 51600
ttaatattcc agcatggaca tactataatt tatttaatta tttcccgaat tgtatgtttt 51660
tgtttctaac ttcacagttt tcaacatgac tgggaacagc ttttcttgct aaatccttgc 51720
acgccacagt aattatctca ctgggccaaa agataggtac tttttcatga ctttcgccat 51780
atattgtcaa aatggaacca atggtgattt gttcagaaat catggtacat catataaaaa 51840
gattactctg caagcatcac aaactgtgat atatagaaat gtgtttattg acatgaaata 51900
gatcatgaag aaagtgatta caaatggtat ttaagcattt taagttatat aggcttatac 51960
tgagcaataa caaagagggt gaataaatga atgaatatgt gattctggaa gggtatgtaa 52020
caaaaggtta acaatggtta gccctgggta aggggattat gtatgacttt tatttccttc 52080
tttttttctt cctttcccct tatgttttta acaatgtaca tgaatcgttt atgaataaaa 52140
taaaaaactt tgatattctt atataggttt agtaggataa catttctcct tcatttcttt 52200
catttttaga aatctttctt cacggaaatt cttttttta aatgtttatt tctgtctttg 52260
aggagtgaga ttgactggct atcttttaat acctgtgtgt attatcatta ttgtatcttg 52320
ctgataaatt tcacaacagt taattatttt attccccatt tcatacttag ctctatttta 52380
ggcaagttca caaatatcat tgaatgagtt ggggcaatag gtacctcctt tggaataagc 52440
atggtgagaa agaattgctt ctggcagatt agtgagaaga gtgagatatt ttctttcgat 52500
gacctctcta ttaaaatttg agtggtaaaa ctttgtgatg attcaggctc ttcataattc 52560
ttaattttat catcctctaa taccaggact tgcccagatc aatattttag gaaacagtca 52620
tgcttgctaa acccagggat ttctattaac cactagataa gacataagtt tgtcatgcaa 52680
caagtattta ttgagtccat aatttgccca gcactgggct ttggtactct gggccttgat 52740
actctaggtt gtgtcagaga tgtctaatat atttaaggtg acagagttca caaatgagac 52800
tttatgaaaa tgaatatccc tttttaagtc tcgagaaata tgatgtgcac ccgcttgagg 52860
ctttattaaa tctccagcga gcatgaactt gtttgtgacc caattgtaga attgttgtat 52920
gatgactatc ccactggcag gcacttcttg tccaagagaa tgtaattgga tgttggtgac 52980
tcaagcaatc ctgggaagac tcctccatga ccaaattaaa gacaacaggg gcttggtttg 53040
tactcagctc tacaccaatc agcatgagac aaagaagaga tccatggcaa agtggggaga 53100
ctactgttta ttttacaagc tagagaagga aaactgcagt tcctgagttg caaaactgct 53160
aagaaatggg agaacacaga actttacagg ctgaaccttg gtgtcttagt tattcctctt 53220
ggctacagaa tgccaaccag taggagattc atcactgaga tattacaggg aaatgaagcc 53280
aggcaagaaa aatatgtatt ccctttcttc tcagcccata aattttgtta ttaataaatc 53340

agactcttat agaacagctt gccacggctg tctctagaaa tgtttttatt agcagaattt 53400
ttattaaaat aaaatacaca gtagttttaa gagtgaacat tatagtttta ggtcataagg 53460
ggtgtcatgg agaacacatg tggatgctac ttgccagtta cttgatctag gccttgactc 53520
ttggttttct gttgctcgga taagttagca tgatttgaca tgcagttaaa ggtgtggtaa 53580
cctgtgattg atttcccatt cttgatgctc cctgcctgga cttctcggaa aatttcaaaa 53640
caattcattc ttctatgagg gctgcctcct tggttgcttc ggctaaacag tgttgagcaa 53700
caagttggga aaggaacgtt gcagtaactt ttaaaaatta agttagaaac aaatgtcatc 53760
aaagtaaatg ataatttgcc aatatgattg agcaaaaaga tgaagatgca cgctccctac 53820
gtatttgctt tgcagaagaa ttaatttgaa aaataataac atattttaaa atgaattgta 53880
aatataaaca catgctaatt gcagagggga atccctgtga ttattcagca gtttgtgttt 53940
gtcaagtgct tttcagtcag tccattccag ctcagcaggg cagaggcttg ggctgttgta 54000
aacttgtggg cagataccca gtgatggggc agacatgagc aggtagggct gacagcatgt 54060
gaattgagtt ttcttcagtc atgctgttgc agctgctccc ttccctcatt gctgagtttg 54120
ccacagcagg taggaaccta actctggagc ctgggatgaa ggagaaccca cattgggctt 54180
gaggaacaag atctgccacc ctggtaggcc ctggttaaat ctcatgcaag tgtagcaatg 54240
agagagggta tgattgagtt ctaatttagg aggaaagggg ataatgtgcc ctttgcaccc 54300
ccaggagaaa tcattgctca tctgtgccta agtagactta tcaagggcag ttggttcaca 54360
atggtgtatc accccaaagg actatagtgt tatgagaact tgccagtgta tttgaatttg 54420
ggtgctggca gatgacatca tgaggtatta tggttaccca taaatatgct agtttattga 54480
gaaggtggta gacatgctag tggatgagag ggaaggacag aagcagttag taaacagcat 54540
ctgcaacaat tcagttaact ggtggttgtc acagtagcat ggtggaaaag ttggcaatta 54600
ataacttcta agaaaactga actaatgaac caatcctgcg tgtgctatgt gtataacctc 54660
cttctcacta ttaacagatt tgttccaaac ttatataaga caatgaaaat aaagcttggc 54720
aatataggga agggatggag ggataaagct gtaaatcacg tcacaggcaa attaagatat 54780
accactggat caagggattt aatgcagaaa gactgatcct aacttatttc ttttatttag 54840
caataagatt tgttacttac attgattatt taaaatgagt tgcattatta gaaaggacta 54900
ttttgaagac aactataata aaatgtcagt aactgatagt agccaagata ttttaaatat 54960
atcaaagttg tgtcattaat attaatgtgt cccttaatat gaagtcctgc ccaggcttat 55020
ttatgtattc aacagacaca tacctgttga agtgtaacag atattctgga cacaagcaat 55080
ggtaaacaag acagatgcag tccctgcttt catgcaattt gcaattgaat ggtctttgac 55140
attttattgt gattgtttta gttatttaat tggagaagtt tttaatttaa atttgtgtta 55200

tattcagtgt taaggaacaa aaatgtaatg tgcatttctg agactcagta acacttctgg 55260
tttttccttt ttcatttaaa gaaaaattta gtgcccaaga taagctagaa tttttggaat 55320
caagtaattg atgacctggg agccaatttt attacaatag tgtttttagt ggtcttagaa 55380
cttttcagag gtggtagctc tgaaaataac actgtaataa attcacacat acatctatca 55440
tccaataaat gttaattgag gcccactaca gcattgtgtt agattctgag gttacaaatt 55500
gttgacccca tgtcgaacat gttgacccca tcaatgtaat cagtttgaca ttacacagtc 55560
atttaaacat taaagtgtca gcggatattt tagttgtaat tttgataagt gctctgaagg 55620
agaaaacagt gggtgttgtg aaaagcagta tttggttgat tgattattat agaggatctg 55680
agaaaactta cttgaggaag gaacatttgg ctgaactcaa agagatgagt aggagttaag 55740
taagcaagga agaaaagaag acacatgaag gaggaagaat gttctagaaa cacacacaca 55800
caggtatatg tatatgtaca tgtatatgta tatgcatatg tcttccttag aaacatataa 55860
acagctgcaa catgattgaa cttatttacc caattaccaa agcttatttt gcaccatgaa 55920
ggtggagata aaggcttttt aagcagcaag gatagaatct ttgtagtttt tatttatagg 55980
ctggttcttc tgacaacttt aatttttcat ctttaccaac ttcatggtct tcagtacata 56040
caatgcaatc attattataa aattatattt tgactcaaac tctaaggtag gatgattcag 56100
ctgtgcgcca tcaacatagc agcatgaatg gtagagacta gtcattccaa acagtgaagg 56160
ggcaacgtaa aactaatttt aatattatat gaaagtactt cttgcccttg actgcttttt 56220
tttttttttg aagaaagcaa actttaaaaa tttattttag atttacagaa ttattgcaag 56280
gatagtaaga gagttctcat atatgcctca cccagtttcc tctattatcg acatcttaca 56340
ttatatggta catctatcat aactaatgaa ccaatattgt ttcattatta gtaactaaat 56400
ctatacttta ttcagatttt ctaagttttc ctctaatgtt ctttttttcct ctccccactc 56460
ccactctgtt ccctcacgta ggccccagtg tctcttgttc ccctctttat gcccattggt 56520
tctcattatt tatctctcac ttaaaagtga gaacatgcag tatttggttt tccactcctg 56580
cattagtttg ctaaggataa tgtcctccag ctccatcctt gttcctgtac aggacatgct 56640
ctcgtgtttt ttttctttct ttttatttta atggctgaat agtattccac ggtgtctatg 56700
tactacattg ttttttttta aaccctgcat accattgatg ggcatttagg ttgattccat 56760
gtttttgcta ttgtgaatgg tgttgcaatg aacctacatg tgcatgtgtc tttatggtag 56820
aacaatttat attccactgg gcatataccc aggaatggga ttgctgggtt gaatggtaat 56880
tctcctttta ggtctttgag ggatttccac actgctttcc acaatgggtg aactaattta 56940
cactcccacc agcagtgtat aagtcttccc ttttctccat aacctcccca gcatctggtt 57000
tttttgtttt gtttgtttgt ttttttagta tttaataata gccattctga ctggtgtgag 57060
atgatatctc atcatggctt taatttacat ttctctaatg attagtgata tgtagcattt 57120

tttcatttgt tgccaactgt atgtattttt caatgaggtg tacatcagat cttttgccca 57180
ttttaaaagt ggggttttgg gctgggcgca gtggctcacg cctgtaatcc cagcactttg 57240
ggaagctgag gcaggcagat cacctgaggt caggagttcg agaccattct ggccaacatg 57300
gtgaaaccct gtctctacta aaaatacaaa aattagtcgg acatggtgtc gggcacttgt 57360
aatcccagct acttgggagg ctgaggcagg agaatcactg gaacccagga ggtggaggtt 57420
gcagtcagct gagactgaac cattgcactc cagtctgggc aacaagaatg aaactccatc 57480
tcaaaataca tacatacata catacgtaca tacataaaat tgggtttttg ttttcttttt 57540
gttgagtttg aggagttttt ttgtatattt tgattacaag tcttttatca gccatgtgtt 57600
tcacaaataa tttctcccag tttgtggctt atcttttcac tctcttaatt gtttttttca 57660
aagtagaaat ttaaatttta atgaagccca atttattaat ttttttcttc catattgtgc 57720
tattggtgtt gtatataaaa acttactacc aaattcaata tcatatagaa ttttttctgt 57780
tttcttcaag aagtagtttt ataattttgc attatatgtt tagatcaatg attcacctta 57840
agttttgttt aaggtgtaag gtttgtgtat aagtttttct ttttccacat caatgtccag 57900
ttgcttcagc aacattttct ttttatatat atcttaaggg taatcagcgc aatattttct 57960
gaaaagatga ccttttttctc atttaattgg ctcttctttg tcaaagatca gttgacctta 58020
tttgtgtgga tctatttctg gacttcttac tctgtttcac tagtccatct gtttatactt 58080
taaccagtac catactgcct ttattactgt agcctttatg gtaagtcttg aaatgaaata 58140
gtgcaagtgc tccaccttct tcagaatttt gttcttcttc agtattaaag gctattctag 58200
gtcttttgcc cttctttaaa catgttggaa tcaattgtca atatctacaa aatagattat 58260
tgggatttgg atttagatta ctctgaatct gttaattaag ttgggaagaa ttgacatttt 58320
atcaatattg aataatatga acatgtaata atattgaact ttcaatctct attatctctt 58380
catcatttta agatcttctt tcatttttca ttgttttata gatttttaca tataggcctt 58440
gtacatactt tgttaattta tatcttagta ttgaatgtac tattataaat ggtattttct 58500
aaactttgaa ttcctgttat tcatgatgat atgtaggaaa gaaattgact tttgtatatt 58560
gacattagat cctttaaccg tggcatcatt acttattagt tccaggggag attttgttgt 58620
tgttgttgat tcattggaat tttctgcata gataatcatg ccatctgtga ataaagatgt 58680
tttatttctt ccttcccaat ctatatatct tttatttctt tttttgcctt attgcacttg 58740
ctggtatttc tagcataatg tataatagga ggaatgagat aagatatctt agaattatcc 58800
tcatcttcag gggaaagtgg ttagtttttt gtcattaaga ataatgttag ctattgtttt 58860
tttaaatttc ctatatgaaa ttgaggaaat ttctgtctat tctgaatttg ctgagttttt 58920
aatcataaat agctgttgaa ttttgtcaaa tagttttcct gtgtcaatta atatgatcat 58980

atgacttttc ccgttttcac tgttaatgtg gcagattata ttgatttatt ttcaaatgtt 59040
gaatttgcca tcagacatgg aataaatccc atttgttcat gatgtataat ttattttatg 59100
catcgtttgt tctgtcttgc taacattttg ttgagatttt gtgccagtgc tcaggagaga 59160
tattggtctc tagttttact ttcttataat atctttatct gatttgggta ttaggataat 59220
tctagactca gaatgagtta ggatgtgttt tctctgcctg tttactaaca cagattgtag 59280
agaattggca caatttcttt cttgaagatt tgttagaagt aatcttgcca ccacctgagc 59340
cagatgattt ctttagaagg taattagtta ttgaatcaat atatttaata tatatagaga 59400
tatttaggct atttatttct ccatgtgtga gttttggtag tttgtgtatt tcaaggaatt 59460
ggtccatttc atccaaatta tcaaattcgt gagcatagag ttgttcataa tattccttta 59520
ttatcctttt aatctccaag agaccagtcg tggtgacttc tctttcattt atgatattgg 59580
taatttatgt tttctgtctc tctttttttt ttgccagagt ctaactctgc cacccaggct 59640
ggagtgcaat ggtgtgatct ctgctcactg caacctctgc ctcttgggtt caagtgattc 59700
tcatgtgtca gcctcccgag tagctggtat tacaggcatg ctccaataca cttggctaat 59760
tttttttttt gtatttttag tagagatgaa gttttaccat gctggccagg ttggtcttga 59820
actcctggcc tcaagtgctc tgcctgcctc ggcctcccaa agtgctagga ttacaggcgt 59880
gagccaccgt gcccggcttc tttttcttaa ttagcctgaa tagaagttta tcaattttat 59940
tgctctttta aaataaccag tttttgtttc actgagtttc tttatcatgt ttctgttttc 60000
aattttattg gcatctgctc taatttcaga tgctccttga cttatgatgg ggttgtgtcc 60060
cagtacatcc actgtaattt gaaaatatca taagtctttt gacttatgta atgcatctaa 60120
cctaccaaac attatcgctt agcctaacct cccttaaatg tgctcagaac acatacatta 60180
gcctacagtt gagcaaaatg atctggcaac aaaacacact atagagtatt gatggtttac 60240
cccgatgatc acatagctga ctgagagctg cggcttgctg ctgctgccca gcattaagtg 60300
agagtattgt tccatatatt gctagcacag aagatctaaa ttgaaaattc aaaatacagt 60360
ttctactgaa tgcatgcata ttacttttgc accattgtga agtcaaaaaa aataataaat 60420
caaaccatct taagttggga actgtctata ttattctttc tcttctgctt gctttaagct 60480
tatcatccag tttcagcttt ctacatatgg ctagccagtt ttcccagcac catttattaa 60540
atagggaatc ctttccctgt tgcttgtttt tctcaggttt gtcaaagatc agatagttgt 60600
aggtatgcgg cgttatttct gagggctctg ttctgttcca ttgatctata tctctgtttt 60660
ggtaccagta ccatgctgtt ttggttactg tagccttgta gtatagtttg aagtcaggta 60720
gtgtgatgcc tccagctttg ttcttttggc ttaggattga cttggcgatg tgggctcttt 60780
tttggttcca tatgaacttt aaagtagttt tttccaattc tgtgaagaaa gatattggta 60840
gcttgatggg gatggcattg aatctgtaaa ttaccttggg cagtatggcc attttcacga 60900

tattgattct tcctacccat gagcatggaa tgttcttccg tttgtttgta tcctctttta 60960
tttcattgag cagtggtttg tagttctcct tgaagaggtc cttcacatcc cttgtaagtt 61020
gtattcctag gtattttatt ctctttgaag caattgtgaa tgggagttca ctcatgattt 61080
ggctctctgt ttgtctgttg ttggtgtata agaatgcttg tgatttttgt acattgattt 61140
tgtatcctga gactttgctg aagttgctta tcagctgaag gagattttgg gctgagacaa 61200
tggggttttc tagatataca atcatgtcgt ctgcaaacag ggacaatttg acttcctctt 61260
ttcctaattg aatacccttt atttccttct cctgcctaat tgccctggcc agaacttcca 61320
acactatgtt gaataggagc ggtgagagag ggcatccctg tcttgtgcca gttttcaaag 61380
ggaatgcttc cagtttttgc ccattcagta tgatattggc tgtgggtttg tcatagatag 61440
ctcttattat tttgacatac gtcccatcaa tacctaattt attgggagtt tttagcatga 61500
agagttgttg aattttgtca aaggcttttt ctgcatctat tgagataatc atgtggtttt 61560
tgtctttgta gagccctcaa attcctaatc acaattgttt atctctttcc tgacattcca 61620
catccaatcc atcagcatgt cttattggct ttactttcaa aataaattaa actcagccac 61680
ttctcagcat ttttaaaatc accctaatac aaaccccctg ctacctcatc aactgcaatg 61740
gcttcctaac ttattttgta actttttgatc tttgagttct tccaagagcc aagagttctt 61800
ccaaagctat aaaccctatc attggcactc ctctgctcta tggaaagcag tggcttctca 61860
tctctttcag agtataatgc aaagctctca ccttagctgg catggccctg tgggattggc 61920
ctcccttgtc ttactttttc cttgttcagg ctgctgtgac ctctctggtc tttggctctt 61980
actagaaacc tttgaacccg tttccttccc agtgtcagta tttgtgtgtt gttcgttcac 62040
ccttctcact tcattctggt ttctacagca cagagaagta gtagtccctg atctaaacac 62100
cctctccacc tgctttccac tttgtttttt cccctagcac ttaccattat cagatatcat 62160
atatttattt gtttattgtc taacttcctc acaaaaatat gacgttgtga ggatagggat 62220
ttggcttttt gccccagagc agtgcctgac tctcaataac tttgttgtat taagtgaatg 62280
aataaaataa aattaaaatt gattcaaagt gtatgagcat gtgtacattt tacataagtg 62340
atacatgaca ttcttccatt ccttgggggc tcttttaacc attctcaacc agttgtagta 62400
cctttaaaaa atcatgatga agatgatttt gagagctaat tttggtagga gacagcagtt 62460
ttagcctgtc cgctccatgt gcagaataat agcctatttt attacatctg atattcaagc 62520
actagaatct atcaataggt aaataatttc caaaaataaa agcatcagtg ggaaaacagg 62580
gaaattattt ttaaaaaaga atttctagac caggatgtgt gcaatttgta atctctaata 62640
agaaacgtta tagctgataa ttccagcaat tacagaatca tgatcatatc cataatggat 62700
cagtagaggc tctggcttat ataatagctt gccctctcag gattctagag ccccatatgt 62760

aaacacagaa cacatttata ttgattgaca gtgcatgtag gtttctacag attgtgccag 62820

tcagtgtttt ctaaggcagt tatgccatgg taattaaaat tatgggctct gaaatcagcc 62880

tgcctgggtt caaaccccag cttcatgtgt cagcttcctt ggctgtaata aggaatacta 62940

atagcacctg ccttgtggct ttgaaaatta catgatgtaa ctttgtcaag agcttagatg 63000

agcacttggg atatggcgag tgctcaataa atgttagttt accatcatca tcatcattat 63060

tattattact gattgcaagc aagacaaact ggttctcact tcagcagaaa aagaaattac 63120

tgaagtaatt ttggttagat cacagattta acgtgaagga tgaataacca gacttggaaa 63180

acaggtggaa accaagaggc agtcagcatg gcatagcagc cagcacttca ccagtgtggt 63240

gccttggggg cagcctggca ggagccactg ccttcactcc tggactgcag atctaccaca 63300

ggacagcaga ctgaattgtc ctatgtccag acttcacagt cacagggagc aggctgcatg 63360

caggtggggc ccaggtcacc taccttcacc ctagagtctg gagccacaga aaacagtaat 63420

tgtccttgta gcttttgtga tggaaagcaa gtcctgacac ccaccaactc acatactagg 63480

gaattcacca aatgtaggac ggcagctaag atgctgggaa accaagaatt aacaaatgag 63540

tattacacca attagttaat tcattacaga tttcaatatt ttccaaaaaa catcctacaa 63600

agaacagctc actttaatat actccaacaa atgatgaaat ctctccttgg tcatcagctt 63660

ttctaaattt ctgtaacttt aggtgattct gaatttctta gtgattctga acttctagaa 63720

gattctgaaa cagaacagtc ttactttggg atgtattcaa atcctagaga tcagtcacat 63780

caatctgtgc ccttttttcat tatgtaacat gaaacaggag gacctttcca acttgccttg 63840

ggaacctgct cctacaacag ggtatattat cacatttaat aaagaaagag taatatttgc 63900

attgtgaccc ttcatctgag gtcatcggga ctgtgctgtc ctcccaagct ccactgtaaa 63960

gggatagaca acaaccttgt ctcccagtca ttgtaatctg tatcagagag catcattaga 64020

tgaatgaaga tggaaatgtt ctcacggtca gcaggtgggg aaggcaagac ttgctgattg 64080

gaagcaatga accatagttc attctaaggc ttggggagag caactttgag gaaatgaggc 64140

ccctaaactt gccatgtagg agaagttggg gggtggtaaa ttgggcaccc tttatctact 64200

aaacgtaatc tttacttccc caccctctaa tttttctcag ttgggcttga aattgttttt 64260

gcttgttaca ctttatggga aaagaaaggg aaatcctaat aaggtccatg ccagcaaaac 64320

tctagaagaa gaaactaaga atttcaaatt gacagtttgt taatgagaaa agacaaggtt 64380

aaaggatttt gttaaacaga tgaccaaaat aattattgga aacttctgct tctggccaag 64440

gtggaataat aagggctaca tttaccttcc caccagaaaa tataataaca aacaaaaact 64500

ggacaaaaca ctggacatga cacgttcatt aattcattca acgaatattg aataactttt 64560

gatcaatgtc ttcctccctc caagctgaaa acttgatgga gggtaacatg atctgtcttt 64620

gtttaccact gcatcttccg tgaatataca ttgtaagtat tgaacggatt tttactaaat 64680

gaacaaatac tttgataaaa tatttttgat tactgccaat atcagtttct gaattgattc 64740
taaaattctt ctgctggaaa gtagattagc gttagtatga ctgctgaagc cttttaaagt 64800
gggtggtaat actacgtttc gttttgtttc attaacctaa gtgctgttct ttggaatctt 64860
tttaaagtaa gataaattac attcatgaaa gaagcatatt atttttaaag tactttattt 64920
tggaaaggta aaatgcttgt gtagttataa tttggttact cttgatttca ccttaggaaa 64980
aacaatatca ccttctaacc atttcttttt tagtcaaatc tcttgcttct atttctctct 65040
gtagatccgc tattaaagac tgtaatcact gctgcatctt tcctgtaagg cttgatcgca 65100
ttgttaattt ctttctaaac ttgtagagta ggtgtataaa tcgtatttgg gtaatacact 65160
gactaatact gaagaaccag gcattttctt accccagtcc tcacacgaag tagggaaata 65220
caagtcagaa cttatcttct caaaactctg acctcagaat ttcctgagaa atctgaacct 65280
aaaaagattc tttgcttttg acatttttc tctggtgtcc accgcaaggc tcttgctctt 65340
acattttttt tttttttcta atgtttcaaa tagaagatgg tagagtcata tagtacaagc 65400
tcagcatcgg aggggctact ggaggtcaac tagtgcaaat gctttctgaa taatggaatc 65460
ctttgggaat accccttga aagtcttcat ctagcctctt cttggaaacc ctcagtgata 65520
cccctcatcg tctcctatga tggcagcttc tatctttgtc gtcagctctg actattacaa 65580
actgcttcct tacattgagc tgtaactaac taaaaagttt ccacaccctg cttctatttt 65640
agcctttggg ctcataaaga acaagttgaa cccgtcttat gcagaacaac ccatctacgg 65700
gagatgctca ggtttcctcc atgttttcct ttctctaggc taacccttcg ttgttcctca 65760
cttactcact atataacctg atttcatgtc tccttaccct tcttgttact ctaacttgag 65820
taggctagtt tcaggatcca gcaataagtg tgatacatca ggtatgctct gatgagttga 65880
gagtagagtg gacatagcat ctcccttaat tcagatattg tgatctaatt ggcaaatctg 65940
gcaataaaag ttgaaatgcc tgatccagga caatggctgg tcaggtgcca ttgttctcat 66000
cttttacttt taggtgtccc tcaatttgtt aagttagtac ctacgtaatg tcctgaaact 66060
tgttaagttt gtacctacat aatggattaa attcaaaatc tctgtttaac tgaaaacaaa 66120
agcaaacttc ttttcagagc cagaatctgg aatcatacgt aacagagaat gatattgtac 66180
aagttgcttc atctctttaa gtaacggttt ctcaaactta caagattatt gtgagaacta 66240
aattagttct aaagtgcttc catgtaaagt gtatgtaaat gcttaaaata tatagtaagt 66300
gctgaatgca tattagaaat aataataatc tttattataa tttttactat ttcatgagaa 66360
gtacttattt taatactgag caaataggaa gagttcatgg cttctctatg tttttgaagt 66420
gtcatttaat atattatata tataaaacat atatatattt cagtcacaca tttgtccaaa 66480
taccttgaca aattaaaaca aaataagaca aaattctcac gctagttatt tgttataaag 66540

taatagaaca agtgatatgt tataaagagc atttatttct catgtctttg atattaaaaa 66600
tagttgtatt aactttttat caaaacgatt gcttccttca tataaatcta agaattatgc 66660
tgtctgataa atattggaga gattaacttc tttgaaaata tagaagcttt ttgtcttttt 66720
aaaatagttg tttttttgca gatgttaata catttcagca gtacagtatg gccttttca 66780
ggttaaggtg ctgagcccaa acctcaaaga atcactgcaa aaagattgga tcccccctct 66840
tcaccccatt tcgtaattta gttagtgaga accacaactg gctaaacctt tgtggggggc 66900
cgggcactgt ggctcatgcc tataatccca gcactttggg aggccgaggc aggcagatca 66960
caaggtcagg aaatcgagac catcctggct aacacggcga aaccccgtct ctactaaaaa 67020
tacaaaaaat tagccgggca tggtggcagg tgcctgtagt cccagctact caggaggctg 67080
aggcaggaga atggcgtgaa cctgggaggc ggggcttgca gtgggccgag atcccgccac 67140
tgcactccag cctgggtgac acagcgagac tccatcttaa aaaaaaaaca aaaaaaaaca 67200
aaaacaaaaa aaaaaccacc tttgggggga aattatcaaa taaaacaaac tctttttaga 67260
attttacaac ttttatgtta ggaaaaaaca aatacatttg tgaaaagctt aaaatccagt 67320
aaatgacttg agggacttgg ggcaatccta gggtgatgag gagcaggtta gtaacagtga 67380
aggacttagc accccagggg gccagaggct gtaatatacc ttatgagcaa gtcattctta 67440
tttagtcttg cccattaaga agtctacttg gactaaatgc ttttaaaaat gcccttttaa 67500
tttactatta aaagaatatt cctagcagaa gtagtcttgg atgctaaatt ctattttaag 67560
aataactaaa attagaattc tgttcttttt ataacacctg ttacacacac acccctacct 67620
agtgtgtcgg aatcagtttg tatgggctca ccaaagccta ctgttcaatt ttcaggagtt 67680
ttgtaagcca tttgatgtca gacaagtggc ctgaagtttg ttatggtggt ggtatttaca 67740
ccatgaaaat tggcatgtta tggtggtagt atttacacca tgaaaactgc tacaaataga 67800
aatctttttc ttcttctctt ggagagccac ttgttgaaca cttaccagct cacctgtgct 67860
tgaaagtatt tcttcaaata aaatgaaagc tcgttagctt tgaaaatttt ttgtataaaa 67920
gtttacacgg gaaaaaaata aactaatttt tttttccacc tgtgttttca gggatacgaa 67980
aagaccgaag aggagggaga atgttgaaac acaagcgcca gagagatgat ggggagggca 68040
ggggtgaagt ggggtctgct ggagacatga gagctgccaa cctttggcca agcccgctca 68100
tgatcaaacg ctctaagaag aacagcctgg ccttgtccct gacggccgac cagatggtca 68160
gtgccttgtt ggatgctgag cccccgatac tctattccga gtatgatcct accagaccct 68220
tcagtgaagc ttcgatgatg ggcttactga ccaacctggc agacagggag ctggttcaca 68280
tgatcaactg ggcgaagagg gtgccaggta agaatgcgaa gcgcagcttt taagagtcaa 68340
tagcttttca agaacttgtt gtgatgtcat gggagaaata gtgggggaaa aagaagcaat 68400
aacatgttat gtaattggtt tcaaggttac aggagatgtg ttcattttca gtatcaatac 68460

actgtaattt tccaggagat taggaaataa tatttttaaa tcagaatcta gaagactgaa 68520
attcttaaat tgacataatt tatttttaac ccatctcatt tcatggttgt gtgagttatt 68580
ccagggctca aggcctcttc tgtgtctgta ttccccagga atccaggaat tcacttctct 68640
gcctctcatc ctcatcctca tcattaaaat gagcagattt gagctcagga agtgcttgaa 68700
taaatgaata aatgaacaaa tgaaaggtga tttttgaaag tcttaatttt aagaagtctc 68760
tgagccctgt tacagctcaa ttttcccata aactagaact gctctctaag gctgtgacat 68820
ttctcctttt tcctctcctc aaaatctacc tgctgttctg atatctccct gacagccacc 68880
atagtgagat tctatttcca tttccaatct ctcttttgta agggacagag aaacagctag 68940
agaaatatgg agccatgcgc tctgaggact ttagcaggct tcaactctat ctgcaagtga 69000
gtttcactta gcgaatgaaa ttggaaactt aaagtgggta gggatgaggg ttcccggaga 69060
aggtgtaata cctcagtctg ggaattggga gcatctacaa ggaacacact caattctggg 69120
aggttcctgt agatttcaga gatttagcag ggcttcccag cactctgctt cccaacctgt 69180
cattggacca gtaaaccctg ctctcaaact gttgtggtgt ccagcatgct ttggcaaggt 69240
aatgaaagat aacatgacat ggacatatgg gtgacatcct ggagagatga cagaagcctc 69300
tgtttaagga caagatttgc catttagatt ttgcacccac tgtataataa gagccttagg 69360
attgggctgg aatcgcccta gcaggcatga tggcagcccc tctggactgg caatatgcag 69420
ctttttttgca agtgttccat gtccaagtca ccccacccag ctttctactg ctcccgtgga 69480
agctctggtg aaacgcaagg aaagcagctg ctgttgactg gatttttctt tcacatgaaa 69540
ctttgaagcc tcatagatgc ttattggcct ggatgctatt aaacctttaa aaatcccttt 69600
ctcatcttga gagtatttga gaaacatgtc tggggcattt tgcccaccct cctccaggtt 69660
ctgtgtcagt gagtgatatg gtttgcctgt gtccccaccc aaatttcatc ttgaattccc 69720
atctgttgta ggagagaccc atggcaggta gttgaatcat gggagcaggt ctttcccatg 69780
ttcctgtgat agtgagtaag tctcaagaga tctgatggtg ttaaaaaggg gagtttttct 69840
gcacaagctc ttcttctctt gtctgccacc atgtgagatg tgccttccac cttctgccat 69900
gattgtgagg cctccccagc cacaatggaa ctgtaagtcc attaagcctc tttcttttgt 69960
aaattgccta ctctgggatg tgtctttatg agcagtgtga aaacagacta atacagtgag 70020
tctgggtcag tgtgtgttta tgttgaacgt agtggacttg tggtgtcccc agggcaccct 70080
gtggggaatg ttggcctgtg gctttgctac ttccaggggg atttggcatg gagaatgtgt 70140
gttttaagta atagatagat tatgattgaa gtgtgttatg ggctgagttg tctctcctca 70200
aaaagacatg ttaaagtcct aaccccccagt atctcagaat ctgaccttct ttggaaatag 70260
tgcctttata taggtaatca acttcaagtg aagtcattag cacaagccct aatccaataa 70320

ggactggcat tctaatgaaa ggggaaattt agacatagaa acagacatgc acagaagaag 70380
atgatatgaa gagacacagg aagaggacgg tcatgtgact ggagtgctgg gtctgcaagc 70440
tgagaaatgc caaggtttac tggctaacat cggaaactag aaggggcaag agtggactct 70500
ccccctcaga gagagtatgg ccttgatttc agacttctag cctccagaac tgtgaggcgt 70560
acatttctgt tgtttttgaa gccacctagt ttttgatact ttgtatggga gccctaggaa 70620
attaatacaa agtgtattaa aaatagaatt ttcttctatt gtatttttga ggcaaaagaa 70680
ataaggagct attgatttaa ggaggaaggt ttggctcatc tagtatagac ttggctaatc 70740
atttactctt gtatatttct cttctgcctg cccaagtagt tcactgaaca ggcttagaag 70800
ttgatttata attgtaagag tttacacatc agaagttaat ttataattgt taacagtttc 70860
atgcatctac ctcataaata ccttgtaaaa gattttaccc ttcagaataa tgttttcctc 70920
tggtacttta gtagtttatc cttttttttgc atctttgatg catttgaatt catcctggta 70980
tgctgtgtga gctggggctc caatgtcgtt ttcctacaga tggctccaca gttgtttcgg 71040
tttcattcga acagcctcct acagaagtat ttttcagact tcttttatac ttgacttcca 71100
agtttactac tggttaagag cttttaaagt aggggaaaag atatgaccca ttggcctact 71160
cgtttgatta ttttttccaa ttctgaaatc aaaaattaaa aaatttattc aaagttatag 71220
tttcacttaa tttatattaa ctgcatagat agtgtgattc atctgtcctg cttggacata 71280
tttagtaact tttaattaat cttagagata aaagatagaa atctatgaga cttgagacat 71340
tcaaataaga ccagtactga taagaggtaa tcaggtgaag tgccatttgg ttgtaatagg 71400
acatcaaaat tgttccccaa gagtgaagga tgttccattc tcacttttcc catctgtcct 71460
cccaaattct ttcactcttc ctgatgcttt gaaccaactt gaaaagttag tccactcaga 71520
acctcagctg ttaaactttt agcctctgca aacaaactag ccgttttcct ctttgtccct 71580
ttaccatcaa gctcagttgc ttctcctact cctctccctt ctgccagggg ctccaccctt 71640
gttcctcatt caacagtgga atcatctgtg tgcctgtctc ctctttactg tgtaccattg 71700
ggtcctcttc ttcaccatct ctaaatttat gcttcttccc tgttccactg tcaccagctt 71760
ggtctacatc ctactttgat catttggatt taggtcccac cctaatcgag tatgacttca 71820
tcttaacttg attgcatctg caaagacctg atttccaaat acgaccacaa gtaaaggttc 71880
aggtggacat gaatattagg agagacagca cttcaattaa cagtttggca ttaggacttt 71940
attatctagc ttagtaattg ttttaacaga taaataatag tatcccctaa agtttaaaat 72000
gcagaaccag attatgcctc agggtgccta acctgagagg aagagtcttt cttcaacaga 72060
ctctgggaaa gacctttgta aaaaggctgg tgaccattct atttgatttc tactttccaa 72120
tattaactgc ttaagtcaga caatctcctt ggcccacgct ttcccctgcc attctcccct 72180
tcacataatg tcttcatttt atttttccct tgttttattc cctgaactcc cttctctcta 72240

ttcagattcc ctgcccattt tttctcctaa tgagacgcct gacttcctac ctcctttttg 72300
aaacttcaaa ctgcttagca aatacaattt tgaattgagt ttgctgtcac agaacaaaaa 72360
ctagtccttg gtccagagta tcttgcagga ctgaatcaac tgtggtctaa gacacaataa 72420
taactcttaa ttattttctt gtttccttca tggtatattt gttgctggat gatgagttta 72480
tattatggtt gaatttcctt tttgaaatga aaagatgaat agtctttatt ggctgggtat 72540
ctatttttat ccagaagact ttgcaaatga gaaatatttc tacaatctga gtttaattta 72600
taaaattttt ttttttgaga tggagtctca ctctgttgcc caggctggag tgcagtggca 72660
tgatctcggc tcactgtaag ctccgcctcc tgggttcatg ccattcttct gcctcagcct 72720
cccgagtagc tgggactaca ggtgcccgcc accacgcccg gctaattttt tgtatttttt 72780
agtagagatg ggctttcact gtgttagcca ggatggtctc gatctcctga cctcgtgatc 72840
cgcccacctc ggcctcccaa agtgctggga ttacaggtgt gaaccaccac gtgcgaccca 72900
catgtctgta tattacagcc gttgaaggac acaacttttg ccattgggcg acccagtccc 72960
ctagtaagtg ttcaaaaata ttcgctatta tcattgttgg tgatttcgag gatttggata 73020
taattaattt gagcctaatt ttttaagact attacataac tatacaaaaa aatcatgaga 73080
gagcctggaa ttttgcaaaa ctaggataga aaccagtttc taaaaagcat tttaaacatt 73140
cagcttgtgt atttcttttc acttgtattt tgtgatctgc ctccatgctt tcatgccttt 73200
ctcttcaaac aatcttatta ccactaacca aaaacaatta aaatttgatt gttttgtttt 73260
tacatgttgt tacagttaag aaaaaaaaat tctaggtatt gacccctgcc agttttcgga 73320
gttaatacta actagtgatt tggggctaca tttaagtgct ttaagctatc ctacaagctt 73380
aaagtagtta tacatgcaaa tacttaaatg aattaggtac aagaatacag aggtaagaaa 73440
tagaatctat tgtagaataa atcctatttc agatgtgact gttttaggat ttgaatcaac 73500
cacatattac agaaaacccc aagataaagc cttggcataa ccaagacaaa gtttattttt 73560
ttctctcaca taaaattagt ccagacttat tttaccagtg gtatggcaac tccacagtca 73620
aagggaccca agctcatctg accttcgatt cccttatcct gggacaggct tacattctca 73680
aggtcgactc ctggcctaat ggggctactg gagcttcagc catcacactc tgtgcttcct 73740
gttatatcta ctggtcagaa cttcagatac atctgtctaa aaggaacgct gaaaatacag 73800
tctttaagct gtgcaattgt acctggaata aatgagttct tttaataaaa agagttgaat 73860
ggatatttgg tgggcaaata gcagtccatg ctacaataat aaacttcaca attttagaat 73920
tagatatctt gtaatgaata ttctgacgtg gtgtcaactt ataccataga tagattataa 73980
ctttgaaaag gaattagaac acaataataa taaattcaca ctaaggctct tttggggaaa 74040
aaaaatctct accatttatt gagtgtttat tatcttcaca acatccctat gagttaggga 74100

ttatttttgt ggacatttta cagataaaga aacagaggca ttgagatgat aactagcttg 74160
actaaggagt ggcggagctc tgggcagtgt ggttcctgtg tgcccataag gccattgcac 74220
aatgctgctt catcatgtaa ttcagaaagt attgcaggat gtggcagttg gtcatcatga 74280
atgttcattg ttatcttgtg gctttgtgaa aaattctggt cattcaaacg atttgactgt 74340
tgagattctg tgcacatgag attgtactgt gtacatgttt ctaaaaatgt gtgttataga 74400
tcaaaatgca cacactcatt tacctctaag aacagttctt atattgaagg gaaattatct 74460
gtacgtgaga gaacaacgtg gttttgagta caagtggtga acagacattg aatagttgtt 74520
ttcagaaagc atcactcccc tgtacctcag aacccagcct cagtgctggt tgcgagccaa 74580
cagctttgat gtcctgaagt ttttgcatca cttctctttt gcccctcttt gggattcaaa 74640
gatgataagt ttgaagtgga gttctaccgc ttccattgga agagaaaaag tttccgtgtg 74700
tgtgtgtgtg tgtgtgcgtg tgtgtgttta cggagaggag ttctatgcat ctgcagaggg 74760
tgctgccatc aacaaggaga acagagtggg atgaagggtt tgggaagcca ggatgggcat 74820
ctctgacgag ctccagaact ctccacctaa gtgtgcaagt gtaaacactc cctgatgtgt 74880
gaactggcca tctcaagact aagtatttag caggaaatgc cccctattca acccttgcct 74940
tctcaggtgt agaggttggg ttgtctcttc catctttgtt tgaattatgt cattaatcaa 75000
ccttagtgaa agatcactta gtcatttgtg acagcataag ttcttaattg ttaggaatca 75060
ctggtgcggc acacatcttt ttctatccat gaacaacgtc aaatgcttat tttctcatga 75120
gcttttattt tttcctttta aaaaaagttt cttaggataa acacagcctt tttccttgtc 75180
tctcccttgc cctctcatct tttctcaat ctttatattc ctatatgtca ctgaagagtc 75240
cccgtgccaa cgctgtgcag tgggaggctc ctacctccac cagcttttga ggaggttgta 75300
gtcctgcaac cttagaggtt ccacagccaa gctgggggtc tttctggagc atgggtggtg 75360
aatctgagat ctatgcaccc aggaagcctg acacattatt gtggtgtctc aattcttttt 75420
tttttaatta gaaaaattgt atcaaattgc atttggtgag agcaaaaata aactgaagtt 75480
ggttgagctt tggaagacta caagccactg taatatttaa gatttcttga cctccagaac 75540
taacatttgt cctgtcagag aaaataatta ctcctgttga gaatacatgc attaaagtaa 75600
gatgttcact actctatatg atcaccaaac attaaataat atgttttaca catgtatgta 75660
ctatgtgttc aagtgtttat aaatccatgg agccaataga atgtaagttc tatgagggca 75720
gaaattttaa tccattttgt tcctagaaca gttcctgaca catactggtg ttaggggtag 75780
gtcttcagat tttatggatc aaatggaatc tccacactta aaaaaactta gagaaaaact 75840
gccttaatgt gcccatagtc tggttggaaa tggcagctcc aggttcattg attgctttgt 75900
tcattcggca cttttcactg gacatgctat gagcccagca gtgttctggg tttttgggat 75960
acaccaagcc ctgcctcccc ggtgcttaac attctagtgg gggagacaga aagaaagcaa 76020

acatggtgga caacttaatt atctattttt gaaagttgac aggaactgtg gacaaaagaa 76080
aacaagagct ggagacagca gtgccagggc agggtgaggg tcaagttaga gtaagcctca 76140
ttgagagatc ttttgagcaa cacctgaagg aggaggtgag aaagttagcc atgtggaggg 76200
agcagcattc caggcaagtg gcccaccaag tgcagagtcc tgacagcaag agcagttctg 76260
gaatattcta gaaacaggaa gaagaccaat gtgactagag cagagtgagg caggagtgag 76320
agaaaatgtg atcaggaaag taaggtcctg tggccactaa gattttggct tttattctga 76380
aggaaatgag gactcattgc aggactttga gagcatgatc tgacttgtca caagtgttct 76440
ctttgtctac tgggttgaga agacatccaa agggggccaa agattgaggc agggagagca 76500
gctaggacag gctctagcaa tctaggtgat aaatgaggac cgatggtggc tctgacaagg 76560
gtggtaatgg agacatggtg aggagtgacc acatttcaaa tatatcttga ggtagagctg 76620
acaggatttt cccgatggtc acgcgatata tacaggcttg ttgaaaaaca tgaaaatgat 76680
aaagaagttt atgtattaag aggcaaaagc tatgatgttc aactcctcta aaaccacttc 76740
ttagtaggtg aacattgtta aacagttttg tgaatctgct tccagatttt ttctaggcat 76800
gttcatacac atatacacac ataatttatt tttacccagt tgcgatcatg tatcatctga 76860
tccaaaactc gctatttttc agttaatatg cagtgactgt ctttccaaac catagtatat 76920
atatatatct ctaagaattt ttcaaatctg ctatagtttt taaacatatt tattgttaca 76980
tacatatgtg tataagttac ttatttgctt atttctttga atatctttgt ctagctttgg 77040
aatcacataa ataatgagta caaatggtaa agaaatttca tgtggaagga tatatagaaa 77100
aagtacaagt ctctcttctc atccactcta tctcattacc taaaaggtta tccttagagc 77160
tttatataat acatccctga agtttagatc attttattgc ctcccaattg ttgagagtaa 77220
ggaatttagt acatatacct ttcctttcac tttcctaccc tctacctact taatgagttc 77280
gattatagtt tttcagttca cccactgtta aataatatac ctatatttat aatataccta 77340
tataatatac ctataataaa cctatatttt tgacttatca actttggaca tatctattgg 77400
tgacttgtga tgagagatga aataatttgt acatttaccc ttcttttcca ttcttttccc 77460
ctacccattc tccccttttt ggctaaatga ttgcttttag gttgataagg tttataatag 77520
ctactgttct gtggttataa aatttgtttc atattttatg tgaaatttga ttatacaact 77580
agcattcaaa acattattat gtaaatatta gttactgtaa tcacacagtg acgctcaagg 77640
aggtatttac taagcataca gatcattcct tctggatcca actcagcagt tgctttgctg 77700
tgccttacct catctttcgt ggattccttt tcttttgatt gcaatatatc cttgagtaat 77760
ttttcagaat cgatatgtac tgtctgaact ctcttatacc caaaaaattt gctttcacat 77820
ttgctaatag tttgacagag tatcaaattc tgctttcaaa ttctttcccc ttcatggctt 77880

tgaagatact gccccactat atattctaaa atccattgct gctaatgaga aattctggtg 77940

tcactctgac tcttatttct ttcatatcac ttggcatttt cttccgagaa aatattactt 78000

atcttcagaa tgatgaacct tctctatcat gtctctaggt gttagggctt ctttcattta 78060

atcctgtagg tcctcaatag gctttttaat taaagtctca ttttattcgg gctctgtgaa 78120

atttttgtt atgttttggc ttctttttct cttttgttgt ctctcttttc tccttgaatg 78180

tctttctgat agatatagca ttcccattat ctatcatgtc tttcagatta tcctacattt 78240

ttctctttgt tttctttttc taccatgttt agaattctct tacacgtaag acatcactta 78300

ctagatcttc agtcatatcc tttttattat tcagtctgtg catttttaat tttcaatttt 78360

ggcaaccaca tttttaattt ctaagaatct attttgctaa tagtactttt gtcctgaaac 78420

tttttatatc ctgcaatatt tagaaagagc acacccttat ccacagctga ttaggagtac 78480

actctgaatg cagggcacga tgggcgaaca ttttgttact ggcctcctca agcctcaatg 78540

cccagaagtt attgcctcca tcagaaaccc ttcttttgtt ctatccctca cttccaaatg 78600

aagttccagc ttaatttttg ctgtcactgt agtgggacaa gcaaatccct atttcacgtg 78660

atcattgctc aaacctctat tgacaatcct gctttgcatt cccttattta gagtgggatg 78720

gaaaataaag gctatgaagg gggaagaatc tgatcccacc attctccttt ttcattgcag 78780

ttttctcctt ttttatggat gcaataagct gttgcaatct ctcaggatgg aaaagaacat 78840

tttaaaacta ctctactgcc agaatgatct cttttttttta attatactgt aagttctggg 78900

atacatgtgc agaacatgca ggtttgttac ataggtatat acgtgccatg gtggtttgct 78960

gcacccacca acccatcatt tacattaggt atttctccta aatgctatcc ttcgcctagc 79020

ccctcacccc ttgacaggtc ccagtgtgtg atgtttccct ccctgtgtcc atgtgttctc 79080

attattcaac tccccccttat gagtgagaac atgtggtgtt tggttttctc ttcctgagtt 79140

agtttgctga gaatgatggt ttccaacttc atccatgtcc ctgcaaagga catgagctca 79200

tcctttttta tagctgcata gtattccatt gtgtatatgt gccacatttt ctttatccag 79260

tctgtcattg atgggcattt gggttggttc caagtctttg ctattgtgaa tagtgccaca 79320

ataaacatac gtgttcatgt gtctttatag tagaatgatt tataatcctt tgggtatata 79380

cccagtaatg ggattgctgg gtcaaatggt atttctggtt ctagatcctt gaagaatcac 79440

cacgctgtct tccacaatgg ttgactaatt tacactccca ccaacagtgt aaaggcattc 79500

ctatttctcc acatcctctc cagcatctgt tgtttcctga ctttttaatg attgccattc 79560

taactggcat aagatggtat gtcgttctgg ttttgatttg catttctcta atgatcaggg 79620

atgatcagct ttttttcata tgtttgttgg cctcacaaat gtcttctttt gagaagtgtc 79680

tgttcatatc cttcgcccac tttttgatgg ggttgttttt ttttcttgta aatttgttta 79740

agtaccaaaa acagatacat agactgatgg aacagaacag aggcctcaga aataacacca 79800

cacacctaca accatctgat ctttgacaaa ccggacaaaa acaagaaatg gggaaaggat 79860
tccctattta ataaatggtg ttgggaaaac tgactagcca tatgcagaaa actgaaactg 79920
gaccccttcc ttacacctta cacaaaaatt aactcaagat ggattaaaga cttaaatgta 79980
agacctaaaa ccataaaaac cctagaagaa aacctaggca gtacaattca ggacacaggc 80040
atgggcaaat acttcatgac taaaatacca aaaagcaatg gcaacaaaag tcaaaattga 80100
caaatgggat ctaattaaac taaagagctt ttgcacagca aaagaaacca tcatcagggt 80160
gaacaggcaa cctacagaat gggagaaaat ttttgcaatc tatccgtctg acaaatggct 80220
aatatccaga atgatctcta ttttattttt tatgacatat taatcatgtt tgttctcttg 80280
tgtcctctct ttctttctct cgattttcca gtggtccatg gtggtctatt tgtagggttc 80340
atatttacaa atgggagtca aggtgactcc actacagtta tgtgaaaaag tttccctgcc 80400
atgcctctcc ctacactggg cgagctgatc tggttccggg tcagtggatg gagcatttcc 80460
tctcgcaggc acaccttcag gctggtggga gcaacttggg ccatggactg gaaccatgtc 80520
aatggagaag acttcctctg tgccaggtcc tggtcagagc tgccgtcttg atttatttgt 80580
atccctcttt ccgtctgtgg taaagatctg gaggttctta agctctctct ggcctcaaaa 80640
cccacagcag gaaatttcct cactcagatc acccacgtgc ccacccagat tgagggcggg 80700
tctgcctttc ccagctcact gactcaaatg ttaatctcct ttggcaacat cctcacagat 80760
acacccagga gcaatacttt gcatccttca atccaatcaa gttgacatta catattaatc 80820
atcacagcaa ccaaaagatt atctcattta atccttacaa tagctctgtg tagtgggtat 80880
atattttcct tttgctgaag aggaagtagg cttagagggg ttgagtaatt tgcccagaat 80940
acctaggtag tagaagatag tagagccatt atattctgtc tgcattcaag agtgtgctgc 81000
tttatttgtt cctgaattgc acacaagcta aagaatacaa ctggatgttc agtctactcc 81060
tattttgata acttggctaa ctttactgca gagtctgcag aggtgaccaa ttttactctg 81120
ggagatacca gggaatccgc catgtaattc ctcaggctga gtgttttgga aacacaaagc 81180
tgtcactgct attaagtgat gttttttcct gagagtctac tatgctccca gtaccagcct 81240
ccgcaggccc tctgtctacc ccttttctgt cctgatgtgt tctagcaggc actgagaaag 81300
tcattgtaga agttaccatg actttccaaa tgcttccaga agcagaacac aatttcacag 81360
gagggccaat aagtatgaat gccaccttga tagaaagtga agttttgcgt cctgttgaac 81420
actcagtaaa tgcttcctga atagatgcag gattggcaaa acatagctcc ccagctttca 81480
taattcagac tcagaggccc ttcgtgctcc tgttattctt gttacttcat gtcaggtacc 81540
taaaagggtt gtttttcagt tttcgtcatt gatttaaagc agaggttagt aaactttttc 81600
tgtaaaggcc aggtagttag tattttagcc tttgtgagta tttggtctca caaaagcagc 81660

catagatgat atgtaaatga atgtgcatgg ctgtgttctg ataaaacttt atttaaaaat 81720

gaggggtcat ctgggctgtg gtttttcaac ccctgattta aaacaaaaac aaaatgtgct 81780

atgcctgtta gatgctacta catttgattc tgtgttttcc atatttttct aaaatgtact 81840

gaacaggaaa tgaacaaact gatgacacaa tgatctaatt taatcattga gataagagtg 81900

gcgccaactc ttattgagta tttgttatgt gtcaggcact tggctaagca ctttacatgt 81960

tttctatgat ttataaagca aatattgtta ttatcttcat tttttgctga gggaaggaat 82020

aaagtcacag ccagcttaca gaggctgccg gaggtcacac atcaccagta cactgaaaca 82080

cgtggtctga ttacaggatc catgatctcc tgtacagatc atagtgatat tgacctgaga 82140

ggaaagagcc taggacttac aatcagaaat ctgggttcac atttcaactc cgttccatgc 82200

caaattacat taaaactggg cctcagcttt cccacctgtg aaagaggaat aatactagga 82260

cttgccctcc caaccttaga gattgctgtg aggataaaag cctatatgaa tgtgctttat 82320

aaactataaa caccatacaa atgtttgtta taagtctgcc atttgaatac atcttagtct 82380

ttttttccta tgtgaaataa aacattttaa gacaagcaat gaaattcttg aaactataac 82440

aggtttttaa gggtgttgat ccccaaagcc gtcttcacta ttttgctccc tctctgactc 82500

gctctgtctc tgcctagcag cgctagtctt ttctacgtgt ttgatctgtt gttcctgtgt 82560

ttaggcccat ccctactaca cagcggaagt cagctatttt ttctctttcc tttgctctgc 82620

tgagcaagat taaactcttt tcacctggac ttttcctaga ttactcaggc tgggtggaaa 82680

tggggtagct gtgaggctgg gatgttactg tacaattcaa atttatgatc tgaacttaac 82740

tagatcttgc tgaagattga cctggctagc acagttttga ggcacctatc tttcagtccc 82800

ttctgtcctc ttggcctggc cactggccac atgttagtag cttacttcta aggatggaat 82860

gccatgcatc tcctacaagg ttgtctctat gcttcacaca aagtaaaaga atggaggcaa 82920

ctttgaatgc tgctcatttt cactcaggca atccctttac aatattgtgc ttagtaaact 82980

ccccccactc tttttttccc ttagcttgtt tttttttct aataaaagtc tatatcaatt 83040

tgatgtatta gttagggttc tctggagaaa cagaaccagt aggatcaatt gattggtcaa 83100

tcaatcaatc aagacaaaaa gagattcatc tggaaggagt tgattcatgc cattgcggtc 83160

attggctagt ctgaaatctg taaggcaggc tggctgactg gaagctcagg cgggatttct 83220

ctgctgcagt ctcaaggcag aattccgtcc tcttggggaa acctcagtct ttgctcttaa 83280

agctttcaat tgattggatg aggcctacac acattatgga gaataatctg ttttacttaa 83340

agtctactga ttgtaaacat taattatatt taaaaagtac tttcacagca acatttagac 83400

tagtgtttga ccaacaactg ggcaccacgg tcaagccaag ttggcatata gaattagcca 83460

gcaaacttgg tattcacaat ctgtcattat agtcttatct ataaacctaa ggtttagaga 83520

aacgctggtc attctgaaca attccctgtg aatgcagaga tcaaggagcc tccccttaac 83580

tgggtaggca gtgtgaaata cctgcagtgt acttgacctt actgttcacc cactggcagc 83640
tgttcctaca gcttggcttc agatatataa tattacattg gctaacttca aagagcattg 83700
ttttccaagg atagagatgg tgatggtgta tattactttt accagcccca gacctgtaag 83760
aggttctcat ctcttttgtt taagattttg ttgttttttt ttaatgctca ataaaagaag 83820
ctttattctt tcattaaaaa acaaatctca gaagaacagg gaagaagagg gtagcctatg 83880
aaagtgtgat tgttatttga taccatcgtg acttagcttg ttttcaccta agactctgag 83940
attctattct gtttatgtga gatttgggct gagttcaact actatccata ttaactgaat 84000
cataaaacaa ttaatccatc atctggttac attttggtgg ggagggagca tttaacacaa 84060
cactaagcca catatttcac tatttttttt ttttgcagca aattgactta actgcttgct 84120
ttcacaccat gcctataaaa ccttttttggt tttagaggtt ctatgtgtct ggaaagtaca 84180
gtgaagtatc tcatcagaat agtattaata tttcttatga catttcatat gtcctgatat 84240
gggttaatgg agaaatatca taagagtagt aattctcttg ctgttattat ttgtcctatt 84300
tagacaaaca gaacatagaa ttaataagaa ttcaaaaact ttaatcactg atagagatta 84360
aatgatgttt atattagtca tcatcaccta atatcttaaa tatttaacct tttatgcagt 84420
gtttgcctct ctacttgggt atgcccttaa cagtaaaaag tgttgataat gctctccctt 84480
ttggcactca cagaattgct atacatatat atgtataaaa tatatgtaaa tataaaaata 84540
catagttaaa aaaataaatc taccactaaa tatagtaagg gagttttaaa ctgaggtttt 84600
tgaagtcttt aagaatttat ttagagactt cttttgttgg aagtatggtg gctagatgcc 84660
atgaaaaacc cactgaaaac aagtgtaagt gctaggtaat atgttgaaac atatctctca 84720
tatcctgtgc aggatgttct gagaagtttc tatttgtatc tgtggagtgg ttctagtctt 84780
gttcatcttg cattttaagg tttggaggta ttttatcttg ccttgcttct ttaccacctt 84840
gctttaattt ccccctatat tgccatttct aaagtaatga ctcattttca agggtgggtc 84900
tgaaaggaaa gaaataataa aaccagagat ctggagaagt ttctcgttca tggtcttatt 84960
cactttttaa taagagcatc tagaacagtg agtggtgccc cagcagatat ctggaaattt 85020
ttgtggaatt gaattgaatt tgttcttaga gtaaatgaca ataaagaaaa tagtattatg 85080
gaaactttcg acccaaggga aatatcaagg ctaaagttgc tatattattt ttcttctgat 85140
ggatgctgag tttttctcct taaaaaacaa atccacattt aaacataagg cattgtttga 85200
tggcctatca cattcagtag aatggatttg agaaatacgt gtaggatgcc tattaaaaac 85260
tctgttttga aattaaacct aagactataa gattgtctgt ttaaaaaaat tattgctgct 85320
gtaatttgag caaatctgaa ctatattgaa ctttagaaat tcaaatgtat gcaatcttgt 85380
aatataactt actccatcgg tagacatagt tctttgtccc acctggcttc cagcagtttg 85440

tctgtctgtc attaggctaa actcttcatg ttcctaaaca tgttttcctc aaaaggggac 85500
atatttcttg ggtcagatgt aaacatcaag ctaaggagtt ctgctgtgcg tctagacaaa 85560
taaggttcat tttcatcctc tacatagtgt taaacttgaa tttaagatca cacagattcc 85620
tttacacgtt accaatgtaa tgatatagac aatctcactt tattcattca ctctgtttaa 85680
ttcattcaat aaaaacgtat acattggctg cctactgtgt gccagttggt gaagatataa 85740
agatgattaa ggcaagggtc tgtgctgaca tgcctaatgg gcaggctgac acacctaaac 85800
gccagggaaa aagagggatg agacctgggc ctctgaagcc ctggccacct ggctcgaagc 85860
tcactttaat gtgcttctgg gtgtattcca tgacttttta aaggcgctta tgtttccttt 85920
ctggtaaagc atgtttaaca tgcaaaatgc ttatttcatt gagtagcttt taaaaagtgt 85980
tcatagaatg tctagaacag tgcttgtcta atagaagtat aattcaggtc acgtaggtaa 86040
tattaaattt tctagtagcc acattaaaaa gtaaaaagca acaggcaaag ttaatatttt 86100
tttttttcct gagatggagt cttgctttgt cacccaggct ggaatgcagt ggtgtgatct 86160
cggctcactg caagctccgc ctcccgggtt caagccagtc tcctgcctca gcctcctgag 86220
tagctgggac tataggcgcc cgccaccaca cctggctaat tttttgtatt tttagtagag 86280
acggggtttc accgtgttat ccaggatggt cgttaatttt aataatatat tcattaattt 86340
taataatata ttcatttagt cctatataaa aaaattagca tttcaacatg taatcaatag 86400
aatatattat tagtcaacta ttttgcattc tttgttttgt accatctttg aatttcaatt 86460
tgtattttat acttacagtg catctcaatt ttaatgctaa atgtcatcgg agatattcga 86520
tctctgttta gctttaatat atttttgggt tgaaaaagta aattcacata accaagttct 86580
tccaaatata cttgaaagtg tttctgataa ctgagttatc aacttaaaaa tgtaagttaa 86640
tgacaataaa tgaaagaaaa attctgttcc ttagctgcac tgaccacatt ttagtgttca 86700
atagtcgcag gtggccagtg gctatcatat tggatagtac agctttagaa tgacagttca 86760
gtgcaacaag tgctataata gactcatgaa ctggaagctc cgggaagtta agaatgggat 86820
caatgaagtg ggtcttgaaa aaccaataaa aattcaccac gtagagagga ggagggcgac 86880
tactccaatt caatcttttg aaagcatgtg aaggagcaac agtagacttc tttaattatt 86940
taatagtaat ttattgaaca tgaattatgg gaggtgcatg gtgtgggtgc tggtgagaca 87000
tagttcctga gctcaagtag cttggtgtct aaatattcat gggcccattt ttcagaaagg 87060
atggatatat gtgtgatcct gggtgtgcca aatgctgtgg cttcctgaag cttagatttc 87120
cagcttgtca ccttcaaggt taccttgtga ataggacttt tttgagctgt aagtaaattt 87180
actttgccta tttatttcca atggaaaaaa agcttttttta aaaaataaat ctcatcttat 87240
tgcctatgat tggccaagac aacatggccc atacagaagg tttttgatgg cttctgaggt 87300
ctgttatcat ttgcttattg gcatttcagc tgtcaaccag ggttctgtca aattccattc 87360

ctgcctttag ctcttttact tgaatacctg gggcaatggc agaagtggtt gctatttgtc 87420
acctttccag gatgttagtc gtgtccttga gacaaataga aaatttaaag tcagatgact 87480
tgattcctct gccagttaag acccttagag agtcctcaga atgttgctta tttttaaatt 87540
tcaagtccct ggtaaggatc aagtaaactc cccaatttgc agatttctat ccagttgact 87600
atggattttg cctgttgctt tgtttccacc aactctccct gaagatgagg cgcacagaca 87660
gacaactcac aggcaagaac agcctggtcc atcttgaaag attctcaaga ctattctcca 87720
caagataatt gtctactttt aaaaatattc agtaaaggga atttttgctg ttatccttgg 87780
ttgtgttttt agtatctcat catccttcta gttgtaggag gcttttccta acatctaacc 87840
catatgtctg ttgtctcatc aggtgtttct attaaggcta cttccccatc aatcttaatt 87900
tttttttaa tcttctgaga tgtataggtt aagttgaaat cagagacttt cataaaatgg 87960
taagatggcc atttaaacgc aactatgagg aaataatgta agcaggattc cattggagaa 88020
ccaaacacta gcaacaacta acttggtaat caatgttggg acttgaagtt aggctaaaat 88080
caatagtaat ggcactcgta tgtacaaatg cagaactttt tacacacaat gatttttccc 88140
tctgttatta tacactagct gtgtctgagt agacagtcca gctccactac ctgcagtcca 88200
ccctgggctg tgtaatctga gcactgatgg gctgttattt gtacgtatta cttctatgac 88260
actgtttttt catctgtgtt agaaatgtct ttctttcttg aataagctgc ttcaatttct 88320
tatagacaga ttctgctttt atgacccttg tttctacaaa gtaatctcta ctcatgctga 88380
aatctcagac aattttaaca aatatttaga gtacttaatt tttctttgaa ttctaaatat 88440
tatccttctt tagttcacca tagttggata ttttgagata actgtggagg aaacagcact 88500
gatcctgggg ccatgaaacc tgatttcaag atctagtccc ttctttaatt ttgcacaact 88560
aatttaatca cattcatcct taatttcatc atcattaaaa tagcagagaa taattcccgt 88620
ttcatggagt tgatatgcaa tgaaatagca tatcaatgta tgtatgaatg tcccccaaca 88680
tagagtacta cacaaatgaa acgtgtcact cagcataacg ttatgtgctc cttcctgcaa 88740
ctggattgca ctcaggcctt caaaaatgaa atttaaggcc tgttaagcag acccaacatc 88800
aacagcatat tctctctctc ttgtaagtat tgtctagttg ataaaaaatt tcaaaaacat 88860
gtcttaatca aaaacaagat gatccagcac aggtttaata aatgttttgt gaatatggca 88920
cattcgtgtc tcattactac agttttccta tgctgtcttt ctcaataatt ccccccaaaa 88980
ttgtagtgtt tacattatgg catttatagt tacctctgaa cctaaaaaat taacttcaaa 89040
agtatttaaa aaaatcatta tatttaaaac catttcatgt ttaaatggtt tgtacagggc 89100
aatgaaagga aatgtagtaa taaacacaga atcagatttg gtcactaata tttttctgca 89160
gttgaaatat atgtagactg gcttagggtc taaatagcat tgaatcctgt taccttccat 89220

ctataatcaa ttaatgtatt atgacattgt tgtcctcaga tcactgggat ctcatggtaa 89280
gtaagtaaag ggattattct gtgcattttc ccaatatcta tattagtttt aatatacctt 89340
tatgttaatg tatgacactg acacttagta attggattaa cttcctatca gaatttgttt 89400
ttcactcata ctttgtatac atgtcttaag ggtagggtag atgtacattt ttttctgtgt 89460
tagcctatct gtttctgtga cactacatgc tttctgtcct ccaatttgtg tttctttccg 89520
tgtacaaata tgcatcatct accatctaca tctacaaaac atgtaggctt ccaatgtttt 89580
catgtaaata catttcatga gtcctcagta gagtgttaga tgagtggtta attagtaatg 89640
ttaataaatg taaattacaa taaaaacatt tataatgtta ataaatataa gtttattgaa 89700
cttaaataat ttaaacatct tctgaaatgt agtagaaatt atgattagcc ataactagta 89760
cagtattttt tgcttgttta attttttgg tgatattcca tttgtggaat gagtgtgtgt 89820
gtgtgtgtgc agtacagaca gaaaggagag aaacatatct gcatgttatg ttagaaagag 89880
tagtgttagc taccctacca aatttacata ctggctgaac acagtatgta aaaaattatt 89940
tttttagtcc attgctggtg ttccttgttg ttgggtgact ctattccaca tggtgatgca 90000
gggaaccaat ctccttcctt ctgtggctgt gtaatccctt gcaaccttga agtcctctgc 90060
atctaacttt aggatgagga aagtgaaggt ggaggatata gccctcttga ctgccttgac 90120
ctggacctgg aacatctctt ttgctcccat tccatgggga agaactagtg acatggtggc 90180
ccatagctgc agaggggagg ctgagaaatg cagcctctgg ataagaagcc tactcccagt 90240
ggttactgta cattgtggga gggaagcttg aattctgatg gacagtgaga accattctcc 90300
acacaataac gaaatgacgt cttctcaacc ttggcaatgt agcatcctgt gacttaagta 90360
tgtaaataat tatcagtaca ggtcagtgaa aaattaggtc acctttccct tccactttta 90420
attctatagt ttaatttgcc tatctgctac atattatata tatgggaata gataagatta 90480
tctataaaca tgtataaatt tgtttcctac actaaaaaga aaaatgacaa agaagaatta 90540
agttagttgc ccaaatccac acaataccac caggagaaag tctaggaacc ataccttcta 90600
aatccaggtc tacacttctt tagtgcacta aattttttcc taatatagct ctgaggagac 90660
tggtgtttaa gaggagaatg ttaagccaaa agcccatttc acttggctct gtacagacag 90720
taagttaatt tccaggatgt ataactcctg attttctgtg atgacagaga aaatactgac 90780
ctgatttggg taactctgag gtttggagat cttgaatagc ctcatgttcc tgaacttctt 90840
taccatacat gaattacttc ttaggaagaa acatttattc tgtagaattt ggtttcgcat 90900
tttattttta ttttcttaga cactggtagt tggaaattca ggaagaattt gactacaaaa 90960
aacatttatt aaaaagagtt tctaatctca cctgctaagt gctacaagga aaaaaaaaaa 91020
tacattttga gccctcaagg ggctccaaat acggttgaag gaaaattaga cccagaatag 91080
tatatgatta ggaacaaaac gatggcagag aaatgagagt ttagagaaag gagagataat 91140

cctgagttac cttaggaaga gaaaatttca cagagaaaac ggcccccaca ctgatttggg 91200
gaagatgatt cagacttgtc tcaatggcat gaggagcagg ggccagatgg gaaaggacat 91260
cgtggtggaa atgacacgat gagcagaagc ttagaagtac ttctgcttca cgtctgtgat 91320
tctgcctctc cccttgtttc tgcagtgcta agttgagtgt agttccccac acaaaccctg 91380
agctttcttc ccctccaggc tgctccatca gtcagacagg agtgccttct caggggatcc 91440
tcttcctcat cttatcgggc cactcatctc gtcgcagaca ttcccatact cctggttgac 91500
ttgctcttcc ctctttttaa ctgacatgag caggcatcac ctcttccagg aaacttccct 91560
gacaccaggc tgagtctctc tggtgtcggg gaaggcttct gtattactgc atctgccatt 91620
ttgatttaag gttttctgct cattccaact caacagtgag ctctttgagg gcagaaattg 91680
tgtcttattc atttttcttg ttatgcccta aaaatgtatt gtgataatat ataagtaacg 91740
tacaatttac cattttcacc tcttgaagtg tatagctcag tggcgttaag tgcattcaca 91800
ctgttgtgca accattatca ccattcatct ccagaacttt ctcatcatcc caggcaaact 91860
ctgtatccat tacacaatag ctctccattt cctcctgccc tcagcctcag gaaacctccc 91920
atctactttc tctttctacg cgtttaacta ctcatataga tggaatcatc caacattttt 91980
ccttttgtgc ctggcttatt tcacttgaca taatgttttc aaggttcttt tatgttttct 92040
tatgcgtttt tatatctctc tatctttgat agcacccaat acatagaaga caatgaatgt 92100
ttttgtgagt gaaaacataa gaccagaaag aaataggatg tcatattcta gggacactca 92160
agacactttc ttgaggacag aggcactgta gcttggaatt tggaagatga ggttggcaag 92220
gtgggtgggg aaaggatgga aaagtgatac gtttcttgaa gcatgtggat ttgcttccat 92280
aagcagtggt ggaccagtgg gcacccttat ctattaaaag aatgattttt tttgtgactc 92340
gtgtagagca ctgtcttagt tacctacccc agtggtagat aagagaagcg gattaaaaag 92400
agatctgaac ctgaggaaat ggagactacc aagtttttttg taaatatgtc tgttataaca 92460
tatattatct agtacttgcg atgcctgtgc caaagcatgc tttttggttt agttaagcct 92520
acttagctcg ctaatttcag taattttggc ttgaattgca aaaagttgat gggggagatg 92580
ggggagtctc tgacatcctt cccccactca caggtttgaa aaataaaatt atgaagagca 92640
aaaggatctt tttctgtggt aaattgtact tcatgacaat aaacgagttg tggttttagt 92700
ggttaattta taaattaggc catctggttc ttctgtaatc taaagatttt tatatatatg 92760
taaaatacaa agttgtcctg ggcaactgcc ataaatccat tcaatcaaca ctgaatttac 92820
taatcaacta ctaaataagt aaaagatcct atagtagatc cttcaagaaa tggaaattga 92880
gacattaggt cactataaac cccaaccctt gtaatctttt tgtggcaacc ttaggaaact 92940
tggaatgctt tttacactct gataaaggtt taattgtaat catagcccca gtgttgcaaa 93000

atgagtcaga agatacaaag tatctcatag gataatatta tagctgtagg tacttcaaat 93060
ggacaagtat aacacattat gactgctaat atggatgcat tgggaggaaa ataagcccta 93120
agaagcaata tgcagaaaag ctatgattgc tggagacaga tagatccaag aacctaggta 93180
atttttcagc aaactgtggt tccactttcg cctctttctt gacattgact gtgtttttaa 93240
cagggaaaaa gttatctgaa atttttgata gagttcaaac tttaaatcgt gttcactttg 93300
ctaaaactcc actggatgga gtggcagaag agaaaaaaca cagaaataaa agaaaagcag 93360
taatggagaa tggaaaatct ttaaagatgc taccatcctg aaaagattcg aaagcaagcc 93420
caggaaagcc acggggaggg aaagagcgct tagaggtgtc cccaaagctt tcagaagtga 93480
tgagggaaac cccagctctt cttgttactt cttggcattt tgttcctcta tacgttagct 93540
tttggttaat ctttcatgtt acttattatc tctgttttaa aatttcacat taactcattg 93600
gtttcatatc ccacttaaca aattagatat tttccttggg cttattaaga aaattatttg 93660
ctctcatatg ttatggcttt agattttctg tgtagctagg actgttctta aatttttatc 93720
tttacctttg ttatttctct gaagcatccc cattggggtg gcagtggggg gtattaaatg 93780
gttatgtaaa atttacatgt aattgtgcaa tttacacata gcctcaaata taatgaggcc 93840
tagtctagct gcccatacca agaggatatt atagttgcca ataaaatcag tattatttct 93900
tttttttttc tgttcttttt tagcttcttg caaacattca ctcagaatcc tttttgaata 93960
tcagttttgt gaaaagcacc gtgctgggtt ctttgggaaa agccaagatg agggcaaatg 94020
catcctcctt cctagtgagt ctagcacaca gatgactcag gaagtcatgc actggaggag 94080
gagtatcttc tagaactggg gaaaggttaa ggaagggatc agaaaccttt ttcactcaac 94140
caattacatt ttcctgaaag catgttatta atcgtacatg atcttgcaga ccccagatag 94200
agactatatc actatatcaa ttgttttctt gaacaactgg agttaattct catatgtttg 94260
gttacccttc aagtatttat gtttaggatc tgttttcttc ctcttaactt acattccaaa 94320
gtttaggtat ccttcccctc catttgctgt ccaaaacagc atcctttcat gtagctacat 94380
gccattggaa tgatttggtt tttgccatgg attcctagtc ctaaatttgg acttttattg 94440
gtatctaaga tgtcatggac agaacccaca gatgttgtac aaaaagggct gagtggacag 94500
tccaacagag ttacatcatc tgttccaaac acccttgtaa tggttgtatc agcgtcaatg 94560
tctgagttgc tgtcctggtt ctcctggcca gtcctcagct ggccatgcct gttcaggcac 94620
cagagaaagc ttcatacctc aggcaaatct ttacaaggga tttgaaaggc aagaaacatg 94680
tattttggag gttttacctg tttatttcat gtatagcttc atattaacag attttgctat 94740
cacttaaaag taaataaaat gattttgatt tctcaaaatc atcttataat ttattaatct 94800
attttaaggc ttgcatttac atttaatctt caaatcttga tgcattttca cagagtttct 94860
ttagttagtt aaggatatta atgacttcct ttaatagttg atgaagtgag tcatcatcga 94920

cagtgagtca ctaaggccaa ctcttgtacc tgctcttaaa tagcagttgt tttactagat 94980
ttgtactgaa acctttttaa aaatctgttt gtcttatata taacaagcat ttccagtagt 95040
taataatact ttcttagctc ttcaatcatt ttatgagagc attacaagaa aagcaggaga 95100
tggggaaaga gatatcttgt ttgccatgta ggatgcatga aattcttact tagtctgttg 95160
tgttttggat tcgagagaaa atatgtaatg aatcaaaatg tttcctacag tcctctgaaa 95220
agatgtaatg agacagtctt ccagcagcct ttgatcatct gggaagtcat tgactttgat 95280
ccttatccta tcaggggctc ttaccctggg gtccataaat acccaggcat ttttaagaag 95340
tggatttcag tataacttgt ttcattttgt gatttttaaa aatattttgt tttatgcact 95400
taaaacctta atctgagaag tcatctgtag actataccag atttccaaat ggaataaaaa 95460
tatttaagaa ctctcttatg tgtgtgtttc aacctataaa ttttatatgt gtagctcatg 95520
gttaggtggt aagtagagaa tttgctatct gtgtaatatt tggtactttt tataatgaaa 95580
aaggaagtaa tacaatgata ggtaccattt atttgttgac cacccatatt ttccagggaa 95640
tgtgatagat actttttata tattctttct agttttcacc ataacatgct gcagggatag 95700
tgttaccatt ctcattttgc agatgaagaa attgaaaccc agaacaattc gttcattcat 95760
tcaaaaaata tccatagagc atacactaag agaaggcact ctgctaggca ccagaaattg 95820
aataattgac cccaaatcac ctaactcata agtggcaaag ctggacctaa caaagcccat 95880
tatcccttct atcacacatt ccatcctcat aatatcatat tctacataga aaaacttaac 95940
agtcctgttg agacacttgg aactctaaga aggtagtcaa cagatacttt tctctttata 96000
taaatatgat atctaattag cttttcctgg aaaggagtga ttctgccttt ttttaattct 96060
agcattccct gagcaaatta gagcccaggt aatgatttcc tgagataagt caattctctt 96120
ctaggacaat ctctagtctt gtcatcatga gatggaataa caacggtcta tgtcatttca 96180
gtgacggcac ctgcatgtcc ttgtatctaa gaaaagacac ctccttctct gccttgctaa 96240
atatttgtaa actttgcttt aattggtata tgtgccactg ttttgtatgt gggaggggtt 96300
tatggttttg ttttgttttt tggtcttttt tagtaacccc aatccaaact attctcattt 96360
gtaacctaaa atacttagaa caagaggctt tgcaatcttg aagaccgaat acatttttac 96420
tactaaatgt aaggcagatt tgagccaccc attttgtagt gcttgattat ggcaacccga 96480
ggaaactaat acggttactc tttatcttta ttaggaaata ctgctgtagt tcctgttgta 96540
atccttaggg agccaacatt gttgatgaga cctagtacca agccagaggc catgttgaac 96600
tagcaagata tgggatagtt tttactttca aagtaattag catctagagt ctagagagtt 96660
tctcttacag ggtggcctgg tgtcttagtc tattttctgt tcctataata gaatacctga 96720
gactgggtaa tgtacaaatt ataaagattt gtttgactca caatcctaga ggctgggagg 96780

ttcaagatca gacagccaca tctggccatc ctttaatgag ggcctcatgc ggctatttga 96840

ggtccaggat gcctttttat ccttttttatt aggttatttt tatatataaa tgaaaagaaa 96900

aacataaaca gaaaatacaa catgacatca tgaatgaggg caagcacaaa ctgcccaatt 96960

taagtttgaa gttataaacc taaaatattt taaaacaaaa catttctctt ggtgttttgc 97020

aaaagtaatg ctttcttcta ttgggcagaa attgcagcct gttgggatga aggagagaat 97080

agaactaata tttattgagt agctaccatg gctttctggg acatctgcta acccccttaat 97140

ttttacagta accctaagtg gtaggccagt ccatctgcat tttacagaca ggaaatgctt 97200

tcagagatat taattagatg gctgtagacc acagaagaat tgaaagtcag gtcttcttga 97260

cttttcagct catgacttca ttacttctgc cctcctcatt ctgttgggag atgggtggga 97320

gtgtgccctc cagataatca gaaagtccca tgttgggatg tgatttggaa atagctagtc 97380

ctcaggcctg tggacgaagg ctattgtagc ctcaagaaag aaagagtcag cttgatattg 97440

gcaaggtggt tatttagtgt ggcagctgca taagagcatt ggataggtgg tatgatatgg 97500

gatgggggtc agggtgagag ctgctcacac ctccttatta tggcagggca aaaccagaat 97560

agtcacagtg atggtgatgt agggtaggtt caagagaaag ctcatgttct tctagcattg 97620

agactcacaa tatttcaaaa caagtgatta tttattccat tactatttat tttggtgatg 97680

gatgttgcat ttattttatt ttctaacagt agtacatatt tggtaagagt tgtttgtgac 97740

attctttatt taaatggatt aaacccattt tcttaatttt tttaagtgtt tgtcctgtta 97800

tcgaagatat cttaaagtgt tacagaatgg gaatcatagc tttaatgagt ctgcatataa 97860

gagagtccaa acattttttt tttggagaga aggaaggtca tttcaactgt actataggaa 97920

cgatagggcc catgatccat ggtatggcaa taagttacta ttttttggag aataaatcgc 97980

aaaatatggg acgtgaagac tgcttggtat ttacacagga gtctagttct tctgatacta 98040

tatattctat aagctttctc ccagaaatcc aaagttttat ttagcttgta gccatcattg 98100

tgccacaatc ttccttccat gatttatttt tttcccatac tgtactcttg cactgagtct 98160

ttggcattga aagggaaaga gatgaattat tcaactcttc ttctattgag tgaaaagcca 98220

atacagaaaa aaaaatgcaa caggcaattt catgactgac attttggaga acattggggt 98280

catcatatga ctgagaaaat gattatctct tttgaaggtc aggaaaccaa tattttttttt 98340

tccagtgaat ctactgaggg aaccaaccac ctagcttttt gtaaatagca ctcctcaggg 98400

aaatgttgct tttttctttt gcagtgtttc tgctttgaat cttcatagac tgtatcccat 98460

ggaagcagga atataattac tgtagtgctt actttttttc atgtctctac tgaagagcca 98520

ccctgattta tcttttactc caaggtagtg tcgaacttta caaggattta gattatatta 98580

gtcattttgc ttggagatct ctgcacactt ttcaacatca tttctaagag ccttgccaca 98640

ctcagtaaaa gagggtgagg cttcaagaat ctctgtgtac caacgcaaat taggcaattt 98700

gtaagaagtc cctagtagag cagagtggag agagggctca ggatctcgtg aggcctgggg 98760
cagggaggtg gacatgctat gagctccttt cacagtcaac acaaagttaa cacatcttgg 98820
aggaaaaacc gttactgtat aatggaaggc agaaggcagg gatgtctgtg cttggtgatg 98880
aagctataag tagtgagctg atttatgatg gaggggtgaa ttgtgccatg aaagagactc 98940
ccctcaattt tattagccag attactgccc atatgcgggg tgaggagtga gcattttaag 99000
gcatttctct ctgttttcaa tatatcatct ctggattcac tattactggg acttgtgaat 99060
atagactagg gattaaaacc tgagtttgct tttaaaaaac tttattttat taaatgagat 99120
ttctgagcaa ctgatttcac ttagtcttat caaactattc ttactacttt ttcgatatgc 99180
attctgtccc tcctgtgtga aggccacagg cccatggccc ccatgtcatt gagatggagg 99240
tcctcaagtg atggcgggat gaatcacctc tcacttgcca cctgcgagaa tgtctatcac 99300
tgatacctct ccttatgtcc cgtagcagct gctaagctcc cacgttatta ttggtgacac 99360
tttctagtgt tcaggagaga acaggattta ctttttgaat gcctctaaat tattatagca 99420
ggttcatttg gaattgcata tcagaacatt cttcctgttt atcaggaaaa taaaccaagg 99480
tcttgaaact tgatgtctat ccatagttgg cccaatataa ttttgcatgt ggtttattac 99540
attatttttc tccattggct atgaactgaa ggcatctcat ggatatttat tccacgctga 99600
aaaggtagtt agatgctgtg tggtaaagtc gagaccagta tattggaaga aaagagacac 99660
tccatctaat aatctttcat agtcttcaca ttcattgaga aggttacttg ataacctttc 99720
ctgcacactc tctcccaacc ctgacccagg aacttgcaga gcacactgtg caacagatct 99780
aataaccctt gtctgcaggc aatatgcaca gctgggaaag ataattaaat agactctttt 99840
tgagaggtct acaacatttc aacggaagga tcgagcaatc cggctggcct ccgactgaaa 99900
actattacca acagacatac ttcagacaga ttccatgatc accatcctta tgtggcacaa 99960
gtgcttatgg ggagcttctt tggcttccaa aaggccacta aaactgtcag acctaaataa 100020
tactggaact gtaaagtggg aattaaaaat tataattcca tttaacatgt aacgttctca 100080
tcagaaaggg gcctttggtt ttccatattg atcggttaca tggtcagctg caattgtaac 100140
tcatgagaca cacccagata tcatcctgcg tgattctttt tgaggtttat ttagggtgtt 100200
agataatccg aggtaggttt catcatgggt ggcaatatta catatagcaa atgtcatgag 100260
aagagagaca aagcctccct tttgaatgtt aacatttatg caataagttt aatgtaccgt 100320
gtaagtttac attactatta tgagacttaa cctaaaaaat tggggtgaaa tcttaaagaa 100380
ttatgaaatt aagaaatgaa accagagggc tttcgtttta agtactgatc acttaaacca 100440
aaaccagttt ggcttatcct atgagatcgt gggtattgag aaagaggaac atttgctgct 100500
gcatccggag atgtttcctt gagagcaaga ctagttcctt ttgacccagt acatcctaca 100560

ggtaaatcac tgcatgctgt agagatacct gttccctccc ttcccccacc ccctggggat 100620
ctgaaaccaa atcattgctt tttcatcttt tacttatcct tataaacaaa gccacccact 100680
ttctcatttt tcctgatgcc aagtccttct ttacatgagt ggaatccact cttttcaatg 100740
gaaagacacc tgctctgaga caggctacac tgtcatgagc cttggcatgg ctgagtgatg 100800
tagtggaagg tgcaataaat ttaaacttta aaaatgcaaa atttgatgga atcatgcatc 100860
ttgtgccttg atttccatcc tcatctgtat ctatcttctt ttttaaaatt taattcaatt 100920
tttataatgt tgacacaatt atgcagattc atagggtaca cagtgatgtt ttgatacata 100980
taatgtgtgg tgatcttatc tatcttcttg agtacacact tgcttgagga cagtcatcaa 101040
ataattgcat tttgaatatg tgaaggtttt tgacattact gcacccaaaa aactcatatt 101100
gccagtgagg tgatatggcc taggatttta tcagctacag ccttctcttt cctttctgta 101160
cactccagtg gtggctaatt ttctttcctc tctcacagaa gtatgaatga ctaaaagttc 101220
tcatctctat tcattcctac tttctaaaac ttcagatcgg aaatttgaat tacctctaga 101280
ccaggatttg tcagtctctt tactattgac attttgggtc agatcattct ttcattcttt 101340
cttggttgag ggttgatat tgtttggtgt gtcctcatcc aaatctcaaa ttatagctcc 101400
catagttccc atgtgtcatg ggagggaccc ggtgggaggt aactgaaaca taagcaaagg 101460
tctttccctt gctcctctca tgacagtgaa tttctcatga tatctaatgg ttttataaag 101520
gggagttccc ctgcacacgc tctctctctt ccccgttgcc atgaaagatg tgactttgct 101580
ccttcttccc catgattgtg aggcttcccc agtcacaagg aactgtgagt ccattaaacc 101640
tttttctttg taaattaccc agtctcaggt acgtctttat tagcagcttg agaacagact 101700
aatacaaggg gctgtcttgt gcattttagg atgtttaaca gcatccctgg actccaccag 101760
ctagctgcca gtagcaacct ccacttcctc cagttacgat aactaacaat gtctccggac 101820
atttctacct atcttctgtt gtgtcagggg gtgggggagg taaaattgcc tctggttgag 101880
aatcaccact ctatgctttc ccaactcaat gttctataag ctcctcagac acactgtact 101940
ctaaactgca cactcacttt atcttctgca acaagtctat tccttttctt ttctgtcttg 102000
gtgacatcaa cactcaccta gacactaaag ctagaagctt taagttaccc atggattcta 102060
ccttctccct caccaaatta tccagttaac tatcaagtca tctatgaact gcttctgaat 102120
ccagacctcc cctctatccc cattcctctg catggcacag gtcctggttg tctctgctgc 102180
agattccttg cttctgcacc actgactcac tttcttggtc ctgctactct ttctttcagt 102240
ccatctccca tactgctgct ggagaaggct ttctaaggca cagctgtgat catgatgctt 102300
cctgactcac ctttcagtgg ctctctcaat tcttaacatg gagaaacccc atctctacta 102360
aaaatacaaa gttagccggg catggtggct catgcctgta atcccagcta cccgagaggc 102420
tgaggcagga gaatcgcttg aacatgggag gcagacgttg cagtgagcca aaattgtgcc 102480

attgcactcc agcctggatg aaattccgtc tcaaaaaaag aaaagaaaat actgtgtaga 102540
cttcttatct taataatcat gaatcactat tacaagtgtc tcaaacgcaa atttctgaag 102600
agtcacaggt gacccagtga gcccttgtcc aggctaaaaa cacctggtgg ctgaattctg 102660
aacttcacac attcgaattt ctcttccatt gcttctgaga gaccctccac cattacggtt 102720
ccttatcaga gtcatatcta cctataaggc tcatttcagg tgccgtcttt ttataaaatc 102780
ttccttcaaa ctttctcctc ttcagccttt atatatacaa aaggcttttc tttcttttcc 102840
ttttctaagt ttccattgta gttggtttgc ttttctcttt attattctca ttccaccata 102900
ttagttatga ttggatgtct tcttaccttc cctactagct cataaacttc ttgtggtcaa 102960
aaaccagatc ttgttcgtct tgtgtgatct ccagtttacg aagtcttcca tgatacccaa 103020
caaatatttg ttaattgcac tggccaaggt cacccaggtg gcttgtggcc aaatcagaag 103080
aatctacatc ttctgatagt catggcagct gttattccca tccaactcac ttcctgtgca 103140
ttgctactca tcaattcccc acttattctt ttaaaaacat tgcataaata catatctatg 103200
tgttttggaa agattttctt aatctataag cgcatttggc gtgtgcttat atgtctgctt 103260
atatgccaaa tttgaaattc caaatttcgc catttggaat ttcaaatggg gaaaagcaaa 103320
gactcttatc tttcccataa tcaaacccct tatgaagtaa aatccttaag gtctctctac 103380
aggttaaaaa taattaggtg atatgatttg gctgtgttcc cacccaaatt tcacctttaa 103440
ttgtaataat ccccaggtgt caagggtggg gccaggtgga gataattgaa tcatgtgggt 103500
tgttttcccc atactgttag tgtggtagtg aataagtttc atgagatctg atgattttat 103560
aaatgggagt ccccctgcac aagctctctt gcctgctgcc atgtaagatg tgactttgct 103620
cctccttgcc ttccgccatg attatgaggc ctcctcagcc atgtggaact ctgagtcaat 103680
taaacctctt tcttttatat attacccagt ctcaagtatg tctttattag cagcatgaga 103740
acaaactaat acattaggta tatgttaatg atgaggaatg tgtataaata catatacatt 103800
tatatttaaa tattaatatt tacaaataac atgatattaa aaatatggtc tataattttt 103860
taaattatct acctttctaa tcattaacat gcactaaata ttattaaatt tcaatattat 103920
ttaattacca tcttggtgtt tgaggataac tgcttttgat ggcattgata gatgcattaa 103980
cagtgaaaca ttgtgaccaa cagcccatct gagattttat acttgtagaa gatggcttct 104040
gagtgacagc tgagaagact gtttatgtct ttgccctaca ggttcctaac ccctttaata 104100
aaatagagct ctctgatgca gatgacacat gggccttctt tttgctcttg tcccatcaca 104160
ttgtacatag taaacatttt tagcaacaaa tagtagatac ttataacttt aaaagctaag 104220
tggttgacag ctgagaggca gatgatggta atttcatcat ttttctcata tctcaggcat 104280
tgtgacacta cctctgcaag gtcaactgtc tccataggct gttttcattt gcgtagaaat 104340

agggggggaaa gatagcttga agtcatcagg agccctgcaa cccatggatt accaatgttg 104400
ctaactggag cctggatttc atcttcaatt gattgtaatg atgtcttgtt ctctcaaatc 104460
tttgcaaatc taggcattta taagactatc ctgtaggtcc cttacaagac catgaggatg 104520
ctagaactta ccctagtctt ttcttgtaat tgcttaaatg ttagtattgg caaatggggt 104580
ttggtgatta taagagtaaa aaaacctgct gccatcccac atttgtgagc agaggataca 104640
tttcctatct tgtgtccatt taaaaagaat gattgcttga ttggcttcct gatgacaacc 104700
catgatatac tgattctgtt agtttataac tttcctagta gtcatatgct tataggccaa 104760
ttttatcctt gccatgctta gcctagtcac aatttccagt tcttctctgt atcctgcagc 104820
agtgagttcc aaacacttaa tgctgtcatc tcttcctgtg aaaaccaaga aataaaggtt 104880
attataaggt ataaataaag aacccaatag aaaacacact gtccatgtct ttgattgtta 104940
caccacagca gagaatcaat ttcttaacct gcttacttat ttaccattct ccaatggtgc 105000
caattctgag atccatcatg actttatcat tagaaaaggg gatgtaacaa acatgatgag 105060
atgttacggg aatactgctt actgcatgat atggtttggc tgtgtcctca cccaaatctc 105120
atcctgaatt ttagcctccc atacttccca cgtgttgtgg gagggacccg gtgggaggta 105180
attgactcat gggggtggtt tctcccatac tcttctcctg gtagtgaata agtctcacaa 105240
gatctgatgg ttttataagg ggttttccct ttcacttggc tctcattctc tcttgcctgc 105300
tgccatgcaa gacatccctt tgctcctcct tcatcttctg ccatgattgt gaggcctccc 105360
cagccatgtg gaactgtaag tcctttaaac ctcttttctt tataaattgc ccagtctcat 105420
gtatgtcttt atcagcagca tgaaaacgga ctaatacact gcatgctcag tatactcagg 105480
cactctggaa actaggtttc tacttcggac ccctgagaga aaagggttat gtatctccaa 105540
gtgtggtcat gacaagcatc actactgtgt gcagttgggc ctgtggattg agccacctta 105600
gcctctgagg aaagcagaac ctgagagcat gcttaattca gagtgtgtgt gacttggagt 105660
gtggaaagga agatttccag atttgcggta ttggcgactg aaaggatggc aatgctattg 105720
atggagatta ggaagacagg agagaggtag atgttgagca actccgagga gtttttcctc 105780
atacatgttg ggtttaaagt gcagggaaga tatcctgaag gttattccag gcagaaattt 105840
ggagctcaga agagacatca gagctgaaga taaaaaattt gagagccatc tccgtagcta 105900
aaagtgtgga acgaatgtga tgactagaca agataataga gaaaaggtat ccaaaggcct 105960
gaggtcaaga cctcttaaaa ggctgacttt tcagggtttt aggtagaaaa agagtcccag 106020
cctggggtta aggcagtttt ttgagaagta gaggtacaaa gttccttgtt ccaaaggaag 106080
ggtgtctttc aaaagaaaaa ggatgggccg ggtgcagtgg cttacgcatg taatctcagc 106140
actttgggag gccgaggctg gcagatcacc tgaggttggg agttcgagac caacctgacc 106200
aacatggaga aaccctgtct ctactaaaaa taaaaattaa aaattagccg ggcttggtgg 106260

tgcatgcctg taatcccagc tactcaggag gctgaggcag gagaatcact tgaacctggg 106320
aggcagaggt tgcagtgagc caagttctca ccattgcact ccagcctggg caacaagagt 106380
gaaactctgt ctcaaaaaaa aaaaaaagag agaaaaagaa aaaggatggt gaattgggcc 106440
aaataccaga gagatggaca ggagtaagaa gaaaaagact aaagatttgt tcatcaggag 106500
ctcattgttt ctaaagggag catttttagt aaagaggaag gaggaataaa atgcagtata 106560
atatgttaaa tctaagtata atttataaaa gagaaaatgg gggtggaata ttacaaaaaa 106620
ggaactataa accagctctt tgaggtattt gtcagtgagg aaaacagtgg ggctctagtt 106680
cttgatagca acagcatcaa agggaacttt ttaaactaaa atattatgaa tttttcaaac 106740
tgaaataatg agagaataat acaaagaaat ctcatctgcc cataatccaa attcagtagt 106800
tattaaaatt ttgccccatt gcttaatcca ttaatctcat ttcattttgt cctttttctt 106860
tgtttatttt tggctaaaac attttaaaga caaatcctaa atatcataca tcagtaggca 106920
tttacaaaaa aatttcttga taatcacaat gccattctca taccaaataa acttataata 106980
attctgtgat atcatctaaa aaacagttct tttctacatc aaaaatgtct gtttatagtt 107040
gatttattca atttagagtc caaacaagtt ccatatatta gacattaggt cattaatgtc 107100
tgtctatttg aatttataac aatttccact taatttattt tctccagctt ctgtggagac 107160
taggtaagtt gttttgtagc tgtctggata tatctgattg cttccttgca gtgtcattta 107220
acttgttcct ctatcctcag tgtttctgca catggaagtt agcctcatag caggggttgg 107280
catactttag cctgcttacc aaatacgact cccttacatt tttgtaaata aaatgttact 107340
ggaacactgt cacatatgtt ggggtcatag aaagagtttc cgtgtcccca agttataatt 107400
gcattcactt atattttctt ctagtaattt ttacccttac atgttagggg tcaatttgga 107460
atttctcttc atatacagta tgcaataaag aatggctttt atatttttcc aaatgtatat 107520
tcagttgtct caacaccatt tattaaaaag tcagtatttc tcaagtgatt tgagatctac 107580
ccttaattac acagtaaatt tctctgtagt cttgtctttt tctaatatgt taattctgta 107640
ccaagatttg ttcatctgtt aatggatcaa tatcacatag ctttgtttat agaaggtgca 107700
cattgtttta attctggcca tgctatccca ccaaatattg ccctttgatt tttcagagtt 107760
ttactgtgga ttcttccttg tttctttttc tatgtgacct ttagaattaa cttgtttaat 107820
tctataagta aatttattga taattttatt gaaattatgt aaaatataaa ttggcttagg 107880
aagtactgat atctccatga cattaagtct tcatatctaa gaccaagggg tgccttttca 107940
tttgttaaat attctttgtg tctttcagaa gtgttttcaa gtatccttat gtgtattttg 108000
cacaaatttt tatatttgtg cctattggat gccacgtgca gggttaaaaa atattatacc 108060
tattttatat agatatatat gtatataaaa atatatatta tatatagtta taatacataa 108120

atatatttta atatattata taatatatac atgtattgta tttatatatg tatttatatg 108180

tgtatctata aatatacata tgtatgtatc tataaatata catatatgtg tacatataca 108240

catatataca cattatatgt atgtttatac acatataaat acatatataa atatactaga 108300

aagtccatgg ggtttcctag tatacaatca tattgtctgc aaatagagat tgttatacct 108360

tttcctttct attccaaagt gtctaaaaga ttttttctta gctagtggca ttggatgaca 108420

cctataatgt cttctaaaaa tagtagcagt cataggcacc atttccttat tcttgaatat 108480

tcattcatgt tacaaagttt ataggaattt ctgaattatt aagtactttt aataggaatg 108540

aaggttattg tcattattgc atcaaaattc ataagaaagt ttggtgtcaa atttgtgctt 108600

tgtgtgtagt cttcatcacg cttttttttt tttttttaac cagtgttaaa catccattac 108660

aaaaatagtt tggaagtttt tctttttttt tttcctatgt cctggaaggt gtaagtagaa 108720

ttggaattat ttgatcttta agatgtaaaa tattggtaga attctacctt tgaaagcacc 108780

tggacatggt gacttcttaa taggtgagct ctctggaaac tttatttctt ctgtagtaat 108840

tggtctcctt agactttcta ttccttctgg agtcagatat tgtatatcaa atcttctgtt 108900

aaagttatcc atttcttctt ggtattccaa tttttcacat tgagtaagtg agaagagaag 108960

tttagagaaa gaagaataaa aagaaatgaa caaagcctcc aagaaatatg ggactatgtg 109020

aaaagaccaa atctacgtct gattggtgta cctgaaagtg atggggagaa tggaaccaag 109080

ttggaaaaca ctctgcagga tattatccag gagaacttcc ccaatctagc aaggcaggcc 109140

aacattcaca ttcaggaaat acagagaaca ccacaaagat actccttgag aagagcaact 109200

ccaagacaca taattgacag attcaccaaa gttgaaatga aggaaaaaat gttaagggca 109260

gccagagaga aagattgggt tacccacaaa gggaagccca tcagactaac agcggatctc 109320

ttggcagaaa ctctacaagc cagaagagag tgggggccga tattacacat tcttaaagaa 109380

aagaattttc aacccagaat ctcatatcca gccaaactaa gcttcataag tgaaggagaa 109440

ataaaatact ttacagacaa gcaaatgctg agagattttg tcaccaccag gcctgcccta 109500

caagagctcc tgaaggaagc actaaacacg gaaaggaaca actagtacca gccactgcaa 109560

aaacatgcaa attggaaaga ccatcgaggc taggaagaaa ctgcaactaa caagcaaaat 109620

aagcagctaa catcataatg acaggatcaa attcacacat aacaatatta accttaaatg 109680

taaatgggct aaatgcttca attaaaagac acagactggc aaattggata aagagtcaag 109740

acccatcagc gtgctgtatt caggaaaccc atctcacgtg cagagacaca cataggctca 109800

aaataaaggg atggaggaaa atctaccaag caaatggaaa acaaaaaaag gcaggggttg 109860

caatcctagt ctctgttaaa acagacttta aaccaacaaa gatcaaaaga gacaaagaag 109920

gccattacat aatagtaaag ggatcaattc aacaagaaga gctaactatc ctaaatatat 109980

atgcacccaa tacaggagca cccagattca taaagcaagt ccttagtgac ctacaaagag 110040

acttagactc ccacacaata ataatgggag actttaacat cccactgtca acattagaca 110100
gatcaacgag acagaaaatt aacaaggata tccaggaact gaactcagct ctgcaccaag 110160
cagacctaat agacatctac agaactctcc accccaaatc aacagattat acattcttct 110220
cagcaccacg ccggacttaa tccaaaattg accacatagt tggaagtaaa acactcctca 110280
gcaaatgtaa aagaacagaa attataacaa actatctctc agaccacagt gcaatcaaac 110340
tagaactcag gattaagaaa ctcactcaaa accgctcaac tacatggaaa ctgaacaacc 110400
tgctcctgaa tgactactgg gtacataacg aaatgaaggc agaaataaag atgttctttg 110460
aaaccaatga gaacaaagac acaacatacc agaatctctg ggacacattc aaagcagtgt 110520
gtagagggaa ctttatagca ctaaatgccc gcaagagaaa gcaggaaaga tctaaaattg 110580
acaccctaac atcacaatta aaagaactag agaagcaaga gcaaacacgt tcaaaagcta 110640
gcagaagaca agaaataact aagatcagag cagaactgaa ggagatagag acacaaaaaa 110700
cccttcaaaa aatcaatgaa tctaggagct ggtttttga aaagatcaat aaaactgata 110760
gactgctagc atgactaata aagaagaaaa gagagaagaa tcaaatagat gcaataaaaa 110820
atgataaagg ggatatcacc actgatccca cagaaataca aactaccatc agagaatact 110880
ataaacacct ctacacaaat aaactagaac atctagaaga aatggataaa ttcctcgaca 110940
catacaccct cccaagacta aaccaggaag aagctgaatc tctgaataga ccaataatag 111000
gctctgaagt tgaggcaata attaatagct taccaatcaa aaaaaagtcc aggaccagac 111060
ggattcacag ctgaattcta ccagaggtac aaggaggagc tggtaccatt ccttcggaaa 111120
ctattccaat caatagaaaa agagggaatc ctccctaact cattttatga ggccagcatc 111180
atcctgatac caaagcctgg cagagacaca acaaaaaaag agaattttag accaatatcc 111240
ctgatgaaca tcgatgcaaa aatcctgagt aaaatactgg caaaccgaat ccagcagcac 111300
atcaaaaagc ttatccacca tggtcaagtg ggcttcatcc ctgggatgca aggctggttc 111360
aacatatgca agtcaataaa cataatccag catataaaca gaaccaatga caaaaaccac 111420
atgattatct caatagatgc agaaaaggcc tttgacaaaa ttcaacaacc cttcatgcta 111480
aaaactctca ataaattagg tattgatggg acctatctca aaataataag agctatctat 111540
gacaaaccca cagccaatat catactgaat ggacaaaacc tgggagcatt ccctttgaaa 111600
actggcacaa gacagggatg ccctctctca ccactcctat tcaacatagt gttggaagtt 111660
ctggttaggg caatcaggca ggagaaggaa ataaagggta ttcaattagg aaaagaggaa 111720
gtcaaattgt ccctgtttgc agatgacatg attgcatatc tagaaaactc catcgtctca 111780
gcgcaaaatc tccttaagct gataagcagc ttcagcaaag tctcaggata caaaatcaat 111840
gtgcaaaaat cacaggcatt cttatacacc aataacagac aaacagagag ccaaatcatg 111900

agtgaacttc cattcacaat tgcttcaaag gaataaaata cctaagaatc caacttacaa 111960
gggatgtgaa ggacctcttc aaggagaact acaaaccact gctcaacaaa ataaaagaag 112020
atacaaacaa atgcaagaac attccatgct tatgggtagg aagaatcaat atcatgaaaa 112080
tggccatact gcccaaggta atttatagat tcaatgctat ccccatcaag ctaccaatga 112140
ctttcttcac agaattggaa aaaactactt taaagttcat atggaaccaa aaaagagccc 112200
gcattgccaa gtcaatcata agccaaaaga acaaagctgg aggcatcatg ctacctgact 112260
tcaaactata ctacaaggcc acagtaacga aaacagcatg gtactggtac caaaacagag 112320
atatagacca atggaacaga acagagccct cagaaataat gccatgtatc tacaactgtc 112380
tgatctttga caaacctgag aaaaacaaga aatggggaaa ggattcccta tttaataaat 112440
ggtgctggga aaactggcta gccatatgta gaaagctgaa actggatccc ttccttacac 112500
cttatacaaa aattaattca agatggatta aagacttaaa tgttagatca aaaaccataa 112560
aaaccctaga agaaaaccta ggcaatacca ttcaggacat aggcatgggc aaggacttca 112620
tgtctaaaac accaaaagca atggcaacaa aagccaaaat tgacaaatgg gatctaatta 112680
aactaaagag cttctgcaca gcaaaagaga aactaccatc agagtgaaca ggcaacctac 112740
agaatgggag aaaatctttg caatctactc atttgacaaa gggctaatat ccagaatcta 112800
caaagaactc aaacaaattt acaagaaaaa aacaaacaac accatcaaca aatgggcgaa 112860
ggatatgaac agacacttct caaaagaaga catttatgca gccaacagac acatgaaaaa 112920
atgctcatca tcactggcca tcagagaaat gcaaatcaaa accacaatga gataccatct 112980
cacaccagtt agaatggtga ttattaagaa gtcagggaaa aacaggtgct ggagaggatg 113040
tggagaaata ggaagacttt tacactgttg gtgggactgt aaactagttc aaccattgtg 113100
gaattcagtg tggcgattcc tcagggatct agaactagaa ataccatttg acccagccat 113160
cccattactg ggtatatacc caaaggatta taaatcatgc tgctataaag acacatgcac 113220
acgtatgttt attgcagcac tattcacaat agcaaagact tggaaccaac ccaaatgtcc 113280
aacaatgatc tggattaaga aaatgtggca catatatacc atggaatact atgcagccat 113340
aaaaaaggat gagttcatgt cctttgtaag gacatggatg aaactggaaa ccatcattgt 113400
gagcgaacta tcacaaggac aaaaaaccaa acaccacatg ttctcactca taggtgggag 113460
ttgaacaatg agaacacgtg gacacaggaa ggggaacatc acacaccggg gccttttgtg 113520
tggttgggga gggggagggg atgcattagg agatacacct aatgtaaatg acgagttaat 113580
gggtgcagca caccaacatg gcacatgtat gcatatgtaa caaacctgcc cattgtgcac 113640
atgtacccta aaacttaaag tacaataaaa ataaataaat aaataaataa ataaatacta 113700
gtttggctgc attgctttga ttttcttctt taggggcctg ttcatacata caaaggatac 113760
catttacctg tcttctatat gactttctct taaatccttt cattcttttg ttttaatttt 113820

tatcttcaca tcctttattt ctgtcactgt gctgtccata gagtttgtct gctgtgtgtc 113880
cttataatta agtctatgtt ctgaatgttt ttccttttt agaaattcag ttctaaagtg 113940
tttaatttct cgtattttt tttctgttgc ctcaccatat catttctgag ttttctgttt 114000
ctgaaatgtg caactaactc tttccaagca ttgaccagat tcttcagtct ttttaattca 114060
ttctgaaata actgggctac agtttcatct gctttgtgga cagaagtgcc acaaagagcc 114120
gaattgtcag tgcagaccca catgaatcat agatcttaac gaggttttta ctaacgacta 114180
gcaaaggata caagctaaaa atgggtacaa gcaaacacag catcattcat cactgtaaag 114240
actctgaact atcacatgga acttcaaaag gattcttctt ctttgctgca atgtgttttg 114300
atttttggag tgatagatgt ttgctaacta cgcacgtgac aaaaatttgc ttagaggaag 114360
ccatgttagt tttgatgcta ctcaactttg tattttgtta gtcatgagat agaaagcctg 114420
tgagattaca tgcccttctt ttagcagctg catcccataa aattaaaaat tccagttttt 114480
tttaaaccat gaacaattta gtcagactta atatatccta tttaaaacat tcttagataa 114540
gaacttcctt tgttattttg ctaatataca atcccaccaa atgtctacaa gaagggctag 114600
ttaatcaata taccaaagat caaatctatt tatatgaacc acaaatacaa aacataatta 114660
tacatatata attttatata atttaattta taattatata cattttatag cagcaatcaa 114720
aactatcatg tattttggga tgaatttaat aaagaaatag caggagctct gtagaaacaa 114780
gtatgaactt ctattggaag acagtaatat cataaaacat taaaaagaaa gctataccat 114840
gtttatggtt aggaagattc actattgaaa atacatcaat tcttgacaaa tcggtctata 114900
aattcagtgc gtgtccaatc aatatattta gctcatttaa aatgtatatg gaaaaggcta 114960
agtaccaaag ataatcaaat gctcttggaa gatgaaaatt aaagttgaga gatggggtaa 115020
aaaactataa ttcagttcta aagtcataaa gctataataa ttaatacagt atggcattgg 115080
catagaaata agcacattga ctaaataaat agaaaataca gttcagaaac caaactctac 115140
atttatgaaa aactgaccta tatcagaacg agcattgaga ctacaggaga acagaggaac 115200
tagtaagtta atggtcctga cacaattgat atctacatag aaaaaaaaac ttaatccttc 115260
tttactctat gtaaaaacaa taattccaga tgaatgtagg acttaatgga gagccaaaac 115320
taaaactttt ataggaaaac atagaaaaat acctatctca gggtcgggaa ttaggtcttc 115380
aacatggcac caaaagtact aaccataaaa gaaacattaa taaagtcaac ctttttaaaa 115440
tcaacaactt taattaaaaa accagggcca ggcacggtgg ctcacgcctg taatcccagg 115500
aggccaaggc aggcagatca cgaggtcagg agatcgagac cagcctggcc aacatggcaa 115560
aacctcgtct ctactaaaaa tataaaaatt agctgggcgt ggtggcgggt gcctgtagtc 115620
ccagctactt gggaggctga ggcaggagaa tggtgtgaac ctgggaggcg gagcttgcag 115680

tgagctgaaa tcgtgccact gcactccagc ctgggcaaca gtgcgagact ccatctcaaa 115740
aaacaaaaac aaaaacaaaa tcaaaacaaa aaacaaaaca caggttgctg gaaaaatggt 115800
tacttatttt tattatgctt tataaaatat acagttatat tatcaaactt cttttgtaag 115860
aatcaaatat tatgctgaga atatggcaag aaaaaagaaa acaacctcca ccactcataa 115920
tggcattccc tttacccacc atcaactaag tcaggaacct gcgtcacttt caagttttct 115980
gaaatgtgga ctgaaaatac ttgtggaggg ccctcacaga tgcatttaga aatggtatgt 116040
tctggaaaat ggaactgaaa tgttttagcg agagtcatgg gcccaaacct tgaaatagag 116100
gttggtggag ttttctatag tatgtaatta caacacaata agactcaaaa gcatttcaat 116160
aaagtgtgga tggaagacaa gatactgctt caagggcttc attagctttc tgcagactgg 116220
agctctccaa ggcatgggga ggaaccctgt ttacctttgg ttcagaatca gttttaacca 116280
tcattgaggg ctaggtcaga gtgcagcatt aagtccttgg gaatacactg ggtggggaaa 116340
gaaaagagag caatgttttt ctaaaagccc agaaatgggc ttactgtgtt taccagttgt 116400
ttccaggata atgtattgca gtgccaattg ctgatgagat ggtaggatta tacttcagtc 116460
cctgctttac atttatttct taaagaagct tctggtaaat tagagcaata gcatcggctt 116520
agtttagtgt tgttctgttg gactaaggat atcagttcta tccgtatggt cgggcctaaa 116580
gcctgggaaa tatttaatga agggagagag ggggagagag tgagcatgca aaagagagag 116640
agaaaaacaa ataacaaaac aaaaaccaag acatttccct ttatagtaag aatgatgagg 116700
aaaacatgtt tagccataca agatatcaag ataatctctt atttctttct tgaaaatgca 116760
agtacaaatg cctgcaaaga taaaatatcc tctggatgga gtggaaaggt tcaccaggcc 116820
tctgaaatca cgtgaatgat gttgcgcttt gctgttaatg aagctcggtg catttttcat 116880
ttcagtttct actaagcatt tatgagctat tacccttccc ttccctaaac tgcgttgttt 116940
tttaaaaagc cttagaggca ttccttctag aaaataaagg taagtgttaa gtggtgataa 117000
tttggtaata ggtgtcatgc taatggccat tgatttaggc cactggctta gagtactcct 117060
tcccctgcat gacactgatt acaaatactt tcctattcat actttccaat tatgagatgg 117120
actgtgggta ctgggagtga tcactaacac catagtaatg tctaatattc acaggcagat 117180
ctgcttgggg aagctagtta tgtgaaaggc aaatagagtc atacagtagc tcaaaaggca 117240
accataattc tctttggtgc aggtcttggg agcgtgatct agattacact gcaccattcc 117300
caagttaatc ccctgaaaac ttactctcaa ctggagcaaa tgaactttgg tcccaaatat 117360
ccatcttttc agtagcgtta attatgctct gtttccaact gcatttcctt tccaattgaa 117420
ttaaagtgtg gcctcgtttt tagtcattta aaattgtttt ctaagtaatt gctgcctcta 117480
ttatggcact tcaattttgc actgtctttt gagattcaag aaaaatttct attctttttt 117540
ttgcatccaa ttgtgcctga acttttaaaa tatgtaaatg ctgccatgtt ccaaacccat 117600

cgtcagtgtg tgtgtttaga gctgtgcacc ctagaaacaa catattgtcc catgagcagg 117660

tgcctgagac acagacccct ttgcattcac agagaggtca ttggttatag agacttgaat 117720

taataagtga cattatgcca gtttctgttc tctcacaggt gataaacaat gctttttgtg 117780

cactacatac tcttcagtgt agagctcttg ttttatggga aaaggctcaa atgccaaatt 117840

gtgtttgatg gattaatatg ccctttgcc gatgcatact attactgatg tgactcggtt 117900

ttgtcgcagc tttgctttgt ttaatgaaac acacttgtaa acctcttttg cactttgaaa 117960

aagaatccag cgggatgctc gagcacctgt aaacaatttt ctcaacctat ttgatgttca 118020

aataaagaat taaact 118036

<210> 47
<211> 21
<212> DNA
<213> Artificial

<220>
<223> Synthesis

<220>
<221> misc_signal
<222> (1)..(21)

<400> 47
cttgctcttg gacaggaacc a 21

<210> 48
<211> 22
<212> DNA
<213> Artificial

<220>
<223> Synthesis

<220>
<221> misc_signal
<222> (1)..(22)

<400> 48
caaactcctc tccctgcaga tt 22

<210> 49
<211> 1973
<212> DNA
<213> homo sapiens

<400> 49
gggatattgg agtagcaaga ggctgggaag ccatcactta ccttgcactg agaaagaaga 60

caaaggccag tatgcacagc tttcctccac tgctgctgct gctgttctgg ggtgtggtgt 120

ctcacagctt cccagcgact ctagaaacac aagagcaaga tgtggactta gtccagaaat 180

acctggaaaa atactacaac ctgaagaatg atgggaggca agttgaaaag cggagaaata 240
gtggcccagt ggttgaaaaa ttgaagcaaa tgcaggaatt ctttgggctg aaagtgactg 300
ggaaaccaga tgctgaaacc ctgaaggtga tgaagcagcc cagatgtgga gtgcctgatg 360
tggctcagtt tgtcctcact gaggggaacc ctcgctggga gcaaacacat ctgacctaca 420
ggattgaaaa ttacacgcca gatttgccaa gagcagatgt ggaccatgcc attgagaaag 480
ccttccaact ctggagtaat gtcacacctc tgacattcac caaggtctct gagggtcaag 540
cagacatcat gatatctttt gtcaggggag atcatcggga caactctcct tttgatggac 600
ctggaggaaa tcttgctcat gcttttcaac caggcccagg tattggaggg gatgctcatt 660
ttgatgaaga tgaaaggtgg accaacaatt tcagagagta caacttacat cgtgttgcgg 720
ctcatgaact cggccattct cttggactct cccattctac tgatatcggg gctttgatgt 780
accctagcta caccttcagt ggtgatgttc agctagctca ggatgacatt gatggcatcc 840
aagccatata tggacgttcc caaaatcctg tccagcccat cggcccacaa accccaaaag 900
cgtgtgacag taagctaacc tttgatgcta taactacgat tcggggagaa gtgatgttct 960
ttaaagacag attctacatg cgcacaaatc ccttctaccc ggaagttgag ctcaatttca 1020
tttctgtttt ctggccacaa ctgccaaatg ggcttgaagc tgcttacgaa tttgccgaca 1080
gagatgaagt ccggtttttc aaagggaata agtactgggc tgttcaggga cagaatgtgc 1140
tacacggata ccccaaggac atctacagct cctttggctt ccctagaact gtgaagcata 1200
tcgatgctgc tctttctgag gaaaacactg gaaaaaccta cttctttgtt gctaacaaat 1260
actggaggta tgatgaatat aaacgatcta tggatccagg ttatcccaaa atgatagcac 1320
atgactttcc tggaattggc cacaaagttg atgcagtttt catgaaagat ggatttttct 1380
atttctttca tggaacaaga caatacaaat ttgatcctaa aacgaagaga attttgactc 1440
tccagaaagc taatagctgg ttcaactgca ggaaaaattg aacattacta atttgaatgg 1500
aaaacacatg gtgtgagtcc aaagaaggtg ttttcctgaa gaactgtcta ttttctcagt 1560
catttttaac ctctagagtc actgatacac agaatataat cttatttata cctcagtttg 1620
catatttttt tactatttag aatgtagccc tttttgtact gatataattt agttccacaa 1680
atggtgggta caaaaagtca agtttgtggc ttatggattc atataggcca gagttgcaaa 1740
gatcttttcc agagtatgca actctgacgt tgatcccaga gagcagcttc agtgacaaac 1800
atatcctttc aagacagaaa gagacaggag acatgagtct ttgccggagg aaaagcagct 1860
caagaacaca tgtgcagtca ctggtgtcac cctggatagg caagggataa ctcttctaac 1920
acaaaataag tgttttatgt ttggaataaa gtcaaccttg tttctactgt ttt 1973

<210> 50

<211> 8244
<212> DNA
<213> homo sapiens

<400> 50
gggatattgg agcagcaaga ggctgggaag ccatcactta ccttgcactg agaaagaaga 60

caaaggccag tatgcacagc tttcctccac tgctgctgct gctgttctgg ggtgtggtgt 120

ctcacagctt cccagcgact ctagaaacac aagagcaaga tgtggactta gtccaggtaa 180

atgctgcatt gcatgtgaca ataatgtaaa tttttagttt gtatttttct gcagtaatgt 240

aatagagttt tttaaggata ggtttcttat aaagagattt tttttttttt tgctagaaac 300

agcaccctcc acccaaaatg tatctagcca tgcatacgct ctctttttcc agttggaggt 360

gagagtgaac taaaggaaca acatccaaac ttgtctccac aaattgtaga cttgtaacag 420

tatgcaaatc ttgctctaca aaaattgctc tactatgaag ttcttacttc acaataacta 480

atagtaggga catttctact ctgaaatttc atttctcacc aaaaataagg ataaaagcaa 540

ctgcgaaatc taaacacaag gtttaaaagc agttctctgt tttggctaaa acttaatgcc 600

atcaattttt agaataatga gaaagatttc ccatgcagtg tttcgataat tttcttttgt 660

cagaaatacc tggaaaaata ctacaacctg aagaatgatg ggaggcaagt tgaaaagcgg 720

agaaatagtg gcccagtggt tgaaaaattg aagcaaatgc aggaattctt tgggctgaaa 780

gtgactggga aaccagatgc tgaaaccctg aaggtgatga agcagcccag atgtggagtg 840

cctgatgtgg ctcagtttgt cctcactgag gggaaccctc gctgggagca aacacatctg 900

acctacaggt aggcagactg agggccagag tgagagagct atttcccatg acagtggtaa 960

gaagtcatgt ccaatgtgtc tctattttgt ttcattctaa ggattgaaaa ttacacgcca 1020

gatttgccaa gagcagatgt ggaccatgcc attgagaaag ccttccaact ctggagtaat 1080

gtcacacctc tgacattcac caaggtctct gagggtcaag cagacatcat gatatctttt 1140

gtcaggggag gtaagttctt cttatagcac cttaacctca ccttgcccct agatctcgta 1200

gctttgcaac tggaattgat tgtttaagat gaatgttgat tttatgggct cgaagaggga 1260

aactgcatca cagttgatgg aagtctgttg gcctcttaac aaagctaatg cttgcccttc 1320

tggcttagct tacataagaa ccacaaggaa tctttgttga attgtttctt tcagatcatc 1380

gggacaactc tccttttgat ggacctggag gaaatcttgc tcatgctttt caaccaggcc 1440

caggtattgg aggggatgct cattttgatg aagatgaaag gtggaccaac aatttcagag 1500

gtaagccaat caaatttgcc tctctcaatt ctcccacccc ttcatgtttc attgtaatat 1560

tggttgattt agttgaaaga atgcattcat agaagtatat atttttctat taccactcag 1620

aaaattagtg atacacttag aaaagtgaaa gagcggtctt ttctgctagc taacaacatc 1680

ttagcagtga caactaggcc actctaaaat ttttattaag aaaattgttg aaaactttga 1740

tgttttaatt gtgggcatca acattgaaac tttttcatta gaagaaaaca gtgaaattta 1800

acagcggtca taaacttatg tggaaattaa agaaaaggaa ggctttttgt gttttccaaa 1860

atttccagca gtcaacattt atcagttttg taaataagaa aaatattaaa tattaggctg 1920

ggcgcagtgg ctcatgcctg taatcccagc actttgggag gccaaggcag gcagatcaca 1980

acgtcaggag tttgacacca gcctgaccaa tatggtgaaa ccctgtctct tctaaaaata 2040

taaaaattag acaggcatgg tggcacacgt ctgtaatctc agctactcag gaggctgagg 2100

caggagaacc gcctgaaccc aggaggcaga ggttgcagtg agccaagatc atgccactgc 2160

cctccagcct gggtgacaga gcaagactcc gtctcaagaa agaaaaaaag aaaaagaaaa 2220

atattaaatg ttaatagaac attttgcttc tgtgtgaaat agtacgtgtc atttaatcat 2280

tgataatatt gattaatctg tggtgcgctt tcagtcagct gagtttgtct tttaattgta 2340

tcaatgtaat catagagttt aggtttcaac atgtctcttc gttattaata aatgcatgcc 2400

ttctgccttc ggaaaaaaga tatacatgca tatatttcaa aagtcactga taaaatagta 2460

acatgagtcc ttgtagttgt aaattattta ttagttaaat gcctcaaaac tgaatacctt 2520

tcaaataatt acaaattgat ttattggcct ttttagagta caacttacat cgtgttgcag 2580

ctcatgaact cggccattct cttggactct cccattctac tgatatcggg gctttgatgt 2640

accctagcta caccttcagt ggtgatgttc agctagctca ggatgacatt gatggcatcc 2700

aagccatata tggtgagtat ggggaaaaac attacggaca agggcctggt ggtctttata 2760

tgtatttcaa ataaaacagc cattagcgat atccttgaag aagctgtctt cattgtttat 2820

gtagttttct aatactaggc aaaactaact ctaggtattt tattttcctt aggacgttcc 2880

caaaatcctg tccagcccat cggcccacaa accccaaaag cgtgtgacag taagctaacc 2940

tttgatgcta taactacgat tcggggagaa gtgatgttct ttaaagacag gtaagatgtc 3000

agttcttctt tgtgatctgc atttgtaata ataacaacat tcaccactat gattaactat 3060

tatgcttcac atgttactta tttcatgctg atttacatct ataatcacat ggtagcaacc 3120

acatgcattt gtgcaacata atgtacttgt aacaactttg tttttctaca tgtattaggt 3180

caactgattc tcagaaaata tagttgattt tgaaaatcag tatgtatgat ctttttttac 3240

agattagcaa agtaagtcag agagaaggaa agagatcttt tgggggcata gagctcattg 3300

gtagcagagc agttattggt cttctgttcc ctatttcagg tctcttttca ttatgccact 3360

ctttatggtg ataagtatca ttggactttc ttaggagaaa gtggtccttt ccatagcaac 3420

atctcactga tgagggttgc atagccatcc tttaattatc tcccctgcca tcctggaaaa 3480

atggccctcc caaagcctat taacttgaag ggtctctgtt gccccctgga ggcccagaga 3540

agaagagaag agagggcacc aatgctggga aagtccccaa gaactgatga aatccctcct 3600

gggacagata ggggcagcca caacatggtg cttccctctt caaagaagaa tcctagaact 3660

ccctctgcag catgtggact caaaaagttg gggtcatctc acatgcaagg aggaggcctg 3720
ctctctgtac catagtgatt cctaaaggat caggcaaaat aataacacac tcttttggtg 3780
tgttattgca caccgcattt ggtgaatgat gcagtccaca gaaagatcaa cacatagaaa 3840
ttcagggtgg cctgaaaggc tcattctctg cagcctctca agtgctgtgg cactgtttta 3900
ctactctgta gtcgcatctc tcctagaatt cagggcactg gctcataatt ctttctgccc 3960
tctctttact ttttaagtgt aaaagataag gctggaggta aactatacag ggtgtggttc 4020
aatgctggga caagttaaga aaacacatgg ttttaaagga aacctctcag tacaacagga 4080
cacacctatc aaaccaaata ttactagatg gaacaggacc aatttgagga tttgccagaa 4140
ttgaaaggac acagagatca tttaaatgtc gccatgtcga ggagacatga aaggctagag 4200
atagagatag taactgattt gcctaaggca tcacagccag gcagaggaag gactggacct 4260
agaagtcagg tttccgacag ccaaggccgt aggcttccct gtggcctgga atctggagct 4320
cttctgtgtc agcccacata acagtctatg ctgtgctgtt accctagtcc ctattaagct 4380
ctataatcca ctttgtgtaa atactactaa ccaagggaca acaaagggat tggctgccag 4440
agcggagttt aaaaatcaac cttagcctct taccagctgg gtggccttag acacctccct 4500
tagtctcccc aacctcaatt tctcatcctc aaatactctc ttgtagtgtc cttgtgataa 4560
ggtctgatgt aaggaatggg cctggtgtgt tgtaagtaat cactaaataa taggatatta 4620
gagtcatggt ccagttggta atgtgcacag ccgatcccta atggctcacc aaagctgact 4680
gttaaatttt gaagaatttt gcaagctagc tgtgaaccat agccattatt aaaaattaaa 4740
ttataggcca ggtgcagtgg ctcacacttg taatcgcagc actttgggag gcggaggcgg 4800
gggaatcatt tgagatcagg agttcaagac cagcctggcc aatatggtga aaccctgtct 4860
ctactaaaag tacaaaaatt agctgggcgt gttggcctgt acctgtaatc ccagctactc 4920
aggaggctga ggcaggagaa tccttgaatc tgggaggcag aggttgctct gagccaagat 4980
tgtaccacta cactccagcc tgggtgagag agtgagactc tgtctcaaaa aagtaaataa 5040
ataaaaaaga gataaaactt aaattatata aacaaaatta aattacatta aaataaaagg 5100
taataaaaat aataaaaatt catcagtttg tcataatttt tactgcattt ttctacttat 5160
ttatgttctt aaagttactt atgcctattt tatttgtatg ctatacaatg gtgctccact 5220
gagactcttg aaattcatca taatgggagt atttcaagct agtgaaatca gcaaatgcta 5280
taaaccaacc cttctttgga gagctgaatg ttatacattt accagcacac cactagtgcc 5340
agcaacaaga aggagctgga gctggatatc tgccagagat taccagaaat tagatggaaa 5400
cttttcttgc tttttttcta atcagattct acatgcgcac aaatcccttc tacccggaag 5460
ttgagctcaa tttcatttct gttttctggc cacaactgcc aaatgggctt gaagctgctt 5520

acgaatttgc cgacagagat gaagtccggt ttttcaaagg taatctttct aattattttt 5580

cactgcttta ataaatcaat ttttcattcc ctttggggct gagatataag gttactagct 5640

acattctctc cagggacttt tgaaaatctc agtctcacca accaaatcaa tgaaagtcca 5700

tcaacttaac taaacggtgc gtagcgcatt ttggagtcat tttaaacaaa aggcaaatga 5760

ctggagatag aacgttctgg cctttttaat gaatctagat ttacaaaaat attttctgca 5820

caaactatgt atttaaaacc ccctccatat tcctgggagt actcaaagta aaactcgaga 5880

cactatgcca cgcaccaata tttttgcaaa cgtgtgttta acgtagtaca atgagtaaga 5940

catttgactc agccatacac tcaaggcaaa gaattaaatg caggtcagat gggccaaacg 6000

ctttgaaaca ggatttttag cctttaaatt atgaaattaa atggatgtta gagacagaaa 6060

gtttcaaatt cctttttcat gaggggtaaa tagcacgtgt gtacagtaac agaaatcttg 6120

gctttagtat atgagaccag ctttatgtga tgaggggctt actgagtata aacacggaat 6180

ctggacacag cattaattca agtatttgga actatcttct agtcaaatgc agtgatttga 6240

atccaggatt gcaaaccctt acaggttatg agtcactaca gcacttcacc ctttatgata 6300

caaatggttt gaacttccac agtccatgag caaacctgga gaaccgaagt ttatatcacc 6360

aaggtggcat aactaactct ttagtcattt gaacatctga ctgtggaaag aagcccccaa 6420

ctccataaac aagtcattct caaagcttgc tgcagattag aatcaactaa gaagcttcca 6480

aagatagaca catttaattg gtctgggata gggtcctggc atcagtattt tcaaaaatct 6540

ccctagatga ttctaatctg cagccagggt actgcactag catgtgaaac taagaactta 6600

gctgaaaatt cacttactgc ctattgaagt cttaggcaag tgttttatca ctctttcttg 6660

attggcaggg aataagtact gggctgttca gggacagaat gtgctacacg gataccccaa 6720

ggacatctac agctcctttg gcttccctag aactgtgaag catatcgatg ctgctctttc 6780

tgaggaaaac actggaaaaa cctacttctt tgttgctaac aaatactgga ggtaagttta 6840

agtgcagaga atgttagggc ctcagttctt tctacataaa aagttctaga atagcccatt 6900

tcaaagtgag tctcaaactg ccttggccgg catcctttcc ttcagatggc tggtgccaat 6960

ctatcatcta tttagtctat tgaccaataa tcatcacaca atgcctgcac tgggaatcca 7020

gagaatgtag gggatgaat tttaaaagga tgtagaattg aggacattca tcttaacgga 7080

gctactttgg ccaaaagtta cttatcttcc atagtatgtt acctcgatag gacatagcgt 7140

agaacctact ggagaattcg atgctggatt tcagaggggc cttaaaggcc ttttagttca 7200

cccaacattc caccttatcc catccactcc ccatttcaca aatgatctat aaaatgaaat 7260

attctggtta aattcctcat tgtgtatttt ctgtaatgat tttcagagtg cacatgttta 7320

gcaaaaggtc tgacactctt aactcttttc tgaccaggta tgatgaatat aaacgatcta 7380

tggatccagg ttatcccaaa atgatagcac atgactttcc tggaattggc cacaaagttg 7440

atgcagtttt catgaaagat ggtaagtgaa tatacatgta tttcctccat ttcattgccg 7500
tagcaataac atcattattg ttagttggaa cagcaaatgc caaaaatatc tgtgctcaca 7560
gatgaattgg tttctcaaga attcaagtta aacctagaaa ctacttgtat aatgtagtaa 7620
gtctcttttg ttttgtttct aggatttttc tatttctttc atggaacaag acaatacaaa 7680
tttgatccta aaacgaagag aattttgact ctccagaaag ctaatagctg gttcaactgc 7740
aggaaaaatt gaacattact aatttgaatg gaaaacacat ggtgtgagtc caaagaaggt 7800
gttttcctga agaactgtct attttctcag tcatttttaa cctctagagt cactgataca 7860
cagaatataa tcttatttat acctcagttt gcatattttt ttactattta gaatgtagcc 7920
ctttttgtac tgatataatt tagttccaca aatggtgggt acaaaaagtc aagtttgtgg 7980
cttatggatt catataggcc agagttgcaa agatcttttc cagagtatgc aactctgacg 8040
ttgatcccag agagcagctt cagtgacaaa catatccttt caagacagaa agagacagga 8100
gacatgagtc tttgccggag gaaaagcagc tcaagaacac atgtgcagtc actggtgtca 8160
ccctggatag gcaagggata actcttctaa cacaaaataa gtgttttatg tttggaataa 8220
agtcaacctt gtttctactg tttt 8244

<210> 51
<211> 23
<212> DNA
<213> Artificial

<220>
<223> Synthesis

<220>
<221> misc_signal
<222> (1)..(23)

<400> 51
acacctctga cattcaccaa ggt 23

<210> 52
<211> 20
<212> DNA
<213> Artificial

<220>
<223> Synthesis

<220>
<221> misc_signal
<222> (1)..(20)

<400> 52
ccgatgatct cccctgacaa 20

<210> 53
<211> 3546
<212> DNA
<213> homo sapiens

<400> 53
gcggctgccc tcccttgttt ccgctgcatc cagacttcct caggcggtgg ctggaggctg 60

cgcatctggg gctttaaaca tacaaaggga ttgccaggac ctgcggcggc ggcggcggcg 120

gcgggggctg gggcgcgggg gccggaccat gagccgctga gccgggcaaa ccccaggcca 180

ccgagccagc ggaccctcgg agcgcagccc tgcgccgcgg agcaggctcc aaccaggcgg 240

cgaggcggcc acacgcaccg agccagcgac ccccgggcga cgcgcggggc cagggagcgc 300

tacgatggag gcgctaatgg cccggggcgc gctcacgggt cccctgaggg cgctctgtct 360

cctgggctgc ctgctgagcc acgccgccgc cgcgccgtcg cccatcatca agttccccgg 420

cgatgtcgcc cccaaaacgg acaaagagtt ggcagtgcaa tacctgaaca ccttctatgg 480

ctgccccaag gagagctgca acctgtttgt gctgaaggac acactaaaga agatgcagaa 540

gttctttgga ctgccccaga caggtgatct tgaccagaat accatcgaga ccatgcggaa 600

gccacgctgc ggcaacccag atgtggccaa ctacaacttc ttccctcgca agcccaagtg 660

ggacaagaac cagatcacat acaggatcat tggctacaca cctgatctgg acccagagac 720

agtggatgat gcctttgctc gtgccttcca agtctggagc gatgtgaccc cactgcggtt 780

ttctcgaatc catgatggag aggcagacat catgatcaac tttggccgct gggagcatgg 840

cgatggatac ccctttgacg gtaaggacgg actcctggct catgccttcg ccccaggcac 900

tggtgttggg ggagactccc attttgatga cgatgagcta tggaccttgg gagaaggcca 960

agtggtccgt gtgaagtatg ggaacgccga tggggagtac tgcaagttcc ccttcttgtt 1020

caatggcaag gagtacaaca gctgcactga taccggccgc agcgatggct tcctctggtg 1080

ctccaccacc tacaactttg agaaggatgg caagtacggc ttctgtcccc atgaagccct 1140

gttcaccatg ggcggcaacg ctgaaggaca gccctgcaag tttccattcc gcttccaggg 1200

cacatcctat gacagctgca ccactgaggg ccgcacggat ggctaccgct ggtgcggcac 1260

cactgaggac tacgaccgcg acaagaagta tggcttctgc cctgagaccg ccatgtccac 1320

tgttggtggg aactcagaag gtgccccctg tgtcttcccc ttcactttcc tgggcaacaa 1380

atatgagagc tgcaccagcg ccggccgcag tgacggaaag atgtggtgtg cgaccacagc 1440

caactacgat gatgaccgca agtggggctt ctgccctgac caagggtaca gcctgttcct 1500

cgtggcagcc cacgagtttg gccacgccat ggggctggag cactcccaag accctggggc 1560

cctgatggca cccatttaca cctacaccaa gaacttccgt ctgtcccagg atgacatcaa 1620

gggcattcag gagctctatg gggcctctcc tgacattgac cttggcaccg gccccacccc 1680

```
cacgctgggc cctgtcactc ctgagatctg caaacaggac attgtatttg atggcatcgc   1740
tcagatccgt ggtgagatct tcttcttcaa ggaccggttc atttggcgga ctgtgacgcc   1800
acgtgacaag cccatggggc ccctgctggt ggccacattc tggcctgagc tcccggaaaa   1860
gattgatgcg gtatacgagg ccccacagga ggagaaggct gtgttctttg cagggaatga   1920
atactggatc tactcagcca gcaccctgga gcgagggtac cccaagccac tgaccagcct   1980
gggactgccc cctgatgtcc agcgagtgga tgccgccttt aactggagca aaaacaagaa   2040
gacatacatc tttgctggag acaaattctg gagatacaat gaggtgaaga agaaaatgga   2100
tcctggcttc cccaagctca tcgcagatgc ctggaatgcc atccccgata acctggatgc   2160
cgtcgtggac ctgcagggcg gcggtcacag ctacttcttc aagggtgcct attacctgaa   2220
gctggagaac caaagtctga agagcgtgaa gtttggaagc atcaaatccg actggctagg   2280
ctgctgagct ggccctggct cccacaggcc cttcctctcc actgccttcg atacaccggg   2340
cctggagaac tagagaagga cccggagggg cctggcagcc gtgccttcag ctctacagct   2400
aatcagcatt ctcactccta cctggtaatt taagattcca gagagtggct cctcccggtg   2460
cccaagaata gatgctgact gtactcctcc caggcgcccc ttccccctcc aatcccacca   2520
accctcagag ccacccctaa agagatactt tgatattttc aacgcagccc tgctttgggc   2580
tgccctggtg ctgccacact tcaggctctt ctcctttcac aaccttctgt ggctcacaga   2640
acccttggag ccaatggaga ctgtctcaag agggcactgg tggcccgaca gcctggcaca   2700
gggcagtggg acagggcatg gccaggtggc cactccagac ccctggcttt tcactgctgg   2760
ctgccttaga acctttctta cattagcagt ttgctttgta tgcactttgt tttttctttt   2820
gggtcttgtt ttttttttcc acttagaaat tgcatttcct gacagaagga ctcaggttgt   2880
ctgaagtcac tgcacagtgc atctcagccc acatagtgat ggttcccctg ttcactctac   2940
ttagcatgtc cctaccgagt ctcttctcca ctggatggag gaaaaccaag ccgtggcttc   3000
ccgctcagcc ctccctgccc ctcccttcaa ccattcccca tgggaaatgt caacaagtat   3060
gaataaagac acctactgag tggccgtgtt tgccatctgt tttagcagag cctagacaag   3120
ggccacagac ccagccagaa gcggaaactt aaaaagtccg aatctctgct ccctgcaggg   3180
cacaggtgat ggtgtctgct ggaaaggtca gagcttccaa agtaaacagc aagagaacct   3240
cagggagagt aagctctagt ccctctgtcc tgtagaaaga gccctgaaga atcagcaatt   3300
ttgttgcttt attgtggcat ctgttcgagg tttgcttcct ctttaagtct gtttcttcat   3360
tagcaatcat atcagtttta atgctactac taacaatgaa cagtaacaat aatatccccc   3420
tcaattaata gagtgctttc tatgtgcaag gcacttttca cgtgtcacct attttaacct   3480
ttccaaccac ataaataaaa aaggccatta ttagttgaat cttattgatg aagagaaaaa   3540
aaaaaa                                                              3546
```

<210> 54
<211> 27049
<212> DNA
<213> homo sapiens

<400> 54
tgtttccgct gcatccagac ttcctcaggc ggtggctgga ggctgcgcat ctggggcttt 60

aaacatacaa agggattgcc aggacctgcg gcggcggcgg cggcggcggg ggctggggcg 120

cgggggccgg accatgagcc gctgagccgg gcaaacccca ggccaccgag ccagcggacc 180

ctcggagcgc agccctgcgc cgcggagcag gctccaacca ggcggcgagg cggccacacg 240

caccgagcca gcgacccccg ggcgacgcgc ggggccaggg agcgctacga tggaggcgct 300

aatggcccgg ggcgcgctca cgggtcccct gagggcgctc tgtctcctgg gctgcctgct 360

gagccacgcc gccgccgcgc cgtcgcccat catcaagttc cccggcgatg tcgcccccaa 420

aacggacaaa gagttggcag tggtgagttg ctgcgctggc ctcaaggaac cacgtttaga 480

caaacttcgg aggcaaagga tggggtgtc tctcccccctg ccctcggcgg tgggcacaca 540

gcgtggggga ggggcttcgg taaacagctt gggggtctt tggcaagcta ttggagtgat 600

ctcttgcaaa cggtggggga tcttttgtaa gcaaagaaaa cctttgataa acaatttggc 660

aaccttcagc atacagcagt ggggcgaaaa acaagccaga gagtgggaga ggggtgacct 720

ggcaaactac tggaaaggga ctcttatgta aatagcgggg acatcctcta gtatctggga 780

catttggcgg gggggggggg cggtctttgt aaacaacttt tggacacatc tgggcagttg 840

ctaagggctc ttgccaagcg tctaggtaag cctttggcaa acagctacag gggtcgtttt 900

tgctaatgta ggactttggg aacggtgctc tggcacacaa tttgggtaaa cttatgcgcg 960

cataaggatg ggagggagct tggtcaaagc ggggcttggc aaatttctag gaaccttcgg 1020

cacagatccg gagagggacc tttaaacaaa cagctcgagg gggccatttg ggaagtgatt 1080

ggggcaggag ggaagcagag agcgcatctt ttttctcctg gcttaatgga caaattggtc 1140

aagggctggg cctcggaaag tttcctcgaa cttctccaaa gggtcggaga aaagaaggag 1200

agagctggcc cggcaggagg gaggaggagt ggggcaggcg ctggagggcc cggcgcgtgg 1260

ggcgggggcg gactgcgctc cgctcgggtc ggagagcggc cagagagccc tccttcctgg 1320

ctgggctccc aaaccgcggt tcagatgttg tcttgtgagc gtgcgcgcgc ctggctggag 1380

gggcactgag cctggccgca gtgttgcgta agtagggcct gaggagagag gcaggccagg 1440

gacccatcaa gatgtccttt ccagtccagg ggatatctgc tcccttgttg cttcagccac 1500

cctgccctcc agcctccccc tcaacctgtc cgggagaaga cgcccatgcc ctctggtcac 1560

cctaggaact ccttcaggag gtgacaacta ccctctccca actctcaccc ttccaaacag 1620

ctaggtctca gccccctctg gatagatggg ttgggaaacc tccatacatt tttagagggc 1680

tactttttc aaggatctgg agctggctgg cataatgatg tggctgttgg gtgggggtga 1740
gagtggagct ttgggacaat gacaaagcta cttggtttcc tacacctttt ccagccaatg 1800
ggtcacagga tgaaccccta aattggggtt cagcctgggg gccacatggg ttcttggctt 1860
gcacaggaag gaattcaaga gcgagccaac agagtaaagt gaaaacaaat ttattaagaa 1920
ggtaaaggaa taaaagggtg attattccat agacagagca gggaatgggc tgctggactg 1980
agtatacata tggttatttc ttgattatat gctaaacaag ggtggatta tttaagagtt 2040
ttccagaaaa agggtgggga gttcctggaa ccgagggttt ctctcccttt tagaccatat 2100
agggtaactt ctcaatgttg ctatggcatt tataaactgt cacagcgctg gtgggagtgt 2160
ctaatgtatt taacatgcta atacattata attagcatat aatgagcagt gaggacgact 2220
agaggtcact ttcattgcag tcttggtttg ggtgggcttt ctccagcttc tttactgcat 2280
cctgttttat catagggtc tttgtgaccc gaactgtata tggtgaaatc agtcctacca 2340
acctcctgtc tcattctgtg actaagaatg catgcctgcc ctcctgggaa tgaagcacag 2400
caggtctcag cctcatctta cccagccccc cactcaagat ggaggtgcct ggtttgaaca 2460
cctctgacaa atggaagtct gtgttgtcca gaggcaatgc agtgggggct taagaagata 2520
actctggact tagaccgctt ggcttcaaat caaagagtgc atgaaccaac cagctggcct 2580
agtgatgatg ttaggcaagt gacttctcag tttcttcatc tgcaaactgg gaaatttcct 2640
atctcagggt taaaagagag gtaatcttag gtgcttacct agcacatggt aagtactcaa 2700
taagcactag ccattattct attatattat tattattgtt atgtcctcca tttccccttt 2760
cctaactact tccaacaagg ctaggaccgt tgtcagaaga tctcctggct cctttcaaca 2820
ttctaacaca gtacttatca catacttcat tttgttttat ttgggatcta tcagcctccc 2880
tgctaggatt tgaaccttag agacagaatt gtctatcttt ttcacctgtt tttcctcagc 2940
agtagcactg ggcttggtac atggtaggta ttaaaaagta tttgttgaat aaataaatga 3000
gtcggttgct ccctgaacct ctgcagggac ctgctcagtt tgggcctcag tttgggcagg 3060
gccagttgta tgactcatga aggacaggac tactttcttt ttgcaaatgg ctgattcagg 3120
atttaaaccc aggtctgtct actgccagag cccatgcaca tagtccttga atctctttag 3180
aagaaaacag gaggaaaaaa gtccccaagt caccagccgc aaggtagata aatgagttga 3240
tagaggtaaa ggaaggatgc tggagtataa tgattccata tcacagcctc tggagccaga 3300
ttgctttgtc ccaaatccag atctaccatt tgtgaactgt gtgatcgtaa gcaagtttct 3360
taacctctct gtgcttcagt ttcctcctct gtataatagg gttagtatat ctacattaca 3420
gagttttgag cattaaatga gttaacatat tctgacttta gtgcctggac atatagtcag 3480
cacccagcac caacaagtgg tagccatgct ctctatagct gtatgtcctg tcgctcaact 3540

aatgggtgac cgtgctggtt tgggaaccca gtactccacc cagtgctctg ggacccctgg 3600
cttatttcct gtctggacta tggcactggg ttgggggct gattgctaca gcctgctttg 3660
gtcagtactg tgccatccta atgtggctaa ttgccttggg ggtgtgcatt tctttcagca 3720
atacctgaac accttctatg gctgccccaa ggagagctgc aacctgtttg tgctgaagga 3780
cacactaaag aagatgcaga agttctttgg actgccccag acaggtgatc ttgaccagaa 3840
taccatcgag accatgcgga agccacgctg cggcaaccca gatgtggcca actacaactt 3900
cttccctcgc aagcccaagt gggacaagaa ccagatcaca tacaggtgcc ggggcagggc 3960
ttggggaggc agggccatgg ggctgaggga cacgagtctc cttgacccat gcattctctc 4020
cactcagggg atccatgagg tgtcttttgt gaaggcttga catcttaggg agtttcaata 4080
cacagttctt agaaaatgaa gtcagacaga cttgagtttc atacttgctg gccatgagac 4140
attgggttac ttttcagtgc tgtgaatgtt ggctttccaa tctacaaaat ggggcttaaa 4200
aaatgccctt atatggtatg ttggggaact aagctaggct gaactaatac aaacggcttc 4260
tatgattttt gacacagagc aagagtgtag taatgctagt cattgttatt gtttttagat 4320
gaggtcattg ggatgggcag aaggaacgtg atgattggta gctaattggg tttagcagcc 4380
caattgggtt ataactggtt gctaattggg ccaagagctg tgctctgtat gggtggtgct 4440
gggttccaat ggtgggatct ttgccctgtg ccccataatg cactacttat ggccctcccc 4500
caccagccag aagtgacaga tggaactcct gagctggggc ctaaaaatgt tggctggcct 4560
gggactccct gggtcttcaa ggcattctgg gagggttgtc cttggcagtg agccctgccc 4620
agcctacaca cacatacacg caggcacatg ctcacatata catacacata cctgtgcacc 4680
cttcccgtgc ttgtgcatat acttgtatgt tcacatacac acacacactc aaactttttc 4740
atacatctgt gcacacacac atacttgcat atacatacac ttatgcacat gcatacacac 4800
tcacatgcag ttctaccacc tccaggatca ttggctacac acctgatctg gacccagaga 4860
cagtggatga tgcctttgct cgtgccttcc aagtctggag cgatgtgacc ccactgcggt 4920
tttctcgaat ccatgatgga gaggcagaca tcatgatcaa ctttggccgc tggggtaggc 4980
agaagatggg gcagaagagg ggccagcagg gatcagtgtt gagacgaggg ggtgagatgg 5040
acattagagg ggcgtgggga tcctaagggc ggcttagata ggacagtaga tggtgtgggc 5100
cctgggggtg gtttagatgg gggcagcaca acacggagtg gacactggag agccacgttg 5160
acatgggggc agatggtgtg ttgtggtata acctggaggc cgtctaaacc cgaagcaagt 5220
ggtctactgt gctgtccaat atggcagcca ttaggcagaa tatacatggc tgtttaaatt 5280
taaattagtt aaaaatttta aattagtttc ctggtcacac tgggcacatt ttaagtgctc 5340
aaaagacaca ggaggctagt gactattgat tttggacagc acagacaaag aatattcctc 5400
ttaatcacag aaagctctgt tggatgatgc tggtagacac tgaggaactg tagatatatg 5460

agagtggttt ggaaacagtg gagcagtgta gacactagag gaagggtcgt agaacctggg 5520
gccaggtggc catgtgagtt tatggtgtga acactgccag tagcacagac ataaaggtat 5580
gtggtataga cactgagtgg taagtgatga aggtaaagag caggggaaac aatgtagaca 5640
agtgggtggg tggtacaggc cttggtgaca aggtgggcat gaggattcat gttgcagata 5700
ttggtgatag tgtgacatga aggcaggatt gtggttaaga gggcagtatt gacaggatgg 5760
aggatggcat agacactggg gatgatgaag atggggtgag gacactagtg tggtaatgtg 5820
ggtatgtggt acatatagtg tcatggtggt agagtagaca atggaggtag agggtatgga 5880
tactgggagt catgtaaacc tgggagagca gtgtagacca taggatggta acaaccaggt 5940
cagcagatct ctactgagct cctgttgaca gtcctggata accccactgg gacaagggaa 6000
ggggacagat gctgggtggg ctgacaggct ccacatgtag atggggtgtg gaggggtttc 6060
agggtctagg tggcacagct agacgctaag acccagtgtg tgtttcagag catggcgatg 6120
gataccoctt tgacggtaag gacggactcc tggctcatgc cttcgcccca ggcactggtg 6180
ttgggggaga ctcccatttt gatgacgatg agctatggac cttgggagaa ggccaaggtg 6240
agaaaggggc cctctgcatg ccccagacct tctctcctgt cctctctcca ctccatttgc 6300
ttggaccaga gaggtgggag gggaggaaag tcacacatct gggtgagtca gaatcttggt 6360
ctccaaagaa ggcctggaga agtccaacct ccccctccca tgtcactctt tagtggtccg 6420
tgtgaagtat gggaacgccg atggggagta ctgcaagttc cccttcttgt tcaatggcaa 6480
ggagtacaac agctgcactg ataccggccg cagcgatggc ttcctctggt gctccaccac 6540
ctacaacttt gagaaggatg gcaagtacgg cttctgtccc catgaaggtg agcatccact 6600
ctagtcccca agactttcca ccccaagcct ccagcttccc gggctcccca gtgtgctctt 6660
ccctccacac tctccaggac tggctgccac tgccagttaa tgagcatccc tccaacgtcc 6720
ttcactcagt tccccccat cctgatctga gccattgctg tttctctccc accagtacca 6780
tgatgaacgt agtactctaa ttaaatcaag ttgatttttt ttaagtttca ccattagcaa 6840
tcatttagga agtcacagat ttgatttgct aattatttat ttctaatata tattggagtg 6900
aacttagaac tattacaatg cccagtggtt ctctctagaa ccagtcttta cacttcagtc 6960
aagattgata cttcaagggc atgaggaaag gcttgctggt agctggaaac ctagcagtgt 7020
gatagggagg caggactcag accagggctg gactcaacct ggtttgcgta ctccaactga 7080
ggaatttcag ctattgctcc tgccttggtc ctgatgctgc ctctgctaat gcgtgtgtgt 7140
gtgtgtgtgt gtgtgcctgt gtgtgtgtgt gtgtgtgtgt gtgtgtgtgt gtgtgtgtgt 7200
ctgggcacgg gggctatact atctgcctct cttgggcact gtctttttct gcctcctgtc 7260
tagcttgtgt ctgcatctct gtgtcttgat aaagacttta ggcttgactc agtatttgtg 7320

tttgtgtgct tgtcagtgcc tgagatgcaa catttcagag attttgaagc cacatggggg 7380

aaatctttta aaatggggta cagatattga catacacact caatcctttt taaaaatatc 7440

cttgacacac ttaatagtca taaatcacaa aaagacaatg taacttcata ctctatgaag 7500

tcatcatatt tgataattct gcagattgaa agcccagaaa acctggcgaa aatggaaaaa 7560

agtctaaaaa tttttaacac tatcttcttt cccatgtcac aaagactcta tagtctacat 7620

agactaaaga ctgtcttttt gtctgtaatt ttatgtctgt gaatctccct aaccccactg 7680

taactgaaaa ctaagcacca accatcttga cagaatgaaa gaaacaaaga gctagttaat 7740

tcccttcctg tttatgcttt tactaccaga gctaaacttt atattaagta agagtttaag 7800

ccattcacca ttaataagtc agacattttg aaataaaaat attttttgta tcttgcgttt 7860

cccggtaggg attacatgtg gcgttaaccc ttccctgtgt cacccctctg ataggaacag 7920

aaagctttgg tgatagttag atgtggaaac agctgtattt cataaatttg ggaagggtta 7980

ttccctgcat tggacattag ccatttactt tgtgcttagc cagatgcaag tacgcacaac 8040

acgcacgtgt ctgggtctcg aggcagagat tgttgttact cagcccctgc tgatggctga 8100

gtcagtgtct gattcttgcc atccttgtca ggggaatttg ggaggagaca ggggcttcct 8160

gggtggtaga aatgagaggt cagaggcagg gggctggcat tggtcgaagt taacgctttg 8220

cctttctcaa ctctgaggcc actaaggtat ctggactcag tcttgccttt tctctaggtg 8280

ccattgtttc tggcattgca ggacaaggtc ttttggtaga gaggggagtg gtgaggatac 8340

agggcctcga gttctgtggt gggaggccag ggactgggct ccattccggc catggtggag 8400

ttaacggggg ctggtgaggt cacctctggc gccgttgtgc acagatggtc cttgttatgg 8460

acttgtggtt ttgctggttg gcaattccag agacaccatt ttttagcttt gggtggatga 8520

ggggaggatg gggagaatcc aagacagaca ggccaggctc tgaaccccac tccctccccc 8580

aactctccat ttatggcccc tgtcttgaag atgtcaaagt aggagcccag agtgtttagt 8640

aaaagggacc tgaaggcacc acctccctct ccctccctcc tcctcttcat tctttctctc 8700

cttcctacag agactgcaac aaggccatcc aggaaggggc ctggggttga caggaggtaa 8760

agcaagcttt ctgtgtatgc gtgtgcacgc acacacacat atatgagagt gacagactaa 8820

gcaagagaga ttgagattag tgtgttttgt agtttttagt tacaaccttc attcacttct 8880

ttcatttctg tgaccatgag cagtttgaga accatctttg tcctagcctt gatttctcca 8940

cctatcaaat ggagagagat tctcgtggtc ctgcctgcca tgactgatat attcatgtca 9000

tacatcatta ttacatgaaa tttgcatggt acaggtgtgg accaggcaga atggacagtg 9060

ccccttgggc tgaggcaggg ttctagaagc ccttgctgtg tgccaggtgt caatcatttt 9120

gttgttgggt ggtccacaag gtcccacggg agggattggg ataaccaggg aagcaggggt 9180

tctctctggg agctcagaga agcagctcct taccaaccag tagagatgtt ttcttaatat 9240

tttaacaatg catcaacttc actggtgtga accggcaggt gggcagccag ccagccctgt 9300
gcccatggaa gcatgtctca ttcacatcct tccctctctc ccccaccctt agccctgttc 9360
accatgggcg gcaacgctga aggacagccc tgcaagtttc cattccgctt ccagggcaca 9420
tcctatgaca gctgcaccac tgagggccgc acggatggct accgctggtg cggcaccact 9480
gaggactacg accgcgacaa gaagtatggc ttctgccctg agaccggtgg gtgccactcc 9540
ctctccctcc ctcagggccc agcacctgct gtctgacaaa aaaaaaaccc ataagactcc 9600
gagtgcccac ctcctttccc caagacaggg gtgctaagac atctgtgcga atgacatcca 9660
caccaagatg tccgtgtagc tagtcaggat acttgtcacc tggtatggcc tgggcactga 9720
aatcagaaca gacattgggc atcaacagtt gaattggtga cacttaaatg tcaggcctgc 9780
tcaggatcct ctgaccgacc tgcatccctt ttctctcagg ctctctggca gagacttctc 9840
tctcctcctc tctgaccctg caatctctct tccctgcctt tcctggtctg tatctccacc 9900
ttttagtctg ttttttatta agagctccgt gctttcctcc ctgtctcctt cctgccctcc 9960
tctctatccc tccctccttt ccttcttccc ttcctccaac aggaatttac tgagcgcctg 10020
ctttgtgcag catacacaga ggttggtctc tgcaaagccc tgcccaaggg ggctcacagc 10080
tgaggggcat gtgctggtgc catcagcttc atccaactcc ctttgcccct agcccatctg 10140
ccccccttcca tgggaggctc attagaactc tggaatctgg cttcctgggt ttgaattgca 10200
gctctgatac ttcctagcag tgttggctta gggaaatttc atcatctctc tgatctattt 10260
cccatccatg aaacgcggtg ctattcagtc cctcctgcac aaggctgttg tcaggggtgg 10320
gtgagatgag tctaaagata cagtgcctgg gactgagtct aacttaggtg tggttcatta 10380
aggtcagcgt catgtcattg cttcctggtg gtagcctcag actctttgct gcgccttgac 10440
ccgtatccct aaccccacag ccatgtccac tgttggtggg aactcagaag gtgccccctg 10500
tgtcttcccc ttcactttcc tgggcaacaa atatgagagc tgcaccagcg ccggccgcag 10560
tgacggaaag atgtggtgtg cgaccacagc caactacgat gatgaccgca agtggggctt 10620
ctgccctgac caaggtacga ggccctggtc attggacaga gaccctggac attgcccttg 10680
cccctaaact tgctccaaaa accttcctga gacctcaccc acttcaagca tagacactgc 10740
cccccagaca cccacctacc cagacccgcc cacctgggct gagacaatgc ccatctctgg 10800
tggagccaag gtccctgctt agatcctgcc catccaggtg gaggcacagc ctccaaaatc 10860
agaccctggt agaccagatg ctgctactca cctcctgggt aagaactggc cttcctgagg 10920
ggtcactgct cttgtcttcc ccttcagcca ccctccaggc gggcttgtga ctaccaaaat 10980
tggctgaatt ctgagcacag attaccagag agacaattct cctaagagac attcccactg 11040
cacaaagagg tgccttcaag gagctggtag gcagtagaag aaaggaaaaa aggaagagcc 11100

tcatgcttga tctgacacca tggtccacgc agccctgggg gagcccccag agccctctct 11160
cccccatcag tcagcaccat ctgttaccag ggtgtaaatg gtgggtcctc gtcagtcctg 11220
ggtgctgagg atggagctga ctgggacagc tctctgcccc ctaggactca caagctcagg 11280
ggagagagtg accaggagac agcttgtgac cttactatgt gctcagtgca gtgagcaggg 11340
tgcttggggc cctgtgccca atctaggcta cagagcccag ggttgcaggg ttgcttaaag 11400
ctccctgatg gcccataatg gccaggtcag tgggcaggtc cagggcctgt cagaattagg 11460
caagcctgtc tttatgttgc tttcattctt tttaatcatc atgagatgga acttggcaga 11520
tgtcagtgga cagaggctgg ccaaaggagg ttttcttatc ccctgctaaa ttccttgcta 11580
ggacacagtc tgattttacc agcttaaagg tgggattctc caccttcctg agtggttcaa 11640
gtatccagag ctcagtggct cacccagggt gaatattata gtctcctggg gctgccatga 11700
caaagtgcca caaactgatg gcttgaaaca aaccccatgt attctttcat gcttctggag 11760
gctggaagtc taaaatcacg gcgtcagtaa ggccatgttc cctctgaagg atgcgtcact 11820
ccagtctatg cctctgtcat gacatggatg gccttcttcc ccctctgctt gcctctcctc 11880
cttttttttt tttttgagac ggagtcttgc tcttgcccag ctggagtgca gtggcatgat 11940
ctcggctcac tgcaacctct gcctcctggg ttcaagtgat tctcctgcct atttccaaat 12000
aagttcacat tcacaggtcc ctagggttaa gacttgggca aatcttttct gggggggacgc 12060
atttaaccac aacagggtgt gacacaggtt ttgctcgctg gctgtggctc agcctgtgca 12120
accacgctaa gtgtgaaagg tttttactgc tttcacctgg gcagaaatgg tggcaacatg 12180
gggccagcct atccccactc ttcttacctc ttcagagagg ctgaggagct gggtttgtag 12240
agctcatctt ttatagagag acataaaaag ccaggaagca ggattttaaa atgaatgggg 12300
ttttaagtag aaaattcgca gatactactt gttctgttcc tttatagcac acctgtcctg 12360
atactgactt ggaaagagac aagaagactt tggctgaccc cacttggaaa caataggcac 12420
taatcccaaa attgtgtatt gatcccagaa ttctcaggaa aagaaaaaaa aaatcaatac 12480
atgccgcttc cagggttctc agccttactg tggggctgtc ctcaaaagtc tgcatcactg 12540
ggtcaggtct tgacttctct ctcatctctc ttcctgtctt tcaaccctct ctcctccctg 12600
caaccctcag ggtacagcct gttcctcgtg gcagcccacg agtttggcca cgccatgggg 12660
ctggagcact cccaagaccc tggggccctg atggcaccca tttacaccta caccaagaac 12720
ttccgtctgt cccaggatga catcaagggc attcaggagc tctatggtaa acctccgggc 12780
gggggttggg ggtggagggt gaggaggggg gaggtcatgt agcctgggat ggaggcccag 12840
ggggtgggac cagcaagatc tcatccagcc acgagtgctg gagacgaggg caggaaatgg 12900
gagtgttgac ctggtcttgg gaaggcagag cagctctcca aggggatact tggataactc 12960
ttccctatcc gaacaatgct aatatccatg tcactcttag cgctccattt ctgggtttct 13020

cccattttct aggtgagtaa ggtcacctgg tgcaaggtta cacaggtttg agcaacccag 13080
tggagtttag aatccaggag aatagccaag tacaggacag aattttccat catgtggaat 13140
gaccttaaca atacgggggt ggaagtttct agttttcata ataagctttg atttttagta 13200
tgtagtagaa acagaacaac cagtgcctta agccaattat ttatttattt atttatttat 13260
ttatttattt atttatttat ttattgttgg ttcttacagt gaggctagag tttaaaaagt 13320
cagttcattt ttaaaaaata tttaatggtc caggtgatat ctgagccagg ccaaaatagt 13380
gaaagtggtg cttgagtgat gaagtttggg gactgctaga ctcaggaaaa agcccaaggt 13440
ctgccgtgag ggtgctgggg ttcaaatgtg gcctctgccc ttcactagct gcaagatatt 13500
gtgtaagtct ttatacctct ttggactcag tttcctcatc tctaaaataa ttgcttatca 13560
tgaaaagtgc catactgtca gttctggaca caccagcggc cccctgaccg ccctgactct 13620
tgtgggcata agaatctgag ctttgggttt gaatccttgg gcaagtcact caacttttct 13680
gggcctcagt ttcctcacct gtgaaatggt gcaatcatga taccccactc ccagggctat 13740
agagaggact gatttgggtg atgtcagtaa aaagcatata taaagtacaa agggccccag 13800
gactccccaa gtccaggcat cttcttgtta ccttacggag cttacactaa ggccagaagg 13860
cgatttcttc tgactcttag atggttgggt gggcacccct gggggctcac cctagtgggg 13920
agaacctctg gagctgcaga gagtctaagg tcaggtgttc tccccagggg cctctcctga 13980
cattgacctt ggcaccggcc ccaccccccac gctgggccct gtcactcctg agatctgcaa 14040
acaggacatt gtatttgatg gcatcgctca gatccgtggt gagatcttct tcttcaagga 14100
ccggtgagtg caggagcttg cttcttgtcc tccttgtctc ctgtcctctg ctcttatacc 14160
attattcttt tccctcactc ttcgctgaag actccgccaa atgcttccca gaatgacctg 14220
aattaggcag tttctgctgt gtatcaaaca acctccaggt ttcagtggat ttgcacccaa 14280
gcattttgtt tgctctctca tagtctgtgg gtctactagt tatcttgact ttcagctaca 14340
gtttgggttc aggtgtgctc tgcctgttgt tctcatggga ttagcagcta ctcatggcag 14400
atgacagggt gcaagaggcc aaactgaact acacaagctc atttaaagcc tccactcaca 14460
tcttgtctga gaatattcca ctgcctgaaa taagtcacat ggccaatccc aagacctacc 14520
ctagagggta gtactgcaaa gtcacatgga aatgcatatg aatgcataat cctaaaatca 14580
aaatggaggg aagaattggg aaccataatt cagtctacca caggccttct agaatggcgt 14640
tgccctcaaa tggccaaatt actctgtgta tgggtggtga gtgactgcag agtagaaaga 14700
ggtggtattc ttcatgctat agataaagat caccactcag ttgactaaag actgaggttt 14760
accctaatgc cctagttccc tggagaatgg caggcagtat ctaggacctc attaaagggt 14820
ctgtccttcc attcttctat catccatcca tctacccatc catccatcca tacctatatc 14880

tttccatcca tcatccatcc agccagccag ccatctatta tccattattt atgatccagc 14940

catccattat tcattattca tccatttatt cattcataga gtatagtagt tgagaaaatg 15000

gactctgaag ccagattctc tggcttagaa ttctgtaact gctacttact ggttatgtaa 15060

tcatagacaa gttaattaag tatcttcttt gtaccttggt ttcctcattt ataaaatggg 15120

gctaatattc caacctatct cctagggctg ttgtaaggat tagatgaact tagtgctgag 15180

aacaaaaaca atgcctgaca cagagtaagt gtattcatat tagctattgt tattgacaaa 15240

taattgtttg gcatctactg tgccaaatac tttgctaggc tttagtagct ggaggaggaa 15300

gaatgatgtt ctcgggacag tggggtgggg ggctgctaga tcagtgttct caaagacagg 15360

cgcatgggcc tcctataaca gaaccatcta gagtgtgggt attaaaaggt gggttttctg 15420

ccccagcttc agaccctcct gactggaact ctggggctag gccccaggaa tctgcgtttt 15480

agcaaattct cccagtgatt ctgatgcaca ttaaagtttg tgcatcacaa gtccagtttg 15540

agaaataggc atggagaact gacatggcaa gatgaaagaa aagtgatcgc agaggtgtta 15600

ggtgcttttg tgagcacgtc ccagtcaagg aaggcttcac agatgcagca gcatttgagc 15660

agagccttca aaggagaagc aagaatctga tgggggtgac aagttaggag gttctttgag 15720

acagagggac ctgcagagcc aagccatagt gatgtgaaag tttctggatt agccagtgag 15780

aatctggtga ggctagaaga tgttccataa actttcagga agagtttcct ctactctagg 15840

cactgggcca aattctgggg tgcagatgcc ttgcccttca cagggtctgc tggccaatgg 15900

ggaaatggca agaatgcaat gagagatgag acttagaatg tagtgagaaa tcaggtcccg 15960

tcaggcctcg aatgcccaaa tcaggatata taacagcaga tgtcagcact gctctgcagt 16020

tcccattgta aggatctggc aatgtgcttc atctggagct cctcccacta agaaagccct 16080

gaccacacat gcagatctca gatgtgtggg gcctaggaaa ggatgcagag cccagaccca 16140

ggctggagcc atccctggcc taccacatca cagttttctt tcaaaaggaa cgtgatgagt 16200

gtttaatggg tttatttgat taaccagtaa atattaaact tcaaccatga gcaaaaattg 16260

ctgctaggca ctggtgaggg tgggggtcat gaaggacagt attgcagggt gattaaactc 16320

agactttgga gtcagatctg gatatgaatc ttggtaataa ctaatttatt tagtcattga 16380

tgtagtgata actaagtaat ttgaatccta acctctctga gcttcatttg ctaaataaaa 16440

attgtgacac cttcctacgc taggatgttt ttctgcaaat aacagagacc atccctctgc 16500

aaactcaatc cagcctaaac agtagagaga aattgctttc atttggtaca aagtccaggg 16560

gtagagctgc ctgcaggggc agagagaggg tcagtgactt gatgtctttg tccactgcta 16620

gcctctgcac ttacttcatc ctaaggccgg ttctacctca tggtcacaag aaggttacca 16680

gtggcagttg gggctctaat ccctgccctc tcccaggaag cgagagagaa agagacacgc 16740

acagatacac acacacagac ccccagatgt gaaatacaag tctgtgtgtt ccgtctttac 16800

accactgtaa gtcatatggc aactgctgaa tcagtaacag tgctagggaa tgctaaactc 16860
tgattggctt agaccagtag ttctcaaacc ttggcatgcg ttagaatcat ctggaaggct 16920
tgttaaaaca cagattctgg gccccaagcc cagagattct tatctgtaga tctctgtaag 16980
gacccaatca tttgcatttc taatgagttc tcaggtgacg cagatgctgc tggtcaggag 17040
actgcacttt gagaaccagt ggcttatacc aatcagtatc tacccctaga atgggggtag 17100
gggacacagg gtcagaggag gtgaaggctg gatcataaat gtggagtttt gtgaagaagg 17160
atggtgggga aatagataat taatgctagg tagaccatcc agagtgcatg ctgtcttaaa 17220
agattgtggg gattaaatta caaggcacat aaataaagca catggcattt agtaggaccc 17280
tcaataaatg ataactctta ctcttgacaa agtacacaaa ttcatttcat tttaattcag 17340
gaaatacttg agtgtcatca gctcctgccc caattggcct ctaatcaaat aggagagtta 17400
agacaaacag acactactgt tccaggcact cagagttgat gattttgaga ggcttagaga 17460
gggaaaacac aatatggatg gaggtagtca gggaaggctt cctggaggag gtagagggag 17520
agaacagggt aggaggatgt ttctcagctc cagcagattg cagacaggaa ctctgcccgt 17580
tctccagtcc ttctccaacc ttcctttgat cctgcctccc actcccatca tggaatcatc 17640
tctgggatgt ttctgggtgg gtttgggggt gtgtgtggtt cgagctgcag ggtgactgaa 17700
gatgtggttt cctgtgcccc cttgcctcct gccaggttca tttggcggac tgtgacgcca 17760
cgtgacaagc ccatggggcc cctgctggtg gccacattct ggcctgagct cccggaaaag 17820
attgatgcgg tatacgaggc cccacaggag gagaaggctg tgttctttgc aggtgtgtgg 17880
gaagcaccct tccttggccc tcagctccac agggctctgc accagggctc ctgggtgtcc 17940
caggctcact ccccctctca aaccacagtt cctatgcacc cgtgggtgct ccctttcctt 18000
tatggattca ttctttcaac attatttcct gaagtgcgca gctcctctgg gagattggtt 18060
ccgacaccca acatcagata gcattaaccc tcagtgtaca catttcattt tcttaattct 18120
tttcaatccg tctcatttct tttctagaag aaaaaaagtc tcagtttagt gctggtgtgt 18180
ccctaacgcc tttgtagtga tacagtcttt ggcaggtaac agttttagtt cgaatgtttt 18240
aaagaatatt tgttttcatt tttactttta gacataaatt ggttttaaca gaggcagtta 18300
gttataatag tgtggagttt caaatcagga cagaaacgga ttattcagta actgatattt 18360
tgacagtcaa aaaacatttt ttaataaatt tatttttaaa gatttcaaaa tgaattcact 18420
gaaagcagaa tgttaagtaa ataatagtac agggagcccc tagaagtggt agaaatcatc 18480
aaggtgaggt ctgtatggct aaggtttact catttaacat ttattggagc atttccatgc 18540
gctgggcttt gccataccct ggggatacaa gctctgcctt cgcgatgctc acaaaccact 18600
cagggagaca gacagctggg ctcttatgag gggtaaagaa ggatttagga ggtccctgtc 18660

ctggtctgag gtgggatcct agagaagata aaggctcagc caagactatt gtctccacca 18720
cctggctccc acctccccca atccaccgta atcatgggtc ccggagagga caactgctgg 18780
ctcctagggc accttttgc aaattagcaa aaggtgcctc tttatgacag caaggccctt 18840
agagtgcacg acttggtgag cagagtgtga gctggattta tcccccaact catgtccttg 18900
accaggcacc ttctgtaatt gacttctaaa gccctctgtg tgagccctga ttctgggcac 18960
tgcaaccagg agtgagcagg aagttgcact ctgttgggaa tggctgcagt ggggcccgtg 19020
gacagacaga tgatgggagg aacaggctgg cctagggcat gtcagcacca gccaacacac 19080
cctttgcttc caccccaggg aatgaatact ggatctactc agccagcacc ctggagcgag 19140
ggtaccccaa gccactgacc agcctgggac tgccccctga tgtccagcga gtggatgccg 19200
cctttaactg gagcaaaaac aagaagacat acatctttgc tggagacaaa ttctggaggt 19260
aagggagggc ggtgggtagg acagcagcac agttggctgc gagagacaga gaagccgccc 19320
tgaggcttca agcctcctgg gctgagttca gaggttggtg ggctctggat gccctctctc 19380
tggtatctaa cctctttggt ctctggcacc tctgagatgt tgccaaaggt gaaccattct 19440
tgtacacatt gctgatataa ttctggtgga atatcctttc tctttgcaga attcttttga 19500
ttggcccatg tctggtctgg accctcacat tttgaaatga ctcactgaag aaggggggtaa 19560
taaaaataaa gataataact gctcctattg acactatgtg tcagagactg tcccaagggt 19620
tctatgggat aggaattatt attcctccca ttttacagat gaggaaacct gggttcagaa 19680
aggttaggta accgtggctg ggcacagtgg ctcacgcctg taatcccagc actttgggag 19740
gccaagatgg gtggatcact tgagctcagg agttccagat cagcctggcc acatggtgaa 19800
accccatctc tactaaaaat gcaaaaaaat tagctggaca tggtggtgca tgcctgcaat 19860
cccagctact agggagactg aggcagggga atcacttaaa cctggaagac agaagttgca 19920
gtgagctgag atcatgccac tgcactccag cctgggcaac agagtgagac tccaactcaa 19980
aaaaaaaaaa aagaaaaaag aaaggttaag taaccagctt ggagtcacat agccaggtag 20040
ggacaggttg ggatttgaac cctggcattc tgtctcctgg gaaaacatgt tagtcttcac 20100
tcttcactac tattctatac ccgagatggt ggaatggggg ttccaagaga tgggggaaca 20160
tgaaggcagt ggacaaaata tgaagaagac ttagagatgg gtgcaccagt catccataga 20220
agccagcatc caaatcagac cccatggaag gagcaggtgc cctgtgtcat gctcacagca 20280
ggcccgtggc tgtgggtgca ggttggcagg ggaacaattt ggaagcagat ggttctgtgc 20340
tccaggttat ctacaggaaa gggtccagtt ctgaggggct atagtcatga ccagggaacc 20400
taatatccag gagtggctgg tgagaggtag cccaaacctc aggagtttga ctgtaattct 20460
tcacactttc tagaaaagga aacggaggtc caaagaaatt aaataacttg ttcaagctat 20520
agagatatgt gtatttgcac aaagccagga ttatagctac ttctcttgta ttctaactga 20580

gtaccccctc cactatacct gccagtccat ttgattagtg aggaaagagt ggtcggggca 20640
caaagacttg tggtgtgtct ttccatcccc tccctcctgg tatccccaca ttccttccat 20700
ggctgccact gcatctgggc cctacccaag aattttagag ggcacttcct gaggcctttg 20760
tctgccatcc ccaaaacaat cccgcttact tcctaaggcc tctgtcttct ccccctccc 20820
tcctccccac tactctgccc cccagctgcc ctgcattcag aaactcttta tatcccaggc 20880
ctgcccatgt cagggtggaa ttccagacat attgctaaga cacttagcaa cctaagctga 20940
ttgatatctc aagcctcaag aaagaaaaca gtcaggcagg aaaccacaga gcaggctttc 21000
cacccaccag ctccagacac tagccccaga cagtgtgcac gcacacgcac acacacacac 21060
gcgtactgcg aatggcatct cagcattgta ggagccctga atctccaagt cttagaattg 21120
tggctggtga gccgcacaga ctcacagaaa cgtgaatctt agaatcctaa agccctgaag 21180
ttttcaaatt atggtgttgg aagaactttt taaatcactt ttccatgcac tgtatctatg 21240
taaacatgca aaaacagacc tgttcctagc atataggaac atgattgtgt gcgcatgtgc 21300
gcatacacac acacacacac acacacacac acacacacac acacacacag gcatggaggt 21360
tgtggcagtg ccttgctgga tttccaggca gagttatttg cctcccagtc cttttccaca 21420
caagcccacc tcccaaagcc agagccagga cagacagcgt gctctacaga ggaaccaccc 21480
tgagacttcc tgaaaaggga cagagctgaa gggcctatca gaagctctgg gctggcttcc 21540
aaaggagaca gatctctatt ccaagagaag tggtgaatga gcttgtgaca ctggccctgc 21600
cgtggagtct gcagaggccc cagaggcatc tttgctggag aaggagagag agccccagag 21660
tgacagaggc tgagcagctg tcttcagcat gccaagaaga gctcaggccc tgcttgtaga 21720
agctttcact aagccacaaa tatatgttcc acatctgctg tgtgccaggc acttagctag 21780
ctgctggaga gacgggggtc aaaaaatcat cacaccctgc cctcatgtag tttatagtag 21840
aatgagggaa aattatgtaa acacatttga atcagataat gatggctctc agtatggggc 21900
ctctaccata gcccagcctg aggctatgca ggtcacctat atttctcagt tgaggtagat 21960
actgttagct ccttttccca agtgtggaaa ctgaggctaa agtggctcaa ggaaaagctg 22020
ggacacaaat atctgtgttc ttaacacctg ctatacatgg caacttctac agctgcagag 22080
aagtaccaca tcttctgaca ccacagtgct tgtattcttt ctggatgaaa agaacttgga 22140
agcaaactaa ttctcttatc tgagactcag tttcctcatc tgtaaaatgg gcttgacttc 22200
tgccatcccc accttctgtg ggtagaatga gaaagaaatg agggccagtt caagtgtggg 22260
acacacatct ccaaactatc ctaacgctag ggaatgtagt ttgagggctc agtggaggtg 22320
ccttggatgg gagtgttggg attttgagct agggaacttg gttttggtga cccagggcat 22380
gaggaagcat gccaggtttt gtaagaatga gttttatgct gcacctggga ttaaggaagg 22440

cgttagttct tcggggtgat ggctgaggga gaagtccctt tgagggagaa gtctcctcta 22500
gaagggagca tatagggaaa gctgctgttg gggtccaggg tggaggtggt caaggctggc 22560
tgcagaaagg aagggagccc cctaggaggc actgtggagc aaaaggtggc tgctaccagg 22620
atgggatgcg aacacagatt catctagaaa cagctccact tgggcaagac ttgtttacta 22680
ggatatccat agctcccata gtgggagctc agtaaatatt tatggaataa tgaagtggag 22740
aggcaaagga atcaacatga cagcagcatt taaaaagttc tccaaaaatc ttaaaagaca 22800
gaaggattgc tagccaggga gaagaacata ctaaccgatg cccaaagtat ttacctgaat 22860
taaaggcggg gcccaggatt gctgccgccg gaactgaggt ggcatgtggc ccaccctgtg 22920
actaacaggc cttggactta cacgtacctt ccaccttgca ttgtaacctc actgccccag 22980
ccatggtcat ctcccagggc agggactgtc atctccctgt ggcagatgaa gaagcagcaa 23040
tttttccaag ccatgaagtg aaagaatgat ggggcagatt cccatccctg ggcttctagt 23100
ttagcactcc tcggattaca gcagggattt gtttgtaagg gagctaacat ttcttaagta 23160
cctactaaat atttatgaga catggggata gaggctctgc atgtgctgtt ttatttaatg 23220
catgcattaa attaaataca ctaaattaga cacaacccca ttttacagct ggtaaaactg 23280
tagctgtatg gaaagtggtt aacacactgt ttggcatgta gtaagtgctt tataaatgtt 23340
agcagtaatc atgagattgt ttttatgggg tcatccaaag tcatataatt aaaagtggta 23400
gatctgaggc tcagactcag gtctgattcc atgcccttcc ttccactgca gctggctgct 23460
tccctgaagg gctaggtcca gtttccgagg cctatccagg agccatcagc tgggtgctcc 23520
catccaggcc agggggaag tgtcctttag agaggccctg ctggttcact gtgtctgttt 23580
ctttacagat acaatgaggt gaagaagaaa atggatcctg gcttccccaa gctcatcgca 23640
gatgcctgga atgccatccc cgataacctg gatgccgtcg tggacctgca gggcggcggt 23700
gagccaccca ggactgtctc cgctttctag gactccccgc ccctagccag ggcccagctc 23760
cttgcaaata cacacttctt tattgtgcag ggcaggcagg caggcggcgc ccaggaaaac 23820
aaatcaactc cttcatccta gtgctgggaa ggtttccaga tccctgacct ctgctccttg 23880
gcctggctcc aggcagtaag acccagaaag acttctcaca gctgcagggc ctggagccag 23940
ggcaggggag ggcggaaggc ctgggccagg ggagaaggaa catgggctct gtgccgtgga 24000
ttcattctgg ggtttgatcc aggcccattt gcgttttgca ggaggcctgt gaccacctcc 24060
cattagcaga aggatggctc catttgagac aaggcaaact ttgtcatgct ctgttgctca 24120
tgtggcaaag atcctccact caaccagttt gcccctggcc ccctgctgat taaaacacaa 24180
ccagtagcag cgagggatag aatttggagt ttgaaaatca tactcttgag gaggcatctg 24240
tgagctttct agggtcctgg aaatgggtcc agtttggctt tttttagagg agatttcaac 24300
taaattccac agattttttt ttttttgagc acctactgta tgctaagcac aggccctgac 24360

ctccagtagc tgaaggtctt acatgtccaa gaaaatgcag tgtacaatca gatgctagat 24420
taggagttat cttcaagaat aaacatctat tgagcaccta ctacatgcta gcatattaat 24480
agtaaatgag attatgtatg tcacgagtca caaactccaa cagctccatg tgtcaggcag 24540
gtagcacaaa tgagtaaagc aggcaggggc agacagtggg gaactgtgga gtctgtgcgg 24600
aatcagagca catgcctcat ttacaggaga cactttcttc tcaccttcag ataattattg 24660
gcgggcaaga ttaggggcct aggcagctgc catggttcac acctgtcatc ccagcactct 24720
gggaggccaa ggcaggagaa ctgcttgagc gcaggagttt gagaccagcc tgggcaacat 24780
agtgagactc tatctctaca aaaaaatttt aaaaaacatt agctacacat gacggcgcat 24840
acctgtagtc ccagctactg gggagggtga gcagagagga tcgcttgagc ccaggagttt 24900
aaggctgcag tgagccgtgt tcatgccgct gcattccaga ctgggtgaca gagtgagacc 24960
tggtctcaaa aaaaaagaat ggggcctggt gttgttagat attctatatt agatattcta 25020
gttttttaa aggaagccag aaatctgtac ttctgtgtga ctttttggat gtttacatgt 25080
tggcaagtta ttcagattta aagacaaaac aaaacaaact tgtgagagtc aaataaaacc 25140
catctgtggg cctgacagga ctggatatat cctgttttgc cacctctggt acatggaatg 25200
tgcttagttt gatgcttgtg ttaagtggag ttattgtttt taataatagc atatgttatc 25260
tgacttaatg ctcactgcaa gccctagaag gttgataatc tttatcctca tcttagagat 25320
gagcaaacag ggaataaaag agcttatttc acagccattt cccttcttcc actcccagag 25380
ggggctcttc tgggcttttg ttgtgttttg gttttgtttt tgttttcttt tcttcaatca 25440
gctcagattg aaacaattga attttttttt tttttttttt gagacagagt ctccctctgt 25500
cgcccaggct ggagtgcagt ggcgcgatct tggctcactg caagctccgc ctactgggtt 25560
cacaccattc tcctgcctca gcctcccgag tagctgggac tacaggcgcc taccaccacg 25620
cccggctaat tttttgtatt tttagtagag acgaggtttc accatgttag ccaggatggt 25680
cttgatctcc tgacttcgtg atctgcccgt ctcggcctcc caaagtgctg ggattacagg 25740
cgtgagccac cgcgccccgc cacagttgag tttttttttt ggcgggggga caaggtcttg 25800
ctctatcacc caggctagag gcacaatcat agctcactgc agcctcgaac tcctgggctt 25860
aagcgaccct cccacgtcag cctcctaagt agcttggact acaggaatgt accactatac 25920
ccagctaatt taaatatgtt tttcgtagaa atgggggtct ctctatgctg cccaagctgg 25980
tctcgaactc ctgggctcaa gcaatcctcc tgcctcagcc tttcaaagct ctcttctcgt 26040
ttaaaagctt cttccctatg ccaggcagaa attcaaagtt cccaggaacc ttctgtgtgc 26100
cagaatgtca ggataagggg gtcacggtct cttctttctc tatcccaggt cacagctact 26160
tcttcaaggg tgcctattac ctgaagctgg agaaccaaag tctgaagagc gtgaagtttg 26220

gaagcatcaa atccgactgg ctaggctgct gagctggccc tggctcccac aggcccttcc 26280

tctccactgc cttcgataca ccgggcctgg agaactagag aaggacccgg aggggcctgg 26340

cagccgtgcc ttcagctcta cagctaatca gcattctcac tcctacctgg taatttaaga 26400

ttccagagag tggctcctcc cggtgcccaa gaatagatgc tgactgtact cctcccaggc 26460

gccccttccc cctccaatcc caccaaccct cagagccacc cctaaagaga tactttgata 26520

ttttcaacgc agccctgctt tgggctgccc tggtgctgcc acacttcagg ctcttctcct 26580

ttcacaacct tctgtggctc acagaaccct tggagccaat ggagactgtc tcaagagggc 26640

actggtggcc cgacagcctg gcacagggca gtgggacagg gcatggccag gtggccactc 26700

cagacccctg gcttttcact gctggctgcc ttagaacctt tcttacatta gcagtttgct 26760

ttgtatgcac tttgtttttt tctttgggtc ttgttttttt tttccactta gaaattgcat 26820

ttcctgacag aaggactcag gttgtctgaa gtcactgcac agtgcatctc agcccacata 26880

gtgatggttc ccctgttcac tctacttagc atgtccctac cgagtctctt ctccactgga 26940

tggaggaaaa ccaagccgtg gcttcccgct cagccctccc tgcccctccc ttcaaccatt 27000

ccccatggga aatgtcaaca agtatgaata aagacaccta ctgagtggc 27049

<210> 55
<211> 19
<212> DNA
<213> Artificial

<220>
<223> Synthesis

<220>
<221> misc_signal
<222> (1)..(19)

<400> 55
cctgagctcc cggaaaaga 19

<210> 56
<211> 23
<212> DNA
<213> Artificial

<220>
<223> Synthesis

<220>
<221> misc_signal
<222> (1)..(23)

<400> 56
attcattccc tgcaaagaac aca 23

<210> 57
<211> 1828
<212> DNA
<213> homo sapiens

<400> 57
ctacaaggag gcaggcaaga cagcaaggca tagagacaac atagagctaa gtaaagccag 60

tggaaatgaa gagtcttcca atcctactgt tgctgtgcgt ggcagtttgc tcagcctatc 120

cattggatgg agctgcaagg ggtgaggaca ccagcatgaa ccttgttcag aaatatctag 180

aaaactacta cgacctcaaa aaagatgtga aacagtttgt taggagaaag gacagtggtc 240

ctgttgttaa aaaaatccga gaaatgcaga agttccttgg attggaggtg acggggaagc 300

tggactccga cactctggag gtgatgcgca agcccaggtg tggagttcct gatgttggtc 360

acttcagaac ctttcctggc atcccgaagt ggaggaaaac ccaccttaca tacaggattg 420

tgaattatac accagatttg ccaaaagatg ctgttgattc tgctgttgag aaagctctga 480

aagtctggga agaggtgact ccactcacat tctccaggct gtatgaagga gaggctgata 540

taatgatctc ttttgcagtt agagaacatg gagactttta cccttttgat ggacctggaa 600

atgttttggc ccatgcctat gcccctgggc cagggattaa tggagatgcc cactttgatg 660

atgatgaaca atggacaaag gatacaacag ggaccaattt atttctcgtt gctgctcatg 720

aaattggcca ctccctgggt ctctttcact cagccaacac tgaagctttg atgtacccac 780

tctatcactc actcacagac ctgactcggt tccgcctgtc tcaagatgat ataaatggca 840

ttcagtccct ctatggacct ccccctgact cccctgagac ccccctggta cccacggaac 900

ctgtccctcc agaacctggg acgccagcca actgtgatcc tgctttgtcc tttgatgctg 960

tcagcactct gaggggagaa atcctgatct ttaaagacag gcacttttgg cgcaaatccc 1020

tcaggaagct tgaacctgaa ttgcatttga tctcttcatt ttggccatct cttccttcag 1080

gcgtggatgc cgcatatgaa gttactagca aggacctcgt tttcattttt aaaggaaatc 1140

aattctgggc tatcagagga aatgaggtac gagctggata cccaagaggc atccacaccc 1200

taggtttccc tccaaccgtg aggaaaatcg atgcagccat ttctgataag gaaaagaaca 1260

aaacatattt ctttgtagag gacaaatact ggagatttga tgagaagaga aattccatgg 1320

agccaggctt tcccaagcaa atagctgaag actttccagg gattgactca aagattgatg 1380

ctgtttttga agaatttggg ttcttttatt tctttactgg atcttcacag ttggagtttg 1440

acccaaatgc aaagaaagtg acacacactt tgaagagtaa cagctggctt aattgttgaa 1500

agagatatgt agaaggcaca atatgggcac tttaaatgaa gctaataatt cttcacctaa 1560

gtctctgtga attgaaatgt tcgttttctc ctgcctgtgc tgtgactcga gtcacactca 1620

agggaacttg agcgtgaatc tgtatcttgc cggtcatttt tatgttatta cagggcattc 1680

aaatgggctg ctgcttagct tgcaccttgt cacatagagt gatctttccc aagagaaggg 1740

gaagcactcg tgtgcaacag acaagtgact gtatctgtgt agactatttg cttatttaat 1800

aaagacgatt tgtcagttat tttatctt 1828

<210> 58
<211> 7808
<212> DNA
<213> homo sapiens

<400> 58
acaaggaggc aggcaagaca gcaaggcata gagacaacat agagctaagt aaagccagtg 60

gaaatgaaga gtcttccaat cctactgttg ctgtgcgtgg cagtttgctc agcctatcca 120

ttggatggag ctgcaagggg tgaggacacc agcatgaacc ttgttcaggt aattaacact 180

aactgacctg gccaggtggg gagcttccag agtaagctga aaccattctg tgtgtcttaa 240

aacccacata gaatgcttga gcttactgtg taggaacagt tcaagaatag agagttatag 300

agtgttggaa gtggaaaagg tctcattatc acaccatttg ggcttctcct cttatgtagg 360

aagaaacaaa ggccagaaat attaggtgtt ttcttaagat cacacaacac catagcagta 420

gcagcactgg ggcttaaggc acatgagtaa caagtggaga gtggatgaac catatcttgc 480

tttggaacag cttcagtttt ttttttaatg caacgtaatt ttagctaaat atttgtgttg 540

ctagtatcat attataaaat ttgccattat ttcagcaagc tagagaaata ctccaaatag 600

attaagaagt gagcaactgc aaaaacttac tataaataga tagctatagt aaaaattgta 660

agagtgacct aaaaactata cttattctgt tagaaatatc tagaaaacta ctacgacctc 720

aaaaaagatg tgaaacagtt tgttaggaga aaggacagtg gtcctgttgt taaaaaaatc 780

cgagaaatgc agaagttcct tggattggag gtgacgggga agctggactc cgacactctg 840

gaggtgatgc gcaagcccag gtgtggagtt cctgatgttg gtcacttcag aacctttcct 900

ggcatcccga agtggaggaa aacccacctt acatacaggt aatggttcag aggggttttc 960

acataagatt ggaattttat aaactatatc ataggagaaa atagcatctg cttcaatttt 1020

tctcctacag gattgtgaat tatacaccag atttgccaaa agatgctgtt gattctgctg 1080

ttgagaaagc tctgaaagtc tgggaagagg tgactccact cacattctcc aggctgtatg 1140

aaggagaggc tgatataatg atctcttttg cagttagagg taaaaaaaaa aaaaaaaaaa 1200

aaaaagcaaa acaaaacaaa acaaaaaaca catgaaatgt atccattcat ctatctggtg 1260

ttgttgtact ggaaaaggct caaaagggat ccttgttaca tatctttcca ataaattttt 1320

ttcctactag aacatggaga cttttaccct tttgatggac ctggaaatgt tttggcccat 1380

gcctatgccc ctgggccagg gattaatgga gatgcccact ttgatgatga tgaacaatgg 1440

acaaaggata caacaggtta gttacacatc tctcagaatg attttggcat taattttttt 1500

atatgtagaa atctgttaat aaccaagaat gctcaagaaa tgagatgttc tggattttcc 1560

agtggatcag agatcagcag aatatatttt tgttttaatt attattgaaa atattttcag 1620
atgctttaac agttttccta tattggtaag cacaatatag gaaatatcat taagtatttc 1680
tttaatgact ggatctttgt gatctagggg taaccattat taagaaaaat ttctcatatc 1740
aaaatacaaa cattcaaggg atagtatatt gcactgaatc tacattaaaa tgtgctctgg 1800
atgttactta tgtaatttcc tccatttact tctttactct tcaaaccaga aataagaatt 1860
aaatttcaga aactgagatt ctcttttttt tttttttctg gctgaggcat tattttagta 1920
cttttaggtt aaaagaattt ttatgggctt gttctgaagg aaaagactaa ctcccaaata 1980
caacgttcaa actgtttagg tagaagaatg tttctcagtt cataactgta tgttttgggg 2040
aatgacatcc aaagttttca aaaaaatcag ctaaaacaat taacactact tctaaaaaaa 2100
taggaaaaca attaatacta tgtctgaggt ttaactggga tttataatga aaatgtaaac 2160
ctctgtagga cgtacaaatg tgagtttagt cctggataga attcttgatt ggttgagtta 2220
acgaattctt gtaaagaaac cttcctgcaa actctcaaat gagattgttc ctagcatcac 2280
aagttacaca agtaatacta gcaaaagtaa ggtagattta cttactatat tgatcataat 2340
tacccaatgc ctcccaattt tagcattatg ggtgttttta aaaatatgaa ggaaaaaatt 2400
ttgaataaag ccagtgttta tgtattctaa atctgttgtg tgacaaatgc tagacatgat 2460
tgggcatact gtgttttaaa ccaattttgc aaatcctatg acatctatca ttattcatgt 2520
ttttagtctc aatatgtact tcatgatgat gaggctgaga cattatcaat taacatgaat 2580
atagtttcct ttaactgaga ctgcaaatgg caaatgtaaa tatattaaaa cagtgtttag 2640
ctttgcattg acaaatgaag gtaaatatct gatatgctaa gcaagtgggt tcattccatt 2700
cctgagtaca agaaaaggga tctggttctt ctggagttaa tatgcttgtg aggctggggg 2760
tgggtttggg gccatggggg gcaagtggat gtaactggga aatcaatctc aaagattgat 2820
tacaggtatg agttttaaaa tacacttagc tttccattaa gtgtgtaatt agtcaattgt 2880
tcaattaacc aacaaacaag gtgtaaaaag ccctgtgtgg ttccaagaag taaaaaccaa 2940
tacaatgcac agcagacctg tgtaatgcac atgtatttaa aaaataagta actattgttc 3000
tcaatttctt aaatagggac caatttattt ctcgttgctg ctcatgaaat tggccactcc 3060
ctgggtctct ttcactcagc caacactgaa gctttgatgt acccactcta tcactcactc 3120
acagacctga ctcggttccg cctgtctcaa gatgatataa atggcattca gtccctctat 3180
ggtgagtgac actgggaaaa tcagccattg tagttttgca atgatggtct tcagaagagc 3240
tttcaaatgc tacataaagt atttatgatt tagttcaaag caccgtgaga atgtttggaa 3300
agcatacaaa tgggcatata ttgaaacgta tcacatttcc cctaatgttc cctttaggac 3360
ctcccctga ctcccctgag acccccctgg tacccacgga acctgtccct ccagaacctg 3420

ggacgccagc caactgtgat cctgctttgt cctttgatgc tgtcagcact ctgaggggag 3480
aaatcctgat ctttaaagac aggtcaggct gaaaaattat attttcctac cattttaatc 3540
tacaatgttt agaaaggaaa acacgcagaa acttgataga gattcttaga catttaaaac 3600
aaaaacgttt cttctgagct tggaatcatt tagatctggt gctgcctttt cccacatttt 3660
cctaaaattg tcaggttcat ttttgaaggc acaggctgat ctggttctta gcttctaatt 3720
gctgtttcca aatgtcttaa gtcacagtta cttttcaaaa ggcatgagta gatctttcga 3780
ataataattt tatttttact tcctttaaaa atgacatcag gatcaacatt acatttaatc 3840
aagtaattta cttactgatg gatcaatatg cacatgacaa acattcaaag agcacaaatg 3900
agaagcaaat taaaaatttt agttcccagt tctccctgta ctcatccaga gtcaagactt 3960
tttgtgcatc cttccagaat ctttctacac acatacaaga atatatttac acagcacata 4020
cacgtataca aacacggcct agcttttgga agagggtata gcacacgcac tgttttgcat 4080
acattgcagg taattaaagc ttatgctggg ctaattcatt tgcacgatag aataattttt 4140
ctttcataga agacattact ctggaaacct atgacgggag actcactaca gtcttctgaa 4200
gaaaaaggaa agaaattttc taatggcaat atacctgatt tctatctgtc atccagccta 4260
gaagatgaga gaatggggga agctgggtga ggcaagacgg tgaggaaaat attctaaagg 4320
tgaaagattg tttttcaatt tacatttctt tttacatata ttttaggcac ttttggcgca 4380
aatccctcag gaagcttgaa cctgaattgc atttgatctc ttcattttgg ccatctcttc 4440
cttcaggcgt ggatgccgca tatgaagtta ctagcaagga cctcgttttc atttttaaag 4500
gtaattacta tatcatcatt tgtttaactt gccaaagtaa accctctcat aagaaattga 4560
acaacacttt aattttccct actacaagtt ctggctaata cagtagtcaa cggtaatgaa 4620
gaaataacca gggctgatat cattgtattt atatttcata gatgataaaa ataaagacaa 4680
gaagataaat tacccagagt tgtaaattct ctcatttgac cttagttcat tcattcagtc 4740
tgcaaatttt tgtgaaaagc ccactgtaag ctggtgacta aatctaaaat ttgggctact 4800
actttaccat gaaaatcaaa gatgctaaag tgtatttcta aagcgaaagg gcttaactgt 4860
tataattcaa tatgactctg aattatttat atttaacagg aaatcaattc tgggctatca 4920
gaggaaatga ggtacgagct ggatacccaa gaggcatcca caccctaggt ttccctccaa 4980
ccgtgaggaa aatcgatgca gccatttctg ataaggaaaa gaacaaaaca tatttctttg 5040
tagaggacaa atactggagg tgagatgcaa gagaaatgac tttgtatgga gcctaattag 5100
ggaaattagt ttcttttact aaggaaatcc cccaccccaa ccttaggcct gcttctctct 5160
tagtaggaag gatgggactt cagacaactg tgggaaagtt cttactttaa aacagcagca 5220
ccttaggctc tcctgctttc agtcccaagt gcagttggct ggtgcttccc ttggtgctgt 5280
caccccacct gcctggagcc ccctcttctc tcctcaacct attcaaatct tgcccatcct 5340

tcaagtcctg gctcaagtct catcacctcc atgcagcttt ccctcgtgga ctttagaccc 5400
attgggaaaa tgttctgaga acccagaccg caatttagcg ttcccttccc attgtctttt 5460
agtttggctc actgtctcac tgtgtgtttg tcactgtctc attgtgtgtt tgttttgtct 5520
tcctaacact actatatgat tttcaagggc tacctctaac ctcacccact tgtcacctct 5580
acctgataat gatggctcat ttaagagatg atgatcaata gcagtccagt ttatggatac 5640
aacccaacac tcacagacag gttgattcct gggtcatttg gccagacttt gagacatcga 5700
ggccgaccat ctgatatcca gccaatgggc taaaaacaca cttactgtgc tttgcttctt 5760
atcctaatcc cttttcccag gaggccacca gagaagattt agatgtgcag gggaggggac 5820
agcaggcata tatgggccaa ttgtggtagg gaattaaaat tgcattttag atgttgattc 5880
aattcacttt atatatatgt tcacactttc atgtctttgt gacagaaaga tgattaaggt 5940
tctttctgtc caggcttaag aaatatgatg acaaacagca tgtgctacat acagcaggat 6000
gcggcccctg ttccggatgg agatatggct ttcagaactc tttacagtac cattaaagtc 6060
acatatttct atagtcccaa atatgcttgg catttatttg cagtatttat aaagtttagt 6120
aaaattccac attaaaccta acatgatcta agatgttcgg taacatggcc tctatgcaat 6180
gctgagctgt ttcttttact tggtacagat ttgatgagaa gagaaattcc atggagccag 6240
gctttcccaa gcaaatagct gaagactttc cagggattga ctcaaagatt gatgctgttt 6300
ttgaagaatt tggtaagagg atacttactg tgttggccat agagcacttg aaacatttta 6360
tgatatcaga gggaagagga gcattgaaac tttttaataa caagtgtttc ttagggccag 6420
ttcccttctc aaaattttga ttcgcctaca tttgtactca ttatagaact acttcacaaa 6480
ataacttgtt tggaaccata aagataatta cacaaatctc gctactttat cttatacatg 6540
catataaaat caaagtgctc cagtgtcact tacatctcga gctaacatgt ggagtcaagc 6600
tcgactttca gattaccatg ttagcataaa aatcccatca agtggtgtgg ccaggtttaa 6660
tgttggttag ctggccttct cctccacccct atcttattgc tgctactaat tcccagggag 6720
attttttgtc tgtctttcct gagttgtcag tcattgccca tgccagtcat ttgcaaatac 6780
ttacctttcc atgtgcccaa agtttaaaaa aaaaaaaaga ttttgttttc tactcaaggt 6840
aaggaagtgg aggctgatga tctttattga aaggtaacct gatcaccatg tttgcagagc 6900
actaaagggt cccctggtgt tttgaaatag ttgctgtgct tcctaggctg caatgcaggg 6960
aaaagggagg aggatttttg gggaagaaag aaataggtga tgagagtttc caagcaagtg 7020
gagaatttat ctgatcggaa gcagaataac cctgtaacct ggaatctggc tgtgttttca 7080
gacagagctg gcaccgagtg ctgtctccat tcttccttgc tgagtaacct tgaggaagca 7140
tcgaaatctg tctcaacctc aacttgctca atctgtaaat ggagttagac atacaacctc 7200

atagggttgt tgcagaaata aatgaaaata aattcatgta acaagcttgg catagtccct 7260

gacataaagt aaagctctat aaacactttc tatttttagt tctctatgat tctgagtttt 7320

gtatattgtc aaatgtcttt atttggaaaa atatcttact atttcccttg tctgtatttg 7380

cagggttctt ttatttcttt actggatctt cacagttgga gtttgaccca aatgcaaaga 7440

aagtgacaca cactttgaag agtaacagct ggcttaattg ttgaaagaga tatgtagaag 7500

gcacaatatg ggcactttaa atgaagctaa taattcttca cctaagtctc tgtgaattga 7560

aatgttcgtt ttctcctgcc tgtgctgtga ctcgagtcac actcaaggga acttgagcgt 7620

gaatctgtat cttgccggtc atttttatgt tattacaggg cattcaaatg ggctgctgct 7680

tagcttgcac cttgtcacat agagtgatct ttcccaagag aaggggaagc actcgtgtgc 7740

aacagacaag tgactgtatc tgtgtagact atttgcttat ttaataaaga cgatttgtca 7800

gttatttt 7808

<210> 59
<211> 25
<212> DNA
<213> Artificial

<220>
<223> Synthesis

<220>
<221> misc_signal
<222> (1)..(25)

<400> 59
aaaggataca acagggacca attta 25

<210> 60
<211> 23
<212> DNA
<213> Artificial

<220>
<223> Synthesis

<220>
<221> misc_signal
<222> (1)..(23)

<400> 60
cagtgttggc tgagtgaaag aga 23

<210> 61
<211> 2387
<212> DNA
<213> homo sapiens

<400> 61

agacacctct gccctcacca tgagcctctg gcagcccctg gtcctggtgc tcctggtgct 60
gggctgctgc tttgctgccc ccagacagcg ccagtccacc cttgtgctct tccctggaga 120
cctgagaacc aatctcaccg acaggcagct ggcagaggaa tacctgtacc gctatggtta 180
cactcgggtg gcagagatgc gtggagagtc gaaatctctg gggcctgcgc tgctgcttct 240
ccagaagcaa ctgtccctgc ccgagaccgg tgagctggat agcgccacgc tgaaggccat 300
gcgaacccca cggtgcgggg tcccagacct gggcagattc caaacctttg agggcgacct 360
caagtggcac caccacaaca tcacctattg gatccaaaac tactcggaag acttgccgcg 420
ggcggtgatt gacgacgcct ttgcccgcgc cttcgcactg tggagcgcgg tgacgccgct 480
caccttcact cgcgtgtaca gccgggacgc agacatcgtc atccagtttg gtgtcgcgga 540
gcacggagac gggtatccct tcgacgggaa ggacgggctc ctggcacacg cctttcctcc 600
tggccccggc attcagggag acgcccattt cgacgatgac gagttgtggt ccctgggcaa 660
gggcgtcgtg gttccaactc ggtttggaaa cgcagatggc gcggcctgcc acttcccctt 720
catcttcgag ggccgctcct actctgcctg caccaccgac ggtcgctccg acggcttgcc 780
ctggtgcagt accacggcca actacgacac cgacgaccgg tttggcttct gccccagcga 840
gagactctac acccaggacg gcaatgctga tgggaaaccc tgccagtttc cattcatctt 900
ccaaggccaa tcctactccg cctgcaccac ggacggtcgc tccgacggct accgctggtg 960
cgccaccacc gccaactacg accgggacaa gctcttcggc ttctgcccga cccgagctga 1020
ctcgacggtg atgggggggca actcggcggg ggagctgtgc gtcttcccct tcactttcct 1080
gggtaaggag tactcgacct gtaccagcga gggccgcgga gatgggcgcc tctggtgcgc 1140
taccacctcg aactttgaca gcgacaagaa gtggggcttc tgcccggacc aaggatacag 1200
tttgttcctc gtggcggcgc atgagttcgg ccacgcgctg ggcttagatc attcctcagt 1260
gccggaggcg ctcatgtacc ctatgtaccg cttcactgag gggcccccct tgcataagga 1320
cgacgtgaat ggcatccggc acctctatgg tcctcgccct gaacctgagc cacggcctcc 1380
aaccaccacc acaccgcagc ccacggctcc cccgacggtc tgccccaccg gacccccac 1440
tgtccacccc tcagagcgcc ccacagctgg ccccacaggt ccccctcag ctggccccac 1500
aggtcccccc actgctggcc cttctacggc cactactgtg cctttgagtc cggtggacga 1560
tgcctgcaac gtgaacatct tcgacgccat cgcggagatt gggaaccagc tgtatttgtt 1620
caaggatggg aagtactggc gattctctga gggcaggggg agccggccgc agggcccctt 1680
ccttatcgcc gacaagtggc ccgcgctgcc ccgcaagctg gactcggtct ttgaggagcg 1740
gctctccaag aagcttttct tcttctctgg gcgccaggtg tgggtgtaca caggcgcgtc 1800
ggtgctgggc ccgaggcgtc tggacaagct gggcctggga gccgacgtgg cccaggtgac 1860
cggggccctc cggagtggca gggggaagat gctgctgttc agcgggcggc gcctctggag 1920

gttcgacgtg aaggcgcaga tggtggatcc ccggagcgcc agcgaggtgg accggatgtt 1980

ccccggggtg cctttggaca cgcacgacgt cttccagtac cgagagaaag cctatttctg 2040

ccaggaccgc ttctactggc gcgtgagttc ccggagtgag ttgaaccagg tggaccaagt 2100

gggctacgtg acctatgaca tcctgcagtg ccctgaggac tagggctccc gtcctgcttt 2160

ggcagtgcca tgtaaatccc cactgggacc aaccctgggg aaggagccag tttgccggat 2220

acaaactggt attctgttct ggaggaaagg gaggagtgga ggtgggctgg gccctctctt 2280

ctcacctttg tttttgttg gagtgtttct aataaacttg gattctctaa cctttaaaaa 2340

aaaaaaaaaa aaaaaaaaaa aaaaaaaaaa aaaaaaaaaa aaaaaaa 2387

<210> 62
<211> 7653
<212> DNA
<213> homo sapiens

<400> 62
agacacctct gccctcacca tgagcctctg gcagcccctg gtcctggtgc tcctggtgct 60

gggctgctgc tttgctgccc ccagacagcg ccagtccacc cttgtgctct tccctggaga 120

cctgagaacc aatctcaccg acaggcagct ggcagaggtg ggcaaacacc tagtctagag 180

ttggggaggg ctgtccgtga gggtgttgag tgtcccagag aggatgcagg gcctcagagg 240

agatgcttta ggggtgtgtt ggtggtgatg ggcgtatctg aagaacagag gtgtccaggg 300

ttaggcagtg gggggtcttg tggaggcttt gagcagtgat ggccagaaat gggcaatggg 360

gctttcctag gtgggaaatg ggaaatggtt tggggtgggg gaggcattgg agggttctgg 420

ggtaagcata ggctgggagt gaacaggggc aaaccttatg cagctgtggg gtagaaatgg 480

gctagaggca tccaggggtg agaaggagct gaggatgtct aaggagggga gatccctggg 540

tggtcagaaa gcactggtgt ctggaaagca tttaatgctt tattaaatgt tagtccctgc 600

tgggcatgac ggctcacact tgtaatccca gcactttggg aggctgaggt ggtaggatcg 660

ctgaagctca ggagtttgag cccagcctag gcaacatagt aagatcctgt ctctacaaaa 720

aaattaaaga aatagccagg cacagtgatg tgcacctgta gttccagcta tgcagaaggc 780

tgagatggga ggatcgcttg agtccaggag gtccaggctg cagtgggctg ataccgtctc 840

tccgaaaaag aaaaagaaaa aagactccct ccatgagtgt ctggagggag tcctttggcc 900

ccagctgggc agagaaaggg gtcagagatc tggcatgtgt gtgtcccttc atccacagga 960

atacctgtac cgctatggtt acactcgggt ggcagagatg cgtggagagt cgaaatctct 1020

ggggcctgcg ctgctgcttc tccagaagca actgtccctg cccgagaccg gtgagctgga 1080

tagcgccacg ctgaaggcca tgcgaacccc acggtgcggg gtcccagacc tgggcagatt 1140

ccaaaccttt gagggcgacc tcaagtggca ccaccacaac atcacctatt ggtgagccgg 1200

ggccgtgggg gcagcggggt ggggcgggga ggccaggtct ggctcttggg ccagcggtga 1260
acatgtcctg tcttggacgc gtccctgggt ttcactattt aatgtgtggc ccctggggag 1320
tgtccccacc tctgagcctc tgtttctcct tcagggaaat ggctcttgca atccaagtcc 1380
tcctgccagg gccattgtga gggtctaagt agacaaaaaa aaaaaaaaaa aaaacagtct 1440
ggaagcaatt tatagatgag agcgtggacg gcagagagca ttgtgtatgt tgaagtctct 1500
gcgatatggg gtgtccctgc tgccccgctc cagcctttca cttctgacct ccttcctctg 1560
gctcttacgc tacaggatcc aaaactactc ggaagacttg ccgcgggcgg tgattgacga 1620
cgcctttgcc cgcgccttcg cactgtggag cgcggtgacg ccgctcacct tcactcgcgt 1680
gtacagccgg gacgcagaca tcgtcatcca gtttggtgtc gcgggtgaga acgtgaggag 1740
ggaaaatcca agagacctgg gcggggtcag ggaagggagg accacggaga gcgtggaggc 1800
agcagtggcc ccggcttcct cttgcctgcc cgcgctgccc tggcttatac ggcccctcct 1860
gccagacagt gcacagggcc agggcgccag gctgggagag cttcgcgcag gcgggatttc 1920
agcccgcact tatttcggag cccttgcctt gggcagcgca caatctgcgc agcagtactc 1980
ggctaaccct cttcctctcg acctgtttct tcagagcacg gagacgggta tcccttcgac 2040
gggaaggacg ggctcctggc acacgccttt cctcctggcc ccggcattca gggagacgcc 2100
catttcgacg atgacgagtt gtggtccctg ggcaagggcg tcggtgagat tctgagtcct 2160
cctggcccct gattcccttc attctctccc actcatcacc cgccgcccta actccggtcc 2220
cccctcctcc tgcagtggtt ccaactcggt ttggaaacgc agatggcgcg gcctgccact 2280
tccccttcat cttcgagggc cgctcctact ctgcctgcac caccgacggt cgctccgacg 2340
gcttgccctg gtgcagtacc acggccaact acgacaccga cgaccggttt ggcttctgcc 2400
ccagcgagag tgagtgaggg ggctcgccga gggctggggg cgcccaccac ccttgatggt 2460
cctgggttct aattccagct ctgccactag tgctgtgtgg cctgcaattc accctcccgc 2520
actctgggcc caattttctc atctgagaaa tgatgagaga tgggatgaac tgcagaccat 2580
ccatgggtca aagaacagga cacacttggg ggttataatg tgctgtctcc gccttctccc 2640
cctttcccac atcctcctcg ccccaggact ctacacccag gacggcaatg ctgatgggaa 2700
accctgccag tttccattca tcttccaagg ccaatcctac tccgcctgca ccacggacgg 2760
tcgctccgac ggctaccgct ggtgcgccac caccgccaac tacgaccggg acaagctctt 2820
cggcttctgc ccgacccgag gtacctccac cctgtctacc aggttcagcc ccgccctctc 2880
atcatgtatt ggcccccaaa acgcggctct tccctcccat cagtttgtct ttccactctc 2940
attggtcctc aggacgaccg tgactccgcc cacctacacc acatttccac cactatccct 3000
gacttccaat ggccccgccc cagccactaa ggttcggcct tttctgccca gctggccgcc 3060

tcttccttgg tctggtgtcc caggcaccgc ccacgggtct agcctcttct caggagtgct 3120

ctacagcgcc ccctaggcca ccaagattgt ttagctccct gtcgggtcgg cccctgactc 3180

cttattggac tcatccatct ggctcatcca aggccttggg tctctccagc tgactcgacg 3240

gtgatggggg gcaactcggc gggggagctg tgcgtcttcc ccttcacttt cctgggtaag 3300

gagtactcga cctgtaccag cgagggccgc ggagatgggc gcctctggtg cgctaccacc 3360

tcgaactttg acagcgacaa gaagtggggc ttctgcccgg accaaggtag gcgtggtccc 3420

gcggctccgg ggctggggtt cccggcagtg gtggtggtgg ggtggccagg gctgggggct 3480

cggcccggcg ctcacgtctc aggctccctc tccctccagg atacagtttg ttcctcgtgg 3540

cggcgcatga gttcggccac gcgctgggct tagatcattc ctcagtgccg gaggcgctca 3600

tgtaccctat gtaccgcttc actgaggggc ccccttgca taaggacgac gtgaatggca 3660

tccggcacct ctatggtgag gcaggggcag ggatgggagg aggaggggaa agggcgtggc 3720

tgtgccacag taccaaagaa ttgggggttg gggatcgggg gaggaacggg gcgtgcagga 3780

gaggtgggac ctcaacgtct gtctggaagc agagcctggg cccagtcgct gccatgtcag 3840

tgcttagagg tggtgataaa gagactctag agagagatag gtgtgacttc aaaagccagt 3900

ctactctggg catggtggct cacgcctcta atcccagggc tttgggagac ccaaggcggg 3960

aggattgctt aagcccagga gttccagacc agcctcggca acatagccag actcccatct 4020

ctacaaaaaa taaatgagca aggcgtgaag gcacatgtct gtagtcctag ctactctgga 4080

ggctgaggtg ggaggatctc ttgagcccag gagttcgagg ctgtagtgag ctatgattgc 4140

accactgcat tccatcctgg gccatagagg atgtcgctta aaacgaaaaa gaagaagaag 4200

aaagtcctgt ggtttgggaa gggaggctga gtgaggaggg gcctgtgtgc cagaggaggc 4260

ttcactgaga agcttagggg agcagatgtt ctaggggtac agaggtatgc aggaatagga 4320

agagtctcac cccgtgtctc tttttaggtc ctcgccctga acctgagcca cggcctccaa 4380

ccaccaccac accgcagccc acggctcccc cgacggtctg ccccaccgga cccccactg 4440

tccacccctc agagcgcccc acagctggcc ccacaggtcc cccctcagct ggccccacag 4500

gtccccccac tgctggccct tctacggcca ctactgtgcc tttgagtccg gtggacgatg 4560

cctgcaacgt gaacatcttc gacgccatcg cggagattgg gaaccagctg tatttgttca 4620

aggatgggtg aggaggcggg gttgtgtgga tgcgggaggg ggctttgcgg aggggctgcc 4680

cgtcccttcc cgcccactgg ccctgtgtcc aaggcttaga gcccgtcctt tccctcctcg 4740

ctttctcagg aagtactggc gattctctga gggcaggggg agccggccgc agggcccctt 4800

ccttatcgcc gacaagtggc ccgcgctgcc ccgcaagctg gactcggtct ttgaggagcg 4860

gctctccaag aagcttttct tcttctctgg ttagttacct actttccctc ccccgcccgg 4920

tcaatcccca tcagtcaagg aggctcaaga gaccatcgat aacccacgaa acgtcttgtg 4980

cgttttagaa aaatacgccc cctggcggac gcagtttagc aaacgtaggg gcggctgagt 5040
ttctgccccc tcctctccac gccctcgcgt cgctctaccc agcgcctctg cccctgggtt 5100
gcagggactg cgggcacgcg ggctaggaaa ggcctcgccg gaatctccct cctcgcgttc 5160
taggagtacg tgctccctct gcgcccccaa accgacgtga ccctcctccc ctgcagggcg 5220
ccaggtgtgg gtgtacacag gcgcgtcggt gctgggcccg aggcgtctgg acaagctggg 5280
cctgggagcc gacgtggccc aggtgaccgg ggccctccgg agtggcaggg ggaagatgct 5340
gctgttcagc gggcggcgcc tctggaggtg agcgccgccg cggccgccgg caggggggagc 5400
ccgggcgccg tcggtccgtc cgctagccgg ctcagcacct gtctcctccg cgcctgcccg 5460
caggttcgac gtgaaggcgc agatggtgga tccccggagc gccagcgagg tggaccggat 5520
gttccccggg gtgcctttgg acacgcacga cgtcttccag taccgaggtg agggctgagg 5580
aggatccctt cgtgagacac cacactaagc tcctcttagt gagtggtcaa attctgagcg 5640
aggaagaaaa agcccttgga aatggaaaca aatgccccag cacagacaag atcccagcag 5700
aggcagaggc cttctccagg tcatttagga agtcagggat gcaaccaaga ccaggaccca 5760
gatttcctgc ctccccggct ggaagctctt tctccttcag tacaggacgg caggtggttt 5820
gtatggaagc tcagttatta gacaacagtc atcaagtgcc gataatgtgc caggcactgt 5880
gctacaggga gagataagac aattcacagc tctgtgactt tgggcaagtc actgcttctc 5940
tactcttcga gcctcagttt ccccatctgt aatatgggga ctatagctgg aattacactt 6000
gacttccctt tcttaccagt cacatccaaa cagttgacaa ggtgaacaag atttcctgcc 6060
accaaaatct ttttcgagtc tgtcattttt tttgccatct tctttataaa caccccagcc 6120
caaaccatac tggctgtcca ggacctttaa caaattccat gagattagag agggggtagg 6180
agtgaagggc aatggtcttg ggagtgaccc cagatgaatt ccaaggtcaa agaaattaag 6240
aggatctgac actccacccc cgtgttctca tctcttccca ctcctcctgt tatttactct 6300
gctccaccca cactggctgc tctttgaaca gatcaaggtc attcctagct tacagccttt 6360
gtgccagttg ttccctctgt ctggaaagct tcccctccag attgtcactg ggccatccca 6420
ctgtcttcct tcaggtttca gtgctaaggc cattgcttca atgaggcctt ctttgatgct 6480
tattatctat ttacttgttt ttattttctc catagctttc tatattttct tttttttct 6540
tttttctttt ttttttttt tgagatggag tcttgctctg tcgcccaggc tggagtgcag 6600
tggcacgatc tcagctcact gcaacctccg cctcccgggt tcaagcgatt ctcctgcctc 6660
agcctcccaa gtagctggga ttacaggtgc ctgccaccac gcttggctaa ttttttgtat 6720
tttttagtag agacggggtt tcaccatctt ggccaggctg gtcttgaact cctgacctcg 6780
tgatccaccc gcctcagcct cccaaagtgc tgggattaca ggcatgagcc accgcaccca 6840

gccgctttct atattttcaa aaccaatctc atttatttat gtgtttgctt aattgtctct 6900

tgcctcacta gagtgtaagc accaagataa ttgagatcat gcctgcattt tttctgctta 6960

tccccagtat cttgaacaaa gcacatagta gatgctcagt aaatgatgaa tgaacagatt 7020

tgttcaatga atgagcgttg aatgaattgt tctgagcatt aagatagttg gtctattcat 7080

ttgttaattc attcacaaaa tgtgtatggt gtacctactg tgtgctaggc tctgtggcag 7140

tgctttgggc actgaggtct gtgccctcca gcatctcaca gaacctcaca gcatctcaca 7200

ggttgggggg atggaggtga tatgtgaaaa ccttagaaag ttctagaaat ggcagaagag 7260

atggttgtca agatcttgtt cctatttctg tatatgtggg agaattagaa tcactcctct 7320

tatgcctgcc tgtctcctgc agagaaagcc tatttctgcc aggaccgctt ctactggcgc 7380

gtgagttccc ggagtgagtt gaaccaggtg gaccaagtgg gctacgtgac ctatgacatc 7440

ctgcagtgcc ctgaggacta gggctcccgt cctgctttgg cagtgccatg taaatcccca 7500

ctgggaccaa ccctggggaa ggagccagtt tgccggatac aaactggtat tctgttctgg 7560

aggaaaggga ggagtggagg tgggctgggc cctctcttct cacctttgtt ttttgttgga 7620

gtgtttctaa taaacttgga ttctctaacc ttt 7653

<210> 63
<211> 26
<212> DNA
<213> Artificial

<220>
<223> Synthesis

<220>
<221> misc_signal
<222> (1)..(26)

<400> 63
cagtaccgag agaaagccta tttctg 26

<210> 64
<211> 22
<212> DNA
<213> Artificial

<220>
<223> Synthesis

<220>
<221> misc_signal
<222> (1)..(22)

<400> 64
taggtcacgt agcccacttg gt 22

<210> 65
<211> 2276
<212> DNA
<213> homo sapiens

<400> 65
aagcccagca gccccggggc ggatggctcc ggccgcctgg ctccgcagcg cggccgcgcg 60

cgccctcctg cccccgatgc tgctgctgct gctccagccg ccgccgctgc tggcccgggc 120

tctgccgccg gacgcccacc acctccatgc cgagaggagg gggccacagc cctggcatgc 180

agccctgccc agtagcccgg cacctgcccc tgccacgcag gaagcccccc ggcctgccag 240

cagcctcagg cctccccgct gtggcgtgcc cgacccatct gatgggctga gtgcccgcaa 300

ccgacagaag aggttcgtgc tttctggcgg gcgctgggag aagacggacc tcacctacag 360

gatccttcgg ttcccatggc agttggtgca ggagcaggtg cggcagacga tggcagaggc 420

cctaaaggta tggagcgatg tgacgccact cacctttact gaggtgcacg agggccgtgc 480

tgacatcatg atcgacttcg ccaggtactg gcatggggac gacctgccgt ttgatgggcc 540

tgggggcatc ctggcccatg ccttcttccc caagactcac cgagaagggg atgtccactt 600

cgactatgat gagacctgga ctatcgggga tgaccagggc acagacctgc tgcaggtggc 660

agcccatgaa tttggccacg tgctggggct gcagcacaca acagcagcca aggccctgat 720

gtccgccttc tacacctttc gctacccact gagtctcagc ccagatgact gcaggggcgt 780

tcaacaccta tatggccagc cctggcccac tgtcacctcc aggaccccag ccctgggccc 840

ccaggctggg atagacacca atgagattgc accgctggag ccagacgccc cgccagatgc 900

ctgtgaggcc tcctttgacg cggtctccac catccgaggc gagctctttt tcttcaaagc 960

gggctttgtg tggcgcctcc gtgggggcca gctgcagccc ggctacccag cattggcctc 1020

tcgccactgg cagggactgc ccagccctgt ggacgctgcc ttcgaggatg cccagggcca 1080

catttggttc ttccaaggtg ctcagtactg ggtgtacgac ggtgaaaagc cagtcctggg 1140

ccccgcaccc ctcaccgagc tgggcctggt gaggttcccg gtccatgctg ccttggtctg 1200

gggtcccgag aagaacaaga tctacttctt ccgaggcagg gactactggc gtttccaccc 1260

cagcacccgg cgtgtagaca gtcccgtgcc ccgcagggcc actgactgga gaggggtgcc 1320

ctctgagatc gacgctgcct tccaggatgc tgatggctat gcctacttcc tgcgcggccg 1380

cctctactgg aagtttgacc ctgtgaaggt gaaggctctg gaaggcttcc cccgtctcgt 1440

gggtcctgac ttctttggct gtgccgagcc tgccaacact ttcctctgac catggcttgg 1500

atgccctcag ggtgctgacc cctgccaggc cacgaatatc aggctagaga cccatggcc 1560

atctttgtgg ctgtgggcac caggcatggg actgagccca tgtctcctca gggggatggg 1620

gtggggtaca accaccatga caactgccgg gagggccacg caggtcgtgg tcacctgcca 1680

gcgactgtct cagactgggc agggaggctt tggcatgact taagaggaag ggcagtcttg 1740

ggcccgctat gcaggtcctg gcaaacctgg ctgccctgtc tccatccctg tccctcaggg 1800
tagcaccatg gcaggactgg gggaactgga gtgtccttgc tgtatccctg ttgtgaggtt 1860
ccttccaggg gctggcactg aagcaagggt gctggggccc catggccttc agccctggct 1920
gagcaactgg gctgtagggc agggccactt cctgaggtca ggtcttggta ggtgcctgca 1980
tctgtctgcc ttctggctga caatcctgga aatctgttct ccagaatcca ggccaaaaag 2040
ttcacagtca aatggggagg ggtattcttc atgcaggaga ccccaggccc tggaggctgc 2100
aacatacctc aatcctgtcc caggccggat cctcctgaag cccttttcgc agcactgcta 2160
tcctccaaag ccattgtaaa tgtgtgtaca gtgtgtataa accttcttct tctttttttt 2220
tttttaaact gaggattgtc attaaacaca gttgttttct aaaaaaaaaa aaaaaa 2276

<210> 66
<211> 11467
<212> DNA
<213> homo sapiens

<400> 66
aagcccagca gccccggggc ggatggctcc ggccgcctgg ctccgcagcg cggccgcgcg 60
cgccctcctg cccccgatgc tgctgctgct gctccagccg ccgccgctgc tggcccgggc 120
tctgccgccg gtgagtgccc gccactcgcc ggccgctcct cgctgagggg gcgccgggca 180
cgcgggctgg gcccagcggc ggatccggac cgaagggggc gccccgggtg gcctccagcg 240
cccggtaccc gaaacgcttt ctggttccct ctaggcgtga tagacagcga gcttgcagtc 300
cctgggggtg tgaaggggag ccggcgccgg catcgttcgg gctggtggga cgggactcca 360
cgctggactc acgcttgctc ccagcgtggg gacctgcctc tcgcgctcca gccgcgggtg 420
ctggagtgtg cgttgaagga agcagcagag ggagtggtaa cagggccccc tattcatcgc 480
agggacaaag ccgagcagat cccaggcagg tgtcagcctg caggtgtgtg gccgcagtta 540
gtacacctcc aggtgtgcgg tgggatgaag gataaaggga gaagggaggg cagcgctgtg 600
ctgcgggaaa tggggtctaa gcctggagat gtcccccgcg ggcacctgcc ggcagcagtt 660
ttgggaggct tggagccagg agaacatgag tatgaaatag tatgagtgca gtgtgtgtgt 720
gactgagagg tggctgtcag aaagaagcgg ggagagaaag gaaagggaga aaatgtgtgc 780
tcaggagagg agaagaaagc ccaggtaaga gaggacagca gagtgaggag aggagggact 840
gtcatggagt gtgtgtgaca gcttgcatgt gtcccaaaag ctgccccttc cctggggcgc 900
ttccaggcac cagtcacagg gcctggggcc agagcctggg ccactccact ccacagcgga 960
ggcatccaga cagctcgggc agggaaggga gcaggagggt gtgacaggca ggagtctcag 1020
gactggcctg gtgaggaggc aggcctggcc aggctgggac cctctgtcca gccactctgg 1080
ctctgctcag ggcagccttg tcctgtgctg gctatgggag cagaaaggga tgggatgggt 1140

ggggtagagt gaatgggggg catctcagtc tggagtcttg cctccttccc agcccctctt 1200

ggttcctagg acttgggaca gagtcaggaa tcactgtggg tagacattga accacaggtc 1260

tggaaattgg agagatctgg gtttgaacca tgtgaccctg ggtgaatcct gccatgcctc 1320

agaatctcac ccactccatc tctaatggga gtgcaggtgg gagggggcaa tggtgcctac 1380

tgctgtgtcc actgttgagc aatgaggtga tgccaggctc actagaaaca ctgtcacctg 1440

tagctgccac tttgacgctt gtgcatggtc agtgctggag ctgggggcca gcttggggtg 1500

gggtctgagc tggactgtgg tctgtcactc tgcaaacacg cagggagcat gtggggtcac 1560

ctctgatgtg tttatccccc ggctgccttc agcaaggctg ggagagctct gtaaatattt 1620

atccagccca gttcccagct ttcagggttg atggaagccc tgcagtccga tggatgggac 1680

gaagctaccg tccctcctgg agccagcagc agaggggtga gaataggaag attaggtgga 1740

agcttgaggg agaagatatc cctgcttgcc acctggctgt gtgagttggg caggttgctt 1800

gacctctctg agcctgcatg gtgatgctcc cgtcttcctc catagcctaa caagtgccct 1860

cctcgaagtc ttgcctccca ccgacatcag aaggcatagc tatgattaat tatacccact 1920

agaccaactg caaactgagg cccagaaagg ggcacaatga gcccagcctt tcgcagtgtt 1980

tcctggcacc ctggggtcct cccttatctc ccctctatct ggtgcctccc attttctgga 2040

ttgcaagttg accccagggc ggggttgact ccgagtctct aagctctgcc aggacactgg 2100

gtcagctgag aaattccttg aatgtgtctg cagctgaggt ttggtgtctg gcagaagagg 2160

gtggggctga gtgagctaag aacccaccac agggcaggcc aagggaggct actggccaag 2220

acagagggaa tgcactggaa gcagaaatgc ttcttgggaa agtgggtttt ggagaaaagc 2280

caagagctgg ggagacaggg ggaagccctg gaggtgggag gcatgagccc caggtctagg 2340

tccagctctt gcatgacttg ctgtgtgacc ccgggcaaag tccttgagct ctctgggctc 2400

tgtctcatcc tctgggaaat gggggagctg cttcttcctc aagctcccca ggggtgtaga 2460

tgaggccagt ggtggcctgt gtctaccacc tcacctcact gaatcctgag agtcctggga 2520

aggccaagcc cacctgtctc acaagtagca agctgcttca tgccgtgcct tgtagttagg 2580

gcatctggcc ccaggccaga aaggacttgg aaagatgaag tgtgggagga cagtgaggca 2640

gaatgtgtgt cctatcccag ccatggggaa actgaggcca agagctcatg ggctctaaaa 2700

aaataatggg ctctaaaaac aacctcacta gctagaggcc tcatttctag catctcctgc 2760

tcactcagtt tctaagaatt taatgaacaa gacagggaca gggagaagag accttgatat 2820

gtgggagacc ctgcacaagg cgcttctctt ttctaggcct gaaagctccc ttctagctgc 2880

aacttctgtt ttaccattta aagcctgaag attttgttgg gtagctagag gggcggtggg 2940

tgatgggaac tgtagggact ggcaagggca gattcccaag cgtgggaggg gagggcctac 3000

agggacaagg aacagggcca ggtaagaggt gcttttctgt gccagaggct acagcaggca 3060
ttacagaagg atgtcatgta gccctttgct gtggcatcat tcccatttga cagataggaa 3120
agttgaggct aagagaggaa ggtgacttgt ctagagtctc catgtcatag aactaggcct 3180
tctgaacctc agtggctggc tctttttgtg acactaagac tgtcatgatc caggggtttg 3240
ggcatggact gggtgcctca tgagggaagg acaggaaggg acagtggaag gggtgggggc 3300
atgcccgtgg ctctccctcc atgactgtct gcaccaggac tcactagagg gcagaggaga 3360
gaagagattt ctgaccatgg gcatagcagg gctggcaggc tgggctctgg gttgtttgga 3420
gctcccaggg gagagcacag ctctgccctt tccagggagg gtcttcatac ccctgccagg 3480
ctggggaaag gaatctgttt ctcctgtaag tcaagggggc agtgtggcga ggaggaggag 3540
gcaggcccct ctggcagccc acatcctcca tgggagaact tggatatgta gcatctccac 3600
ctgcctggtg gatattggaa gcccaacgaa cctgcctgca aaatgcccgg gaaatagcag 3660
gcgctgaata atttgcacct caccaaggtg aggccagcct gggtccctcc ttcaggggaa 3720
gggtaactca acccctgcag agcagagcag aggtagcagg gagctgggtg ggctgtgagc 3780
acagacagtc cgctgcctgc cagctgttgt ctgatcaagc tgcttaactc ctctggggcc 3840
catttcctca tcctggaaat gagggtgatg atggtggagt tggtgagagg ttccatcagg 3900
ggctaagagc aagtctgtgg agctgttgcc aaggtcctag cctgcaacca gtgctaagta 3960
cttttttttt ttttttgag atggagtctc gctctgtcac ccaggctgga gtgctgtggc 4020
acgatctcgg ctcactgcaa gctctgcctc ccaggttcac gccattctcc tgcctcagcc 4080
tcccgaatag ctgggactac aggagcccgc caccacgcct ggctaatttt ttgtattttt 4140
agtagagacg gggtttcacc gtgttagcca ggatggtctc gatctcctga cctcatgatc 4200
cgcctgcctc ggcctcccaa agtgctggga ttacaggcgt gagccaccag gcccggccaa 4260
ccagtgctaa gtacttatta acaataagcc caggccgggc gcggtggctc acgcctgtaa 4320
tcccagtact ttgggaggcc gaggcaggtg gatcatgagg tcaggagttc aagaccagcc 4380
tggccaagat ggataaacct catctctact aaaaatacaa aaattagcca ggcgtgaggc 4440
tgggtgcggt ggcgcatgct tgtaatccca gcactttggg aggccgaggc aggcggatca 4500
caaggtcagg agatcgagac catcctggta acatggtgaa atcccgtttc tactaaaaat 4560
acaaaaaaat tagccgggca tggcagcaag tgcctgcagt cccagctact caggaggctg 4620
agtcaggagc atggcatgaa cccggaaggc ggtggttgta gtgagcccag atcacaccac 4680
tgcactccag cctgggcgat agagcgagac tctctctcaa aaaaaaaaaa aaaaaaatta 4740
accaggcgtg gtggcaggca cctgtaaccc cagctactcg ggaggctgag gcagagaatt 4800
gcttgaaccc gggaggtgga ggttgtagtg agccgagatc gcgccactgc actccagcct 4860
gggcaacaga gtgagactcc atctcaaaaa aacaaaacaa aaaaccaata agcccagaat 4920

cggccagaac ccacaaccca gtgagactgc gctgtgccag gtaaccatgc aataagcaag 4980

ctcggaaatg gggggaagct gcttcagggc ctcacgccct aagtagttcc atagcctccg 5040

tgttcagaag gcccccttc atggtggggt gttcttgatg cctcagctct gggatcagga 5100

gcagggagcg ttgggacgct gatcagatcc ctggggtgta tggagcctgg gagagctgcc 5160

aaaggctgag ggtgaggtgg ggcctgagtg gctgagctcc taccccaaat atggctgtga 5220

ggaggctgca gttgcccaga ccagacaggt gctgagtctc tcagcaagca gcacagctcc 5280

atccctctcc ttcagtgcag gaaggacact tggcttctgt gcggtgtcca gagccaggcc 5340

ttagccttag gcctgagcca ccagagtcct ggcctggcct tgccatgccc ttgctgggtg 5400

actttgcagg gtctccaacc tctctgagct tctgcttttc tcatgcacaa gaactgtaac 5460

ttctgccctg gagacttata gacaggtagc aggatgtagc ttaggtctga atcgccatct 5520

gtggtcctgg ggctttggcg agtggggctg ggcacctggt aattaactgt ccccaccctc 5580

cccgcttgaa gaaggcaggc agatcacaga tcagctccca ctgtactcct agccctggtg 5640

gggtgtgacc aaaaccacct ctgctagaag ccaggcctca gtggccaggt gccttcccgg 5700

gtgctgggcc tgtgctaggt gctgtacata cctcaccttg tccagtccac aagtcagtgt 5760

cacccccagc agtcaggtta cactgactga ggccacactg cagggctaca gccgctccac 5820

actggggagg aggtggggga aatcctgggc aggagagagg gcagatcctg ctcacatgac 5880

agggacagga gcatgaccca ggtgtgtctg aacttagcgg acacaggaag aaagggagtt 5940

ggaagcaagt ttctgtgggg agcaggagga ggttgcctgg tgttccttcg gaggaagctt 6000

tttggggtcc attcctggag tgtatggctc atagccagtc ccagtgtgcc cccaccccca 6060

gacctcattg gcctaagtag ctggagtagg tgacaggcag cccagggccc tccacgatgt 6120

gggggacagc ttgatgcctt ggaacaaggt gccaagaaac cagagagcca gccagatgcc 6180

aaagggccct gccatgtgcc ggtgcccttt ccctctccat ttgcccagcc acacagtggg 6240

ctggggttgc acgtgtgttt gctgacaggc cacatctcta actgtgggcc atgtggacct 6300

taggcctgac cagaccctca tgtcatcctc ctgcctagga cgcccaccac ctccatgccg 6360

agaggagggg gccacagccc tggcatgcag ccctgcccag tagcccggca cctgcccctg 6420

ccacgcagga agccccccgg cctgccagca gcctcaggcc tccccgctgt ggcgtgcccg 6480

acccatctga tgggctgagt gcccgcaacc gacagaagag gttcgtgctt tctggcgggc 6540

gctgggagaa gacggacctc acctacaggt aggggcctgg gagcaggaca ctaggatgcc 6600

acctgtgtgt ccgtgggtaa gccagctgcc ctcacagctg ctgcttgaga cacaggccag 6660

ggtagatctt cgtgtctaac agacctgtgt gtccactgaa ccccagggag gtcatctatg 6720

ggcaaacccc ctgaaacccc aacttagaca catacacata tggagaccct ccctcagcag 6780

aggggcagag cctccgtcat catgcaaaga gtcgcagcac atgcctgcgg acgggtgttc 6840
agtcactcag gcagccttta caagagacct gtgaggacca ggctctggga ctccacggtg 6900
aatgaggcag acacagcccc atcctctgtg tcagtctgag gtgggtgtca gccatgtcat 6960
tgtccaactc taccatcaca acttgggctt cgagcaggtg gagacagtgg taagcgggga 7020
gaggcaatag tgggcatctc actgggtgac ctgggaggac cctgggcagg tgatggggaa 7080
gctgaggctc acacatcctg cgggtgggga cccagcctga agaatgggct ggtgtcacac 7140
agcattggag ctgagactgg ggtctttaga atttcctagg tgggggcctg ggaaccaaca 7200
ggggctcaag gaaccaaggt gtccccacag taagtggcac tgtcaggtct aggatggggg 7260
tctcgggacc cctggtcctg gttctttcca ctgaattcag acacttgtat ttgcctaagt 7320
atgagcaaac cacatacaca tgtgcccatg tggccaggga gaccagtgcg ctgaagctga 7380
ggcccagagt acacctggcc tgtgtcctga gtgttcacac acccaccaag catccagggg 7440
caactcctgg tgcctcagcc atcgggggct gtcccttccc tgaggcccag gcccctccat 7500
ctccctccag gatccttcgg ttcccatggc agttggtgca ggagcaggtg cggcagacga 7560
tggcagaggc cctaaaggta tggagcgatg tgacgccact cacctttact gaggtgcacg 7620
agggccgtgc tgacatcatg atcgacttcg ccaggtgaat gggcggcctg ggacccctcc 7680
gggaacagcc tcgcctgcca gcagccactg accccgcccc cacccatctg taggtactgg 7740
catggggacg acctgccgtt tgatgggcct gggggcatcc tggcccatgc cttcttcccc 7800
aagactcacc gagaagggga tgtccacttc gactatgatg agacctggac tatcggggat 7860
gaccagggta tgggctgggg acccattttc cagatggggc aaccgaagat cataaagaat 7920
ggggactcgc caaggtcact gagctggggt ctggagctgg atgtcctggg caggaggttc 7980
gggggttgct gagccacctc cctttttcag gcacagacct gctgcaggtg gcagcccatg 8040
aatttggcca cgtgctgggg ctgcagcaca caacagcagc caaggccctg atgtccgcct 8100
tctacacctt tcgctaccca ctgagtctca gcccagatga ctgcaggggc gttcaacacc 8160
tatatggcca gccctggccc actgtcacct ccaggacccc agccctgggc ccccaggctg 8220
ggatagacac caatgagatt gcaccgctgg aggtgaggcc ctgcctgcca gtccccctac 8280
tcctctgctg gccactgtga ctgcagcata tgccctcagc atgtgtccct ctctcccacc 8340
ccagccagac gccccgccag atgcctgtga ggcctccttt gacgcggtct ccaccatccg 8400
aggcgagctc tttttcttca aagcgggctt tgtgtggcgc ctccgtgggg gccagctgca 8460
gcccggctac ccagcattgg cctctcgcca ctggcaggga ctgcccagcc ctgtggacgc 8520
tgccttcgag gatgcccagg gccacatttg gttcttccaa ggtgagtggg ggttggggat 8580
ctgctcgaga gacttcccag agccaggaat gttatggcca agggcaggaa cagacagatg 8640
gatccttagg gacacagtgg atagggagag ctgccccaaa gcctgggggc cgagggagag 8700

agagtgtggt ttgttcctca ggcacaggta ggaggttctc ggaggtggct cttgagatag 8760
gagcagcgtg gaagggattg cacggtgggg cctcgtgttg gtgcgttcaa ccctcagcca 8820
ccccatgggg cggggttcta gagatgaggc ctctggggcc ccgaggcagt gaagtgactc 8880
actgtgagtg cagctgggaa gaggcagggc agggaattga tccaggtcta tcatcctaga 8940
gctgggattt ccatcctcaa ctggcagaga tgagagcctg gagcattgca gatgccaggg 9000
acttcacaaa tgaaggcaca gcatgggaaa cctgcgtggg ttccagggca gtccagcctg 9060
caggggccca gggagtggtc agtaggcatt tgtcacagcc aaatgccagt ggaaggagca 9120
gccgcccagg cagccctcta ctgatgagag taacctcacc cgtgcactag tttacagagc 9180
attcactgcc ccagcttatc ccaggcctcc cgcttccctc tgcgggtggg gtgctgagca 9240
ggcattattg gcctgcatgt tttactgatg aggaaactga ggctgggaga gtctgtggta 9300
ggggtcaagc aggtccacag tggcggggca tggcagtggt ggctgggcag gtccttgcag 9360
ccttccctct ccggcaggtg ctcagtactg ggtgtacgac ggtgaaaagc cagtcctggg 9420
ccccgcaccc ctcaccgagc tgggcctggt gaggttcccg gtccatgctg ccttggtctg 9480
gggtcccgag aagaacaaga tctacttctt ccgaggcagg gactactggc gtttccaccc 9540
cagcacccgg cgtgtagaca gtcccgtgcc ccgcagggcc actgactgga gaggggtgcc 9600
ctctgagatc gacgctgcct tccaggatgc tgatggtgcg ttgggggtga ggcagctggt 9660
gggaggtggg cacagcagcc gcttctccca cctggtggtg gctgggctcc cacatgcctg 9720
ccacaggaag tctggctctt catcacaggt cctttgtcca gagccatctg ccctcctctc 9780
ggtggccggc tagtgctaca ttccatattg cagatgagga aactgagggt cagagaagtg 9840
caaggtctta ccctggtttt tcagccacag ccagtagaac aataaactgc tgtacactga 9900
gggccaacaa tgctctaagc tccttactgg tctcatccag ttctcagaac agccctctga 9960
tgtgacacct gttgtgaacc cagtttccag aggagcaaac agaggctcag gcaatgaggc 10020
ccctaacctg gactaccctg gtggtccctg ctcctaacca ctgacccacc cagcctccca 10080
caaccacagg gggctagagc cagtccagtg ctccctcccc tgctaggctc ctcttctgtg 10140
ctctttctcc cacatcagga cccactggga gagctatcct agggtagcct ccagctccag 10200
gactccaggg tgcccgtcaa tagcctggct aatttaatag atgcaggaga gagtgatgtg 10260
gagggtggtg gggcaacgg gacttgcttt cctgagaggt gggactcagg cctctgaggc 10320
tctgggtacc tgtcaggctg ggtattagcc cagcccagat tccggggcag gcagaagggc 10380
tccctagagg gaagagaggt tctgaaaggc cggccctgga tcctgcagga ctcgaggaac 10440
tcagcagtgg ccaagggctt cccactcagc cctcccttag tgcccatccc tgggcacagc 10500
ctgacaggca ggagtagggc ccagtgtccg ctcgcccagg cttgaccacc ttctcttctc 10560

aggctatgcc tacttcctgc gcggccgcct ctactggaag tttgaccctg tgaaggtgaa 10620

ggctctggaa ggcttccccc gtctcgtggg tcctgacttc tttggctgtg ccgagcctgc 10680

caacactttc ctctgaccat ggcttggatg ccctcagggg tgctgacccc tgccaggcca 10740

cgaatatcag gctagagacc catggccatc tttgtggctg tgggcaccag gcatgggact 10800

gagcccatgt ctcctcaggg ggatggggtg gggtacaacc accatgacaa ctgccgggag 10860

ggccacgcag gtcgtggtca cctgccagcg actgtctcag actgggcagg gaggctttgg 10920

catgacttaa gaggaagggc agtcttgggc ccgctatgca ggtcctggca aacctggctg 10980

ccctgtctcc atccctgtcc ctcagggtag caccatggca ggactggggg aactggagtg 11040

tccttgctgt atccctgttg tgaggttcct tccaggggct ggcactgaag caagggtgct 11100

ggggccccat ggccttcagc cctggctgag caactgggct gtagggcagg gccacttcct 11160

gaggtcaggt cttggtaggt gcctgcatct gtctgccttc tggctgacaa tcctggaaat 11220

ctgttctcca gaatccaggc caaaaagttc acagtcaaat ggggaggggt attcttcatg 11280

caggagaccc caggccctgg aggctgcaac atacctcaat cctgtcccag gccggatcct 11340

cctgaagccc ttttcgcagc actgctatcc tccaaagcca ttgtaaatgt gtgtacagtg 11400

tgtataaacc ttcttcttct tttttttttt ttaaactgag gattgtcatt aaacacagtt 11460

gttttct 11467

<210> 67
<211> 23
<212> DNA
<213> Artificial

<220>
<223> Synthesis

<220>
<221> misc_signal
<222> (1)..(23)

<400> 67
aagacggacc tcacctacag gat 23

<210> 68
<211> 18
<212> DNA
<213> Artificial

<220>
<223> Synthesis

<220>
<221> misc_signal
<222> (1)..(18)

<400> 68
ggcgtcacat cgctccat 18

<210> 69
<211> 5003
<212> DNA
<213> homo sapiens

<400> 69
ggatccattt tataagctca aagataatta cttttcagac taagaatatt tagggtaaaa 60

agtactgttc aacatctcta ctgaggatgt tatgatgtag cacactctat aagctggagc 120

taaaggaaac tttccttaaa gtgctattta ctaaaaattg gaacacattc cttaagacaa 180

atcgaagtgt ggcacacaac atccaaactt ccatcataga tacagaggtg ttaccatctc 240

ccactcccaa atttctttgt cacgctgagg atactcaaga ggagcaggac atgttggtcg 300

cagcaggaga aacttgaaag cattcacttt tatggaactc ataagggaga gaatctctta 360

tttagtatcg tccttgatac atttattatt ttaaaagata atgtagccaa atgtcttcct 420

ctgtgttaaa tctttacaaa actgaaatct taaaatggtg acaaaaattc tacttctgat 480

agaatctatt catttttcca attagatagg gcataattct taatttgcaa aacaaaacgt 540

aatatgctta tgaggttcca tcccaaagaa cctgctattg agagtagcat tcagaataac 600

gggtggaaat gccaactcca gagtttcaga tcctaccggt aattggggta gggaggggct 660

ttgggcgggg cctccctaga ggaggaggcg ttgttagaaa gctgtctggc cagtccacag 720

ctgtcactaa tcggggtaag ccttgttgta tttgcgcgtg tgggtggcat tctcaatgag 780

aactagcttc acttgtcatt tgagtgaaat ctacaacccg aggcggctag tgctcccgca 840

ctactgggat ctgagatctt cggagatgac tgtcgcccgc agtacggagc cagcagaagt 900

ccgacccttc ctgggaatgg gctgtaccga gaggtccgac tagccccagg gttttagtga 960

gggggcagtg gaactcagcg agggactgag agcttcacag catgcacgag tttgatgcca 1020

gagaaaaagt cgggagataa aggagccgcg tgtcactaaa ttgccgtcgc agccgcagcc 1080

actcaagtgc cggacttgtg agtactctgc gtctccagtc ctcggacaga agttggagaa 1140

ctctcttgga gaactccccg agttaggaga cgagatctcc taacaattac tactttttct 1200

tgcgctcccc acttgccgct cgctgggaca aacgacagcc acagttcccc tgacgacagg 1260

atggaggcca agggcaggag ctgaccagcg ccgccctccc ccgcccccga cccaggaggt 1320

ggagatcctc cggtccagcc acattcaaca cccactttct cctccctctg cccctatatt 1380

cccgaaaccc cctcctcctt cccttttccc tcctccctgg agacggggga ggagaaaagg 1440

ggagtccagt cgtcatgact gagctgaagg caaagggtcc ccgggctccc cacgtggcgg 1500

gcggcccgcc ctcccccgag gtcggatccc cactgctgtg tcgcccagcc gcaggtccgt 1560

tcccggggag ccagacctcg gacaccttgc ctgaagtttc ggccatacct atctccctgg 1620

acgggctact cttccctcgg ccctgccagg gacaggaccc ctccgacgaa aagacgcagg 1680
accagcagtc gctgtcggac gtggagggcg catattccag agctgaagct acaaggggtg 1740
ctggaggcag cagttctagt cccccagaaa aggacagcgg actgctggac agtgtcttgg 1800
acactctgtt ggcgccctca ggtcccgggc agagccaacc cagccctccc gcctgcgagg 1860
tcaccagctc ttggtgcctg tttggccccg aacttcccga agatccaccg gctgcccccg 1920
ccacccagcg ggtgttgtcc ccgctcatga gccggtccgg gtgcaaggtt ggagacagct 1980
ccgggacggc agctgcccat aaagtgctgc cccggggcct gtcaccagcc cggcagctgc 2040
tgctcccggc ctctgagagc cctcactggt ccggggcccc agtgaagccg tctccgcagg 2100
ccgctgcggt ggaggttgag gaggaggata gctctgagtc cgaggagtct gcgggtccgc 2160
ttctgaaggg caaacctcgg gctctgggtg gcgcggcggc tggaggagga gccgcggctt 2220
gtccgccggg ggcggcagca ggaggcgtcg ccctggtccc caaggaagat tcccgcttct 2280
cagcgcccag ggtcgccctg gtggagcagg acgcgccgat ggcgcccggg cgctccccgc 2340
tggccaccac ggtgatggat ttcatccacg tgcctatcct gcctctcaat cacgccttat 2400
tggcagcccg cactcggcag ctgctggaag acgaaagtta cgacggcggg gccggggctg 2460
ccagcgcctt tgccccgccg cggacttcac cctgtgcctc gtccaccccg gtcgctgtag 2520
gcgacttccc cgactgcgcg tacccgcccg acgccgagcc caaggacgac gcgtaccctc 2580
tctatagcga cttccagccg cccgctctaa agataaagga ggaggaggaa ggcgcggagg 2640
cctccgcgcg ctccccgcgt tcctaccttg tggccggtgc caaccccgca gccttcccgg 2700
atttcccgtt ggggccaccg cccccgctgc cgccgcgagc gaccccatcc agacccgggg 2760
aagcggcggt gacggccgca cccgccagtg cctcagtctc gtctgcgtcc tcctcggggt 2820
cgaccctgga gtgcatcctg tacaaagcgg agggcgcgcc gccccagcag ggcccgttcg 2880
cgccgccgcc ctgcaaggcg ccgggcgcga gcggctgcct gctcccgcgg gacggcctgc 2940
cctccacctc cgcctctgcc gccgccgccg gggcggcccc cgcgctctac cctgcactcg 3000
gcctcaacgg gctcccgcag ctcggctacc aggccgccgt gctcaaggag ggcctgccgc 3060
aggtctaccc gccctatctc aactacctga ggccggattc agaagccagc cagagcccac 3120
aatacagctt cgagtcatta cctcagaaga tttgtttaat ctgtggggat gaagcatcag 3180
gctgtcatta tggtgtcctt acctgtggga gctgtaaggt cttctttaag agggcaatgg 3240
aagggcagca caactactta tgtgctggaa gaaatgactg catcgttgat aaaatccgca 3300
gaaaaaactg cccagcatgt cgccttagaa agtgctgtca ggctggcatg gtccttggag 3360
gtcgaaaatt taaaaagttc aataaagtca gagttgtgag agcactggat gctgttgctc 3420
tcccacagcc attgggcgtt ccaaatgaaa gccaagccct aagccagaga ttcacttttt 3480

caccaggtca agacatacag ttgattccac cactgatcaa cctgttaatg agcattgaac 3540

cagatgtgat ctatgcagga catgacaaca caaaacctga cacctccagt tctttgctga 3600

caagtcttaa tcaactaggc gagaggcaac ttctttcagt agtcaagtgg tctaaatcat 3660

tgccaggttt tcgaaactta catattgatg accagataac tctcattcag tattcttgga 3720

tgagcttaat ggtgtttggt ctaggatgga gatcctacaa acatgtcagt gggcagatgc 3780

tgtattttgc acctgatcta atactaaatg aacagcggat gaaagaatca tcattctatt 3840

cattatgcct taccatgtgg cagatcccac aggagtttgt caagcttcaa gttagccaag 3900

aagagttcct ctgtatgaaa gtattgttac ttcttaatac aattcctttg gaagggctac 3960

gaagtcaaac ccagtttgag gagatgaggt caagctacat tagagagctc atcaaggcaa 4020

ttggtttgag gcaaaaagga gttgtgtcga gctcacagcg tttctatcaa cttacaaaac 4080

ttcttgataa cttgcatgat cttgtcaaac agcttcatct gtactgcttg aatacattta 4140

tccagtcccg ggcactgagt gttgaatttc cagaaatgat gtctgaagtt attgctgcac 4200

aattacccaa gatattggca gggatggtga aaccccttct ctttcataaa aagtgaatgt 4260

catctttttc ttttaaagaa ttaaattttg tggcatgtct ttttgttttg gtcaggatta 4320

tgaggtcttg agtttttata atgttcttct gaaagcctta catttataac atcatagtgt 4380

gtaaatttaa aagaaaaatt gtgaggttct aattattttc ttttataaag tataattaga 4440

atgtttaact gttttgttta cccatatttt cttgaagaat ttacaagatt gaaaaagtac 4500

taaaattgtt aaagtaaact atatcttatc catattattt cataccatgt aggtgaggat 4560

ttttaacttt tgcatctaac aaatcatcga cttaagagaa aaaatcttac atgtaataac 4620

acaaagctat tatatgttat ttctaggtaa ctccctttgt gtcaattata tttccaaaaa 4680

tgaaccttta aaatggtatg caaaattttg tctatatata tttgtgtgag gaggaaattc 4740

ataactttcc tcagattttc aaaagtattt ttaatgcaaa aaatgtagaa agagtttaaa 4800

accactaaaa tagattgatg ttcttcaaac taggcaaaac aactcatatg ttaagaccat 4860

tttccagatt ggaaacacaa atctcttagg aagttaataa gtagattcat atcattatac 4920

aaatagtatt gtgggttttg taggttttta aaataacctt ttttggggag agaattgtcc 4980

tctaatgagg tattgcgagt ggc 5003

<210> 70
<211> 92153
<212> DNA
<213> homo sapiens

<400> 70
ggatccattt tataagctca aagataatta cttttcagac taagaatatt tagggtaaaa 60

agtactgttc aacatctcta ctgaggatgt tatgatgtag cacactgtat aagctggagc 120

taaaggaaac tttccttaaa gtgctattta ctaaaaattg gaacacattc cttaagacaa 180
atcgaagtgt ggcacacaac atccaaactt ccatcataga tacagaggtg ttaccatctc 240
ccactcccaa atttctttgt cacgctgagg atactcaaga ggagcaggac atgttggtcg 300
cagcaggaga aacttgaaag cattcacttt tatggaactc ataagggaga gaatctctta 360
tttagtatcg tccttgatac atttattatt ttaaaagata atgtagccaa atgtcttcct 420
ctgtgttaaa tctttacaaa actgaaatct taaaatggtg acaaaaattc tacttctgat 480
agaatctatt catttttcca attagatagg gcataattct taatttgcaa aacaaaacgt 540
aatatgctta tgaggttcca tcccaaagaa cctgctattg agagtagcat tcagaataac 600
gggtggaaat gccaactcca gagtttcaga tcctaccggt aattggggta gggaggggct 660
ttgggcgggg cctccctaga ggaggaggcg ttgttagaaa gctgtctggc cagtccacag 720
ctgtcactaa tcggggtaag ccttgttgta tttgtgcgtg tgggtggcat tctcaatgag 780
aactagcttc acttgtcatt tgagtgaaat ctacaacccg aggcggctag tgctcccgca 840
ctactgggat ctgagatctt cggagatgac tgtcgcccgc agtacggagc cagcagaagt 900
ccgacccttc ctgggaatgg gctgtaccga gaggtccgac tagccccagg gttttagtga 960
gggggcagtg gaactcagcg agggactgag agcttcacag catgcacgag tttgatgcca 1020
gagaaaaagt cgggagataa aggagccgcg tgtcactaaa ttgccgtcgc agccgcagcc 1080
actcaagtgc cggacttgtg agtactctgc gtctccagtc ctcggacaga agttggagaa 1140
ctctcttgga gaactccccg agttaggaga cgagatctcc taacaattac tactttttct 1200
tgcgctcccc acttgccgct cgctgggaca aacgacagcc acagttcccc tgacgacagg 1260
atggaggcca agggcaggag ctgaccagcg ccgccctccc ccgcccccga cccaggaggt 1320
ggagatccct ccggtccagc cacattcaac acccactttc tcctccctct gcccctatat 1380
tcccgaaacc ccctcctcct tccctttcc ctcctcctgg agacgggga ggagaaaagg 1440
ggagtccagt cgtcatgact gagctgaagg caaagggtcc ccgggctccc cacgtggcgg 1500
gcggcccgcc ctcccccgag gtcggatccc cactgctgtg tcgcccagcc gcaggtccgt 1560
tcccggggag ccagacctcg gacaccttgc ctgaagtttc ggccatacct atctccctgg 1620
acgggctact cttccctcgg ccctgccagg gacaggaccc ctccgacgaa aagacgcagg 1680
accagcagtc gctgtcggac gtggagggcg catattccag agctgaagct acaaggggtg 1740
ctggaggcag cagttctagt cccccagaaa aggacagcgg actgctggac agtgtcttgg 1800
acactctgtt ggcgccctca ggtcccgggc agagccaacc cagccctccc gcctgcgagg 1860
tcaccagctc ttggtgcctg tttggccccg aacttcccga agatccaccg gctgccccccg 1920
ccacccagcg ggtgttgtcc ccgctcatga gccggtccgg gtgcaaggtt ggagacagct 1980
ccgggacggc agctgcccat aaagtgctgc cccggggcct gtcaccagcc cggcagctgc 2040

tgctcccggc ctctgagagc cctcactggt ccggggcccc agtgaagccg tctccgcagg 2100
ccgctgcggt ggaggttgag gaggaggatg gctctgagtc cgaggagtct gcgggtccgc 2160
ttctgaaggg caaacctcgg gctctgggtg gcgcggcggc tggaggagga gccgcggctg 2220
tcccgccggg ggcggcagca ggaggcgtcg ccctggtccc caaggaagat tcccgcttct 2280
cagcgcccag ggtcgccctg gtggagcagg acgcgccgat ggcgcccggg cgctccccgc 2340
tggccaccac ggtgatggat ttcatccacg tgcctatcct gcctctcaat cacgccttat 2400
tggcagcccg cactcggcag ctgctggaag acgaaagtta cgacggcggg gccggggctg 2460
ccagcgcctt tgccccgccg cggagttcac cctgtgcctc gtccaccccg gtcgctgtag 2520
gcgacttccc cgactgcgcg tacccgcccg acgccgagcc caaggacgac gcgtaccctc 2580
tctatagcga cttccagccg cccgctctaa agataaagga ggaggaggaa ggcgcggagg 2640
cctccgcgcg ctccccgcgt tcctaccttg tggccggtgc caaccccgca gccttcccgg 2700
atttcccgtt ggggccaccg cccccgctgc cgccgcgagc gaccccatcc agacccgggg 2760
aagcggcggt gacggccgca cccgccagtg cctcagtctc gtctgcgtcc tcctcggggt 2820
cgaccctgga gtgcatcctg tacaaagcgg agggcgcgcc gccccagcag ggcccgttcg 2880
cgccgccgcc ctgcaaggcg ccgggcgcga gcggctgcct gctcccgcgg gacggcctgc 2940
cctccacctc cgcctctgcc gccgccgccg gggcggcccc cgcgctctac cctgcactcg 3000
gcctcaacgg gctcccgcag ctcggctacc aggccgccgt gctcaaggag ggcctgccgc 3060
aggtctaccc gccctatctc aactacctga ggtgagggcc cgggacgggg cacgcccagc 3120
gcgtccggga gtagcggttc cgttggcggc ggcggccgcc aaccctcagc cccagcccca 3180
gcgcaccgct gcgctccccg gggcggccgg agagggtggg cagcgggaca cagcacaggg 3240
gcagttgcct cccttcttct tccctcctct cctcactctt ggggacacga aggtgggcgc 3300
agaatatact atttttgggg cgtgcctccc tgaaagctgt ttttttgttt gtttttttaac 3360
tttccgaatc ttccagattc cgaagcagaa ccaaccccga tttaaaacgt gcagcgtcac 3420
actaggtccg ctgtagccca gtggggcaga aagtgcgcgg cgagttgggg gctttatgaa 3480
atgcttcttt cttagaagaa ggacgtttac caggagtgct tgtcttggag aggagttaag 3540
gcaccgttcc cccgggaggg gtgggacttg agaggtggcc ggccagaacc gaaagcagca 3600
ccatcttagg gatttgaaca cttcagtggc tcagttttct taagaatctc aagattaaaa 3660
ttaagttcac gtgggaaatg tttaaactgt ggatttaaac gcctgtcact gcattgcacc 3720
gttttcttat tattgcttgc tattcactac aatttttttt atatacaggt ttaaaaaaca 3780
ctactttgca tactgaagta atggaatgta aaaaaagaat gctctgtttg gaatcttatg 3840
ttgtgaatag gcaaaacagt gtcagtgtat tggacaatac tttaaaatga caaacatata 3900

cttgcttaag taagcaatga ttacagggtt gtgttttaaa aactcaaaac caaaacattg 3960
caaagtacca tcgaactttt aaagccaaac catatttgtt ttgacccagc atacagacag 4020
gaaggacata acatttcatt tgtcaaagac taaattgttt ctatataaag agttttgtag 4080
aaagatttcc ttttaaccga ctttaacttt ctaggacata atattataca ctaattattg 4140
ttcttttata ttggtgctac tgatgaatgg ctaatcattt gcaagtatgg tgaatccagt 4200
tacggatagt ctattaccaa gtttagtttg catgtctttc aagtgtatat atacagttct 4260
gtttttaaaa tctcctttca ccctgttaat actggtttaa gaaaccttta gtattagata 4320
gtggtgcact taaaaataaa tggagtactt tgttttgcat ttcaaggccg gattcagaag 4380
ccagccagag cccacaatac agcttcgagt cattacctca gaagatttgt ttaatctgtg 4440
gggatgaagc atcaggctgt cattatggtg tccttacctg tgggagctgt aaggtcttct 4500
ttaagagggc aatggaaggt aggctccttt cccctgatcc tttattattg gtttaattgt 4560
aaatggagac catctaatat tgtatagatt tcaattattc cttgtttctt ataagaaatg 4620
gtgatatttc catataattt aaaatatatg atgacatttt aacaatatgt ttttatttat 4680
gatactcaaa atggaatgtg gttgggtact ataattgcat actctttgac taacactttc 4740
agtattagac ataagtcata aaaatcttga ggacagtgct actattgttc cttaactgct 4800
tagctttgag gaaacagctt tgttctaata gtacttttat atatctatta tgtaggtata 4860
tgtgtatgca gtactttaaa attttgatta aaagaaaaat ggtagttgac acatatgtac 4920
atgtatgcgt atacatattt gtacatacac gaacatatat cacaacatgt atatgatgca 4980
gttttctaca tgatactgtc ttttgactac atgaatattt atgtaatatt tacaaagaag 5040
taattctaaa caaatttttg aattctcttt ttgttcagta tatttttgta agtgtataag 5100
aggacaggat taaacagttt aaaataaaaa acctggacat cacagtaaca taatttctaa 5160
agaagtattt tgctttaagt aaaactttca tgtttttaaa ctcattgaac ttacatgctt 5220
aatgatttca gatttacttg catagtgttt cagattttaa ctttcaaaga aaatattttt 5280
gaatttcttt ctacctaaag tctaagcagc caaacatctt tacatttgaa gataaaaata 5340
cattgaaaga tttcatattt taataccagc aataaatgta ttctataact atgtaaaatg 5400
aagcttagga ttccctctgg agtgctgaga tcacacctag acaaggaacc aaggatctga 5460
atgttggctt tttgtttcct gttctgagga tttttgtttg tttgtttgtt tcaacagccc 5520
tctccttaca agcagaaaag caggtagtag gaaatttcac tttaagggag ctttcaaagg 5580
agttcttcat aacaaatatt tgctttgtat atttttagaa catgattttt tctcacaaaa 5640
gatgaatgta ttttactgat gttgaacata ttcagcttta gggggtttga ttgcatttta 5700
aactaattga ggcagtgtta aaagtggtac ttgagaaaat agggcaactg atagtggctc 5760
ttacccattg acattattta tttacagtta cagttggaag ttctttgtgt ggaaaagtca 5820

gttttccaat gggtaattgg agttaccatt tttatctgac tttggttctg gtttcttaaa 5880
cattgccttc tgcattaata tgatttcctc tcttcttaaa gtctcctaag gatagtataa 5940
ttttataagt gaatgactcc cttagaatcc tcttaagccc aatttgtcct accccagctc 6000
cttcttttga aagataagca aatctcaaag acataaatat gagtttccaa aggtcacaaa 6060
actagttagt agctgattgt tagtaaacat aggttaaata tttttacatt gcagcgcttg 6120
taaatcagag atgatatgca aaagtagata tataaactgt ttgattcaca gaagttattt 6180
cataaagtgc atatatagaa caaagagcac cctaactaaa atacaaatgc tttctcgtca 6240
ttttgttaga atagcatcca aaactgtaga cgaagtcttt ccaaatgtac tcttagaaag 6300
catttgttgg actccggctg ttggcatggt cctatagtct tgagtactag aagtgaagca 6360
cctttattta gcagtaatta caaagagtta cttaagattg atgcagataa atcattcatg 6420
aaactagaac aagattatga actacattag taagttcctt cattcagcaa tttatgccaa 6480
agatacactt tccctgactt cacttttctc tgccttgaga taaaatgagg ataacagtgg 6540
ctatttctta gggttgctat aaagattaaa tgagctgata cttgtaaagt atgtaaaaga 6600
aggcctgaca tattatcagt ttccattgac atttctactt tcaaggaact tgtaatatag 6660
ttagggaggt aacatatgca cataaaacat ctaaataaag attctcagta aatgcccaag 6720
taagcaattc tgtaatgtat atgagatctg tgtggtttgt gagtttttgt atttggacag 6780
agcgaggtgg ttatgggttg aaatatgtat attcttgaat gatgaagaag tctacatgga 6840
agatatgaac atttgattag taaaggacaa ataagctttc taggcattga ggagagcttt 6900
aagtgtatac agtcaaagaa gagtgaagaa attaagatta cactgactaa gcattgaatg 6960
ttcacattag gaaattgaga gagttaaagt ttgagaaact agattgctag tgtttgggtg 7020
aatttggaga atcggtaatt taaggcaaga gaatatagag aatgttctag gagttttcag 7080
gaatgagaaa gataagtaga aggacttata tcaggttcaa aatcttcaat aaagcaatgc 7140
tgcgtgatga gctggttcaa ggtggcgctg tgtgtggatg ccaatatggc cagaagttta 7200
aagtaaacag gcaacaatat ggatactaat gttgccaagg atgagaatga aaatgatggc 7260
attataaaat gctttctctg tgccagtgac tatttcagtg ctttgcaggt cttaacttat 7320
ttagttgtta taatgttggt attattatta ttactcccac tttacaaatg aggaaactaa 7380
gacctgtaga tgttaagctg tcttaatacg cttaggaaat ggtaaagtca ggattcaaat 7440
ccaggtggta tgaatccaga atcccggccc ttgaccactg tgcttccttt ctcataatag 7500
gaaatgcagt caaagaaaaa caatagaggg ttagaagaaa agatgtgagc caagtgatga 7560
aacatctagg aaggtaaaag tgaatcctaa aggagaatgc aagagcaggg gtaatgagaa 7620
gtgtgtgata taaaaggatg gatcatatta acttcacatt tagggcagca ataagaagac 7680

ataagcagga gccaacagcc agtttcttca cctccctccc atgttaggag agggaggtga 7740
taaagcctat aaagtcattc tggatgactg ggtttccctg gatatgagac agaagagaga 7800
agagataaag aaataagata ccactcagga aatgggagaa aggagttggg aaaaatgatt 7860
tcttttaagc taattgaact gtttaggata tagtattggc caaaaaccag ttggttggat 7920
agcccattgc attcctttac caagacttgt aggtttggag tgaaccatga aaggaccagg 7980
aattgactta atgccttcca aagagaggaa gtaatcatga cctgccagtc ctacaaatgc 8040
agactactaa cctggtatga tgaaggaaag gactatttct caatggctta tctttgccag 8100
tacacagtaa ccagcccagt gctaggaata tactaggcat ccagtagata actgctacat 8160
gatccagtca tcataactga taacgccaca cttttatttt ttggatgctt tactcagtga 8220
cagcatttgt tgtgaataca tttggtgtaa tatcattaaa cacatgttat aatacaattg 8280
aaatgtatta cttagaagac actaagctaa gtaggtattg aaggattttc aattgtattg 8340
catattatgc tcactttttt tttttttttt ttgagacgga gtctcgctgt tgcccatgct 8400
ggagtgcagt ggtgtgatct cggctcactg caagctccgc ctcccgggtt catgccattc 8460
tcctgcctta gcctcccgag tagctgggac tacgggtgcc ccccaccacg cctggctaat 8520
tttttgtatt ttttagtaga gatggggttt caccatggtc tcaatctcct aacctcatga 8580
tccgcctgcc tcggcctccc aaagtgctgg gattacaggc gtgagccacc acacctggcc 8640
tgtcctcatt cttttattca tatattaatt gttcatgaag taattacctt aattacatta 8700
gttcatgtat ttattgagta cctgccatgc gccaggcact atgttaggta ctggtgaaac 8760
cacagtaaaa cgagagacct tgctggctgt tgactaaagt ttatagtgtg gtggtggaga 8820
gagacatttt acctatttgt gtacacatga ctaattgcac atgtggtaag tgattcgttt 8880
atgcaatgtt tggacaacta gagaattgac ccctctgtca gataatgggg aaagtttttc 8940
agacctagac atcacaataa atagactgca gaggaaacta gacagaaatg aaatactttt 9000
atgaataaag tgtttctttc aaaagttggg tatacttggt gtcataggct agtaatgaaa 9060
actggtttgg tagcatagtt ctccttgata cagcatcaga aagagaaaga ttgaacatcc 9120
aatttttcat cagcaacttc tgtgatttat ttgttctata tttagagctc tgtaatgctt 9180
tctccatctt ttgtagtgca cagatcatcc agcaatctct tctactataa tctatttgaa 9240
tttgaataat atttgggctc tgagaagata ttttgccaat aatcttattt atattcattt 9300
aatctccaat agagtctgct ctatagcagc tttcacatta ccctaaaaat aaatatgtag 9360
taattcacta gattttattt caaaactcta tatccaattt cttttcaagc tgaggtctct 9420
cagtttacta tttagcatgg ataatgatag actggtttta agcaccactt tacattaggg 9480
ttcattgaaa tctctactgt actgaaaaag aaatggttaa aaagatagca tttggtgtca 9540
tctgttcata tttggttatt tagagctccc agatttttaa tactctttca gaacatgtac 9600

tttaatacca ctacaagagc cagaggagaa gcagtggtat tagccatgcg tgctagtgct 9660
aataactgct cctgttctgg ccactgaatc attgtacttt aattactcaa gtaacacaaa 9720
aatccatctc ctttaaaaaa tgaaaatgtt atcaataagg cttaagtcct cttgatcaat 9780
acctctaatc tgttcccttg cctaccatct ttctatacct ctacctcagt ttttcccatg 9840
tgtatgtgtg tgtggaatat gttctctggg gtatgttaat atataaatgg tattatatat 9900
cataatttat gcatcatgtt attttgatac aaaaatgact tgaagctcta ttgttcatat 9960
atgtagttcc aacttgtttt tttaaccact gcttaatatt tcataatatg aaaaatcatt 10020
atatttagga attactaatt acactattac ttgagtaaac ccttttttgta catttctcct 10080
ttggtacatg tcttgcttct ctttccagaa tatattgaag tattttgata cagttctaaa 10140
tgaactagat gattagcttt ctacctgctg ctaagatgat ttgattcact aatttattca 10200
cttcagatac atgtattgat cctacattta aaagttgctt gtgctgggac tgaagatgaa 10260
aatgtatgca gaccctgtct ttgacatgca caaaaccaag aagaagaaaa caaaacagaa 10320
caaacctgtg gtacaactag tgaacatgag aataccttga tacaagttat gccagagccg 10380
tggccaacac accgtagtat tcagttagac acatggagaa gaatggcagt ttattctgct 10440
aacatatctt gatgttgtgc aagaggctta ttcatttaat agtcacttaa gtcgccttca 10500
ctataaccca ggcactgttt taactgcttc actggattag ctcaattaat ccttgtaaga 10560
gtcctttgag gtggaggttc tgttagtatc tatattttac agatgggtgt aaagagaggt 10620
ttctactatt caggaggtct gactctaact tctgtatctt aaattcctgc ttctgtgctg 10680
ttttcctaga ctgttttgta tttgagcaaa tattaattac tttatacttt ttcagataaa 10740
tacatggtct cttataggag actggttttc aaattgtgct ccttaactca gtcaagttcc 10800
ttcttctatt cagaacagct ctgtatttct ttatttggtt tatatttcca cttgagattt 10860
ttattgggac aaaggattct cagccaacct ttttttaaaa agcactcctt tataacagga 10920
aacccttaga tctcaatcaa gtatttattc gagtgactcc taagaagttt tttggctcta 10980
tttgctggta tctttttggc atttgtgcaa agacgagatg accatttgtg atcaagattt 11040
aaagtccaac tgctcactct tctaacagca tggccacatt attagccgaa aatcacatct 11100
gagttataga gcttttgctt ttgtcagaaa aaaaacaaag taactccagg gaatctttta 11160
tcagtcactt ctttaaagga ttgattgaaa taattgacac ttaagacaga cactcaaaaa 11220
taggcacact aatcatttac acaagggatt tccaacccct aggatgtgga ctggtaccac 11280
agccagtcca tggtccggtg cgggcccatg gcctgttagg aagtgggcca caaagcagga 11340
ggtgagcagt ggccaagcga gaattacaga ctgagctccg cctccctgtc acatcagcag 11400
cagccttaga ttctcataag agtgagaacc ctattgtgaa ttctgcatgc aagggatcta 11460

ggttgcgcac ttcttatgag aatctaatac ctgatgatct gtggtagaac aatttcatcc 11520

agaaaccatc ccacaccatc catggaaaaa ttgtattcca caaaactggt ccctgatacc 11580

aaaaaggttg aggactgctg gtttacatct tggtttttaa actccattgt catttaagaa 11640

catcagaaag tcacatactc tgtatatctt ccctatctac ctaatttgag gagaggccga 11700

tgaacacaat atccttcttg tggctagatg gccctcatat aattaactat ataatgcata 11760

cttcatataa tctatcatca agacattttt ttctcctctg atgagatatt atgcttttta 11820

ataagtgata tgtaaatagc ctattgggtt ttccttttta ttttaactcc tgcaaaacaa 11880

gtacatttta atgctcaatt ttaaaaaatt aacaggtttt gagctttatt tttaactgct 11940

actacagcgt cttgtgttgt gtactttata tgacatttta aggaatcata actttttttct 12000

taaagcatag tatttactag taatcattat ttctttagga aaaaaatgca aatacttttc 12060

atgctattta ctcttctata accaagtgat ttatttatga gatagaatac acatgtagtg 12120

agtatggacc gtgagctcaa aacagtgctg tagcatttgt acgaaatata aaaattaaag 12180

ggtaaaaata atggttatca tttattgagt gtttactaaa ttcaggtact cttctaatgt 12240

ctctttgttt actaactcat ttaactcttg caacaaacct atgagacagg ttctattttt 12300

ctcattttac agataagatt tgttcagtta gtcaacagta gagcagaact ccactcctgg 12360

agtcaatgcc cttaacttcc actggatatt cctttgcagg aaaagataca gtctttgatt 12420

tcaaggagcc tgcagtgtgt gtgtggaagt gagtgattaa cagacaacac tgcaaattta 12480

gtgtttgatt aaattgtgtg ttgcagactt agggtgctat aagtgaacta gagaggaaag 12540

taccaagagt tgtcattact gattgcctat atataataga aaccatgcta aatgctttat 12600

atttaatatt tctattcttc acaacaatcc taaaaagtag aatttaccat ttccacttca 12660

tatgtaagca aagactcaga agttagttaa cttaccccaa ttaatagtag tactggcaga 12720

gatgtgtctg cagtagttag aaaaggtagt ttattgagct agtaagattt attcaaagcc 12780

tttaaaaaca gagacagtaa ttaagaaacg tttatttttg atagtttttt taatataata 12840

atattgtgac tcctggcgta ttatacacaa gaaaaatttc aggtgggtca acaacttaat 12900

ttttttttta tttgaagaaa agaaacctaa aagtatggaa agatgatgga ggtatgtatt 12960

tatagtcttg ggatggaaaa ctttctaaat ttgatataaa atccagaatt caaaaacaaa 13020

tttgctccat ttgctatgta atctcaaatt tcttatataa ggcaaaaaat tcataaagtc 13080

aaagacaaca acaaattagg aaactgtaac atatatgtaa atcaaaagga aattattacc 13140

catatacaaa atattcttac aaatcaagaa aaagacaaac aggaagaaaa atgaacaaaa 13200

aatattaaga cagttcacaa agtacagccc acagaagtat gaagaaggct caactcacta 13260

ataagcatta ctgattaaaa taataatggg aatttcagca atttgagtac tggaataata 13320

atgaataaca aataatgacc attggagaat ctgaagatag tttggtattg tcaattaaaa 13380

tatgcgttac tttggcccaa caattctact tctagaaact catccttaca gaactctaga 13440
atacctgtag gtggatgttt attgcagtat tacattattt ataatactga gaaactgaat 13500
acagcccaaa tgtctgttaa aaagagatta gcaaattgtg gtataccctt gcaatgaaaa 13560
gagtgagtta gctgtctata ctacagatgt caagaagtct ctaaaatatt tgattttatg 13620
aaaaagcaag gaacaatatc attctgggta aaaaattgta cccatttgat cttgctaggt 13680
atagagagct atctaaaaga aaataactaa gtgcttactg tcaggatgag agacttccaa 13740
tttctgatga catgttttgg tctttttttt tttttttttt tttttgaggt ggaatctagc 13800
tctgtcaccc agcctggagt gcagtggttt gacctcagct cactgcaacc tgtgcctccc 13860
tggttcaagt gattctcctg cctcagcctc ccgaatagct gggattacag gtgcccacca 13920
ccatgcccag ctaatttttt tatttttagt agagacaggg tttcaccgtg ttgaccagat 13980
tggtctcgaa ctcctgacct caggtgatcc tcccgccttg gcctcccaaa gtgttgggat 14040
tacaggcatg agccactgca cccagcttca ttctttaagc aagtatttac tgaaggggct 14100
gcaattacac accaacttgg tgcctgactt tcctccacac tggccccgcc tagtctcagc 14160
tagcctctgc tgccctaatg gtgtccagtg accatatgtg ccacttttca ccttcaggta 14220
ccctagaatg ttgaaagaga ctaacaaaga atgaaacttg aagaatattg aagcaaaagc 14280
ttgaggttac tctgtatcat gaattaagga atccttcctg actagatgga gccaagaatg 14340
gacacaggcc cacaattcct cacacacaat ctcaatattc aaaaagcact gcaaacaaaa 14400
attttgttgt agtcacttgt caacaatacc taacctgacg tgaatttgtt tggcagcaaa 14460
atctgatctg aaatcacatg aggaaatctt gtagtctatg taaatattcg tacattttgc 14520
tgcataaata taatgtttta ttaagtgttg ctctatttta tctttctaaa atacaaacaa 14580
ttttgaattt tgaaacacaa ttggcccaat acatttcagc taagagattt tggacatgta 14640
ctcagaaatg atttatagac tatgttgaga gtggtgcctg ctggcttttt gcaatgtagc 14700
agccctgaaa atttttataa acaattctta tcatttattc ttatcttttt tttttgagag 14760
ggagtccccg ctctgtagcc caggctgaag tgcagtggca caatctcagc tcactgcact 14820
ctgcctcctg ggttcaagcg attctcctgc ctcagcctcc cgagtagctg ggattacagg 14880
catgtaccac cacacacact aatttttgta gttttagtag agatggggtt tcaccatgtt 14940
ggccaggttg ctcttgaact cctgacctga agtgatcctc ccaccttggc atcccaaagt 15000
gctgggatta caggcgtgag caacagcgcc cagcctattc ttatcatttt taataaattc 15060
ttagtaccat gctgggttct acagggagct ataaaagata aataagccat attacctaac 15120
ttcatatagt tttcgttcaa tcatggagtt tatcccatat tcaagtataa tataggtaat 15180
atataaatca aatgtgtttt tgatttcaag gggaaaaaga tcatatttag ctagggagac 15240

caaaatgtgt ttttatggaa aggatagcat gctagatggg ctgctgagaa tgcatgaaat 15300
ttttgatagc aagaattgta atggcttttg taaacaaaaa ctacagactg agcagaagga 15360
caatttggct aaagcatgga acatggtcag aatcccttac gattatatat aaattatttg 15420
tgtttttttct aggataattc ttttcatgag gttttgaaag agatcagtga ccccttattc 15480
ccaccccccaa aaaagttagc ttaccactgc tttaaaggaa tggtgaggat tattataaaa 15540
cagaaggtca agaaaatgtg gaatgcacaa tggggtgttt acaattaatt gagtaaagag 15600
tgagaagccc ctaaatattt tttgggcagt gtagtggcat attttaatat ttaagaaaat 15660
tagtatatca gcagtatatt ggaaagatta atgtcagaaa ggtaagggac aaggaaaaca 15720
gtctggggat ggtgcactaa ggacctgagc cagggttgtg gagagaggaa tgggttcaat 15780
gggtagattg gaaccaattg agaagggtga tgaaagcgac acagagaatt gtgggggtgg 15840
gggacgtcac cttcagaaat aggaagtaca tgatcatttt actcagtaaa ggagtgatgt 15900
tgaaagtagt atttaggaag gattatccta aaagcaccat gcagaatata tttgtacaga 15960
gattagatag cttgaagtca gagagaacag ctaggaaact ggtaacacag attctgagaa 16020
gtagtttaat ttcaaattgt ttgtattata ttctacataa tgtcctcagc aggatattga 16080
aataatataa tttttgtgca caaaataact tatgagaatt gagatgttct ggtttgtcac 16140
ttcatttagt cgtctgctta tttattaatt tataccaaaa ataatgccgt tttgtgcctg 16200
gatttgtgtt aaatattgag actcaaaaat ggatgagcca agttcataac ttaggagaaa 16260
ggcacataaa cacgttgcag aaatgcagtc tggtaatatc tatgagaggt gtgagctgag 16320
aattgtggaa gtataggaca ggaagatcta accctggtgt ttaaatgcgg ggtatggaag 16380
gagaaagtct gaatcctgac taagtattag actaaaaata ttaaatctta aaaattattg 16440
taatttcaaa gtttgtggca actttttttgt aattaacagt ggagaccaca atggtttatc 16500
ttctttaagc agagcacttc agtgagtgtt tctttgtgca aaggcccatc cagaggccac 16560
tttggatggg ggggcggatg aaagtatgaa gaactgaacc tctctatgta ctattttaat 16620
agaaattagg gaattgtagg ctagcagctg gggcaaggag taggaaatag aaagggccta 16680
acaactgagt aggaagcaag ggctcaagga gagtagatgc ctgtgagaat ttccctattg 16740
caggcaacaa tccataaatc cctaggctgg tagtgcctga tagcaaactt ggaattgttt 16800
actatctcat gttttaatta cctccagcta cctcccaacc cttaagtaaa tttttgctgt 16860
ttacattttc ctttataaaa ggatatgcat ttcagattgc tgacctttcc tatgatgaca 16920
agtgtctcag tagtatgata cttagaagat atataggccg ggtgcaatgg ctcacgcctg 16980
taatcccagc actctggaag gccgaggtgg gcagatcacg aggtcaggag atcgtgacca 17040
tcctggctaa cacggtgaaa gaaaccccgt ctctactaaa aatacaaaaa aaaaaaatag 17100
ccaggcatgg tggtgggtgc ctgtagtccc agctactcag gaggctgagg caggagaatg 17160

gcatgaacct aggaggtgga gcttacagtg agtggagatc acaccactgc actccagcct 17220
gggtgacaga gcaagactct gggaaaaaaa aaaagaagat atttagcaga aggtacagtt 17280
aatctgcccc ttcaagttaa agataatgtt catgatagaa aaaaggaggt aacttctgtt 17340
tgaaagacac tgttgttttg tagatagcat tttgaaaaca agtctttggg gatgattcca 17400
ctgttatttg attttgttgt gaaggtatta taagagatca aaattttata gtttcataca 17460
taaacatttt gaaataagat tttctaactt gctaaaaaat cttccaaaaa aattcagtta 17520
ggttctgaac acatttcata atagtggcat ttattgaaca catatgtcag gaacttggct 17580
aaatgtgtta tattaaacac aaagatttta aatcacttgc acagataaat agtaaataaa 17640
atggtagagc tgagattgga acccacagaa ccttctctct tatttgttaa tattacctct 17700
tattcattat ctagtgtctc tttgtcctaa gtatttgtaa tgaaaattga ttgacatcag 17760
ggagagtaga tacttgctag ctgaatttct ggggtaaaag aagcttttca taactggtat 17820
gtggaattga aagtgaatat tatgactaga taatctgccc tgcagacagt cccctcattc 17880
tccatggctt ctgggatggc tgtcttccac cttctcttct cagcctgcca gcactcccct 17940
gtcctcactt tcagctaaca gccttgcttc ctaattttcg gaaaaaaact ttctgaaatt 18000
gctaccacca tctgtatgga cagaagtggc ctctgaacta taatcccatt tttctggggt 18060
taccagagct ctgcagttct caagtccagc cctctctcct tgaccactag atccagatta 18120
ctcctgctga ctcaaggaca ggctctacca atcctcccgc aacatttaaa tcatttttcc 18180
ccttctctac tggataattc ctgtcagcct acaacatgct cttccttttc tcatcttaaa 18240
atataaacaa actacttttg ttgatttcac ttactcttca gccaccaatc atttttcttc 18300
ttttacagca aaactcttct aacacatttt cttctcccat tcactcttaa acctaaccca 18360
atcagttgtt gacctctgtc actccaccaa aacttctctt gtcaaggtta ccatgttact 18420
aaatcttatg ggcaattgtc agtcccatgt tacttgactc attggtagca tttgacaatt 18480
gatcactcca ccttcctgca aatactttat tcgcttggtt ttcaagacac gaccctctct 18540
tgattttcct cactagctcc ccttcaaact gttcttctcc tttagctttt aatatgagag 18600
ggtccagtgc tgagtattct gagtatttgg tcctcttccc ttctgtttat atacttactt 18660
ccttggttat ctaatccagt ctccaaactg tggataaacc acccaaaatc caatcacttc 18720
caaatttcta tctccagccc agacctttcc cctgaactcc agactgaata tctcaaacca 18780
acacatccca aagttggctc ctgatctact tagggttttc tctggtaagc cagtgttctt 18840
ttggtcctca agctatctgc ctcagaatca cctgggtact ggtttgagat gatttctgca 18900
cctcgcccca ggtaagccaa gtcaaaatat ctaagaaagg gacccagaaa tggcatttca 18960
gctagctgtt tggttgtttc ttaaagttct gcttggctca gaggggagcc agatacaaag 19020

ctgagaggtg aaatggttgg ctttgttttt gagaacatta actctagaag caatggggaa 19080
tagaggcaga aggtcatgcc agggagatca gacctaagat tatttaaaat actctttgat 19140
cgagatctgg gggccttcag tcttatgctg agaaaacact acttttcatt ccctaaagca 19200
gtgaacctga aatatagaag gcaggcaggc tgtggcccct accaagaatg ctcctttctc 19260
taggaagaat tctaaaaaat gtgagaggct agggagggag gttggagact tccacttttt 19320
gtgtccttac tatataccag gcactatagt agacacatta tatgtatttg ttataggcat 19380
accttagaga tatcgcaggt tctgttctag acaactacaa taaagcaaat attgcaataa 19440
agcaagtcac acaaattttt tgctttctgg tatctaaaaa agttatgttt acactatact 19500
gcagtctgtt aagtgtgcag tagctttatg tctaaataaa caatgtacat accctgattg 19560
aaaatacttt attgctaaaa ttgctaatga tcacatgagc cttaagggag ttgtaatgtt 19620
tttgctggta gagggtcttg ccttgatgtg aatggtcatt gacttatcga ggtagtgatt 19680
gctggaggtt ggggtgtctg tggcaatttc tttaaataag acaacagtga agtttaccat 19740
atccatagac tgttcctttc acaaaagatt actctgtagt atgcaatgct gtgttttacc 19800
cacagtagag cttctttgaa aattggagtc acttttctca aaacctgctg ctgcactatc 19860
acctaagttt atataatatc ctaaatccta aatcttttgt tgtcatttca acaatgctca 19920
cagcgtcttc accaggagca gattgaatct caagaaaccg ctttctttgc tcatccatga 19980
gacaagaccc ctcatgtgtt caaattttat gagattttag caattcagtc acatctttga 20040
actccacttc taattccagt tcttttgcta ttcctaccac atatgcagtt acttcctcca 20100
tgaagtcttg aatccctcaa agtcatctat aagggtttga atcaacttct tccaaactgc 20160
tgttaatgct gatgctttga ctttctccga ttaaccacaa atgttcttaa tgacatctaa 20220
aatgatgaac gctttctaaa agattttcaa tgtactttgc ccagattcat cagaggaatt 20280
acaatctatg gcagctgtag cctaataaaa tgagtttctt aagtcataag gcttgaaagt 20340
cagaattact cagtgatcca tgggctgcag aatagatgtt gtgttagcag gcatgaatac 20400
attaaccttc ttgtacattt ccatcagagc tcatgggaat tttttttct aagcagtagg 20460
tctcaacagt gggcttaaag tagttagtgg accatgctgt aaatagatgt gctgtcactc 20520
aggctttgtt gttccatttc tagaacacaa gcagagtaga tttagcataa ttcttaaggg 20580
ccttaggaac ttcagaatga taaatgagga ttggctttaa cttcaaatgc ccagcttcat 20640
tagcccctaa caagagagtc agcctttcct ttgaagcttt gaagccaggc attgacttct 20700
cctatctagc tctgaaagtt ctaaatggca tctttttcca agagaagact gttttatcta 20760
cattgaaaat ctgttgttta gtgtagctac cttcatccat gggcttagct agatcttttg 20820
gataacttgc tgcagcttct acatcagcac ttgctgcttc accttgtact tttgtgttat 20880
gggaatggct tctttccttt aacctcataa tccagcttct gttttcaaac ttttctttta 20940

cagctttcat ccctctctca cccttgatag aattgaagag aattatggcc ttgctctgga 21000
ttaggtttgg ctgaagggaa tgttgtggct ggcgtgatcc tctatccaga ccattaagcg 21060
tttctccata tcagctataa ggcttttttt gttttgtttt tttgctttct tatcattcat 21120
gtgttcactg gagtagcact ttttaacttc cttcaagaac atttcctttg catttacaac 21180
ttggctaact ggtgcaagag gcctagcttt tggcctgtct tggcttttga cattcctccc 21240
tcacgaagct taatctttct agcttttaat ttaaggtgac acatatgcaa cccttccttt 21300
cacttgaaca tttagaggcc attgtagggt tgttaattgg cctgatttca atattgttgt 21360
gacacaggga ataggaaatc atgaagaaag agacaaggac agaatgccaa ttggtggagc 21420
agtctgaaca cacacattta tcagttaagt ttattgtctt acatgggcaa ggtttgtggc 21480
acccaaaaac aattacagta ataacatcaa agatctctga tcatagatca ccatagcaga 21540
gatgataatg aaaaagtttg aaatattttg agaattacta aaacgtgaca gagacaggag 21600
gtgaacatgt gctgttggaa aaatgatgcc catagtcttg ctggatgcag ggctgccaca 21660
aaccctgaat ttgtaaaaca tacaatattt gtgaagtaca attaagcaaa gcgcaataag 21720
atacgatatg cttgtatctc ccttaatttt cagctaagag ctgtttgaga agttactgcc 21780
atctcctttt ttcagatata agaattaaaa caaggaagga agttaacttt cctaaagata 21840
cctagccagt tagtaatgga gctggtaacc aaacccagtt ctggcagact ccaaaaatta 21900
aaccgctttc actaaagcac accactgggc actgtgtttg aagcacgaga tgatattagc 21960
aactgattga agatttattg aaaagactta aaactcttat tgtagtcaag tcgtttaaaa 22020
atattttta cttttaaaat ttattattat ttagagacag gctggagtgc agtggcatga 22080
tcatagctca ctgcagcctt gaactcctgg gctgaagtga tactcccacc tcaacctcca 22140
gatattttt taatagacaa ggctttgctt tgttgcaggc tggtctggaa ctcctggcct 22200
caagcaatcc tcccaaagtg ctggagtttc aggcataaac caccttgcct aggctcattt 22260
ttttttttt ttttttagac ggagtctcat tctgtctccc aggagtgtaa tggcacaatc 22320
tctgctcact gcaacctctg cctcccaggt tcaagtgact ctcctgcctc agcctccaga 22380
gtagctggga ttacaggtgc ccaccaccaa gcccacctaa ttttttatt tttagtagag 22440
acagggtttc actgtgttgg ctaggctggt ctcaaactcc tgaccttaag tgatctgcct 22500
gcctcagcct cccaaagtgc tgggattaca ggcgtgagcc actgcacctg gccagttcaa 22560
atcttatata ttttggtgcc tagacttaaa taacatttag gaaaatgtta attaattatc 22620
catatagttc tacctttttc tttcttttt ttttttttta caaagccagt ttcaagtaag 22680
actggggaga ttctagaaag ttataacaga atcttaagtc acttgatatg cctaaacaga 22740
atttgaagaa ataatgaagt tgaacacatt gataaactta ggatttctgt aatcaggaag 22800

aaaaaactaa acttcataga tatgagggaa ggaattaacc atttgcatgt atgtaactgt 22860
tataatccta catcagagta gcacaaggac atgaacaact ttgttgacaa atagttttgt 22920
aatttttaa tgtttttat gtagctgtct taaactcctt tctcaatagt atgtgaggtc 22980
aaatgattgt ggtttctttt taaaattttg catgtaatgt gtagttcctt acattctcta 23040
aatgcttact gtttagctaa ctatgttatt atggatgtca attataacta agtacttcat 23100
acaatttcac gttttataag ttagaagata tgtgaataat tagggcattt ggaagagttc 23160
cagagagatt tttttattta gaaagagagg agcagaacag cttcaggaaa gatagatgaa 23220
aagctgattc attcaattta agatagttga agggaagaaa aaagaacaca ttatttagaa 23280
tgatgacatg aagaacttaa tttcctttt ttacttatac tttaagttct ggggtacatg 23340
tgcagaacgt gcagttttgt tacatagata tacacgtgcc atggtggttt gctgcacccg 23400
tcaacccgtc acctacatta ggtatttctc ctaatgctat ctctccccta gccccccatc 23460
ccctgacagg cccaggtatg taatgttccc ctccctaagt ccatgtgttc tcattgctca 23520
actcccactt atcagtgaga caatgcggtg tttggttttc tgttcttgtg ttagcttgct 23580
gatgatggtt tccagcttca tccatgtccc tgcgaagaac atgaactcat tcttttttat 23640
ggctgcatag tattccatgg tgtatacgtg ccacatttgc ttaatccagt ctgtcattga 23700
tggacatttg gcttggttcc aagtctttgc tattgtgaat agtgcttcag taaacatatg 23760
tgtgcctgtg tctttatagt agaatgattt ataatcctct gggtttatac ccagtaatgg 23820
gattgctggg tcaaatggta attctagttc tagatccttg aggaattgct gcactgtctt 23880
ccacaatggt tgaactaatt tacactccca ccaacagtgt aaaagctttc ctatttctcc 23940
acatcctctc cagcatctgt tgtttcctga ctttttaatg gttgccattc caactgccat 24000
gagatggtat cttattgtga ttttgatttg catttctcta atgactagtg atgatgagca 24060
ttttttcatg tctgtcggct gcataaatgt cttttttga gaagtgtctg ctcatatcct 24120
ttgcccaatt tatgaggttg tttttcctt gtaaatttaa gttccttcta gattctggat 24180
attagtcctt tgtcagatgg atagattgca aaaattttct tccattctgt aggttgcctg 24240
ttccctctga tgatagtttc ttttgctgtg cagaagttct ttagtttaat tagatcccat 24300
ttgtcaattt tggcttttgt tgccattgct tttggtgttt tagacatgaa gtctctgcct 24360
acacctatct cctgaatagt gttgcctagg ttttcttcta ggatttttat ggtttgcagt 24420
cttacattta agtctttaat ccatcttgag ttaatttttg tataaggtgt taggaagggg 24480
tccagtttca gttttctgct tatggctagc cagttttccc aacaccattt attaaacagg 24540
gaatcctttc cccattgctt gtttgagtca agtttgtcaa agatcagatg gttgtatatg 24600
tgtggtgtta tttctgaggt ctcttttctg tccattggtc tatatatccg ttttggtacc 24660
agtgccatgc tgttttggtt actgcagcct tgtagtatag tttgaagtca ggtagtgtga 24720

tgcctccagc tttgttcttt ttgcttagga ttgtcttggc tatgtgggct ctttttttggt 24780
tccatatgaa gtttaaagta gctttttcca attctgtgaa gaaagtcagt ggtagcttga 24840
tggggataac attgaatgta taaattactt tgggcagtat ggccattttc actacattga 24900
ttcttcctac ccatgagcat ggaatgtttt tccatttgtt tgtgtcctct ctgattttct 24960
tgagcagtgg tttgtagttc tccttgaaaa ggtccctaaa acttcatttc taaaaaaaaa 25020
aaaaaaaaag gaatatgcta ccaaatagga ttctttaaat caattgtctc ctccttcaga 25080
aaacatccag aacctgacct cttatcacca cctccactga ccactttggt ttaagccact 25140
cttattcccc ttcggatgta ttgcagtggt ctcctgacag tctcatttct atccttacct 25200
tcctagattc cagaggttga gagtctgccc tcgacttaga agccattgtg atactgttaa 25260
aatataagtc agatcatgtt agccgtctgc tccagtgtct ccagtggtac catatctttt 25320
agagaaaaat caaatttctt acagtggcct tcaaggccct acagcatctg cactctgctg 25380
cactctgatt gtactatcta ctactcttct ccctggctta gccagctcta gccttactca 25440
catcctcaaa caatccctgt catctcttag catcttagca atttcttctt tcctctctaa 25500
ttcctttccc caacactccc cacttctcca acccattcca gcttccagct tccagcttcc 25560
atgagtatcc tgaaacataa caaactttgg ttactgccag tgtctcactt aacacattcc 25620
cccactacag tgttgtcaag ttgtttcatt taacaccagt aacatttaat atcagtaata 25680
gccagattct atcttaggaa aaatatgcct aaaatttatt tgctgaaaaa aaaagaaaag 25740
aaaaatttgg ataataatct gctttgagtt attacaaaca cagcagtatc tgagaagcaa 25800
gactgagtgt cataaggaaa caaccagatt aaataaaagc atagctggat gagataagaa 25860
aatactgcag tgtgatccaa aatgcagtaa cagagttaaa atccatattg gtcgtcataa 25920
agagcaggaa ataacaccaa ttatgtagag ttcacattta aggagttact tcggaatgaa 25980
gagaaattgg taaactggtg aaaattacac actatgattt attgttttta ttatgtacca 26040
gtcattgtgg taacatttca tctaatttag ctcacttaat ccatataaca gccttataaa 26100
atgtttatct ctatattata tgtgggaaaa actaagattt tgattaaaca attttatcat 26160
gctgcagaaa gtgacagaac tgaatttgaa tgaaagtcta tttgactcta aagcatatgg 26220
ccttatcact atgctgttgg acaacagata cgatgaatag agagcacata tgcaacccat 26280
aaatatccag tgattctgaa gaagaaacta aaaaacaagg cagcaaaagt attaattgat 26340
tatagaaaat gactcttcct tatgtatctt ttttacgaga tcatttctac attgtgtcac 26400
cagcacctag aacaatgttg gcacatagta gatattcaag aaatatttgt ttattgaact 26460
gtcataatta ctgttaagca tttattctgt gtctgttatg gtgctataga ttttatatac 26520
attattttac cctcaaagca acttttaaac tgttatttta tagatgagga taatgtagtg 26580

ccaagaagtt aaatgatctg ctcaaggtta tatagctaat aataggtggt agaacacgta 26640
taacagaagt aagtttagca gttaaataaa aattttactt tttagatcaa aaggactcac 26700
tgcatcccag gtaaatgtaa ttttaaaaag ctaaaacttt gcaatattgg taattttta 26760
aaatttttca tgagaaggaa aaacacccta tatgcattta agtaggatta ataaaactgg 26820
ttaactacta agacataaaa attgggttga cattagattt cctctttgag tactaaatgc 26880
tgagattggc aaaaattcca tcaggttgag ttgctattct tgggtgaaga taacatatat 26940
tcttatatat gtgagaggtc aggaatatgc ccagtcttta ccgaagtact tgtttgtctg 27000
atggttggac taatttggga ttcctgtagt gtcctgtagt tcagggtgaa gtttagtaac 27060
atgcgggggt aagagacagg aggtgctatt ctctgccctg ctgctcacca gtgtgctgta 27120
tccctcctca ggatcaccct taaggtctca gatgctcaca cctggtgttc agtgcccagt 27180
ggtctgttct ctagccagta tattctgtgt cgtcttagat tccagtccaa agactattta 27240
gatctgctcc atccttggga atttccacgc actttattat ttcttctagg gcaggggtcc 27300
ccaacctaga acaagcctac gatttgtggg tcataccata ctgactttca ccatttcact 27360
ccttgaacta ttgaacatac attatcattt ctcctgccat tttactcacc tgctgctcac 27420
ctcctgctgt gaatcaggcc acacagcagg aggtgagcag caggtgagtg agcattactg 27480
cctgagctgc acctcccatc agaccagtgg tggcattaga ttctcatagc agcgcaaacc 27540
ctattgcaaa ctgcgcgtgc cagggatcta ggttgtgctc cttatgagaa tctaactaat 27600
gcctgataat ctgaagtgga acaatttcat cctgaaatca ccgcttcccc caaccccccc 27660
cacccctcac tgcccccagt ccgtggaaaa ttcgtctttc atgaaacctg tccctggtgc 27720
aaaaaagatt agggactgct gttctagaga aattaaatgt ttttatgtct actttgttac 27780
aaggttacat gaaataaacc agttaaatgc ttagtatatt ggtctctaat atactcaata 27840
agattttcct tctctccttc accccacacc tttttccttc acttttatca aaaatccttg 27900
gatcttcatg aagatatttt aaaacctact gatttgttat tgtccagctg tctttcacac 27960
tctgctagat agtcactctt cattccactt tacatgacca tcccctgact tactaacgtg 28020
aaaaactggt ctgaaccttt gtctcattct gcatgcatct tcagaatatg ctggattcca 28080
gaaaacatac aaaaaacttt aaggaatagt acagcgaata tctgtaatca acaatttgaa 28140
ggtaagttgc agacatcatg acatttcacc tcttaaggct ccaaaattca tgtcctaagg 28200
acaagaacat cttccattac aaccacaata ttatcacccc aaaattttaa cattgataaa 28260
acaattctaa tattcagcta gtattaaaat ttcttcagtt tttcccaaaa tgttttgtat 28320
agaatgtttt tttattcagg atccagtcag gaattgtgta ttttacttgg ttagcaactc 28380
tgtttagttt tctttaatct aaaaagatct cccacctttt tttgtctttc aggacattgg 28440
catttttttg ggagtctagg ccagccgttt tgtagaaagt tccataatct ggctttgtct 28500

gatttttccc ccacataatt atatgccagt taaacgtttt gacaagaatc ctacacaggc 28560
caacggttct cagacactga tctcatgacc cccttatgtg cttaaaaatt gctacaagcc 28620
caaagagctt ctgtatacgt gaattctctc tctcagtatt tactgtgtta gaaattaaaa 28680
ctcataatgt tttaaaactt tttattagct catttaaaaa acagcaaacc cattagatac 28740
taatttttca aaccaaaaaa tttaatgaca agaatgacat tgttttacat ttttataagt 28800
cattttaatg tctatcttaa tagaagacag ctggattctc atatctggat ctacagtcaa 28860
tctgttccaa tatgttgttt tggtggaagc atgggaagga aatgtggcct cacacagata 28920
tgtagttgga aaatggagga gtattttaat tgtcttttca gacttgacaa gtggtggcca 28980
tttcattagg aattgcaaaa ggatgattct ctatttctat tatcccttct gattttatca 29040
gctataaata tttttaatag ttctctctct cttccaagag gcaatctggt aatcttaaaa 29100
tacacttaat aatggaaagc ctaacagata cttattattt acttttaatt ttcagttttc 29160
aaagttgtta agtttgtgcc ctagtaatct ccagtggttt ccagtgagtt cctcttgttt 29220
ctttcattct ttctcttctt acatatcatt atgtactcat gtttttttaaa atatatattc 29280
aatgtgtttt aattacttga accagttctt tttgatgttc aagtggtttt aattttggtt 29340
ggtgtgaatt atcagtggtc aatgaaacac aattttatta gccctcagta aaagcaaatc 29400
atctgtcaca gcaaagcatc ccaggctcaa cttgcacatt tcctgcccta gtcctggatc 29460
agtcatttcc ccaaggagct ctggttcgtt ttagatgaaa attatattac atgtgaaaat 29520
ctgaattcct gctggagttg tctttacttt tgggcatttt cagtgaagag acctagaaaa 29580
tacctctttt tgaaaaagta aggggatcat gagtttatac catgatttct caacctcaca 29640
ctattgacct attgggccac ataattgttg caggggggga tgtcctgtgc attatgggat 29700
gttattagcc tttttggcct ctctgcaata atacctaccc tctagttgca taaaccaaaa 29760
ttgtctccag atattgtgaa atgtcctggt gaggaaggga gcaaaattgc ctctagttga 29820
gaattactgg tttatgtttg tatttctagc aaaatcaaaa taattactag ttgttttatc 29880
tgtatgtatt gtgtgtgtgt gtgtgtgtgt gtgtgtgtgg attcaaaata ctagtatcag 29940
cattattacc tctaacaaca aaacctgctt tgtagactag ttcttccatc tcctgcataa 30000
aatcttaggc cagtcccaaa gattttaatc agaagaacaa tctcattcca ctaaccgtat 30060
tagtctgttc tcatgctgct gtgaagaaat acctaagact gggtaattta taaaagaaag 30120
aggtttaatt gactcacagt tccacatggc tgggaaggcc tcaggaaact tacaatcatg 30180
gcccaagggg aagtaaacat gtccttcttt acaaggcagc aggagagaga atgagtgcca 30240
gtaggggaaa tgctagactc ttataaaacc atcagatctc atgagaattc actgattgat 30300
cacactgctc atgatgatca cgagaagagc atggggggaa accaccccca tgattcaatt 30360

acctcccact gagttcctcc cacaacatgg ggggattatg ggattacaat tcaagatgag 30420
gtttgggtgg tgacacaaag ccaaaccata tcagtaatca aaatgcccag ggaagcatta 30480
tgccaaattt acaaatcctc ttctaagttt agatttcttc tttgccccaa ttcagcactc 30540
actgctttga gcttgaagcc taaagggaag aacaaataag atctgcttct tctctgttta 30600
tgctgcctac actcacccaa tgacagctga ttttgaccct tccagggcca acgtgtggga 30660
agagaagtta gagagcacag ttttacacaa caagtaggaa ttttagtgtc actggggtta 30720
tctgtgtttg gtgggtgatt acaggctgct cttcctctag tgggatactt tggtgagatt 30780
catggagata ctttactgag agcctctaac tgtaatcctg taatgctgta atgcagtaga 30840
cacctctctt ctctctggac atgaccgtga ccctctgctt ctggacttcc gctgactgac 30900
cctacacaga ttctgttgga tcacaatact ctctaggttg tcctcttagc tggagccagt 30960
ttttaaaaga aagaaacttt tattgataaa tattttcaga aaagcactca aagatgtaga 31020
gcttatcagt aggtaaatga aacgatgtta ttatcaccca gaccaaaaaa tttaaagatc 31080
attaggttcc cagaagctcc attttgcccc ttccaatcat tccccaaaag taaccgccat 31140
cccaatttct aacatgatag attggtttga actattttag aactttatat taatggaatc 31200
agatagtatt tttctatgtc tgcttatttt acacaacatt atacttatgt agttcatcca 31260
cattttgcat ggagctagac atttattctc attgctgtat aataatgcat tgaataaata 31320
aaaccatttg tttacacatt atgctgtagg tgatcatttg ggttgttttc agattgaggc 31380
tattatgaac actgctgcta taaatattct tgtacaaatc ttttattttt tgcatgtgga 31440
cacacctgtc cctacagata agcttttcat ttgcctctcc aaggaccctg gcaatttcac 31500
tggtcctgta ccagtctttg ttccttggcc caggttccta taccatgtga tagtgaaaat 31560
tcacctccca ttaaaggcca atattttatt ttgtttatgt tgtttttggc ataaatgatt 31620
tttgtcctca ccctgaattt tggacagatt cctggctgcc tggcattttc tgtgtagtgt 31680
tttctagccc tgttttcatt gctaaggcag tcttttaagg ttctacattt ttatgcatat 31740
agttcagccc tgccactctc ttccattggg actgaagtaa tatatcccat cctctcatgg 31800
gctttaaacc ccatttctta tcttttaggc atttcttata tctgtatctg aaactttttt 31860
ggatcactgc agagtccagt taagtttgtt cctccagctt tcatttcttt tttcttttct 31920
ggcttataga gatttaccta cctttttttc cagactgaaa ttaagcaaca tgcttctatt 31980
atattttatc cagtatttct gtgtttgtgg tagaaagatt gccctcatat ggaccagctc 32040
atgttgcttc aagcccgtga cttgtgggtc ataccatact gactttcact atttcattcc 32100
ttaaactact gaacatatat tatttcctgc catttgacta tgtattttcc ccttcttttc 32160
aaaaatgcca tgaaaaattg tgaactctct cagacctaag tgaactatgt tcacttgtgc 32220
tcatattgag aagcaacaca aacaatatag gtctcttatt ttacaggtaa gtccctttct 32280

tgtttctaat aaagatattt tatttcctaa taaagatatt ttatttcctt taaagtggaa 32340
ttttgtcatc taaaattttt gtaagtcact aggcaaataa aatgatctaa tcacattttg 32400
atagtgcttt tattttcaca gagtattttt tattaagagt aacacttggg gctgggcacg 32460
gtggctcacg cctgtaatct cagcacttca ggaggccgag gcaggaggat cacaaggtca 32520
ggagatcgag accatcctgg ctaacacggt gaaaccccat ctctactaaa aatacaaaaa 32580
attagccaga cgtggtggtg ggcgcctgta gttccagcta ctcgggaggc tgaggcaggg 32640
gaatggcatg aatccaagag gcagagcttg cagtgagctg agatggctcc accgcactcc 32700
agcctgagcg acggagcgag actccgctca aaaaaaaaaa aaaaaagagt aacacttgga 32760
aataatcttg ttagacttca catttaatta tggtctatta ctataaaaag gtgagaaatt 32820
actgaataaa aagcattctg taagaaaaaa agttaaaagg tggtaaaaaa tcagaagcaa 32880
gtgactattt actatatatg acaataagtg acatatgtct tatactgttg agtataacta 32940
tatttacaaa tataagcgaa acaataggta taattgtata tacacctatc taaaaaatat 33000
ttatcaagct gtattttaca caagagttaa aagtatcaaa ctgagtagtt cctactctta 33060
tagaacctag tagctataga cacaaagtat acaataggaa tacattgaat gtgcagttgg 33120
tacacagaag aaatgtctaa agcctttatg agagcagtca ggtaagacca gatgagggga 33180
gcagttaagc caaggcttga aagacgagtg agattttgtt aagttgataa attggaagga 33240
agggaggaaa gtcatagagg catgagagag tttgggggta atccaaggat gttagtactc 33300
tggagcataa agaacatggg aaagagtatg tgattagaaa gaaaagacat aacagtttga 33360
actcaccccc tgctttcccc accctaaaat agatcagttt tgcaaggaga aagaagcata 33420
gtgctctatt aagctcccca ggtttcaaca ggggcagatg atgcatatat taagggagag 33480
agctagatag tggctcctag tggggtgaac tcccctattt atcattatgc catgtaacct 33540
gtttgtggtg gcttatagct tataagtgtt cctaaatgtc ttataactcc tggtggtgat 33600
aatgtaggta aacagcaaag atggcagcaa atggaatgct ctgagtggtt taatatagtg 33660
acgtgtttta cacttgttgt agaatcacaa tgttatgttg gactgaggag aagaaataat 33720
acgcaatttg agccatcaac ctacggttta tgagtgagtg agtggatcta gacagggatg 33780
tgtttctcaa aaccagaata taaactgtgg gctgttgtct gaagaaaatg gtagcaactg 33840
atgttatgtt agattttcat ctttttctag atttccagga aaatgttgtg acagggctaa 33900
tgcacttgtg cctgaccttt cttcacctgc atatacagcc aggtacctga agtagcgagt 33960
ggctcccact gcattcattg cattcttggt ggctctgagt cctggccatg cattatggtt 34020
cagaggcaat gcctgggccc catgccaaat tcccttagtc ggctctagtt ggaactggct 34080
ggctacaaca tttctttaag ccaaaacatt gcttgagaag ataaaccttt aactgtactc 34140

cccatatttt gtttgcatga taatgcatct actttgcagt tttgatggct catcacttaa 34200

tgttaaagat gatgaaaagt tagagtgaat tgagcttcca gatttcttta agccattcca 34260

gcctgtcaca ggcagtctac aatgatatga gtcgtgccaa cccctcaagg tcgtgacttc 34320

atgccagtgc ccttaattat taaaaattat ccttccaact caaactttaa atattggaca 34380

tctaagtcat tttttcttcc aaacttataa gaattgcatt ctgtttggta actataattt 34440

ccttatttat actcatgttt aaatttatac ttatgtttaa atttatttgg tacttatcat 34500

cagtctcttc ataccagact tcctacctca tacattgtct gctataacgt atctgttgtt 34560

gactggatta catcttttgg aagagcatac tggggtctta tttcccaatt tcatgcaagt 34620

ttgagaatat ctttaagttg cctttaaaac tgaataactt gactagatat agacttcttg 34680

gaccacatct agaaaaaatc taactatatt tgagatttag atgtgtgata atcataatta 34740

taatagacat ttgttaagta cttgctatgt gccagactat tctaagctct tacatgagct 34800

tgtgtattcc ttagatccaa tccaaggtac taatgttatc ttcattttaa ttgccaaatg 34860

tcatctagct actaagtgat gagattgaca ttcacactta aacagtaact ttctatacta 34920

cctctcaaga taattaacct aaaatgtttt aactgtgtga ccttgagcat gttatttaac 34980

ttctagatgc ctcagttaac tctgtataaa atgagaatat taatacctcc aaaggttgct 35040

ttgatgatga aatgagttaa tatatgtcaa tagctcataa caatgcctgg cacatagtaa 35100

gtgcttaata attgtctgtt gttcttttat tatttttatt tgtggatagt aagttgcaga 35160

tctgagcttt gaacaagcag tctggcctct gtaactttgc tcttcggtac tacaatgtag 35220

tgtcagcaat taagatggca ttttaattta gtgggggaaa acatggatta gtcagtaaat 35280

agtgttggga tatagtagat actcaatact tgttgaatga atgagagttt tataaaagta 35340

taaatacatt aacttaaata ttaacaaata gatattaaat accatttaga tttgtataga 35400

cagtttggaa atactttagt ctgttccact gcaataaaaa aataccttag actgggtagt 35460

ttataaatac agaaatattt tctgatggtt ctaaaggctg agaagtccaa gatcaaggtg 35520

gcacagattc catgtctggt aaaagcttgc tgtcttcttc caagatggcg ccccttgctg 35580

catcctcaca tggcagaaag gataaaaagg accaaattca ctccctcaag cccttttaaa 35640

agggtactga ttccatccat ggaggcagag accccacctc ttaatattgt cacattgggg 35700

attaagtttc aatatgaatt ttgaagggga cacaaacatt caaaccatag caagaaggaa 35760

gcaaaaatga gaatagatgc atgattatca aattgtaggt tattttctgt tttgaatttc 35820

cttacgttct tatataattt ctctaataat acaattcatg atataatttg tacagataac 35880

cacttagttg tattttgtat atattgagtt tgaggtacct agaaatatga ctggaaggtc 35940

agagctcata ttgagatttt gggaccatca gaagataatg agacagacta cccacaaagt 36000

gtgtgcagtg gcacaccaca tataaggaat aagcaaagta agaggagttg acaaagggaa 36060

tttaaaagga agtgctggaa agacaggtca agagtcagaa aagagaggcc ccaaaatatg 36120

aatcaatggg acaggtgggg aaagaaatta cctgaagcaa atgattcagg tcaggaaagc 36180

attcacagca tttaggaatg aaaaaatatt tgatgatgtt aagccatttt ataagaatag 36240

tgaatataca agtcaaatta ccacatagga ataaaggaag ttaggtagaa acagcaaatg 36300

tagatgatgt tttcgacaac tttgaggacc aaaaaaaggg gaatgtattc agggagaatc 36360

tagagtgagc tagggttcct ttttttggtg ctggaacttt tttggatgga aaaataatga 36420

gagagcagga catcactgga ctggagagac taaagacttt aggagagaaa ggaggaaatt 36480

gatggagcaa gacttgaaag cagataggaa ggaattgttt gaaggacaaa agcaaaaacc 36540

attggtaagg aggactcttt gagacactga ataaggagat gatatcgtag gccattttag 36600

aaatgtaagt gagacagatc agcagcatgc cagcttctgg tgtttagatc agaccttttt 36660

cctgcgtatt tatgaccaag gagcagacag taggacagcc tctcttcaaa caattacatc 36720

tgcgtgctct atttctcgaa gtctgttttc tgtatagaga aaaccttggt gggcgtggtg 36780

gctcatgcct gtaatcccag cactttggga ggccaaggcg ggcagatcac ctgaggttgg 36840

gagttcgaga ccagcctgac caacatgcag aaaccctgtc tctactaaaa atacaaaatt 36900

acccaggcgt ggtggtgcat gcctgtaatt ccagctactc agaggctgag gcaggaaagt 36960

tccttgaacc caggaggtgg acgttgtggt gagccgagac tgtgcctttg ctttccaggc 37020

tgggcaacaa gagcgaaact ccgtctgaaa gaaaaagaat agaaaatctt gctattttct 37080

gttctctctt cttgtgagat atcagtatag ggtagaaata gatgatcaac tgatagactg 37140

acttacacac accttgctct cacagtcact cctatagcct aaatatataa cttgtggtaa 37200

aaaaaaaaaa aattagagtc aaatcagaaa gtagtatgca gtacactctg ctaacctcct 37260

gtaggactgg cactatgtgc acaaaagtca gcaaagatat gaggcttcac agttaccctt 37320

tattaggtga aggcttaatt cctgggactc taaacaagag atgaacacta tgtgagtgta 37380

catgaacata agtttataga ggcatgcatc tttaagaaaa tatttgtgct tttccagaaa 37440

tgcatgaggt caggaacctt cttgatatca gctgtcacat atataggcct tttggcattt 37500

ttgccctttg ccgtttaggc taatcctttc ccctgcttga ttaagtacat gttttaggta 37560

cctctgagtt cctttactac caactttggc cagaagactc tgattttaat cttaactaat 37620

gtaaaatact acaggaaaat cccatgatct tttaaaatta tgtgtcatgc ttcagcagta 37680

taagtaggct gaagtaggct gatgatatta aaagactcac agtctgtttt aaagaaattt 37740

attgggaaaa aaagagagtg tcaaaaatag taaattaacg tttacatgtt taagctgaat 37800

tttttattt ttgttaaatc taaataaatt agattgttcc gtgccttatt aacatatttg 37860

tggtttaaaa atcattattg aatattaaat tcatgtgcca gaccttttta aaggcatcca 37920

tagtttcctc tacacaaagt gaccccaggg tgatgtgcga tgtgtattgt ctatacttct 37980
ataaagtttc tcctctttat tgagtacttc tatctgtttt aagttcttcc tttatcatta 38040
tttctgttca aggtattttc taatgtgtag tcaaaataag gcaggtatta aattcagtac 38100
aataagtgat ctttttttaca tgctcttcca ttgattcctt aaagagagaa ccagctctgg 38160
gatttttcca atagaactaa gcatttagat tttttttaa aaggtctttt ccagaggcct 38220
cttcaattct ttagtctgta tctgttttct tgaaggcatc aatgtcagaa agaaaggttt 38280
tactagagtt tgaccacctg gtacaactaa tgcttatcag aaacgtgtag ctaatcttga 38340
aatgacatct aaaggcaatc tgatactgac accattaaga agataaacag aaatctcttg 38400
caaaagattt ttaaaggagc cctaggattg attcttcagc tatgctcacc taatttaggc 38460
tcctaaaggc acaggtaaag tactgagcag ttttatagct tcaccaacat tcagctgtgc 38520
caggcaggaa gctggctgag tcatgcactt gctgatgtgt atctagtctc tatttcagtt 38580
gtcactgcat agaatttcca gtgaatctta gatattgtaa ataattttga ctcattttgt 38640
ttttttaggg cagcacaact acttatgtgc tggaagaaat gactgcatcg ttgataaaat 38700
ccgcagaaaa aactgcccag catgtcgcct tagaaagtgc tgtcaggctg gcatggtcct 38760
tggaggtaat tctgatgttt tcatcaataa tatactgtgt aatctttata ctataaaact 38820
gtgtgtcaga aaattgcaat ttcttattca tctttgtttt tgtttttttct gtcttgatta 38880
ttggcagtga ctctgcacat gtgagtgtct gaatgaagca accaatacca ttccattaac 38940
tttacagttt tctagcacat tgaggtctgt taagagaaga aaactcaatt catctatatt 39000
gcctataagt agaggtaatg ggcaatgtac tgacctgtca ttaagtaaca tgtgtgggag 39060
tctaggcttt gctgcccatc tcctgtgtga ccacagctct tcctgggcat cagtctactt 39120
atctgtaaaa cgagagcatt aacattcctt tcagctcaaa aaattgactc tgcaaattgg 39180
tcaggtacat tgcttactag cttctccaag tgattgatta aagcaagatt actgtaactg 39240
gggtaggaat ccatttgatg aagagttata ttaataattc ccttttccca tatttttatt 39300
agaatctcta aaactttcag taatatagca cacatttaaa aaattaatgt cagatagtga 39360
cagatagttt gaatgaaaac atagcagagt gagggagatc aagcgagcca cagttatgca 39420
agacacccgt actcccttga attttttcac ttagccacat cctgcacaac actgtctggg 39480
aatcgtagct tttacatcta gatataactt tttaaaaaca taataaaaat attacataat 39540
tgttaaagct aaaaatgttt tttcagtgca ctaactataa acagcttgtg ttccatggac 39600
gttgtgtgta ttttcctttg ggaaatgcta tatttagacc aatgcttgtc aagtttaaag 39660
cgtatacaac tgtcacctca tttgtttttg caaatgtgct ggaccaaaat taatcaccca 39720
ggtaacatac tgggcacttt acagtaattc atgtaattag atcacttata gaattttata 39780
atttcttgat taaagatttg ttttcctaag ttaagggcag tgattaggtt gacagtagtg 39840

cctggcttaa ggaggtgctc agtaactttc tattaaatga ataaaaccac aacctcagca 39900
gattaaaatt gtttttgagt cccacacctc aattaccacc tctttgtatt tctgtttcaa 39960
ctcagaagtc ttaaggaacc tcctaaattt tagaaaatac aactttttca caaaatcaca 40020
catctagtca atgtcggagc cagaagaaag acctgaaggt attctgaccc tggcgcagcc 40080
attgttttct ccttggcatg cttgccttag aaagtagcag agcatctaat gctgacactt 40140
agattccatt aagatcttta atcttgagtg ttttcccaat gactttaaaa gactcttcaa 40200
ttttatcaaa gaatgaaaat ggcggagttc tggatagaac cttcagaata ctcctgggga 40260
tcagcaatat aacctagaga aatttatgtt atcttttaat attctagggc tttatttatt 40320
tatttttgag acagagtctc actctgtcgc ccaggctgga gtgcaatggt gcaatcttgg 40380
ctcactgcaa cctctgcctc ccaggttcca gcgattctcc tgccacaacc tcccaagtag 40440
ctggtatcat aggcacccgc cattacgccc agctaatttt tgtattttag tagagatggg 40500
gtttcaccat gttggccagg ctggttttga actcctgacc tcaggtgatc tgcccacctc 40560
ggccttctaa agtgctggga ttacaggcgt gagccactgc acccggcctt tagcatatta 40620
ttcaagcatc tgtaatcctt tatcttctct aaccaaactg cttgttactt tagcctgtta 40680
ttcctatgct tcccaagcca tattctacgg gcttcacagc tattgctgca ttttcatacc 40740
ttttatatta cttatttgtt taactctact ccctccatct ctccctcctt tccgtccctc 40800
cctccctccc ttccttcttc tttccttcct ccctcttctc cctcaatcgc tttctaattt 40860
gcaccccatc cctttctttc agtaactgta tatcgagccc tttctagtta ccagatacta 40920
ttctaagttc tgcgcatata gcagcaaaga aaatggacaa aattcctgcc ctcatagatc 40980
ttacactgtg ggaggacgag caacaaatgt acatgtcaaa taatgataag tgctgtggaa 41040
agatattaag cagagttagt ggggattatc aggaagcaga ctcactacta aactcattag 41100
tctaatattt ttgttttgct tagcctcttc ctaagcaata atatctctga aataatggat 41160
ttgatatgcc agttataatt tttctaaaag acattaatat acattcttaa ctatttaaat 41220
atatattcga tgtaccacct aaaaactgct tgtataccat aaatgattat gtgttaaact 41280
ctgggaaaga gtgtattaga ccattttttt ccagctatcg cagctctatt tatttttca 41340
gcttttattt tagcttcagt gggtacatgt gcaggtttgt tacctgggta tattgtgtga 41400
cactaatgtt tgaggtacaa atgatcccat cacctaggta ctgaggtact gagcatagta 41460
ctcaatagtt tttcaaccct ttcttcttct tccttcctcc tctagtagtc ctcagtttcc 41520
attgttatgc tcatgaatac ccaatgtcta gctcccactt ataagtgaga acatgcagta 41580
tttggttttt tgttcctgtg ttaatttgct tagggtcttg gcctacagct acatccatgc 41640
tgctgcaaag gatgtgactt cgttcttttc atggctgcat aatattccat ggtgtatata 41700

tgccacattt tctttgtcca atccactatt aatgggtgcc tcagttaatt ccatgtcttt 41760
gcttttgtga gtagtgctgt gatgaacatg tgagtgcatg tgtctttcta gtagaacaat 41820
ttattttctt ttggctatat atccagtaat gggattgctg gtctaatggt agttctgttt 41880
taagttcttt gagggccggg agcggtggct catgcctgta atcccagcac tttgggaggc 41940
caacgcaggt ggatcatgag ttcaggagat ggagaccatc ctggctaaca cagtgaaacc 42000
ccgtctctac taaaaacaca aaaaaattag ccaggcatgg tggcaggcgc ctgtagtccc 42060
acctacttgg gaggctgagg caggagaatg gcatgaacct gggaggcgga gcttgcagtg 42120
agctgagttc gcgccactgc actccagcct gggcgacaga gcgagactgt ctcaaaagaa 42180
aaaaaaatgt cttttgagaa gtgttcaaac caatttctac cgtggcagaa ctaatttaca 42240
ttcccaccaa cagtatataa gtattccctt ttttccacag cctcaccagc atctattagg 42300
ttttgagttt tttgagtttt ctttttttgag acagagtctc actctgtcac ccagactgga 42360
gtgctgtggc acaattatgg ctcactgcag cctcaatctc ctgggctcaa gtgatcttcc 42420
tacctcagca tcctgagtag ctgagactac aggcacgcac caccacacct ggcaaacttt 42480
ttatttattt tttttttgta acgataaggt ctcactttgt tacccagtca ggtctcaaac 42540
ttctgggctc aagtgatcct cccaaagtgc tggaattaca ggtgtgagcc accatgcctg 42600
gcatgagttt ttaataatag tcattctgat tggtgtaaga tggtatgtca ttgtcgcttt 42660
gatttgcata tcttaatgat tagtgatatg gagcatttta aatgtttgtt ggctgcttgt 42720
atgtctcctt ttgagaaatg tctgttcaag tcttttgccc atttttttgg tgagattttt 42780
tttttttgct tgttcaactg tttaagttcc ttatagattc tggatattag acatttgttg 42840
gaggcatagt ttgtgaacat gttctcctat tctgttggtt gtctgttaac tccattggta 42900
gtttattttg ctgtgcagaa actctttagt tgaattaggt ctcacttgtt aatttttgtt 42960
tttgtttcaa ttgcttttga gaacttagtt agaaattatt tcccaaggct gatatccaga 43020
atcatatttc ctagattttc ttttaggatt cctatggttt gaggtgttat atttaaatct 43080
ttaatctacc ttgaggtaat ttttgtatat ggtgaaatgt aggggtccaa tttcattatt 43140
ctgcatgtga ctaggtaggt atcccagcac catttattga ataaggaatc ctttctgcat 43200
tgcttatttt tgtcaacatt gtcaaagatc agatggctgt aggtgtgcac ctttatttct 43260
gggttctata ttctgttcca ttggtctatg tgtctgctct cgtgccagta tcatgctgtt 43320
ttggttactg tagccttaca gtatagtttg gaattgggta atgtgatgcc tctgactttt 43380
ctttttgttt aggactgctg tagttatttg ggctcttttg attttatata aattttagaa 43440
tagctttttc tagttctgga aaaaatgtcg ttggtagttt gataggaata acattgaatc 43500
tgtaaattgc tttggacagt atggccattt taacaatact gattcttcta atccatgaac 43560
atggaatgtt tttacatgtg ttagtgtcat ctgtgatttc ttttagcaat gttatgtagt 43620

tatccatgta gaaatctttc atctgcttag ttatatgtat acctaggtat tttgtgtgtg 43680
tgtgtggcta ttttaaatgg gattactttc ttgatttggc tctcaccttg aatgctatag 43740
ttgtatagaa atggtactaa tttttacatg ttcattttgt gtcctgaaac tttactgaag 43800
tcatttatca gttttaggag ccttttggtg gagtctttag ggttttctag ttataaaatc 43860
atgtaatatg caagagagat agtttgactt ctttttttca tgtttgaatg ccttttattt 43920
ccttctcttg cctgactgct ctggctagca cttccagtgt taacaggagt ggtgagagtg 43980
agcatcctta actttttcca gatctcaagg aaaattcttc cagcttttgc ccattcagtc 44040
tgatattggt tgtgagtttg tcattttggt ctcttattgt ttttaggtat atttctttgt 44100
tgcctagttt cttgagggtt tttatcatga agtggtattg cattttatcg aaagctgttt 44160
ctgtgtctat tgagatgatc atatggtttt atttttaatt cttctcatgt ggtaaatcat 44220
atttattgat ttgcatatat tgagctaacc ttccatacca ggagtgaagc caacttgatc 44280
gtggtaaatt aactttgatg tttgatttgg tttgctagtg ttttgttgag aatttttgtg 44340
tctgtgttta tcagagatat tgtcctatag ttttcctttc tcatggtatc tcttacaggt 44400
tttggtatca gggtgatact ggcttcagaa aacgagttag ggaggagtct ttccttctcg 44460
attttttgaa atagtttcag tagaattggt accagctcta ctttgtgcat ctggtagaat 44520
ttagctgtga atctctctgg ccagggcttt ctttttggtt ggtaagtttt ttattactaa 44580
ttccatttgg aacccaatat tggtctgttc agtgctttag ttttttcctg atttaaactt 44640
gggacattgt gtgttttcag gaatttattc atttcctcta gatattgtag tttgtgtgtg 44700
tagaggtgtt cacaatagtt atctaaggat ctttcatatt tctgtgggat cagttgtgat 44760
gtcacctttg tcatttctgt ttgtgtttat ttggatcttc tctctttttt tctttgttaa 44820
tgtagctagc agtctatcaa tcttatttat cctttaaaaa aataactttt ggttttgttg 44880
attctttgta tagatttttg ggtctcaatt tcatttggtt ctgctcttta gttcgtcctt 44940
ttcttctgct agctttaggg atagtttgtt cttgtttttt tttttttttt ttttttttta 45000
gttcctctag gtgtgatgtt aggtcattaa tttgagatct ttctaacttt ttgaggtagg 45060
catttagagc tgtaaactct cctcttaata ttgtttttgc tacattccag agattttggt 45120
atgttgtgtc tgttttcatt tattttaatt ttttttttatt tctgccttga ttttattgtt 45180
tactcaaagt tatccagagc aaacttttta atttccatgt aattgtgtgg tttgtacaga 45240
tcttcttgat gttggtttct ctttgtattc cactgtgacc tgacagtatg actggcatga 45300
ttttgacttt ttaaaatgta ctgagtcttg gtttatggcc aaggatgtgg tgaatcttgg 45360
agtatatgtt ctgtgtacag atgagaagaa tttacgttct gtggttgatg ggtggattat 45420
tctgtagatg tttattagat ccaattggac aagcattgag tttaagtcca gaatttcttt 45480

gttagtcttc tgccttgatg atctaatgct atcagtcccc cactattgtt gtgtggcttt 45540
ctaggtcctt agaagtcctt gttttatgaa tctgggtgtg ccagtgttga gggcgtatgt 45600
atttaggata gttaaatctt cttattgaac tctttatcgt tatgccctta tttttccttt 45660
tttactgttg ttaaagtctg ttttatctaa tctaagaata gcaacccctg ctcttttttg 45720
ttttctgttt gcatgctaca tctttctcca accctttact ttgagcctat ggttgtcatt 45780
acatgtgaga tgggtttctt gaatacagaa gatggatgag tctggttttc ctagctggtt 45840
tgccactctg tgactttttt ttttttttag atggagtctt actctgatgc ccaggctgga 45900
gtgcagtggc gcaatctcag ctcactgcaa cctccacctc ccaggttcaa gcaattctcc 45960
tgtctcagcc tccggagtag ctgggattat atgcacctgc caccacggct ggctaatttt 46020
tgtattttta gtagagatgg ggttttacct tgttggtcag gctggtcttg aactcctggt 46080
ctcaggtgat ccacccgcct cagcctccca aagttctggg attacaggtg tgagccactg 46140
tgcctggacc cactctgtga cttttaaatg ggacattgta ttagtccatt cttgtgctgc 46200
tataaagtac tacctgagac tgggtaattt atgaagaaat gacatttaaa tgactcatag 46260
ttctacaagc ttaacaggaa gcatagctag gcaacgtcag gaaacttaca atcaaggcag 46320
aaggtgaagg ggaagcaaga atatcttacc atggcagagt aggagaggtg ggtcagggga 46380
gtgccacaca cttttaaatc attatatctt gggagaaccc actcactatc atgacaacag 46440
catgggggaa atttgcctcc atgatccaat aacctcctac caggttcctc cctcaacatt 46500
gggaattaca attcaacgtg agatttggat ggggatacag agccaaacca tatcattctg 46560
cccctggccc ctcccaaagc ttatgtcctt ctcagttttc aaaacacaat catgccttcc 46620
caacagtccc ccaaagtctt aactcattcc agcattaacc caaaagtcca agaccaaagt 46680
ctcgtctgag acaaggcaag tcccttccac ctatgatctt gtaatatcaa aaacaagtta 46740
gttacttcca agatacaata ggggtatggg cattgggtaa atactcccat tttaaatggg 46800
agtaactggc caaaacaaag gagatacagg ccccaagcaa gttcaaaacc cagcagggca 46860
gtcattaaat attaaagctc caaaataatc tcttttgtct ccatgcctca catccagagc 46920
atactgatgc aagggatggg ctcccaaggc cttcagcagc tccaccctgt ggctctacag 46980
gatacagccc ccacagctgc ttttacaggc tggcattgaa tgcctgcagg tattccaggc 47040
acacaatgca agctgttgat ggatctacga ttctgaggtc tggttgatgg tggtcctctt 47100
ctcacagctc cactaggcag tgccccagtg ggaactctgt gcaggggctc caaccccaca 47160
tttccctttc acactgtcct agtagaggtt ctccatgagg tctctgcgct tgaagcagac 47220
ttctgcctgg acatccaggc atttccatac atcctctgaa atctaggtgg aggtttccaa 47280
acctcaactc ttgccttctt catacccgca ggcccaatac catgtggaag ccaccagggc 47340
tttgggtttg catcctctga agccctggcc caagctgtac cttggcccct tttagcaaca 47400

gctggagctg gagcagctgg aatgcagggc accatgtccc aaggctgcac agagtagcta 47460
ggccctgggc ctggccctca aaaccattaa tccctcttag gcttctgtgc ctgtgatggg 47520
aggggctgct gtgaaggtct ctgaaatgcc ctggagacat tttccccatt gtcttgactg 47580
ttaacatttg actcctcttt actaatgcaa atttctgcag caggcttgaa ttgctcccca 47640
ggaaatgttt ttttgtctct ctcttttcta ccacatggcc aggctacaaa tattccaata 47700
tatttttttgc tttgcttcac ttttaaatgt aagttccagg ttcagataat ctcttttttc 47760
atgcatatga gcatacatgt ttagaaacag ccaggtcaca tacatctgga atgctttgtt 47820
gcttagaaat tttttccacc agatacccca aatcatctct ctcaagttca aagcttcaca 47880
gatctctagg acaggggcaa aatgccacca gtctttttgc taaagcatag caagagtgac 47940
ctttactcca gttcccaata agttcctcac tccatctgag accacctaag cctagactca 48000
ttgtccatgt cactatcagc attttggtca caaccattca acaagtctcc aggaagttcc 48060
aaacattccc atatctttct gtcttctgag ccctgcaaac tgttccaacc tctgcccatt 48120
actcagttcc aaagtcactc ccacattttc aggtatcttc ttaactgtgc tccactctcc 48180
cagtaccaat tttctgtatt agctcattct catactgtta taaagaacta cctgagactg 48240
ggtaatttat gaagaaaaga actttaactg actcacagtt ctgcaggctt aacaggaagc 48300
atagctagga ggtctcagga aacttacaat catggtggaa ggcaaatggg aagcaagcac 48360
gtcttaccag ggcagagcag cagagagaga gagagagagt gagaggggaa gtgccaccac 48420
ttttaaacca tcatatctcc tgagagctca ctcattatca caacaacagt atgggggaaa 48480
tgtgccccca tgatccaatc acctcccatc aggtccctcc ccaaacatta agaattaaaa 48540
ttcaacgtga gatttgggtg aggacacaga accaaaccat atcaggcatt tagaccattt 48600
acgttcaagg ttaattttta tatgtgaggt tttgatccct tgtttagttg ttagctgttt 48660
gctttgttgt ttctattgtg tttttgcttt gtagggtgtg caggctatgt gcttaagtgt 48720
gtttatgtgg aagcaggtat ggttcttttg cttccatgtt tagaactccc atgtaaggat 48780
ctcttgtaag gatggtctag tggtaacaaa ttcctttagc acttgattgc ctggaaaaga 48840
ttttatttgt cctgcactta tgaagcatag tttggcagga tgtgaaattc ttggttgaaa 48900
tttcttttaa gaacgctgaa aacaggcccc caatctctcc tagcttgcaa acctctgctg 48960
agaaatctgt tattatcctg atagagttcc ctttttatgt gatctgcact tttctctggc 49020
tgcctttaag attttttctg taatattgcc tttgaacatt ctggtgacta tataccttgg 49080
tgattttttt tttttgaga tggagcctta ctgtgtcaca caggctggag tgcagtggtg 49140
agatcttggc tcactgcaac ctccgccctc caagttccat ggattctcct gcctcaacct 49200
cccgagtagc tgggattaca ggtgcctgcc accgtgtctg gctaattttt tgtattttta 49260

gtagagtcag ggtttcaccg tcttggccag gctggtcttg aactcctcac ctcatgatcc 49320
acctgcctca gcctcccaaa gtgctgggat tacagacatg agtcaccacg cccagccagt 49380
gatgttcatg tttgtggtgt ctcacagatg ttctctggat ttctagctct ctagcaagat 49440
taggaaagtt ttcttgaatt attccctcaa ctatattttc cagtttgttt tctttttctc 49500
ctactttctg aggaatgcca ataattcata gggttttttt gctttatata atcttatatt 49560
tctcaaaaac agctcattta aaataatttt tttctttatt tttatcagat tgggttagtt 49620
cacaagacca gccttcaagc tttaaaattt tttcttctgc ttgatccagt ctattgataa 49680
cgaatttcaa aatataattg aaggctttaa gtgagttttt caattccaga agctactatt 49740
gatttttttt aagatgttca tgtctttctt aatttcctag attgctttag aagtttatgt 49800
tggttttcaa ccttgtcttt gattacaatg aacttccttg caatccatgc tttgaattat 49860
ttatgtgtca tttctgagtt tccatcttgg ttagggacca ttgctggaaa gctagtgcaa 49920
tcccttagtg atgtcactgc aatcagattt ttcattatgc cagaattctt gcactggttt 49980
cttctcatct ggagacattg gcacttcaaa tttttgtact tattttcatg tgggtagtag 50040
tttttctttt ttttttcttt ccctataatg gtattattat tattatttta ataatttttt 50100
ttgtttccct tttacccctt ttctagggct tgtgcctcta aagaatgctg ggtaggatct 50160
tttcactttg attctacagc cctatgtgct tctttcagca ggttttatac tgggctctgc 50220
aggtcatccc acaggcccat aggcggcacg tataggtaag agctggctgt gaccaatgtg 50280
gctggatata tacttgatcc ttgtttccga gcaaaagctc tccattgtct taggcaatag 50340
gctgattctg attcatagaa tgcacagtag ttggaactcc ctgctcagcc ccaaggagaa 50400
gggaaccaca aagggcaagt ttggaccgag caggtccacc tgcgtcccct gatggcaagc 50460
acaagcacca gtgccaagga agaatccagt gggtggccac caagcaccca gagttgtaca 50520
cagacctgaa gcttggaaac ctcctcagct gcaaattctc tgcatgggag gcatcctaag 50580
ctcctgattc aggagagtgg gtgctccaga tgcctggaga tctgctttgg catggaatag 50640
agaggggccc cctgcaccaa gatctctaca caggaggggt aaatgacaat tatatatagt 50700
gctgcaggaa acatgtgaat gcagataata tcttcaatat accagttttc tttcttttgg 50760
ctatatgccc aacagtagga ttgctgcatc acgtggtagt tctgttttta gttttaggaa 50820
cctccatact gttttccata gttacagtac taatttagat tactaccaac agtgtacgag 50880
aatttctctt tctccatatc ctcaccagca tttatttttt gtctttttag taacagccat 50940
tttaactgga gtgagataat atcttattgt ggttttattt gcatttccct gatgattagt 51000
gatgttgagc atttttttcat atatctgttg gcatttgtat gtcttctttc aagaaatttc 51060
tgttcatttt gcctattttt aaatgggatt actttttttt tttgctattg agctgagttc 51120
cttatatatt ctgataatta actccttgtc agatgaatag ttttcaaata ttttctctca 51180

ttctttgtct cttcacattg ttaactattt cctttgctgt gcggaagctt tttagcttga 51240
tgaaattcca tttgtcaatt tttgccttga ttgcctatgc cttgaggtct tactacaaaa 51300
atatttaccc aaaagtctta aagcagttcc ccaaagtttt cttgtagtag ttttgtagtt 51360
tcagatctta cacttaagtc tttaatcgat tttgatttta ttttcgtatg tggtgagtga 51420
tagtgatcta gttctgtttt ctgcatatag gtagccagtt ttcccagcag catttattga 51480
agagattgcc ctttccccaa tgtatatcca tggcaccttt gtcaaaaatg agttgactgt 51540
taatgcatgg ctttatttct gggttctcta ttctgttcca ttggtctttg tgtctgtttt 51600
tataccaata tcatgctgtt atggttaaga attctcattt ctgacataga gaccatttaa 51660
ataacaaaag ataaaacatg agagaatatt agaaagcttt taaaagattt aataaaatct 51720
tacagtagaa taatagaggt aataaatatg ttttgacatg tcatgacata gattttgtta 51780
ggagcagttt gttcttatct gcttccgtgg ctatgttttg attataaaaa ttatgttttg 51840
agttatttga tttcttcgtt gcatatgatt cttgtcttta atgaatcact tccctacctt 51900
gaacaggtca cagtgaactt ttgctttagt tcactgctgt acagcactga gtgggaagat 51960
agttacaccc ttaaggatga taatatatct tctactttag gtgttctgct tccatcttct 52020
ttcttctaga aaacattttt gtttaatgac tcacaaaaaa atatctgaaa tgaatacagc 52080
tttatatttt gcagttgtaa aaatgctaaa ctagtataag gtaattagct ctttcaaaaa 52140
taagtcgctt tctacataaa agaatgatta aatgaatagt tatcttcttt gaaatttttt 52200
aaatccacag ttacaggttt tatgggtttt tctttttttc tattattttc ttttttggta 52260
gcaagcctta tcttcacatt ttaaaaggtt gtgttagaaa gccagtttct ctccttctcc 52320
ccctacctcc tcctccacct agattcaacc acttcttatg gcaaagagaa taactttctg 52380
ggctagagag tatgggttgt gtagtaaaga tttttgttgt ttttttgttt tctcctccta 52440
cacttgacag ccaacaaggc tacaagtctt ctttggaaga actgcaaaat tcttgaattt 52500
aggacacagt ggtatctttt ctagaagtac agagtcctgc tctgtgcctt taatacccta 52560
agaacaaaaa tcatttttcc tctttaaggc aagatctact gtgggtgatg gctaccacct 52620
acctctctag acctcacaga atcttcttct taatgggcat ctctttttcc acccaaatcc 52680
tggcctttga atcagcatct ctcagagaca gcacctctca cattgctccc caagttcact 52740
gcattggagc ctgcagccaa ctgatgtcct tctcatcagt ctggttgttt accctaaaca 52800
ccatctcaca catgctagct ggggatggtc ctcagactat tcattctcat ttctttgctt 52860
aaaatttttc tgtgtttgta tgggatgtct tttatggttt gccaactaga acttttcagt 52920
agtatctaca agcatttatt attttctaga ctcagtaaaa agttagaatt gaggggttca 52980
aatatctcct ccattgtagt tgactttatt tgaatgatta agacatatac atttaaaaaa 53040

atcaattcta gtatagtttt ggtttttgga aaatacctga tttcacttta tatttcctgt 53100
tgtctagcat ctttgttttc tttgaagtat atacaaaggc acactgtgtt ttgatctgga 53160
aattggaggt gaatcctgaa aaatggaagg caagcactat cagtgctata aaggattgtt 53220
aaagtgtagt gccagtggac tgatcccagc actgcgcagt ggtcattatc attttggtaa 53280
tctgaagtag aagcatgcct ctagacatcc ttccataatt ttatataggt agtgtaagga 53340
tgactgtcta taaattcacc tgaactacag agagacttct gatataataa aagaaaaata 53400
cagagattgt tcaggatggg aatacaaaca ctaggaaact ggaaaagcaa attaaaactt 53460
ctgaattaat agaaaaagtt aaataggaaa gagaaaataa agggaaactt gaccaaggta 53520
gcaaagaaag aaaatggaaa agaggtaatt atgaggcagt ataaaacaaa ctctgtcgca 53580
aaaaaaaaaa aaaaaaaaaa aaaaattcca tttgtagttt taaaaaaata gtaaagatat 53640
agaaaaatta gtagaaaata caaaaatctg tattcttctt ttatcagaat taaataatta 53700
tgcctgccct attttaccaa attattttat taaaacatgc taaagaagtt tataagcttt 53760
tcttctccct gctcccattc tctataggat ttttgctgag atatttcaaa atttttattt 53820
ttaggttata tttaagcttt cacttctaat ctctctcaaa aacctttcat atcacttgtc 53880
ataaaaattt cttctcatag tatcatcatc cagattcagt cacatataca tcaagaataa 53940
ttgtgcaata ctgtttccta gactcttcat cttatctttt caatgcatga tatttaaata 54000
agccatactt tttaatgttt tcaatttttt aatttatatt tatttatttc aaattttttta 54060
ttatatttta agttctggga tacacgtgca gaacgtgaag gtttgttaca taggtataca 54120
cgtgccatga tgttttgctg cacccatcaa cccatcatct acattaggta tttcttctaa 54180
tgttatccct cccttagccc cccacccccc gacagatccc tgtgtgtgac attcccctcc 54240
ctgtgtccat gtgttctcat tgttcacctc ccacttatga gagaacatgc agtgtttggt 54300
tttctgttct tgtgttagtt tgctctgaat gatggtttcc agcttcatcc atgtccctgc 54360
aaaggacatg aactcatccc ttttgatagc tcaatgttat tgcatggtgt atatgtgcca 54420
cattttcttt atccagtcta tcattgatgg gcatttgggt tggctccaaa tctttactat 54480
tgtgaatagt gctgcagtaa acatacgtgt gcatgtgtct ttatggtaca atgatttata 54540
atcctttggg tacataccca gtaacgggat tgctgggtca aatggtattt ctggttctag 54600
atccttgagg aatcaccaca ctgtcttcta caatggttga actaatttac actgccacca 54660
acagcgtaaa agctttccta tttctccaca tcttttccgg catctattgt ttcctgagtt 54720
tttaatggtc atcattctaa ctggcatgaa atggcatctc attgtggtat tgatttgcat 54780
ttcgaccagt gatgatgagc ttttttttca tgtttattgc ccacataaat gtcttctttt 54840
gagaagtgtc tgttcatatc ctttgcccac tttttgatgg ggttgttttt tgtttttttt 54900
tttgtaaatt tgtttaaatt ctttgtagct tcttgatagt agtcctttgt tagatggata 54960

aattgcagaa atttctccc attctgtagg ttgcctgttc actctgatga tagtttcttt 55020
tgctgtgcag aaggtcttta gtttaattag atcccatttg tcaattttgt cttttgttgc 55080
cattgctttt ggtgttttag tcatgaagtc tttgcccatg cctatgtcct gaatgatatt 55140
gcccaggttt tcctctaggg tttttatggt tttcagtctt acatttaagt ctttgatcta 55200
tcttgagtta atttttgtat aaggtgtaag gaaggggtcc actttcaatt ttctgtatat 55260
ggctagccag tttccccaac accatttatt aaatagggaa tcctttcccc attgcttttt 55320
tggtcagatt tgtcaaagat cagatggtag atgtgtggca ttatttctga ggcctctgtt 55380
ctgcttcatt ggtctatata tctgttttgg taccagtacc atgctgtttt ggctactgta 55440
gccttgtagt atagtttgaa gtcagctagc ctgatgcctc cagctttgtt ctttttgctt 55500
aggattgtct tggttatatg ggctcttttt tggttccata ggaaatttaa agtagttttt 55560
tctaattcta tgaagaaagt caatgttagc ttgttgggga tagcattgaa tctataaatt 55620
actttggtca gtatggccat tttcatgata ttgattcttc tgatccatga gcatagagtg 55680
tttttccatt tgtgtcctct ctgatttcct tgagcgttga tttgtagttc tccttgaagg 55740
ggtccttcac atttctcgtg acttgtattc ctagctattt tactcctttt gtagcaattg 55800
tgaatgggag ttcagtcatg tttgggctct ctgtttgtct attattggtg tataggaatg 55860
cctgtgattt ttgcacattt attttgtatc ctgagacttc actgaagttg cttatcagct 55920
taaggagatt ttgggctgag atgatggagt tttctaaata tacaatcatg tcctctacaa 55980
aaagacacag tttgacttcc tctcttccta tttgaatacg ctttatttct ttctcttgcc 56040
tgattgccct ggccagaact tccactacta tgtttaatag cagtggtggg aaaggacatc 56100
cctgtcttgt gctggttttc aaagggaatg ctttcagctt ttgcctattc attatgatat 56160
tcgctgtggg tttgccataa atagctctta ttattttgag atacggtcca tcaataccta 56220
gcttattgag agtttttagc atgaaggggt gttgaatttt gttaaaggcc ttttctgcat 56280
ctattgagat aatcatgtgg tttttgtcat tggttctgtt tatgtgatgg attacgttta 56340
ttaatgtgcg tatgttgatc caggctttca tctcagggat gaaactgacc tgatcatggt 56400
ggataagctt tttgatgtgc tgctggattt ggattgccag tattttattg aggatttttg 56460
catcaatatt catcagggat attggcctga aattttcttt tttgttgttt ctctgccggg 56520
ttttggtgtc aggatgatac caaataaaat gagttaggga ggattccctc tttttctatt 56580
gtttggaata gttttagaaa gaatggtgcc agctcctcct tgtatctctg gtagaattca 56640
gctatgaatc tgtctggtcc tggggttttt ttggttggta ggctattaat tactgcctca 56700
atttcagaac ttgttattgg tctattcagg aattcaactt cttcctggtt tcatcttggg 56760
agggtgtatg tgtccaggaa tgtatgcatt tcttctagat tttctagttt attttcatag 56820

aggtgtttat agtattctct gatggtagtt tgtatttctg tgggatcact ggtgatatcc 56880
cctttatcat tttttattgt gtctacttga ttcttctctc ccttctttat tagtctggct 56940
agtggtctat ctactttgtt aatcttttca aaaaaccagc tcctggattc attgattttt 57000
tttttttaag ggtttttcat gtctctatct ccttcggttc tgctctggtc ttagttattt 57060
cttgtcctct gctagctttt gaatttgttt gctcttgctt cgctagttct tttaattgtg 57120
atattagcgt gtcaatttta gatcattccc actttctcct gtggacattt agtgctataa 57180
atttccctct aaacaaacac tgctttagct gtgtcccaga gattctggta tgttgtgtct 57240
ttgttctcat tggtttcaaa taacttattt atttctgcct taatttcatt atttatgcag 57300
taatcattca ggagcaggtt gttcagtttc catgtagttg tgcagttttg agtgagtttc 57360
ttaatcctga gttctaattt gattgcactg tggtctgaga aactgtttgt tattatttcc 57420
attctttcac atttgctgag gagtgtttta cttccaatta tgtggtcaat tttagaataa 57480
gtgcaatgtg gtgctgagaa gaaggtatat tctgttgatt tggggtagag agttctgtag 57540
atgtctctta ggtccacttg gtccagagcc tgagttcaag tcctggatat ccttattaat 57600
tttctgtctt gttgatctgt ctaatattga cagtagggtg ttaaaagtct cccccattaa 57660
tgtgtgggtg tctaagtctc ttcataggcc tctaagaact tgctttatga atctgaatgc 57720
tcctgtattg agtacgtatt tttaggatag tcagctcttc ttgttgcatt gatcccttta 57780
ccattatgta atggccttct ttgtcttttg atctttgttg gtttaaagtc tgttttatca 57840
gagactagga ttgcaacccc tgcctttttt agctttccat ttgcttggta aatattcctc 57900
catcccttta ttttgagcat attgtgtctc tgcctgtgag atgggtctcc tgaatatagc 57960
acaccaatgg gtcttgactc tttatccagt ttgctggtct gtgtctttta attggggcat 58020
ttagcccatt tacatttaag ggtaatagta ttatgtgtga atttgatcct gtcattatga 58080
tgctagctgg ttattttgcc cattagttga tgcagtttct tcctagcatc gatggtcttt 58140
acaatttggc atgtttttgc agtggctggt accggttgtt cctttccatg tttagtgctt 58200
ccttcaggag ctcttgtaag gcaggcctcg tggtgacaaa atctctcagc ttttgcttgt 58260
ctgtaaagga gtttatttct cctttgctta tgaagcttag tttggctgga tatgaaattc 58320
tgggttgaaa attcttttct ttagaaatgt tgaatattgg cctccactct cttctggcat 58380
gtagggtttc agcagagaga tctgctgtta gtctgatggg cttccctttg tgggtaaccc 58440
gactttctct ttggctgccc ttaacgtttt ttccttcatt tcaatcttgg tgaatctgac 58500
agttatgtgt cttggggttg ctcttctcga ggagtatctt tgtggtgttc tctgtatttc 58560
ctgaatttga atgttggcct gtcttgctag gttggggaag ttctcctgga tactatcccg 58620
aagagtgttt tccaacttgg ttccattctc cccgtcactt tcaggtacac caatcaaacg 58680
taagtttggt cttttcacat agtcccatat ttcttggagg ctttgtttgt tccttttcat 58740

tctttattct ctaatcttgt attcatgctt tatttcattc agttgatctt caatctctga 58800
tatcctttct tctgcttgat aaatttggct gctgatattt gtgtatgctt cacacagttc 58860
ttgtgctgtg tttttcagct ccatcaggtc atttatgttc ttctctaaaa tggttattct 58920
aggtagcaat tcctctaacc ttttttcaag gttcttagct tccttgcatt gggttagaac 58980
atgctccatt aactcagagg agtttgtcat tacccgcctc ctgaagccta cttctgtcaa 59040
tttgtcaaac tcattctccg tccagtttta ttcccttgct ggtgaggagt tgtgatcttt 59100
tggaggagaa gaggcattct gatttttgga attttcagcc tttttgcact ggtttttcct 59160
catcttcatg gatttatcta cctttggtct ttgatgttgg tgaccttcag atggggtttt 59220
tgtgtggaca tccttttttgt tgatgttgat gctattcctt tctgtttgtt agttttcctt 59280
ctaacagtca ggcccctctg ctgcaggtct gctggagttt gctggaggtc cactccagac 59340
cctgtttgcc tgggtatcac tagcggactc tgcagtacag caaagattgc tgcctgttcc 59400
ttcctctgga atcttcatcc cagcagggca cccaccagat tgccagccag agctctcctg 59460
tgtgaggtgt ctgtcgaccc ctgctgggag gtatctccca gtcaggaggc acaggggtca 59520
gggacccact tgaggaggca gtttgtcctt tagcagagct tgagcactgc gctgggagat 59580
ccgctggtct cttcagagcc agcaggcagg aacgtttaag tctgctgaag ctgcgcccac 59640
agccacccct tcccccaggt tctctgtccc agggagatgg gagttttatg tataagcccc 59700
tgactggggc tgctgccttt ctttcagaga tgccctgccc agagaggagt ctagagaggc 59760
agtctggcta cagcagcttt gccatgctgc agtgggctct gcccagtttg aagttccagg 59820
tggttttgct tacactgtga ggggaaaact gcctactcaa gcctcagtaa tggtggacgc 59880
ccctcccacc accatgctcc agcgtcccag gtcgacttca cactgctgtg ctggcagcga 59940
gaatttcaag ccagtgaatc tcaacttact gggctctgtg gggatggtat ctgctgagct 60000
agatcacttg gctccctggc ttcagccccc cttccagggg agtgaatggt tctgtctcac 60060
tggcattcca ggtgccactg gggtatgaaa aaaaactcct gcagctagtt cattgtctgc 60120
ccaaatggct gcccagtttt gtgcttgaaa cccagggccc tggtgattta ggcacccaag 60180
ggaatctcct ggtctgtggg ttgcaaagac catggggaaa gcatagtatc tgggccggaa 60240
cacattgttc cttacagcac agtcactcac ggcttccctt ggctaaggga gggagtttcc 60300
ccactctttg tacttcccgg gtaaggcaat gccccaccct gcttcagctc gctctccatg 60360
ggctgtagct actgtctagc cagtcccaat gagatgaggt gggtacctca gttgaaaatg 60420
cagaaatcac ccaccttctg tgttgatcgc gttgggagct acagaccgga gctattccta 60480
ttcagccatc ttgccagcca gcaaccactc tacttttttt ttttttaatt taagttctgg 60540
gatacatgta cagaatatgc aggtttgtta catagatata catgtgccat ggtggtttgc 60600

tgcacccaac aatccatcat ctacattaga tgtttctact agtgctatcc ctcccctagc 60660
tccccacccc tcaacaggcc ctggtgtgtg atgttcctct ccctgtgtcc atgtgttctc 60720
attgttcacc tcccacttat gagagaatat gcagtgtttg gttttctgtt cttgtgttaa 60780
tttactaaga atggtttcca gcttcatcca tgtccctgca aaagacatga actcatccct 60840
tttgatggct gcatagtatt ccatggtgta tatgtgccac attttcttta tccagtctct 60900
cactgatgag gatttgggtt ggctccaagt ctttgctatt gtgaatactg ctgcagtaaa 60960
catacatgtg catgtgtctt atggtacaat gatttataat cctttgggta tatacccagt 61020
aatgggatta ctgggtcaaa tggcatttct ggttctagat ctttgaggaa tcatcacact 61080
gtcttctaca gtggttgaac taatttacac tcccaccaac agtgtaaaag ctttcctatt 61140
tctccacatc ctctccagca tctgttgttt cctgactttt taatgatcgc cattctaact 61200
ggtgtgagat ggtatctcat tgtggttttg atttgcattt ctctcatgac cagtgaagat 61260
gagctttttt ccatgttttt tggccacata aatgtcttct tttgagaagt gtctattcat 61320
acccttcacc cactttttga tgtttttttt ttcttgtaaa tttgtttaag ttccctgtag 61380
actctggata ttagcccttt gtcagatgga taaattgcag agattttctc ccattctata 61440
ggttgcctgt tcactccgat gatagtttct tttgctgtgc agaaggtctt tagtttaatt 61500
agatcccatt tgtcaatttt gtcttttgtt gccattgctt ttggtgtttt agtcatgaag 61560
tctttgccca tgcctatgtc ctgaatggta ttgcctaggt tttcttccaa ggtttttatg 61620
gttttaggtc ttatgtttaa gtctttagtc catcttgagt taatttttgt attaggtgta 61680
gggaaggggt ccactttcag ttttctgcat atggctagcc agtttcccca acaccattta 61740
ttaaataggg aatcctttcc ccattgtttt ttttggtcag gtttgtcaaa gatcagatgg 61800
ttgtagattt gtgacgttac ttctgaggcc tctgttctgt tctattgttc tatatatctg 61860
tttgggtacc agtaccatgc tgttttagtt actgtagagt tgaagtatag tttgaagtca 61920
ggtagcatga tacctccagc tttgttcttt tgacttagga tgtcttggct atacaggctc 61980
ttttttggtt ccatatgaaa tttaaagtaa tttttttcta attttgtgaa gaaagtcaat 62040
gttagcttga tggagctatt attggatcta taaattactt tggtcactat ggccattttc 62100
aaaatataca ttcttcctat ctatgaatat ggaatgtttt tccatttgtt tgtgccctga 62160
tttccttgag cagtgttttg tagttctcct tgaagaggcc cttcacatcc cttgtaagtt 62220
gtattcctgg gcatttaatt ctctttgtag caattgtgaa tgggagttca ctcatgattt 62280
ggctctctgt ttgtctatta ttggtgtata ggaatgactg tgatttttgc acattgattt 62340
tgtatcctaa gaataagcca tactttttaa tatattttat tcataaaaaa atgcacataa 62400
agcaaatgac atatagaaac aatatgatta acatccacct ctggactcag ttggcttaag 62460
aaaatatgaa tattatcact acacttgaaa ttgattgcat atttcttcct tatcacattc 62520

ctctttatct ttctccagaa acctctatat ctttaatttg gagttggtca ttttcttata 62580
ttattctgta ggatttttta atatctatgt atttctcact aatagatttt ttagttttga 62640
gatgtttttt tctactccat agaaatgcta tgtatattta tgcaatttga tcttactgcc 62700
taatattata tttgtgtttt ttattttgat ttgtattatt ctggtttgtt ttttcctaca 62760
ctgtatgatt tcattgtatg agtatctccc atttatttat tctcatgtat tgacatgtac 62820
ttccacttga tatttgctgt gctttaccta cagttcaatt gtgaacatta ttttacatgt 62880
attctggtgg ttcatgtgtg agagtttctg taggtaacct gcacacctgg gcatgaaatt 62940
tctgactgta atacatgctc atgctgggct ttactagata atgctagatt gtttttaaag 63000
tagtagtagt ttacactcca tggcagctct tcattgtttt gtgtgttcct gtattctgtg 63060
tccttgccgt tttaggtaga tgatgtctca tgtggtctta ctttgcattt ctctggttaa 63120
taatgaggtc agtcatattg ttgtattttg tcatctatat atttcttcct ctgtgaaata 63180
tctgttcatg ccttttcttt ctgatttata ggaattcttt gtgtattttg ggtatatatg 63240
ttataaatat ctataggata gataaaaaca agctatctga taaggcgata tgcagaaata 63300
taaattaggt gaggaagcaa gccatgcaaa tatccaagga aaaaatcatt tcatgcagag 63360
gaacttgtca gtgtaagacc ccaaggcaga aacacacttc aagggcaaga tgggcatcaa 63420
tatgggtgga gcatgaggga gaatagaaga gagaccatct atgcaagggt aagttgggcc 63480
ttgtagagct tgatatgaat tttaacttca ttctaagtag gatcaggagt cactgaaaat 63540
atcaagcaag aatgacatag tctgattaag tctttaaagt tgtatagaaa atagtctaca 63600
aaaagtgaaa gctgggaaac cagccagcat atgttacaat agtccaactg tgattgtggg 63660
gacataaatt atggtgcaag cagtggaaac aggtatttgg atttgggtta tattttcatg 63720
gttatccact aggatttgct gatgtattag atatgaggtg agaaaataag tgaggtatca 63780
gtaatgactc tagagtttgg ggctagagca gcaggtgtgt aaatgaagtt acaaacctag 63840
aagatgcatg tttggaccaa tgcatcaaag ttctgctcta aacatattaa tttgaggggt 63900
ctgttagaaa tacaagtgga gatgctgaga atacagttgc atagttgagt ctagagtttc 63960
ttggaaaagc tgggtctagg actgtaaatt tggtaatcat taatgtctag atgatattag 64020
agacatagaa ctagatgcag acacctagaa agagcataga tatctaagag aagtctgagg 64080
actgagcttg gtgtactcaa agatttagaa gttgagaaga tgaaaaagag ctagacagag 64140
agactgaaag agtgactaca agaatgaaat atgtggaagc caaatgaata agagcgttaa 64200
aagaagaagg aaacgatcaa tgtgttaagt acttacccgg gttaagacca ggactgagaa 64260
ttggccattg ttttttgaca tttagaaatc tttgaaggcc ttgccaagaa taatttggtt 64320
gggataagga gaggaatgac ctgactggag aaggttcaag ggagaattgg aggagaggaa 64380

atgaagagag tgatatttct ccacggaata cattatgtgt gagagggact attagaggat 64440

gcagacaaga gggcaaatga tttcagagaa aagtccctga gtgggtgaga aggactaaga 64500

tttggtattg aaatgtcagg agtatgaatt gtaacaggag ggaaggcaga gtacaggggt 64560

agccatactg tagattggcc acaaagaaaa tattctccaa atggagcttc tttcccccta 64620

agcctccccc aaatcattac ctctgcttct gattcaatcc ctcaaaacct catccatcag 64680

cttgaatgag agacaactat tcagccacca tgtccatccg ctttcttatc ccccttgct 64740

cacccccagc gtctcagccc aaatcttccc tccttccagc caacaaagct cccatgcctg 64800

tcatctcctg atgtcaatgc agcgggcagg ggagtggggg accactgacc ccacatgcct 64860

attctctgct tagattaccc aaaataccag gtgtcgttat ttttcagagg tgcaaaaata 64920

taatacaaag tcattctaaa ctgtcactga attcagagta ctttttgcca ttgtctagtc 64980

actgaattca gagtactttt tgccattgtc ttattttctc aatcaacaaa tgctaagagt 65040

acaattatat gaagttctac agatttattt gttacttgca ttacttagta atggttctta 65100

tgcataaagg ttaggaataa cttctttgaa caaatatcct atataaaaaa acaacaaagt 65160

ggtttttaaa aatcacattg ctgttcacat tccctttttta gtactctctt ctcatattct 65220

tttttaagct atgttaggtc aaaacaacta ctttaataac ttagcaaata tctatcagga 65280

gtccactgtg tagttagcag tttataaagc ttcagatcta cattagtgag tgaaatggac 65340

ctagcccccta tccttacagg gcagcaagcc tggtggagga agctatgttc tctgaaggca 65400

acaaaaacag tcatgcgtca tttaagagta ggatacatt ttgagaaatg tgtcatgaag 65460

tagtcaattt catcattgaa catcatagaa tgcatttacg cagacttaga tggtatagcc 65520

tactacacac ctaggctgtt tggtgtagcc cattgctcct gggctacaaa ccgatacagc 65580

atgttactct gttgaatttt gtaggcaact gtaatgcaat ggtaagtatt tgtgtaccta 65640

aacatatcta aagatagaga cagtacagta aaaaatacta tcttatgaaa acactgttgt 65700

atatgtggtc tgtcattgac tgaaacaata ttatgtagtg caaaccatac agcaacagat 65760

aataaagaca agatattcag agtgggagat ggaaaagaca ataatgtatt ccttcattca 65820

tttagcagac atgtattgcc aaaagacact ggaaaaaata aataaaggtg acaaacaggt 65880

ggtgcctttg ttgaagatta tagtcatgga cagggaaact ttagccctct tcttaacatt 65940

atcttccctt agacacaaac acacacacac acaattcctg gcatatgcta tatactccat 66000

aactgttcct tcccttccct ttgtgactca ttccttcccc cagaaagctc atttgatctc 66060

tgcaaataat tttgaatccc ctttgttttt taaccatggc aattgttttg ttttcccatc 66120

tcctacaagt tttagctcaa ctcgttgggg ctgtttttttt tttttttttt tttttttttt 66180

tttttttttt tttgaggtgg agtttctccc ttgtcacctg ggctggagtg caatggcatg 66240

atcttgactc actgcaacct ctgcctcctg ggttcaagtg attttcctgc ctcagcctct 66300

ggagtaactg ggattacagg tgcccaccac cacacccagc taatttttgt atttttagta 66360
gagacggggt ttcaccatgt tggccaggct ggtctcgaac ttctgacctc aggtaatctg 66420
cccacctcgg cctctcaaag tgctagaact acaggcatga gctcaccatg cctggccatt 66480
ccttggggct ttatagaaca agtataatcc tttttcttta tgacaatacc ctgagttcag 66540
aaatctcaga gacaaatgtt tacagagacc aggtaaataa gtagctagag tgacaagact 66600
taagaaaaga gaccattggc gtaccaccag tttgtggagg gaactggaaa aactggaata 66660
cacatgcccc atccaaaagc aaccattgca actaaacttt aacagattgt tgccacctaa 66720
gtaattcacg gatggtctca taattctggt cagcattgtc tgagccaaac aaaatgtatc 66780
tatgggcatg atcagatact agagccagca gattgcaacc tctgcttaga taattgcagg 66840
tatcagcctt cccttggcta aacagctact tcatactgat aagtagccct tgcctggcac 66900
aaagcaggtg gggctgaatc cagcctgata tcacatcacc acaactttct ctaattctcc 66960
tcaaggcgtc tgtgaactac cagcagccca aataagcatc cttttctcct tcaatcattt 67020
ctcataaagc agattctact catctcacca tccatctttt tcccctaaca tatcagaatt 67080
catcaaagta gaaactccag aaatcatcca gaaatacaaa tgtgatctga ccagcacggg 67140
tataaacttt ctctttcctt aatttagaaa aaggattatt aattttgagt cttagatctc 67200
ataaaagtag agctggattg attttaaggg atcaatgaac ctactgaaat tgtataaaaa 67260
gattttgtgg ggctggcaaa caaatgcttt tcttttaaca tattcttaaa agaacaaaac 67320
ctcaaagaag atttaacaac cactatagac taagtttcaa ttaatgtagt ctgtagcatt 67380
agattttgga aggctgttct atcagaatat ggaccaatac tgagcgtagt cgctactaaa 67440
actctttgtc tttttattca tatcttctcc taaaaagctc catgacccct gcagaatact 67500
ggtataatta cccacacctc acccacccag tgattgttga tgtaactaat tatttaatcc 67560
aagtctgagg cctaaatttt ttttaatcaa gagttagtta catgttttaa tttatttttt 67620
gtaattttaa tatctaacac ttttattcta tttcattcga ttgatgctaa aatgcataat 67680
gctatgattc taagacttag gaaaaataat actatactgg gatatggagt ttatatatat 67740
ttacatgaat ttcttttttt tcttctctgt aggtcgaaaa tttaaaaagt tcaataaagt 67800
cagagttgtg agagcactgg atgctgttgc tctcccacag ccagtgggcg ttccaaatga 67860
aagccaagcc ctaagccaga gattcacttt ttcaccaggt caagacatac agttgattcc 67920
accactgatc aacctgttaa tgagcattga accagatgtg atctatgcag gacatgacaa 67980
cacaaaacct gacacctcca gttctttgct gacaagtctt aatcaactag gcgagaggca 68040
acttctttca gtagtcaagt ggtctaaatc attgccaggt aataataatt tttatataca 68100
gcatgtaata aaaaatatac tatgtttgtt gtattagtac attgttaaat aaaacactct 68160

atgcagtaaa ttaactacaa tataaaacag tatgtgatag tatgtgtttg agcagttcaa 68220
aatatttgtc taggacccta atatgtgtta cagaaatata aaaaattaga gaacacactg 68280
agtcttactg atgagtcaga aattgcatac attaaactta tgctaataag aaatagctta 68340
ggtttatgtg ttcaaccaca gatattagag tagctcagaa atgataaatg gaccattttt 68400
tataaatgcc ctatttattc taacccttaa ttcaaccaac ctcatttggt tgaaaataaa 68460
actgaatgta taaagtcata taacaaatca aagtagtatt cagagtacag taaggtgtca 68520
aaagtaaatt aagaaaacaa aattccctaa cgtaaattac tttaaaaaat aatagcacac 68580
agagaaaaga atctctagtc actattcctc taatttatac ccaaagagag tatatcgccc 68640
agcaacagag agaacttgga ttatatcatc acagaagaat ttcagtcact ttacttatca 68700
gctatttttg ctccctcagc acagctctgt aggagccaag tacttgaaag aataacaagg 68760
atgaaattct cacttggtta aatcttagac tgcagagcca ggagaataca tgtatagcaa 68820
ttgtttgtga aggcattact cctataatta gaaatagaac tttttaaaaa actacattct 68880
cttcacatca gtaataccct tatactgcta tagacaaatt atttttctaa tatctgatca 68940
agaattaaac atattatgtc cttttagtcc ccaagtaaat tcaatggcac ttttagaaaa 69000
agaatttctt cccaattctc cttaatataa ttctcagtta ttattctacc taaaaggttt 69060
gaaatacaat agatgaattg ttactgaaaa ttcaattaca tgaaacagaa agaaaaatag 69120
atacctgttt gttgaatctt tcaaaaaata catattaaga aaagttaata gaattcagta 69180
ttctagtgga gatgaagtaa ttttatagaa aaatcaaaat gcccagattc ttactggctt 69240
cagtctcacc aaaaggcctt aaacagacaa taattttttg aacattctaa atgtttaaga 69300
tttatcacat agtggttatc aaacttgaca gagctttaat aaataaaaat aacccaatag 69360
cattgatgaa ctttttctgt ttaaaaatga gtgtttaaac atgtgtttaa tttttctcca 69420
ttgttctatt atttggaaaa tatgatttct ttggtttttg ttgatatagt ttatattcct 69480
tttgtttgca attttggagg ttctttacta tttaaaaaga ttctagcatc agctggtaac 69540
gttagaaaat aaccagttat acttgaaaag atgacttatt cactttggat aaaagctaag 69600
tgaccttttt aaccggaaac cgttatctat caggactttt tgacccccac acaaccacaa 69660
aaaggaatga gagaaaactc ttacattgaa tttggtggca actttttttaa tgctgcaaaa 69720
tgtaattttt gtgtagtaaa tcagaataat ggcaacacta gtattaccca tactgtttcc 69780
attaattgca aacagggaaa cattgagatt atcaaagtct cttgtggcct gtggactttt 69840
aaagccatat tttcatggtc tgcattttgg gaatagcact attgaatgaa ttatgaaaat 69900
ggtcagtgag ctagagtgac tatttgacct ctgagaaagg acttggatgg acacatgtat 69960
agaaagtact ttagtattat tagctactta aaactgattt caattgactt gaaataagct 70020
tacagttttt attcacttat tcactcaata aatgtctatt atgtacctgc tgtgttcaat 70080

atctaataat tatacagtgg tttagaaaga aaagccacaa ggctgggctc atggtgtgga 70140
tgcattttgg tacagcacat gctagcatct ggtcatcagt tttgcatgta aaagaaattt 70200
atttcgttca ttcactcatg tgtaataaaa ctaataagtc tgtggaagac agacttttgt 70260
taagtgatcc tcactaatta gccagaatgt caactaactt gcttatttac agtgagacac 70320
acacacacac acgcacacac acacaatata aatatacata atacttatgt tgctaggaga 70380
atatattaac agatcagatc attctttcac aataggaaac tgtatttgga aagtcacatt 70440
aagatctgat gcagaatgac catcataaca aaggtgacag cgtttatgaa gacacctagg 70500
catatatttg caggtgccct aagaattctt attgcctggt agggctttct catttcttct 70560
ttgtctacac tcttccatta tcttggatct gttataataa tggtagttta tatttcttca 70620
aaaatatttc agatatatcc ttttgtttct tttcttttt ttcttttttt tttttttttt 70680
ttgagagagg gtctcactct attgcccagg ctggaccaca gtggcatgat cttggctcat 70740
tgcagcctgg acctcttggt ctcaagttat cctaccacct cagcctcctg atgagctggt 70800
agctgggact acaggcatgc gccaccttgc ttggctaaat ttttttatt tttagttgaa 70860
attgggttta actgttttac ccaggctggt cttgaactcc tggactcaag tgatctgcct 70920
gcctcagcct cccaaagtgc tggaattaca aacatgactc accgcacccc acccctttta 70980
ctttaataga gacttgcaag atgctctagc cttatgtcag cacgtttata aaccatctct 71040
taggatacat tgcaaatgag tgattttgca tgggctttta tctgtagcct tagtgggcaa 71100
tattttctta tttgaaatgt agagcaacac gatatttgaa tttttttaat gaacaagaca 71160
tattccctct attaaggaac tctcaatcca gtagcagaga gaagataccc aaacaactat 71220
agtgttatgt gaaagtgata taagatacag atataatgaa agtggggaca gaagaacatt 71280
tttggcttca aggactaaag aagccttcag agacaaaaat gtcagttaaa ctgcatctta 71340
caggagaaac aagactttga taaatcatga taagagaaag cattctagat agaaggaaca 71400
gtatggctga aataagaaaa gcatgagata ggtttgctga ataatgcatt aaaccagcat 71460
attaatggtt ctcaaccttt tcaagtgtgg ggggtccctt agtaacatca aaaattttat 71520
agtctcccca tagggtagta attaaagctt ttagtagcta tggattgaga aaacacattt 71580
gtacatacag tccctcggac actgctacag taagtactgg ccttcgggag atttaatcac 71640
ccaaatggta agaaaccatt gaaaagaaat ttattttgaa ctgaataatg gaaagtaaac 71700
ttagaggtgt aagttatagg ctgatcaagg agggtcttaa aaattactgt agatgttaga 71760
gtttaaatta aaagtactaa ttcacaattc ggtgtcacct ttaaattgtc ctcatcctgt 71820
acatcaatac gttgcagtga aattgatgtg acagaggggg tatgagggaa acacttccgt 71880
tgcataaatt ttgtcttgat atttcagtta acttagtgaa aactccattt aggtcaatgc 71940

tttgaatttt taaaccttgc attttttagg acatacttta ttctttgtgt catttcacca 72000
gactacttgc aattatcagt atcagaatat tagaacagta ttttgcctca tttttctgct 72060
tttgaagcgt gccaaaacaa cttttgagtc agtgagtcat tgcatcttga cagaggattt 72120
tttctcttcc ttcccgaatc agaagactaa attaaattat tgctctccag aaaaaaatga 72180
tgagagagaa atgagagtag catactgaga gtaaaggcct taataaatgt gttgataaag 72240
actaaaccat ggtttttctc acaccctatt ccactgaaat tatgattgcc agtggcaaca 72300
aaaattttta cttgtcaaat tagatgaata tttccagccc ttaatgtgtt tcacctacct 72360
ctgggactat tggcaaagta actatttctt gctaagactc ttgcttaggt atttgtcact 72420
tcccttccag gtagatatgt ttctctccct atgtacattt cccgactcct gtgtggattt 72480
gttcttccac ccacactata aatttgtttg tgttctgggt tctatctttt ccagtcattt 72540
ctcactctac acagtctgca taagctcatt catagtcttg tcttcaacta ccactatatt 72600
tcgatgactc ctaaatacat aatattagcc agaccctcta caaaactact gaactttcaa 72660
ctgcttatta cacagctcca cccagatgac ttctaagtca gcatgacccc ctaaacctaa 72720
ggccttttcc agtatcttga aatggcacct cagcaagaat ttttggtatc atccttggct 72780
cagtttctcc ttcacccctt acccaataaa ttgtcaaatt ctatcaactc cacttattaa 72840
atatatgatt cattatacat gctacttctg ttgctacttc cctgacttag tttggtgctg 72900
gtcttttttt acttggattc atgttgcagc ctcctgactg attctattac catcaatttc 72960
acgtggtgcc caaatgatcc tctattctac ccccagaatc atttttctaa aacaatttta 73020
atcaatcata ttttccattt ctttttccaa atagcatcca aactccatgg cctcaatatc 73080
caagggcttt ctgtgatctg gctcctgaat ttgcagtgtt aatctctcat cattcctctt 73140
cacacgactc taagatctag ttccctaaat gctgtttgct caaattctag ccttcttctc 73200
ttgcccatat tatttttcct acctggcagc ccatctcttt tcccacgtct tcctttgact 73260
gacctgtaac tatctttcag gattcgcatt acttgtcact tgctctggga agatttctct 73320
gagccttcct agactgggtg aggaaaccct gtaccctccc ttgtctcctt tgtgcttgtc 73380
aaccataaca ctatgactct gagttacaat tgtgttctac ttttctatct gtctcacaag 73440
cctatagtgt taaaggcaga aacagcatct ttctaacctt gatcctaatt gcaagtataa 73500
tgcttcctcc caaatagatg gttaaagaat atttatagaa atttaaatta cagaattgat 73560
ttgcaaagaa ctgtactacc ttagattatt agacattaac ttcttattca gcataaatca 73620
tcaaacatac gttggtgatt atacttaatc atattgaagc ttctttgtgc ttctcaacat 73680
tgatacttgt gtatgaaagt tgttgctttg tgtcagaagg tgctgagata tttaatgctc 73740
tgccattgtt gtagattatc tgtttcaatt acatgacatt ttttacagaa ccaaaaccta 73800
catattttat caaaactctg ggagtaagat tggggaactt agtagtactg tcatacaggc 73860

catcttttt ttttttttt ttcttgagat aggctctcac tctgtcaccc aggctggagt 73920
gcagtggcat gattgtagct cacggcagct tagaactcct gggctcaagc catcgtcctg 73980
cttcagcctc cccagtaact tcagcctccc cagcactaca ggtgtgcacc accacacctg 74040
gacaagcttt ttaaattggt ttgtagagac tgggtcttgg ctggtctcaa attcctggcc 74100
tcaagtgact ctcctgcctc gcctcccaaa gcactgggac tacgggcatg agccaccaca 74160
cccagcccca ggccatctct tcactatata ctttctgtta attctttaag ctaaatttgg 74220
acagctgtaa cagggctttt gtcagaaatt atcaggtcaa gtcacttaaa aggaaaagtg 74280
ttttttttt taacatttgt taaacacaat tagtgataat ccatcacttc tccatgtgaa 74340
ggttttcat tttggtcatc tcaaatgtac taagtttacc tgatcattat catcactggg 74400
aatttaatct ctgtgttccc atctttgttt cagcataaag taatgaatga tattacagtt 74460
aataaggaaa aaaatagcat ttctatacta ggcatgttca agtcaattta acattgtaca 74520
tttatcacag ataagcactg taaaaggtgg agtaaataaa ggttattttc tgagttctat 74580
tccattctgt caaacaaaca gaaatatagt ttctgctata aatattggta attttataaa 74640
tttaaagtta ttgtaataag atttgccttt aatgttgtca taaaataaaa gtaagcctgg 74700
atttctagtc ccagaatgca aaaacaaaga aaaaagcaaa aagacgaaaa caaaaactaa 74760
gagtaggata aagtgaatgt aaacatgtaa atgtagacag gatctgtttt tggataaaga 74820
acaatttaat tcttacataa ttttgtaaaa tgtttaaatg tttaaaacta ctcaaaggtt 74880
aacataaaac aaatgcctga agtgaaaaat tacagcatag ttaagtcaaa tatttaaata 74940
ttataaaata cttttttag aaagttataa agttcaccaa gaaagatata gagaataata 75000
aatgtatttg tggagattat tgtgaacact tggttaatgt tttaatacat ctgattaatg 75060
aaaacgaact ttctatgagc ctttttttt ttttttttt ttttttttt gagatggagt 75120
ctcgctgtgt caccaggctg gagtgcagtg gcacgttctt ggctcactgc aacctcagcc 75180
tcctgggttc aaaccattct cctgcctcat cctcccaagt agctgatgag actacaggca 75240
tacaccacca cacctagcta atttttgtat ttttagtaga gacagggttt taccatgctg 75300
gccaagatgg tctcaatctc ttgaccttgt gatccaccca cctcggcctc ccaaagtgct 75360
gggattacag gcgtgagcca ctgcgcccag cccatttttt tgtattatat gaaatatata 75420
atttgttata tataacaaca cacataaaat atatattctg gaaaatatat attccagaac 75480
aaacacataa tttatcttta aaattttttc accattattt tttatacttc agttctatct 75540
tatatgttct ataatatata aaatgcctcc taacactttt ttttatggca ttgtgtttta 75600
gtattagaga cttcccatta aactgactgg tggatttcat actagtatta ctccaatgag 75660
caattctgtt gcattgatag accaataata aatatctcca tagtgttcta cagtgtgcat 75720

aattatccca tttgtctcat agcaactcac tgagttagtt atcattgtga attgtgattt 75780

ttacttatga agaaatttag attcaatgat aataagtgat gtttccaaag tggctgttag 75840

taagtggtag agccaggact ggaacttggg tcttctgtcc tgaatgcctt ccaatttgtt 75900

gagacagatc agcaataggg aggtttatat gtgcctccct ttagaaaggc ttccttttca 75960

aaaattaaga caattcattg atctcttgtc aaatgattct aaattttatc acaaaattac 76020

ccatttttat agtctcttaa atatttgact tacaagcagc atttatgtat tagatttatt 76080

gtattgagct ttataatttt cttaaaaagt tattatgtag acctatactt gaagacacgt 76140

tcaagtcata tgcaaattaa aaaatgataa aacttgcata ccaaaaattt ttttggctgc 76200

ttagttagag agtctttctg cacctgagta aaccaggatt cttggatgcc agtattaaaa 76260

ataaactatg ttaattataa aacacacaag caacaacaaa aaaccctgac ttttaaaaac 76320

aaaagaaaaa gaaaaaaaat aatttactga gaggacaaca ggggttcact gaatagaagc 76380

tagaggacaa gcttagaata tgggcagaag ccaaggcaca tctgaagggc taaaaagcaa 76440

gcatcacaaa tcctcccatt ggttctcatt atctggacac caccaacatc aagaataaat 76500

agtcatatct ttctctttgc tttagtattt tctgattcaa attcctgtta gtatatgttc 76560

catgggccaa acctatgccc caagcccaaa gaaggaggag gtttacccag ctttctccgt 76620

gggaaagtag gaaggtaagg catttgctct cgcaaaaact acaaccaatg gggaactctg 76680

cccacacaga gaaaggaagg aggggaggaa ggaaggaggg aaggaaagaa ggaaagcagg 76740

aaggtattag ggagcagtga gggaggagga ggaaaaagag gaaaggaaga aagagaggga 76800

aggaagaaag gagggaggga ggaagggagt gagagaagaa aggagggaga aggggaagaa 76860

agaaaggagg gaaaagggga gggaagaaag gaggaagaag gggagggaag aaaggaggga 76920

gaaggggagg gaagaaaggg agaaggggag ggaagaaagg gagaagggga gggaagaaag 76980

ggagaagggg agggaagaaa ggagggagaa ggggagggag gaaaggtggg aaggagaaaa 77040

ggttagacat tgaagagcat cccttctcag taaaaggtaa atgctcacta tggcatctct 77100

aattatgaat atgcatttga gtttttggtc ttattgaggc taaggagttc taagaagaga 77160

gtagcaattt ttctactatt cacatgaaga tgggacgatc tgaagtgtat gaagagaaga 77220

gccttctttt ttgctaccta ccttgacatg attaacattg tcaggaaata actttctttt 77280

gctcttgata gctaatgaat taagttttgc tttggtttta cttttgaaac agattatttg 77340

taagaacatc agtggatttt tagagaaaat agatgttttt aaaacagctt tatagaaagt 77400

ggttgatgtt ctcagcaaaa cattgtctcc atacctttaa ttttaaaaat tctccatttt 77460

catcatttca gttatactca gatacaaata actttcaaat ttatattaat atttttagcc 77520

catatatctc cccatacttc taattcatat gtgcagctga taccaggatg ttgacccctg 77580

aatcctatcc aaaactattt tctccctagc aagtttcttc tcctttattt tatctcagca 77640

atgctgttat catcatctac ccttcaagac aaatactgaa agatgtattt gattccttct 77700
tcttcccatc taccggcaat cagttgacaa atgtctttta tttttccctt taaatatttt 77760
tctgatgcac tctctcctct taattccaat gtgttgactt gacttacttc aggcttttat 77820
catctctcac ctgggctttg acagtatctc ccaactgtcc tccctaactc cagacttatt 77880
cccttgaatc cacctaccac atagctaaga atgatccatc tacaatgcaa ggctatgtca 77940
ctcccattgc tccttaatag acttgcaaga ccttggatga tctgacccag cctacatctc 78000
taaacacatc tttgcttctc ccacctttaa gttactcatt gttgaactgg tgatcttccc 78060
ttgagaattg ggaagacatg gagagacttg tgatgattaa gtcaagattc atttgccctt 78120
tccctgccat ccttctgcca ccaacaccat ggaacagcct aaaagaggat gaaagaccaa 78180
agcattcaca ccataccccc caatacacac acacacacac acacacacac acacacacac 78240
acacacacac acacagattc taaatctctt aagccacacg gaagtttaaa acaagactgg 78300
attaagtttc agagaacgaa agtgacctga tgactgtgga atctacccga catagttaag 78360
ggttgttaaa agaaaattag atataacaca actgaaagca gtggttttac aaagtaagac 78420
cagtttatgt ttaaatccca aagaactgag atttaacgaa ctgattatat tccattcagg 78480
cattatctct ctttttttct agaaagtttt tcctgacctc cctcccgatt gactgataca 78540
ctccctttta accttctcta ccccagcact catgtgcttg tttctcctgt tgagttgtga 78600
gctgcttttg aggctaggat tgactcttac tcatcttcat ttttatacag tgcctggcac 78660
ccaaatggtt cttattaaat gggtgttgca tgaatgagtg atggatgatg gattcactta 78720
gcaaaatccc agaaatgcta aatcacagtg actaatttca agtgacttcc aaagttgctg 78780
atattttagg ctgtattatt tattaatgtt ttctgatttt cagtgtaaaa tatgttggga 78840
ttgaaaacat tttattaccc tacgcatacc tattttcaga tttatatgta ttttcaacat 78900
ttttgagata attcagtcat ttttatggtc actgtatttt taaatttgtt ttgtaggttt 78960
tcgaaactta catattgatg accagataac tctcattcag tattcttgga tgagcttaat 79020
ggtgtttggt ctaggatgga gatcctacaa acacgtcagt gggcagatgc tgtattttgc 79080
acctgatcta atactaaatg agtaagtagt aacttttgtt gtttttgtta ttttaagtgt 79140
acatgtagga taattttgaa agttatattt caatagatga tcacatattt agtgttcttg 79200
atatgatgac attactgtca ttttacggta attttatatt tggagctttt tcctcttgtt 79260
tcaaaatata gttatatcaa atccaatttt cttacacatc aagaatgtat caagaaatat 79320
atttaaagat aaacttaaat gtttcattaa tcaatttaaa atttcatagc ttggtactaa 79380
tgacaataat aaatgagaaa tatttttgtt actgacttac attcaaaaag taaaaacatc 79440
tttcttcata catgacaaat tagactgcta atttatttaa aaaattgaaa gataaatatt 79500

tcaatttgct ttatatttaa ataattttaa ttaattttct acctacaacc tttaactgaa 79560
taagaatctt ttcatggtaa tatttatatt gttctagtgg taaatgctga cttagaagta 79620
tataactcac acttatataa gtctttacag tttttagata attttcatgt ttcattacat 79680
tttatccaac aattccttga gataagtaag gaatcagcat tcttcattgt acagtttaga 79740
aaactgagtc tcaggagaat tatataatct gccaaataat ttgaatgaca gtgagattcc 79800
ttatctcttt ttagccaaaa tctagttttc attattcttg ttagcttcat ttgactaatg 79860
tggacattat aaaacaacac aaggttggga aaatgcctta attaaatttt ctcagccttc 79920
agcacttgtg tttaactttg tcaattaatt aatgcttgcc aatgtcctct tttactcttt 79980
tctgtatcat tgtatatttc cctagaaaag tttaaaatat ggtaagcttt gtgaactgac 80040
ttctcctatt taattgcaca caaaattata taatttttca tagaaaataa attgtaagta 80100
aataaaacat ttctgagcat tcttcagctt cacattctca actcctacac ctctgctgtg 80160
ttttcttggt ttaggtattt aaagtgtgat aataaaagtg agaaacaatg ttaatcttgg 80220
tcactgtgta tttaatataa ttttctagtt ttaagatttc ttaacctcaa aaattcagta 80280
ttgtgaaaag tttagaagat gtttaaaatt ccaaatattg ggtttgttta ttaaaagtag 80340
tagtcatttt caataatatg taaggtaatt accaaaacca tattccctga taaattacct 80400
aaactggctg agacactatt ttttttcctg ttgctttttc ttttgtgtat tgtgtgtgat 80460
gcaagacagc ggatgaaaga atcatcattc tattcattat gccttaccat gtggcagatc 80520
ccacaggagt ttgtcaagct tcaagttagc caagaagagt tcctctgtat gaaagtattg 80580
ttacttctta atacaagtga gtgagttcaa gtaacttaat gcaagatatc tagtttctta 80640
attcattaga aaagttgcaa acaatatgat tatatagtta tgtatgaggt agacgtcttg 80700
gattataagt ataaagaaga aatacccaat atattgttat agacattaat aaaattactg 80760
gatttttttca tcttttacct accatataat actaaaatag cctcatcaat ttcttttttat 80820
tttagataaa atgatttaat tactctttca tattcaccta attcagtaat ataaactaca 80880
gtcatgtgcc acataataat attttggtca acaacagacc acatatacaa tggttcccta 80940
agatgatagt ggagctgaaa aattatcatc gcctagtcct gttatagcca ccataacatc 81000
atagcacaat gcactaatca catgttcgtg gtgatactaa tgtaaacaaa cctactgtgc 81060
tgccagtcat ataaagtata gcacatacaa ttatgtacag tacatagtac ttggtaaaga 81120
taataaaaaa ctcttactgg tttatgtatt tactgtagta tacttttcta ttgagagtca 81180
ctacttctac ttttttttaag ttaaccgcaa aacagcctca ggcaggtctg tcagagggat 81240
tccagaaaag ggcatacgtc accctgggag atgacaactc cgtgtgtgtt actgcccccct 81300
gaagaccttc cagtgagata agatgttggg gtggatgaca gtgatattga tgattctgac 81360
tctgtgtagg tctaggctaa tgtgtgtttt gggtattagt ttttttaaaa aaagtttaag 81420

caaaaaagaa aatatttaaa aatagataaa gtttatagaa taaagatata gagaaagaaa 81480
gccaggcacg gtggcatgtg cctgtagtcc ccactacttg ggaggctgag gtgagaggat 81540
cacttgagcc caggagtttt aagtccagcc taagcaacat gtgagaactt gtctctaaaa 81600
aaagaaagaa agagaactgt accttgtgtt tttgttttta actaaatgtt attacaaaag 81660
agtcaaaaag ttaaaaaact agagtttata aagtaaaaaa gttacggtaa ggtaaagcta 81720
atttattata aaagaaataa aaatattttt ataaatgtag tgtagcctaa gtgtacagtg 81780
tttataaagg ctatcgtagt gtatagtaat gtcctaggcc ttcacattct gtcaccactc 81840
actcgctgac tcacgcagag caacttccag tcctgcaagt tccattcatg ttaagtcccc 81900
tatgcaagtg tcccattcgt atcttttata ttggattttt actatgcctc ttctgtgttt 81960
agatacaaaa cacttaccat tgtgttacag ctgcctacag tattcagtaa tcacatgctg 82020
tacaggttca tagcctagga ataaggccta ggtgtatagt agtctatacc atctaggttt 82080
gtgtgagtac actctgtgat aatcacacag tgatgaaatc acataaggac acatttctca 82140
gaatatatcc ccatcattaa gtgatacacg actgcaatta atattggata agcacaaacc 82200
atactctcct ctaccaccac tctcacccca tccccaaagt atttactata taaaatacat 82260
aattttgttt agaattattt ttatcaagat aaattgttaa aacatgtaac tataaagatt 82320
agcatcttaa tataatagtg gtttctttat cactgacaca gtccagtgag ttagcaagaa 82380
acacccaggc tccttctagc cagtggcttc acccccttcct agtggcttgc aaacctctat 82440
tggacatgac tagaaatgag cactgagtga gagagatttt gaaggctaga tctgaaacta 82500
tcatacatca ctttcaccca cattccaagg cacttaagct gcaggaaaag ctgggaaata 82560
cagtctagct ataggcccag aagaaagagg aaattatgtt ttataaagca ttgcattgat 82620
caccagaagg acaggcaaat atccgttcca ctttttctta tatccaagga acatacttac 82680
ctctgcctca gtggatcccc aagggtctgg gtgggcaaag ttttaaaaaa aagaaaaagc 82740
agctgctttg acccttgcat aggcaggatc agccctcaaa tgtacaaact gatgcttttg 82800
tgattcattg tgatttctat gacaaccaca gggagctaag acctcaggcc gagggtgagc 82860
ctaaggtctt tcttggcaac atctttacag ggacttcttt ctaaggtccc aaatctcaaa 82920
tggctagggg gtgcagatca ggagggaagg gcaggagaga taaaccctga aatggaaggc 82980
ttcctcatag aatttatgca aagacaggga tgaatacata accatcttgg gaaccctatt 83040
tggacaagga atttcaaggc taatttggtg acagagcatc aattgaacaa aggatattta 83100
cgtggaaccg gcccagagag tcaactggaa tacatttaat aatacagtaa ttagctcctg 83160
cccttctttg catgaaggaa tgaatcctat agcacttatg tcacagatag tcctagatct 83220
tccaaactgg cttatatcct taacataatt ctctatttat ggataggcaa cattatgctg 83280

gggaattaaa caacaatgga attaatcaac agggaggctt tgctcattgt tgtcacttag 83340
aaacttaggc gatcactaga gcagtggtaa gaaaaggaag tgagattcat acaacttcca 83400
ctcacatacc atagagagta agatttttag ccatttgcca cttcacaaaa aggaagaaaa 83460
agaaactatt taatgaatag tacttacgct ttccacaaat tctaatcaag attctaatga 83520
gatttgggga gaagagggtg aacttgacaa acctactgaa aatccatctt taaaactaga 83580
agaataaata gataagcatt ataaaaatat gcagggaatt agaaagctgc tgttaattaa 83640
tacagcatgg gagaagagat atactgaact tttgggcaaa tcagacaagt atatattaga 83700
atataaatag tatagtggaa accacaaatc aatgagtctg aaacaaataa atgttaagaa 83760
aaatagtttt atatttggaa aaaaactgtt tggatatgta gctcaaatta tatccttgaa 83820
taacagataa ttgagttaaa tgtaaacact caaataatta aaaaacaagc tagaagaaat 83880
agaaataatc ttcaatctca aatacctttt ataaaataaa tgcaaggaga agattggcaa 83940
agtaaaactt tgataatttg gtagttatga acttaaacct ctcatttgcc attaaatatg 84000
aaaatataaa tctccaggaa tagacagtag gttgaacaaa catttttaca ttgttctctc 84060
caaaattaca ctaaaggcca gatgtagtcg gtcacatttc taatcacact ttgggagggc 84120
aagctgggca gatggcttga acccaggagt ttgagaccag cctaggcaac ttggaaaacc 84180
ccgtctctaa aaaaaaaaaa aaaaaaaaaa aaaaaaaaaa aaaaaaaaaa aattagccag 84240
gcgtagtggc aagtgcctgt agtcccaggt agccgggaaa ctgaggtggg aggatcacct 84300
caacctagga agtcgaggct gcagtgagcc atgattgtgc cactgtactc ctgcctgggt 84360
gattgagtga gaccatgtct caaaataaaa ataaataaat aaataaataa aaataaataa 84420
attacactaa aatcttagta aagggattta ctatgaaaaa ttaagaaaaa atgattttac 84480
attcatgttt acagctgagg gaaatatagg gattggttgg taaccttaaa gagagctgtg 84540
aaagtttgaa atgactgcta tttgaagtag tccagaaaat tagtacagcc tctatggaac 84600
accatatgga gatttctcaa aaaactataa atataactac cattctatct agcaatccca 84660
ccactggata tctacccaaa ggaaaagaaa tcattatgtc aaaaagacac ctgcactctt 84720
gtttattaca gcagtattca ctatagcaaa gataggaaat caatccaagt gtgtatcaac 84780
agataatagg ataaagaaaa tgtgatgtac tcacacatat acatacatac acacacacac 84840
acacacacac acacacacaa aatggaatag tattcagcaa tcaaaaagaa tgaaagtgtg 84900
tcttttgcag caacatggat ggaacaggaa gtcattatct taagtgaaac aattcagaga 84960
caaagtcaga taccacatgt tctcgcttat aagtgggagc taaataatgc atgtacatgg 85020
gcatagagta tagaataatt gacagtggtg ccttagaagg ctggaaaggt aggagggggg 85080
tgagacatta cctaatggat acaatgtgca ttattgggtg atggttatac caaaagctca 85140
gatttcacca ctatgcaata tattcatgta acaaaactgc acagatggtc attaaattta 85200

tacaaattta ataaaaagag gttctagata tacatataga tgtacatact agagttaagt 85260
tttcaattga caaaaatttg catagatata ctgaattgcc agaattactc ttcagagtct 85320
cctttatata caagatcttg aaaactcaaa accaaagaat taacttattt gtttttcata 85380
agtttgtatc tctggccttc ttttctaggg gaaatggaaa ccaagttttg attggggtgc 85440
aggggataaa tacaggctta agaaatagta attttcctcc ctgttccctg agatacaaca 85500
atattggaat taggccaagg ctgggcatgg tggctcaagc ccgtaattcc agcactttgg 85560
gaagctgagg tgggcgaatc acttgaggtg aggagttcaa gaccagcctg gccaatatag 85620
tgaaacccca tctctaccaa aaagacaaaa attagtcgga catgatggca cacacttgta 85680
atcctagcta ctcaggaggc tgaggcggga gaattgcttg aacatgggag gcggaggttg 85740
cagtgacctg agattgtgcc actgaactcc agcctgggcg acagagcaag accctgtgtc 85800
aaaaaaaaaa aaaaaaaaaa aaaagcaatt aggccaacta acaatcttgg aatggcctct 85860
cagtgttcca gtaaaagaaa gagttgcaag tctgaggtag aaatgattaa gcttaattag 85920
gaaggcatgt caaaagttga tataggttga aaactaggtc tcctgagcca agcaactagc 85980
caagtttcaa atacaaagta agagttcttg aaggacttta aaagtgccat tccagtgaac 86040
acatgaatga taagcaaaac cgccttattg ctgatatgga gaaagtttta gtggtctgaa 86100
tagaagatca aaccagccac aatattccct taagcctaag cctcatccag aggaaggccc 86160
tgattctcct caatactagg aaggcaaaga gatgtaagga aggagcagaa aaaagtttga 86220
agctagcaga agttggttca tgagatttaa gaagccattt ccataacaaa gtataaggtg 86280
aagcagcaag tgctgatgca gaagctgcag caagttaccc agaagatcta ggtaagatta 86340
tcgctactta tgaaagtagc tgtgctaaaa aacaggtttt cagggtagat aaaacagctt 86400
tccactggaa gaagatgtca tctaggactt tcatagctac agagaacaag tcaatgtctg 86460
tcttcaaagc ttcaaatgac aggttgactc tcttgttagg ggctaatgaa gctggtgact 86520
tgaagctgaa gctggtgact tgaagctgaa gctaatgctc atttatcatt ctgaagatcc 86580
taaggccttt aagaattacg ctcaatctgt tctgcctgtg gtttatgaat ggaacaacaa 86640
agctggatga cagcacatct atttatggca tggtttactg aatattttga cccactattg 86700
agacttactg ctcagaaaag aatactactt tcaaatatta ttgctcattg acaatgcacc 86760
tagtcagcca agagctctta tggatatata caaggagatt aatgttttca tttctgctaa 86820
cacaacattc attctgtagt tcatagatca aagagtaatt ttccactttc atgtcttatt 86880
atttaagaaa tatattttgt aaagctgtgg ctgccataga tagtgtttcc tctgatggat 86940
ctgggtaaag taaattgaaa accttctaga aaggattcac ctttctagat gccattaaga 87000
acattcgtga ttcatgggag aaggtgaaaa tagcaacatt aacaagagtt tggaagaagt 87060

ttatttcatc cctcttgcat gactttgagg gtgttcaaaa cttcagtgga ggaaataact 87120
acagatatgt gaaaagagca agagaactaa aattagaagt aaagcctgaa gacatgactg 87180
aattgaagag gtttcgtggt aaaacttgaa agatgaggag ttgtttctta tggatgagca 87240
aagaaagtgg tttcttgagt tggaatctac ttctagtgaa gatgctatga agattattga 87300
agtgacaaca aaggatttcg gagattacat aaacttagtt gataatgcag cagcaggact 87360
taagaggatt gactccaatt atgaaagaag ttctattgtg ggtaaattgc catcaaactg 87420
ctagcattgc atgctacaga aaaattattc acaaaagagt caactgatgc agcaaacttc 87480
attgttgcct tattttgaga aactgtcata gccaccccaa ccttcagcaa ccaccacccct 87540
ggccaatcag cagccatcaa catttagaca acaccctcca acagcaaaaa gattttgact 87600
cagggaagac tcagatgatt gttaccattt tctagcaata aagtgttttt tagttaaggt 87660
gtgttcactt tttaaataat gctgttgcac acttaataca ttctagtata gtgtaaatat 87720
aacttctacg tggactggga aacaaaaaga tgcatgtgat tcactttgtt gcaatatttc 87780
cttcattgcc gtggtctgga accaaaccgg ctatatctcc aagatatgcc tgtgtaacaa 87840
agtggctgga tataagataa atatttaaat attaatacct ttctcccata tcagcaggaa 87900
ggcattagaa aacttaatga agcaattttt aaaattacaa caaaaagata aatcatctag 87960
gaataatctt aatggcaaat acttgagatt tatataaaga agacaaaagt ttactaagag 88020
atccaagtat tcattaaaaa gaacggaaaa aaaagatcca gattacatgc agctgtaaac 88080
taagtcaaac caagtattta agaagcatgt agaataaggc caggttctct tttgagcaaa 88140
tgctaagtta aatgcacaat ccattgatac aggtctaaaa taggtcagct gaagtgttct 88200
ttaatatacc tattaattct tatgttcttc cagtgaacta gaagtttata tgtcctgata 88260
ttgatagtga tatcaataac actatctctt tcctatataa ggtgtttttt ttctagagtc 88320
atgtatcaga agaaatcatt atatagctca gaaatatatg ttataaattg ttaatgtcat 88380
ctttttatac acaactgcca cttttaattg tcttcttaac agttcctttg gaagggctac 88440
gaagtcaaac ccagtttgag gagatgaggt caagctacat tagagagctc atcaaggcaa 88500
ttggtttgag gcaaaaagga gttgtgtcga gctcacagcg tttctatcaa cttacaaaac 88560
ttcttgataa cttgcatgat gtaagtattt ggttgattcc agaatatcaa tgattattct 88620
ctgaatttct ataacttttt aaatgttaca tgtaaatttt actttgtatg attttctcag 88680
attaatactt gtatgttaaa agtgtttgga tcatgctgct cagacttttt attgtgtttt 88740
tttttcattt tcaatataga ccactcaaat attcttttac aagtatttgt ataatgtgag 88800
ggtaatttaa tagctgatag aaattatggt acatttcctg aatatgttct aaaatatttg 88860
tgcctaaaat tataaaagat tttatatata cagtaaaaac aaaggtatgt atgtgaaaaa 88920
taagactcat gccattttca gtggaattat aaataacatt gctgatgggt gggcaatcag 88980

gcaatacata gcaaaagtta aaatttgcat gcttttcaag tttctatttt ttaggattta 89040
tcttaaagaa ataattagac aaatgtaata tacatgcaag gatgttcacc atagcagagg 89100
gggaaaaaag aaaagcaaac aagaaatgcc caacaccaga ggtggattac taagacaaga 89160
tatctgtggt ttgtcaagat atatgaaata caagctttta ttgctactat gttcagatca 89220
cattaaaatg acagtgaagg cataaaaaag ctatccactc tccaggtcaa cttttgaggt 89280
cttactcctg aatacgaaca gacagccaag attatgagct atttgagtaa agcctctaaa 89340
atgaaaggca gaaagcaaaa taaaaagaaa cttgaaggaa ataaacacca gtgcagagaa 89400
cagaagaaaa cttctaacat cctcagagaa ataagaatga tacggtatcc atgacatgag 89460
aacagaaaac atttttaaaa cagacattta gcaagaaaat acttttggat aattaaatga 89520
agaaaggcag aaattgaaaa ttcaatgaaa gggctgaaat ggaaaggaaa atctgaggaa 89580
aaataaaaga tcaaaacatg gaaaattaga aagaagagtt atagtaaatt tagtattcag 89640
gagattctag cctccaatta ataggaatgt ccagaagaaa aaatagagaa aatcaaggag 89700
agaaatttgt gtaagaaatc gcaaaagttg ctggaactga aacatacaag ttttctaaca 89760
aaaagggtcc ggtgtagagg cactgcacaa ttgaactaag gtacattatt gtaaaattgt 89820
accaggccag aaatgaagag aatattttaa agtataccag tgatgaaaga aacaggtcac 89880
atagacgtta tcaggattca gaatgatgtt ggacttctct gcagcaacac tggaagccag 89940
aagacagtgg agcagtgcct tcagaatttg atggaaaaga aaatcttcaa agaatttcat 90000
acctagccaa accatcaaac aagtacaatg gcagcatgaa agtattttcc aacttgcaag 90060
gtctcctaga gtttacctca gatacaactt tctcaagaac tgctacagga tgtcttataa 90120
caaaacatgg gattaagcca aagatccacc ccaacagaga tgcaaagtga tgtcccagaa 90180
caataactgt gcagagcagt agttagtcta gatgggagca gtaatacaaa gggttctagg 90240
ataccagctc caggaaaaat aaatggaact gagagattac cactaagtat gactgtaatt 90300
gaggtgattt ttacaattct gttgcagaat ttgactatgg ctttgagaca aatatagaga 90360
aaacaaagca aacaagaaca cgaggtgatt cacttttgca aaataaaaat aagtcgtaag 90420
aatggagatg caatccagca gaaaaccata aaaaaatctt tatgatataa atgctggata 90480
ttgactttag ctaaaaattg agccaaaagc tctctctggg gagaagagaa catgtatgta 90540
gttgaggaag agagtgtcta gtataagaga gcttcatttt cttcatttcc tatagtagaa 90600
actaaaacta actttagtgg aaatatagaa atatcaaaag acacaggcta caaaaagttg 90660
aaggtagttg tgtctaggga gctggaatag ggatgggaag aagtctatca ggaaactgct 90720
aggctctatt gatgctgctg ctgctgctgt ttttgttttt ataatcatgt ggtactactt 90780
gactttcaac attatacaca tgaattactt tgataaaaat taaacgatgt tggattattg 90840

gcataggaag aaccaatatg ttgttatata aaacagctag attagcaaat agaatttatg 90900

aaacatgtgt aatattgtgt gtatgcttgg gtatgtctgt atacatgtat aaaaagaaaa 90960

aaaaacttga gtaatacacc aaaatgttca cacagcttat cctgtagaat tgtgaataac 91020

tttcatttct ttaaataaat ctttccataa tatcagaatg ttctatcatg agtatataac 91080

attttacata ttattctaga aaatggtggg agttattttc actatggtaa gaaaaaaaca 91140

ccttgagaag tatttatatt ataaatagtt agaaaaataa attgccatgt ttgaatagca 91200

tatgaattta ttatttttat tacatgtttt ctactcattt gttaaaccaa cagcttgtca 91260

aacaacttca tctgtactgc ttgaatacat ttatccagtc ccgggcactg agtgttgaat 91320

ttccagaaat gatgtctgaa gttattgctg cacaattacc caagatattg gcagggatgg 91380

tgaaacccct tctctttcat aaaaagtgaa tgtcatcttt ttcttttaaa gaattaaatt 91440

ttgtggtatg tctttttgtt ttggtcagga ttatgaggtc ttgagttttt ataatgttct 91500

tctgaaagcc ttacatttat aacatcatag tgtgtaaatt taaaagaaaa attgtgaggt 91560

tctaattatt ttcttttata aagtataatt agaatgttta actgttttgt ttacccatat 91620

tttcttgaag aatttacaag attgaaaaag tactaaaatt gttaaagtaa actatcttat 91680

ccatattatt tcataccatg tagctgagga tttttaactt ttgcatctaa caaatcatcg 91740

acttaagaga aaaaatctta catgtaataa cacaaagcta ttatatgtta tttctaggta 91800

actccctttg tgtcaattat atttccaaaa atgaaccttt aaaatggtat gcaaaatttt 91860

gtctatatat atttgtgtga ggaggaaatt cataactttc ctcagatttt caaaagtatt 91920

tttaatgcaa aaaatgtaga aagagtttaa aaccactaaa atagattgat gttcttcaaa 91980

ctaggcaaaa caactcatat gttaagacca ttttccagat tggaaacaca aatctcttag 92040

gaagttaata agtagattca tatcattatg caaatagtat tgtgggtttt gtaggttttt 92100

aaaataacct tttttgggga gagaattgtc ctctaatgag gtattgcgag tgg 92153

<210> 71
<211> 20
<212> DNA
<213> Artificial

<220>
<223> Synthesis

<220>
<221> misc_signal
<222> (1)..(20)

<400> 71
tgtcgagctc acagcgtttc 20

<210> 72

<211> 27
<212> DNA
<213> Artificial

<220>
<223> Synthesis

<220>
<221> misc_signal
<222> (1)..(27)

<400> 72
tacagatgaa gttgtttgac aagatca 27

<210> 73
<211> 2360
<212> DNA
<213> homo sapiens

<400> 73
acagtgcgga gaccgcagcc ccggagcccg ggccagggtc cacctgtccc cgcagcgccg 60

gctcgcgccc tcctgccgca gccaccgagc cgccgtctag cgccccgacc tcgccaccat 120

gagagccctg ctggcgcgcc tgcttctctg cgtcctggtc gtgagcgact ccaaaggcag 180

caatgaactt catcaagttc catcgaactg tgactgtcta aatggaggaa catgtgtgtc 240

caacaagtac ttctccaaca ttcactggtg caactgccca aagaaattcg gagggcagca 300

ctgtgaaata gataagtcaa aaacctgcta tgaggggaat ggtcactttt accgaggaaa 360

ggccagcact gacaccatgg gccggccctg cctgccctgg aactctgcca ctgtccttca 420

gcaaacgtac catgcccaca gatctgatgc tcttcagctg ggcctgggga aacataatta 480

ctgcaggaac ccagacaacc ggaggcgacc ctggtgctat gtgcaggtgg gcctaaagcc 540

gcttgtccaa gagtgcatgg tgcatgactg cgcagatgga aaaaagccct cctctcctcc 600

agaagaatta aaatttcagt gtggccaaaa gactctgagg ccccgcttta agattattgg 660

gggagaattc accaccatcg agaaccagcc ctggtttgcg gccatctaca ggaggcaccg 720

ggggggctct gtcacctacg tgtgtggagg cagcctcatc agcccttgct gggtgatcag 780

cgccacacac tgcttcattg attacccaaa gaaggaggac tacatcgtct acctgggtcg 840

ctcaaggctt aactccaaca cgcaagggga gatgaagttt gaggtggaaa acctcatcct 900

acacaaggac tacagcgctg acacgcttgc tcaccacaac gacattgcct tgctgaagat 960

ccgttccaag gagggcaggt gtgcgcagcc atcccggact atacagacca tctgcctgcc 1020

ctcgatgtat aacgatcccc agtttggcac aagctgtgag atcactggct ttggaaaaga 1080

gaattctacc gactatctct atccggagca gctgaaaatg actgttgtga agctgatttc 1140

ccaccgggag tgtcagcagc cccactacta cggctctgaa gtcaccacca aaatgctgtg 1200

tgctgctgac ccacagtgga aaacagattc ctgccaggga gactcagggg gacccctcgt 1260

ctgttccctc caaggccgca tgactttgac tggaattgtg agctggggcc gtggatgtgc 1320

cctgaaggac aagccaggcg tctacacgag agtctcacac ttcttaccct ggatccgcag 1380

tcacaccaag gaagagaatg gcctggccct ctgagggtcc ccagggagga aacgggcacc 1440

acccgctttc ttgctggttg tcatttttgc agtagagtca tctccatcag ctgtaagaag 1500

agactgggaa gataggctct gcacagatgg atttgcctgt gccacccacc agggcgaacg 1560

acaatagctt taccctcagg cataggcctg ggtgctggct gcccagaccc ctctggccag 1620

gatggagggg tggtcctgac tcaacatgtt actgaccagc aacttgtctt tttctggact 1680

gaagcctgca ggagttaaaa agggcagggc atctcctgtg catgggtgaa gggagagcca 1740

gctcccccga cggtgggcat ttgtgaggcc catggttgag aaatgaataa tttcccaatt 1800

aggaagtgta acagctgagg tctcttgagg gagcttagcc aatgtgggag cagcggtttg 1860

gggagcagag acactaacga cttcagggca gggctctgat attccatgaa tgtatcagga 1920

aatatatatg tgtgtgtatg tttgcacact tgtgtgtggg ctgtgagtgt aagtgtgagt 1980

aagagctggt gtctgattgt taagtctaaa tatttcctta aactgtgtgg actgtgatgc 2040

cacacagagt ggtctttctg gagaggttat aggtcactcc tggggcctct tgggtccccc 2100

acgtgacagt gcctgggaat gtattattct gcagcatgac ctgtgaccag cactgtctca 2160

gtttcacttt cacatagatg tccctttctt ggccagttat cccttccttt tagcctagtt 2220

catccaatcc tcactgggtg gggtgaggac cactcctgta cactgaatat ttatatttca 2280

ctatttttat ttatattttt gtaattttaa ataaaagtga tcaataaaat gtgatttttc 2340

tgatgaaaaa aaaaaaaaaa 2360

<210> 74
<211> 6318
<212> DNA
<213> homo sapiens

<400> 74
gtccccgcag cgccggctcg cgccctcctg ccgcagccac cggtgagtgc cgcggtcctg 60

agatccccgg gccggatgcg cggcggcccc agctcccgag cgtctgcctc ccccgccctg 120

ggctgcccgg gctccctggg ctccccggcg gctgcacgga gtcaaggcgc cccgtcccgg 180

gcgtcccccg cgggtgccga tccaggctgc ccggagtccg gagcccagag aggagagaga 240

cagctgggga gcctggtcac cgcgggcatc tcccctgcgc tgcagtcgcc cgcctggcct 300

gccttcccgt tcctccgcct cttgccctga cttctccttc ctttgcagag ccgccgtcta 360

gcgccccgac ctcgccacca tgagagccct gctggcgcgc ctgcttctct gcgtcctggt 420

cgtgagcgac tccaaagtga gtgcgctctt gctttgactg atgctgccca aggacctctg 480

atcagcacca ggggagagga ggggctgctc agggagctgg ggtcctccgg attccatcca 540

cagcagggcc agactctccc caggaaatgg gacagggtgg cagcggaggc ttgagaacca 600
cgggggttgg cactggctgg caagggagga agaggccgcc gggactgccc cagcctgcgg 660
gcatctggta gatgaagctt gcttgggtca atccatttct cctggctgga aacccatggt 720
cttccatttg agaactagat acgaacaggg tgaggcgaga gggagaggga agagtgggtt 780
ttgggattgg ggccagttta ccctcaccct ggagtccctg gagcatggga cctttgatga 840
agcctcctcc cgaatctctt ccagggcagc aatgaacttc atcaagttcc atgtgagtat 900
ccacccctac aacagttggc tgcacagaca agttgggaag gcttcagggg acatcccctc 960
cctgccctct gctgcagggc tgcgccaccc cttaccactt ccactccccc tcgcttaccc 1020
cacctttgtt ctctccagcg aactgtgact gtctaaatgg aggaacatgt gtgtccaaca 1080
agtacttctc caacattcac tggtgcaact gcccaaagaa attcggaggg cagcactgtg 1140
aaataggtat ggggatctcc actgcaactg ggagagaaat ttggggacag ggagggatgg 1200
gtgggaggca agagcaggca ggagttagga gctggaggta gggtgggtga catcttcatc 1260
cctatgtgac aagcataaac acacacacac gctcacgaaa cagtggccac acaaatgtga 1320
ggtggggttg gaaggagacc ctgtccagtc ttctggcagg tctgaaacga catctttaaa 1380
atgtccgttg gcagccgggc atggtggctc acgcttgtaa tcccagcatt ttgagaggtc 1440
aaggtgagtg gatcatttga ggtcaggagt tcaagaccag cctggacaac atggtgtaac 1500
cctgcctcta ctaaaaatgc aaaaatcagc ctggcatggt agtggatgcc tgtagtccca 1560
gctacttggg aggctgaggc aggagaattg cttgaacctg ggaggcagag atctcagtga 1620
gctgagatca caccactgca ctccaactgg gcgacagagc aagactccat ctcaaaaaaa 1680
aaaaataaaa gttagttgga atgttcttct ctttctcata ttctctcatc ctcctgtccc 1740
cttgtagata agtcaaaaac ctgctatgag gggaatggtc acttttaccg aggaaaggcc 1800
agcactgaca ccatgggccg gccctgcctg ccctggaact ctgccactgt ccttcagcaa 1860
acgtaccatg cccacagatc tgatgctctt cagctgggcc tggggaaaca taattactgc 1920
aggtgaggtg ggggcaacaa ggaccaaaag ccctccctac agcttcccag aaaccttgtt 1980
accatcccct tctcccagag ggctggccat agcacaagag aagtgcggcc tctggttgag 2040
tcttccctga ggggaggagg cagggaaggc cctctgggtt ggaatgacat cccctatctt 2100
tctgtgttgc caggaaccca gacaaccgga ggcgaccctg gtgctatgtg caggtgggcc 2160
taaagctgct tgtccaagag tgcatggtgc atgactgcgc agatggtgag catcactgac 2220
ctgctgatga cagtggggtg gaaggggaca aacttacatg tccccttatt ccatcacagg 2280
aggactgagg aggtgggggg tgcccgagag ggatgctttc tcctacctgc ctccctaaga 2340
catccctctg tttgtcctcc aggaaaaaag ccctcctctc ctccagaaga attaaaattt 2400

cagtgtggcc aaaagactct gaggccccgc tttaagatta ttgggggaga attcaccacc 2460
atcgagaacc agccctggtt tgcggccatc tacaggaggc accggggggg ctctgtcacc 2520
tacgtgtgtg gaggcagcct catcagccct tgctgggtga tcagcgccac acactgcttc 2580
atgtacggcc ctgggtttct cctcttcgac tcttctgccc caccccaagc acatcccttt 2640
ctccttccca gcaaagtgtt ccgcctcatt tctccctcat ctgcccctgt ccatgcagcc 2700
catggccttg ggacaagtc gtgctttgag gcctctaggg agggaaggaa gaagtggcag 2760
atttcatggg actaagctgt ttgatgggta tcttctccca cagtgattac ccaaagaagg 2820
aggactacat cgtctacctg ggtcgctcaa ggcttaactc caacacgcaa ggggagatga 2880
agtttgaggt ggaaaacctc atcctacaca aggactacag cgctgacacg cttgctcacc 2940
acaacgacat tggtgagggg gaaccccgcg actactgtgg ccataatggc ttggggagag 3000
tgggacccag ggagagactg gagctgaggt tgaagctgcc cggtggggca ggggtggggc 3060
gagggacctt gaagcctcga tatacatgac aaagggagtg gcagggaaga gttccatgaa 3120
gtctgagggg cctggtgctc ctctggagag accctgaatt tccccaacaa gtagcctctt 3180
gcgagtggaa acagccctgt gggtatatgg cttgggctgg gaaggccctg tttatatgaa 3240
ttagaaaaag acacaccttc ctttgtggga tgcagcctct gtctgtgcta ggatatagaa 3300
cttggagaat ggagccttgg gatggattcc agcctaacta cctcagctgg gagtttttgc 3360
agaaacgacc tgtacagctg tatgcagtgg ctctggccat ccaagccttt ttcaacacct 3420
ggaacaaagc ccttggggca tggggcaggg gaggtttcca ggtgataagc gaccagcaga 3480
cctccctgga tgactgacct agggataggc atagctactt cctcggcact tggaggggac 3540
agatggggac cgcctaacca gtagtgatct ttctcctctg accctctgtc ctcccccagc 3600
cttgctgaag atccgttcca aggagggcag gtgtgcgcag ccatcccgga ctatacagac 3660
catctgcctg ccctcgatgt ataacgatcc ccagtttggc acaagctgtg agatcactgg 3720
ctttggaaaa gagaattcta gtaagtgaca attgcgactg acttagaagg tcctgaggag 3780
tgttttgacc tgaaaatgag cccagcgtga tcaagggaag actgcagagt tagaggtggg 3840
agcactgagg cggtggcaga tgggtccagg gatggatgaa gagtgttgtt tagggagcga 3900
tgggctgcaa aggtaaatag atggtagggg ctataggtgg agtaaaggct cagatttgca 3960
tggaagagaa taagggcctt ccctggtaga gatactttat ggttcccctc tctggcagac 4020
tcccagtgga cagataaatc ttgatgcaaa cgcctccctg ttttctccac ctagccgact 4080
atctctatcc ggagcagctg aaaatgactg ttgtgaagct gatttcccac cgggagtgtc 4140
agcagcccca ctactacggc tctgaagtca ccaccaaaat gctgtgtgct gctgacccac 4200
agtggaaaac agattcctgc caggtgagtg ttccaagcat ctctctccac ctcttccata 4260
tctccccaga gctcctgggc ttgttccagc cagcttaagg gtgtctctct ctagccaaag 4320

ccctaagtag ccagaatcag gagctcaggt ctttgagggt ttaaaccagt ccttatgtgt 4380
ttgccagaca ttaccaaaaa aatcccagct ctgcgctagt cacttcagac tgggggcacg 4440
agatcctaga aagaggaaac agtaaaagac aatgtaactc agtgcccagg gtgtgttgtg 4500
aactataaat gatcaggtgt tcaggagagg gaggtgagtg ccaacctgag ggtcagggag 4560
gggaggcttt aaaggaaatg tgacttgata ggcatttgaa gaggcagagg gaagaaagga 4620
aggtgtttca gttgaaagat acaaaactga gaaggaggct ggcatattcc gggtggggag 4680
gagaactagg gtctgggagt gtggatggaa tagtggcaga tgacagggct tttaaagcca 4740
agcaggggat tttaaacttg atgtggtaga aaatggggct gcgtcaggca cagtggctca 4800
tgcctgtaat cccagcactt tgggaggccg aggtggatgg atcacttgag gccaggagtt 4860
tgagaccggc ctggccaaca tggtgaaacc ctgtgtctac taaaaatgca aaaaaaaatt 4920
agccaggtgt ggtggtgcct gcctgtaatc ccagctaatc aggaggctga gacatgggaa 4980
tcgcttgagc acaggaggca agtttgcagt gagctgagat cacgtcattg cacgccagcc 5040
tgggcgacag agcgagattc tgtcctcccc ccgaaaaaaa gaaagaaaat gggaagtcgc 5100
taaggacttt gactgggaaa ctcttccctc tctctggtat ggttgggtga tgggatcaga 5160
aatcccctcc tcacttctct aggggctcatc ttttgtatct ttggcgtcac agggagactc 5220
agggggaccc ctcgtctgtt ccctccaagg ccgcatgact ttgactggaa ttgtgagctg 5280
gggccgtgga tgtgccctga aggacaagcc aggcgtctac acgagagtct cacacttctt 5340
accctggatc cgcagtcaca ccaaggaaga gaatggcctg gccctctgag ggtccccagg 5400
gaggaaacgg gcaccacccg ctttcttgct ggttgtcatt tttgcagtag agtcatctcc 5460
atcagctgta agaagagact gggaagatag gctctgcaca gatggatttg cctgtgccac 5520
ccaccagggc gaacgacaat agctttaccc tcaggcatag gcctgggtgc tggctgccca 5580
gacccctctg gccaggatgg aggggtggtc ctgactcaac atgttactga ccagcaactt 5640
gtctttttct ggactgaagc ctgcaggagt taaaaagggc agggcatctc ctgtgcatgg 5700
gtgaagggag agccagctcc cccgacggtg ggcatttgtg aggcccatgg ttgagaaatg 5760
aataatttcc caattaggaa gtgtaacagc tgaggtctct tgagggagct tagccaatgt 5820
gggagcagcg gtttggggag cagagacact aacgacttca gggcagggct ctgatattcc 5880
atgaatgtat caggaaatat atatgtgtgt gtatgtttgc acacttgtgt gtgggctgtg 5940
agtgtaagtg tgagtaagag ctggtgtctg attgttaagt ctaaatattt ccttaaactg 6000
tgtggactgt gatgccacac agagtggtct ttctggagag gttataggtc actcctgggg 6060
cctcttgggt cccccacgtg acagtgcctg ggaatgtatt attctgcagc atgacctgtg 6120
accagcactg tctcagtttc actttcacat agatgtccct ttcttggcca gttatccctt 6180

ccttttagcc tagttcatcc aatcctcact gggtggggtg aggaccactc ctgtacactg 6240

aatatttata tttcactatt tttatttata tttttgtaat tttaaataaa agtgatcaat 6300

aaaatgtgat ttttctga 6318

<210> 75
<211> 24
<212> DNA
<213> Artificial

<220>
<223> Synthesis

<220>
<221> misc_signal
<222> (1)..(24)

<400> 75
ggaaaacctc atcctacaca agga 24

<210> 76
<211> 21
<212> DNA
<213> Artificial

<220>
<223> Synthesis

<220>
<221> misc_signal
<222> (1)..(21)

<400> 76
acggatcttc agcaaggcaa t 21

<210> 77
<211> 1548
<212> DNA
<213> homo sapiens

<400> 77
cagggccgag ccagcccctt caccaccagc cggccgcgcc ccgggaaggg aagtttgtgg 60

cggaggaggt tcgtacggga ggagggggag gcgcccacgc atctggggct gactcgctct 120

ttcgcaaaac gtctgggagg agtccctggg gccacaaaac tgcctccttc ctgaggccag 180

aaggagagaa gacgtgcagg gaccccgcgc acaggagctg ccctcgcgac atgggtcacc 240

cgccgctgct gccgctgctg ctgctgctcc acacctgcgt cccagcctct tggggcctgc 300

ggtgcatgca gtgtaagacc aacggggatt gccgtgtgga agagtgcgcc ctgggacagg 360

acctctgcag gaccacgatc gtgcgcttgt gggaagaagg agaagagctg gagctggtgg 420

agaaaagctg tacccactca gagaagacca acaggaccct gagctatcgg actggcttga 480

agatcaccag ccttaccgag gttgtgtgtg ggttagactt gtgcaaccag ggcaactctg 540

gccgggctgt cacctattcc cgaagccgtt acctcgaatg catttcctgt ggctcatcag 600

acatgagctg tgagaggggc cggcaccaga gcctgcagtg ccgcagccct gaagaacagt 660

gcctggatgt ggtgacccac tggatccagg aaggtgaaga agggcgtcca aaggatgacc 720

gccacctccg tggctgtggc taccttcccg gctgcccggg ctccaatggt ttccacaaca 780

acgacacctt ccacttcctg aaatgctgca acaccaccaa atgcaacgag ggcccaatcc 840

tggagcttga aaatctgccg cagaatggcc gccagtgtta cagctgcaag gggaacagca 900

cccatggatg ctcctctgaa gagactttcc tcattgactg ccgaggcccc atgaatcaat 960

gtctggtagc caccggcact cacgaaccga aaaaccaaag ctatatggta agaggctgtg 1020

caaccgcctc aatgtgccaa catgcccacc tgggtgacgc cttcagcatg aaccacattg 1080

atgtctcctg ctgtactaaa agtggctgta accacccaga cctggatgtc cagtaccgca 1140

gtggggctgc tcctcagcct ggccctgccc atctcagcct caccatcacc ctgctaatga 1200

ctgccagact gtggggaggc actctcctct ggacctaaac ctgaaatccc cctctctgcc 1260

ctggctggat ccgggggacc cctttgccct tccctcggct cccagcccta cagacttgct 1320

gtgtgacctc aggccagtgt gccgacctct ctgggcctca gttttcccag ctatgaaaac 1380

agctatctca caaagttgtg tgaagcagaa gagaaaagct ggaggaaggc cgtgggccaa 1440

tgggagagct cttgttatta ttaatattgt tgccgctgtt gtgttgttgt tattaattaa 1500

tattcatatt atttatttta tacttacata aagattttgt accagtgg 1548

<210> 78
<211> 21968
<212> DNA
<213> homo sapiens

<400> 78
cagtatccct cctgacaaaa ctaacaaaaa tcctgttagc caaataatca gccacattca 60

tatttaccgt caaagttttt atcctcattt tacagcagtg gagagcgatt gccccgggtc 120

ccacgttagg aagagagaga actgggattt gcacccaggc aatctgggga cagagctgtg 180

atcacaactc catgagtcag ggccgagcca gccccttcac caccagccgg ccgcgccccg 240

ggaagggaag tttgtggcgg aggaggttcg tacgggagga gggggaggcg cccacgcatc 300

tggggctgac tcgctctttc gcaaaacgtc tgggaggagt ccctggggcc acaaaactgc 360

ctccttcctg aggccagaag gagagaagac gtgcagggac cccgcgcaca ggagctgccc 420

tcgcgacatg ggtcacccgc cgctgctgcc gctgctgctg ctgctccaca cctgcgtccc 480

aggtaggggc tgggtcccga acgcctgcgc ctggaacagg gtctaattga gggggttggg 540

ggttgtcaga ggatgagttg gaggaatgcg gttcagtcct cagcatcctc cctaatcaaa 600

taatagtaat tctcgtgctt tgtgcaacgc cacgcggcgc agtacctggc actcagtagc 660
taaggaaata ttagtggagc aaaggcattt agctttacat aatttagtga gtgctttttt 720
tttttttttt ttttttttga cagagtctca ctctgtcgcc caggctggag tgcagtggcg 780
cgatctcggc tcactgcaac ctctgccgcc cgggttcaag cgattctcct gcctcagcct 840
cctgggtagc tgggattaca ggcgcctgcc atcgcgcccg gctaatttct gtatttttag 900
tggagaccgg gtttcactct gttagccagg atggtctcaa tctcctgacc tcagatgatc 960
cacctgcctc ggcctcccaa actgctggga ttacaggcgt gagccaccgc gcccggccca 1020
agtgttcttt ttttaaatgg agttcttcga agcctcttcc ttgcaatttc aaactaggcg 1080
atgggacttt attaatttcg tttcgcagag gaaactaggg cacagagagg ttagataact 1140
ggcctaaaat cacacagcca gtgcttgaat acgcaggatc tgaccctgca gcgcccccca 1200
gcgctctcca cgctgctggg tctccccctc tgagaaacgg ggggacagga ccccctttta 1260
caaaaggccc aaagggaggc tgactgagcg gcgcagagcc agtgctggag acccgggact 1320
gtccctcagg acctttccct ctcactgagg cgactctcac ttacttcccc ggaaaatgtg 1380
gggggctctg ggtcgaggaa ttcgagaagg aactgagtca gggcgggtgg ccacagggtg 1440
ttggggccgc gatgaataac ccggaaagcg ctcgagaccg cgggaggccg ggaatgagta 1500
acagctccgg gatactccga acgcgcagct ggaaagggat gtccgggaag gcccggaggt 1560
cggggaccgg gcctagggac tgggctgcaa tctcggggcg gagcctgggg cggggagaga 1620
gtgtcgggga ggagccagag ggcggggctg gaacctcaag gaagagctac gggagaggtt 1680
acagaccgag gaagagctag gagcggggct agaacctcga ggcggagcca gagggcgggg 1740
ttataacctc gaggcgtaac cagagggcgg agttataacc tcgggaggag ctagaaggtg 1800
ggactagact cttgaggggc aggattataa cctcggggag gagctagagg gcggggctgg 1860
aacctcaagg aggggctagg ggcggggtta taagcttggg gaggagctac ggggctgggc 1920
taggacctca aggggccaag gggcgggggt tacgacctcg gggaggagct acagggtggg 1980
gctgaaacct caagaaggga ctggggcgga gttatgacct caggggaaaa ctaggggcgg 2040
ggagagctaa agggcgaagt tagaatctca aggaggagct agagggaggg actaaagcct 2100
cgaggaggag ctaggggcgg ggttatggca tcggggagga gctaggggc gggaagataa 2160
ggtcaggaag agctaaaggg cggggctaga acctcgacga tgagtctgga gcggggccaa 2220
aacctagttg gagagctgga gggcggagac agaacttcgc gggaaactag agtatctaac 2280
aatagaaact ccggagggct gatggggccg ggcctagaat ttgggaataa attagtgggc 2340
cgggagaggc tctggagccg ggtagaacca gggggagtga gtggagggat agttccatta 2400
gggcactggg agtgacggta taacataaag atcgacgcgg gtggggcaag gctagaacgt 2460
ccccagcaga actggagagg cgtaatcgac cgagggccgg tgcggtgaga aagacctaat 2520

aggagcagga atagaacgta ctgatagaga gggcggggat acgactgtca ggatatacgc 2580
ctcacgagga cagaatgaaa ggaaaaacgg gccaaggcag gactttggga aaggacttgt 2640
gggcagggat agaacgttca gttagtgggg tggaggtaga acgtggacaa cggacagact 2700
ggaagtaccg ccggccggaa cccagcagaa catggacacg aatctgaatg gacggggcct 2760
ggagacttgg tgttggtatt gggacatgca ggggtgagcg gggtcttgg agctaagcgt 2820
agttaacctc tcctctcctc tccctccttc ccccagcctc ttggggcctg cggtgcatgc 2880
agtgtaagac caacggggat tgccgtgtgg aagagtgcgc cctgggacag gacctctgca 2940
ggaccacgat cgtgcgcttg tgggaaggtg agcttccccc caacccacac acccctccag 3000
cgcagcatgg aacgcgagtg attgagaaac cttcctcacc agtaactggg acatgcaaac 3060
taaaaacaaa caaacaagga gagcccattt cacacccacc agattggcaa gcatttaaaa 3120
atctgacaat acccgggtta gtaaggatgt ggagttgcag accgtgtctg tggtggggga 3180
gtaaattggc acatcaacca cttagagcga ttcggcaata tctagtccag atgaagatta 3240
acatatgcta cagctcaact cctcgccatg cagaaatgcc aaagaacctt ggccacatat 3300
acccagggac acatgtatac gaatgttctt agcagcaatg tttgtgacat ggaaaattgg 3360
aaacaggctg ggcactggct cacacctgta tttccagcac tttgggaggc agaggcggat 3420
gaattgcttg aggtcaggag ttcaagacca ggctggccaa catggtgaaa cacccgtctc 3480
cacacacaca caaaaaagga aaaattagcc gagtgtggtg gcacgcacct gtaatctcag 3540
ctacttggga ggctgagaca ggagaattgc ttgaacctgg gaggtggagg ttgcagtgag 3600
ctgagatcat gctactgcac tccagcctgg gcgacagagc gagatcctgt caaaaaaatg 3660
aacgaaagag agaaaggtgg gccgggcatg gtggctcaca cctgtaatcc cagcactttg 3720
ggaggctgag gcaggtggat tacctgaggt caggagttca ggaccagcct ggccaacatg 3780
gtgaaaccct gtctctacta aaaaatacaa aaattagccg ggcttggtgg caggcaccag 3840
ttatcccagc tactcgggag gctgagacag gagaattgtt tgaacccagg aggcggaggt 3900
tgcagtgagc tgagattgtg ccattgcact ccagcctggg cgacaagagc aaaactccat 3960
ctcaagaaag aaagaaagaa aggaaaggaa gagagagaga gagaaagaga gagaggaagg 4020
gagggaggga cggagagagg cagggaggga gggagggagg agggaggaag gaaggggaga 4080
aagaaagaaa agaagaaaat ggaaacgact ttatgtcagt cagcaagtca gcagcagaag 4140
agaaacactg tggtaataat tacataatgg agcgctctac tgcagcttaa agaaggaaat 4200
agagctgcag ataccaatgt ggacacatct cagaaaccta aggttgagag ggaaaaaaat 4260
gttgcagaat atgatacgtt ttttataagc tttaagaaca catgaaacaa cagtatatga 4320
agccaagcac agtagctcac atttgtaccc ctagctactc aggaggctaa ggcagaagga 4380

ttccttgagc ccagaagttc gaggttgtgg tgagctatga ttgccccagc tgggacaaca 4440
gagaccctgt ttctaataaa taaataaata aaataaagtt acatttgagg catctcagta 4500
ctgagatcac taagctgagg gtgtggggga gggatagatg gatccagaaa gtcccagttg 4560
tagtgctgtc actacaagag caggagtggg acacagtggc tcacacctgt aattccagca 4620
ctttgggagg ccaaggtggg aggatcactt gaggccagga gtttgagatc agcctgggca 4680
acatagtgag atgccatctg tataaaaaat ttaaaaatta gcagggtgtg gtgatgtaag 4740
cctgtggttc cagctactcg ggaggccgaa gcaggactgc ttgagcctgg gaggtcaagg 4800
ctccagtgag ctatgattgc accactccag cctgggtgac agagcaagac cctgtctccc 4860
ggaaaaaaaa attaattaat taattaattg aaataaaata gagcaggctc cttgttgggg 4920
tgggggtgga ggcaaggtta actctagaaa aagacagagt tcgactcaaa taacagaaga 4980
ggcctagcag agatcagccc tgggagtgga tgaccttgag accaggagtg tcagaggctg 5040
gttgagctga gaggagcctc tggggtaatg aagatcccct ccttgcagaa ggagaagagc 5100
tggagctggt ggagaaaagc tgtacccact cagagaagac caacaggacc ctgagctatc 5160
ggactggctt gaagatcacc agccttaccg aggttgtgtg tgggttagac ttgtgcaacc 5220
agggcaactc tggtgagtag ggcagccctt gccatcccca accccaagcc atccccagct 5280
caacctcatc atcatcccaa tcctattctt aactttacat cagctgaacc ccaactccat 5340
gccttatcca agtcccaacc ccaacccaac ccatctctag ctcagctcca tccccaactc 5400
attcttgacc ccatatccaa ccccaactca acttcacctc caatcctatg cccaacttca 5460
tgttacccta acactgttcc caactcaaac ccagccctgt tccaagcaca gttccaaccc 5520
catccccaaa tcaaccccat ccttatctca gactacccaa atccccaccc caacgacaca 5580
attactttca tccttaaccc aatccccatc ctgcatgttt gtatgtatgg ttgttatgta 5640
taacttgcaa ctctgccctt aacaagctca cacgcatgca tgagacatag atgagatgtc 5700
tcagtgatgt ttgtccatct gactgggcag gagatggtcc tgcccaatcc attgggacgg 5760
gagatggtcc aagcaataga tactgagggg ccaggattca agcaaagact tgacgttctt 5820
aagggaagtc gaaagagggg aagattaaat gggccgcagt aggctgggag aatcctctgc 5880
aatagatggt ctctgacttg ggcagtgaaa aaacaaatgt gaggaaatgt ccataatagg 5940
ctgggtgtgg tggctcatgc ctgtaatcca agcactttgg gagaccaagg cgggagaacc 6000
gcttgagccc aagagtttaa gaccagcctg ggcaacaacc ataagcccct ttgtcgaatt 6060
tcatggcttc aggctctgat cattttaaga aaaagactta gaaaaggtca aattttccta 6120
aattcccaca ggagaggaag ggtggggaag gaatgggtcc atggtgcatg gatccatccg 6180
tgaaagagat tcctctttct ggacctggtg aggaaggaaa tagggtaagg ccagtctagt 6240
catggttggt gacatcagtg agcagagtgc tgcgtttgca ctgggttttc tttgttcttc 6300

attcatcctg attctgcctg gttttcatcc atcctgacct caaacactaa tccgctggtg 6360
gggccaaatc agatgtttgt tttgaggaag tgacgagagg atgtgggctt cccgtctcgg 6420
tgagatgtgc ttaaggaaag caccaatact tgggtctaag tagtgcattc aatcagagat 6480
gatagcgaaa atatttaaca atattggtca gtgctgggac aaactcctgg agtccgctgc 6540
tgggcctggt gttgcccctt tccatgatgg gtcacaggga catgaagggt cctggggaga 6600
gagttggcag tggtcaggga tccaccagga gcttagagca gcagctatca attcaggaat 6660
ctaataactg agatacctat atcatccctt tttcttttct tttttctttc tttttttttt 6720
tgtttccttt tgtttttgtt tttttttttt gtttgtttgt tcgtttgaga tggagtttca 6780
ctcttcttgc ccaggctgga gtgcaatgcc acgatctcgg ctctctgcaa cctccgcctc 6840
ctaggctcga gcgattcttc tgcctcagcc tcccaagtag ctgggattac aggcatgcgc 6900
caccacgccc agctaatttt gtatttttag tagagatgag gtttctccat gttggtcaag 6960
ctgatcttga actcctgacc tcaggtgatc tgcccgcctc agcctcccaa agtgctggga 7020
ttacagacgt gagccaccgt gcccggcctc atccccattt ctaatgccat tgtaccctgc 7080
ttgtggcaac ttgcatagtt ggttaaactg cactcagtac ttacatgatg ctgggaacac 7140
agcagggacc aagacagcct tgtgccctgc ctgactccat gggactcaca gtcctgtgtg 7200
tgtgtgtgtg tgtgtgtgtg tgtgtgtgtg tgtgtgtgtg tgtgtgtagg ggtgacagac 7260
ccatccatca ccagacagtg actgattatt aggttaatgc aaaagtaatt gtggcttttg 7320
ccattaaaag caatggcaga aaccacaatt acttttgcac caacctaata gatttgtctg 7380
ggctatgaag ggggaaacac aggcagaggg gtcagggctg ggatgaggga agagtaggag 7440
ctgtgggacc ctagaggaga tacctgactc aggtcaggga aggtttccta ggggaagcaa 7500
cagctaagct ggatcttgaa ggatgagtag gaatcagact attaaacagc taatggaggc 7560
tgggtgcagt ggctcacgcc tgtaatccca acactttggg aggcccaggt gggaggattg 7620
tttcagacca gcctaggcaa catgactcta caaaaaaatt aaaaaattag ccagatgtga 7680
tggtacatgc ctgtgatccc agctccttgg gaggttgagg cgggaggatc actatagccc 7740
aggtggtgga ggctgcagta agctatgatc gcaccactgt accccagcct gggtgacaga 7800
gcgagatcct gcaaaaaaaa aaaaaaacag tggaaagggt tttccagcag agggagcagc 7860
agatacaaag gtggccaaaa gcagtgtggc tggacaaagg aaacagtaca gtggggcgag 7920
atgagctgga aagcttggca ggggcatgtt acacaggggt ttctagacat tgtcctggga 7980
gcactggggc atcacagaag attttaaaca gcgaatgtca tgatcgcaat tatttattta 8040
tttatttttg agatggagtt tcgctctttt tgcccaggct ggagtgcaat ggtgcgatct 8100
cagctcactg caacctccgc ctcctaggtt caagtgattc tcctgcctca gcctcccaag 8160

aaactgagat tacaggcacc cgccaccatg cccggctaat ttttttgaat ttttagtaga 8220
gatggggttt caccatgttg gccaggctgg tctcgaactc ttgacctcgt gatccaccca 8280
cctcggcctc ccaaagtgct gggattacag acgtgagcca ccgcgcccgg cccccattta 8340
tatttttaac aaattattct gacctcagga cagaagatag actgaaagga agcaagagta 8400
gatgagggga aaccaacttg gggtgggcg aggttgtcca ggtggagaag agggcagcag 8460
gactagggtg tcaggtgtgc agaggaagaa aagtggatag atttaagagc tattcaggct 8520
aggcacagtg gctcacgcct gtaatcccag cactttggaa ggccaaggca ggaggactgc 8580
ttgatcccag gagttggagg ctgcagtgat cacgccactg cactccagcc taggcggcag 8640
agcaaaaccc tgtctcaacc aaagccgaac aagagctatt aagaagcagg ccttagtggt 8700
ggattagatg tgggacggga cctgcaattc tggtcatgat ggagtcactt gtatcagacc 8760
aacccttctg ccaatgacag ttgcaaatgc tggacaaata ataaacaatt attggaagtc 8820
actggagagc agcaaatgaa aggagaaact tggctgggca aagtggttca tgcctgtaat 8880
cccagcactt tgggaggcca agacaggtga attgctcgag ctcatgagtt caagaccagc 8940
ctgggcaaca tggcgaaacc ccgtctctac taaaaataca aaagttagcc aggcgtggtg 9000
gcacatgcct gtggtcccag ctacttgggt ggctgaggca caagaattgc ttgaacccgg 9060
gaggtagagg ttacagtgag ccaagatcac acgactgcac tccagcctgg gcgacagcgc 9120
gagactccat ttcaaaaaaa aaaaaaagaa aaaagaaaga aaggagaaac ttgaggcgct 9180
acaatctctg aaataacaga agtgtagaat ttgggttcca cagacaacct ggctttttcc 9240
cggagcgcac ttgccaatct gctgctgtgg aagggaaata gagcccagac ggaaagagag 9300
tcgctagact gagaagacgg aggttggagt ttggggctgc cagaatgtct ggaagatgag 9360
agttaaaatc ctggaatgga tgggaccaca gaaaagggag ttcccaacct gcaaacaaat 9420
ctccctcaag tcattggcaa gctctacatg cacagggtga gagtccaaga aacttggaag 9480
agaaaaatag caactggaa gctacaaaag cgaacaaggc ttccagcaga catgtgatgc 9540
tggggacaca ggttgccaag ctgaagaggc cttggtaagc atctcaggct ttccccctaa 9600
gaccacccca ggggtaaggg ccaaagtgaa atagaccagc cctaaacaag cctaggtcca 9660
gtccttgaca ggaatgaaaa cctgactctc tgtggaagaa gaaaacatcg tttaggccct 9720
tgacagattt ttatctagaa tgtctggcgt ctgatcaaaa tttacaagat agctaggcat 9780
ggtggcatgc gtctgtagtc ccaactactt ggaaggattt cttgagcctg agagttctgg 9840
gctgtagtgt gctatatcaa ccgggtgtct gcactaagtt tggcatcaat atggtgatct 9900
cccaggagca ggggaccacc aggttgtcta aggaggggtt gaagcagccc aggtcagaaa 9960
tggagcaggt caaaactcct gtgatgatca gtagcaggat tatgcctatg aatagccact 10020
gcacaccagc ctggacaaca tagcaagacc ccatctttaa aaaaaaaaaa aaccaggccg 10080

ggcatggtgg ctcaagcctg taatcccagc actttgggag gccgaggtgg gaggattaca 10140
aggtcaggag ttcatgacca cactggccaa catggtgaaa cccccttctct actaaagata 10200
caaaaaaaaa aaaaattagc cgggtatggt ggcacacacc tgtaatacca gctacttggg 10260
agactgaggc aggagaattg cttgaaccag ggaggtggag gttgcagtga gccgagatca 10320
caccattgca ctccagcctg agcgagaggg caagactctg tctcaaaaca aaacaaaaca 10380
aaacaaaaca aaacgcaaga ctctgtctca aaacaaaaca aaacaaaaaa caaaaaataa 10440
aaaacagtaa caaaaaacct tacaaggtat cctgaagaaa ataaaaggga ccaattgacc 10500
agaaaccaag agaaaaatcc aactgtagaa acagaattgt agcaactcag atattggatt 10560
ataggacagg aaacatgaaa attgctacaa tcgatatatt caggaaaaca gaagaaagga 10620
tatgttttag cacctggaat ccattttttta aaagagtcca acagaaaata tagaacagtg 10680
actcaaatga agaactcggc agttatgctt gatagaggtc agaaacagca gagcaaaagg 10740
attaaggagc tagaagacag aacagtagaa gcatgtcggt tgatgcacag atgtggaaga 10800
taagggggtc tgccaggttt ctgcccccggg gactggatgg ggccgccttg gacgcgtgga 10860
cttcaagaaa aagagcagtt gtgggagaag atggggagtt ttgtttagga caggtgtttg 10920
aggtatagcc atggggcctc gaagagacag ttgcgtgtct ggagctcaga gagaagtcta 10980
gactagagag agatttgtga gtgatgagca gatagatgga aatgaagtca tgtcaatatt 11040
taccccatga gagagtctat actgtgagag ataaaaaggg cttaaaacag agccctggcc 11100
aggtgcggta ctcacgtctg taatcccagc actttaggag gccgaagcgg caggatcatt 11160
tgagtccagg agttcgagac tagcctgggt aacatagcaa gaccttgtct ctacaaaaat 11220
acaggaatca gccaggtgaa ctggcacatg cctgtagtct cagctactca gtaggctgag 11280
atgggagaat cacctgagct cagggaggtc gaggtggcag tgagccactg cactccagcc 11340
ttgggacaga gcaagaccct atctcaaaaa aaaaaaaaaa gaaaagaaaa gaaaaaggaa 11400
aatagcccta aggatcattg gcatttacaa gagttcacac cagagaccca tgagtgacca 11460
cccagagtgg gcagaactag aaatggggaa ttcaaggggc agtgggagct cagagcaggt 11520
acccgttcca ggctgatggg tcagggaggg cttcctagag gaggtgaaat tgaagatgac 11580
agctgaagaa tgaataggag ttagagaaag tagggctcaa ggaaaaggtg ttctaggtat 11640
gaacagctat gttggcaatg gtgagaattt gtgttttcat tttcatggaa gtttgtgtga 11700
gaagacgtgg gtggccttgt agttattttt ctttgagggg gacgattggg ctttggatgt 11760
aaatgattcg cctaattaca gctagcggcc tgccagttga gaaccttggc tgacatggtc 11820
ctctcacttt tttaagactt ggcctggact cagcttagat gtgtcttggg tcaggacggg 11880
gagggcatgc caagcatggg aaatggtgtg agccaagggc tgggtagagg aatgactctg 11940

gtgtttccag ggaaaagtaa cggatccagt tggccaacac cacaaggttc gtgcaaaagg 12000
cgaagtgatc aggaatcagt caagtgagtc tttgatccct gggacaatta ggtagtgtat 12060
tatctattgc ttatctatta tctattgctg cattacagat tgcagcaata aaataagcat 12120
gtgttatttc acaaagtttc tgaaggtcaa aaaccctgga gtggcttagc taggtggttc 12180
tggctcagga tccttcatga gcctgcagtc gagatgtcag ctggggctgc agtcatctga 12240
aggctcctaa ggtagcttca ctcacatgaa tgctcccctc ccatggcttt ggcacaggcc 12300
tcagttctgc cacgtgaact ttcccatggt gctacttgag tgtcctcaca acatggctgc 12360
tgcttgcccc agaaaaagtg atccaagtga gagagccagg catgtgttct atgatctagc 12420
cacagaaggc atacaccatc atttccataa tatccagttg actacatagg gcatccttat 12480
tcttttcttc ttcttttttt tttttaattt aaagacagag tcttgctatg ttgcccagac 12540
tagtctcaaa ctcccagact caagcaatcc tcccacctcg gcctcccaaa atgctgggat 12600
tacaggcatg agctgctgca cccagccagc cctattcttt aagggcatga ataccaggga 12660
gcagagatta ttgggggcca ttttggagac aggctaccac agggaacaat agaagggttt 12720
taagtaattg ctcattcatt cattcattta acaaatatgc attgaatatc ttatctgtcc 12780
ctggccatgt gccaggtggt gttgggacac agtagtgatc aagacagccc ctggccctgc 12840
cctcctgggg gctcacagtc cagtagagga gacatacctg tcaccagaca gtgaccaccc 12900
agagtgggat gggggagtac aggggtgtgt gggagcctgg tggatcagga agggcttcct 12960
agaggaggtg aaattagagc tgacacctga agaatgagta ggagttaggg aaggcagaga 13020
gcaaaggaaa ggtgttctag acagaaacaa cggcatatgc aaaggcctgg atgccaggga 13080
acatgtgtcg tcgcagactg tgggaagtca gtgtggagca gagtctcagg gtgtagtgga 13140
gggagacgag gcaggagagg tgctcaggcc ccagtatttg ataggctttg aatcccatgc 13200
tgaggatctt ggaatttatt ttcagggcag tagggagcta tggaagagtc ttgagcagag 13260
gagagacagg gtcagatatg agtattagaa atatccttct aagggcccga gtgcagtggc 13320
tcatgcctgt aatcccagca ctttgggagg ccaaggcatg tagatcactt gaggttagga 13380
gttcgagacc agcctggcca acatggtgaa accctgtctc tactaaaaat acaaaaatta 13440
gcccagcgtg atggtgcacg cctgtagtcc cagctattcg ggaggctgag gcaggagaat 13500
cacttgaacc cggggagtgg aggttgcagt gagccaagat ggtgccactg cgttccagcc 13560
tgggtgacgg agtgagactc tgtctcggga aaaaaaaaaa aaaaaaggaa agaaatgttt 13620
ctagggccat gtggagatgc tgcactgggg tgagtagggg gtgacaagaa tggaaaccca 13680
gaggctaggg aggtagctgg agttgaagga ggtctgagct ggactggtcc atggggctgg 13740
aaaggagggg gtggattcta gaaagattca gcaggcagaa taggcagggc tcaatgactg 13800
gctgcagggt gggataagga ggaattgatt ttgagtgagt ccttgcagag ctggggctcc 13860

aggactgtcc ccatggagtc tcactcccct ccccctcttc caaacaggcc gggctgtcac 13920
ctattcccga agccgttacc tcgaatgcat ttcctgtggc tcatcagaca tgagctgtga 13980
gaggggccgg caccagagcc tgcagtgccg cagccctgaa gaacagtgcc tggatgtggt 14040
gacccactgg atccaggaag gtgaagaagg tgagccccaa cctgctggca actcctcctc 14100
cctgctctgc tcctccctaa gactgcactt aacaaacaac cccaaagtaa cagggatgtc 14160
agcaagggac taagttctct catacacaag aggtccagtg ataggaagtc cagggccaca 14220
aggacaggtc cagagtcacc aggaacccag atgccttctc atttcttttt atttatttat 14280
ttatttattt tttgagacag agtcttgctc tctgtcaccc aggctggagt gcaatggcat 14340
gatcttggct ggctgcaacc tccgcctccc aggttcaagt gattctcctg cctcagcctc 14400
ctgaatagct gggattacag gtgtgcatca ccacgccttg ctaatttttg tatttttagt 14460
agagatgggg tttcaccatg ttggtcaggc tggtctcaaa ctcctgacct tgtgatccac 14520
ccgcctcaac ctcccaaagt gctgggatta caagcgtgag ccaccacgcc cggccaccct 14580
cttgtttctt acacattact tccattctca aaatgcctca tagtctaaaa tggctgccag 14640
agctccgggt ctcacatcta agttccagga aggaggaaga aggaaagaag ggacaaaaaa 14700
gggggcactc ctccttttgt aagggttctc ctttctggaa gtcccataaa gcacttctat 14760
ttatatcaca ttagtccgaa cttcatcaca tgactacctg gagaggctgg aatatgtagt 14820
tttcgacaag atctatgact gttctagata aaatgagggt cttatgatga agggtgaagt 14880
agagaatggt tgaatgcctg ctatttcggg caaatcccat cttagtccct cattcacaat 14940
ctgacatctg acctctgtcc cctggccccc atagggcgtc caaaggatga ccgccacctc 15000
cgtggctgtg gctaccttcc cggctgcccg ggctccaatg gtttccacaa caacgacacc 15060
ttccacttcc tgaaatgctg caacaccacc aaatgcaacg agggcccaag taaggaacgg 15120
gagacacagg caaggcctgg ggtcgggcag gggcatgcac tcaggcagac agctgcgcag 15180
tcactctctg gcaatcaagt cctctctggg cctctgtttg cttttctgaa aaatgggagt 15240
atcaggcctt tttttttttt tttttttttt tgagacggag tctcactctg ttgcccaggc 15300
tggagtgcaa tggcgtgatc tccgctcact gtaaacttcg cctcacgagt tcaagcgatt 15360
ctcctgcttc agcctcctga gtagctggga ttacaggtgc ccaccatcac ccctggctaa 15420
tttttctatt tttagtagag atgggggttt caccatgttg gccaggctgg tctcgaactc 15480
ctgacctcaa gtgatctgtc tgcctcagcc tcacaaagtg ctggggtaac aggcatgagc 15540
cactgcaccc agccaggcct ttcttcaaag aagaacttcg taaggattca atgatactat 15600
cagccctcgc caagacctgt gcttcctgtc tcttttgtct ctctcaaatt ttcccactcc 15660
tcccctgctc cactgccacc acctgggctg agccacgctg acttcttgcc caggttgttg 15720

caatgacctc ctctctggtc tccctgcttc cactcctgcc accctacagc caaggctcaa 15780
cccagcagcg agtgattttt tggggggtat ggggggggaca gggtctcgct ctgtcaccca 15840
ggctggagtg cactggcatg atcatggctc actgcagcct caaactcctg ggctcaggaa 15900
ttcttcccac ctaagctact ggagtagctg cgactacagg tgcatgccac cgtacccagc 15960
taattttgta tttttggtag agatggggtc ttgctttgtt gcccaggctg gtcttgaact 16020
cctgggcttg gcctcccaaa gtgttaggat tacaggcatg agccaccatg caccaccaat 16080
tttttatttt ttaatgtaaa tcagatcatg tcaccctcct ttctcaggca cttcaatgac 16140
ttccccttcc tcttagaata agatccaaag accttattct gttggcctat gtaatctggc 16200
acctgtctgc ctcacagtct ttgtttagat tcgtttttgg ggttttgtgt tttctttaga 16260
catagggtct tgctctgcca cccaggctgg ggtgcagtgg cacaatcacg gctcactgcc 16320
acctcaaact cctgggctcc agtgatcctc ccacctcagc ttcccaagta gctgggacta 16380
cagatgcgca tcattgcacc cagctacatt tttttttttt ttctgagatg gagtcttgcg 16440
ctgttgccca ggctggagtg cagtggcgcg atcttggctc actgcaagct ccgcctcccg 16500
ggttcacgcc attcccctgc ctcagcctcc cgagtggctg ggactacagg cacccaccac 16560
catgcctggc taatttttgt atttttagta gagacagggt ttcactgtgt tagccaggat 16620
gatctcgatc tcctgacctc atgatccgcc tgactcagcc tcccaaagtg ctgggattac 16680
aggcgtgagc caccacgccc ggccgcaccc agctaaattt ttaaaacttt tgtagagatg 16740
gggtctcact atgttgccgg ggctggtatc aaactcccag gctcaagcaa tccttctgcc 16800
ttgacctccc aaagtgctgg gattacaggc atgagccgct gcacctggcc ttcatttagt 16860
ttctttcttt cttcctttct ttcttttttt agatggagtc tcgctctgtc agccaggctg 16920
gagggcagtg gcgtgatctt ggctcactgc aacctccgcc tcccaggttc aagcgattct 16980
cctgcctcgg cctcccaaag tgctgggatt acaggcatga gccgctgtgc tgacgttcat 17040
ttggtttcta taatcaccaa agcccatctg gtctcatggc ccttgcagat gaatatcctc 17100
ccttagaaca catcttcccc aagagttcac cctgctggca acttctcatc cattaggcct 17160
cagctttaat gtatcatctt cagggatgct ttcactgtcc ctcccctcca gtgtaatcta 17220
gatccctgtc tctattaccc agcactgtca acagatagaa atgttcccta tctgtcctgt 17280
ccaatattac agccaccact gtatgtggtc agtgagcact tgaaatgtga actgaatttt 17340
aagattcgat ttaatattaa tttatttaaa tgtaaacaac cacatgtggc cagtggctac 17400
cagattgggt agtgtagctc taaattgtaa attctggctg ggcacagtgg ctcacacctg 17460
taatcccagc acttttggag gcggaggcgg gagggtcgcc tgaggccagg agtttgagac 17520
aagcctaggc aacatagcaa gactccgtct caaaaaaaat tttttagtaa taataatagt 17580
aataacttgt aagttctggc caggtacagt ggctcacacc tgtactccga gaacattggg 17640

aggctgaggc aggcagattg cttgatccca ggagtttgag accagcctga gtaatgtgtc 17700
aaaaccccat ctctacaaaa aacagaaaaa ttggctaggc atggtggtgc acgcctgtag 17760
tcccagctac tcaggaggct gatgtggaag aatctcttga gccggggtgg tcgaggctgc 17820
agtgagctac aattgcgcca ctgcactcca gcctgggcaa caaactgaga ccctgtatca 17880
aaaataaata aataagttgt aacttttgtg agagcaagga cagtttcatc ttacatagtt 17940
ttacccagca cccagcacgg tgcctggcca gaaatgggac tcatggggag ttgaaagttg 18000
gggtttctct caagccctcg aaaaccatcc ccctccaaat gccattcaga cgcctgacca 18060
caaggtggct ccatgacatc tctggctcgg agtggaagtc ctggggaggt cattcaagga 18120
agtggagatc caagcactaa ttttcttggt ctctgtgtct ctggtcttct cctaagtcct 18180
ggagcttgaa aatctgccgc agaatggccg ccagtgttac agctgcaagg ggaacagcac 18240
ccatggatgc tcctctgaag agactttcct cattgactgc cgaggcccca tgaatcaatg 18300
tctggtagcc accggcactc acggtgaggc cctctccgag gctgggaggg aacacttatt 18360
gggggtggag agttccgcaa gagattgact tccagttaag ctggagttaa ctgtggtggg 18420
tctccctgtg gaaaaggtgg gatttccttc aggtgggtga atattattgt aaggaattag 18480
gacttgtcca tcaggatgtg gacatttttg ccatggaaaa gtggaattcc ggctgggtgc 18540
ggtggctgaa gcttgtactc tcagcacttt gggaggctga ggccggagga tctcttgagg 18600
ccaggagttt gagaccagcc tggcaacat agcaagaccc ccatctctac aaaaagaaaa 18660
atggaattct ttggtggatt tctcgtgggt tgtggggatt tgctgaagac atcttgggct 18720
ttcatcctgt ggtgtcctgg aaaggtctct gtattccagt ggttctcaaa ctttttggtc 18780
tcaacttctt catgctttta aaaattattt ttggccaggt gtggtggctc acgcctgtaa 18840
ttgcaacacc ttgggagggt gaggaggaga accacttgag gtcaggagta tgagagcaag 18900
cctggccacc atggtgaaac cctgtctcta caaaaaatta caaaaattag ctgggtgtgg 18960
tggcgtgcac ctataacccc agctactcag gggctgagg cacgagaatc acttgaaccc 19020
aggaggagga ggttgcagtg agtggggatc accccactgc actccagcct ggatgacaga 19080
gggagacttt gtctcaaaaa aaaaaaaaaa ctctttattg tttatatcag tgatttctat 19140
agataaatgc cacattaaaa ttaaaactga gaaccaggag tggtggctca cgcctgtaat 19200
cctagcactt tgggaggccg aggtgggtag atcacctgag gtcgggagtt caagaccagc 19260
ctgaccaaca tggtgaaacc ccatctctac taaaaataca aattagtcgg gcatggtggc 19320
acatgcctat aatcccagct actcgggagg ctgaggcagg agaatcactt gaacctggga 19380
ggcagaggtt gcggtgagcc aagattgtgc cattgcactc cagcctgggc aacaagagtg 19440
aaactccgtc ccaaaaaata aaataaaata aaattaaatt aaaattaaaa ctgagaaatc 19500

tttttttttt ttttttgaaa tggagtcttg ctctgtcgcc aggctagagt gcagtggcat 19560
ggtctcagct cactgcaacc tccccctcct gggttcaagc aatcctcctg cctcagcttc 19620
ctgcatagct gggattacag gcatgagcca ccatgcccag cttatttttg taattttagt 19680
agagacgggg tttcaccatg ttggccagga tggtctcgat ctcctgacct catgatctgc 19740
ctgcctcggc ctcccaaagt gctgggatta cagatgtgag ccaccgtgcc cagccaaaac 19800
tgagaaatct ttaaagattt atttatgaat tcatcttata acaataaaaa ctcattacac 19860
attaagataa ataacatttt aatgaaaaat aagtatagtt tccaaagaaa aaatgtaatg 19920
atagccaggc atggtggctc atgcctataa tcccaatact ttgggaggcc aaggtgggcg 19980
ggtcacttaa gcctagggat ttgagaccag cctgggcaac atagcaagat cctgtctcta 20040
cagaaaatac aaaagttagc tgggcatggt ggtgcatgcc tgtagtccca gctgcttggg 20100
aggctgaggt gggagggtca cttgagcctg ggaggtggag gttgcagtga acggagattg 20160
caccactgca ctgcggcctg ggcaacagag cgagaccctg tcttaaaaaa acaaacaaag 20220
aaacaacaaa aaaaaaacca gccaggcgcg gtggctcacg cctgtaatac cagcactttg 20280
ggaggccaag gcgggcagat cacaaggtca ggagttcgag accagcctgg ccaatatggt 20340
gaaaccccgt ctctactaga aatacaaaaa ttagctgggc atggtggtcg cgcctgtagt 20400
cccagctact tgggaggctg aggcagaaga attgcttgaa cccgggaggc agaggttgca 20460
gtgagccgag atcgtgccac tgcactccag cctgggtgac agagtgagac tccatctcaa 20520
aaaaaaaaac aaaaaaaaag cgtaatgaga aaactggcat tattttgctg tgttgcacat 20580
ctctttaatg tccagcttaa tagaagacag ctgggtcctc atggaagctt ctgctttcag 20640
tctcttgcaa tatcacatgt cctggagttt ttggaaaagc ccattgtaca cttatgagag 20700
tgaaaaagcc atgtgaaatc acagatctcc tgaaagggtc tctggggtcc ctgagccaca 20760
ctttaagaac cgctgctcgc ttcctttctt tctttttttt tctttccttt tctttctctg 20820
tctctctttc cctttctttc tttctttctt ccttccttcc tcccttcctt ccttctttcc 20880
cttctccttc cttccttcct ccctctctct ctctttcttt cttttctttc ccttcttccc 20940
ttcctccctt ccttcttccc tccctccctc cctcccttcc ttcctttctt tctttttatt 21000
tgtttttttga aacagggtct cactctgtta cccaggctgg agtgcagtgg cgtgatcata 21060
actcaccaca gcctccatct cctgagctca agtgatcctc tcatctcagc ctcccaagta 21120
gctgggacta cagctgtttt tttcttttttt atgtttgtag aaacagagtt ttgctatgtt 21180
gtccaggctg gtctggaact cctgggctca agctatcctc tctccttggc ttcccaaggt 21240
gttgagatta caggcatgag ccaccacacc tggccctgag aactgctgct ttctctaggt 21300
ggtagtgaag gtggcaccca ctgcaaggtg gcaggtcacc tactggagaa ttccagtcct 21360
gggcccagga gctggaagtc tcactccgtc ttctctttcc tcagaaccga aaaaccaaag 21420

ctatatggta agaggctgtg caaccgcctc aatgtgccaa catgcccacc tgggtgacgc 21480

cttcagcatg aaccacattg atgtctcctg ctgtactaaa agtggctgta accacccaga 21540

cctggatgtc cagtaccgca gtggggctgc tcctcagcct ggccctgccc atctcagcct 21600

caccatcacc ctgctaatga ctgccagact gtggggaggc actctcctct ggacctaaac 21660

ctgaaatccc cctctctgcc ctggctggat ccgggggacc cctttgccct tccctcggct 21720

cccagcccta cagacttgct gtgtgacctc aggccagtgt gccgacctct ctgggcctca 21780

gttttcccag ctatgaaaac agctatctca caaagttgtg tgaagcagaa gagaaaagct 21840

ggaggaaggc cgtgggccaa tgggagagct cttgttatta ttaatattgt tgccgctgtt 21900

gtgttgttgt tattaattaa tattcatatt atttatttta tacttacata aagattttgt 21960

accagtgg 21968

<210> 79
<211> 21
<212> DNA
<213> Artificial

<220>
<223> Synthesis

<220>
<221> misc_signal
<222> (1)..(21)

<400> 79
ccgaggttgt gtgtgggtta g 21

<210> 80
<211> 20
<212> DNA
<213> Artificial

<220>
<223> Synthesis

<220>
<221> misc_signal
<222> (1)..(20)

<400> 80
ggcttcggga ataggtgaca 20

<210> 81
<211> 2876
<212> DNA
<213> homo sapiens

<400> 81
gaattcctgc agctcagcag ccgccgccag agcaggacga accgccaatc gcaaggcacc 60

tctgagaact tcaggatgca gatgtctcca gccctcacct gcctagtcct gggcctggcc 120
cttgtctttg gtgaagggtc tgctgtgcac catcccccat cctacgtggc ccacctggcc 180
tcagacttcg gggtgagggt gtttcagcag gtggcgcagg cctccaagga ccgcaacgtg 240
gttttctcac cctatggggt ggcctcggtg ttggccatgc tccagctgac aacaggagga 300
gaaacccagc agcagattca agcagctatg ggattcaaga ttgatgacaa gggcatggcc 360
cccgccctcc ggcatctgta caaggagctc atggggccat ggaacaagga tgagatcagc 420
accacagacg cgatcttcgt ccagcgggat ctgaagctgg tccagggctt catgccccac 480
ttcttcaggc tgttccggag cacggtcaag caagtggact tttcagaggt ggagagagcc 540
agattcatca tcaatgactg ggtgaagaca cacacaaaag gtatgatcag caacttgctt 600
gggaaaggag ccgtggacca gctgacacgg ctggtgctgg tgaatgccct ctacttcaac 660
ggccagtgga agactccctt ccccgactcc agcacccacc gccgcctctt ccacaaatca 720
gacggcagca ctgtctctgt gcccatgatg gctcagacca acaagttcaa ctatactgag 780
ttcaccacgc ccgatggcca ttactacgac atcctggaac tgccctacca cggggacacc 840
ctcagcatgt tcattgctgc cccttatgaa aaagaggtgc ctctctctgc cctcaccaac 900
attctgagtg cccagctcat cagccactgg aaaggcaaca tgaccaggct gccccgcctc 960
ctggttctgc ccaagttctc cctggagact gaagtcgacc tcaggaagcc cctagagaac 1020
ctgggaatga ccgacatgtt cagacagttt caggctgact tcacgagtct ttcagaccaa 1080
gagcctctcc acgtcgcgca ggcgctgcag aaagtgaaga tcgaggtgaa cgagagtggc 1140
acggtggcct cctcatccac agctgtcata gtctcagccc gcatggcccc cgaggagatc 1200
atcatggaca gacccttcct ctttgtggtc cggcacaacc ccacaggaac agtccttttc 1260
atgggccaag tgatggaacc ctgaccctgg ggaaagacgc cttcatctgg gacaaaactg 1320
gagatgcatc gggaaagaag aaactccgaa gaaaagaatt ttagtgttaa tgactctttc 1380
tgaaggaaga gaagacattt gccttttgtt aaaagatggt aaaccagatc tgtctccaag 1440
accttggcct ctccttggag gacctttagg tcaaactccc tagtctccac ctgagaccct 1500
gggagagaag tttgaagcac aactccctta aggtctccaa accagacggt gacgcctgcg 1560
ggaccatctg gggcacctgc ttccacccgt ctctctgccc actcgggtct gcagacctgg 1620
ttcccactga ggccctttgc aggatggaac tacggggctt acaggagctt ttgtgtgcct 1680
ggtagaaact atttctgttc cagtcacatt gccatcactc ttgtactgcc tgccaccgcg 1740
gaggaggctg gtgacaggcc aaaggccagt ggaagaaaca ccctttcatc tcagagtcca 1800
ctgtggcact ggccacccct ccccagtaca ggggtgctgc aggtggcaga gtgaatgtcc 1860
cccatcatgt ggcccaactc tcctggcctg gccatctccc tccccagaaa cagtgtgcat 1920

gggttatttt ggagtgtagg tgacttgttt actcattgaa gcagatttct gcttcctttt 1980

atttttatag gaatagagga agaaatgtca gatgcgtgcc cagctcttca ccccccaatc 2040

tcttggtggg gaggggtgta cctaaatatt tatcatatcc ttgcccttga gtgcttgtta 2100

gagagaaaga gaactactaa ggaaaataat attatttaaa ctcgctccta gtgtttcttt 2160

gtggtctgtg tcaccgtatc tcaggaagtc cagccacttg actggcacac acccctccgg 2220

acatccagcg tgacggagcc cacactgcca ccttgtggcc gcctgagacc ctcgcgcccc 2280

ccgcgccccc cgcgcccctc tttttcccct tgatggaaat tgaccataca atttcatcct 2340

ccttcagggg atcaaaagga cggagtgggg ggacagagac tcagatgagg acagagtggt 2400

ttccaatgtg ttcaatagat ttaggagcag aaatgcaagg ggctgcatga cctaccagga 2460

cagaactttc ccccaattaca gggtgactca cagccgcatt ggtgactcac ttcaatgtgt 2520

catttccggc tgctgtgtgt gagcagtgga cacgtgaggg gggggtgggt gagagagaca 2580

ggcagctcgg attcaactac cttagataat atttctgaaa acctaccagc cagagggtag 2640

ggcacaaaga tggatgtaat gcactttggg aggccaaggc gggaggattg cttgagccca 2700

ggagttcaag accagcctgg gcaacatacc aagacccccg tctctttaaa aatatatata 2760

ttttaaatat acttaaatat atatttctaa tatctttaaa tatatatata tattttaaag 2820

accaatttat gggagaattg cacacagatg tgaaatgaat gtaatctaat agaagc 2876

<210> 82
<211> 11855
<212> DNA
<213> homo sapiens

<400> 82
gaattcctgc agctcagcag ccgccgccag agcaggacga accgccaatc gcaaggcacc 60

tctgagaact tcaggtagga gaaaagcaaa ctccctccaa cctcttactt cgggcttaag 120

gcagagaact cgcctcccca gaatctcctc cctccatgat cccccgctat tcctctattt 180

tcttttcctc ggacctgcag ccttgggtcg accctgccct aggggtgact gcaggagagc 240

agggaggatg gtcaggcgtc accaacaacc ccatcaccca gtaacaagaa ccttgactct 300

ctcagtccct ctgcatcaag acacttaccc atttcccacc tcatgcctgc taacttgaat 360

gaaacaatcg ctgggaaagc attaagagaa ttaaggctgg gcactgtggc tcatgcctgt 420

aatcccagca ctttgtgagg ctgaggcagg cagataactt gagcccagga gtttgagacc 480

agcctgggca acatggcaaa accctgctct cccaaaaaaa tacaaaaatt agctgggcgt 540

gctggtgtgc ctgtattccc agctacttgg gaggctgagg tgggaggatt gcttcagctg 600

gggaggcgga ggctgcaggg agccaagact gagccattgc acccagcctg ggtgacagag 660

caagaccctg tctctaaaaa tgaatgaaag gaaggaagaa agagagagaa agagagagag 720

gaaagaagga aggaagtaaa gaagaaagaa agaaagaaag aggaaagagg aagaaagaaa 780
gaaaagaaag aaaagaaaag aaagcaaatt taaagcttat gcaaatcaaa gatgttgtga 840
taattgataa ttgagtctgg gctaaattcc ccctgggctg caaaggcaga gagtggtaat 900
gacttctcac ctgcttttct tctaaggctt ttttacggga cacagaggga agggagatgg 960
actggattcc aagattccca cagggcaaga tgggcgaaga ctccctgcca ctgcccgggg 1020
ataagtcagt ctgagtgaga cggagtggga tgggcttaga acctgaacat gtcatggtct 1080
cttcctgcac cttgccctag tgttcactta ccacctgctt gcaggaaaca agaagagcag 1140
ggcccacagc tggccagctc ccctcccctc ccgcctgtct tccagaacga ttccttcacc 1200
agccctcttt ccattgctct aggatgcaga tgtctccagc cctcacctgc ctagtcctgg 1260
gcctggccct tgtctttggt gaagggtctg ctgtgcacca tcccccatcc tacgtggccc 1320
acctggcctc agacttcggg gtgagggtgt ttcagcaggt ggcgcaggcc tccaaggacc 1380
gcaacgtggt tttctcaccc tatggggtgg cctcggtgtt ggccatgctc cagctgacaa 1440
caggaggaga aacccagcag cagattcaag cagctatggg attcaagatt gatggtgagc 1500
cacgggacac caggggaggt gggtggcatg cagaacagac ctaccagaag ccaaggaaag 1560
gctggctctg gcttagccga gccaagcccc atacagctgt gctgcagggg ccaccccatc 1620
ttcttcccac tacactccaa gtcactggac ccttgaatct ccaagggtgt ctgaccagta 1680
gatttaccgc ttattcacca ccgtgtgatc ttaacctcgt taagtttgcc catctacaaa 1740
atgaggatta tttgctgtcc taaagaattc atgagccggg cgcggtggct caaacgcctg 1800
taatcccagc actttgggag gccaaggcgg gcggatcatg aggtcaggag atcaagacca 1860
tcctggctaa cacagtgaaa ctccatctct actaaaaata caaaaaaaat tagccaggcg 1920
tggtggcagg cgcctgtagt cccagctact cgggaggctg aggcaggaga atggcatgaa 1980
cccaggaggc agagcttgca gtgagctgag atcgtgccac tgcactccag cctgggcgac 2040
agagagagac tccgtctcaa aaaaaaaaaa taaaagaatt catggaatta cacttgtgaa 2100
atacttagca tagccatcac tataggaaaa aaatctaagg ccaggcacag tgcctcatgc 2160
ctgtaatctc agcactttcg gagtttgagg caggaggatc acccaaggct aggagttcaa 2220
ggccagcctg ggcaatacgg tgaaaccccg tctctaataa aaatataaaa attagtctga 2280
tgaggtggtg cacctgtaat cccagctact caggaagctg agacacaaga atcactttaa 2340
cccgggaggt ggaggtggca gtgagctgag atcacaccat tgcactccag cctgggtgac 2400
agagtgagac ctgtcaaaaa aaagaaaaga aagagagaga gagagagaag agagagaaag 2460
aaagaagaag aaagaaagaa agagagagag agaaagaaag aaagaaacaa agaaacaaag 2520
aaagaaagga aaagaaaaaa aaaactaagg ccaggcaagg tggcttatga ctgtaatttc 2580
agcactttgg aagattgagg caggaggatc acttgaggcc agaagttcga gacaagactg 2640

agcaacaggg agacccctgc ctctacaaaa aaatttacaa attagccaga tgtggtgaca 2700
catacctgta gtcccaacta ctcaggaggc tgaggtggga ggatggcttg agcccaggag 2760
ctggaggctg cagtgagcta tgattgtacc actgcacttc agcctgggca acaaagggaa 2820
gccctgtctg aaaaaaaaaa aaaaagaaaa agaagaagaa agaaaatatt tagggttcat 2880
ccaggaggca gaggttgcag taagctgaca tcgcgccatt gcactccagc ctgggagaca 2940
agagcaaaac tccaactcaa aaaaaaaaaa aaaaaaaaaa caggaagaaa atatttaggg 3000
ttcataattt aagaacagag aaaaatattc tagcccaaag aaagggttgg gatctgagac 3060
ttttgaagaa aggaaggaga tacagaaaag agatttcatc ctggaatgaa atctccctcc 3120
agagagccct gggaaagcac ggtagccccc atccatcaga gtggagcccc ttgtggggga 3180
agtgggctcg gctgggaacc ctcaattcag cataagcctc acatgtcctc tcctctctgt 3240
cccggtgcag acaagggcat ggcccccgcc ctccggcatc tgtacaagga gctcatgggg 3300
ccatggaaca aggatgagat cagcaccaca gacgcgatct tcgtccagcg ggatctgaag 3360
ctggtccagg gcttcatgcc ccacttcttc aggctgttcc ggagcacggt caagcaagtg 3420
gacttttcag aggtggagag agccagattc atcatcaatg actgggtgaa gacacacaca 3480
aaaggtgagc aggcagggaa aggaaaccca tttcctgggc ctcaagagaa agggaatttg 3540
gaaataaatc cacatatccc agttgggtgc agtagttcac acctgtaatc ccagcccaac 3600
actttgggag gtctaggcga gaggaaggct tgaggcctgg agtttgagac cagcctggcc 3660
aacataacaa gacctcatct cttcaaaaaa tttaaaaacc agccgggcat ggtggtgcac 3720
acctgtagtc ccagctactt gggaggctga ggtgggagga tcacttgagt ccagcagttc 3780
aaggctgcag tgagctatgt ttgcaccacc acactccagc ctgggtcaca gaacaagacc 3840
tcatctctaa aaaacaaaca aaaaccaaat ccacatatcc taaaaaatgc tccttttcag 3900
cattctcttc tctatggaca aagggctgga tgctttaaga accaaatctt aggctgggca 3960
cggtggctca cgcctctaat cctagcactt tgagaggcca aggcgggcag attgcctgag 4020
cacaggagtt cgagaccagc ctggccaaca tggtgaaacc ctgtctctgt caaaaataca 4080
aaaaattagc caggtgtgtt ggcgcatgcc tataatccca gctgctcggg aggatgaggt 4140
tcaaagaatc acttgaaccc gggaggcaga ggctgcagtg agctgagatc atgccactgc 4200
actccagcct gggtgacaga gcaagacttt gtctccaaaa aaaggaacta gacgggttca 4260
tttaaacccc tgactgcagc cctttgacat acatccaatt gaggactggg gactccggga 4320
aacatctaaa aggcttaaaa actttgtcta acttcagccg ggcatggtgg ctcacacctg 4380
taatcccagc actttgggag gctaaggcag gtggatcaaa aggtcaggag tttgagacga 4440
gcctgaccaa catggtgaaa ccccgtctct actaaaaata caaaaattag ccaggcatgg 4500

tggcaggcgc ctgtaatccc agctattcgg gaggctgagg caggagaatt gcttgaaccc 4560
cggagacaga ggttgcagcg agccgagatc gcgccactgc actccagcct ggcaatagag 4620
tgagactcca tctcaaaaca acaacaacaa caacaacaaa atcgtctaac ttcctgatct 4680
tcctgatcat tgattttccc ataggtatga tcagcaactt gcttgggaaa ggagccgtgg 4740
accagctgac acggctggtg ctggtgaatg ccctctactt caacggccag tggaagactc 4800
ccttccccga ctccagcacc caccgccgcc tcttccacaa atcagacggc agcactgtct 4860
ctgtgcccat gatggctcag accaacaagt tcaactatag taagtccaag agccccttcc 4920
ccacagccca cagcaactgc atctcattcc tggggtctcc caaggaatac ccaaaatgtc 4980
accctctgag ggaggaagac cacagggaat gctccccttt aagggaggag agaccctaga 5040
atatactcca gctttgacaa agatttccca agcaggagac atcaggataa tgggaacaga 5100
agacaggagg tttatcccat gaaggatgaa gaagctgaaa tccagagatt ccctcagggc 5160
cacatttgtc cacctgactc cagggtctca tcttcgtgtg ttgctagtgt gattacctgg 5220
ggatgagaaa tcctgctggg ggagttgagg ttaagaggat gaggactcca ggtgctgtgg 5280
ctcacgcctg taatcccagc actttgggag gccaaggcag gtggatcagg agtttgaggt 5340
caggagtttg agaccagcct ggccaacatg gtgaaaccct gtctctacta aaaatgcaaa 5400
aattagccag gtgtggtggc aggcgcctgt aatcccagct actcgggagg ctgaggcagg 5460
agaatcactt gagcccggga ggtggaggtt gcagtgagcc gaacgaaatt gagccacttc 5520
accccagcct gggcaaaaga gtgaaattcc attcaaaaaa aaaaaaaaaa aaaaaaggat 5580
gaggactggg atgaactggt ggctgggtgt ggggaaaatg gaagtgaagg aaggccaaaa 5640
gagacagaga aggcctggcg cggcgactca cgcctataat cccagcactt tgggaggctg 5700
agaaggggga ttgcttgagg ccagaagttg aataccagtc tgggcagcat agcaagaccc 5760
tgcctctaca aaaaaaaaat ttttttaat tagccaggct tggtgacatg catctgtagt 5820
ctactcaaga agctgaggtg aggccaggca cggtggctca cgcctgtatt cccagcactt 5880
tgggaggtca aggcgggtgg atgacctgag gtcaggagtt caagaccagc ctggccaaca 5940
tggtgaaacc ccatctgtat aaaaatacaa aaattagctg ggcatgatag caggtgcctg 6000
taattccagc tactcaggag gctgaggtgg gagaatctat tgaacccggg aggggggaggt 6060
tgcagtgagc cgagatcatg ccattgcact ccagcctggg cgacagagtg agactccttc 6120
tcaaaacaaa caaacaaaca aacaaacaaa atacagaagc tgaggcggga ggaacatttg 6180
aaccggattc ggaggctgca gtgagctatg attgcaccac tgcgctccag tctgtgtgac 6240
agtgagaccc tgtctcttac acacacacac acacacacac acatgcacac acacagagag 6300
agagaaatta gaagatactg aattggcaga agagaaggga aatagaaatt aaaatactga 6360
ataggggagc agtgaacagg ggatacccaa aagccaagag cgagagagag cctggcttcc 6420

agaaatagtg gagaagccag gagaactagg tgaaaaccca gtgctgggtt gccatcagcg 6480
agagctggag ccatttccaa cgaaccatct tgtcgtcttc acagctgagt tcaccacgcc 6540
cgatggccat tactacgaca tcctggaact gccctaccac ggggacaccc tcagcatgtt 6600
cattgctgcc ccttatgaaa aagaggtgcc tctctctgcc ctcaccaaca ttctgagtgc 6660
ccagctcatc agccactgga aaggcaacat gaccaggctg ccccgcctcc tggttctgcc 6720
caagtaagcc accccgctat ctccccgacc taccaacccc tctctcctgg ctccctaaag 6780
tcaccgcccc caggttgaat ttcccagatc tgtgatgctt gcaggacatg catgtgtggg 6840
aggctgatgg gaaactgtgg cctgggtttg attatgagtc ttgcaatcat ccctccccct 6900
gtttctgctg gagggcaggg gacagctctt cctgaccaca cccccacatt gactatcccc 6960
agaataccca gcaaaagccc ccaaaaggag agtcagagaa atgagggagg tgggggccca 7020
atcagtccac atctacttag ggtcgcccca tcagcacttc catccccaac cctttcaagt 7080
caacatccaa acaaaagaaa tcacttccaa ggacggagca gctcaaagcg cagcttctag 7140
ctggggttcc aagaaagcag atttttcgaa atccttctgc agaaggaagc aaagagattt 7200
tttgaaatct ttctgcagaa ggagaaggct ggagctgggg aactccagaa ttatagggaa 7260
gcctcccacc acgctcatcc caaatttccg gatgctataa tgccaggctt ggggaaagag 7320
gagaatttag ttggttagct ggtgcgtgct ctcacttgca tcctctctct tcctcttttt 7380
ttttttctc ctctctctct ggctcataaa aatggaggta attagttgtg ccctggtgag 7440
aagcagagag tgcacaaagg ccccctgctt gagtcctctt cagggttagc tctcagaaac 7500
acaatctgca gaacagattt ttgttccaac atccttgcag gagaatttgc ccttagcttc 7560
ccccacccca gccaggctga ataaaattat gctgaaacta ctgtcttatt tgaggaaagt 7620
aattagtcat aggtgggagg gggtggggag attgcagaag aatgttcatg aatattagga 7680
ttttcagctc taagggggga ctttgtaaac agctttagaa gaagaaccag gccggctggg 7740
tgtggtggct catgcctgta atctcagcat ttggggaggc caaggcgggc ggatcacttg 7800
aggtcaggag tttgagacca gcctggccaa catggtgaaa ccctgtctct attaaaaata 7860
caaaaattag ccagccgtgg tagcgagcgc ctatgatccc agctactccg gaggctgagg 7920
ccagagaatc acatgaacct gggaggtgga ggctgcagtg agccgagatc acgccactgc 7980
actccagcct gggggacaga gcaagaatct gtttcaaaaa aaaaaaaaga aaaataggaa 8040
ggaaggaagg aaaggaaagg aaagaagaga gagagaaaga aagagagaga gagaaagaaa 8100
gaaagaaaga aagaaagaaa gaaagaaaga aagaaagaaa gaaagaaaga aagaaaaaga 8160
aaggaaagaa agaacgaacg aaccaggcct ccctctccaa ccttcacctc cgtccctatt 8220
ctggccactt gattcggggg acacctggta ggggatgggg aaaggtggga gctgccagcc 8280

agaggggacc ccggcttgag cagcctcttg ctgctatctg caggttctcc ctggagactg 8340
aagtcgacct caggaagccc ctagagaacc tgggaatgac cgacatgttc agacagtttc 8400
aggctgactt cacgagtctt tcaggtaaga agactttcct ttgcattttc tcaccccagt 8460
ggactgcggg ggcccctaag aggaaaaagg aacctctcct tgagagcggc agctgatcta 8520
atcctgtatc cacatctgtt tcagaccaag agcctctcca cgtcgcgcag gcgctgcaga 8580
aagtgaagat cgaggtgaac gagagtggca cggtggcctc ctcatccaca ggtgagtctg 8640
gctcaggtga ggctccacgg gtgtcgcctc catcgccctt caggataact ggtccccaga 8700
cccggaaagg accccgcagc cctctcggca cagagcagct ctgtctgtgc tcagccatca 8760
cccactcccc acctgtttct cagcctggaa aacgggcttg ggaccatgga accctgtttc 8820
ctcgcctgat ggctcctaag ttccctgact gtgaaaaggc ctcctaaaga aaaacccaag 8880
ttgttcccac agtgggaagt aaacttaaga aacatgctta tcaggctggg catggtggct 8940
cccacctgta atcccagcgc tttgggggac caaggcaggt ggatcacttg aggtcaggaa 9000
ttcgagacca gcctgggcaa catggcaaaa ccctatctct actaaaaata caaaaattag 9060
gcaggcgtgg tggcatgtgc ctgtagtccc agctacttgg gaggctgagg caggagaatc 9120
acttgaatcc aggaggcaga ggttgcagtg agccgagatc acgctgctgc actccagcct 9180
gggcaataga gcatgactct gaagaaaaga aagaaagaaa gagagagaga gagaaaagaa 9240
agaaagaaag aaagaaagaa agaaagaaag aaagaaagaa agaaagaaag aaagagaaag 9300
aaagagaaga aaagaaaaga aagagcttat caataagccc ttaaaggatt tagataaatg 9360
tgtgtaaggg aagagctgat ccattgctac caagctcctg gaggaaacca ggtctcagag 9420
gatgtcccta aacttttaag gttcatattc aggaaaacaa acaacttcca gctgggctta 9480
gtggctcaca cctgtaatcc cagcactttg ggaggccgag gcaggaggat cgcttgagcc 9540
caggaatttg agaccagcct gggcaatata atgagactgt gtctctacaa aaattagaaa 9600
aaaattagcc aggcatggtg gcatgcacct gtagccccag ttacttggga gactgaggtg 9660
ggaggatcac ttgagcccat gagttcaagg ctgcagtgag ccatgaaggt gccactgcac 9720
tcccacctgg gcgacagagg gagaccctgt ctctaagaaa aacggcgggg gtgggggtgg 9780
tgccagtgcc agcatccctc tgttctaaga cattgtccct tctcttgcag ctgtcatagt 9840
ctcagcccgc atggcccccg aggagatcat catggacaga cccttcctct ttgtggtccg 9900
gcacaacccc acaggtgagc ctggaaccca tcacgttcca catcctccca cccattcttt 9960
ctctcaggaa ctagtcccga cagatgcaga catccctcta tccctgagag ggctctgggc 10020
agggaaccca taaccctacc ctgcttcctg tcccaagagg aggctacctt ctatcaccca 10080
cagacagtgc cgggtccccg ctctgtgact caggcagctg cgactccaga cagctcactc 10140
atctgcctag atctcagtcc ttccacccac atccagcctg atgagctgtc ccactccttc 10200

tgcttctcaa cccccatggt tcttccaccc tcaggaacag tccttttcat gggccaagtg 10260
atggaaccct gaccctgggg aaagacgcct tcatctggga caaaactgga gatgcatcgg 10320
gaaagaagaa actccgaaga aaagaatttt agtgttaatg actctttctg aaggaagaga 10380
agacatttgc cttttgttaa aagatggtaa accagatctg tctccaagac cttggcctct 10440
ccttggagga cctttaggtc aaactcccta gtctccacct gagaccctgg gagagaagtt 10500
tgaagcacaa ctcccttaag gtctccaaac cagacggtga cgcctgcggg accatctggg 10560
gcacctgctt ccacccgtct ctctgcccac tcgggtctgc agacctggtt cccactgagg 10620
ccctttgcag gatggaacta cggggcttac aggagctttt gtgtgcctgg tagaaactat 10680
ttctgttcca gtcacattgc catcactctt gtactgcctg ccaccgcgga ggaggctggt 10740
gacaggccaa aggccagtgg aagaaacacc ctttcatctc agagtccact gtggcactgg 10800
ccacccctcc ccagtacagg ggtgctgcag gtggcagagt gaatgtcccc catcatgtgg 10860
cccaactctc ctggcctggc catctccctc cccagaaaca gtgtgcatgg gttattttgg 10920
agtgtaggtg acttgtttac tcattgaagc agatttctgc ttccttttat ttttatagga 10980
atagaggaag aaatgtcaga tgcgtgccca gctcttcacc ccccaatctc ttggtgggga 11040
ggggtgtacc taaatattta tcatatcctt gcccttgagt gcttgttaga gagaaagaga 11100
actactaagg aaaataatat tatttaaact cgctcctagt gtttctttgt ggtctgtgtc 11160
accgtatctc aggaagtcca gccacttgac tggcacacac ccctccggac atccagcgtg 11220
acggagccca cactgccacc ttgtggccgc ctgagaccct cgcgcccccc gcgcccctct 11280
ttttcccctt gatggaaatt gaccatacaa tttcatcctc cttcagggga tcaaaaggac 11340
ggagtggggg gacagagact cagatgagga cagagtggtt tccaatgtgt tcaatagatt 11400
taggagcaga aatgcaaggg gctgcatgac ctaccaggac agaactttcc ccaattacag 11460
ggtgactcac agccgcattg gtgactcact tcaatgtgtc atttccggct gctgtgtgtg 11520
agcagtggac acgtgagggg ggggtgggtg agagagacag gcagctcgga ttcaactacc 11580
ttagataata tttctgaaaa cctaccagcc agagggtagg gcacaaagat ggatgtaatg 11640
cactttggga ggccaaggcg ggaggattgc ttgagcccag gagttcaaga ccagcctggg 11700
caacatacca agaccccgt ctctttaaaa atatatatat tttaaatata cttaaatata 11760
tatttctaat atctttaaat atatatatat attttaaaga ccaatttatg ggagaattgc 11820
acacagatgt gaaatgaatg taatctaata gaagc 11855

<210> 83
<211> 23
<212> DNA
<213> Artificial

<220>
<223> Synthesis

<220>
<221> misc_signal
<222> (1)..(23)

<400> 83
ggctgacttc acgagtcttt cag 23

<210> 84
<211> 22
<212> DNA
<213> Artificial

<220>
<223> Synthesis

<220>
<221> misc_signal
<222> (1)..(22)

<400> 84
cgttcacctc gatcttcact tt 22

<210> 85
<211> 2222
<212> DNA
<213> homo sapiens

<400> 85
gagtcccggc agtgcgggac tccggtagcc gcccctccgg tagccgcccc tcctcccccc 60

gcgccgccgc cctatatgtt gcccgccgcg gcctctgggg catggagcac gctgcccagc 120

cctggcgatg gcaacggcga cgggggtggc ggaggagcgc ctgctggccg cgctcgccta 180

cctgcagtgc gccgtgggct gcgcggtctt cgcgcggaat cgtcagacga actcagtgta 240

cggccgccac gcgctgccca gccacaggct ccgagtgccg gcgcgggccg cctgggtggt 300

gcaggagctg ccctcgctgg ccctgccgct ctaccagtac gccagcgagt ccgccccgcg 360

tctccgcagc gcgcccaact gcatcctcct ggccatgttc ctcgtccact acgggcatcg 420

gtgcttaatt tacccgtttc tgatgcgagg aggaaagcct atgccactgt tggcatgtac 480

aatggcgatt atgttctgta cctgtaacgg ctatttgcaa agcagatact tgagccattg 540

tgcagtgtat gctgatgact gggtaacaga tccccgtttt ctaataggtt ttggcttgtg 600

gttaacaggc atgttgataa acatccattc agatcatatc ctaaggaatc tcagaaaacc 660

aggagatact ggatacaaaa taccaagggg aggcttattt gaatacgtaa ctgcagccaa 720

ctattttgga gaaatcatgg agtggtgtgg ctatgccctg gccagctggt ctgtccaagg 780

cgcggctttt gctttcttca cgttttgttt tttatctggt agagcaaaag agcatcatga 840

gtggtacctc cggaaatttg aagagtatcc aaagttcaga aaaattataa ttccattttt 900

gttttaagtg cgtttttcat gaaattatct tcaacttgaa gctttccaat ggcgcttctc 960

tatggacttt gtaaataagt tatatctttg taattttcct gctactttat cattttcaag 1020

atgtcctcta ggaatttttt ttctagtaat tttgcaatct acctaataag tacctaaata 1080

cgctgaaatg gaggttgaat atcctactgt gtaacaggtc agaatttcaa gctctgggta 1140

ataactgctg atatttttttc taatttcaaa tttacctctt ttggctatgt cttgccaagt 1200

gtgtatgaga ctagacttta caactgtctt tgatggcatt ttcagaacaa taaatgtcac 1260

aatcccttct atagccccct acagtgatct cttcaaggtc aactgcagtg ttgcttccct 1320

ccccctatag ggctggaatc tgtctaggag ccctctctcg gaggccatag aggctggggg 1380

tagccattgt gcagtcatgg cccgggggaa acttgccaac cttcgtgtca ggtgctgtgt 1440

ataagtggag aacttgggga tagaggagga agctcctcgt ggcccttcca aggtgaggca 1500

aaggcatctg gacttgttcc agcccagccc accgggtgac atcaccgggc agggaggggt 1560

gctggtggtg gttcatacgg agtaagctgc tctgcctgtg tgagtggctc ctgggcccta 1620

aacaggcacc tttaggccat gggtcactca ccgtgagcca tcaatgtgct ctggtctgac 1680

atggtttctc tctgtcttct agtctagacc tagttttttt gttctgttcc ccacgtatgg 1740

atatagtaga gattgttgtc tgtgaaattt ctcttttgta gattttgagt tttcccttgt 1800

agtgtaaaga atgatcactt tctgtaacaa taacaagacc actttttaag atttatcctg 1860

tttgttcttt gttgattgaa acataataat tgttaaaatt ctctacagcc ttctttttct 1920

tccatagcta atcttccttc taatagtttt tgctttctgt tttgctgttg ttgctttgca 1980

aagctttccc ctcatagcct gtacctgtta tcaatataaa ataatcttcc tgttgaatgc 2040

ttcatgactt gaattctact ttgataaaaa cattgccata ctgcttttta tcttgatgaa 2100

ttcatctggc attgctttgc cttatcatct catctggagt ttttaaatgc catttgtttc 2160

agttgtcttt aacaacataa taaatagact ttgccattta aaaaaaaaaa aaaaaaaaaa 2220

aa 2222

<210> 86
<211> 36113
<212> DNA
<213> homo sapiens

<400> 86
gagtcccggc agtgcgggac tccggtagcc gcccctccgg tagccgcccc tcctgccccc 60

gcgccgccgc cctatatgtt gcccgccgcg gcctctgggg catggagcac gctgcccagc 120

cctggcgatg gcaacggcga cgggggtggc ggaggagcgc ctgctggccg cgctcgccta 180

cctgcagtgc gccgtgggct gcgcggtctt cgcgcgcaat cgtcagacga actcagtgta 240

cggccgccac gcgctgccca gccacaggct ccgagtgccg gcgcgggccg cctgggtggt 300
gcaggagctg ccctcgctgg ccctgccgct ctaccagtac gccagcgagt ccgccccgcg 360
tctccgcagc gcgcccaact gcatcctcct ggccatgttc ctcgtccact acgggcatcg 420
gtaacgtccc cggcccccgg ccccctaccc tactcccggc ccggcgtcct ctccgaccct 480
cccctcactg cccggtgccc tctccccgaa gcctccccca cccagatgcc ctctccctgc 540
cagatccccc ggggcgtccc ccatctgcac gggtgccctt ccccgagtcc cccggccccg 600
ggagtttgga aaccagaagg gattcgtctg gaaagggcgg gaaacaaagg actggaaggg 660
agtgatggaa aaaaacatga tgaaactgat ctggaaagag ccactcagtg gtcctaggtt 720
tggtttcctt agccgggcgc ggtggcgcct gtctgtgatc ccagttactc gggagtctga 780
gatggaagaa tcgcttgaac ccgagaggcg gaggttacag tgagccgaga tctcgccact 840
gccctccagc ctgggtgaca gagcgagact ctgtctcaaa attaaatata taaaaataaa 900
gagttaaaaa gtaaaaatgt aaaataaaaa tgaagactag attttgttcc cttagatcac 960
gactgtgggg gcggggggga gggggttggc ctagagtcta atgctgatgg tcggtcttga 1020
gggccactgg tttctactcc tttctgccgg ttccccttttt ggtatctttg gtgatggtgg 1080
agctgttttc tcccagccaa gtgatcagct gcaactggaa acctgctgtc ctaaaactgc 1140
ctagcagttt ctccttatag gcttgatcac tttcagagac tttcatgtcc accttatgca 1200
agggacccca ggattgtggg ggtggaaagc cagcaacctt aaaccttaaa caaacgaagt 1260
cttaaagatg acttctaaat ctttcaaggt tttgttttt ttgaatattt gaaaactggc 1320
tgtgtctgaa atcttcaagc cttctaataa gttggggttc tctccaggtc gtatcttttg 1380
gcttttacac agccaaagca aacgacaggt cgaaagttca aaattaaatt ttaaacgttt 1440
tcattcaggc tctggaaagc ttcacagtta aaaaggatgt ctctacaccc aaggaagttg 1500
aactcactgg ctgtgtgact atgggcagtt tacccaacct ttctgatttg gggtcccacc 1560
ttaaaacact cacttcccag agagacagga agaactcagt gtgtgtttat aagcctctct 1620
tctttctcct ggtgtcatgc attccagcga agagaaagta cacagctcca ctacttggaa 1680
ccagtgttgt acccagcaca gtttttggta cctgagttcc ctgaaaacca gcaccttacc 1740
ctgtaactgg tgcagtctgt gtcctcagtg tgctttgatg acttgcactt taaacaaggg 1800
caagtcaaca ttcatcagag gcttagtgag tactgtgtgc ctgaaactct ttaccattgc 1860
tttatttttt ttttggaatg ggatcaacca ttgctacaat ttgaatatta gtcagacttt 1920
gctgtttgta tatacttttc cataaatata ccagaagctg tatgtctgtt tctttatggc 1980
taaaagcaat ctcaggtcag agtattcggt gctatcctag tcccatcagt catgaccctg 2040
gatgagttac taagctgccc cgcagcaagg tttccccacc tgtagaatgg gcatggtgat 2100
ggaacccccc tgtgggatca catgaattta gagcaaaggc aactccctaa aatgcttgca 2160

tggtgctggc gcatctgcgt gctcagtaca caatacctag cactattttg cattgctgtg 2220
aatgcacatc cagacatgta ctctgtgtgt cattcatgtg cactgctgcc agcctcatgt 2280
gccagcaaaa ctgtggtgtt ggaaatcaag ggaacacttg acaaagtttt gcctgacatc 2340
ccaagtggaa gctttttaac ttgagacatc atgtttcatt gtacctacat tcactcaagc 2400
agaaaaatga atgcagcctc ccaaagggcc agagtcacct tgaatgatag aacttgaaca 2460
gctcattcat acagagtaga acacggaggc acagcatgct gctgtcttcc ctgaggctgt 2520
tggataagga gctgctggga tgaaagccca cttcctatac taatagttta aaagctgctc 2580
caagaggatt ggctgggtca ggtatagttt ttttttaaa ttgctaggct attttttttt 2640
tgagttgtac atgttattgt gtacatagtc tatgtggttt ggggacattt acattttcca 2700
aatagaaaag ataaacattt tccattctac ccaagcaact aatttattaa acagtcactt 2760
gatctaagtc atgagcactt ggaagagaac cagagtggaa acagcattta gcactgaaca 2820
caaaccatcc cagcagcccc tggaagcgtc ctggagccct ttatttgcac agggccctgc 2880
agagaacgga gaacaccaca cccggtgaga ggaggctgtg ggtacaagga tgactcttat 2940
gaaaggcagg agaaaagtga gcctgaggag gcgctggcca ggcagagcca gtaccacctt 3000
acacaacctg acaggtgtgc acacaggccc tgtgctgacc tgtgaggttt cgctggagtg 3060
aggttgggcc ctgccttggg gaactagagc ctcttttggg agtggcaagg accagagcca 3120
ggtccaggga ctttccacag tccatgaggc ctgaaggcag cagcagagct acagctcttc 3180
ccagagcaac tgactgttct gggaacaatg agtacaggac agtcaggtca aggagggagg 3240
aaaggtgaag gaagggatag agggaatggt gcgctttcat gtatttattc agcaaacact 3300
taaggaagac ttacgtgcca gtgaggattt ctggacctgg cgttgtgctg gggatatagt 3360
gacaagacat tccctttgag ttgaggagta aaagacaggt tatggagaat ctatggggtc 3420
ttttggacac aaacaccagg gagggaagga caggctgcct ggaaggggac agggctgggg 3480
tgggggggata gccatgacgc aggacgagct gtggtcctga ccagttcccc caggagggca 3540
aaggacattt tcaggaacat ctttagggct cacggtagcg acaaggattg cctgcctcct 3600
atctgtgctg aagcctcctt ctgatcccag ggcgcttggg ttcttgggtg caggggcggc 3660
agcgtctcac tggggcccta gtcatgtaga gcatggagtg gcgtctggca tggtggcagc 3720
ccctggaggc aggtgccttc cccagcctcc tggcaggcct ccctagggcc ttccgaagcc 3780
tgtgcctact cctatcacct ttgccctctg ctctgtgatt acagtgacgc cccgctgcat 3840
ttaccctgtt ccctggccct ctattttgtc tccctgggct ggtgctgtaa cccaggtcct 3900
catcatctgt gttggttctt gctgggcttc ttcactcttc cccactggtc tttctctacc 3960
ctcgtttgta aatcctgctt tgctagacac agcaaaagtc tcccggagtc aagggcatta 4020

gctctgaact gtgtcggagt cgctgggggg ctttgcagac tgtgcctgac ctcatgctct 4080
ttctctccac ccacgcagct ttgttcagta ttccctgaac ccaggctgct ttgctcatgg 4140
attttccttc cccactatca gcagtgaatt agtcagggga ttgtctctgc attgcacatt 4200
catttaacaa ataactccga ggagcctgag caggaggtgc cgcttgccac gggagtcctc 4260
agtgactgcc tttgttagga ctggtggcca aatggtgccc agagtgcaca gtcagatgcc 4320
cagaggcctc ctctttctga cacctgtccc aggtttgtac tcagccaggt ccatgacctt 4380
gtgggacttt gcttctgtct ttatctctga cgatggtgcc tcaaaacatt gcacctactt 4440
tgattccttg tatctctcgt gtaatcctat taatgggctc ctgtggggag cctagcccag 4500
cttcactttt aaatgtgcgg tgggtaatgt taaaacacag cagggaactt tgagggtgaa 4560
gtgtgttaaa agataaactc aggtgcatta aaattgtagt ttttttaggc tgggcgcagt 4620
ggctcatgcc tgtaatccca gcacgctggg aggccgagga aggtggatca cacgaggtca 4680
ggagatcaag accgtcctga ccaacatgct gaaaccctgt ctctactaaa aatacaaaat 4740
ttagctgggt gtggtggcgc atgcctgtaa tcccagctac ttgggaggct gaggcaggag 4800
aatcgcttga acccagaggt ggaggtgcag tccagcctgg gcaacaagca ctaaactcca 4860
tcttaaaaag aagtcatagc gtttttttgga gcagaccaat tcatgaatca gcaccaaacc 4920
acaagcagta tgagctccac ctagaaggtg tgtgggaaag ccattttgta agacgtgttt 4980
gcacaagcaa gacaaaggaa gcatttgatt ggtcagagtg gaaagttccg gaggttagtg 5040
ggtggtttct ccttggaaca atctctcatt agaagtcaat tggcagtttc tgattgcttt 5100
aagtttcctt ccactgttgg gtttccatgt gcttatgtaa gacacaagac accatctcag 5160
cctgatggcc ttctaattaa tttgttttaa taagggcata ctgaatcctt ggcccttgcc 5220
tcatagattg gaataatctg agaaaagaaa aacttctatt caaaagtgta ggatgatgaa 5280
aagtgaaata atgatagcca agcacttatt ttaagcatgc tttgttggca gctgtgcaat 5340
ttggccatca tttcaaaagt cttttacaga aggaaaggct aggtgaaagg aacagaccca 5400
aatctaggaa gtgccataga tttcaatggt ctcacgttgt taggggaaaa aagcaataaa 5460
gtcgatcatt aaaattaacc actctcagtt ttaaacattt aaaaattatt aaatccacca 5520
tgacctaaat tactcccata taaccaaatt ctctatttca ttgttcccaa acttggggggt 5580
acaacattat aggttgtcct gaaagatgac gtccagacgc tttgagagtc aaatcatttg 5640
ctgaataaat gccttgttgt tataagtaaa ccaagggatt attattccag tttgttggga 5700
aaatatttca agacatactt ctttagttcc ctgtgtgaat taaacaggca tagaacttgc 5760
ttgcaagacg acaaaaccca aacatcttac tttaaacgct ttaaagaata gtcattccaa 5820
gtagttgctt gcattttcat cctctggaat gcatactgtg ctatattttg agtctttccc 5880
ctttggagca ggtcccatcg agcatatgag tatgaacagt tatttaccag ttacttgggt 5940

ttatactgta tgggtaattc tgcccgtttg aatagctgga gaaatcactc aagtgctgct 6000
taggagcaga cgtttgagaa gctcagagtt ctcagtgccc ttgtgtgggt gccaccccac 6060
agccctcgcg gacagtaaga atggacatgg aggcagcccc gatgattttg ttcttttcaa 6120
taatccattt ttcttcctgg gtgcttatag ggcactttct ttttccttag atggataatc 6180
tgtgtttgct tgtttattaa tttcgcctga ttttctccaa gcctttttag gaaattgaat 6240
ttagcccagg aaattgaatt tctccgtgtc tctcattctg tctgttctga atgttaggtg 6300
ccaggactta agatacttgt caatattgta tgtgaacaaa cttacaggtg aaatacatgc 6360
ttgttgtaga aaacttagaa aataattcaa ggaaagaaag aaaagttgcc tataatttca 6420
tcttagagat aaaactgacg atattgtggt attatctttt tcccatatct ttcattgctt 6480
ttaataagtt tttttctaag cagtagagat acacacatac acatacaaca tcttgtaacc 6540
tacttttagc agttggtaag tatttcttct gacttaatag aaacacttaa tgaaaatgac 6600
tgacagtgag gatttcatat gcttgggcca gacattccca tgtctcaacg ggtcctcatg 6660
gtgtggtggc tggagcacct gggaaaaggg acattgccag ccagccctgc ctctgtgatg 6720
ttctctggcc atgtcaccat attcctgtga gcttccacgt tctgatctgt aaaataggga 6780
taatagtcac agttttgcag tgtaaaaaaa gggcatttag tataggatta ggaattatta 6840
gaaacacttt gataaggaac tataagatgc acttttatat tatatctaag cttgtaaact 6900
taaccaaaag gcaactgcaa atcgtcctgg ccattaaagc accaaataaa ttctggcaca 6960
ttaatactct cagttcagct ccatcatcaa acatgtctcc cattcctggt ggcctcacag 7020
acaccaaggt ttgcagtttt catgggttat aaaaacaact tgatgaaaag caagtcttat 7080
tctttttttt tttttcaatt gagatggggt ctcccagtgt tgcctgggct ggtctcggaa 7140
ctcctgggct caaacagtcc tccttccttg gcctcccaaa gtggtgggat taccaccaca 7200
ccaggccaaa gccttgttct ttgcctggaa agaaataaga taaaatagga aaattataac 7260
atgacagaga acaaagtgtc agatttaatc ttacatttac tctgattaat cagcaaacag 7320
aatctctgtg tagcttccca gacctgactg ttttgtattc gttagcttgt aatcagaaca 7380
gaaaacagat atatgacaca ttactcaaca tcctttttaa tccaagtgct tcatagtttc 7440
tcttaaaaat ggtttccatc ttaagggact gtcaccttct tgttcaatcc tttattttct 7500
caaagagagg gaatagttgt ttcccattgt tcctctatgg aagggtgatt ttgggtgcga 7560
atgggatggt gtaggagaag ggctgtgag tgcccatgcc tgtcctaact ggggtagtct 7620
gggccgcaga ctgatgtgct gtgagttcca tgagtaattt atgtccaggc aggtgcaccc 7680
accttccttg ctatctgaaa ttggccccat ggccctaagt aggaccctgt catttatgcc 7740
tggggtccgc tgcactctct caagtgctgt cgtccctcta gagtctaaac aaagactcag 7800

gtaaaagcca tgcccagaca ccttcttccc gaccccaccc cacgcgagtt ccaaattggc 7860

cacagtcaaa ggaaaatctt caccgacaac aatggatttc caaggaagta aactgagtaa 7920

agctaaaggg aatggcccgc aagaagaaaa gccttccagg agatgctgag aggggcagag 7980

cgcttccttg gagcggagtg ctaggtccaa gtcagagctc tctagaggga gggaagtcag 8040

gaagaagatc tcaccttctc ctagcaactg gaggcagagg cagcactccc ctcctctccc 8100

ctgtccacca ctctgttgac ttcagcggtt ttatgacagg cctataggca tcagggttac 8160

tggtgtcccc ttccagggca ggaatcagag aacaccagaa gagactggtg tcgatctttg 8220

taaggctcgg tttccagtcc ttgcagttct cagacattca catgagacaa aaaagaggga 8280

caaatcttat cctgaggttc tttgtgaatg tcttatccag ggcatgtgct agagcagaag 8340

tcattcaaaa atgcagctac gcagaagcac gtcctcactc ccagagcaca gccaaaccta 8400

gaccgcagca gctgcagcca tgtagcacca gtgccctgag caagttgatg tcaagttgat 8460

gtctccaaaa tataacacac ttgtgctaat tcagactaat tgtcagcagg cttttctgga 8520

aagagccagg tagtgcatat cttaggcttt gtgggccaag atactgggta ggtatttacg 8580

atgagagaaa acacattttc acaaaagttt cagtggtaaa ttcaaaatat attatcttag 8640

tgcatttttt gtaatatatg tctaatgaga agaatagaat tcttttcttg gtgagggata 8700

acatttcact tattgggatt caaagttagt gtttcctatt atcaacatct attgtaaatg 8760

ttcctctcag tgttgatctg tcatgagatt ttacgtattt tatctctata aatggaagcg 8820

ttgacatcag tccacatgta tatgatttta attgagcatg atcatcactt ggaaggcact 8880

catggaatcc tgttagattc ttctcttgat ctttgccttt ccatatggta ttacattaca 8940

gattcatatc ttcctatgga agattagatg gcaactcttc cactgcacag ctaaatggat 9000

tttgaaatat ggaaatccag gttcacacac tgtatttaaa cttagaattt atatccactg 9060

caaactcatg tgagcatgga gagctcactt cttttaattt taatagcatg gaaagtacag 9120

aacgcagttt gatatgactt gtgattggaa caatggcagt tgtcatcaaa atgactttaa 9180

cacagtatga gttttgcaca agtgctgttt tgccttgcag ctttaggttg aatttattat 9240

caagtctgca gtaaaagcta cttaagccct tcagaactca ataatggtgg tgagggcagt 9300

tcttgctcag aaaagtttca gtctcagctc tgagctcaaa ataatgtgat gaaactttat 9360

taagccatca aactggtgta tttatgtggg acaaatcggc atagtcagct tatttgtgac 9420

aaaaagttag agaactgatg atagttgagt ccatgagagg gattgtcatt cactgttgac 9480

actatgggct taatcacaca caacagattt tctgcaaaag acctgctgat acaaggaata 9540

gcatgaaaca ccttacattt ttacaagttt gtaagtttgt aatttgtttt ttttttgtcc 9600

catacatttt tatcaccatc atttgcaaca gcttagcaga ttccactttg gattctacta 9660

aattcgtgtt ttctcaacat ctttgaaaat attctcaccg gtagttgttc tgcacagact 9720

atctaaagag cctaaccctt cattcacttc agccttgatg tgaactcctc aaataaacaa 9780
ctgagccata tcagtaacat ccatagactc acccagagcc gacaaagact gctcatcatc 9840
tgccttgctt tttcttttt aattattatt ttgagacagg atctcactct gtcacccaag 9900
ctggagtgca gtggcatgat cctcagctta ttgcagcctc cacctcccag gctctgatga 9960
tcctcccacc tcagtctccc aagtagctgg gactacaggc atgtaccacc acacccagct 10020
aattttcata tttttgtag agagggggtt tcgccatgtt gcccaggcta gtcacagcca 10080
tctacctgcc ttggcctccc gaagtgctgg gattacaggc atgagccact gcatctggtc 10140
ctgccttgct taaatagact gttgagggtg ctcccagtgt ccttaactcc tctagcaagt 10200
gttctcccca aaaggctaat tgtgagaaac aagtgtgtat tctctggaca cgcctctttg 10260
gttgctgctg ttgattaact caccgttgat gcacagcttt ccttgcttca ctaaccaatg 10320
agccgcccga taacctactt tggttgcagc ctcattttcc tttgttttgt gaagaaatcc 10380
tgctgccttg gcctctgtgc cttaaatgtt ctagtttttc tgaccagtgc ttttctgtga 10440
gttgggaata ggctggtgag tgcagagtct gataatatta tcctctatta tattcttttg 10500
acacagttct agtgtcattg ccagctgacg tttttcaggt cagaattcaa cccacaactt 10560
agaagaaaat tgggagaaat ggatcaaggt tcgctggatg tgattctgat atgccagagg 10620
gaacgtccac cacatacgca gaatgtttcc tgggagttgc ttctctcaca ctgcatttgt 10680
ccattgttta aatgggtgac atccaatgca gaagtgtctt aagccatttt caattctgtg 10740
tatttcaacc ttgctaatat tttcccttcc tctttttcac cttgccttcc tgttatccag 10800
tattgaattt aggatggatt tttttcaggt ccagaggccc gacctcttat gaactgagtc 10860
ctttctattt taatagacat cttgctaaac caaatccagg ccttacattc ccttttatg 10920
tcttgctctt tttttctgta tttcctcaaa cttagaattt gaacactttc tcacctctat 10980
cctgcagttt gcctagtggg aaataacttg aggccgtaac tctcagggaa gttttctctc 11040
aactttctca taccggtgtc ccttttgctc tttgccgcat tgagaatctc ctaagctgac 11100
ttcagaagtg ggctgcttgc tcttggtact tctcagcttg gtacactgag ggtagagtg 11160
agagccactc tgtcttctgc cattaattct gtccatccgg gtctaacaca aggcctcctg 11220
ttctggttga caaatgggcc gaggcaggaa ggatgtctga tcacaagaca agattgcctc 11280
ctgtttttaa gggagcctat gacagtccaa gaagcccaca cgtacctctg tcaaaaccaa 11340
attctgcttg ttccacagga aatgttggta tgcaagcact tgcagtgtgc ctacatccca 11400
aatgccagcc catggtaccc attgtctgcc cggatctgaa tcatgattcc aagacgcaga 11460
gagaaattgc tgggattcgt tgttcttatt cattgcttgg caacactgca gcttccagga 11520
gaatacccac ctcactgatg acacaacatc tgaatgcaca cttactggcc aacacgtgct 11580

ttgctgtaaa tcagtatgaa gtcttcctag gggtcctggc ctgaatgaga tgagaacctt 11640
ggaggctgct tttgactcat gtgaatgtct tccccaggag ttaagtggga gtgctgggga 11700
gagagtggct tgaggatgca cagccagcat gacactacta ttatgtgatt actgtcgacg 11760
ttcataacca taactaatga ctaattatga ctttaccctg gcgcacatgg tcagaatgga 11820
aacaaataac aagctttaca gtttttcctg ctctattaag gtataattta caaataataa 11880
aattcaccca ttttaagtct gtaatttggg ctggtcgcgg tggctcaggc ctgtaatccc 11940
aacactttgg gaggtagagg caggtggatc acctgaggtc gggagttcga gaacagcctg 12000
accaacatgg agaaaccccg tctctactaa aaatacaaaa ttagccgggt gtggtaacac 12060
atgcctgtaa tcccagctac tcgggaggct gaggcaggag aatcgcttca acccgggagg 12120
cggaggttgc ggtgagccga gatcacgcca ctgcactcca gcctggacca caagagcgaa 12180
actccatctc aaaaaaaaaa aaaaaagtct gtagcttgat gaattttggt gattgcatat 12240
agttgtgtaa tcatcaccgc aaatcaagag gtagaacagc tgtattcctc taaaaaactc 12300
cctttgtgtc attgtggcca gtctccaccc tgactctatt tttgccttgt ctggatttcg 12360
taagtctaaa ttcagtttca caatctattt ttttttattt tctttattat tatacttgaa 12420
gttctagggt atatgtgcag aacgtgcagg tttgttacat aggtatacac atgccatggt 12480
ggtttgctgc acccattaac ccgtgatctg cattaggtat ttctcctaat gctatccttc 12540
ccccgatccc ccaccccaca acaggcccca gtgtgtgatg ttcccctccc tgcgtccatg 12600
tgttctcatt gttcaactcc cacttatgag tgaagacatg cagtattttg ttttctgttc 12660
ttgtgatagt ttgctgagaa tgacagtttc tagcatcatc catgtccctg caaaggatat 12720
gaactcatcc ttttttatgg atgcatagta ttccatggtg tatatgtgcc acattttctt 12780
tatccagact atcattgatg ggcatttgtg ttggttccaa ggctttgcta ttgtgaacag 12840
taccacaata aacatacgtg tacatgtgtc tttatggtag aatgatttat aatcctttgg 12900
gtatataccc agtaacggga ttgctggggc aaatggtatt tctagttcta gatccttgag 12960
gaatttggtt ggtaggctat taattactgc ctcaatttca gaatttgtta ttggtgtatt 13020
cagggattcg acttcttcct ggtttaatct tgggagggcg tatgtgtcca ggaatttatt 13080
tatttattct agatttttct agtttatttg catagaggtg tttatagtat tctctgatgg 13140
tagtttgtat ttctgtggga ccggtggtga tatcctcttt atcatttttt attgcatcta 13200
tttgattctt ctctcttttc ttctttatta gtctggctag cagtctatct attttgttga 13260
tcttttcaaa aaaccagctc ctagattcat tgattttttt gaagggtttt tcatgtctgt 13320
ctctccttca gttctgctct gatcttagtt atttcttatc ttctgttagc ttttgaattt 13380
gtttgttctt gcttctctag ttcttttaat tgtgggttag ggtgtcgatt ttagatcttt 13440
cctgctttct cttgcgggca tttagtgcta taaatttccc tctacacact gctttaaatg 13500

tgtcccaaag attctggtat gttgtatctt tgttcttatt ggttttaaag aacatcttta 13560
tttctgcttt cattttgtta tttatccagt agtcattcag gagcaggttg ttcagtttcc 13620
atgtagttgt gtggttttga gtgagtttct taatcttgag ttctaatttg attgcactgt 13680
gatataagag actgtttgtt atgatttcca ttcttttgca tttgttgagg agtgttttac 13740
ttccaaccat gtggtcaatt ttagaataag tgtgatgtgg tgctgagaag agtgtatatt 13800
ctgttgattt ggggtggaga gttctgtaga tgtctactag gtccacttga tccagagctg 13860
agttcaagtc ctggatatcc ttgttaattt tctgtctcat tgatctgtct aatattgaca 13920
gtggggtgtt aaagtctccc actattattg tgtgggagtc taagtctctt tgtaggtctt 13980
taagaacttg ctttatgaat ctgggtgctc ctgtattggg tgcatatata tctaggatag 14040
ttacctctgc ttgttgcatt gatctcttta ccattatgcc cttcttcgtc tcttttgacc 14100
tttttggttt aaaatctgtt ttctcagaga ctaggattgc aacccctgct ttttgctttc 14160
catttgcttg gtaaatattc ctctatccct ttattttgag cctatgtgtg tctttgcacg 14220
tgagatgggt ctcctgaata cagcacaccg atgggtcttg tctctttatc cagtttgcca 14280
gtctgtgtct tttaattggg gcatttagcc tgtttacatt taaggttagt attgttatct 14340
gtgaatctga tcctttcatt atgatgctag ctggttattt tgcctgttag tttatgcagt 14400
ttcttcatag tgtcaatgat ctttacaatt tggtatattt ttgcagtggc tggtaccggt 14460
tgttcctttc catgtttagt gcttccttca ggagctcttg taaggcagtc ctgatggtga 14520
caaaatctct cagcatttgc ttgtctgtaa aggattttat ttctccttca cttatgaagc 14580
ttagtttggc tggatatgaa attccagata tgaaagaaaa gaaaattctt ttctttcaga 14640
atgttgaata ttggccccca ctctcttctg gcttgtaggg tttctgccaa gagatctgct 14700
gttagtctga tgggcttccc tttatgggta accagccctt tctctctggc tgcccttaac 14760
attttttcct tcatttcaac cttggtgaat ctgacaatta tgtgtcttgg ggttgctctc 14820
ctcgaggagt atctttgtgg tgttctctgt atttcctgaa tttgaatgtt ggcctgcctt 14880
gctaggttgg ggaagttctc ctggataata tcctgaagag tgttttccaa cttggttcca 14940
ttctccccgt cactttcagg tacaagagtc aaacgtagat ttggtctttt cacatagtac 15000
catatttctt ggaggctttg tttcttttca ctctaagtct tctcgcttta tttcattgag 15060
ttgatcttca atgactgata tccttttgcc acttgattga tttggctatt gatacttgtg 15120
tatgcttcac gaagttcttg tgctgtgttt ttcagctgca tcatgtcttc acctgttctt 15180
ctctaagcag gttattctag ttagcaattc ctctaacctt ttttccaggt tcttatcttc 15240
cttgcattgg gttagaacat gctcctttgg ctcagaggag tttgttatta cccgccttct 15300
gaagcctact tctgtcaatt tgtcaaacta attctctatc cagttttgtt cccttgctgg 15360

caaggagttg tgatcctttg gaggagaaga ggcattctgg ctttttgaat tttcagcctt 15420
tttgcactgg tttttcccca tctttgtgga tttatctgcc tttggtcttt gatgttggcg 15480
atatcgatac tatttttttt cggtttgtta gttttccttc taacaggccc ctctgctgca 15540
ggtctgcctg agtttgctgg aggtccgctc cagaccctgt ttgcctgggt atcaccagtg 15600
gaggctgcag aacaacaaac attgctgcct gttcctacct ctggaagctt catcccagag 15660
gggcacccgc cagatgccag ccagagctct ccagtatgag gtgtctgtcg gcccctactg 15720
ggaggagtct tccagtgtgg atacacaggg ggtcagggac ccacttgagg aggttgtccc 15780
ttatcagagc tcgaactgtg tgctgggaga accgctgctc tcttcagagc tgtcaggcag 15840
agacgtttaa gtctgctgaa gctgcgccca gagccgcccc ctcccccagg tgctctgtcc 15900
cagggagctg cggttggctc tgcccagttc gaacttcctg gggctttgtt tacactatga 15960
gggtaaaacc gtctacttaa gcctcagcag tggtggatgc ccctcccccc accaagctct 16020
agcatcccag gtcaacctca aactgctctg gagcagcgag aatttcaagc cagtggatct 16080
tagctttctg ggctccatcg gcatgggacc cactgagcca ggcactggag agaatggtct 16140
gctggttgcg aagactatga gaaaagcgca gtgtctgggc cagaatgcac cgttcctcct 16200
ggtacagtct ctcatggctt cccttggcta ggaaagggaa atcccttgac cccttgcact 16260
tcctgggtga ggcgatgccc caccctgctt cggctcgccc tccgtgggct gcacccactg 16320
tccaaccagt cccagtgaga taagccagtt acctcagttg gaaatgcagg aatcacccac 16380
cttctgtgtc gatctcgctg ggagctgcaa gaccggagct gttcctattc agccatcttg 16440
cccgcccacc cttcacaatc tattaatata tagagagcag taatattgcc caaaaaggga 16500
aagttaatct ttgtggttaa tcctctcccc tgagtctcat tttgcctttt ctagaatttc 16560
ataagtctaa attggattta tttcattggt aacagtgcta ttcatgttgt ctttctttct 16620
tctcgggtca gttttggtca tctgtgtctt tcaaggactt tgtctatttc atcaagttgt 16680
taaatttatt ggtataaggt tgttcataat agttccttat tatctgagtg gatcacgtga 16740
gcccaggagt tcaagaccag actagagatc agacatagag agaccctgtc tctacaaaaa 16800
aaagttttta aattaaccag gtgtggtggt gtgtgcctga ggtcccagct actcaggaag 16860
ctgaggcagg aggatcactt gagcctagga tgttgaggct gcagtgagcc atgattgccc 16920
cactgcactc tagcctgagt gacagagcaa gaccctgtct tgaaaaaaaa aaaaaaagag 16980
ttccttatta tcctcttaag gtctgtagga tatgtagtga tgtcccctct ttcaatgtgt 17040
tatacactta tattaaaaag aaaattcaaa cagagaataa cattttgcat tctctttata 17100
attttctaat ttcttgtcct tttattttaa ctccttaaac ctttagtaaa atgcacctaa 17160
gccagtgtgg tacaacagaa cgtgatcttt cttttttttt ttttaattat actttaagtt 17220
ttagggtaca tgtgcacatt gtgcaggtta gttacatatg tatacatgtg ccatgctggt 17280

gtgctgcacc cactaactcg tcatctagca ttaggtatat ctcccaatgc tatccctccc 17340
ccctcccccc caccccacaa cagtccccag agtgtgatgt tccccttcct gtgtccatgt 17400
gatctcattg ttcaattccc acctatgagt gagaatatgc ggtgtttggt tttttgttct 17460
tgtgatagtt tactgagaat gaacatgatc tttcaattgc cccgatttac atttaagctc 17520
agccactcac tagcttgtgg ccttgagcaa cttactttac cactctgagt cttaatttta 17580
tcctctatca aatgtggata agagtgcctc tgcctggtta gctgagctgg gggcaaggag 17640
ttgctggggg aattgggggt ggggctagca ggtgaaagtg ctcagtgaat ggaagccatt 17700
gcaatggtga tggatgatgt gatgatgtgt tccaaatctt attacttaga ctaggactag 17760
ttaatggttt taggatagtg gaaatcttga gagcctcttg aagcccctag ttttctcaaa 17820
ttcagtgtat tctgttttat attgcatgta agtagaatct ggagtttgta cactgtggta 17880
aacatgattg aaacgtggta atgctttatt gaaaaatctg ctttttggct ggatacagtg 17940
gctcacgcct ataatcccaa cattttggga caacaaggca ggaagatcac ttgaggtcga 18000
agtttgagac cagcctgagc aacagattga gacttggtct ctacaaaata ataataataa 18060
taataataag tgtggtggca catgcttgta gtcccagcta ctcgagaggc tgggacagga 18120
agatcgctta agccctggag ctcaaggccg cagtgagcca tgatcaagcc actgtactgc 18180
agcctgggca acacagcaag aacctgtctc aaaaaataaa attaaaatca aaatccactt 18240
ttagcttaga tataataaat tagtataaat gttactgagg atgacatttg tacaagaaag 18300
taagatttaa aacccaaatc atttaagata ggattacaga aatgattatc tttaattttt 18360
taaaaaattg tgcctgtttc ttgtttccta aggtgcttaa tttacccatt tctgatgcga 18420
ggaggaaagc ctatgccact gttggcgtgt acaatggcga ttatgttctg tacctgtaac 18480
ggctatttgc aaagcagata cttgagccat tgtgcagtgt atgctgatga ctgggtaaca 18540
gatccccgtt ttctaatagg tgagtgtcca cagcagtgaa ctccgccttg ttcacatcat 18600
tgcttttata ttgatgtccc agtggtttct aatgagaaag tccaagcttc ctgtgagaac 18660
aaagacaaca gagaaagcag cagcaccccg gagtcccatg ggagagccca gctgtctcag 18720
cacagctggg ggcagagagg tgacgcaggt gctgggcacc ttcccccgca cgggcaggca 18780
ctgaagcaca ggtgcctggt caggccaaga acccaaaaat agcagtgggc tgtagcagaa 18840
tgagcaggga ctaaagattg agaccctttg gccactcatt gaccacataa tctcctgcgg 18900
ttccttccat gacatgagga aaacccagac tcggttagct tcctacagtg cagcttctgt 18960
gattctatga acttgaatca acttttaggt ctatgaagtt gttgaatttt tttatttgta 19020
tcagttaatg tgaattaatg tacattttca acctctaatt ataacttttt aagttctaag 19080
caaaaatgaa aataaaacag ccatcaaaaa tatttgcaag agcattaaaa attaacactc 19140

tggctgggtg cagtggctca cactgtaatc ctagcacttt gggaggctga ggtgggcaga 19200

tgacttgaat ccaggagttc aagaccagcc tgggcaacat ggcgaaactc cagctctaca 19260

aaaaatacaa aaacgtagct gggcatggtg acacgcacca tggtcccagc ttactcagag 19320

gcactgaggt gggaggatca cctgaggcca ggagatcaag gctgcagtaa gccatgattg 19380

tgccactgcg ttccagcctg ggtgacagag tgagactctg tctcaaaaaa aaaaaaaaaa 19440

aaaactcagg gatcttatat ataagaaagg ttcttctata tatttgttta gtctatccta 19500

gcatttagat attaaaccat actgaggtgt tgcttacaat ggtagcatgc taaaagtctg 19560

aagtcagtct gtcagatttg caaactaccc tgcagtgctt caccttaagc tacataaagt 19620

gaccttacat tgcattgtgc tttttctcca tgtgtattaa tttataatca tagaaataaa 19680

ctaacacttg gatagtactt tgttgctgag agatcccttt cacatttctc agccatgttt 19740

tttgttggtg aattagaatt ttattccaag gtttcatcat acctgaggat cgttacagtg 19800

agacacctgt ccctcctcta caaaaggagg gttggctgag aaggcgtttc aagatccttt 19860

tctagctcca gagtctgtgc ctccatgaaa tgctagacta aattctttgg tactaaactg 19920

ttgtacacta cccccaaata tcttcttggt ttacctgctt tttgactact ttattttctt 19980

ctgccctacc tgtcaagagg aagaacaggc ttttgagttc ctgtgcagat cacacgattt 20040

atgcctcccc atgttctctg aggcagaaag gatgtcccta agatgtgaaa ggtcagaaga 20100

gttacacagc tggcccaaga catctagacg cagtgaatgg cagagcggtg atttccacca 20160

cctcaccaag ctgagcttcc gcaagtggta acaaggaata agaggaaggg gaatttcttt 20220

cttttttttct ttttaactaa agatacagat tttaaatgtt tgccagagag tctcatggga 20280

ctcacctcta tggtgaacaa caacagaaga accctcctgt ttaaagtagc tccgcagatg 20340

gttcgcatca cgtagaggtg actgtgaagc tggtcagaga ttccaaacac caatcaattg 20400

tagacagacc tcttgacttc tctacaatta aacagaagga tttgaaaagg aaaaaatatg 20460

ccaagtggaa gacaatctag gttacctttt gtgtgttata tttattgcct agagtttatt 20520

gtttttgtga ggaaacagac gtgaaattac ataaataagt aattatgcct actgtagtta 20580

ccatgaagta ataagcaaat aataataata tatttttttt tttgagatgg agtttctcta 20640

tcgcccaggc tggagtgcag aggtgcaatc tcggctcact gcaacctctg cctcctgagt 20700

tcaagagatt ctcctgcctc agcctcccga gtagctggga ctataggtgc atgccactat 20760

gcccaactaa ttttttttat tttttttagt aaagacaggg tttcatcatg ttggccagac 20820

tggtcttgga ctcctgacct caggtgattc acccgcctca gcctcccaaa gtggtgggat 20880

tacaggtgtg agccaccatg cccagcaaat ttctaacaaa aggtttttca aaacagcaaa 20940

agtggttaat ctaaaatatt ttatagaaag attttaagtt ttttttttgt ttgtttgttt 21000

gtttttctt gaggcagcct gtcatccagg ctggagtgca gtggcgtgat cttggctcat 21060

ggcaacgtgt gcctcccagg ctcaagcaat tcttgtgcct caacctcaga gtagctggga 21120
ttacaggctt gcgccatctt acctggttaa tttttgtatt ttcagtagag atggggtttc 21180
accatgttgg ccaggctggt cttgaacccc tggcctgaag tgatccaccc accttggcct 21240
cccaaagtgc tgggattata ggtgtgagcc actatgccca gccagatttt aagatcttat 21300
aaatggaatt aaatatttag cttagatgaa cacaagtact agttttcttc caatctaagc 21360
aataaaggga gcttgtctcc tggaagaaac ataaagtaac catgttattt agacaacatg 21420
attcctttct agaatctcat attggaagcc aaattaccta ggggtgaaat actaaaatcc 21480
caaaaaggta gcaaatcaac tataaatgta tggatccaat aaatgactta caaaacgtat 21540
tataaaactt ggtatgaact gctttgctta ggaaaaaaaa ataaaagctg attacagtgg 21600
caaaatcaga aggaagaaat cttgaaaccc ttgctataga ccagaaggtt ttccatgtcc 21660
tttttctaac tgtgaaaaag tattcaatgt cactggtgat gactcagata ataatgatgc 21720
cccacgtgta cattgattta atattgaact gaatgccagc actaagcagc atctgtcttt 21780
cccattttaa aaaagtatat cagaaccatc aagttaacct ctttttatca atatttcttt 21840
tataaaacac tgttttggcc ttttgccttt tcttgatgga attaattgat gtgatactaa 21900
caactctcct ttaagtgatg aaaaatccct gcggattcgt aagaatagga gagaaaactg 21960
gggataccgt gggattctgg ggagaaccat gcaggcggcc acgggagact cttgtcaaga 22020
accgaggagg aaggactgac cgagcaatgc tgattgatga gcctgcagga attctgctgt 22080
gggggaaaca tagatgcgca ttaatttgtt taaatgaggt tgtaaataag agaagaccag 22140
agatctactc ctaggaactg ccacccttca aggggtgacg tacagccagt ggtgacagag 22200
cagcacagga gcatcacccc tcctggttca tgtggtggag atggcagcat tgcctcatgc 22260
agattttgtg agaaataagt gggatgcgtc tgtctggtgg gctgactccc tgatgctccc 22320
tggaaaggag ccgtgtgccc aagtcagatt tctgtccata cttgtcatcc tgaagatgta 22380
gattgtgatg aacaggtcaa agtattttga gtacattaga tgatgtattt gtattattag 22440
gaaaacaaag ccctgcttgg catacctgac aacagtcctt aatggttttt actaccagtt 22500
atggctaata tgttacacta acaatggtaa tctgaagggt tgcaataata ctgttcagtc 22560
aggctggggc tcgtagtgaa attttacggt ttattagcca taatcatctt gcaatttttt 22620
tcctttaggt tttggcttgt ggttaacggg catgttgata aacatccatt cagatcatat 22680
cctaaggaat ctcagaaaac caggagatac tggatacaaa ataccaaggg gtacgtacag 22740
aaagtgaaga atttctgtga aagttgcttg ccatggttcc tggctatttt agtgttgcca 22800
gctctaagaa gtagtagcgt agtagttatt agctacaagt tttcagtgta agtcatcatt 22860
attaataaca agtgctattg ttattgtctt attagaataa caaaagaccc aaatctagct 22920

aactgaacag ttattaaaca aagcagaaac tccagttata tctgtgcttg ggaagagagg 22980
atgaggaaga atttatgatt gacgaggagt gtctgacact tttgtttcta gcagtataat 23040
ggaatagata ctttgaccct cctctgaaaa cagtttaaaa cattggaata ttttgaaaat 23100
cttcttaaac gcatcagtat actatcaaga aaggaactaa tcatcagagg acaagatcta 23160
ggtgaatgtt ggaatctgga gcgtgtcttt tgagaatttg tgatgaggcg ccgaccacca 23220
tgctggttta taacccagat tcgcactact tgtatagtat gaaaaatctc aagccataaa 23280
ttaatttccg tggcctgggg tagtggtgga ctctcctggc cagcaaaagc ccagatcccc 23340
aaggggatgt gcattcaacc tgagtctcaa agaacccaat gtgccacgtt ccacagagta 23400
agaggcacgg tcacaaggta cagaacccac aaggaagtac agcaccatga gggagagcgt 23460
aattcaacat gaaagatttc agatgttaga attaatggaa acattataaa gtgagtactt 23520
gaaatgcttt ttaaaaggag tagattacaa ttaggagtaa gaagcaatgt accataaaag 23580
gactagaaac caatagagct tcagaaaaaa ttttttaaag tacataaatc taaaacttta 23640
atggagaaag ggcgtgacat cacttagttt gaaattcttc cacttatgtt cctagaaacc 23700
atatgagaat agcaacaaaa acaaacaatg ctacaaacct caaattactt aaactgtgac 23760
cctgagaacc tggaatcagc aaagccagct ccagacgtga tggtagtgtg atagaagtgg 23820
cagagaagag aaaacatgac agagtcctag acaggctgct gatcacctat tcagcccctg 23880
ggtggattgg ggaagaatcc tctctttggt gttgggctta tgagcaaact gcaccctcat 23940
ggggtcatcc caaggagccg cttcctctgg caggtacagt ggctgggaga gcacagtgta 24000
agctggatgg tggccccctc gcttcctgcc cagcaccctt gcatggggaa cagcatgatt 24060
ccaaattaat cggcaagtgt tcatcccctg aaggagaggg ccctgtcctg aggaggagct 24120
gccatgagcc agactaagaa gggacagggc acagtcctgg ggaaggcaga atgaaaaggt 24180
gcagaagata ccagaagcca cagactggca gatttgagaa acaccacttc caaaagaaag 24240
ggcatcccca gagacggcag gatcccatgg gagggccctc ctgggccagg ctggttacgg 24300
aaaggcctgc ggtgccttat acacagggtc ctgtccactg ttccacagta gttgagatgc 24360
cacaaaaact tttcagcccc tgtccctcca tttcctgctc tcagtgtctg atccttgttc 24420
taatccaaca tattcaaacc tgaacaacaa aaaaggtaca atccccaaat ctgtgcagtc 24480
ataacattag gacagcaaaa aagcagcaaa acttacctga gcagaaacgt gtttcacaaa 24540
gcaggggaaa attgtaatca aaattccaac atgaatttta aaatgtgaat ggaggccaag 24600
cgtggtggct gacgcctgta atcccagcat gttgagaggc cgaggtgggc agatcacatg 24660
aggtccagag ttcgagacca acctggccaa catggtaaaa ccccatccct actaaaaaca 24720
caaaaattag ctgggcgtgg tggcacgtgc ctgtaatccc agctacttgg gaggctgaga 24780
cacgagaatc acttgagtct gggaggcaga gggtgcagtt agctgagatc atgccactgt 24840

attccagcct ggacgacaga gtgagactct gtctaaaaaa aataaaaaaa ataaaatgtg 24900
aatggagcag ttgcagagtc tatacccatc agctaaagaa cacctgcgac gaacccgcaa 24960
tctacaaact tattacctgt cgcagtaaag ggagacctct ccctgtgatg tttccccaca 25020
gcatgtagcc gccttcctga agctagtaat caacccagct tcctgacggc ctgtggccat 25080
caatggcatc tctgtgaaca gacaggccaa aggaaatgca gaagcttggc tgtaactctc 25140
aggcagcagg aggtgacacc agacatcggt gcagatgaca tgggacacat gagacttagg 25200
gaagaaaatg cttctaaaat tcttaatttc tcctctccaa gtactaacca ggctcagcct 25260
tgcttagctt ccaagatcag acaagatcaa aattctttat ttctaatcat ttaaaaaaaa 25320
aaacaaacca cagaagcatg ttagaagata gaaatggaaa agcaaagaat atgaaaccta 25380
gactgggtgc agtggctcac acctgtaatc ccagcacttt gggaggctga ggcaggagga 25440
tcacttgagg ccaggagttc gagaccagcc taggcaacat agccagactc ccatctccac 25500
aaaaaaattg aaagaaaatt agccaggcat ggtggtgcac acctgtagtc ccagccactc 25560
aggaagctga ggtgggagga tcacttgaac ccaggagttt gtggctgcag tgagcctcga 25620
tcataccact gcactccaga gggagaccct gtctcaaaaa aaaaaatctt tttttttttt 25680
ttgagacggg gtctcgctct gtcacccagg ctggagtgca gtggcgcgat ctcggctcac 25740
tgcaagctct gcctctcggg ttcacgccat tctcttgcct cagcccccccg agtagctggg 25800
actacaggcg cccgccacca cgcctggcta atttttttgta tttttactag agatggggtt 25860
tcaccgtgtt agccaggatg gtctcgatct cctgacctcg tgatccgccc accttggcct 25920
cccaaagtgc tgggattaca ggcgtgagcc accgtgcccg gccaaaaaag atcattctta 25980
ttgtgaatga tgttttggtg atagctggag aaggtaggga aaatatatcc atcctaggca 26040
cattgaagtc catgaagaag aaatccaaag cattggaaca gaatatattt taaaaactaa 26100
ttcaagaaag cttttgtgaa gtaaaacaag acacaagagc gtctgccgtt tgcaggtacg 26160
caccctatac cggaggaagt ggacaggaaa cagtgacgat catctgcacg tgccatgagt 26220
ggcttgtaag ggaaagttcg ttagattctt cagggcagcg ttccgtgcca gggtcttaga 26280
ggcagcaaca cgagaggccc aaggaacgtg gaagccaaaa tattatccta gctttggagt 26340
ccctcatgta aaatctgtca ggttttgaag actcaggtgt agggagtctg agtgctgtgc 26400
cccatgaaga ctccaccatg gtcggctttg tgaccagctc ccttcgctgc agactcttaa 26460
ttttgcaggg cgttttcatg tgacacattg atggtttcta atacacaggt gtgccgtcta 26520
catccagtga ctccgtgagg tctttcagaa acgcttcgca ggactctgtt aatcatggtg 26580
ccataactgg ttagggtcaa aggagcaagt gacaaaatga acaaataaag agataaagag 26640
aggtgcacag gtgtgacgtc catagtcagt acatacaggc tgaactaggg cttaggacaa 26700

gtgctgtggg ctgtgtgtga caaccagttt gtggttttgt aatgtggcat ctgtaagcag 26760
cctgtgtctc aagaggacat tttttagcag attggcatct gcaggttttc cagtctaatt 26820
ccatgcttac cagtccaccc atacggttcc accacaccgt gtgtcccaca ccagacactt 26880
aagagagaag gttcactgag caggcggcat gcaggaaggc atcgtggccc cagccctgcc 26940
cacaggctgt ctgccctttg ggtgactggc atctgcaggg tatggtacaa aggtccttcc 27000
acgtctggga ttcacctgcc cagggctctg atgaaataca gatcatgatt aacgtaatct 27060
tgtagaaagt tgaagcagtt acagaaatgg gtgaataaga aagtgaaagt gactaagctc 27120
catggcccag aaggaacagc gctgtgaatg ctttgggtcc attcttgcag gtgcttctgc 27180
aggtgcacat gcccacacat ggcgaatgac attctattta ttgggcatta agtttgatat 27240
ttttatgttt tatattgtca ttaaaaagtt cacttgtcaa tgaatattga tatttgtatt 27300
agtctgtttt cactctgcta taaagaacta cctgagactg ggtaatttat gaagagaaaa 27360
tgtttaattg actcacagtt ccgcatggct ggggaggact caggaaactt acaatcatgg 27420
cagaaggcaa aggggaagta aagaccttca catggtggca ggagagagag cgagtgaagg 27480
gggatctgcc tcactctttc aaaccattac atgtcatgag aattcactat cacaagaaac 27540
cgcctgcatg atccagtcac ctcccactgg gtcccttcct tgtcctgtgg ggatcacact 27600
tcaagatgag attgggtggg gacacagaat caaaccatat cagtattatt ttacaattct 27660
gaattatttc aagcttacag aaaagtagta gagagttcat tattgcaaac gtctacatac 27720
cccaccttcc ttaagaaaaa gaaaagcact gtgctgtctc gggctcagca aaaccatttt 27780
ctttcttttt aaaaaaatag accatctcag gctgggtgtc atggctcaca cctgtaatcc 27840
cagcactttg ggaggccaag agaggtgggt cacctgaggt caggaattcc acaccagcct 27900
agccagcata gtgaaacccc atccctacta aaaatacaaa actccaaaaa aaaaaaaaaa 27960
aggtagccaa aagccctcat gcatgcatgt aagttgcagt gtgctcagag atgtagtctc 28020
taccctatgc tgcagtattt gctgaaattg gtcttatgac ttagggcgta ataggtttgt 28080
cttttttttt tttttttttt tgagacagag tctaggtctg tcacccaggc tggagtgcaa 28140
tggcacgatc tcggctcatt gcaacctctg cctcctgggt tcaagcgatt ctcatgcgtc 28200
agcctcccga gtagctggga ctacaggtgt gtgccatcac acctggctga tttttgtatt 28260
ttcagtatag acaaggtttc accatgttgg ccaggctggt ctccaactcc tgacctcaca 28320
tgatccacct gcctcagcct cccaaagttc tgggattaga ggcgtgagcc actgcgccca 28380
gcctaatttt tttttaaaaa aagctctgtc cttacatgcc ttcagaattt tctcagctct 28440
gcagacatag tgtccagttg gaacctgggg cagatcacac caggcctctc taaacacact 28500
tgagtaacca caaagctaca gcctgagggt gagtcccaag tccaaggagg ggccttctgc 28560
cctccctctc tgctgggacc cttctcccca cccctcatct ggtctttgga gtgtgccagc 28620

cattgctgtc tgcctcaggg cctttgctgc atctcttctc ctgcctggag cactcctgag 28680
agcacctcca gcacctgcca ggctggccct cacctgcaag ccttgaatcg accctcacct 28740
tccctgtggc ctgttgccag ccactctgtg atagtgacct cctccacccc atcccagctc 28800
cggggccccc tcactctgcc ctgcccctcg ctactcctcc ccctccatga gctgtgtgct 28860
ctccttgttc atgacactgg agttcatggt tctagatgtg gggagggtgt ctgtccaagt 28920
gcctgggcag ggcatggccc ataatacgga ctcagtgcat ttgtgaagat gtgacgaatg 28980
aatgaatctg tgcttgaaaa tgaatatctt tgtccctcat tgctctaggg ttctatctag 29040
gtccatttta catggtttac tctgtattct ctgcttgcat gatccatcag caggtgcact 29100
ggtttattgg tgtcttccac tgtaactgta tgtgtgtgta ttcacataga caggtattga 29160
tatacatatg tagttgatga tcagaagttg tgatatgaat tagtaaacca tgtaacgctg 29220
gatatgtctt actgattaac attctgtaag gataatattt ttccgttctt gaatttatgt 29280
tctccaggta agtattcact agcatctctg aagtccgtat ttcattttgt agtaaatgca 29340
ctactttggt ctgtgttttc ttctaggagg cttatttgaa tacgtaactg cagccaacta 29400
ttttggagaa atcatggagt ggtgtggcta tgccctggcc agctggtctg tccaaggcgc 29460
ggcttttgct ttcttcacgt tttgtttttt atctggtaga gcaaaagagc atcatgagta 29520
agttttaaaa cacttttacc atttgtaatt tgttctttga ctatattatt accatttttc 29580
aggctagatt tttgaagtgt taatttaaat cgctgaattc cagtttattc tgtgctttgg 29640
ccaaacaagt acaaaaccaa atagctgtag aatcacccag ctctagggaa taatcaggtc 29700
cacatcagca cagttgttct ctataccacg tccttagcct ctctgaattg acagtgctga 29760
attcagtggt caagttagac accaagcagg ggattcaggg ctctgaatgg gaaatactga 29820
ttaaaacaga tccctgactg agcaatagcc aatggccagc ttgcgaatgc tgcaggttga 29880
ggaagtcctg aagaggtagg tgaatctcag agcccagcga cactggcaga actcatacac 29940
acaagagctc tccagctatg tggtggctta ggccatggct ctgaacctgg ggtccttaat 30000
gggctacaga agctctgcat gccccctaaa atgacatcat tttccagaga ccgggtcctt 30060
ggcttttatc tgatccccca aaaggcttgc gagccatttg accaacacac tcataaagaa 30120
atcaaaggga ctgggcgcgg tggctcatgc ctgtaatccc agcactttgg gaggccaagg 30180
ccggtggatc acttgaggtc aggagttcaa gactcacctg gccaacatgg tgaaatcccg 30240
tctctactaa aaatacaaaa attagtcagg cgtggtggcg ggcgcctgta atcccagcta 30300
ctcgggaggc tgaggtacga gaatcccttg aacctgggag gtggaggctg cagtgagccg 30360
atatcgcacc actgcactcc agcctggttg agagagtgag actcagtctc aaaaaaaaaa 30420
gaaaaaagga aaagaaattg aaggatgtca aggctgaagt gaatttagag aacatctttc 30480

taatccaatg gatgaggaaa ctgaggttca gggagcagag tctgaggcca gccaggagcc 30540
ccatctcatt gacgggaaga gatctcagga gcaacgtggg atctttacct gccctgacat 30600
tggcgcgtgt ccctgacaat aaatgatgga gactgggctt agcaagcggc cagcctggta 30660
tgcacattta ctatgtcccc tgagccaccc acttcatcca ctcaaaacat ctaatcgaaa 30720
tctccaatgc tgctgatgct acgaggtgca aaaatgtata actctgtcct acttcatgga 30780
gtttatcatc tttttggga ctaaggacag agagaaaaag ctaaaagaag aatagattga 30840
tatggtatag tgggaagagt acctaggtgt acttcccagc ctcaccacga gtttgcagat 30900
gcaaaggatg attattggag ataagaagaa gaaaggtcat atctgaagta tctctttgtc 30960
taattttttt tttaagacag catctcactc tgtcacccag gctggagtgc agtggcacaa 31020
tctcagctca ctgccacctc tgcctcccag gctcaggtga tcttcccacc tcagccttcc 31080
aagtagctgg gattacaggc atgcaccacc aggcctggct aatttttat atttttgta 31140
gagttgtggt ctcactatat tgcccaagct ggtcttaaac tcctggactc aagtaatcct 31200
cacacctcag cctcctgaag tgctaggatt cgaggcgtga gccaacgtgg ctggcctctt 31260
tgtctaatac ttgaagaagt gatagcataa atgtgagctc taagagcagc gccccgcatt 31320
cttcgagaca ctccatcgtg ccagtggcct ggtattttca cgcatagaca gtgaggagag 31380
agcgagatca gagagccacg acctacctaa atcccagagc tgctgagaaa aacagagggg 31440
tctgcggaaa gttgagtgag tgccgaaggc agagagggtt tgagaaggag gctcaatgcc 31500
aggcatggca gaaaacactg tggccagga atgggccttc tgtgctgaga actgtgtctg 31560
ccagaaggtc atatgagagt cctaagccct ggcacctgtg aaagcgacct tactggagaa 31620
cgttatcagt tgagatgaga aggggaaacg tggatagacg tacaccaggt gatcacacag 31680
agtgggaagg ccatgcgaag atggacacag aggcgggggc gatgcagcca tgaacccagg 31740
aacacgtggt gccaccagag ctgggggaaa tgggactgga accttccctg aaccttcaga 31800
gggagcgtag ccctgctgcc accttgattt ccaactcctg gcatccagaa ctgggagaga 31860
atcagtggcc gttattataa gccacccagt gtgtggttaa ctttgttcta gcagccacag 31920
ggaactgata cgccagcctt ccagccattt aaaattgact gggaaagtaa gggcaggagt 31980
atgattggtg aacccctctg ttttctcctc cgccagatga ctttgttgtc accggtttgg 32040
ttgaaataca acgttatctc tctaaatata cattctacaa tctgacttcc tcccctctgc 32100
gcccgctgct cccagcactg cacctggcct gggcctcact tcccggtgaa gatgccatcc 32160
ttttatattc ctaatatttg atgtgaagaa gtgagtaatg gctcatttct cattaaaaga 32220
aacaaatgag ataacactga cgtttaaaaa aacacggatc agagtaagat tgaacatggg 32280
cacaccaagt cagtggcaaa gatgttttct caaatgaaat caggaaacag gaaagcccct 32340
ctcagtattt gaatatgaaa catatagaaa tggaaaataa ataattcagt tatctaaatg 32400

aaagtctttg ctaatgaaaa cccaaagata gtctttgcag aagagttcat gagttgaaag 32460
agctctttgt cattcatttg ctgtttaaat tggtgtcagt gtgaaaccag ttttaagccc 32520
tgaagaatta atcaattcag ttttgcagaa gtatgttcag aaatgtagga cttacaagct 32580
gagaataaaa taaaaactcc tttttaaaaa atattttcat aaacccaact aattaagcca 32640
tcaaataaaa gtattccaaa agaaaacatg ccaaggatct attccttgca ctccatttat 32700
aatgttcttt tttttttga gacagtctcg ctctgtcgcc caggctggag tgcagtggcg 32760
cgatctcggc tcactgcaag ctctgcctcc caggttcacg ccattctcct gcctcagcct 32820
cctgagtagc tgggactaca ggtgcccgcc accatgcccg gctaattttt ttgtattttt 32880
agtagagacg gggtttcacc gtgtcagcca ggatggtctt gatctcctga cctcgtgatt 32940
tgcccgcctc agcctcccaa agcactggaa ttacaggcgt cagccaccgc gcctgactgg 33000
attataacgt tctaaaaaca aaattgtatt caatgaaatc tttctataat aatcagtatt 33060
taaattcata aatgattgca ttgatagtta atgcattgat ttctaggtca gaaacagtca 33120
actgtgctgg gttgctaaca aatattctct taaagggcaa aatttaaagg tattctaagg 33180
agtaagaaaa atttaatcat ttaaaaataa atattttatt tctatataat ggtttctgtg 33240
aaatacactt aacctcttca gccaacatcc cttcattgtg tgagaacacc ctgacctcac 33300
acatgttctt tctgatgtca cggagcccag ccttgctgcc cagcctggtg ggggggcgcg 33360
ttttctcggc atcctgcggc agtcctgtgt ttcccatctc ccacctgccc tctcgtgttt 33420
gcccatttca ggctctcctg ctgtcatgta ctctgccctt gccttttcgg gccagggtgc 33480
ccatcttccc acgggcagtt tctgggagag aggcgctcac ctgctatgtc actgtgcggc 33540
ggccagtgcc gctggcccat ggggaagctc ttcagcactt gcaagtgctg gatggcctgg 33600
atcccagcag gcctgtgaac accttcagcc tctaaccagc gaacatgttt gcaatgcact 33660
ttcgcaggtt tgacaccagc tgttaggctt ggccacgttg tgtgttgggc actcgtgggt 33720
ccatgcgcca ccactgagcc acgtagctgg aaaggctgca gtctcgaggc ctcatgccag 33780
gggaagtgac gcttgatccg gaagtcctga ccgacttctt agaattgttc ttattacatg 33840
ccttttttggg gacacagaac atacaaaagt aaacgtggcc gcccagtatg ttcgaattct 33900
ctatttgtgg attccggtgt taaacttaac taagaccagc agggttttgc caactccctc 33960
tcagtttctc gtcctcatat gtttctacca cattaacaat gagctaattg ctttctatca 34020
tgatttttag tctgttgttt aatctttctg tgtgtgttta tagaaactcc ctttctccat 34080
cttgtcaaat tatttctaat ttttactcca tgcacttagt tgccaccttt cccaactggc 34140
tattatcccc aaaacaaatg agcacgcctg cattgataca atttatatca aaacggaaaa 34200
taatgtttgt ttcgagcaca aggctgaatg gtggacaatg ccatattgca tttccccaga 34260

tttcaccgaa tccttactaa gagcgttcat gagcctcctg gtatgcagct gggtgcccac 34320
tctacacacc ctgggctagg ccagtagttc ccaactgcag gctggagaaa actgctggag 34380
tcaccagcag gtattcagga agcacctact tggtcctgcc cctcctccag cctgcatgct 34440
ttccccgggg gctgaaaaga cagctccgca atgtgctgat cagtattttc atcaaatgtc 34500
agtaattttc cttccagata gaaaggtgaa ttctatgggc tacttcttct gaatcacact 34560
ttatgattag atatatttaa ttcttcatga taataggcaa aattaaacta taagtatgaa 34620
atgccaaagt ttttgttact acatttaaaa aactgagtac tcttttgtaa tgaaaaatat 34680
tgtcactttt gttagcattg gttaaatgtc taagcgacag aattatttcc tttttaatt 34740
ttttttctt aggtggtacc tccggaaatt tgaagagtat ccaaagttca gaaaaattat 34800
aattccattt ttgttttaag tgcgtttttc atgaaattat cttcaacttg aagctttcca 34860
atggcgcttc tctatggact ttgtaaataa gttatatctt tgtaattttc ctgctacttt 34920
atcattttca agatgtcctc taggaatttt ttttctagta attttgcaat ctacctaata 34980
agtacctaaa tacgctgaaa tggaggttga atatcctact gtgtaacagg tcagaatttc 35040
aagctctggg taataactgc tgatattttt tctaatttca aatttacctc ttttggctat 35100
gtcttgccaa gtgtgtatga gactagactt tacaactgtc tttgatggca ttttcagaac 35160
aataaatgtc acaatccctt ctatagcccc ctacagtgat ctcttcaagg tcaactgcag 35220
tgttgcttcc ctccccctat agggctggaa tctgtctagg agccctctct cggaggccac 35280
agaggctggg ggtagccatt gtgcagtcat ggcccggggg aaacttgcca accttcgtgt 35340
caggtgctgt gtgtaagtgg agaacttggg gatagaggag gaagctcctc gtggcccttc 35400
caaggtgagg caaaggcatc tggacttgtt ccagcccagc ccaccgggtg acatcaccgg 35460
gcagggaggg gtgctggtgg tggttcatac ggagtaagct gctctgcctg tgtgagtggc 35520
tcctgggccc taaacaggca cctttaggcc atgggtcact caccgtgagc catcaatgtg 35580
ctctggtctg acatggtttc tctctgtctt ctagtctaga cctagttttt ttgttctgtt 35640
ccccacgtat ggatatagta gagattgttg tctgtgaaat ttctcttttg tagattttga 35700
gttttccctt gtagtgtaaa gaatgatcac tttctgtaac aataacaaga ccacttttta 35760
agatttatcc tgtttgttct ttgttgattg aaacataata attgttaaaa ttctctacag 35820
ccttcttttt cttccatagc taatcttcct tctaatagtt tttgctttct gttttgctgt 35880
tgttgctttg caaagctttc ccctcatagc ctgtacctgt tatcaatata aaataatctt 35940
cctgttgaat gcttcatgac ttgaattcta ctttgataaa aacattgcca tactgctttt 36000
tatcttgatg aattcatctg gcattgcttt gccttatcat ctcatctgga gtttttaaat 36060
gccatttgtt tcagttgtct ttaacaacat aataaataga ctttgccatt taa 36113

<210> 87
<211> 22
<212> DNA
<213> Artificial

<220>
<223> Synthesis

<220>
<221> misc_signal
<222> (1)..(22)

<400> 87
ccactgttgg catgtacaat gg 22

<210> 88
<211> 27
<212> DNA
<213> Artificial

<220>
<223> Synthesis

<220>
<221> misc_signal
<222> (1)..(27)

<400> 88
gttaaccaca agccaaaacc tattaga 27

<210> 89
<211> 3286
<212> DNA
<213> homo sapiens

<400> 89
gcgtgtcggg cgcggaaggg ggaggcggcc cggggcgccc gcgagtgagg cgcggggcgg 60

cgaagggagc gcgggtggcg gcacttgctg ccgcggcctt ggatgggctg ggccccccctc 120

gccgctccgc ctcctccaca cgcgcggcgg ccgcggcgag ggggacgcgc cgcccggggc 180

ccggcacctt cgggaacccc ccggcccgga gcctgcggcc tgcgccgcct cggccgccgg 240

gagccccgtg gagcccccgc cgccgcgccg ccccgcggac cggacgctga gggcactcgg 300

ggcggggcgc gcgctcgggc agacgtttgc ggggaggggg gcgcctgccg ggccccggcg 360

accaccttgg gggtcgcggg ccggctcggg gggcgcccag tgcgggccct cgcgggcgcc 420

gggcagcgac cagccctgag cggagctgtt ggccgcggcg ggaggcctcc cggacgcccc 480

cagccccccg aacgctcgcc cgggccggcg ggagtcggcg ccccccggga ggtccgctcg 540

gtcgtccgcg gcggagcgtt tgctcctggg acaggcggtg ggaccggggc gtcgccggag 600

acgcccccag cgaagttggg ctctccaggt gtgggggtcc cggggggtag cgacgtcgcg 660

gacccggcct gtgggatggg cggcccggag aagactgcgc tcggccgtgt tcatacttgt 720

ccgtgggcct gaggtccccg gaggatgacc tagcactgaa aagccccggc cggcctcccc 780
agggtccccg aggacgaagt tgaccctgac cgggccgtct cccagttctg aggcccgggt 840
cccactggaa ctcgcgtctg agccgccgtc ccggaccccc ggtgcccgcc ggtccgcaga 900
ccctgcaccg ggcttggact cgcagccggg actgacgtgt agaacaatcg tttctgttgg 960
aagaagggtt tttcccttcc ttttggggtt tttgttgcct tttttttttc tttttttcttt 1020
gtaaaatttt ggagaaggga agtcggaaca caaggaagga ccgctcaccc gcggactcag 1080
ggctggcggc gggactccag gaccctgggt ccagcatgga ggtggtggac ccgcagcagc 1140
tgggcatgtt cacggagggc gagctgatgt cggtgggtat ggacacgttc atccaccgca 1200
tcgactccac cgaggtcatc taccagccgc gccgcaagcg ggccaagctc atcggcaagt 1260
acctgatggg ggacctgctg ggggaaggct cttacggcaa ggtgaaggag gtgctggact 1320
cggagacgct gtgcaggagg gccgtcaaga tcctcaagaa gaagaagttg cgaaggatcc 1380
ccaacgggga ggccaacgtg aagaaggaaa ttcaactact gaggaggtta cggcacaaaa 1440
atgtcatcca gctggtggat gtgttataca acgaagagaa gcagaaaatg tatatggtga 1500
tggagtactg cgtgtgtggc atgcaggaaa tgctggacag cgtgccggag aagcgtttcc 1560
cagtgtgcca ggcccacggg tacttctgtc agctgattga cggcctggag tacctgcata 1620
gccagggcat tgtgcacaag gacatcaagc cggggaacct gctgctcacc accggtggca 1680
ccctcaaaat ctccgacctg ggcgtggccg aggcactgca cccgttcgcg gcggacgaca 1740
cctgccggac cagccagggc tccccggctt tccagccgcc cgagattgcc aacggcctgg 1800
acaccttctc cggcttcaag gtggacatct ggtcggctgg ggtcaccctc tacaacatca 1860
ccacgggtct gtaccccttc gaaggggaca acatctacaa gttgtttgag aacatcggga 1920
aggggagcta cgccatcccg ggcgactgtg gcccccgct ctctgacctg ctgaaaggga 1980
tgcttgagta cgaaccggcc aagaggttct ccatccggca gatccggcag cacagctggt 2040
tccggaagaa acatcctccg gctgaagcac cagtgcccat cccaccgagc ccagacacca 2100
aggaccggtg gcgcagcatg actgtggtgc cgtacttgga ggacctgcac ggcgcggacg 2160
aggacgagga cctcttcgac atcgaggatg acatcatcta cactcaggac ttcacggtgc 2220
ccggacaggt cccagaagag gaggccagtc acaatggaca gcgccggggc ctccccaagg 2280
ccgtgtgtat gaacggcaca gaggcggcgc agctgagcac caaatccagg gcggagggcc 2340
gggcccccaa ccctgcccgc aaggcctgct ccgccagcag caagatccgc cggctgtcgg 2400
cctgcaagca gcagtgaggc tggccgcctg cagcccgtgt ccaggagccc cgccaggtgc 2460
ccgcgccagg ccctcagtct tcctgccggt tccgcccgcc ctcccggaga ggtggccgcc 2520
atgcttctgt gccgaccacg ccccaggacc tccggagcgc cctgcagggc cgggcagggg 2580

gacagcaggg accgggcgca gccctccccc ctcggccgcc cggcagtgca cgcggcttgt 2640

tgacttcgca gccccgggcg gagccttccc gggcgggcgt gggaggaggg aggcggcctc 2700

catgcacttt atgtggagac tactggcccc gcccgtggcc tcgtgctccg cagggcgccc 2760

agcgccgtcc ggcggccccg ccgcagacca gctggcgggt gtggagacca ggctcctgac 2820

cccgccatgc atgcagcgcc acctggaagc cgcgcggccg ctttggtttt ttgtttggtt 2880

ggttccattt tcttttttc tttttttttt taagaaaaaa taaaaggtgg atttgagctg 2940

tggctgtgag ggtgtttgg gagctgctgg gtggcagggg ggctgtgggg tcgggctcac 3000

gtcgcggccg cctttgcgct ctcgggtcac cctgctttgg cggcccggcc ggagggcagg 3060

accctcacct ctcccccaag gccactgcgc tcttgggacc ccagagaaaa cccggagcaa 3120

gcaggagtgt gcggtcaata tttatatcat ccagaaaaga aaaacacgag aaacgccatc 3180

gcgggatggt gcagacgcgg cggggactcg gagggtgccg tgcgggcgag gccgcccaaa 3240

tttggcaata aataaagctt gggaagcttg gacctgaaaa aaaaaa 3286

<210> 90
<211> 21787
<212> DNA
<213> homo sapiens

<400> 90
ctggaactcg cgtctgagcc gccgtcccgg acccccggtg cccgccggtc cgcagaccct 60

gcaccgggct tggactcgca gccgggactg acgtgtagaa caatcgtttc tgttggaaga 120

agggtttttc ccttcctttt ggggtttttg ttgccttttt tttttctttt ttctttgtaa 180

aattttggag aagggaagtc ggaacacaag gaaggaccgc tcacccgcgg actcagggct 240

ggcggcggga ctccaggacc ctgggtccag catggaggtg gtggacccgc agcagctggg 300

catgttcacg gagggcgagc tgatgtcggt gggtatggac acgttcatcc accgcatcga 360

ctccaccgag gtcatctacc agccgcgccg caagcgggcc aagctcatcg gcaagtacct 420

gatgggggac ctgctggggg aaggctctta cggcaaggtg aaggaggtgc tggactcgga 480

gacgctgtgc aggagggccg tcaagatcct caagaagaag aagttgcgaa ggatccccaa 540

cggggaggcc aacgtgaaga agtaagtatg gcttgctggg gtcggggccg ggccgggcca 600

gtcacggtgc tgatggttct gtcttccttc cttctctcct ccctccctcc cttacttcct 660

cttaacaccc tgagctggac ccgtctggcg cctgtgtcct ccgtgccagg gagagcgtgg 720

ttgggggcct gcgttacgga ctttcactca ggcaaggcca gttgtcgcag cggggcgtgc 780

gtttgcatgg gctcttggac tccagttaaa atgccctggt agcgaaaccc tcctgagaag 840

ggagcggccc ccaatcccct aagactagcc ccttggctcc cccagctgtc caaggagcag 900

aggcgcccag tggaatcagc ctgtgtttgt ttgggccccg agagtttgtg tgcggccgcc 960

aacacgtttt ctgcgcagtg tgtggccgtt accggggcca ggcgaaatgt gatttgttta 1020

tcctgtcaga ggggaaccct gggctgccaa aaataactgt ttgcaccggc ttatcagtca 1080

gcaggaggga aacgtagcct ttcctcattt gccagggatg tgacgctgga agcatccctg 1140

gcccccgggg ctggaagccc tgcccgaggg ggactgtgcc tccctcccga attgcatccg 1200

gaagacctta cttttccaac tgacttcttc aggcacgggg ctgccgctgg gcatcccgga 1260

cgcctctgca tctgtgcgcg gagaagctcc tacctagggc agcactggcc ggcctgagcc 1320

tctcccagct ggtgggggtg gccgggggtg tccctgcctt atcgcagcca gacacgctgc 1380

acctgccgcc gcctggcggg ccctgcccag gccctgctcc tttcccagcc ttcttaactt 1440

tcaaaacttt gcctcctgtt tccaagaaag gaccttatct gctgggctag gcctcgaggg 1500

actggcaagg acaccctgcg cagggcacca tgaccttgga aggaggtccg gggaggggct 1560

gctgcggtgt ctgctctcac cttcctcctg ctcaggggcc ctggggctcc tgccgtgtct 1620

gctctcacct ctctcctgct caggggccct ggggctcacg tacatttttt tttttttttt 1680

tttgagacgg agtctcgctc tgttccccag gctggagtgc agtggcgcga actgggctca 1740

ctgcaagctc cgcctcccgg gttcatgcca ttctcctgcc tcagcctccc gagtagctgg 1800

gactacaggc acccgccacc acgcccggcc aatttttttgt atttttcagt agagacgggg 1860

tttcaccgtg ttagccagga tggtctcaat ctcctgacct cgtgatccgc ccgcctcggc 1920

ctcccaaagt gctggtacta caggcgtgag tcaacgcgtg cggccttttt tttttttttt 1980

tttttttttt ttgagacgga gtttcactct tgttgctcag gctggagtgc agtggagtgg 2040

tcttggctca ctgcaatctc cgcccctgg gttcaagtga ttctcgtgcc tcagcctccc 2100

gagtagctgg gattacaggt gcctgccaca gtgcccagct aatttttttt tttttttttt 2160

agtagagacg gggtttcacc atcttggcca ggttggtctt gaacttctga cctcgtgatc 2220

cacctgcctc agcctcccaa agtgctggga ttaaaggcgt gagccaccgc gcccggccag 2280

cacgcttctt aatcagcatg caggctgtga catgcggaaa ccaggggcac aggctctgaa 2340

gtttgtactc tggagctgga gcagtcaggg ttaaatggga gcggctggga agggtgtgtt 2400

tccacgaggc ctttctgatg tctcagtgcc cagtctggac ccgggtagaa ggtgtggcca 2460

gtgccttgag tcagtggagc agttgcctgt gcctggtctc tgtgagatgg gttggtcagg 2520

ggctaggcag agcctgtgcc tggtctctgt gagatgggtt ggtcaggggc taggcagagc 2580

ctgtgctggg ggtagcaggt gccctctaaa ggggccagtg acagtctctc tcccagcagt 2640

gagaaaccca cgcttagggg ccgggcgcgg tggctcacgc ctgtaatccc agcactttgg 2700

gaggctgagg tgggtggatc gcctgaggtc aggagttcga gaccagcctg gccaacatgg 2760

tgaaacccca tctctactaa aaatacaaaa gttagccgag tgtggtggcg tgtgcctgta 2820

atcccagcta ctcgggaggc tgaggcagaa cattgctgga gcctgggagg cggagcttgc 2880

ggtgacccga gatcgcacca ttgcgctcca gcctgggcga cacagcgaga ctctgtctca 2940
aaaataaata aataaataaa taaataaata aataaataaa taaatctggc ttgagttttg 3000
acaaatgtat atgcagggtc aatgggaaga cagacgctgg agggtggaag cctgactgtg 3060
agagtgagcc ccctgctttc ctgaacagtc agtggaggag gtcctgggag ctcaggtgag 3120
cacctgaacg gtagggcaga gcctgagggt cttggtggag acaggcaaga aggttgcagg 3180
aacctagtct ggcccagtgc ctgggagaac gggagggaac acggaggcag ggaggcagag 3240
gggttggcag gacctggact ggactgtgcg tcgcagctac ccacagcttg accccactgc 3300
cttgaagtga gtgatgcacc ctcactagtg ttcagcgtcc aatttcatcc tggccctgag 3360
tgtgtaacgg ctttgccctt gcggggcccc ctgggatgag ggtgagccc tgtcccttcc 3420
tggtcactgt ctgccatcag gagctgcccc gcttacagct gtgattcagg agccactgca 3480
tttcctccag ccccctgggt gctgccaggg ggtcctccta ttctgggagc tggtgtgttc 3540
tgactggagg ctgccatgtg cagagtgctg tcaaggagaa ggggtgtcca ctgcccagcc 3600
ctgaggccca aggccttggc cagccttgct gccagcgctg cagggaaaaa gcctcctttg 3660
tgtgtgggaa gtttaataaa ctccgctcag attgtgtctc gcagcgagtg tctggaacct 3720
tccagacaag cctcaggcgt ccggtcctcc agttggtgtg gaaagcgtgg gcgatcacca 3780
agggggtgg gttggggcag atggagccgg cgtgagtccc gtctcttccc ttccttccca 3840
gaaaggcagc cctggagtcc atgccttgtc ccgctctcac cggcaaaaag tataatctta 3900
ttagaaatag gaaagttcca aaaagcatca atgagttaaa aagagggctg ggcatgttcg 3960
gtcatgcctg taatcccagc actttgggag gccaaggcgg gtggatcacc cgaggttggg 4020
agttagcctg accaacatgg tgaaaccctg tctctactaa aaatacaaaa ttagctgagt 4080
gtggtggcgc acgcctgtaa tcccagctac tctggaggct gaggcaggag aatcacttga 4140
acccgggaag cagaggttgc ggtgagccaa gaccatgcca ttgcactcca gcctgggtaa 4200
caagagtgaa actccgtctc gaaaaaaaaa agggctgggg ccgggcgtgg tggctcacgc 4260
ctgtaatccc agcactttgg gaggccgagg cggtttgatc acaaggtcag aagtttgaga 4320
ccagcgtggc caacatggtg aaaccctctc tctactaaaa atagaaaaac tagccaggtg 4380
tggtggcaca tgccaataat cccagctact tcatctgagg tcaggagttc aagaccagcc 4440
tggccaatat ggcgaaactc cgtctctact acaaatacaa aaattagcca ggcgtggtgg 4500
cgcgcgcctg tagtccagct actggggaag ctgaagcaag gagaatcgct tgaacctggg 4560
aggcggaggt tgcggtgagc cgagatcgca tcattgcatt ccaggctggg caagcctagg 4620
agagaagagc gaaattgtgt ctcaaaaaaa caattaatta aaataaaatc agtgccatag 4680
gctgtttctg gtaggcagtg ggttcctagg gtggagcagg gaagggctgg aaagggtcgc 4740

ctgtgcccag cccgactccc cgtgcttctc gggggacagc acggctagct gctgctgcgg 4800

agcccacagt ggggttctgg ggggggggt gcaggcagaa ggccgccatg cctccctccc 4860

cctttccacc ccagtgagag tgggctcgaa ggcagggagt ctgcccgcct tgtggagcct 4920

ccgcagaggc acccagggct gccgtgagcg cacagcctgg cggtgtccca cggccctgcc 4980

ctttcctgtc acttccacct gttgcaaaac actgtgcttt cgtatctttt ctttctacct 5040

gtttggaaat gtgaaaatca ctgctgccgt aggaaacggg cccagatccg ggctgtggct 5100

ctcccatccg caccgccccc gcggtgtcca gccaggaggt gggaggtggg cggcactcag 5160

tgtgcctggt tgcggcgatg attggcagat gcttggacct atggtaaaga ggattttcgc 5220

gagctgggag tcagtcctgg actttgtagg tctttacatc ccaggcggcc tttatggggt 5280

tccagcatct gctgaggccc ctgcgccagc catgtgaggc ctgggtgtcc tcgaggcctc 5340

tgtttgtgcc cctctctggg cagcgggagg gagagctctg agacccagag ggagctcagg 5400

ggctgggcag ccgcagaaca gcgggggtaa tgggtccact ggggggttctg ggctgtgcac 5460

acagtgggct tccggtgccc agccagctgg ggcaggcctg gaatgttggt tcctctctca 5520

ggatctgggg cgctgctgta gctgtcgggg aatcagtgag gcggctggac ggcccgtata 5580

tcccacaaac gccaccgtat tgctcaaact ggactcaatt ttcttaacac cttttttttt 5640

ttttttttt tttttttgg agacagggtc tcactgtatc gccgaggctg gagtgcagtg 5700

gcgcaatcat agctctgggc tcaggtgacc ctcctgcccc caaggtagct gggactgcag 5760

atgtgcacca ccacacccga ctagtttttt gtagagatgg ggtttcgcca tgttgtccag 5820

gctggtctca aactcctaga ctcaagctat ccgcctgcct gggcctccca gagtgctggg 5880

attacaggta tgagccactg cttcgccacg ttcaccttta tttacttatt tatttatttt 5940

tgagacggag tctcgctctg tcccccaggc tggagtgctg tggcacgatc ttggctcact 6000

gcaagctccg cttcagcctc ccaagtaagc tggaactaca ggtgcctgcc accaagcctg 6060

gctaattttg tttttgtatt tttagtagag acggggtttc accgtgttag ccagtatggt 6120

ctcgatctcc tgaccttgtg atccgcccgc ctcggcctcc caaagtcctg ggattacagg 6180

cttgagtcac tgtgcccggc ccaccttttt attttttatt ttatagagac aagggcttgc 6240

tgtgttgccc aggctggtct tgaacccctg accttaagta atcctctctg gcctcccaaa 6300

gtgctgggat tacaggtgta agccatagta ctgggcctaa agttcacctt tttttttttt 6360

tttttttttt ttgagatgga gtctcgctct gtcacccagg ctggagtgca gtggcgtgat 6420

cttggctcac tgcaagctct gcctcccggg ttcacaccat tctcctgcct cagcctcccg 6480

aatagctggg actacaggcg cctgccacca tgcccggcta atttttttgt atttttagta 6540

gagatggggt ttcactgtgt tagccaggat ggtctcgatc tcctgacctc gtgatctgcc 6600

tgcctcggcc tcccaaagtg ctggggttac aggtgtgagc caccgcaccc ggcaaagttc 6660

acctttttaa agtgtgtaat tcagtggcat atagtacatt gacagagttg cacaactatc 6720
acctctatca gcttccagaa cattcttgtt accccagaag gaaaccccgt ctctgtcagc 6780
catcactccc cattccctcc ccagtcccgg cacccacgca ttcccttcct gcctctggat 6840
tggcctgtcc tgaacatttc acacgagtgg tgtcacacac cgcgtggcct tttgcgtccg 6900
gcttgtctca cggagtgtgc tgtcctcaag gggcatccgc gccgtggcct gggtccgaac 6960
ctctctgctg ttcacggctg cgtcactttc cagtgtgtgg agggccacgc tgcgtttgac 7020
tgctcatctg tggctggaca cttgggctgc tcccgcctct gcctgtgaat tgtggctgct 7080
ttggtgcacg tgtgctcagc cggcgtccct ctagaatcct tgagggcctt gtttcagcca 7140
catcaggagg gattcaggga ccagggcacg cgggtctgga gctgggtgtt cctgggtctc 7200
catgtgcctc ccagtcctgc tctgagccgt gggaggccgg ggcgagggcc aggcccatct 7260
cttccctgta tgatctgttt ctaggcagac ggaggctcct agccccacct ctctgtgagc 7320
ttgagtggcg tttatgactc tccttcctat ttcctgtcct gctaatcaac aagtcagcta 7380
aacagtccat gctgcctggg caagcccagt aggcccctaa cgtggcacct cggagcggct 7440
ggcgatgccc caagagccgg gcttccccct cctggaggct cttcctgttc ctcgccaagc 7500
tcttaggttt cgctgggcct cctcttctgc tctttttctg gctggagacg tcagaggaag 7560
cctggcgatc tagagaagct gatgtgcacc catcatcccg gaggtgactg ccaccaggcg 7620
ggggtggctc cacccccact cctcccctgg cataaatcgg gggacgcacg tgcctgcgca 7680
gaatgccttg gtccctttta attaaacaga caacccagtg tcccttggtc tatgctgacc 7740
tgctcaggag tgcctgggct cttccacgtt tcagctgctg tgtgtcctgc tgctgtgaag 7800
tgtccccgtt gaggtggagg cagggcaggg cgccttcggt gagggcgagt cctcctgtgc 7860
ccgtttcccc atctgtagaa tgatggctca tggaggagac atggtgtcgg caggtggtgg 7920
ccggcgcagg gaggtttcca ggagggagtc ctccaccctc atttgctttt agggccgtgc 7980
agcctcccga gcggccccca cgaggctctg aggctgcatg caggttgagc tcctggcctc 8040
actgcccctc ctcagggtta ggactgtgct ggagcctggg tgggtgtggg gtggtccctg 8100
cacaggcagc aggaggtggg ccagagcggt gctggacctg aaggcccgtg gctccctcag 8160
ctccactgag gaggctggaa tagtgtcctg ggccaccttc cctgccggta gccggctcct 8220
gctgagggag ggggtgcttt cgcctcctgg tggagcatca ggctcttggg gcctgtgcgt 8280
ccgagcatct ggcctctgct gtctgaaaac agcaccttcc ccgccccccg gccaccgcat 8340
ctcctctgaa tagaaggcaa gtcttgcctc ccaggaatgg cctgtgacct tcatcagcgg 8400
gggaggttag gggcacacag agccctgggg aacaagggga tctggggctg agccgggcct 8460
ctcgggccca ccccgagctc tcctgagcag cctggcaggc aggaaggcgc tgggccccgg 8520

ggcctttgcc gagcccagcc tcaccctcag cgtccccctc aatgccctgg gtgcctgcca 8580
ggctgtggac acaggccctg gccagttcta taaataacct tgccgggccc tgctgggaat 8640
tggtgccacc accacatagc ccagagagaa cggaaacaca gggcttctca gcgagcagac 8700
caggcctgcg gtgaccagtg gaagtggggg acgctttcag ggctgggcag cccgacaggg 8760
caccgggcac agccaggggc cggaaccagt tgctcactgg ctccgcttct gggggactgt 8820
gtccctctgt gccccgtccc catcacacct tgctttccct tcatcctgct ctcctagccc 8880
ctccccagac tggcggcccc ttgtccaggc ccagccccag cacagcctgc agccctggca 8940
gagctcaggc cttggccttc tgaggttgcc ctgagggcag tgagcaatga gccctgggac 9000
tggtgcactg ggagggaaag gtgagccaag gcttcctgca agaggcagcc ctgagctggg 9060
ggtggggaag gctgggtcgt gggatacccc tgacacccca agtttttttt ttgagatgga 9120
gtcttgctct gtcacccagg ctggagtgca gtggtgagat ctcgactctc tgcaacctcc 9180
gcctcccagg ttcaagcgat tctcctgcct cagcctcctg agtagctggg attacaggcg 9240
cccatcacca cgcccggcta atttttgtat ttttagtaga gacaaggttt caccatgttg 9300
gccaggctgg tttcgaactc ctgacctcag gtgatccacc tcggcctccc atagtgtcgg 9360
gattacaggc gtgagtcacc gcgcctggcc ccccagctgt tttttatgct ccatgcttgg 9420
gcttcctgtt aagaattaaa aaagaaaaaa aaaactatgg agatggaatt tacatactgt 9480
acaactcatt cgttcacagt gtacaattca gtggcattaa gtacattcac acggctgtgc 9540
aaccactact tctaattcca gaacgttcgg tcaccccaaa agcaagcccc attcccattg 9600
tgtcactctc catcccctcc accagctcct ggccactctg aatccacttc ctggctctgg 9660
attggcttgt cctggatatt gcatagaaat gggatcctgt cggccgggcg cggtgactca 9720
cgagagcaag agatccagac catcctggcc aacatggtga aaccccatct ctactaaaaa 9780
tacaaaaatt agctgggcgt ggttgcgggc acctgtagtc ccagcgactc aggggctgag 9840
tcaggagaaa tcgcttgaac ccgggaggca gaggttgcag tgagctgaga tcatgccatt 9900
gcactctggc ctggcgacag agtgagactc cgtctccaaa aaaaaaaaaa caagaaatgg 9960
gatcctgtca tcccagcgtt ttccgaggcc gaggtgggca gatgacttga gcctagtagt 10020
tcaaacgaga ccagcctggg cagcacggtg aaactgtctc tactaaaata caaaaattag 10080
ctgggcatgg tagcacacgt ctgtagtccc agctacttgc ggggctgaga tgggaggatc 10140
gcttgagcct gggaggtgca ctccagcctg ggcgacaggg aaaccctgtc tcaaaaaaaa 10200
aaaaaaaagt gcatcacaca ctgtgtgtcc tttagtgtct cactgtgaca tcctcaaggt 10260
gcagctaggc tgtggctcga gtcagagcct cactggtttt tgtggctgag tcttgttgag 10320
tgcgtggatg ggctgtgctg atcctcgtct gtggaagggc ccctgggtgg ttccacatct 10380
cggctgctgt gcgtcctgct gctgtgagca tctcgtgctc cgtttctgcg tggcgtgtac 10440

tgttcctcag ggtgtgtacc tgggagtgga gctgctggct cacagggtaa cagacctttg 10500
gagaagctgc agacgcttcc acgcccaccc actgggtgtg aggaccttgc tcctcgtcat 10560
ttttgttttt gtttaatttt ttcttttatg agacagggtc ttattctgtt gtccaggctg 10620
gagtgcagta gcacgatcac aggtaactac aggctcaagt gatcctccca cctgagcctc 10680
ccgagtagct aggacttgag gcacgtacct ccacacctgg ctaatttttg tatttttagt 10740
agagacaggg tttcgccgtg ttgctcaggc tggtctcgaa ctcctgagct caggtgatcc 10800
acccgcctcg gcctcctata gtgctgggag aaatgaagtg tctgcgacag ggcggcagct 10860
gcagaggggg ctgtgctctc tggcctgttg tgccccaccc ttgtgacagg caggtgggcg 10920
tggccaactg ggcggcagct gcagaggggg cagtgacagg cgggtgggtg tggcaggacc 10980
ccacaatgtc tctttctgtg gcgtctcctt cttcgcctgc cccttcctat gggccatcct 11040
tccagctcac ctgcggccca ccctccaagg agtgggaccc tcggagttgg gagcccttgg 11100
ctgcgtggga ctgtactgtg tgacggagcc atcaccaggg ctccaactca ccctggctcc 11160
tgccacagtc tgggctccat ctgtgctacc attgcagtag tctggtttct aggaagcaaa 11220
actgttactg agttacaaag ctcaattaag agtgtccttt gggaggccga ggcgggcgga 11280
tcacaaggtc aggagatcga gaccatcctg gccaacatgg tgaaaccctg tctgtactaa 11340
aatacaaaaa aattagccgg ctgtggtgac acgtgcctgt agtcccagct actcgggagg 11400
ctgaggcagg gaaatcactt gaacctggga ggcagaggtt gcagtgagct gagatcgggc 11460
caccgtgctc cagcctaggg acagagcaag actctgtctc agaaaaaaaa aaaagtgtcc 11520
taactgtgtc ctccaaagcc ctcgccggcc gatgacagac tagagggcgc tgtgctccca 11580
ccccctaccg ccctgagcct ggacgcgtgg cccctgcagg gccctttccc acagcactgt 11640
gaactcacag cttctctcta gggaagggag gaggtacgcc acttccacag ggagatgggg 11700
aggccgactc cagggatcca ggccatcatc ctgacgttgg gtcggctgat acacccctgt 11760
cctctctgtc ccagggaaat tcaactactg aggaggttac ggcacaaaaa tgtcatccag 11820
ctggtggatg tgttatacaa cgaagagaag cagaaaatat atcctttccg gtgttgggac 11880
cgcggggcct ccgtgggagg ggctggggcc ctgggtccgc ctgcctcgag gcctgctcct 11940
cttcccgtct ccttgaagga gactggcaca ccagggccgt ggccttccct ggttccccgg 12000
aagtcagcca ttgtggcaat ggctgcgcag cttgctgaaa ggggccctga gccctggccc 12060
ctgtgtcttg ggcccgtggg gtgtcaagtc cctttttct cagagtctcc tcccaggcta 12120
accaggggtg tagccacggt ctgcctgaga caggccacgc gggctgaccg ttgtgggcca 12180
ttttggtcgt ggctgggcgt gtcctcgtgt catctgtgga cacccccatg ggtcttacgg 12240
gcacagcctc cctacgggga ctttgcttcc taaggccctg tgcccagagc aagagccaga 12300

agtggtcctg aggctggggc tgtgttccct gagccacgcg gtcaggggcc ctgggaccgt 12360
cctgcatggg cccgagcctg cttggggggg cgtccaggag gcaccatccc ccgcccatgg 12420
gcagggtggg ggacgtgagc cccgcaggaa cgctgcccca agagtcagcc ctgtcctccc 12480
cttccccgta ggctccttcc tcctgggacg ctggggcccc tgggcctttt cagaggggtg 12540
gctgagggca gggtgggccc tggtcccgag gaggggcaag gtgggtgcag agggtccctc 12600
cagagcccct tttctggccc ccgtgctccc tgggcctgtg agtggggccg ccccctgagc 12660
tgtgtgtcct tagcgcccca cgtatatggt gatggagtac tgcgtgtgtg gcatgcagga 12720
aatgctggac agcgtgccgg agaagcgttt cccagtgtgc caggcccacg ggtgcgtgcg 12780
cggggcaggg gccagggtgg ggcgggggcc gggggccagg cagggcaggc tcctttccgt 12840
gaggccacac tgcttgtcct gatattcatt gacatgaagg cccaagtttt tttgtttttt 12900
tgtttttttg tgtttttttt cgagatggag tctcactctg tcgcccaggc tggagtgcaa 12960
tggtgcgatc tcggctcact gcaagctccg cctccgaggt tcacgccatt cttctgcctc 13020
agcttcccga gtagctggga ttacaggcgc ccgccaccac gcccggctaa tttttttgtat 13080
ttttagtaga gacggggttt caccgtgtta gccaggatgg tctcaaactc ctgacctcgt 13140
gatccgcctg cctcagcctc ccaaagtgct gggattacag gcatgagcta ccacgcccgg 13200
ccttgtaaag gcccaagttt ttaaaaacag ttttggggtc ccccatgtgt ggcatccaca 13260
ggcagggctg ctgccaacct cccgcctcca tctttgctgg gcctgctgcc tgaggccagt 13320
ggcctgcttc cagcccatcg ctggcagccg cctgccctga ccagatctcc tggatgcagg 13380
tctgtggcct cagagtcagg gccccttgct gctgcaggac cacaggggca gggaggggcc 13440
tgctgttcca gcaagacttt ggggtgcagc cggcctgtgg cccacaggaa aatgagacct 13500
gtggacatcc ggggccctgc cagacgtggc tcggccggac gagggtggcc actgcaggcg 13560
caggtgtggc tccctgctgg acctagcctt tcctctgtcc tgtgtgcctg gacttctgtg 13620
acttcccagc tgggcctgtg gtgtttggga ggctcccagg cagctgcaaa ggggacccct 13680
gtgaggggca gggaggcctc ggccccagga cgggtgtgtg ctgcccgcag gtacttctgt 13740
cagctgattg acggcctgga gtacctgcat agccagggca ttgtgcacaa ggacatcaag 13800
ccggggaacc tgctgctcac caccggtggc accctcaaaa tctccgacct gggcgtggcc 13860
gaggtaggca cgtgctaggg ggggccctgg ggcgccccct cccgggcact ccctgagggc 13920
tgcacggcac cgccacaggc actgcacccg ttcgcggcgg acgacacctg ccggaccagc 13980
cagggctccc cggctttcca gccgcccgag attgccaacg gcctggacac cttctccggc 14040
ttcaaggtgg acatctggtc ggctggggtc accctgtaag tgccccgccc ccccgggcac 14100
tcaccacacg cacactccga ggggcctctg cgtcttgggc agctgccggc ctgtgggcgc 14160
agggcgtggc caccggccca gaccctctct ggccacagcc gctagggggt gcttacttta 14220

tggaaatgta actcatacgg cagatggtgg ttcacccgtg tgaagtgcag cctggcccgt 14280
cagggatctt cacagagtgg cacggccgac cctcctccca gagccccaca gggaagctgg 14340
gcgggtgaca gcagctccag gccccttccc cgggtgggtc cagaggacac tcccctccta 14400
ccccgtagcc tccactagtg gaaggtggtg aagacagagg tgtccttgag tccacagggc 14460
ctctggtcca gcagccacgg gacgcctctg tccctggggt agagctgggg ctcctagggc 14520
gtcaaccacc ttgactgacc acgcctttct tccctcccct cgaaatgaag ctacaacatc 14580
accacgggtc tgtacccctt cgaaggggac aacatctaca agttgtttga gaacatcggg 14640
aaggggagct acgccatccc gggcgactgt ggcccccgc tctctgacct gctgaaaggt 14700
gggagcctca tccctctgcc cgcagcccca gggaggcggg gcttttgtgc agaaatgtag 14760
ggttgggggt gtcaggtggg gggctattgg ccccgagacc ccagcaggca ttgagaggac 14820
tgagtggaga ggccgacctc cccgcagggc ctggtttgcc aggtccctca gctccaccct 14880
gcttctgggc cctgttcacc ctccgaactc ccaccccaga gggcagtgct gccctgcgcc 14940
tcccccagcc ccaccctcgg gggctccctg gcttgcaggg tctgtcaggg ttgtcctgct 15000
gcacttccta cgcatggcag caggtggcac tggccgtccg tccatctgcc cagtggcctt 15060
gggagaacgg aaccgccctg gccgtccagc ccagccctgt ctccctgcca gccgcgcaca 15120
ggctgtcccc ggcatgtccc aggagtggag tggcctctgt cagggagacc gcctgtgcgc 15180
ggggtccccc ttaggagcgt ccaggtatca cccagggcct gacaacagag gctgggcagg 15240
cggggacggt tggtggggtc tcaggcctgt gcccagctga caggctcctc gccggcttct 15300
cctcagggat gcttgagtac gaaccggcca agaggttctc catccggcag atccggcagc 15360
acaggtgagc ggcccctggg ggcagtgggg ccgaggctgc agggaggccg gccatgtggg 15420
cagctggttg agcgggcgct agagcagggc gtggtggggg tgccaggctg ggctggggcc 15480
agaccccgtg cagcgcccgc agttctcggg gcccgagtgg ggtctctggg cagtgtcctg 15540
ttaccggcca gacccaggcg ccttgtccga actggggtct gagtgaggac atgcgtccgt 15600
ccctgcccta ggcatggaga tgcgccagga agggcacagc tggtcccaaa cactggcgag 15660
agcctctctt tttcccctcc tcctggggct cccagcagca gggtgtggct gggatccagc 15720
ccagggcccc cagctccatg acagggaaga cagagcagcg gacggggtca gcaggcccca 15780
cagtgccgcc tccctcactt cgtgggctct gctcctctgc accagcccct ggaggccctt 15840
gagccgtctg ctggagcccc tccgagcccc gaggccaccc actgagaccg gctctgggag 15900
tgggagtgtc cggacccctg aggcgctggt gctgattgtg ccttgggggt ctctgcacag 15960
ctcgggtcat ctgggcgcct ggcggggact ggggctgccc cccgatagcc tcctgggctg 16020
ggatgtgctc agggcccccc agaccccctt ctggcctttg ctggctttgc agccagcatc 16080

catctggtgg gtgctggctt ctgagtgcca cctgggacac aggcctcagg gtggagggga 16140

catctgtcag gcttggagtc aggtcagcct gcctgctcct agaggacatg gctgagcttc 16200

tgtggtcaca gccacccctt gcacggcctg gtcccagctc ctgagtgtgt ggcaggtacc 16260

ctgggcccag aggagctggg tcggaaaact ggaccgccct ggtgccagcc tgacaggcgc 16320

cactgcttct gggcgtttgc agctggttcc ggaagaaaca tcctccggct gaagcaccag 16380

tgcccatccc accgagccca gacaccaagg accggtggcg cagcatgact gtggtgccgt 16440

acttggagga cctgcacggc gcggacgagg acgaggacct cttcgacatc gaggatgaca 16500

tcatctacac tcaggacttc acggtgcccg gtgagtctgg cgggggcccc tgcccggctc 16560

tgctgactcg gccaggatgt cccacgggag cagggtgcct gcctgtctgc aacaaggaca 16620

gcttctgccc tctggtggcc aatcccacgt ccccaaagcc tccagcccac ctgcaggctg 16680

cctccgccct gcgggccgct gggacatggc tgaaaggtgt ggggtcagcg ggggcaccag 16740

cccaggcctg tctggccagg agggttcctc aggcgtctct ccgggtgctg cccagccagg 16800

caccacccac cggccttggc ctgagtccca gcaggagcag gcgggggagc cccagggtcg 16860

ggggagggta ggtgagagtc agggtgcagg gtggcccctc agacagctgg catgagagag 16920

ggtccagtgg ccctccctcc cgtcgtccct gaggcctgcc cgctggccct gatgccggcc 16980

gcccttcttc cctaggtggc gaggaggcgt ctgaggcagg gcttagagcg gagcgcggct 17040

tgcagaagag cgagggctca gacctttcag gagaggaagc ctctcggccg gcgccgcagt 17100

agtgcctgag gaggagctca gggccttagc gtaggggcgg cccacattgg cagccagccc 17160

ctccccgcca tgctcccggc ttggctgtgt tcggcccagg gctgggccgt gtcataaaga 17220

gttttgcagt gtatctgcag ggtggatgct tgctgcgctc gggctggagc ctgagggggc 17280

tttctgcttt actgtttcag cgggaagtgg tgggcagggg ccggcctgag aagggggggta 17340

cgccaggcag gttgggatgt gaggacccag tgcacagggt ccacccccgg gcccgagggt 17400

cccagaatag tgggggccct gcagagagcc ccccattagg tccctcagca ctcctgggcc 17460

cctcatcaaa cccctaggct cagctcagta gctggtcccc aggagagtac agtgtggggg 17520

cccccgagag cacagtgtat gggggtcccc gcggggtaca gtgtctgggg gccccccagg 17580

aggatgcagc atgtgggggc cccccaggag ggtacagcgt gtgtggggcc cccaggatca 17640

cagggtctca gctcctgggc tcttggattt gcagcaccac gaccatcgcg tctggtctgt 17700

tggaacggga ggtgctgctg ggtaccctgg tcactagggt gtgctgggag gtgggggccc 17760

ctcatggtgc ccatccttgg ggcctggctg caatttgacc ccaggcccag ggtcttctgc 17820

ctttcagagc cctggtgccc tgagacgggc agccaggcaa atcccaggca ggagggccag 17880

ttagggcagg gccagcccag gcaggttgcg agagtcccta ctgggacgtg gaccacacgc 17940

tgacccccac ggccgcccct gcaggccagg atccctgagc caggacccgg cactggcttc 18000

cctcctgggg accctcaggc ctgtgtgaga cctgggctgc cctggggtga ggtgcctggg 18060
aggagagcag gggctgggcc acctttttca tgaccctgct aagcccactg tgggtggtga 18120
ggaggagtac ccagcagggg gaagggccgc cagaccactc ggcatggctg aggcctcagt 18180
actcagtact gggtactcag gtggacgccc ctcccactgc tcagatgctg gggacaggct 18240
caacttcagg cttcagcgtg agccccgtcc ctgacctgca gagccccctt gcgttggggc 18300
aggacagccc ggcgccctcg ggtcaggcca tcctctgcgc tctgccgggg ctgctgcatc 18360
ggcctctgcg tgcctccact ttggcctcac gtgtccctac ccaggatgcg ggtcctgctg 18420
ccaacaccca gatcccaggg aaggggcttg ggctagatcc tgggcaccag tgcagacagg 18480
agtgtggggt ggggcaaggg cccagtggcg gctgtgcccg ctgatgcaga gctggggcac 18540
cttgtccaca gggtctgccc caccagagac gggctgggcc caagctcaga cctatgggtg 18600
caattggtgc ctcctcacca aggtcttttt tatctttttt tttttttgga gacagagtct 18660
tgctctgtcg cccaggctgg agtgctgtgg tgcgatctcg gctcactgca acctccacct 18720
cccggattca agcaattctg cctcagcctc ctgagtagct gggattacaa gtgcgcgcca 18780
gcatgcccgg ctaatttttg tatgtttagt agagacgggg gtttcaccat gttggtcagg 18840
ctggtctcaa actcctaacc tcatgatctg cccacctcgg cctcccaaag tgctgggatt 18900
acaagcgtga gccaccgcga ccggcccaag gtgctttttt aaagctagtg acttcctgtg 18960
cacatggggt gggtgtgtgg caagttctgg aagctgctga gtcagccact ggtccattct 19020
cggagctggg gctctgcact gggcacatga gctctggggc agcccggggc ggcctcccac 19080
tgacttcgcc cgggaggggc ctcggggatg gctcggcagc cagtgtgttc gcggagtcct 19140
cgccaaccac cacggctcct cgcagggaca gtacgtgggc agcttcctgc actttcccct 19200
gccattgtga gaacagtgtc cacctgggca gggtgggcag ccccaggcct gtgggtttca 19260
ccagggtgct ggtgatggtt ggtggctagc agggactggg ggcagctggg ggccctggca 19320
ggctgagctc tgctccccgc gtggttctgt gctggcattt cgcgtgcctg gcctgagcct 19380
ggcccgagcc tggccctcct gtgtcctcac agatgagcat gtggcggctc ctgggcctct 19440
agaaccaacc atgggccagg gtgccccagg ggagcacggg agggtcctgc cttgtcagct 19500
tgcctcctac tcgtgaggtt cctgcagtca gtacctgggt ggggtcccac ctgcggccat 19560
ggcaggtgca acagacgtgg tggaggggac actcctgccc aggccatctg cgggaggctc 19620
agccccgggg ggtgcctccc agagctgctg gggggcagca tttcaggctg gatacacctg 19680
ggcctgaccc gggggcgggc atggcctggg cagcagctgt aagtgcgtcc ccgtggtggg 19740
ggccagccag gtccctgtgg ctctggggtt gcgcccctca gctcaggcca cacttgccgt 19800
ctccctccca ggacaggtcc cagaagagga ggccagtcac aatggacagc gccggggcct 19860

ccccaaggcc gtgtgtatga acggcacaga ggcggcgcag ctgagcacca aatccagggc 19920
ggagggccgg gcccccaacc ctgcccgcaa ggcctgctcc gccagcagca agatccgccg 19980
gctgtcggcc tgcaagcagc agtgaggctg gccgcctgca ggtggggcgc ggcggggccc 20040
gggtggggca tgtggggaca acgcctggat gccacagcca gccgtgagca tagcccgcgc 20100
tagtcagtca tggtgaccgt cacgtggctg cgcgtggttg ccatgtggcc tttgggtggc 20160
ttggccacgt agcgatcccc gtggagggtg ccgtctcggg gcctggtgtc tggccagcgt 20220
gctggtcatg gaggcctacg tgtggcgggg ctctgggggg gcgtgccgtc ctcacagcca 20280
cctctcagag tgggtgcatt ccgaggaccc tgccctgggc ctggcgcccc ctccccatgc 20340
ccgcgccgct tccaggaaag gcttatgctg ggctcagccc agaggctttt gagcaccagt 20400
gggtggtggg tggtggggag gggccgcggc ctccatggct ctgccggggt gccgcaggct 20460
ctgagccagc tgccaagtat ggctgaggct gagtcgtgcc ggacgctgcc ctgtctctcc 20520
ctgtgtgcct gcctcctctc ccagccccag ccccagcccc gggtgggaga cggagtccca 20580
gaggtgtcag agacccttaa gtcacctgcc gaggatgcgg ggtggatggg ggcccgaggc 20640
tgaagcccct gccttgccac agcccctctc ccaggttttg gggccaccg cctgagttac 20700
atgtctgtcc cccaaatggg tgcccacagc ccatccacca gcgtcagagc ccgccaggcc 20760
ccactgcaaa aggccacaca atgtaccccg ggagtgactc aagggtggcc ttccctggcc 20820
tcccctgctg ccccccagga gtccggtagc cccatgactg tacctcagct tctccatcct 20880
cccaggggcc cgcgggaggc ggagaaccgg tgcccaggct gacctcttcc gtcttccttc 20940
caccctgcag cccgtgtcca ggagccccgc caggtgcccg cgccaggccc tcagtcttcc 21000
tgccggttcc gcccgccctc ccggagaggt ggccgccatg cttctgtgcc gaccacgccc 21060
caggacctcc ggagcgccct gcagggccgg gcagggggac agcagggacc gggcgcagcc 21120
ctccccccctc ggccgcccgg cagtgcacgc ggcttgttga cttcgcagcc ccgggcggag 21180
ccttcccggg cgggcgtggg aggagggagg cggcctccat gcactttatg tggagactac 21240
tggccccgcc cgtggcctcg tgctccgcag ggcgcccagc gccgtccggc ggccccgccg 21300
cagaccagct ggcgggtgtg gagaccaggc tcctgacccc gccatgcatg cagcgccacc 21360
tggaagccgc gcggccgctt tggtttttttg tttggttggt tccattttct tttttctttt 21420
ttttttttaa gaaaaaataa aaggtggatt tgagctgtgg ctgtgagggg tgtttgggag 21480
ctgctgggtg gcagggggggc tgtggggtcg ggctcacgtc gcggccgcct ttgcgctctc 21540
gggtcaccct gctttggcgg cccggccgga gggcaggacc ctcacctctc ccccaaggcc 21600
actgcgctct tgggacccca gagaaaaccc ggagcaagca ggagtgtgcg gtcaatattt 21660
atatcatcca gaaaagaaaa acacgagaaa cgccatcgcg ggatggtgca gacgcggcgg 21720
ggactcggag ggtgccgtgc gggcgaggcc gcccaaattt ggcaataaat aaagcttggg 21780

aagcttg 21787

<210> 91
<211> 18
<212> DNA
<213> Artificial

<220>
<223> Synthesis

<220>
<221> misc_signal
<222> (1)..(18)

<400> 91
gggaggccaa cgtgaaga 18

<210> 92
<211> 27
<212> DNA
<213> Artificial

<220>
<223> Synthesis

<220>
<221> misc_signal
<222> (1)..(27)

<400> 92
catcaccata tacattttct gcttctc 27

<210> 93
<211> 5496
<212> DNA
<213> homo sapiens

<400> 93
ctgtggcttg ccccagagct gatccttgtc tttgtccact tctcagcgag gatggcactt 60

cagggagccc ttcccttact atcgcagaga gagcaggccc tccccagtca tgtccaaccc 120

agaactctgt tttgttttct tcatagccct agcatcacag aaaatcaccc tgtgcattca 180

tggatgtcca cgggggcaag ggctttgtgt tgcttaaccc agcatcctga accgtgtttg 240

ttgaatgaat acagaacccc gtttgctctg ggagagcaca gaaaacagtc ttctatcata 300

tatcatagcc agctgcaaac agcagatggc ttcccatatc ccagagagta agaaccagag 360

agagagagaa agagagagag tttgggtctt tctcctctgt gcctgctctc tccagagaaa 420

ctggaggggt agcagttagc attcccccgc tggttccacc aagcacagtc aaggtctcta 480

ggacatggcc acccctcacc tgtggaagcg gtcctgctgg ggtgggtggg tgttagttgg 540

ttctggtttg ggtcagagac acccagtggc ccaggtgggc gtggggccag ggcgcagacg 600

agaaggggca cgagggctcc gctccgagga cccagcggca agcaccggtc ccgggcgcgc 660
cccagcccac ccactcgcgt gcccacggcg gcattattcc ctataaggat ctgaacgatc 720
cgggggcggc cccgccccgt taccccttgc ccccggcccc gccccctttt tggagggccg 780
atgaggtaat gcggctctgc cattggtctg agggggcggg ccccaacagc ccgaggcggg 840
gtccccgggg gcccagcgct atatcactcg gccgcccagg cagcggcgca gagcgggcag 900
caggcaggcg gcgggcgctc agacggcttc tcctcctcct cttgctcctc cagctcctgc 960
tccttcgccg ggaggccgcc cgccgagtcc tgcgccagcg ccgaggcagc ctcgctgcgc 1020
cccatcccgt cccgccgggc actcggaggg cagcgcgccg gaggccaagg ttgccccgca 1080
cggcccggcg ggcgagcgag ctcgggctgc agcagccccg ccggcggcgc gcacggcaac 1140
tttggagagg cgagcagcag ccccggcagc ggcggcagca gcggcaatga ccccttggct 1200
cgggctcatc gtgctcctgg gcagctggag cctgggggac tggggcgccg aggcgtgcac 1260
atgctcgccc agccaccccc aggacgcctt ctgcaactcc gacatcgtga tccgggccaa 1320
ggtggtgggg aagaagctgg taaaggaggg gcccttcggc acgctggtct acaccatcaa 1380
gcagatgaag atgtaccgag gcttcaccaa gatgccccat gtgcagtaca tccatacgga 1440
agcttccgag agtctctgtg gccttaagct ggaggtcaac aagtaccagt acctgctgac 1500
aggtcgcgtc tatgatggca agatgtacac ggggctgtgc aacttcgtgg agaggtggga 1560
ccagctcacc ctctcccagc gcaaggggct gaactatcgg tatcacctgg gttgtaactg 1620
caagatcaag tcctgctact acctgccttg ctttgtgact tccaagaacg agtgtctctg 1680
gaccgacatg ctctccaatt tcggttaccc tggctaccag tccaaacact acgcctgcat 1740
ccggcagaag ggcggctact gcagctggta ccgaggatgg gccccccegg ataaaagcat 1800
catcaatgcc acagacccct gagcgccaga ccctgcccca cctcacttcc ctcccttccc 1860
gctgagcttc ccttggacac taactcttcc cagatgatga caatgaaatt agtgcctgtt 1920
ttcttgcaaa tttagcactt ggaacattta aagaaaggtc tatgctgtca tatggggttt 1980
attgggaact atcctcctgg ccccaccctg ccccttcttt ttggttttga catcattcat 2040
ttccacctgg gaatttctgg tgccatgcca gaaagaatga ggaacctgta ttcctcttct 2100
tcgtgataat ataatctcta tttttttagg aaaacaaaaa tgaaaaacta ctccatttga 2160
ggattgtaat tcccacccct cttgcttctt ccccacctca ccatctccca gaccctcttc 2220
cctttgccct tctcctccaa tacataaagg acacagacaa ggaacttgct gaaaggccaa 2280
ccatttcagg atcagtcaaa ggcagcaagc agatagactc aaggtgtgtg aaagatgtta 2340
tacaccagga gctgccactg catgtcccaa ccagactgtg tctgtctgtg tctgcatgta 2400
agagtgaggg agggaaggaa ggaactacaa gagagtcgga gatgatgcag cacacacaca 2460

attccccagc ccagtgatgc ttgtgttgac cagatgttcc tgagtctgga gcaagcaccc 2520
aggccagaat aacagagctt tcttagttgg tgaagactta aacatctgcc tgaggtcagg 2580
aggcaatttg cctgccttgt acaaaagctc aggtgaaaga ctgagatgaa tgtctttcct 2640
ctccctgcct cccaccagac ttcctcctgg aaaacgcttt ggtagatttg gccaggagct 2700
ttcttttatg taaattggat aaatacacac accatacact atccacagat atagccaagt 2760
agatttgggt agaggatact atttccagaa tagtgtttag ctcacctagg gggatatgtt 2820
tgtatacaca tttgcatata cccacatggg gacataagct aatttttta caggacacag 2880
aattctgttc aatgctgtta aatatgccaa tagtttaatc tcttctattt tgttgtcgtt 2940
gcttgtttga agaaaatcat gacattccaa gttgacattt tttttcatt ttaattaaaa 3000
tttgaaattc tgaacaccgt cagcaccctc tcttccctat catgggtcat ctgacccctg 3060
tccgtctcct tgtccctgct tcatgtttgg gggcctttct ttaactgcct tcctggctta 3120
gctcagatgg cagatgagag tgtagtcaag ggcctgggca caggagggag agctgcagag 3180
tgtcctgcct gccttggctg gagggacacc tctcctgggt gtggagacag cttggttccc 3240
tttccctagc tccctggtgg gtgaatgcca cctcctgaga tcctcacctc ttggaattaa 3300
aattgttggt cactggggaa agcctgagtt tgcaaccagt tgtagggttt ctgttgtgtt 3360
ttttttttt tttttgaaat aaaactataa tataaattct cctattaaat aaaattattt 3420
taagttttag tgtcaaaagt gagatgctga gagtaggtga taatgtatat tttacagagt 3480
gggggttggc aggatggtga cattgaacat gattgctctc tgtctctttt ttcagcttat 3540
gggtatttat cttctattag tatttgtatc ttcagttcat tccactttag gaaacagagc 3600
tgccaattga aacagaagaa gaaaaaaaaa aaaagcagca gacaacacac tgtagagtct 3660
tgcacacaca caagtgccca ggcaaggtgc ttggcagaac cgcagagtgg gaagagagta 3720
ccggcatcgg gtttccttgg gatcaatttc attaccgtgt acctttccca ttgtggtcat 3780
gccatttggc agggggagaa tgggaggctt ggccttcttt gtgaggcagt gtgagcagaa 3840
gctgatgcca gcatgtcact ggttttgaag ggatgagccc agacttgatg ttttgggatt 3900
gtccttattt taacctcaag gtctcgcatg gtggggcccc tgaccaacct acacaagttc 3960
cctcccacaa gtggacatca gtgtcttctc tgtgaggcat ctggccattc gcactccctg 4020
gtgtggtcag cctctctcac acaaggagga acttgggtga aggctgagtg tgaggcacct 4080
gaagtttccc tgcggagtcg ataaattagc agaaccacat ccccatctgt taggccttgg 4140
tgaggaggcc ctgggcaaag aagggtcttt cgcaaagcga tgtcagaggg cggttttgag 4200
ctttctataa gctatagctt tgtttatttc acccgttcac ttactgtata atttaaaatc 4260
atttatgtag ctgagacact tctgtatttc aatcatatca tgaacatttt attttgctaa 4320
atcttgtgtc atgtgtaggc tgtaatatgt gtacattgtg tttaagagaa aaatgaaacc 4380

cacatgccgc cattttcctg aatcaaattc tgcagtggaa tggagaggaa aatacttcta 4440
ggcaagcagc tagactggtg aattggggga aatagaagga actagtaact gagactcctc 4500
cagcctcctc cctattggaa tcccaatggc tcctggagta ggaaaaaagt ttaaactaca 4560
ttcatgttct tgttctgtgt cactcggccc tgggtagtct accatttact tcaccccaag 4620
tcctgctgcc catccagttg ggaagccatg attttcctaa gaatccaggg ccatgggaga 4680
tacaattcca agttctcgct tcctcctttg ggcatctctt ctgcctccca atcaaggaag 4740
ctccatgctc aggctctcag ctctcgggcc agtgctctgc tctgtccagg gtaggtaata 4800
ctgggagact cctgtctttt accctcccct cgttccagac ctgcctcatg gtggcaacat 4860
ggttcttgaa caattaaaga aacaaatgac tttttggaat agccctgtct agggcaaact 4920
gtggccccca ggagacacta cccttccatg ccccagacct ctgtcttgca tgtgacaatt 4980
gacaatctgg actaccccaa gatggcaccc aagtgtttgg cttctggcta cctaaggtta 5040
acatgtcact agagtatttt tatgagagac aaacattata aaaatctgat ggcaaaagca 5100
aaacaaaatg gaaagtaggg gaggtggatg tgacaacaac ttccaaattg gctctttgga 5160
ggcgagagga aggggagaac ttggagaata gtttttgctt tgggggtaga ggcttcttag 5220
attctcccag catccgcctt tccctttagc cagtctgctg tcctgaaacc cagaagtgat 5280
ggagagaaac caacaagaga tctcgaaccc tgtctagaag gaatgtattt gttgctaaat 5340
ttcgtagcac tgtttacagt tttcctccat gttatttatg aattttatat tccgtgaatg 5400
tatattgtct tgtaatgttg cataatgttc actttttata gtgtgtcctt tattctaaac 5460
agtaaagtgg ttttatttct atcacaaaaa aaaaaa 5496

<210> 94
<211> 62226
<212> DNA
<213> homo sapiens

<400> 94
ctgtggcttg ccccagagct gatccttgtc tttgtccact tctcagcgag gatggcactt 60
cagggagccc ttcccttact atcgcagaga gagcaggccc tccccagtca tgtccaaccc 120
agaactctgt tttgttttct tcatagccct agcatcacag aaaatcaccc tgtgcattca 180
tggatgtcca cgggggcaag ggctttgtgt tgcttaaccc agcatcctga accgtgtttg 240
ttgaatgaat acagaacccc gtttgctctg ggagagcaca gaaaacagtc ttctatcata 300
tatcatagcc agctgcaaac agcagatggc ttcccatatc ccagagagta agaaccagag 360
agagagagaa agagagagag tttgggtctt tctcctctgt gcctgctctc tccagagaaa 420
ctggaggggt agcagttagc attcccccgc tggttccacc aagcacagtc aaggtctcta 480
ggacatggcc acccctcacc tgtggaagcg gtcctgctgg ggtgggtggg tgttagttgg 540

ttctggtttg ggtcagagac acccagtggc ccaggtgggc gtggggccag ggcgcagacg 600
agaaggggca cgagggctcc gctccgagga cccagcggca agcaccggtc ccgggcgcgc 660
cccagcccac ccactcgcgt gcccacggcg gcattattcc ctataaggat ctgaacgatc 720
cgggggcggc cccgccccgt taccccttgc ccccggcccc gcccccttttt tggagggccg 780
atgaggtaat gcggctctgc cattggtctg agggggcggg ccccaacagc ccgaggcggg 840
gtccccgggg gcccagcgct atatcactcg gccgcccagg cagcggcgca gagcgggcag 900
caggcaggcg gcgggcgctc agacggcttc tcctcctcct cttgctcctc cagctcctgc 960
tccttcgccg ggaggccgcc cgccgagtcc tgcgccagcg ccgaggcagc ctcgctgcgc 1020
cccatcccgt cccgccgggc actcggaggg cagcgcgccg gaggccaagg ttgccccgca 1080
cggcccggcg ggcgagcgag ctcgggctgc agcagccccg ccggcggcgc gcacggcaac 1140
tttggagagg cgagcagcag ccccggcagc ggcggcagca gcggcaatga ccccttggct 1200
cgggctcatc gtgctcctgg gcagctggag cctgggggac tggggcgccg aggcgtgcac 1260
atgctcgccc agccaccccc aggacgcctt ctgcaactcc gacatcggta agcgctcctg 1320
gtgccccgcc cgagccccac gctgcagcca ggactgcagc gctgcttagg gaggcagggc 1380
gagccccact cctttcctct gccccaggag aggggcagac ggggttgggg cggagtggag 1440
aaactcgatg tccttgggcg ggggcgctgg catagctgag aggggaagat gccctgcaga 1500
ggaaactcac agtggctgag ggagcccctg gccgcctttg ctttcctaac ttaggtcgtg 1560
aggttcctac cggtcctttt gacatctgga aaatgtcccc attcactact aacggaggaa 1620
gggctagaag agaagggtgg ggaaagggtt cccaaaactt ggaatgctaa ctaaagtgct 1680
gggaaattga atagttaaaa aaaaaaaaaa aagttgcccg ccatgtgcac ggaaagttgc 1740
aggagaaact tggagtactc cgagtctgca tcatctcctg cgcctatgaa attcattctt 1800
ttcactgaga cccaacccag ctgcaggctt cccaggcaag cgctaaccga gcttctggga 1860
ctgccaagtt tgggtggatt taagggggaa aatgaggaga aagctgtggc attgacccac 1920
gccctggagg cactgtgttt gagagtgttg atagccagca gaaggtgcga ggggtcactc 1980
accacatagc tgtgaaaatt cacaggaagg ctattttttt ccccccttgaa tgtgtgtggg 2040
gagggggaga atgggtagtg gaaaacagat ttgtcaccaa aagaacaaac ctgccaaggg 2100
tcacatcacg gtgacagttc cagactggac ttagtgtctt gagtagagag accctgtaaa 2160
gagacctcct actccttagt gtgtatggag ggccccgaga gcatcctggg aggcttgtct 2220
gggagtgttt gggacacttg tgtttggatg gctaaaggtg gagtggcatc tgggaaacat 2280
ctctctcctc ctttccagcc cataaagcgt gcaagtccag tggctctgct gggagagtgt 2340
gtgtgtgtgc actgggctgg gctgggcggt ggggtgggac agccttgccg ccctctgcct 2400

gtgtcgattc agaggtgaag aatggagcgt gtgtgtttta agtacagtca taaaggagaa 2460
ggaaattttc cactgagtct tgggtgaggg actctaccag cgtgttgtgg tgaggagggg 2520
gaggaggtag gagcttggca ggggaggcag tgagggccgg gaggggcact gctggtgggc 2580
tgggctgagc ctagaggtca gagtgggggt gtggtgacca gaatgtcagt gcagttggca 2640
gctgagggtg tttgtgtgac ctgaggtcac ccttgtaaga ggctccctgg ggcctgcatg 2700
gtacatcctc ccctctcctg ggggaaccca gctggcccag gatttgtgga ttgcttagaa 2760
agatcctttt gggcagcctg aagactgggg agggttttcc tccctcctcc acctccaccc 2820
acattccact taccctttcc ctcctcatcc cagttggtaa cattcccacc tgattagtgt 2880
ggggagttgc tgctgcagct gagttctggt tttgcccagc aggatcacac acgtgcggcc 2940
aggaggctgg caggtggaat cagggtgtgc aggggtgcag cccaagtcct tttctgctgg 3000
aaaatgggct gggtgacttc acttgcgttt ttttggtatg tagtgggagt aggtccccat 3060
tcttcccccct tgccctttgc tttggggttt ctaccctaaa aattgaccac ggaaaagtga 3120
aaacaagcat gagtagatcc tgcttccaat gggggccttc ccgtctgctt actgcttagg 3180
tttcctgcgg tgggtctgag caatgcagaa agtttcttac aatttctctg gattgaaaca 3240
agaaatagct ccctcgctcc tcttcataaa tttctcagag aaaatctaag ttaccaggaa 3300
gtagccaaag accaggagat tgtggaatca aaaagaaaaa aagaaacaaa aaatcttcga 3360
tgctctggca cggatcccaa attgttctgc ctgggtttgg tgagctgcac tagtggcagg 3420
gatgagctaa tgcttttaaa atgggaggtg tgggtgtgag ctgcttgcct gccgacctcc 3480
ctcgccttgc acagcaccct gctggcgtcc agtacttggc tcccaagttt cattgctcag 3540
agcctttctg gtttccagtc tgggttgaaa ctgtctattc tcgccctcaa acaaatatga 3600
aggagctccc cattccttgg gacccacaca accccaccag tcaactttgt agccccctaa 3660
aggtgttcac atcagcccat caacttcctg ttttaaaacc atcatggtgg ttttctcttc 3720
atggcttggt gccatgttgg cctagcaaaa ggcacaggag gcctggcctg gtcctctccg 3780
gggggctgcc cgatggcctt tcctcctgtc tctgctcatc cacaggtgtc tctttttgtc 3840
ttttttcccc aaagtcctag ggcctggcat ggcattctgg gggaggagga gttaaaaggt 3900
gtttctatgg caactccagg ccctgcaccc tatggaagtg tttaggagaa gaataaacta 3960
catgccggct tataagacac actgtggggg aaagaaaggg acttggcagc aggcaggatg 4020
tctgtggtcc ccctccctgg ccagtcattg cccctgaaac tcctgacaaa accttgactc 4080
tagaagagga atgggagggg gctggagatg aagaaggcgg cctcggcccg ggcaaatctg 4140
ccctggcagc tctgtccagc tcaggaaggg gtgggctgtg ccaaggcctt tctgaaaatg 4200
tgcgtgtgtg cgtgtgtgtg tgtgtgcgcg tgtgtggctt ctgcaaaggt ggctggcagg 4260
gctgaggggg gttgcagcag agaaagagaa ttttgcttca gaatgccaag cccttctctc 4320

cttctctctt gccgcctttg catgggagga atggggctgt aggatgaagt gtgttcgggg 4380
cctgcctctg agcctgttgg gaatttgagc atgaggctca gctggggggc cggctgccag 4440
ccccagagga tggcccctgg atagctccaa attgttctga caggggccag tgccagcaga 4500
ttctttcagt atcagatctg aggttgtcgt tttagcaaag aagccatttt caaagaaacc 4560
agatccatcc cctctccctc ttacacacaa ttacacctca acactgggct ttgcaactta 4620
aggaaaatag aaagaatcag actttggaga ggcgccagca cttggtaggg caagtctggg 4680
ggacccaggc tgtggatgtc ttggaaccat ggaaggagga gagctgctat ccagctggag 4740
gagggaagag acatgtctta ggcccctcct ggttgcacgc acttcccaaa tgccatctct 4800
ttcacccccct cccccgaccc cccacagcaa ccttaagagt aggtattgca cccatttttc 4860
agatggaaaa cactgaggct gaagagactg tgcttgtcca aggccaccca gttgctaaat 4920
gataaagcta aaacgtatta tcagttatac catgcttcaa aaacctttca actttcattg 4980
acacatgaca ttctaaggtg accaggagat ggaatggatg gttaacattt gaagaatgct 5040
tttcttctac atctgggttt tctttaaaaa tatgaagata gccaaaatgg ccagaggttt 5100
taattaaaca tcatctttag ggagggagag cttattatta ttctagtgta tcatagagga 5160
aaattctaat gttctttggc tattagatct ggaaaagacc cttgaaacca tcaagccaca 5220
gtaataaaag gacttggttt gtctcactgt ggcccaagga gaagtaaggt taatagagac 5280
aagacatttc agggcttcaa gactcttccc ttaaaggtgg gatcagttag ggctccccag 5340
tgggaggtac ccggtctgcc agtgtggtag gaggaaagcc agggggagtc aggagactgg 5400
gtcccagctg gagaaggtgc ccctctgaca gttctgtgtc cctttcacag tccctggata 5460
gataaggcta gaaaagtggc cacagaagta gcttgggtgg agggattttt gtttgttttg 5520
catgaacagc aggaaccatt cagctccact gctgaatggc acattccaac aggttcatta 5580
attaaaaaca aaaacaacaa cacgattctc catcgcactt ggcttttacc attccagcca 5640
gtatggacaa ggaggcccct tcttctacct tttgtcatag tctactcctg ggtggccttg 5700
agaggcaggg tggagtggtg gaaaggcaat ggggagggca gatctgagct ccactccttc 5760
ctctggggct tgttggctgt gtgtgcctag gcacatatgg gctacctttg agctttagtt 5820
ttcttattta tacaagggga ataacaattt gtaccatctt ggcctgtttt atctatattt 5880
taaaagctac aagtatatga atacattcta tgtccatgta tgtatctctc tgtatatcta 5940
tgatatatcc atttctattt atcatttatc atctatctat ctatctctct atctaaaatc 6000
ggagaagagt aaaatccttt ctgattactt tttctaactc tagtcccttc tccaaatcct 6060
tttcttccta tacatttaca tacataggta tccatagcaa atttatagta gtttttccct 6120
tagaaacatg aatagtagaa cattaaacat catggaatac cacataatac cacatatagt 6180

attctgcttg tttcccattc aacaatgcgt actagagatc tttctactta atatatatat 6240
aaaactgtca gaggagcacc ttctccagct gggacccagt ctcctgactg ccccccctggc 6300
tttcctccca ccacattggc agactgggta gctccccctg gggaccccta actcatcccc 6360
acctttaagg gaagagtctt ggaaccttga aatgtcttgt ctctattaac tttacttctc 6420
cctggccaca gtgagacaaa ccaagtcctt ttattactgt ggattgatgg tttggaggat 6480
atatatatat aaatatataa tatataaata tataatttat atataatata taaatatata 6540
atatataata tatattatat ataatatata ttatatataa tatatatata ttatatttac 6600
atatatatta tatatataat atatatattt cttttatgta tttcatgagg ttataatttt 6660
aaacagctaa taagtataga gtgattacta tgtgccaggc attattctaa gtcattaact 6720
tacattaact catttaattc tctcagtttt atgagggagg tgtgctattg tcatctccat 6780
tttacagctg aggaaactga ggcttagaaa aatgaggtaa ctcgtttaag gttatactgc 6840
tagcaaggaa tacagcctgt atttaagccc cttcaccctg gctctaaaat atatttaaca 6900
actatactat aggtcacatt ataccatatc atattatata tactactaca tactcttata 6960
ttattataat tatataatat gcattttcaa ttttaatagt tcctgccaat ttggcctcca 7020
cagtggctgt gttaacacag ttcttttcat atatgcaaca tgaggggatg gaggaatgtt 7080
agagccctgt gcttaagaga ctcccatgct tctctcaaat agaaaaacaa tttataattt 7140
ttattgactt actgagtgtt ttttttcctt ctttttgtct ctttgaaagg gagcaaaatt 7200
agggccacga agatcaagtt aatgggagta ttttcatagg gagaggaagg ctgcaatgcc 7260
aggaaagctg tgacccttga cttgttgcat gctggagtaa ttcggttgaa gatagcttta 7320
tgtatttatt ttgctttcag aataagcagt ggaccattta ttacaccaag ttgtttgact 7380
ttgaggactt acatggactc aggttcaaag gtatatgtgg ctgtgttttg aacacacata 7440
cacagggttt tgctttctga ctcaaagctg tccgctggcc aggctcatgg tggcagcaag 7500
ggcactcact catctgtctc ccttcttgtt gcctatggat ggcacttggg tgagagcagt 7560
gggatggtca caaggaggag gatggactgc cgagagggag gctgggtgga ccccagccaa 7620
gaaggacgct ggggtctcat tattatggga gctagaaggc ctggtttagg aaagaattcc 7680
tgttctgatg aaagctcttc agtggactct taggataaag tatcatcaaa tggcttttac 7740
aagggccagt gtggaccctg cctacctcat cctcatagtc tcatcactgg tctgtcctgc 7800
tccagctgtt catactctgt ctctctggct cttggcccat gacacccccct cggttttgaa 7860
cagtcctcat taacttctca ttctctaggt ctcctttggt tcctttccct gatcactcct 7920
tggcattggt acagacgaac agtcataaac cttgcccata ttttaagcat gctgaaaaac 7980
accatctcta gtaaaaccca aattctccaa agctgcatgg gaaactccca gaggggatca 8040
ttcctgcgct gggagaaaga acttagggct gcgtgccacc cagagggata aacctaaccc 8100

actttagagg gtaagcaccc actatgctct ctgcattact tgataagtct agtcctgtct 8160
tctggtggac atgataccct agcaccctcc cagagaagaa agtggagatt tctaaatgcc 8220
aaccctgcag gagatcacaa acataatact gtaattttca tagtaagttt ggtgccctta 8280
ctatgtgcca ggcactgtac caagctcctt acttgcctat cagtcatccc agctgcctga 8340
tgagctgtct caggctaggg aagccaaatg gcttgcccaa gctcacacca cttgcaacct 8400
atacttgctt agcctatctc caaaacctgt gtttccaatc agtctctcat atagaccaag 8460
ggctacaact ttttagactt gggaagattc tacccatttt gcgacctctc cacatgagtc 8520
tttggctccc cactgttggg gtccccgtca gtctgcttat ctttggtttt cttcctctcc 8580
agacatgcct tccctgctgc cccaaatttt tattactgta tagtcagttc tgggatccta 8640
acataaccaa gccttacctc ctaacccagt gccctttcaa ctctctcagt ggttcccaga 8700
cttttcaaca ctatggaatt ttttaaaatt aatttttccc tccaataagt tctgtgtttg 8760
gaacagttat ccgacacaat ttcatgtgtt gagtgttgat taactttcca ttaaaaaaca 8820
aattaaaata taactagaac tgctgatact agccagtcat tattctgaca caatcagcta 8880
atatactaat taattatagc cgaaagcaag gacggttggt gttttcataa gcctgggaag 8940
ttttctcatg ggttcctggg tgattgcatt gtcggatttg ggttggctta ctgatatgcc 9000
taaaatagct cctggcttct ttggtatatc gctgtccttt gcccctcctg agaatcttta 9060
gtttcatgcc agcttagcat ctcttctccc tgtgggcagg tcccatgcat ttactgccat 9120
tgtcacttct ccctgttacc cacagtgctg ccaccatgcc tgggatagac agcaggtaac 9180
tgatgtgtcc tagttgaggt ccctatcctt tcagaggagg tgacatgggt tcagatattt 9240
tcctatgttc atgtctggca tccaggggta cacaaagtat tgattctagc taaagggact 9300
tactaaatac tgtttgacag gaggcccaca ggtgtctatg tgggctgcag tggaactaag 9360
aggtcttttt gttcagctgt taaaaaatta gtgtttgtag cttcagaggg gccccagaga 9420
acctttcctg acccatttag aggccatctg tgtggctagg atggctttgc tgtcacagaa 9480
gggacagtcc acactaccca acactcaagt ctgatttatg ttcatgtaaa tccgatcacc 9540
taatggtggc attcacggct tggagacaag aaactataag tgtttccaag cctgcagcca 9600
cctggctttt tgtgggggtg gggaagggaa ggaaagctgt acgatctggc ctcagagagc 9660
ccaaggtaaa gttcagttag tggaaaaaca gagccctaga tgattgggcg gggggtgggt 9720
actggggagt ggagagggga agtcctgagc tgaccaagcc tccaagagcc attccggaac 9780
tagagatctt aaatactaca tggtaattat cccctctgcc tctgcccatg agggtgtcag 9840
gcacagggcc gcctggctgc ctgcagagaa gagctgtttc attcagaaac ttcagagctc 9900
ctgcaatgcc tggattagga tgtggggatg gtgagtttcg caaccagggt cagatttcat 9960

gaagacttgg tttcgtgact tgaggtgttt gactaaggtg tttgcatctc atgtatttag 10020

gaagggctcc cttttgactt agactttatc atcatgaata agctccctgc cttttcgatt 10080

tggaaacatg atagctctag tgagctctgc tactcccaag cttgggcagg ttctttgcct 10140

cagtttcctc atctctaaaa tgggggggaaa tatattatgt accacgtggt aacttcattg 10200

gcaattaagt gaaatacttc atggaatgtt tagcactaaa tattccttag tgttgaaaag 10260

gctgggaacc actgagacag ttgaaagggc actgggttag gaggtaaggc ttggttatgt 10320

taggatccca gaactgacta tacggtaata aaatttgggg gcagcaggga aggcatgtct 10380

ggagaggaag aaaatcaaag ataagcagac tgagggggc cccaaaagtg gggagccaaa 10440

gactcaggtg gagaggtcgc aaaatgggta gaatcttccc aagtctaaaa agttgtagcc 10500

cttagtctat acaagagact gattcttggc acagaatagc tcaataagaa taagcatgag 10560

cttttagtag tagtgttgtt atagagcaga gctgatattc tcacttcact ttcggtgagg 10620

aaaatggcgc tctgtggtgt gatgtgatgt tttccagggt catatagaga gagctgagtt 10680

tcaattcaaa atcgtggttt gatccccccg cctccaaatc taatgtgttc cattttgtca 10740

cagtgcttcc cacagggcag agaaagaaac aacaaagttc ctactctatt ttcacaactc 10800

ttcacatttt agagtcttaa gtccatttga tctcatttga tgacaacttc gcaacgagga 10860

aagcaggatg gtgattgggg ggtcggtcat agttcctagt tagtggatgt cagtattgag 10920

aatggcgggg ttctgtggct tagccatata gccaatacat ggaatgggat tggaacccat 10980

gtcttctgag ggcaagccca acacttgtca aggatctggt gctgcccttc cctctttaga 11040

cagctcccat tgataagcag aggtagaagt cactagggta gccaaagaga gggccctaga 11100

aatggaatgg tgacaatgaa cttggctgaa gagtcagcaa ccgctcccct atccactgcc 11160

agggctagga ggtgcagtgt ggcatggagg aggagcactg gctggagtta ggaggcctaa 11220

ggtctagtcc tagttcatca tctgactctg gagtcccctt ggtgatactg acctccctgg 11280

gtttggtctc ttatctgtaa aacagcagga tattggctct atgtggtgtg tatttcactg 11340

aatgcctact atggtcaagt ttttctggct ctaagttccc ttggaaaggg ctctctactt 11400

tagctgaata actcagcacc taaatctgtc aaccacaccc ttcagtaggt aactttgtta 11460

gaaatatggt ctattaaggt aagagccagg atttctgtgg ttgcaaaagt ggatgcgggc 11520

tctgaacaaa acctctagtg agctgagata ctttatcaga tgacatattt gaccaccaaa 11580

cttctgttgg agcagggctg atttgccaaa cagacagcaa ccttgcagac ttagtcgatg 11640

gctagaatta agactcaagg tcacttcagg gcctgtcact gattgtggga gggcttggta 11700

aaaattgaac ctcattaaca ccactgaaga agctgataga caagaaatgg gattaagact 11760

caatactcat caaaataagg ctttccttat ccacagccta cctgagcagt gcctcactct 11820

ggaaaatgtg gttcatggct cggctcaaac attttctgtt gctgagagga cttctctcat 11880

ggaagtctgg ctcagatgaa cacatgtgtg atctgatgga acctttgttt aagtgaatga 11940
ggagggtatt tggaagactt agatcaaacg tgtgccccat tgggcagtca ctatctgctc 12000
ccctgacagg gagaaacccc agtgacagga tagaaagctc caaagatcct gaggaagttt 12060
ggaaggtcaa acttcaccac tgcaaggact gagagatcca agaatctcag aatccaggtt 12120
ggtctccagg gtgcagttct gaacaaatga gtgcagtgga taaaaagctt atacttaagg 12180
tgggaaggtg ggtgtgggtt ccagttcctt ctttggctga ttctcttttg gcctggattt 12240
cctcacttgt catccatgcc atgtcacaaa cccagtgcct acagggacca ggcacagaag 12300
ctggatattt gagtatcaga catattggaa tattactcac tcctaccaaa tatgtttatg 12360
gttccccatt ctcttggttt ccattttgga gagctaaaca ggtactaaaa atgatttctt 12420
tattataaga caagcaataa aacagtgggc aagtgtgatg ataaacagca tctgagcttt 12480
ggctttggag ttggggagac aatagagagt ggcagtaact gtggtgaacc gtacagtcca 12540
ggtcccttct ggaggggaca gctgctgtcc agctccagcc tatcaggatg tgagtctagt 12600
gatgccagat ttccagtctc ttaggacaag caagaaatcc aaatttttaa tgtaacatct 12660
tctgaaaaga tacacacaca cacacacaca cacacacaca cacacacaca cacacacaca 12720
cgtggaccaa acaaaacatg tccatgggcc agattcagcc ttgtgggtca ccagttttgt 12780
gatctggaat ccattttatt tctagaatct cctttcaatt ctcggatctc tgagtgagta 12840
acacccaatt tttttttttt tggcaaaatg tgtcctgcat ttcccacact ttctgagctg 12900
gagcacaggt tctggagtta gaccacttgg ctcaaacctt ggcctcgttc atggctagtt 12960
gcctgtccct ggacaagtta attccctggt ctcatttttc ctatctgtaa aatggagtat 13020
gcaataatag tactcttaac ctcataggtt gtggaagaat tcacagacac aatggaaata 13080
aaaggcctgg ctcattgcct ggcatgtagt aaggacctga taaatgtcag ccatcatatt 13140
attactacat ggtattactg cctcccctta gatgggagcc aagtcttgct caccagctca 13200
ttgcctgatg ttagtaggca ttcaataaaa gtatcatgaa tgaataagaa catttgagaa 13260
agacagcatt ccagcctttc caggcaatac tcgtgtgtgt gtgtgtgtgt gtgtgtgtgt 13320
gtgtgtgaga gagagagaga gagagagaga gaaagagaaa gaaagaaaag aaagaaagaa 13380
agaaagaaag aaaacaaaga aaggaaagaa agaaaaaaaa agaaagaaag aaggaaagaa 13440
agaaagaggg agggagggag agagggagag agggaaggaa ggaaggagag agagagagag 13500
acagaaagaa agaaagaaag aaagagaaag aaagagagaa agaaagaaag aaagaaagag 13560
attggtttga aacattggga gctgagctta atgagaaaag gccattccta actgctaagc 13620
taccgtatta aacagtggca agtaccttgg aaaataaaac agaaaccact gcccatcttt 13680
ttctaacgga gaaagacaag agatggcttg accagagtgg agatttagaa cttcaggtgc 13740

tgataaatat ggtaacatca tggggtgctt tggcagctgg atagatttta tttattcagg 13800
gctcctttaa gccagaggag attcctggag gctgctaaaa tagaatgctc cacctggata 13860
gttaacatct ggattttatt ttgtttcaaa aagttaatta caaatagttc gaattccttc 13920
tggtatccct ctgagaagag tttcttactg ggtggctcac aagcggggag acagaaaggg 13980
aaaaaaacta gattgatatt gctcaggaga aacaggattg tggctacacg tgagccatgt 14040
atgcaatttt gaattttccc atagtcatgt ttaaaaaaac gaggaaaaag tgaaattaat 14100
tttaatacta aacccaatat gtataaaata tgaccatttc aatatgtaat caattttaca 14160
cttattaata agatcttaat atttttaaaa ttaagttttt gacatccatt atgtatttta 14220
cactcatagc atatctcagt ttggactagt tgcgtttcag ctacctgtgg ttagtggccg 14280
ccatgttaat actacagcac aggtcaagat aacatttgca aaggcaaggc atcttcccct 14340
ccccatttct agtttcatga actgtgcaca gggatatggg gctgttcgag gtacttttgg 14400
gctgaccaag gctcagaggc tactgacagc tttgctgcaa gtaacttcta ggccttgtgg 14460
gtcccagtgc agggaaccca tgtgcggtga cactggagaa gccatctgat ccaggtctct 14520
cacttgacag atggggaaac tgaggtccaa agaggtacag cagcttggtt taagagatag 14580
agatggaact ggaacaaaaa taataaccat ttgataatat tttaacattt attgatttgt 14640
ttttatatgc caagaccttt taaagcatta tctcctttaa actctcataa ccactcattg 14700
aggtaggcac tcctatttct actttgcaga tgaaaaaaat tgggacttaa gaggaattaa 14760
atatgaaagc agctactatt tgttgacagc tattttaaga tctttatata catatataca 14820
tgtaataaat tctattcttt actataaaca tacatgtcag aaattattag tgttatcatc 14880
cttgtcctcc tcattccact ttgcagatga aaatactgaa gctgagagag actaggtaac 14940
ttacctagta tgtacctaca tagtgaagcc agatttgagc cggacctcct gactccagag 15000
tccgagcaat ctcaccaatt ctgcccacgg tcaccccact gcatgatggc agagctagta 15060
tttgagcagg tgtgtctgtt ctgtggtctg tggtcttcac cgcccccacta gacagcctgg 15120
atttttctgc tcttgagtct caacactttc tgccttactg tgccactagt aaggaacaga 15180
acagggaggt ttttgacccc agcactgatt gaagggccca ggcaaagcag gctcgtctcc 15240
tctggcttgg ctggttccca ggctgcccta acccatgaag ccagattctt ggccaagtta 15300
agcagggtgg gaagaacact tgtccattcc gccttcccag ttgggcgagg tcagtgtacc 15360
aggttggaaa gtggtatgtg tctaattgca ggggcggggg taggggggag gccagaggat 15420
taacactctt taaatagaca tcagaaaact aagaatagcc ttgctgcctg gtaattgatg 15480
ggcattcctg actacgggcc acacactcag taggtgctcg ataaacatgg gtgaaataca 15540
ggaataaaca aatactgaaa atgggtcatt gggtgagcac ttagccagta aaaatctgtg 15600
ctttgtataa aaagaacttt ggactgaggt tatatgagtc ttcctgcagg ctggagacct 15660

ctgttacctt tacttctcta cttttgttgt cttgagacca gctttttat ttttggtgag 15720
gagaacagca ggcagttcaa atgtcaggga aagacaagtt cagcttgctt tttccttatt 15780
tctgtcttgt tacctgctat cctgccaggt tagaaagatg gaatcactcc cttttgccct 15840
atcatcaaga cttcagtgtt ccctggagca ctctaaattt gccatcagac ttggatcatc 15900
ctctaatacc aagctcctgg gtccttcctg aaggctgcct ccttgtcttg ctgagaagaa 15960
ttaaggtcgc ttccctggga ggaattcagc agcacctctt gtaagtcatc aggtgtgctg 16020
taaggatggg gcaggcaaca tttctttgta ctgtgggaag gcctaaatca gtgtttctca 16080
aggtgtgatc cttgcaccac caatcttaag aaatgttctt tggagccggg cacagcggct 16140
cacgcatata accccacctt gggaggctga ggcgagagga tcgcttgagc ccaggaattg 16200
aagaccagtc tgggtcacat agtaagattc cctgtctcta ctaatttaaa aaaaaaaaaa 16260
agaaaaggtg gttgggcctg tagtcccagt tactcttgag gcttaggtgg gagaatcgct 16320
tgagtccacg aggttgaggc tgcagtgagc catgattgta ccactgcact ccagccaggg 16380
tgacagagca agactctatc tcaaaaaaaa aaaaatgtta tttgggagag ctgtttattg 16440
tattcccctc ttggagagac atgtggctat tagcatagta aaagctctga gaagtcctgc 16500
tgcatggtga cctgtttagc tctgtttaat ccagtgttct ctgaacatat ctagccaagg 16560
aactaatttt cccaagaagc acctacaaat gcagttggtg agacactgcc cagatgagga 16620
ttctgaaaac cccttttgcc cacttgcctg ccattgtgat tgctgagtag ggaagaagaa 16680
attaggaagt atcatctgca ttacacaggg aagctgctct cctagggttg ccaaacagaa 16740
atggctgtag tggtgccagt gggtgctttg tagggaccag aagaaaaagg aggaagggaa 16800
ggctgacctt caaggtcaca catttgccag atttaaagtt ccttcctgtg gtcatgactt 16860
gttttataat cttgcttggc tcagttctgg gcacacatca gatttttaaa tcctaggatc 16920
ttgtttatac cgttaaggac atgattgaag gacattgtgg tgagtgtgaa ggtgagatgt 16980
ttctgattga aggtccatgg ggcaggcacc ctgcgagcag gtaatgaaca gtaggggaca 17040
taccggtgca accccaccct ggtcccctgg tcgtctgggc cagatctctc ccctctctgt 17100
tctatacttc tcttacatca ccgtcctacc taatcatttt accgatagga gcacctttgg 17160
gattctgatt aaagctatgc atacaaattt caagagcttc agaaattctc ctaaaagcta 17220
tcttggatca ttagaacact tgccctagag tatcactgag tcaataatgg tgttgaaact 17280
aaacagcaac aaaatccccc caaaaaggta acctaactta gtctgaaatt gtctgaaaat 17340
taatggcact gccagttcag tggctgctca gagcgttttg gtgaagaaat tggctgatgg 17400
atattttgca gtagtctaca ggatttgacg tgggatgcca tctttatgga catggctctg 17460
ctgggcaaag caaggacggg cacatccagc ctatgcggtc catcaggggc actcacattt 17520

agaatggggg agtcttagtt agcccagggg ctgggggcag catggtagtg tagaaaaagg 17580
ggccagtgcc tccagaaaat gggaccccag gcctggctct gctccttctg ctgtgtgatt 17640
ctggttgagt ggccttccct ttgagtcctc tggatgaagt taaggagaag tcttgggttc 17700
tcaagtagtc actattcaga ctctcgcttt cagagtattt aaaggaggaa aggaaacaga 17760
aggatagggc tggtggactt aaaaggccgt gtgtttgccg ccaccccatt tgctcccagg 17820
gctgggtgtt tgtctcccct ccggcgctgg gcctcctgct tggcctccag ctcttgcacc 17880
attaactgca gtcagaaaag ctgatgtcac tgtggccggc ctagggggaa agtctcagga 17940
taaaactgct tccacctgga tctggagaag gggaagtgac tggcaagaga ttcgcaggta 18000
gggctcagag accaggagga atccagtctg ggtaagaatt ccctgtcatg gggctctgaa 18060
gccacgggcc aggcaggtag tggatttcaa gccagccagc ggggtcactt ggcccctgca 18120
gctactcctc gccccctcct gcataatttc ctatttcctg cctcctcctc ctctgacaca 18180
gttataaata acctcgaggc tggcctctgc gcttaagtat aggtgagcca gaggaaagtg 18240
gaggggatca aggtggagct ggatctgcca cttggccagg gggtcacagg gtttcagatt 18300
gctcagggga attttgtttt cttgtttttt ccactaggga gtgaggtagt gaaagaggaa 18360
ataagctgtg ttggcatggg gagccttggc cttgcaggcc ggtgctgggt ttcacccgca 18420
aggagctgcc tctctgctca ctgcagatcc ccaggaccaa aggtctggaa agaggcttag 18480
tgagaaggca tctcaggaca caaagaggac acgatgcctt gctggtgagg tcctgcggtc 18540
tccttctcga ggcccagccc ggccccatat tggaggccaa acatgtagaa gggtgtatct 18600
catttttac cttttttct caaccaccag ccaacgatgg gtatgtgatt tctaaggatg 18660
tccattgact ataaaggtcc cattaataca tatgctatgg acagattctc tccttgaaca 18720
caggtttatg gagcacctac tatattccag gtgctgggca gagtgctaag gtcacaaaaa 18780
tgaatcagct gtggcctcta cagcctgttc ccctcttggc atagatctgc ccttcccccct 18840
ccacagtctg gacaatcaag gacctggagt aagcacactc taggggccgc gtctcccagc 18900
atatcttttg ttttcgaaag ttcaaaaaac agaagaggag gattctgaaa cagcaaactt 18960
gctgggtcat ccatagagta tattaactgg ctatcttgct atctgtccat aacttgctac 19020
ctgtccatag tgtatattaa cttcctggga gcccactcac tgcctgactt cctgttctgc 19080
gcatgggccc aagggaagtt ctcgcaacca tagttttctg tgatatccat cctaccacat 19140
ggcttcctgg gaaacccatt agcttttttg tttgttttag agcccaaggg aatcagggcc 19200
ttgaggggat tttttggtgt ggggggtggt gcaagcttct aacactgaga gtgggtgctt 19260
ttctgattcc tatgaaggtg gagtgtaggt gtcgttcctg gctcagcaga gtcagccctc 19320
tcatcctatt caggccgtct ctgaggctgg ctcctgtgcc caagcccttc ctcactccct 19380
cagggcatga tcacagggca tttccttagc attcctcctt tctcccctgt gtgtgtgtgt 19440

gtgtgtgcgt gcgcgtgtgt gtgtgtgtgt gtggtgtgtg tggtgtgtgt tctactccgt 19500
gtgtgtgtca tcctctgctc atccacccat tccactgcgt gtgtgtgtgt gtgtgtgtgt 19560
gtggtgtgtt ctactccgtg tgtgtgtcat cctcctctgc tcatccaccc attccactgc 19620
agcacctagc atcacctagg ggcctggaaa acctgcacct gaaagccgta actccccggc 19680
acctggctgg ttgacttctt ccccccttaac cccatttact ttgaggatgg aggtagtctt 19740
tagggtcagg ctgagatttg tcctgaccca tgcttagttg gcacatcttg cttctgcaac 19800
atgcttgctt agggttgggc aggggcccgg agaaaacaag tcgtggcatc accaggacct 19860
gattaccccc aggcaccaag gagcacctgg gaccaagggg aggtttgggg cttgggatat 19920
gttttagggt gaaggatcac agaagcttac tcatacctat ttgaaagcca agtcaacaga 19980
tgtgggttct tggaaggaga aaaggaggtt aactgcttac ttattagatt agaagtgatg 20040
tatgaatgat aaagagcaga gtttgccaga tattgctaat taattctctc actgcttatt 20100
ttatggatga taaagggtag ggagagacat gttaattggc ttcaaggtct ctgagccaag 20160
catgaccaat ccgagaaacc caggtggctt catgctcttt caatatgatc tcatccctgc 20220
actagagaat ttatattttt gagcataaag aatacccacc ggtgcaatct cccaggctct 20280
tctgaagggt ttggagagac agtggaggat gtcaaaagag atgaacttag agtcaaaaaa 20340
agcagagatg gaatcccgtc tctgcctttt accactggag ttagttgact aaaatgtaaa 20400
ctctgagcct tggttttccc aattgcaaaa tgggaaagac cccatttata atgcaggtgg 20460
gtgagagatg ccagcagtga cagggacttt cagtaaaggc tgagctgaag ctgaatttga 20520
aaagcacatt ctttacgttc cattgaagat ctgcacttgc tcttctgcta accgctaagg 20580
tgtcatcagt gctgtgctat taaaatcaga agtcaaagaa ggtgggtgtg gccctcatag 20640
ccagagttga tagaaatgga tttattaata taatggatgg tccatagaaa ggtgaatgac 20700
ctggttaaca gacagggatg gagtggagac ctgtctgccc tggtcttcag gctggttctg 20760
aaatcacaga attaagatga tctcagctga ggggcaaggc aaatcggatg gaagagcgga 20820
ggcccagaga agcgaagccc tttcccaata tctcacagca ggtggggagt gggggaaagc 20880
agcttccagg gggtggattc ctgaaccagg tatttgtcac tgtcccacgt agctcctctt 20940
gtcaccatat gtcccactca gtggagggat acttcctgca agacaaagag ggcctcatgg 21000
ttcccccgtc actcactgcc ttccactccc tgcttctgat tattctgtaa gaagttgagg 21060
ccgagcgcag tggctcacgc ctgtaatctc agcactttgg gaggccgagg caggcggatc 21120
acctgaggtc gggagttcga gaccagcctg accaacatgg agaaacccca tctctactaa 21180
aaatacaaaa ttagccgggc gtggtggcgc atgcctgtaa tcccagctac tcgggaggct 21240
gaggcaggag aattgcttga acctgggagg cagaggttgc aatgagccga gatcgcacca 21300

ttgcactcca gcctgggcaa caagagcaaa actccatctc aaaaaaaaaa aaaaaaaaag 21360
aagttggagc tctgtctgtc tgtcctttcc ccaactcccc atctccgagg ctggctcctg 21420
tgcccaaagt cttcctcgct gtctctgggt atgttcacag ggtatgatca catttcccct 21480
gcatgccctg cgtgggatgt gctccatgtg ctgatgcctc tggggatttc cagatgatgt 21540
ccctggactt ggcagagcag tgtggggaat ctgggacttg cacttcctat cctcatggag 21600
tctggtctct caccttcaca ggccactaag aggaaaggca actggtgtga cgtctgggga 21660
gggaagtatg ttcctgtgca tcgaaagcct caaacatgtg catacacttt aactagcaat 21720
cttacttcta gaaatttagt cctaagaaat aactaggcaa tggagtccat tgctacattg 21780
tttatcccca aattaatcta aatgtctatt gcaaatagac tgcttagatt atggtgcgtt 21840
catacagtga agatctgcac ttcctcttct gctaaccgct aaggtgtcat cagtgctgtg 21900
ctattaaaat cagaagtcaa aggaggtggg tgtggccctc atagccagag ttgatagaaa 21960
tggatttatt aatataatgg atggtccaaa gaaaggtgaa tgaaagagtc ataaaagatg 22020
ttgaggtagt tctctattga tgccagaata gccctatggt gcacttttga gtaacagaag 22080
ccaatttgtt aaaaacaaca acaacaacaa caacaaaaaa aaaacagaga gagagagatg 22140
aatgtgcatc cagatgctta gagaagagtc tgaatgtctg tatactaaat atcaacacta 22200
gttactctgc gctgtgggat tttggatgat tttaatttga tccagtgttt tctggaaaat 22260
aaaatttcac tacattgaca catcatttga taatcagata aaagaacaat ggtgggaaag 22320
caaggactca agtgaccctt gcttttgggt ctagagcatc atgtaggaag ccagcacagc 22380
ccatcccttt cctaggcgcc accttctggc tcctggtggg acttgtaagt cccgtggtca 22440
gttacctgat tgctcttacg gacctgacgc tggaaatcct ctctctccaa ggtccctgca 22500
ggagatggga cctgccacag agaatgccgc aagggatgtt tctgttggga gctgtgagtg 22560
gggtggatgt cctctgcctc tcttaaatag atgagctaga tttgaacctg gggctggcca 22620
aagggagtct cctctcagaa accaggatgc agtgggccgt ctgtgtccca gccccaaagc 22680
agccagggtc cagtggatta gcacagagcc gaccaggcct ggagggtggt ggtctgggat 22740
tgggtgagca gccaccagca ggaatagcaa ttggagggaa ggagaagcat tgccaagagc 22800
aacttagggg gccgtgagtg caggtctctg gacagacagc atggtaactg gctgaggcag 22860
gaagaagctt ggtgggctcc catggtgcta ataataggcc gtgtaggagc aggggaggca 22920
gctccagact tggagtgaaa agacgtgggg tgcagtcaga atgaactccc cagccatcca 22980
tgtggtctca ggcaagtcac tctgcctcca gagccccatg tccactccta taagatggta 23040
catccccaca gtgccctgcc acaggtgatt ataggtgaag gtcttttgac acctatatgt 23100
ctaagggtca tggagtgaca tcttatgaag tcagcacata gaaagtcaaa aaacccttac 23160
attcctgtaa aatacagtgc aggccaggcg cggtgactca cgcctgtaat cccagcattt 23220

tgggaggcca agggtggcgg atcatgaggt caggagatcg agaccatcct ggctaacacg 23280
gtgaaacccc atctctacta aaaatacaaa aaattaacca ggtgtggtgg cgcacatctg 23340
aagtcccagc tacttgggag gctgaggcag gataatcact taaacttggg aggtggaggt 23400
tgcagtgaga cgacacctcg ccactgcact ccagcctggg cgacagagca agactccatc 23460
tcaaaaaaaa aaaaaaatac agtgccatct ctccataact ccacctgagc cagcagttcc 23520
atcagtgttg tgacagatta ctattttttt tttgttgtca agtaccctca ggttgagcag 23580
ctgagatgct tttagggagt gagacaatct caggatctgt ctttaaaccc cgtagtcata 23640
tagggactgg caccaccaga gacaacagca gatccctgtc tggagctcac tgaagaatca 23700
ggggtgggag gaattgccct tttaaaatgg agtgtatata tgtatatatt ttttgcataa 23760
ttaaataata tatatatgtc attgaactca ggctggttag aggcatgcat gtatgatata 23820
aattctctgt atacaaataa aaggaagtta cttgtattcg ttgaatgggt tgttatgatg 23880
actcccttca taggaaacat ttggatccat ttcctctgct ttcgatgttg ttctttcgct 23940
tctgaaccag tgtttgaggc agatgtcttt gatactgagt aaagacatgg cagtttgcat 24000
tcactctctg accaaaagag atgctcccta ctcttgcaga atactgattg cccatgacaa 24060
ccagagtact tttataagaa aaggagcaca cagaaggcag aaagggacct tgtttgggga 24120
ggcggctttg agcagtgact ttatgacata aacgattagg ctcttcctgg ccccgcctgt 24180
cttgggatat ctactcagac cctggaactt tgaaatcgca ggctgggtcc aatgagcgca 24240
gaaaagggac aggactcagg cagataggat ttctttcggg tttggtaaac agctggcctc 24300
taggggtagg gacaggggca gtgggtggga ggacttgggg gagggatggg tgagctcgtg 24360
ggaactgaga aactgtgtct agggcttcct tagttatact gaatgctgca tgtagaggtc 24420
tgcagggtgg ccagctgatg gctggcggaa gatctgctga cttggattca gttactacta 24480
aaatggcagg tttagaaaga ggagagaact tctagatcca gaggaaataa tcaggtaagt 24540
atgctaagat gtgtgtatgt gttagccagg cacggtggct cacgcctgta atcccagcac 24600
tttgtgaggc tgaggcaggt ggatcaccag gtcaggagct caagaccagt ctggccaaca 24660
tggtgaaacc ccgtctttac taaaaataca aaaattagct gggtgtggtg gcgggcgcct 24720
gtaatcccag ctactcggga agctgaggca gagaatcact tgaacccgca aggcagaggt 24780
tgcagtgagc cgagatcatg ccactgcact ccagcctggg cgacagagcg agtttccatc 24840
tcaaaaaaaa aaaaaaaaaa aaaaaaaaaa aaaaaaaaaa aagatgtgtg tatgtgtgta 24900
tgcttatata tataatacat atttatttgt acatatgtgc atgtacagat ataaacacac 24960
aaggatgttt atcatagagt tgtttataaa agcatccaac taacatttct gtatctatgc 25020
aattgaaaac aacatagcca ttcaaaggga tgaggcagag ctatatgaac tgatatattg 25080

ggattgaaat aaaccagatc accaagcaca ttttggatga ttctattagc aggggacgta 25140
tccacaagtt tatagtagca tagggaaaag tcttgaaggt aatataaata taaagcatag 25200
ttataactgg acagtgggag tttaggtcat ttacacaatt attttatagg tttttgtttg 25260
tttgttgact tcttttttc ccaacctttc tttagtgaag ttgtattact taagttaaaa 25320
aggaaataat gagcctggtg tggtggctca cgcctgtaat tccagcactt tgggaagccg 25380
aggcaggagc atcacttgag gtcaggagtt tgagaccagg ctggtcaaca tggtgaaact 25440
ccatctctac taaaaataca aaaaattagc tgggtgtggt ggtgacacct ataatcccag 25500
ccattcagga ggctgaggca ggagaatcgc ttgaacccgg gaggcggagg ttgcagtgag 25560
ctgagattgt gtcactgcac tatagcttgg gagacagagc aaactccgtc tcaaaaataa 25620
ataaataaat aaataaacaa aatttaaaaa ggtaataatg taagaaaata aagagcatgg 25680
agaaagtaga aaaccagata acctcggtcc tattttgtta aattttggga aatttcagta 25740
tctgcattgt acagatgaca ggcagaatct tgagaagtta acactttaaa agaaagaaag 25800
aaaaaaacta gatattcact atgtgtcatc ttgttgacta ttctcttctc tgaacttcct 25860
ggcatcttag ttagcctatt tgagctggaa taaaatgtac tacgtgtctt cagattctgc 25920
aaacatttac caggcaccag ggcgtccact atccagtccc aagcctgcca ctcaggagtt 25980
catgggggag tgagtgcatg tggcaggcat taggcagggt ttttgcattc tgctctccca 26040
cttgatccct gtgacaaccc taggagcctg ctgccactga tcttcttatt ccacttgagg 26100
gaacctagag gcagagacag gatttgaatc caagcctcca gctccaaacc ccagctgaag 26160
gaagcaagct ggaacccaag atgctttcaa tccctgctgc tctgagcact gagatttgtt 26220
cactggaatg ctggagtttt gctgatcatt gacctcgcag gggcccactc tgaagttaaa 26280
aatccccgag tgaataaatc tccagcctct cctgctgttt ccttcccagt tctgaaagtg 26340
agggcaggag agatgagctt tggccaggaa ttcagcgagt gactttgcgt ggagtgtcag 26400
cttctccaca ttgctgggtg ttagcttacc cctgtctggc accagcagca aaaacagagc 26460
caggcgtggc ctatagcaaa gctcccagtt taaatatgtg aattcatttg ggcctggaca 26520
gctgcatagc tcaggctggg cagaatctgc caccctaccc cgtttccttg aaatctatac 26580
aggaacacag ttccaaatac cctggtgcca gtccctgcaa gctcctactt gtacaaatgg 26640
attttaaaac atgtgatgat atctacctcc tcccgtacct ctgggctttt ctaggtgtca 26700
gcatgaatct catgtgagct ttacaatgac cccagagtgg gtgggcaagg gagaggtggt 26760
tatgactatc atgaactccc ttgcttttac aagccactcc cctacaatcc tttctccaac 26820
aagtagacag tgaactttaa aaaatgtcag tcacatctct gccaatgccc tgctcaaagc 26880
cctccagtgg cttcttaatt cctcaagaag ctctagaagt ttctacggga tctggcctct 26940
gtctcccttg gccttgctgt tgccacatac ttctagctat tcctcaaatg ctccaggctc 27000

caagtttgtc actgtctctt ttgaacttgc tgtcctctct tccaggaaca cctcatcccc 27060
cagatactca ctggctggcc ccttctcatc atggaggtct caactcaaat gtcaccttcg 27120
tagagaggcc ttccttgcct cccttcccct cctaccagcc tccttgtcta ttcccctttg 27180
cctcctagca cctgccatca tattgggtat ttgtcttatt gtttggcctg attcctcctc 27240
cgttacttag gcaccaggag agtagagacc ttgttggtct tgttcaccat ctagaagata 27300
cttgaaactc taacatttgt taaatgcagg aaagaatgcc cttttgacag ctaggaaatt 27360
gaggctgaga caagtgaagt gtcttgccct ggtttcccag ctagtgagtg tctgtgctgg 27420
attcaaactt agctcttatg ctccagcctt caagtggggg aaactcaaga gggcaagttc 27480
ttacggggcc acttaggagc agacagcttc ttaggacaac attcttttat gttgatgtga 27540
agtagcaaat ctttcagaga tacaggcctc taaaaaattc tgccatctgc cggctgctgt 27600
gttagagcca gcctcagaaa aaggtcagag atccaatccc tggctgggtg ctgtgcaccc 27660
aggaaagaag gagcagaatt gaaataagtc ttttccactc ttctagatga gccttctagg 27720
ccacaggcag ggcataccca tgcatagact ttaagggcct ttatttggtc tgtttgcttc 27780
ccagttgggg aaacaggccc agggacagga agtgacctgc ctgatgctac ctcatgagtt 27840
agtggtccag gcagttagaa ccaaggtttc ccaatccagc gggagttctt acctacccac 27900
tatagacttc ttggccggtt ctgggagact gagctctttc cttccctgtg tttgcctcat 27960
cagcacgctg gactgagcgt tcccagattc agtgctggag ccagcggtgg ggaggggagt 28020
aggggctgaa gagtatatca gccaagcttg gggagacag tcacataagg aaattcacac 28080
tcttcctctc tcattagggg gccccacaat gaggagggga aagacaaaaa acaaggatct 28140
tgggtacatt ggtccttacc tccccactgc cccaggagcg gtcttgaggg ttggaggaga 28200
ctaccagcct cctccctata gcggacttga acttggcaca tggaaccagc tgaaactggc 28260
accatttcta ccctcagagg gtaccgctga agaagacggg cagagatgaa agaggaactg 28320
cttaattcag ctcggcactt cttagcagct cccccatccc attgccggct ttagtcccag 28380
gccagagacc catgaggtgc gttgaacatt actgataccc tctttgatct aaaccctcct 28440
ttcgtgatca cgcctggtgg ctcaaagaat tgctccccca gcttactgtt cctgttggtg 28500
ctgaaagaat gctcgtaatt gttaccaaac agtggacgga gcaaggcaac atgtcccctt 28560
tgcccagctg gctcccagtt ggcacttcat tatggcaaag acagaggggg tggggtcccc 28620
gggccttcgg catttcattt gcacccacat gtaggctcac ttcttggcct gggaattgat 28680
cggtgccaga tgctccatct agccagcatc cccaccgcag cagggctgcc cgcctcctgt 28740
ttcgtgaaaa acggcccgga ttttcatcag accttaattg gcctgggtct ggccttcatt 28800
ctaccccagc ctcttcacag actgctggcc actggctgtt ccctcctcca gccaggacaa 28860

tgccggtccc cgggcactga ccctggccca ggcaggcctg ccaactccat agctctcatt 28920
tctcactctg cacatcagtg ctggcttccc acacccacag ctcctccaga aggaaggttt 28980
gtcagcgggc ttcaccatgc actctgacgt accacttttg gcccaaccaa actacacccc 29040
acccttgggt agaatgggac caagatgcga ccctgacaga cttaaatgag aaatgagcat 29100
tttccagtga cgcctcctct tcctgcctct tgctgggcct ctctctgcat tgctctgggg 29160
tgttcactgc acttggactg ataccagtca atgtccctcg cttcaacctg acctgaaact 29220
gaaatgctcc aaacaagagt aagtggggca gccgacattt caggaggagg aaggttggca 29280
gccaggctgc tgtgagcttg cacggcccat atgggtccca gctagtactt tgcattttct 29340
tttctctctt cttggaaccc cttcgtcctt tttcccgctg ccctctagcc tcttcactgt 29400
cttgggaaag gggcctctc acaaaacagt agtcaccgct gctgttcact gagtggttac 29460
tttgcaccag gcactgtgct aagtacctca aggggagtat ctcccttaat gcctgcaaca 29520
tcctatgagc cttgtatgat tgcatctccc ttttcaagct gaaaagaagt tacatgcttt 29580
gcccgacagt ctatagctag gaggagctgg agctgagatt caaccccagg ttctctggtg 29640
cccaagtgct catgtctttg ccctgtgtgc ccctctttca cagaaacaga gcttcagcca 29700
aatcctggaa cggaagctct tgggaagctg cctgtgacct ttcttattgt gtcccctcag 29760
cacaggtcat gcacaggcag ctgggaatag atatgcgagt taaaggaatt ctcgttggaa 29820
aacaccagag ggatccacag ggccaccccc ccgcccccgc aataccactg tgtgtctgtc 29880
cctgcagggc cctggaatcc cacagttgtt tcctgtaccc aggcctgggg agccagccag 29940
tcacatggac cgtgagtgta tccgcctcac gcttctctgt ggcccctggc ttctcccagt 30000
ggattccgca aggtgtttcc tgcaaatcct gctgatggtc ttcgctggca cgtagcaaaa 30060
tccacccaga acaccgcgat ctgctggcct cagcctggat ggggttttgt ttttgtttag 30120
cttttaaaaa aatgtgtgtt ttttttttccc caaccagggg cacaggggtg ggggaagagg 30180
agaaacatga aggtaaaaat attgacagag tgaaaagtat tgaatattcc ttctatttca 30240
aaaatagcag cttccatggc tgggatgcag gctggctaag ttcaggttgg gctgaggttg 30300
aagttgagat caaggtggca gcgtgaccct tatgcctcaa gcatgtcctt caggagggtg 30360
tgggagcctg aggcagaaga gagctccttc ctgttttaaa aacacctttt tctgagactc 30420
tcaaagaatg tttgaaatca gccatccaag gtgctctgcc atttttaaga gggaggacgg 30480
ggctggatgg tgtccctggg aattgaaggg acccagagaa gaggtgaatt gtttaaagtt 30540
aactcagagc attagagtgg gctgatatcc tctgaagagt ctacttgtct gtgagagctg 30600
tttcagctac ttcaaagcca gatgatcagg gcagcggggc tggattttca tcttcatggc 30660
tgtgcacatc tccacttgat atgtgggggt cggagttggg gggcagtaaa gcctggtccc 30720
catggagatg ctatcctgga agcattttta ttaccaagga ctagcatata attggaagat 30780

tgaaggcaga agcccttatc ttctcggcat tctttagaat tcttctgtat gtagatttca 30840
gaacacacag agccagagca aaagacctta cttggcatta acaccaggat ccacctgccc 30900
ctcccttcct ggcattaatg tccagtgctc ccctaatgca ctttaagctg tgaagttatc 30960
ccaggtttat ttacactgta actaatttac tcttggctgg actgtcagcc tggaaaatag 31020
gatgtcactg caccccccccc aacctccacc aaaaaaagac agtggctaat gaagaaaaca 31080
gattgttgaa gggcactaaa attgaggcaa ggagctgaat aatgtgccct cctcaggggc 31140
aggcagggca ggcgtggcag gctgtcggca ttgttacaga tcagcaatgt cccactagtt 31200
agcaaatggg cccctctcag tttgaatgtg cagctgatgg accaggtggg gtgattaggg 31260
gccaactccg tacttgtttc tgtccccagt tttgttaagt gcagatcacc cagacagatc 31320
ccaggctgag aaagagactg aagacaaagc tagaagaggc agctgaagca tcctaatgtc 31380
ttaactcaag tttgactttt cagaaatgtg attttagaac atagcaaaca ggaagcacgt 31440
gacaacttgg accactacaa cgaagagaaa gtggaaccca ggggaaatgc atgagttaga 31500
aatataaagt gatgggactc ttagattcta gggttaattg ggacactaga atatgtccag 31560
tccaacgtgt tcattttgta caataaaaaa caagggctcc aggaggaaat gatttgtcaa 31620
actcacatag aaaatgaatg gaaaaaaaca gaaagagtat ctagcttcca attcctggcc 31680
cagggctctt cttgctattc aatactttct acgtaataag cctgggtttt tttttttttt 31740
caaaaaaatt attatacttg gccacccaga tgagtacagt tatttagtag agtacgtaga 31800
gttattcact aaatcacatc aaagttggga gacagagtct ggtttgaaaa ccctcgagta 31860
ttttatttta ttttatttta ttttattgtg ataatactta acatgagata tactcgcccc 31920
tctcttttc tgagacagag tctcactctg tcgttcagct ggagtgcagt ggcatgatac 31980
tggcttcctg cagcctcaac ttcctaggct caagtgatcc gtttccctca gcctcccaag 32040
tagctgggac tacaggtacg tactaccatt cccagctaat ttttttttcg tggttttcgt 32100
agagacaggg ttttaccttg ttgcccaggc tggtctcgca ctcctgagct caaggaattc 32160
acctgcctcg acctcccgaa gtcctgggat tacaggcgtg agccaccgcg cccagcaaga 32220
tctaccttct taacacattt tttaacacta gaagtactta aatagatgat ggtgaagtct 32280
caagctgtcc taccagcagt aaaaagattc caagtgggga aggtgaactc tcaaccttgt 32340
atactgatcg gtggctcctg ggaaatttgc atttatcccc taacaaagcc attaagagag 32400
tcattggccc ccagcaagac tgagatggtc gaaggacatg ggaccatgtc ataagcaggg 32460
agagaccatc ctaatctatg aaaatattta tcctggagat gatttgggaa acatgaccct 32520
tcttttcaaa tatcttgttt ttttgtaagc ccaagatgtc acctaggtgg gagaaagcca 32580
cagggaagca gtccaaagga gaattttttt atagtcaaaa aaaatggctt gacatggaac 32640

aggctgacct aggtcagaga tggttgcaca ctgggcagtt taaccttggg atgggactgt 32700
agaagaaatt caaaggagct tacactgagt gaccctgaag gtcccaccca acctaaagtc 32760
tgtggcctgt gaaatgtgcc catgccactg tgccgtagtt taacatgtcc ttctggaagg 32820
caggtaggac ccagagatga gaagtagggg ctcccaattc aggctgcctg gactggcatg 32880
ctatgtccac cacttccgca ttagatgatc tggggagagt cacttcttct ttacaggcct 32940
cgatttctaa aagatgggct aatgacaaca cttactttat agggccctag tgaggctcaa 33000
atgggaaaat ccacggaaag catttagcac tgatcatgca tggaaagtgc ctgatagctg 33060
atggcaccta ttgtaactaa taatagtatt agaattagta ttgtaatcat tgacaatagg 33120
atttttataa ttagggaaag tggggaacta cccacatgac catcctcctg ctttctgtgt 33180
cagtgtttga ccaaatgtgt tcatccctgg tactctcatt gcttatcctc cgagagcact 33240
ggcatataat ggaaaatgta ttccaatttg ttctgttatt ccagccagga acatctagga 33300
acaggggttg gaggtgtgct tcagggacta cctgagccca gctttttttt ttttgagatg 33360
gagtctcatt cttcttgccc aggctggagt gcagtggcac gatctcggct cactgcagcc 33420
tccgcctcca aggttcaagc cattctcctg cctcagcctc ccaagtagtt gggattacag 33480
gtgccctcca ccacatctgg ctaatttatt tttttttttg tatttttagt agaaacgggg 33540
ttccaccgtg ttggccaggc tggtctcaaa ctcctgacct caggtgatca gcctgccttg 33600
gcctcccaaa gtgctgggat tacaggcatg agccactgca cccggccaag cccacctttt 33660
gataggagcc tcccatgggc aggcagagta agaagtgtca catgcagcca ctggtcactt 33720
aggcaaaacc ctgccagctt tcagatgtgc agtggaggtg gcagggggag gtggctggtg 33780
ctgtgtatgt tttgcagata ctctgtggtg tggcaattgc tctagaggac tctagtgtcc 33840
agaagcaggc atgtgcagtt tatcattaca gccctccttc aaagaggaca gctggccctg 33900
tgctttgcag ggagaggtgc actccgggtg gcccataaac ccagaaaata tgattcctat 33960
ctggtacagg gtaccatcct ggcacctgga gaggggtctg catgagaccc agaaatgact 34020
ggatccacaa cagacctagt ttgtggaaca gtcctaaaga atcctccatt caagcttata 34080
tctgtcaccc agggaactgg acattccctt cagtcagaat gaactagtgg cctccccttc 34140
tcttcagcca gggtctttgc agtcctcagc catgtgacct ccgacctgtc agtcttggtc 34200
tgattgctgt acccttgttt gtgctgtttc cattgcctgg accatccttc ttttctttcc 34260
atgatcaaaa ccctatacat tcttctgagt cagttcatgc agcggaattg gcattggaat 34320
gttgcagcta attgcacata ctgggtgaca gagtgcacag tagagaaagg tagggtgata 34380
cactcttctt aggaagcaat caataagggc caagatggaa aagatgtctt atttcaccca 34440
ctgagcacct actatgttcc agacactgtg agatgttgcg gttagataca attgtgagca 34500
aagcaagatg taattccggc agaatttagt gagtgtatgg gggcagggaa gaaagaggat 34560

gagacatgat tcaaatgaat ctacgaacat atagttacat acacactagg gcccctgaga 34620
atacaaaaca ggggttctgt tctagtctta tgcttgcagg aggtggttgg ggatggcttt 34680
atgtgaggaa gtgtttttg attttaaatg tgaagtatga gtggcagttg atgggtaatg 34740
gggagcagaa gagggtctca tgcagataga atggtatgtg caaaggcccc aaggctttgc 34800
aactgagaga tttagaccag ggctgccgat cagatgtcta aaaaagtata cttgttcgcc 34860
ccttgccata ctctttggtc caaagggctg agctccctgt cttcaaacca gggggcttca 34920
ggtcagttac tggacttctc tcttcctgcc ctttgtctgc aagccaaggt accctcaatc 34980
tggcattgag agccctgggg cccacccagc ttgtaagtgg agatttgaat ccagacttca 35040
ccattggatg agattcttag acattacaag agttatggag agttaactcc acctcaaagt 35100
tctcaccatc tgtaacttat acaagccctt ctctcataat tcagtcttgg gaattttccc 35160
tatctccgca tttccaatgc atttgacttg gtggggtgtt tcagatttgt taaatattta 35220
gtagtagaaa gaagatgagc tttgctgctg ggcatatttg gattcctagc tgtgtgactt 35280
ctgacaagtt tcttaactat cctgagactt gttttcttcc tatactcagt ggaaataaag 35340
ctacacattt tggggaatgg ttttaagagt aagatgtact gtttgtaaag tgtgtctgtc 35400
ttgttcactt ttctgtttct gccacctgac acaatgcctg gcacatagta agtgctcagt 35460
gaatatttac tgaatgaata agaggtttag gcaccatgat tgcacagaca cgatgttcat 35520
agagtagttg gtcttctcct tctttatcaa tgactccatc tccctgtcac acttcaaggc 35580
cagggcaggt ggttcagaaa taccagaggt tggctttata gttctgccat tacccacttt 35640
gaccaaagag ggcagcagtt agcattcttt acttatttat tttatttaaa atgaaggttt 35700
ccttaaaagg atttggatct tctctttaat cattcctccg caccttcagt gctattgatt 35760
cctgataatg gtcttagggc ttgaagacct atgtatgggc tcgtttttct cgtacctgtt 35820
cctacctgag tgcccactat gtgagtgatg tggttgtgag ctggaaggga ggtgctgagt 35880
gagtagggga acacccgctg cagcaggtgg gccttccaaa ctatggccct gggtgtccac 35940
aaccttacag aatgaagctt gtggaactta aaggtgagga aagcctacac tattttaaaa 36000
gccacatgaa gggtaagtta tagatgtacc agggtctcct tggcatctgt gctgtataaa 36060
atccatagac agagcagcca ttggctcttg ttcaaaccac aagaaagtct cctgcccact 36120
cctggtgcat tctgggaaca gatttgctgt ctgcaaaggc tgcatctgcg ggtctggcgt 36180
gcaaaggagg gtggttcttt ggctaatggt ggacttgctc ctaggcctat cccagagtcc 36240
ctcagctctc ctagcaaaca aaatcccaat taggagaaaa ataaaatgct atagatggct 36300
cctgagatgt tgccagctgt ttgggcttca aaaatgcccc gacctgaaag ctggaagttc 36360
tgagaaccgt ttgagggcat ggtatttttc tgggcactgc ttcaaagggg ctgtggttga 36420

agaccatgct gctattctgg tggccacttg tgtgggataa agtttcccat gggcccccgt 36480
ggagaggctg gagcactctc aggggatagg gggtggtctc agccccccctc accgagtgca 36540
cttgcatggc agtaagcaag tcggcgcctc tgacccctga gtgggcttga gcttttgaga 36600
ggtcagcatg ccccccttaaa cttgatgtct cctcagagca cagcacagat gcccagccaa 36660
aacacccagg gcgaaaccac tccaagaggt gaaagggctt gaggggagac agggtacggc 36720
gggagacaga gatgcatgaa atactaacgt tgaagattag aaatgggatg tgcactggaa 36780
tgagatcatt tgtggttctg atgcaaagac ccaggcttgt ttctaggggt ttcttggggc 36840
ttcgggggcc tcctgtcaat ggactgtttg gacacccatt tggatacatg caatcctaaa 36900
gtctatgggc agtgacagag tgataagtct ttatcactag cctcagggag tagaagccca 36960
cctaagaaat ggcactgtcc catgttggag caagacccaa cctaccaagc tgggagttac 37020
cgcccactgc aaggcacccc tgtactttgt tggactctct gttttctgtc tctccaatgg 37080
gctggactct ccagcctccg ggcctctgcc caaccatgac cagaaggtcc tctcctttct 37140
ctttctctta actgtctcta gtgattctct gatcccagct caggcaggat gtctccctcc 37200
ggcgcctcca ggctggctgg gtgtgtctgc tgcatgctcc tgtggctccc tggactataa 37260
ctccctgtct tcttgtctca caagagtaaa ataagcttct taaaggtagg aaccttgtct 37320
gcctgcttat tactgaatcc ccaagacatg gcacaggatc tgagatgcat agttcctcaa 37380
atacttctga ataaatatca gatgagtgaa tcaatgaatt tcaagtggac ttgaggagaa 37440
ggaggggggg tggcaaaata aaacaaacaa taaacactat ggcagagagg gacacaggac 37500
ctggagatgg ggcctggata aacagcctgc catgctcttc atatttcctg agtgctacca 37560
caaggaagag gggcagctgg aggccagagg gcctgcagag gagtcagcag gtctagcaag 37620
gagtgccttg ggtggagact ccctggggct aattttggtg tcaccactga cttccttgag 37680
ggtgctgagc cttgggatct tcatgtgttg gtagcttttg ggtgctgtcc ttccacagac 37740
acatcaacaa gttcatgggg gtctcctgta atagggtcct gtgacttgcc tctttttgga 37800
ggtcagtctt caatattttg gaacaatgtc ggtattcggc ctctccagat cacattttta 37860
cttgtccctt ccttttctga gtaactctat ggaacaagga ctcctgaccc agcactccag 37920
gaagccaaac acaaaaccat ccccgtcttc gttattgtat tacctttggt tccctcccac 37980
agcctcattc attcattcat tcaacatttt tgggaaccct ggccacaatc taggctctat 38040
gccatgtgct ggagtcccag atgcagtccc tgccatccgg taattagcga tgctagtgag 38100
tgtccaagga accaatcatt aaagaacacg tgctggaaga aagagaggaa agccaagcag 38160
aagagaggtg gactagcttg gcctggggtg gacagaaggc ttcctggagg aggtagcatt 38220
tcagctgggt gggtcttgca ggaagccaaa ggtgcaggca gatgcacagg gaggaagtga 38280
ttctcaccta ggcaaggggc atcataggac aggttcatat agtgatgggg gctggaagct 38340

tcagagtgat agggtttgag gtatggtccc agctctgaag agccatatat atgccaccga 38400
gctatgcaga ataatcctgt agatactgca aacccaaggg aggtgtaggt gaggatgtaa 38460
atgagggttg tggatcccat ttgttcctgg gagaatgtgg gggacttagg ggagaaggac 38520
tgtgtttccc actgtctatc ctttggggct ttgacatttc ccacctgaca gccatttgca 38580
gagtaaaagc ctcagggatt ctaatcatgc ttggtgaaca ctttgaggta gtcgcctcat 38640
tggaccctta gaaacccagg tcacagaaac accatcaccc ttctgagatt cacagtcctc 38700
cctccccagg taagtcggcc ctgaatccag gaatcagcca gccctaggac ttttgccctg 38760
tcccaggcca aggtatctgt acctttcttc caaattgctg gtggccccag gaagaaatca 38820
agagtggctg actttaagca gttagattaa acatcaaatg cattcatccc aagtccaggc 38880
atatacctag aaggagcgac tcttaaattt tgtcatttca ttgtgtaaaa ctgaggttgc 38940
cccttaacac atgaaggcca gcatgggatg ctaattttgt gttgtgttct tttgggtaat 39000
ctcaaagttc caggaagcct gaccttccta agcctgtgtt tccttgcctc cccttcccgg 39060
ccccttcatt tcatctacaa cagacgtcag caggggccct gagtttttcc cagaccgctc 39120
aagcagaaac ccatcctgct cacaaggtga ggagagccag gagctcacaa aggcccctcc 39180
tccccttccc cgggaggtcc aggacagggc atccccagga cagacctgcc ctggggaccc 39240
cggaacacaa ggcaggtact gagaaacagg aatctctggg cagggaggtt ttcatatcct 39300
ccaggtttaa tagctttccc tgagatggaa acttgggttg aaatgatttg cagctttcca 39360
gattttctga gtgtgtgtct ttgggtttat ttttagccac aggcattcat ttcttgccaa 39420
aaaaacctgc ttaaataaaa ccaagattaa tatttttact taacggaaag agttagtcag 39480
gacaggcttt gaactgggac tccttatctt gatggggagt gcatgttaac ggtctggcct 39540
tggcgttgac atcaacgaaa agggcctttg aaacggacaa gagagaaggg ccctggggga 39600
ccagggtagg atgcaggaaa agcctatttg tgggacttct ctgaagagag agagttcctc 39660
ttcactcagg ctgctcgccg ctccccaccc ccagccccca gcccccatgc tcttgcagtg 39720
ggaggggggt catcatcttc ctggaaaggc gggagatcgg ctctggcttt gctcagagcc 39780
cggctgtgat tctgcctcac ttccagctgc tctccttacc gccgcccccct ccgcccccca 39840
cccagatcag atccagactt cttcctcctc tggattcaga cgcagcaaac tgagtctctt 39900
ctgcaggatg ggctgaggct cagatctgat gccatctcca gggcgattcc ctgacacccc 39960
tcccctgaga tggacttcat ctcttcttgg ctctgtcctg gctttccgtc cttgcttata 40020
ttgctgagcc tgccttactt gagtgtcatt gctggtctct ctcacctctg gcccttgagt 40080
tcctaggagc cttatgtatc tctctatccc tgtatacgtc ttcacaatac tgaaacacct 40140
tcctggttgc tgaagagttg tgtatctgac cctgtgaacc gagaattgct gttacccccct 40200

tttgattgaa taagacttag aaagactggt catttgacag agcttgcaca ggaattattt 40260

atctgttttt aaatcactct gtgtgtggca ctttatacaa gaaatcacac gtagcttccc 40320

atattcagca aatacatatg aggtgcctgt tatgtgccag gcactcttcg ggcagctttg 40380

gctacagcaa taggtaaagc aaagaacctg ctgcccgggg aggcttacat tttaatgggg 40440

agagatgatc agtaaagaag caagttaatg tataatgtca gatggtggcc agtgtctgat 40500

agagcacagt gtcaggggcc aagggattgc gaggaggggg aggaatgtgc atggatcagg 40560

atcagggaag gtcccccgcc caagtgaaga aagagtgttc cagcagaggg aactgcaagg 40620

tcagagaccc tgagagcatg cttgggtgct agggtgtgca gtagagtgag ctacaatttc 40680

ctgagattaa gaaggccatg agagccccgg gtgcagggaa ggcctgttat ctggagctca 40740

gtgttggatg ggtcgaattt gagatgtttt attaggcttg caggtataaa tgtagactaa 40800

atgtagactc tgggatatat aagtaaaaag atcatcaggg aggggaacaa catacactgg 40860

ggcctgttgg tgggtggggt aggggaggga gagcattagg aaaaatagct aatgcacact 40920

cggcttaata cctcagtgat gggttgatag gtgcagcaaa tcactatggt acatgtttac 40980

ctttgtaaca agcctgcaca tcctgcacat gtaccccaga acttaaaata aaaatacaat 41040

ttgtttaaaa aaaaagttca tgagagttct aggttggcgg tggcaaaagc gtgggagctg 41100

tcagttgtgc agatggtgct gagacccaca agctggaaga acatagcagg accacgagcc 41160

tgcacctcga tgaccggact gagcaccaag aaggtcatgg gtgacttcga caagcactgc 41220

tttggagagg ttaggggga ggctgcctag gttattgcag gcacctttgg aggctggtaa 41280

ttatcaccca caatttatct gctgggatgg gaagtgactt gcccaaggtc acagagaaac 41340

agggcagcga cttcacccag accccacgtg ctccatgcct gtttttcatt ccagtacgcc 41400

gtgatcactt ctccagtttt cttgatctgt cctttcagct tctgagtctt tcaggagggt 41460

ttttttggga gttgggattg tggtgatcaa taggctggga atcagaggga cccagtccag 41520

gcaggcccct caggcctctg cttttcaccc ttagttccga aggccctttc ctctcaccta 41580

gacatcatca aagcatcccc agagaggcag cagatatctg agaaggacct tgggaagggt 41640

cactgaggaa ggttctcaag atcctggggg gatgaggaaa tgctgaatca caagaatctt 41700

tcgggttcct cttgctgttt tcctctgatt agtaaattta agaatctaca agtcatcacc 41760

atcagcaata taacagtgta tcatttgtct agcttcaggg ttacaaagta cctttgtaca 41820

tatacattgt ctcatttaat taatcaatct aattaacttc aaacttcctg tgttgggaga 41880

acccttaggg agcctgtggt tttgtggttt ctaagttatt tgtttttatt ccatgaaagc 41940

cttgccgggg agcacaaagg tctaaacaga tgatacgata tactgttata ctgctgatgg 42000

cgatgacttc tagattttta ttactaatca gaggacatgt gtttgtgtgt ctgtgtaagt 42060

gattgggaag ggtggtgggt gggtgatcaa agggatgtgg ggctcactgg gagcttatca 42120

agggtccttc cttttaggta tcaatcttct tctctgatcc tctgcagtga aagtggggaa 42180
tgcagtaaaa ttatgaacta ttttgcaacc agtgtgaaat cccatgtaaa ggtttttagg 42240
tggtctgctg tcctggggga cctcccattt tttttttcc cccactagga tgcaaaactg 42300
cttacaggct acctgatcct cattgtccac agttggaaag tagggacatt aacagccaca 42360
acagtagcca ccttaccagc ttgccatgag gacagagtta aagaacatgc acacgttgct 42420
tggcacctaa gaggcattcg ataaatggta gtggttgtta tcagtatgtt ctagttccct 42480
tccttctcct ctagattcac taatgtgatg ctaagaaaag caagcagttt gtaattcttg 42540
aatgggtgag cagctggaag agtgagagag cctggctctg ccctccttgg gcaggctaca 42600
ttgtagagtg tccaaggtgg gcaggaaaaa ggcttgcttg gcgggctcca ggccgacctc 42660
cctgtgggtc cctgacctat tgcctttgcc atgagcactc cggcctcagc aaggccgctc 42720
cctttgtcat cctgtgggag atgataaaat acacacgaag gggccgtgag acaagctggg 42780
tgacagggat ccctctttcc tcctttttgg tgtgttttca tgggtagggc atcattccaa 42840
cacttccaga tccctctttt cctccaagtc taggccgaag atgcgagccc atggtccagg 42900
cttttcttcc tacctagagc ctgcctgctg gggtcccatc agaagagcag ccccttccct 42960
gggactgaca ctaacagtga cttctgcatt tgaagaaatg cagtctaccc actaaccaga 43020
cgtcccttac ttatccttgc tgtcctttta aaaaccatgc cttaaagcag caaaatcatt 43080
ctatggggtc tttggagaga caggaatggg ggtgttgtgt gtgtgtgtgg ggtggcggtg 43140
gtatctcacc tgctagatag catgaatcaa ctcctaagtc atggagagga tttgtactgc 43200
tcttccgttg atgctaccat gggtggaacc tggactccta agttagtata cattaattag 43260
ttaaggagtc cttgatcccc agaccagcca aatgggtagc attgttgctc ggccttctag 43320
tctgccagta ggaaagtcca accattaggt cggggaagaa gggtctggat ttggttgaca 43380
atggttggat gggggataga agcagagaga gagagggagg gcagctcaag ggtatcttgc 43440
cccactctgt ttatgctgat gaacagcaca tgtgcaattt ctggagacac accagtaagc 43500
ttttctggaa actcaatccc aattgccatt ggaaaattgt tctttgttca aaacttgtgc 43560
cactgtggtc catactgcta gagctggagg tggggagagg cagcatgaag gtatatttat 43620
ggaagaggtg tgccttgttt gccttgcttt tgtaagctct tactagcact cttgcccaaa 43680
caaatattca aaggaagcaa agacagttca atagagatat atgcctctat gcctggcttc 43740
tcaggcgcat gatatattca agagggcccc atatatattt atagatcaat gtgcccatta 43800
tataagttca cacatcctag ttagtttctt cttagatact tttacattga aatactgctt 43860
attccttaat caatctcttc cttgtttctt cttcttcctt tttttttttt gctgagacag 43920
agtcttgctc tgtcacccag gctggagtgt aatggcatga gatatagctc actgtagcct 43980

cgaactcctg ggctcaagca atcctcccac ctcagcctcc tgagtagctg agactacaga 44040
tacacggcac catgcctggc taattgtttt aaatttttat aatttttat tttttataga 44100
gacaaggtct cactgtgttg cccaggctgg tctggaactc ctgagctcaa gtgatcctcc 44160
tgccttggcc tcccaaagtg atgagattat aggcatgtgc caccacaccc agcctcttcc 44220
ttgcttcttt tgaccaccag atccctagaa gcccagattc tcttcctaag agcaaaagaa 44280
attctagaga gacaagagag gctcagaggc tgagattcac actgagcctc acctctccct 44340
tctctgattg gtgcttctgt accttggaga ctagtcctct agccataaac tttttttttt 44400
tgagatggaa tctcgctctg tttccaggct ggagtacggt gatgcaatct cggctcactg 44460
caacctccac ctcctgggtt caagtgattc tcctgcctca gcctcccaag tagctgggac 44520
tacaggcggc cccatgccca gctaattttt gtattttagt agagatgggg tttcaccatg 44580
ctggccagga tggtctcaat ctcttgacct cgtgatctgc ccacctcagc ctcccaaagt 44640
gctgggatta cagtggtgag cctccacacc cggccagcca taaactttta aaccttatct 44700
ccccactaga ctagccatca gtggaagcgg gaaataggac ttctatttct tgttgtcacc 44760
ctcaccagca cccaacccaa tgttccagac accaacctag ttcatgccta tgttggtttg 44820
attgttgggg gcactgggta gacagtgtag ggccatact ccagcatgaa tgggaataaa 44880
gacaacctgt gtccagcctg gcccatctgt aaagctggct tctagccacc acaacaaggt 44940
ggaaggaatg gctagacatg ctgccagtta ccagtttgaa aagtgtcttc tgttgttgga 45000
aaagcacaac ctcctcccac ccccacttccc ttgttccctg tggcccctgg gccagcgaat 45060
gcctcgggag aaccctgatt gccaaacagg agagtacaaa atagcacaca tttgaattac 45120
agaggagaaa ctggagcaaa tacggacaga ttaatgcaca aaccaaatag gtggatctgg 45180
ggttggccag caattctact ttggaaggac tttgagaatc tatttggagg gcagaatgta 45240
acttaatttt agttcagttt agtgaagttt tgtttttgta aggaaaacac tgatgtggcc 45300
tcttcactgg gccatctctg cctcagactt ttggaaccat gactgtgatc ttgtttggct 45360
ggtcttagta ggcaattctt gggttacaca tgggtgataa ggtgggatgg gtacatgtga 45420
tagagtaggc actcctgatc ccaaaatacc tcctatactt gttctgacta cttcatctgt 45480
gcttctccac ctctccctga agttctgtaa ggctagaata tttttatgag gttgcctttc 45540
ttctcatcct agggagaact cccaggcctt agcttacagt gcatgttaga agtctgaaga 45600
tggtatgccc taagatggaa taaaaataga catcaccata gtggtgataa tgctaataac 45660
agtggtagca aacacatgag gtttgggatg gactacatac tgttctcagc gcttatatta 45720
atacgaccct cacagcaact ttatgaggca ttgtttcatc attcccatct tacagattgg 45780
gaaaccaagg catagagagg tttagcaact tcctgaaggt cacccagcac cagggctggg 45840
atttgaaccc tggctctcag gctccagaga ccaggctctt taatgcaatg ctatgttgct 45900

gctccaatag taataagcaa atcacttgtt agccacatga gaggcactgg gctaagaaag 45960
ccagagagat tcaggaggaa agagccccag agagatcata cagtaatggt ggaacccaaa 46020
cttcaatgca gcttcctgac tacaaaaacg tattcttttc actgaactat cctcagatga 46080
aaaggttcca agctcaggct atttggggta aggaaggagt tggctggttg tatgtgcaga 46140
agcttccagc agtactcagc tttatggggc aggtctgctt gtgacatctt ctgtctaaat 46200
caagtcaagc catttttgaa ccaggatgtt gctatttaaa ggaatccctt ttgccttgcc 46260
cctcccacac cccccggcca agactcgctc ccttgtttac cttctgtgtt aatgaggcag 46320
agaagatggg tccaaatcat ttcaggaaga atcagtggta aaggctggca gacatgcctc 46380
tgcacggagg tgaagctgtt ataacctgcc tgtgagagtt aatatggagg aaacagtccc 46440
cggggcctcc gggaggctgg cttgtcgccg agaatactac cgcaatgtta gaaacagagg 46500
tggatgcagg atcttgacag acattggcct gggatcttgc caacctctcg tttttacagc 46560
agacttggaa acaaccactc gcctcctcct aatttgagct gcctccgcta aattgctcac 46620
tttgccaatg actgtgtgtt ttgccatcgc cggcagggaa ggtctgaagg ggccattgat 46680
tcatctgaac cctgacttca tgtctgactg caagggtagg atccaagctc atttggaaag 46740
aaacaagaca gaatccagta tggatgagga atcagttctt agtaaagatt ttttaaaaac 46800
agagattaga gtcaaataac tgggatacat agtggagcat ccacaatgag gaaggataat 46860
ggagctacca aaatgtgtgt gtgtgtgttt gtgtttgtgt gtgtgaaggg tattttactt 46920
aacaagcatt tattgagtat ctactccttt ttgggctaag tgcaacttga gataagaaag 46980
attgatagga tctcagggct gaaggagttc aatttgtaat tcaaagtatg actgcataag 47040
caagccccat tccaattcct gttgaggtct tagcatggaa gtatccagca ccctcatcac 47100
accccctgat agaggcactc tctgccctaa tggcagcccc cctgcctttt gttgctggtt 47160
cttattatta gacgattttt ttctttggtt gagccaatga atgttactct ctgcaatttc 47220
cagttttggt attcaagacc tgtccatttg ctcctccatg tgccaatttt acaaatacct 47280
gaagacagct cttgggtgct tctttttcac acaacgtaga aagagaagca caaccttgta 47340
gaatctaaaa gtcaagtcct ctcctctttc tgttcccagt gcatgtggta aatgcctctc 47400
tttcagccct caacccactg ggtgtaatta cttttgtatt catagttaac tagtatctac 47460
tgggtaaatc ctttctgcca gacattgtag tgggaaattt cataagatct ttcgttcaat 47520
cctgattcca attccctgat gtaaagagag ttataccctc tttatagatg agaaaattga 47580
ggctcagaaa gaggaagtgg tttcacttct tggtctgtta ccttatctag atcatgtgga 47640
tgctaatact gcccttgtaa gacagttaag agcagtaaat aagtgaaatg tacatggcac 47700
atagtaggtg tgcaataaat gggagctgtt ttcattggaa cagcccttcc atggttagcc 47760

catttcattt ttggtgatga taatgatgat gatattaata atatttgtct agtgcaaact 47820
atgtccaaga cagtatcaag agtttcacat ttgtcatctt gactaatccc ttcagttcaa 47880
gagattagta tcattatgat tcctgtttta tagaagagga ggccagaaac aaagttactt 47940
acttgcatac agtcgcaata gccataaaca gcagagctga gattggaaca gttctgtctg 48000
gctcctgagc cccgactccc aattgctcct tcttctaacc acatccccat agagctcaga 48060
ctctattgca tcccttgcta tctaagcact ggataccaag ggctcaatca gaatgctaac 48120
tgggggcact gtgggaagct agggtgcacc acaaccctgt agacatgtca agatgtggtg 48180
aattaatgca tgcaaaggaa gagccccaaa tgaggtggtt agaaaggggt cctagtggaa 48240
agagaacaga tctgggagac catggcattg tcagatggac aagggagggg ggacaatggc 48300
tctaacagga gaagtaggtt ttggattatg gtaaagtcac aaagtcaaga aaatcctcag 48360
ggtcggcggg ggttgactat agccttggaa gttctggagc tggcaattgt gaatatttgg 48420
ggtaattaac ttgaatggtg gctgtttctt tttcctccaa cctccctctc accccaggga 48480
attcccactc ttgtaaacac aatcatctat tccagttggc tccaaggtaa gagtgcaatt 48540
ccaggctcca cagaggctag cgtgcccgcc ctgagatgct gttcctgatg tggttcaggc 48600
cttcctaacc tcttattgtc tccttctgtc tctgcagtga tccgggccaa ggtggtgggg 48660
aagaagctgg taaaggaggg gcccttcggc acgctggtct acaccatcaa gcagatgaag 48720
gtaggtaatg tcatcaccct ggctccggga aggtttttgg gtttttgcca gaagagtcct 48780
ggctaaggga ggcaaagtgg gttggaactg gggtgtgtgt ctaagcacca gccagaccca 48840
gcccttctgc ctgctggaga gggagctgct aaggctgcat aaagggatgg gtagggaggg 48900
ggcatggggc tgccttgtcc aagagcccat gcaggtggag tcactccaag gcacttgtta 48960
ggaagctgag ctctggagcc agatggcaga tagccttgtt agctcgtaac catgagacct 49020
tggtcaagtc actgcccctc tctgggcttt aatcttccca attgtgaaat gagggggttg 49080
gactagatca tctctatgaa ctcttctgag ctctgactat ctgggactca acagtgctta 49140
caggtttcat gcagccactg aggttacagc ttaggtttac agccctcaaa tctttacatg 49200
gaaactgtgc tgatcacagg aacctcatcc caaggcagga acttcacctt ctttccctat 49260
tttcccagct gattgtcttc ttgcatccta gatagttagc aatgtttgct aagcatattc 49320
ttatcattaa acatattact gaggatatga acatcatgaa gtacaggaag gaggtatgcc 49380
cttaagaaaa agaacaccag atttgaaatc tagagaacct gcctcctagt ctcacctcta 49440
ctcctaaata gcctgcttac cttgggcttg tccctttcc tgggccgcag ttttgccacc 49500
tgttaacagt gtggggttgg gcttggtgct cctaacatcc tctctagctc aggcattcta 49560
agcaccctgg gatgcgggcc atgttacttg taaggaggag cgggtggtat acaatggaag 49620
ggtgtactgg tgattgcctt agcacagaag actggggctt caccttgaat aactcagcaa 49680

ctggttcatc accccatctt tgtaagcacc tctctacatg cagagctccc agctaggagc 49740
cagggaggcc gtgagggaga tacagtttcc atctttagga actagagtca ggttgggaga 49800
gaagaggcag gtatgtgtgt gactcatcag aagttcaatg tggggtgtgc tgaggtccac 49860
aggaactgtc cagccccttta acttattgct catatttgca tccctagcaa ttagggtagg 49920
acctggcatg caaaagtgac ttggtgaatg cttatctgtc taatacgggc atgagggtga 49980
gaccccacag gcctggaaac tccccagagg agacaagccg caatgtccag gtagaaggga 50040
gtggaggagt gacgcagccc acggtggtac ccaaccatcc ccaaggccaa cctccaggct 50100
ttcttattcc tgtgtcctga ggctggatgt actaattttg tggaacctgt tgctttggat 50160
tcaattttgc aaacatccac agccttctgg ttatgtaatg tccagagtca gccctggcca 50220
gttgcccttg gctgcctaag cttggtcatg agaccagctt atgtcttcag acagccctgg 50280
ggagagaaaa atgggcaggg gtggggaacc agcaagagca acaacaacgc aaaaagccca 50340
ctttgcaacc tctggcagac agcgcgggaa tgccaaagca tattagacca aagcagaggt 50400
tcagaaaaca cacgaacctg cctggtctgg aacaatcctc attacgagac actcggtggt 50460
cagcagagag ctgagaggga aatagctggg aggcagcagt tcctaagcct gcctggtttc 50520
cttcctacca tcagggcctg gagaccaggg tgaccagagg cccttcctgt ggacataatg 50580
tcccattccc ccttgctggt gtcagaccag ccacgggtgg ttgtttagag accaaacaac 50640
acttgctagc ctatcagcat cagacactcc cactcccagg ctggtgggct tagaagacac 50700
actcccactc ccagactggt gggcttaggc tttcggttca ccatccttcg cctgagctca 50760
tgaatcatgg tgctgggaat tagatgtagg atccctcgtc ttacttctga cagaggcgca 50820
aaggggaaat gacttactca gagtcaagca tttaactaag ggcagatctg atccccaagc 50880
tgaggcatcg gaatttcttc ccaatcctgt gttctttata tgactcccaa atctccctgc 50940
catcttaatc atcagtcacc atctcatgcc tttcattcac tctgaccctc catagagggt 51000
atcatgatcc atagaactgg tcagtgccct tctatccaga gacaccactg gaatctgctt 51060
gtctctaggt accagcctgc aagtcagaca agatacaggc tagaagtaat agtccgtcca 51120
tccatcctct cgtgcttcct tcccaccctc tctctgtgct tagtactgta ctatgaactg 51180
gggatacaaa gataacactg cttataatta tgataagact tggcatcagt taacatttac 51240
tgagcaattg cttagtgcca ggcattctgc taggtctttg catagatcag tacttatact 51300
aacactgtaa aatagataac accttgtcct ctgtttgaca ggtgaggcaa ctgagacctg 51360
ggaggtcata taactgttaa gattatatgg ccacaaagtg gcaaacctga gcaaaactac 51420
agggtctttg cctataagga gataatacag tatggagcag caggctgatt catcattcct 51480
acccactttg gggtatataa atgcccactc catatgtttt tagttcccag ggaacaaaaa 51540

gaggtcagtt tgggtttgtt ggtttatcat ccagcagtgt agggctggac ttgtaatatc 51600
ctgcaaaggg tgtatcagaa aaatctttgt aggagggaag agagagaggc cagagaggca 51660
agcctgcatg agttttggca ctggtccctg ggagactttg ttctagcaga ttagacagtc 51720
taccctagag agtcagaagg ggtgagcaag ggcttcctta gagcaggact aaaggaactt 51780
ggagaacctg agactccgag ccgccctgtc tgacctctct cttctctcac tgatggaagg 51840
caggcagacc ttctcccgtc tgctaactct ggagcctctg acccatctct caggaacact 51900
tcagtacaca gagacctgcg atatgtgtgt gtgtgtgagg gagcagggtg ttagaatgtt 51960
gagagagctt attgcaatcc caaacttgga aattagctgg gctgtggggt gaaggtgaaa 52020
gtttccagaa ctggctgctc taagctgtgg ggaccttatc tcccatcagg accatgagaa 52080
accgatccac tgtgccagga gttattgttt atgtagcgga gacatgctct ccagctggct 52140
gcaggggaca gaacgtgggg gacattatgt cccagctggt cctgggaggg tcccatgggg 52200
agacccgctg gtcatgtgct ttgcatgtcc tgactgtagg cagagcagac ctccagggac 52260
ttccttcctt tagtggtttg tctaaccaaa tgagcaatag cagggtctcg cctttgaagc 52320
tctagctgga agcatctttg acccccagcc cacagggaac cattcttctt gcaatgcccg 52380
tggttcttcc tgccttagga ctctcatcca gccctctgca gagactggct ccatttctac 52440
attctctgcc tgtcctgcta gacaaattgc tgtatagaga aagatcatgg ccgtgatagc 52500
tcggtcggtc tgtaaataag aatttgtggg tttaaatgag tccgtgatgt acaccagcat 52560
ccagaacagt gttgatcttt ggtaggcatg caagacatag ttggaatcat tgaggcaact 52620
catgctgtca atgtagagtg aaggccagac atttcataag aaatatgcaa atgtccccag 52680
actcagaaac ttcttttttta ctttatttgt cccttcactc caagggaagc taacatcttg 52740
gaactttgtg tagatgctac caagaagaag aagaggggcc cagaggcatg cagaacccag 52800
ctgaaaaaga acaacccact tatgtctgaa gttctaaacc agctttaact actcttgtta 52860
agagctgagg aagggactta ggagaaaggg gaccaaactc attcctcctc tccttacgaa 52920
tttcagtata acttgttcta agctaacttt tattgaatgt tgtgggctga ctgctattct 52980
aagtcatttg cttctctcaa acaccctaag aagtgctttg acccatttta cagatgaaga 53040
aactgagacc caaagaggtt aagtaacttt ccaagtggcg aacacaggtg gcagagaaga 53100
ttgaagattc aaactctggc tgtcagcctc caaaacctga atttaccact gatctccatg 53160
gcattggttt ggattagatt acctttaaca tcttttccaa ctctgagatt ctaggattct 53220
ctgatgtcag gacattataa acctattata actctcctaa atttgtcaaa ccaattctag 53280
atctctatga ttacagacat gatactttta gaatcaatat ttaaatcaag gtaggattat 53340
tacacagaaa acacaactga gtagaatcta cctttgccat gttgttcttt tcagaattga 53400
ccctaaaaca cagcgctatg tttgtcagag ccagtcattt gtaatgtgtc agaatttcga 53460

ctctaggcaa gtgctaggag ctaaaatgca ggttccttca aacttccaag tagctgcggt 53520
gccaagctga tttcgatgag ttgagttctt actgagcagt tcaaatgacc agactgtaac 53580
caggggcttc attctctcac ccacgaagca ctagggcagt caaggggaaa gactgacgca 53640
gaaggaatga ggttcccatg ggctgggtcc aaaagaccta gagcgagtgc ttagttgaaa 53700
catttgccaa aacctcaagg aacacccaca caaaaataag aagttgctcg gcacagaagt 53760
cagggtaggg gatgttcttc gaaggccaca ctccatggca ggcatacagc agcacgctta 53820
aacatggttc ctgttaaggc tagagaggca ccattcacat agaagaagtc atcatcgtga 53880
ctctacccac cccgcgcccc caccgcgtaa ttgaatgctg agtgtggacg gcacttcttc 53940
cagcacttgg ctgcgttacc gtgctcagtt ggcaagcctt gggggagaag agaggttcag 54000
aaggaagacc atctgagtcc atcagaacaa tgtgtgcttt taacaaggca ggggcgccca 54060
aactcagtag gtccaacaga ccaagttatg ctatgaaggt cttgtggacc acctaccccc 54120
gccaaccagt caccacttaa aagatgagaa aacgccagct cacaggccaa atgggaggca 54180
tttagaagag acaccaatga agagatgtct ggctttgtat taattactgt aggtaaatta 54240
ataccaaatg ccaggaagta aagtagaggc attttcctat tataaaccct tgggacaagc 54300
ccagagacca ccagcagtcc agggcttgtg tacgctgaga actgagggga taacagaatt 54360
gtgcaacacc ttggtgcccc accgcccccg ttaccaaatc tcacagttca cagtatcgcc 54420
ttggcacact gggcttgtgt aatactcatg gacttgacct ggaatcacaa agcggactca 54480
gcttctgtta cattcattca ccccagtgac tctgggcaca cactgtgcct ctgaaggagg 54540
cacagtggcc ataaatggaa atagcatttg cctgcttctg gggaccttgg tcatagactt 54600
ctgaggcttt cctgggagcc agtctaagat aatgggtaag agtatggact ctgaaggcag 54660
agtcctctgc ttggaatcct agccctgcca tttcccaact gtgtggcctt aacaagctac 54720
ttagcctgcc tgtgactcag gctcctcatc tataaaatgg gcataatagt tcctacctca 54780
tagggttgtc atggtgattc gtgggctaat attaaaggag atttctcaac ctcagtacca 54840
ttgacatttg gggccggata attctttgtt ctgggggctg tcctgagtat tatgggatgt 54900
ttagcaacct ccctaccctc tacgaactgt tgtgacaacc aaaattgtct ccagacattg 54960
ccacatgtcc caaatatttc ctaggggggc aaaattgcct ccagttgaga accactgtat 55020
tagattatgt cgtagataca aatagccaaa tatcagcaat ttcacgtggt tcaatctaac 55080
aattataaag tatttaacag ttaaaactct ttgaactttg tagatacatt tatgtgcttc 55140
atctgggata aacgaaccca atgctttcac cagacgctca gaagaataaa aaacatggag 55200
aatttctagc ttgaattcaa cgttcaccag ccaaatagaa tttctataaa gtggggtttg 55260
taatacatcc ctcccagggc tcttttaagg atactgcaag gagaatgtga gtgagaatgt 55320

ttttgtgggc tggaaagtgt cacagagata tcatgagaat gctggctgag ttcacgggac 55380
tcaggtccta aggttgacaa agaaaaagga cctttgtcac agctacttct ctggatttct 55440
gaaaaggtgc tgggatgtca gacatgtgta gccgaaggag tgttgtgggg cagttggaat 55500
ggggggtatg gctggctgca caggaaggtt tctctgggaa ggcaccaacc tggctagtca 55560
agaggatcgg tggcttcatc tggggtgaag gggccctgg ggtgggtaga tcagtgaccg 55620
gccactggac agctgaatag gggagcgaga ttccctcaga tccaatgtca gcaagccttc 55680
tgggtactga gactcctctc ctgtgttcct cttattttat tgatacataa taaatggaca 55740
tattttgaga gtccatgtgg tattttgata tattcaaaaa tgtatagtaa tcaaatcagg 55800
gtaattggga tattcataac cttaaacatt tatcgtttct tcatgctggg aacatctgaa 55860
ttatcctcta ctagctactt tgagatatac aataagttat tgtttactgt agtcattcta 55920
ctgattcaat gaacactggg tcttacttgt tctgcgtact cgtatttttg tacccattaa 55980
ttatatctct ttgccccaat ccttattacc tttcttgcct ctggtaacca ccaatccact 56040
gtccatcttt atgagattta ctttcttagc tcccacatga gtgagaacat ggtgtatttg 56100
tctttctgtg cctggcttat tccgcttagc aaaacgacct ccagttccag ctgcattgcc 56160
acaaatgaca agatttcatt cttttttctg gatgaatcat attccattgg gtatagatac 56220
caggttttgt ctatccattc atctgttgat gggcacttag gttgatttca tatcttggct 56280
attgtgaata ttgctgcagt caacatgaga gtgcagaccc ctcttccata ttccgatttc 56340
ctttcctttg gatacatacc cagcagtggg attagggatc atacgggtgt ccaaactggg 56400
aaagaagaag tcatgatgtt ccttttgccc acagatgtac cgaggcttca ccaagatgcc 56460
ccatgtgcag tacatccata cggaagcttc cgagagtctc tgtggcctta agctggaggt 56520
caacaagtac cagtacctgc tgacaggtaa tggccaactc tagcttctag gccagggttt 56580
ggccaaggtc cactgtttct tgacttcaga agaacacata cggagtagtt gactcaccaa 56640
atgtccagta aattgtaagg agtctctaat gttgaattca tcccacttac ccagtgctag 56700
gagcttgagg ctcctacact taggctggac tttggaatta gctccaccac tcacctgggg 56760
gaggaaatct cctaatttgt agcttctcaa ctttactgta aataaaaagg gagtatctgt 56820
tcctctccac ctcacactcg acctgttagg cccgtctttg aaagttattc atctttaggc 56880
agtcagtgta gtaaaggaga aaacaaaaac aaaaatcatt ataaagggag tgaagagttg 56940
gcctctagtc ctagctccag tatttaatgg gcttgggacc tttggcaaat cactttacct 57000
ctccaagcct cattatcctc atctggaaaa tggggctaca gtccatttta aaaggtgttg 57060
ggtagggtga aataaaatca tgggtctgaa aagtacccag ccacagtgcc tgggctaagt 57120
gggaacatag taggtgtgaa ttctctctgg gctaggctct ggacaaaaca acctctcctt 57180
gttttcttct ttcctcctgt aggtcgcgtc tatgatggca agatgtacac ggggctgtgc 57240

aacttcgtgg agaggtggga ccagctcacc ctctcccagc gcaaggggct gaactatcgg 57300
tatcacctgg gttgtaactg caaggtaagc tctggggtca ctgggggaag gaggggaggt 57360
gctgacttgc agccctagaa acatcagctc ccaatgcact gggtgccagg ccctcggctg 57420
ggaagggtat gcatgtgtta ggccagggca gaggggcagg tctgagcaga tatagtaagg 57480
attgttgccc caggtggggc agtgaggaag gcagggaagg aagaatgcct ttctgctgta 57540
atcggctgcc tccatgatga ccacttggaa gctgctgggg catgagggct cttgagtccc 57600
tggccccacc tggcagttca aggctggagg tataggatcc tgacactggc aggttctccc 57660
aagtggtttc aagtgggctc accctggtag ctgactcatc gctaacccca ccccaggtag 57720
gcaaatgggc ccaggactgc agagaccaga tgctacagaa ggtctctctt taccaggccc 57780
atccccctga cccaagtata gggccaaggt gcagcctgcc tgctgtggga ggaagttggg 57840
ccattctctc acttcatctt caaaacagtc ctgtgtggtt ttaattacta tgatctccat 57900
tctgcagatg agagctcaga gaacttaagt aactagccct aggtcccaca gttcactgaa 57960
tctggtttcc aacctagggc agctagactc caaagtttat gctctaacct ttctaattcc 58020
tcgattccct aaaacaactg agatctccca cctgagacga aaaagtcctc tcagtagtct 58080
aattctgcta cttactggct gtgtgacctg cagcaagtta cttaacctct cagagcctcc 58140
taaaactgtg ataagaatta aagagaataa tatcaaagca cttatcagaa tgtctgttag 58200
acagcagatg ctcaagctag tgctatttat attatgatca ctgagggact gatgtggcta 58260
ggcttcccat gcagtggccc cagggtctga atccaggctc ggtagcctca ggcctgggca 58320
taccatggca gagtccatca actgctgcct gttatctaat tgcagatcaa gtcctgctac 58380
tacctgcctt gctttgtgac ttccaagaac gagtgtctct ggaccgacat gctctccaat 58440
ttcggttacc ctggctacca gtccaaacac tacgcctgca tccggcagaa gggcggctac 58500
tgcagctggt accgaggatg ggcccccccg gataaaagca tcatcaatgc cacagacccc 58560
tgagcgccag accctgcccc acctcacttc cctcccttcc cgctgagctt ccccttggaca 58620
ctaactcttc ccagatgatg acaatgaaat tagtgcctgt tttcttgcaa atttagcact 58680
tggaacattt aaagaaaggt ctatgctgtc atatggggtt tattgggaac tatcctcctg 58740
gccccaccct gccccttctt tttggttttg acatcattca tttccacctg ggaatttctg 58800
gtgccatgcc agaaagaatg aggaacctgt attcctcttc ttcgtgataa tataatctct 58860
atttttttag gaaaacaaaa atgaaaaact actccatttg aggattgtaa ttcccacccc 58920
tcttgcttct tccccacctc accatctccc agaccctctt ccctttgccc ttctcctcca 58980
atacataaag gacacagaca aggaacttgc tgaaaggcca accatttcag gatcagtcaa 59040
aggcagcaag cagatagact caaggtgtgt gaaagatgtt atacaccagg agctgccact 59100

gcatgtccca accagactgt gtctgtctgt gtctgcatgt aagagtgagg gagggaagga 59160
aggaactaca agagagtcgg agatgatgca gcacacacac aattccccag cccagtgatg 59220
cttgtgttga ccagatgttc ctgagtctgg agcaagcacc caggccagaa taacagagct 59280
ttcttagttg gtgaagactt aaacatctgc ctgaggtcag gaggcaattt gcctgccttg 59340
tacaaaagct caggtgaaag actgagatga atgtctttcc tctccctgcc tcccaccaga 59400
cttcctcctg gaaaacgctt tggtagattt ggccaggagc tttcttttat gtaaattgga 59460
taaatacaca caccatacac tatccacaga tatagccaag tagatttggg tagaggatac 59520
tatttccaga atagtgttta gctcacctag ggggatatgt ttgtatacac atttgcatat 59580
acccacatgg ggacataagc taattttttt acaggacaca gaattctgtt caatgctgtt 59640
aaatatgcca atagtttaat ctcttctatt ttgttgtcgt tgcttgtttg aagaaaatca 59700
tgacattcca agttgacatt tttttttcat tttaattaaa atttgaaatt ctgaacaccg 59760
tcagcaccct ctcttcccta tcatgggtca tctgacccct gtccgtctcc ttgtccctgc 59820
ttcatgtttg ggggcctttc tttaactgcc ttcctggctt agctcagatg gcagatgaga 59880
gtgtagtcaa gggcctgggc acaggaggga gagctgcaga gtgtcctgcc tgccttggct 59940
ggagggacac ctctcctggg tgtggagaca gcttggttcc ctttccctag ctccctggtg 60000
ggtgaatgcc acctcctgag atcctcacct cttggaatta aaattgttgg tcactgggga 60060
aagcctgagt ttgcaaccag ttgtagggtt tctgttgtgt tttttttttt ttttttgaaa 60120
taaaactata atataaattc tcctattaaa taaaattatt ttaagtttta gtgtcaaaag 60180
tgagatgctg agagtaggtg ataatgtata ttttacagag tgggggttgg caggatggtg 60240
acattgaaca tgattgctct ctgtctcttt tttcagctta tgggtattta tcttctatta 60300
gtatttgtat cttcagttca ttccacttta ggaaacagag ctgccaattg aaacagaaga 60360
agaaaaaaaa aaaaagcagc agacaacaca ctgtagagtc ttgcacacac acaagtgccc 60420
aggcaaggtg cttggcagaa ccgcagagtg ggaagagagt accggcatcg ggtttccttg 60480
ggatcaattt cattaccgtg tacctttccc attgtggtca tgccatttgg caggggggaga 60540
atgggaggct tggccttctt tgtgaggcag tgtgagcaga agctgatgcc agcatgtcac 60600
tggttttgaa gggatgagcc cagacttgat gttttgggat tgtccttatt ttaacctcaa 60660
ggtctcgcat ggtggggccc ctgaccaacc tacacaagtt ccctcccaca agtggacatc 60720
agtgtcttct ctgtgaggca tctggccatt cgcactccct ggtgtggtca gcctctctca 60780
cacaaggagg aacttgggtg aaggctgagt gtgaggcacc tgaagtttcc ctgcggagtc 60840
gataaattag cagaaccaca tccccatctg ttaggccttg gtgaggaggc cctgggcaaa 60900
gaagggtctt tcgcaaagcg atgtcagagg gcggttttga gctttctata agctatagct 60960
ttgtttattt cacccgttca cttactgtat aatttaaaat catttatgta gctgagacac 61020

ttctgtattt caatcatatc atgaacattt tattttgcta aatcttgtgt catgtgtagg 61080

ctgtaatatg tgtacattgt gtttaagaga aaaatgaaac ccacatgccg ccattttcct 61140

gaatcaaatt ctgcagtgga atggagagga aaatacttct aggcaagcag ctagactggt 61200

gaattggggg aaatagaagg aactagtaac tgagactcct ccagcctcct ccctattgga 61260

atcccaatgg ctcctggagt aggaaaaaag tttaaactac attcatgttc ttgttctgtg 61320

tcactcggcc ctgggtagtc taccatttac ttcaccccaa gtcctgctgc ccatccagtt 61380

gggaagccat gattttccta agaatccagg gccatgggag atacaattcc aagttctcgc 61440

ttcctccttt gggcatctct tctgcctccc aatcaaggaa gctccatgct caggctctca 61500

gctctcgggc cagtgctctg ctctgtccag ggtaggtaat actgggagac tcctgtcttt 61560

taccctcccc tcgttccaga cctgcctcat ggtggcaaca tggttcttga acaattaaag 61620

aaacaaatga ctttttggaa tagccctgtc tagggcaaac tgtggccccc aggagacact 61680

acccttccat gccccagacc tctgtcttgc atgtgacaat tgacaatctg gactacccca 61740

agatggcacc caagtgtttg gcttctggct acctaaggtt aacatgtcac tagagtattt 61800

ttatgagaga caaacattat aaaaatctga tggcaaaagc aaaacaaaat ggaaagtagg 61860

ggaggtggat gtgacaacaa cttccaaatt ggctctttgg aggcgagagg aaggggagaa 61920

cttggagaat agtttttgct ttggggtag aggcttctta gattctccca gcatccgcct 61980

ttccctttag ccagtctgct gtcctgaaac ccagaagtga tggagagaaa ccaacaagag 62040

atctcgaacc ctgtctagaa ggaatgtatt tgttgctaaa tttcgtagca ctgtttacag 62100

ttttcctcca tgttatttat gaattttata ttccgtgaat gtatattgtc ttgtaatgtt 62160

gcataatgtt cactttttat agtgtgtcct ttattctaaa cagtaaagtg gttttatttc 62220

tatcac 62226

<210> 95
<211> 20
<212> DNA
<213> Artificial

<220>
<223> Synthesis

<220>
<221> misc_signal
<222> (1)..(20)

<400> 95
ctgctgacag gtcgcgtcta 20

<210> 96
<211> 25

<212> DNA
<213> Artificial

<220>
<223> Synthesis

<220>
<221> misc_signal
<222> (1)..(25)

<400> 96
ttacaaccca ggtgataccg atagt 25

<210> 97
<211> 1463
<212> DNA
<213> homo sapiens

<400> 97
atcccctctc ccagtgcttc ccctctgctt ccagatcgct tcatgactta ggcagggaaa 60

cagaggtcag ggcctccttc caggcttccc tctgcatctt actgagtatg caggtcggaa 120

gagcctcggg tcctgcctcc gcgggtggcc tagagccaaa ggaaggcgga gcccgtcggg 180

gcgggattgg cccttagggc cacctcataa agcctggggc gaggggcaca acggccttgg 240

gaaggagccc tgctggggcc gtccagtccc ccagacctca caggctcagt cgcggatctg 300

cagtgtcatg cctgggagcc ctcggcccgc gccaagctgg gtgctgttgc tgcggctgct 360

ggcgttgctg cggcccccgg ggctgggtga ggcatgcagc tgcgccccgg cgcaccctca 420

gcagcacatc tgccactcgg cacttgtgat tcgggccaaa atctccagtg agaaggtagt 480

tccggccagt gcagaccctg ctgacactga aaaaatgctc cggtatgaaa tcaaacagat 540

aaagatgttc aaagggtttg agaaagtcaa ggatgttcag tatatctata cgccttttga 600

ctcttccctc tgtggtgtga aactagaagc caacagccag aagcagtatc tcttgactgg 660

tcaggtcctc agtgatggaa aagtcttcat ccatctgtgc aactacatcg agccctggga 720

ggacctgtcc ttggtgcaga gggaaagtct gaatcatcac taccatctga actgtggctg 780

ccaaatcacc acctgctaca cagtaccctg taccatctcg gcccctaacg agtgcctctg 840

gacagactgg ctgttggaac gaaagctcta tggttaccag gctcagcatt atgtctgtat 900

gaagcatgtt gacggcacct gcagctggta ccggggccac ctgcctctca ggaaggagtt 960

tgttgacatc gttcagccct agtagggacc agtgaccatc acatcccttc aagagtcctg 1020

aagatcaagc cagttctcct tccctgcaga gctttggcca ttaccacctg acctcttgct 1080

gccagctaat aagaagtgcc aagtggacag tctggccact gtcaaggcag ggaaggggcc 1140

atgacttttc tgccctgccc tcagcctgtt gcccctgcct cccaaacccc attagtctag 1200

ccttgtagct gttactgcaa gtgtttcttc tggcttagtc tgttttctaa agccaggact 1260

attccctttc ctccccagga atatgtgttt tcctttgtct taatcgatct ggtaggggag 1320

aaatggcgaa tgtcatacac atgagatggt atatccttgc gatgtacagt atcagaaggt 1380

ggtttgacag catcataaac aggctgactg gcaggaatga aaacaagaaa aaaaaaaaaa 1440

aaaaaaaaaa aaaaaaaaaa aaa 1463

<210> 98
<211> 5821
<212> DNA
<213> homo sapiens

<400> 98
cctgctgggg ccgtccagtc ccccagacct cacaggctca gtcgcggatc tgcagtgtca 60

tgcctgggag ccctcggccc gcgccaagct gggtgctgtt gctgcggctg ctggcgttgc 120

tgcggccccc ggggctgggt gaggcatgca gctgcgcccc ggcgcaccct cagcagcaca 180

tctgccactc ggcacttggt gagtccgagg tccgcgaggt ccacagcagg gggtggttgt 240

gggggattag tatctggcca gcagtacacc aggagtccag aggaaaaggg aggagctcaa 300

attcttaccg gttgtctctt gctgaggctg atgggtggag aaggaccagg cgccatagca 360

accttgcaag cccagaacct gactgattca taaggaaatg aagatttcat cagggacttc 420

catagcgacc tccctcactc cggccatact gcaattatgg agtgtcatta cacaaggtgt 480

caccggagct atctgatgat tattctgaac tactagactg gggacagaaa ggggagggga 540

gagggcttgc attgtaacta cccgtgtctg tccaggccta taactgaggt tgccaccaag 600

cccctatggc aacacacacg ttctgtcctt tctcttccct ctgcctggaa tgccctccct 660

cacaccctta aattcctggc agactcctac tcatccttca agggctaact taggtctctc 720

cccttctctg caggctcccg ggctccttgg tgccgcctca gcatctctaa ccgtcttagc 780

gctcctacta tgctgtgctg ttgttcttta cacgtgtgca ctccactaga tcaagagctc 840

ggcaggatgg ggaccctgtc ttaatcacat ctatgggcct agtgcctagt gcaaggccag 900

gcacagagga agaacatcat caacagtttt tggttattaa tgctcaaatg gcaagagatg 960

caatacctca gtgcagggtc agaacattta tgaattcaat aaacatttac taatatcaac 1020

tgtaggcggg atgagaggcc ttgaccacag caatagataa acttcacacc gactgccctg 1080

aaggggctcc tcctcccttc tctactctga ctcctaccct gtgccccact gcacaaacgg 1140

ctatggagaa ctagagctct ccagactaac tggttcaaaa ggcagaaaca gcttagcggt 1200

tataggacct aataagcctg aggggtttgg aacttccagt gactgcaggg gaggtgtttc 1260

caggtccccc ttctcacaca tcacgttgct cacaacccag ccacaccaaa accaagaact 1320

gccctataga cttcttcatg gaccctggat catccagacc tcaggtgtat tgcccccacc 1380

agctgctgaa ggttccctct tttctcttcc tatgcagtga ttcgggccaa aatctccagt 1440

gagaaggtag ttccggccag tgcagaccct gctgacactg aaaaaatgct ccggtatgaa 1500
atcaaacaga taaaggtaca tgggggcagg ctgggatgtg tagcccctaa ggctgatgga 1560
gaaagtggag tctgtgttct gggagggctg gtagctttca ggtggcctgg ctcagtaatc 1620
cttgggtcta gatactgaag tggaactgga tgagatttgg aggcagagcc catagaatcc 1680
cagggatatt gccagggagg gcctcagcat ccatctggtc ccacagctgc tggtgtatgg 1740
tgccactaca ggtacctact ggaataaacc aaggtttagg tgtttttgt tgttgttgtt 1800
ttgttttaag agagaaaata acccagtagg gacatctgag gacacaaaat cctcaactag 1860
acttttgctt acatttttag gctgagaagt gagaggtttt tgtctgttaa tatatacagt 1920
acactcactg acccaatatt gccttttttt gtaattgcct gtcagatgtt caaagggttt 1980
gagaaagtca aggatgttca gtatatctat acgccttttg actcttccct ctgtggtgtg 2040
aaactagaag ccaacagcca gaagcagtat ctcttgactg gtaagttaaa agaccaacaa 2100
ctggccttaa tataatagat tctgccctag gggaggtagt gagaaaggag ccagggtctt 2160
ccttgggcag taggctgagc caggtggcat tttcttgggc ataaaacaga gctgtgttag 2220
tgacagacaa cctctgggtt aggaggagcc ttgtaacctc cctttaaatg taagagttct 2280
tagtacagtg accctggcca ccgtttcact attttaagga caggctgctc acccagtgta 2340
gggcagctgc ttccgttttt caaatagttt aaattagtat aagatttttg gcaacttgag 2400
tcgaagtctc ctctctaaaa ctgctccttg caggtcaact ctgccttttg cacaggactt 2460
aacaggagct tgagtctaac atagttctgt ctctgtgaac ttgggcccag gcaggattcc 2520
tccatactga attggctggg atagaacagt taaagaatca cacaaaaata tgactccata 2580
aatgaatgca acattaaatt attttggagt tcagagaacg gaactatttg gtagttgtga 2640
actatcaggg aagcttcatt gtgtaggtgg gatctaaact ggcctgaaaa cataaaacaa 2700
tatttagaaa gaaagcaagg gaaagaaaaa gcacagcagt gggggaggga acaggtgaag 2760
ggattccacg atctatccac acattcagca atgaatgacc acttagtcac tgccatgcct 2820
tggccaggag agtggagatt cagtaacaag gcacagtcct taaaagagtt cactgtccag 2880
acatggctct tgatgagtca taggcactgg aggctacaat gataagggct tcctagctct 2940
gaggatgtgg aattgtacag aactgaggag ctcctggatg gaagaggcag aggcaggaca 3000
caccactgag ccctgcagtt cccaggtcta gcagaccagg ccctttcaaa ctctattatc 3060
gcacctcctt cctaagcacc tgccctctgt gtgtctagcc cgctgctagg catggaggta 3120
cagcagggga aatgaggaaa catgttccct gccttcaggg aggtcaagtc taactgaggg 3180
tggggagaaa tgacaaaaaa tagtctaaga aaagttatca atggtgccca ggtcctacag 3240
gagaaggcac aagctccttt gcctggtgtt gaaagctctt tgtgaccaga acactgctgc 3300
cctctctagc tttgtcaata tcagttccca gcccctcact ttcccaagaa ctgaactcag 3360

ctacctgtgg ttgcagaagg tatgctgcca ggctgtgtct ccctgcctct gcacaggata 3420
gtggcaatga caggaagccc ttcccccaac ctgtttggta ggcagactcc cactcatccc 3480
ttcaaggccc aatgcaagca cagtcttctc tgagacacct tctttgcccc acactccttc 3540
catagatgga aataattgta tgttcctttg ttcttatact gcactttgta tagaaccaca 3600
caatagtgta atttttccct acttatccat ctaccctact catttctaag ctccttggga 3660
gcagagatcc tgtcattttt atatctgtat tcctggtact agatcacagc agtcactcag 3720
tgagtaaatg attgagcgac tgaggttcta aaagaggcat gggctagcta ggtggtcaga 3780
gatggctttg aggtggcact gaaagggtct ttgaaaggtg aaaaggaatc tacggccata 3840
ccaccctgaa cgcgcccgat ctcgtctgaa aggtgaaaag gagcttcata ggtaaaaagg 3900
gggaaatttc ctatggggaa ggtaaaagac tcaccaatct agagcaggag cctggcctgt 3960
gcagatactg ggaagggctg gaaaggggaa gggacctatt atttattgaa tatcttctgt 4020
gggaccacaa ctgtgctggg aactttatag acaatctctc atttaatctg acaactttat 4080
aattttcatc ttccagctga gaggttatac aacttcctta agatcattca gctagtaaat 4140
gacagaaaag aaagggggat cccacacaga ctttgacttt aaagaccaca ttcttctctc 4200
tgtaccatgc taaacgaaaa ggccttcacc agatcccaca actctgcctc gtacaaacat 4260
aatctgctta tctttttgct agaaagatta tagtagtttt atttattggt cccttcactg 4320
agccccatgc caccactgcc accaaagtca ttggggaggc tgcaattctg ggcttttcaa 4380
ggacctgggc ccctggggct ccaggagaag aatcctgatg cttgactctc ctccatctcc 4440
ccttgaaggt caggtcctca gtgatggaaa agtcttcatc catctgtgca actacatcga 4500
gccctgggag gacctgtcct tggtgcagag ggaaagtctg aatcatcact accatctgaa 4560
ctgtggctgc caagtaaggg aatgtccatt tccaaagtct ttagggatgg gggaagggtt 4620
ctgagccttc attcatgcat gaatttattc acttactaga ctatacttga gatctgcact 4680
gtatgaagtt ggggatgaga aagcaataca gtctataggc cctgatctca aagtacttac 4740
actttggctg ggaaggtggc catcacagga ggcagactga gataactgta acacagctac 4800
cctttaaaga gagtctattc tttgccagcc actgtaatag taaatttact gtaaatgttt 4860
aatttagtct ccaaaacata tttatgaaga attaagattt ccattttaag gtgaagaaat 4920
gaaagctctg gcaagtgaag tgctcaaggt cagtggtaga gtaaggaccc tcagagctgt 4980
ctgactccag agatgccact aagtggcaat attgaatatg gggaaccttg gggaaggaga 5040
atccaggaga agactgggga ctgggaaagg cttgataaga tagggggcat ttgagatggg 5100
ctggaaaggt acgtaggaat ctgaaaggca gcaatggaaa aagaaggaca ccctactcac 5160
tttaatgttg tgtggccatg actctagatc accacctgct acacagtacc ctgtaccatc 5220

tcggccccta acgagtgcct ctggacagac tggctgttgg aacgaaagct ctatggttac 5280

caggctcagc attatgtctg tatgaagcat gttgacggca cctgcagctg gtaccggggc 5340

cacctgcctc tcaggaagga gtttgttgac atcgttcagc cctagtaggg accagtgacc 5400

atcacatccc ttcaagagtc ctgaagatca agccagttct ccttccctgc agagctttgg 5460

ccattaccac ctgacctctt gctgccagct aataagaagt gccaagtgga cagtctggcc 5520

actgtcaagg cagggaaggg gccatgactt ttctgccctg ccctcagcct gttgcccctg 5580

cctcccaaac cccattagtc tagccttgta gctgttactg caagtgtttc ttctggctta 5640

gtctgttttc taaagccagg actattccct ttcctcccca ggaatatgtg ttttcctttg 5700

tcttaatcga tctggtaggg gagaaatggc gaatgtcata cacatgagat ggtatatcct 5760

tgcgatgtac agtatcagaa ggtggtttga cagcatcata aacaggctga ctggcaggaa 5820

t 5821

<210> 99
<211> 20
<212> DNA
<213> Artificial

<220>
<223> Synthesis

<220>
<221> misc_signal
<222> (1)..(20)

<400> 99
caccctcagc agcacatctg 20

<210> 100
<211> 20
<212> DNA
<213> Artificial

<220>
<223> Synthesis

<220>
<221> misc_signal
<222> (1)..(20)

<400> 100
tggccggaac taccttctca 20

<210> 101
<211> 1421
<212> DNA
<213> homo sapiens

<400> 101

acttactgcg ggacggcctt ggagagtact cgggttcgtg aacttcccgg aggcgcaatg 60
agctgcatta acctgcccac tgtgctgccc ggctccccca gcaagacccg gggcagatc 120
caggtgattc tcgggccgat gttctcagga aaaagcacag agttgatgag acgcgtccgt 180
cgcttccaga ttgctcagta caagtgcctg gtgatcaagt atgccaaaga cactcgctac 240
agcagcagct tctgcacaca tgaccggaac accatggagg cgctgcccgc ctgcctgctc 300
cgagacgtgg cccaggaggc cctgggcgtg gctgtcatag gcatcgacga ggggcagttt 360
ttccctgaca tcatggagtt ctgcgaggcc atggccaacg ccgggaagac cgtaattgtg 420
gctgcactgg atgggacctt ccagaggaag ccatttgggg ccatcctgaa cctggtgccg 480
ctggccgaga gcgtggtgaa gctgacggcg gtgtgcatgg agtgcttccg ggaagccgcc 540
tataccaaga ggctcggcac agagaaggag gtcgaggtga ttgggggagc agacaagtac 600
cactccgtgt gtcggctctg ctacttcaag aaggcctcag gccagcctgc cgggccggac 660
aacaaagaga actgcccagt gccaggaaag ccaggggaag ccgtggctgc caggaagctc 720
tttgccccac agcagattct gcaatgcagc cctgccaact gagggacctg caagggccgc 780
ccgctccctt cctgccactg ccgcctactg gacgctgccc tgcatgctgc ccagccactc 840
caggaggaag tcgggaggcg tggagggtga ccacaccttg gccttctggg aactctcctt 900
tgtgtggctg ccccacctgc cgcatgctcc ctcctctcct acccactggt ctgcttaaag 960
cttccctctc agctgctggg acgatcgccc aggctggagc tggccccgct tggtggcctg 1020
ggatctggca cactccctct ccttggggtg agggacagag ccccacgctg ttgacatcag 1080
cctgcttctt cccctctgcg gctttcactg ctgagtttct gttctccctg ggaagcctgt 1140
gccagcacct ttgagccttg gcccacactg aggcttaggc ctctctgcct gggatgggct 1200
cccaccctcc cctgaggatg gcctggattc acgccctctt gtttcctttt gggctcaaag 1260
cccttcctac ctctggtgat ggtttccaca ggaacaacag catctttcac caagatgggt 1320
ggcaccaacc ttgctgggac ttggatccca ggggcttatc tcttcaagtg tggagagggc 1380
agggtccacg cctctgctgt agcttatgaa attaactaat t 1421

<210> 102
<211> 12951
<212> DNA
<213> homo sapiens

<400> 102
cttactgcgg gacggccttg gagagtactc gggttcgtga acttcccgga ggcgcaatga 60
gctgcattaa cctgcccact gtgctgcctg gctcccccag caagacccgg gggcagatcc 120
aggtgcgggg gccagccctg cgcgtggctg gggatgaggt ggtcgtggtg atagcctgtg 180
tccaggcatc cgcgcagggc gggccctcaa atgacctcac cttctctcct aggtgattct 240

cgggccgatg ttctcaggaa aaaggtaatg gcttcgcggg gctggggtgg agctccttcc 300
tcttctccgg ggaccccttg tcccgtccct cccctcccct tccctcccct cccctcccct 360
ccccttccct tccctcccct tcccttcccc tagaaggacc agcacagcct cctacagctc 420
ccgcctgggg tgctcctccc ttgaattcag tccaggagga agtctctgcc ctcttctgcc 480
caggccaagc ccctcgtcct gtgtggacgc cactccctcc tggagctggt gacagctgct 540
tacagcttag ctgtcttccc caccaagtcc tctgagaagg tggcaaccag ttgtgtcccc 600
tgtaggccag gcctttttgt acacccctat tcaatgtggc tgtttccttc taaggccaag 660
gaaacgtagt cgctttctaa accaaggagt ctgaagccgt ggagcctctg ctctcctgag 720
gtgatagaac cattccctga cccgggtggg gctagtgagt ttcttgagta aactacccac 780
gcaccattct ttttgttttg tttttgttct tctagaggta ggatcttgct atgttgccca 840
ggctggtctc aaactcctgg gctcaagcaa ttctctcacc tcagcctccc aagtagctgg 900
gactacaggc gtgcaccccc ccgcctccac ccagctaatt ttattttatt tttatagagc 960
tggggtcttg ctatgttgcc caagctggtc ttgaactcct ggtctcaagc aatcctccta 1020
cttcagcatc ccaaagtgct gggattacag atgttagcca ccatgccctg ccccaacatt 1080
cttttatggc cctggggatc acttcagctc aaaccccttg ctcaggaaga tgtggctcag 1140
agttggactt cttggaccca gaagcaagtg cttttgacgc tgcacacaaa gactttctga 1200
aattaattta gaaaagctgt atgccaggtg tggtggccca cgcctttaat cccagcgctt 1260
tggaaggctg aggtgcgttg atcacttgag gttaggagtt tgagaccacc ctggtcaacg 1320
tggtaaaacc ccatctctac tgaaaaaaaa aaccaaaaat tatctgggca tggtggcagc 1380
ctcctgtaat cccagctact cgggaggttg aggcaggaga atctcttgaa cccggaaggc 1440
aggggttgca gtgagctgag atcgctccac tgcactctaa cctaggcaac agagcgagac 1500
tccaccccaa aaagaaagaa agaaaaactc tgaactctgg gaacaactct gggatgaggt 1560
tactttggaa tgcagtcgca ggttccctct acatgtagcc tttgcttctg ccttccccac 1620
tacatcttgg agaaggttac tcctcccaca cttcctggga ccacctgagt accattcctg 1680
gacctcttcc ccatagagaa ttctgacttc caaccctctt tgtagggata ttataccctg 1740
cctgctctgc cctgctcttt tctggctgtg gtgggctcag tctgcatacc actagggaca 1800
atgaggagcc aggcttgttg gggaggggtc tccttctccc actcctcccg ccgtggacct 1860
cacctgaccc tctctcctct tgcagcacag agttgatgag acgcgtccgt cgcttccaga 1920
ttgctcagta caagtgcctg gtgatcaagt atgccaaaga cactcgctac agcagcagct 1980
tctgcacaca tgaccggtca gtccctgccc cctgcagtcc tgtccagtgg aaaatcacaa 2040
ggcacaggac acactgttag gactctcttt aatggggatg gttaatcatt tgaacattga 2100
atgattcaaa tcagcacact ttccaaggtg cttggcaagg tagcgcacac tctccactcc 2160

ctgggctgga gccagtggtt ctccactgag ggtgattttg ccgccagggt ccatttgaca 2220
atgtttgaag acatttctag ttgttgcaac tggagggggg aggggatgct tttgggcttt 2280
aatgtgtaga aatcagggac actgctgcta agggtcctat ggtgcagagg acggccccca 2340
tgcaagaacg agctggcccc aaatgtcagg agcctgccag tgttcagaaa ctctgccgta 2400
gggtttcagc ttcacacagg ctgcagactg gtttggtttg gcctgcacgt tgatttttgt 2460
ttaatttttt agttgtccgt tgttggctgg ctcccccgtc acctggcagc cttcacgctt 2520
ccctgtttta tgtgtagctg tttgagctcg ctggacattt ccgcctgcaa cctcagtttg 2580
ggagttaaat tcacttcctt ggcagcagat gtgggcctga tgtttctgag cctgagacgc 2640
tttgcttggt cctctggact tgtccacctg ggcacccagt ggcaaagcca tgctgtgcca 2700
cacattatag ggcttcagcc tcagagccct ggctgggagc tgtatccgag agttgctatg 2760
gctgtgcaga gaacagatcc acccggcgtg tcgccttcgg tgggagctga ggggctcctg 2820
aagccagatg ctggtggagt ggagggtgct tggggcttgg agttgcatgt gggaatttaa 2880
ccgcacttcg tgaccatgct gtctgatgta ggtcatttac ttttccaaat ttgcttcctc 2940
attcctaaga tgcgatgtcc acggcacagg gtggtgttac acctggtggg gacagggaaa 3000
gcagaggagg tcacttcgtt ccagctgttg gaagtacaac ttctggagtc agtcagatcc 3060
gggattaaat atgagttctg cccgtgtgtc acaagtcatc tctaacacgg gccacagagg 3120
ccaaggctgg gccagcagca ttgatggctc gagaggctgc ccttgcaggg gccacagctg 3180
gcctcccacc tgccctcact ttgtctttct ctgtttaggg agggaagagg gaatttaaaa 3240
tgcccaaaat actgtttcac acattctttc cagaactcga agtaggatta tagcaaggta 3300
ataacgaaac aatagttgta aagtatgttt ttttgtttgt ttgttgtttg tttttgggac 3360
agggtctctc tctgtcaccc aggctggagt gcagtggctc aatcatagct tactgttacg 3420
tgaccccaaa cccttgggct caagtgatcg tcccacctca gccccctgag caggtgggac 3480
tacaggcgca caccaccaca cccagttaat ttttacattt ttttcacaca gtgtctcgct 3540
gtgttaccca ggctggtctc gaactcctga gttcaagtga tcctcccgtc ttggcctccc 3600
caaagattac gggcatgagc tgctgtgtct ggccagaata caggatttta aaaatttatg 3660
ttttgcaaca taattaatat aaagacaaat ataacccagg cccagttcta gttattcatt 3720
cttctgaatt ttaaaaggaa acatttggct ggcccctaat ggtatcatgg gccctggtac 3780
ctgatgaagt tggcctagtc tgcccccagc tcctgaacag tggaagagtt tttagtctca 3840
ttgagctttg tactggacat tactaatttc taatccaaag catcaagtga agtggcttgt 3900
ataaataact ggttttcctc tgggaggcta aggcgggtgg atcacttaaa agttaggagt 3960
ctgagaccag cctggccaac atggtgaaac cccatgtctg ctaaaaatac aaaaattagc 4020

tgggtgtgat ggtgtgtgcc agtagtccca gctactcttg tggctgaggt gggagaatcg 4080
cttgagaccc ttgagaattg ggaggtagag attgcaggga gccgagatgg cgccactgca 4140
ctccagcctg ggtgacagag caagactctg tttcataaaa aataaataaa taactggttt 4200
tctggacgag ggcctttccc ataggtgcta acttctcaaa gcccggctgg gtgaacactg 4260
agcctgcttt gcaggtagca ggtggtcacg acagtgccat tccctggccc ctgcattgtg 4320
gcttctggcc tccctggccc tgctcacgct ctggctttct cttcccagga acaccatgga 4380
ggcactgccc gcctgcctgc tccgagacgt ggcccaggag gccctgggcg tggctgtcat 4440
aggcatcgac gaggggcagt ttgtaagttg gcttgtcttg gcatcactct tcctgccagc 4500
ttccgctctg tcctcccgtt ttccctcgct gacttggaag ttatctgatc ttttagtaaa 4560
ataacaaggt taaatagcta caactagtgt tggaataccc tctgaaggcc cctttctagt 4620
ttccctgtca tagtgtcata gtcttgtagg attcgtttta cttttttttt tttttttttg 4680
agacggagtt ttgctcttgt tgcccaggcc ggagtacgat ggcacaatct caccgcaaac 4740
tttgcttcct gggttcaagc aattctctcc tgtctcagcc tcccgagtag ctgggattac 4800
aggcatgcgc caccacgccc agctaatttt atatttttag tagagatggg gtttctccat 4860
gttggtcaag ctggtctcaa actcccaacc tcaggtgatc cgcccccgcct tgaactccca 4920
aagcgctggg attacaggca tgagctacca cacctggcca ttgtaccttt ttaaaaatac 4980
atatatctat ttactggcaa gatgcagtga ctcacacctg taatctcagc ctgtgggagg 5040
ccaaggtgga cagatcactt gagcccagga gttggagact cacctgggca acatagtaaa 5100
accccatctc taccaaaaaa aaaaagaaat tagccagtca tagcagcgca cacctgtggt 5160
ccctgctact caggaggctg aggcagaagg atggagcctg ggaggtcgag gctgcagtga 5220
gtggtgatag caccactgca ctccagcccg ggcgacaagg ccagaccctg tctcaaaaaa 5280
aaaaggggga ggtggggagt aatgtttggt ttgcctcatg gttccttttg cttgtttctt 5340
atacgtttat tttcttgttg ttgaagtacc ttttttagta gtttttgcag ccaggaggta 5400
tagatgggaa gctgccagtc tttgtatgga aatctttctt ttgtcatcta gtttaagctg 5460
ggcagcaaga ggtaggttga tcttgtgtgg gtttgggttt tttttttttt tttgagacgg 5520
agtcttactc tgtcgcccag gctggagtgc aatggcgtga tctcggctca ctgcaacctc 5580
tgccacccgg attcaagcga ttttcccacc tcgcctccca agtaggtggg attacaggca 5640
cccaccatca tgcctggcta atttttgtag agacaagggt tcaccatgtt ggctaggctg 5700
gtcttgaact cctgacctca ggtgatccac ccgccttggc ttcccaaagt gttggaatta 5760
caggcatgag ccgccgtgcc cggccttttt aatttttatt tttttgaga tggagtcttg 5820
ctctgttgcc ctggctggag tggagtgacg tgatcttagc tcacagcaac ctccgccttt 5880
tgggttcaag cagttctgcc tcatccttcc gggtagctgg gatcacaggt gcgtgccacc 5940

atgcgtagct catttatgta tttttaatag agatggggtt tcaccatgtt ggccagctgg 6000
tctggaactc ctgacctcag gtgatccgca tgcctcagct cccaaagtgc tgggattaca 6060
ggcgtgagcc accacgcctg gtcttgatct tgttgctttg aaaagtagca gcgctggtca 6120
ttgtgttttt gctcagagga aggccgccat ctctctaatg ttacctctgg tcaggtattc 6180
tatctgttct ctctcagcac aatgtgtgta ggggaagctt tgtttcattt atcctgcttt 6240
atagctggtg tgccttttca tttctgggga aggaatgaag ccattatcac ttcaggtatt 6300
tctctcctca tccatctctg aggtgttctg ggttccatct tccagagtgt gttttgtttc 6360
agtgactatt tttacatctg ctgctctaat tcatcatgct ccgttttgtt tgacaagtta 6420
ctgttgggtt atttttaaat ttatgctgtt ccttccatta tgttcctgaa aatcttttct 6480
tagacttttc cagatttttc tatttcctca ggaacatatt ctgtggttga gtttctgggt 6540
tattttctgt tatcttagtt ttctttcctc tgctttggag attttatttt tgttagttta 6600
tcacaaagaa tgaaactgaa actctctcca aggggtttag cagacttgac ctcttaggta 6660
cttttagggt tgcctcgaag tacacaatgt ggtggtttga tataaacata acaggaattt 6720
atttctcgct cacagacccc ctacgtggtt ccaggccggt tgatggggag gccgcccacg 6780
aggcggctta ggtcgccctg gctggctgta tacagacacg gaggggaaga gacgtggcgg 6840
agcccctggg tgtgaggttt tcatgggcct gaccagaagc tgcaaacgtc acttctgctg 6900
atctttcaaa gactagaacc tgggcacagg gccacctata cgtttagtat acttagtcca 6960
gttcgttttt tgtttgtttt taaaaacagt cttgctctgt ggcccaggct ggagtgcagt 7020
ggcgcagtct cggctcacta taacctccat ctcccaggtt caagtgattc tcccgcctca 7080
gcctcctgag tagctgggat tacaggcttc tgccaccatg cccagctaac cttttgtatt 7140
tttagtagag acggggtttc atcatgttga ccgggctggt ctggaactcc taacctcagg 7200
tgatctgcct gcctcagcct cccaaagtgc tgggattaca ggcgtgagcc accacgcctg 7260
gccacactta gtctagttct ataccctgga ggaagaataa atgagttggt ttggtgagtg 7320
cttcgaggtc tctacccgcc ctgcctccca gcacagagcc aggccgctct ggcctgaata 7380
ccctgcccgg acgtcacagg gcctgtcccc tcaaaaggcc agtcctgcct tcctggttct 7440
gttcttgccc aacattctgt atgagtcaca gctgcaaatt ccattcccgt ggggaggctg 7500
acgggtccct tcccctgtgc ggggcatctg ccctgtggag ttgaggctgc cagtgtccgc 7560
tctgggttcc cgaccacccg gcagctggca tctcctcccc gcttgggtat ggccattccg 7620
tttctgacct tcagaggtgc gcccctgagc acccccatgc ctctgcgtac gtggagacgt 7680
cgttgttgct gccccgtgct tgagggactc ctggcgagaa agtgagccca ggctgggaat 7740
agggctgcag ctgttctctt ttgctcccaa actgtggcct cagaatgcat ccaggatttt 7800

tgcatcagct ttggggacat ggccctctca gaacaaggaa gcttcagctt tggcaaggct 7860
ctccctcctt cagacctgcc gctgtgagtt gttcaatagc tctgttctcc tggctctgcg 7920
taaaccttgt tgacagaggc tgacccagac cccgaggca gaaacctttc ccttctcctt 7980
cctcgacatc caaatgccct gagtcaggag ccagcgtatg aagtcctgtc ccctgttcag 8040
cctataggag ggatttctcg gtctacttcc tccctggcca gcaagtaaaa cttgagttca 8100
ttcagtgagt atttattaca ccctacccag acatcagcat tctgccctgg cctctgtgtg 8160
cccttgttct cttcaagaag ttccgggtca ccagcctgac caacatggag aaactccgtc 8220
tctactaaaa atacaaaaat tagccaggcg tggtggcgca tgcctgtaat cccagctact 8280
tgggaggctg aggcaggaga atcgcttgaa cccggtaggc gaaggttgca gtgagccaag 8340
atcgccccat tgcactccaa gcctgggcaa caacaagagc aaaactcagt ctcaaaacaa 8400
aacaaaacaa aagaagttca gggtcttccc attgcaagca gttctagatc gaggagaggg 8460
gttcctagca tgggacccag cagaaggact gtccttcgct ccttcattgt ctacgtggac 8520
agtggatgaa gcccagccga acctgccttg ttcccgtttt ctgggtcagc agggaaagcc 8580
tttcacagag tagccaccgt gccatcctga ggaaggccct gggtcagaag cttctgtgct 8640
tctttgtacc ccgggcgaga cacacaggtg ctcacactgc tctgtagaaa ctgttggcat 8700
ccaagagaga ctcacctgga aatctctgga aaacctgaag ctcctagctg gggtgctgt 8760
gcttcagatg ctggtggtgg gtgggcaccc ttgcatcaac agctgcacag tgtgtggtgg 8820
gcttgcaggg tcgcttggca atagtaggag ctctgattta ttttttaaa ctttttttct 8880
ggctgggcac ggtggctcac acctgtaatc ccagcacttt ggaaggccta ggcgggcgga 8940
tcacttgagg tcaggagttt gagaccagcc aggccaacat ggtgaaaccc catctctact 9000
aaaaatacaa aaattagcca agcgtggtgg cacacacctg taattccagc tacttgggag 9060
gcagaggcac aagaattgct tgaacctggg aggcagaggt tgcagtgagc caagattatg 9120
ccactgcact ccagcctgga tgacagagcg agactctgtc tcaaaaaaaa tagacaaagc 9180
caggcgcagt ggctcatgcc tgtaatccca acactttggg aggccgaggt gggtgaatca 9240
cgaggtcagg agatcgagac catcctggct aacacggtga aaccccgtct ctactgaaaa 9300
tacaaaaaaa ttagccaggc gtggtggcgg gcacctgtag tctcagctac tcgggaggct 9360
gaggcaggag agtggcgtga acccaggagg cggagcttgc agtgagctga gatcacgcca 9420
ctgcactcca gcctgggcga cagagcgaga ctccgtctca aaaaaaaaaa aaaaatagac 9480
ctttttgtgt tttctgttct actacacaag taatacaggt tgagtattcc ttaacctaaa 9540
tgcctgggac cagaagtgtt tcggatttca ggttttcgaa tatttgcatg ttcataatat 9600
aatgagacct tgggaatgag ccccaagtgt aaacacaaaa tccatttatg ttttatagac 9660
atcttaggca catagcctga gagtaatttt atgtatttag taatttggac gtgagccaca 9720

gtttttgact gtgacctgtc ccatgaggtc aggtgtggaa ttttccactt gtggtgggcg 9780
ctcaaaaagt ttcagatttt ggagcctttc aggttagaga catgcaatct ataataagtt 9840
taatctagga aaagttaggg tctggcacag aggctcacgt ctgtgatccc agcactttgg 9900
gaggctgagg caggcagatc actggaagtg ctggacgggt ggggaagtgc cgggtgcaag 9960
aaccaagctc tttgactatg gacctcagcc tgaggttggt caagaggtgg agtgagtggg 10020
ggctgaggac cttcatcctg aaaccctgat gcaggagagt ctggggtctg ccttctaccc 10080
tcatgtggcg ggtgaaggag caaggttctc aactcaggag ggttcttccc ctctccattc 10140
ccacccaggg gacatctcac aacaactaga aacaattttg tcgcagctgg ggggtgggag 10200
gtgtgttcct ggcatctatc taatgggtgg gggcgaggga cgcagcccaa caccctacac 10260
tgcacaggac acagcgagat ccggcctcaa atggcagcca tggcagcgtc agccctccag 10320
ggggcgcgcc cctggcgcag gtggtgtgcc ggcccacagc tccttgcagg ctgggagctg 10380
cattttcgtg acatgtcatg agtcctcaga gaaaaagagg gaacgagtgc atggtgggga 10440
ggggccctgg cgtgctggag tctctgggtt tccttctcca gagacccctg cagtcagctg 10500
agcgcaatca gtcacgttgg gctttgcttg gatctcactg gaatttttcg agccacccct 10560
tagtcctcac cttgctaagc cctcacgtct caataacctc aaacctcagt acctgggctg 10620
agaaagcctg agtggccctg ggagagagac cctgcaccca aggacaagga catccctgct 10680
tcacccaacc caaaggccag tctggacata tgaactcaac cagctaagag tgatatgatt 10740
gattgatgag aatcaccaga gcacttgcca gagtttcagc ttctccctgg gccaaagtga 10800
agtttgcttt acacagtaaa tgtgctctgt gcaggtcctg aatttagaag gctgtgctgt 10860
gtcatcctgc tctgtaaatg gccagtagga cccccgcccc ttctcaaggc acattacccg 10920
tttaaaacgg gggaggcaag agcacaaagc gcccacctat tcaccgaaga gcatgtatat 10980
aacttagggc cttccatcct taaacaacag gaccttcctt gctcttaagg aaaaggaaac 11040
aggttcagag acgttaattc attgccaagg tcacacagat aatgggtcca gcgaagagtg 11100
gtgtccgagc ccaaggcagc aggcctttgg ccactgcagt gttaaacagc acagctggtg 11160
tggaagtccg gtgctgagtc ctgggtacct ggactcggag ggaagctggc tgcaggggga 11220
aggggctgcg cagttgtgga tgtacctgtc gtctgctggg gggcgtgcgg gtggacacag 11280
tcccccggcc tggggagcct cgtgggagaa ttaagagtta ctccgggcca aatggccgga 11340
gttgtcagat ctggcagcgt cttcgctggg gctccaggga gctgctgctg gggtggaagc 11400
tctcacactc tttctccacg tgccctttcc agttccctga catcgtggag ttctgcgagg 11460
ccatggccaa cgccgggaag accgtaattg tggctgcact ggatggaccc ttccagagga 11520
aggtaaggcg tctgatccag gtctggagct gggattgagg agggcaagag gcttctggat 11580

gggcacagag acaccagctc tgggtgacca gggctcagcc accacagggt tacggccgag 11640

ctgctcaggc cttggctgag ccaagggact ccatggtctg tgcagactgc gtgccatctg 11700

ttgcggcagg tgctttgaat tggcaaaggg acagagccgg gcatggtgct ctgggggttg 11760

ggggaaggac taaggtcaga gcaaactctc ctggcttcag tgcttgtgaa tcagagggtt 11820

taaaagaaaa acccacctgg taaggtggct gagcgccctc tgtctttcca tgggagcgca 11880

gccatttggg gccatcctga acctggtgcc gctggccgag agcgtggtga agctgacggc 11940

ggtgtgcatg gagtgcttcc gggaagccgc ctataccaag aggctcggca cagagaagga 12000

ggtagctcca cctgccttcc ctgcaggccg gcggggtggg ggtatggctc tgcctccttc 12060

ctgtcctggc ccttcaccca tcccctgtcc ctgcggccag gtcgaggtga ttgggggagc 12120

agacaagtac cactccgtgt gtcggctctg ctacttcaag aaggcctcag gccagcctgc 12180

cgggccggac aacaaagaga actgcccagt gccaggaaag ccaggggaag ccgtggctgc 12240

caggaagctc tttgccccac agcagattct gcaatgcagc cctgccaact gagggacctg 12300

cgagggccgc ccgctccctt cctgccactg ccgcctactg gacgctgccc tgcatgctgc 12360

ccagccactc caggaggaag tcgggaggcg tggagggtga ccacaccttg gccttctggg 12420

aactctcctt tgtgtggctg ccccacctgc cgcatgctcc ctcctctcct acccactggt 12480

ctgcttaaag cttccctctc agctgctggg acgatcgccc aggctggagc tggccccgct 12540

tggtggcctg ggatctggca cactccctct ccttggggtg agggacagag ccccacgctg 12600

ttgacatcag cctgcttctt cccctctgcg gctttcactg ctgagtttct gttctccctg 12660

ggaagcctgt gccagcacct ttgagccttg gcccacactg aggcttaggc ctctctgcct 12720

gggatgggct cccaccctcc cctgaggatg gcctggattc acgccctctt gtttcctttt 12780

gggctcaaag cccttcctac ctctggtgat ggtttccaca ggaacaacag catctttcac 12840

caagatgggt ggcaccaacc ttgctgggac ttggatccca ggggcttatc tcttcaagtg 12900

tggagagggc agggtccacg cctctgctgt agcttatgaa attaactaat t 12951

<210> 103
<211> 19
<212> DNA
<213> Artificial

<220>
<223> Synthesis

<220>
<221> misc_signal
<222> (1)..(19)

<400> 103
attctcgggc cgatgttct 19

<210> 104
<211> 24
<212> DNA
<213> Artificial

<220>
<223> Synthesis

<220>
<221> misc_signal
<222> (1)..(24)

<400> 104
gcacttgtac tgagcaatct ggaa 24

<210> 105
<211> 5698
<212> DNA
<213> homo sapiens

<400> 105
aggttcaagt ggagctctcc taaccgacgc gcgtctgtgg agaagcggct tggtcggggg 60

tggtctcgtg ggtcctgcc tgtttagtcg ctttcagggt tcttgagccc cttcacgacc 120

gtcaccatgg aagtgtcacc attgcagcct gtaaatgaaa atatgcaagt caacaaaata 180

aagaaaaatg aagatgctaa gaaaagactg tctgttgaaa gaatctatca aaagaaaaca 240

caattggaac atattttgct ccgcccagac acctacattg gttctgtgga attagtgacc 300

cagcaaatgt gggtttacga tgaagatgtt ggcattaact atagggaagt cacttttgtt 360

cctggtttgt acaaaatctt tgatgagatt ctagttaatg ctgcggacaa caaacaaagg 420

gacccaaaaa tgtcttgtat tagagtcaca attgatccgg aaaacaattt aattagtata 480

tggaataatg gaaaaggtat tcctgttgtt gaacacaaag ttgaaaagat gtatgtccca 540

gctctcatat ttggacagct cctaacttct agtaactatg atgatgatga aaagaaagtg 600

acaggtggtc gaaatggcta tggagccaaa ttgtgtaaca tattcagtac caaatttact 660

gtggaaacag ccagtagaga atacaagaaa atgttcaaac agacatggat ggataatatg 720

ggaagagctg gtgagatgga actcaagccc ttcaatggag aagattatac atgtatcacc 780

tttcagcctg atttgtctaa gtttaaaatg caaagcctgg acaaagatat tgttgcacta 840

atggtcagaa gagcatatga tattgctgga tccaccaaag atgtcaaagt ctttcttaat 900

ggaaataaac tgccagtaaa aggatttcgt agttatgtgg acatgtattt gaaggacaag 960

ttggatgaaa ctggtaactc cttgaaagta atacatgaac aagtaaacca caggtgggaa 1020

gtgtgtttaa ctatgagtga aaaaggcttt cagcaaatta gctttgtcaa cagcattgct 1080

acatccaagg gtggcagaca tgttgattat gtagctgatc agattgtgac taaacttgtt 1140

gatgttgtga agaagaagaa caagggtggt gttgcagtaa aagcacatca ggtgaaaaat 1200

cacatgtgga tttttgtaaa tgccttaatt gaaaacccaa cctttgactc tcagacaaaa 1260
gaaaacatga ctttacaacc caagagcttt ggatcaacat gccaattgag tgaaaaattt 1320
atcaaagctg ccattggctg tggtattgta gaaagcatac taaactgggt gaagtttaag 1380
gcccaagtcc agttaaacaa gaagtgttca gctgtaaaac ataatagaat caagggaatt 1440
cccaaactcg atgatgccaa tgatgcaggg ggccgaaact ccactgagtg tacgcttatc 1500
ctgactgagg gagattcagc caaaactttg gctgtttcag gccttggtgt ggttgggaga 1560
gacaaatatg ggggttttccc tcttagagga aaaatactca atgttcgaga agcttctcat 1620
aagcagatca tggaaaatgc tgagattaac aatatcatca agattgtggg tcttcagtac 1680
aagaaaaact atgaagatga agattcattg aagacgcttc gttatgggaa gataatgatt 1740
atgacagatc aggaccaaga tggttcccac atcaaaggct tgctgattaa ttttatccat 1800
cacaactggc cctctcttct gcgacatcgt tttctggagg aatttatcac tcccattgta 1860
aaggtatcta aaaacaagca agaaatggca ttttacagcc ttcctgaatt tgaagagtgg 1920
aagagttcta ctccaaatca taaaaaatgg aaagtcaaat attacaaagg tttgggcacc 1980
agcacatcaa aggaagctaa agaatacttt gcagatatga aaagacatcg tatccagttc 2040
aaatattctg gtcctgaaga tgatgctgct atcagcctgg cctttagcaa aaaacagata 2100
gatgatcgaa aggaatggtt aactaatttc atggaggata gaagacaacg aaagttactt 2160
gggcttcctg aggattactt gtatggacaa actaccacat atctgacata taatgacttc 2220
atcaacaagg aacttatctt gttctcaaat tctgataacg agagatctat cccttctatg 2280
gtggatggtt tgaaaccagg tcagagaaag gttttgttta cttgcttcaa acggaatgac 2340
aagcgagaag taaaggttgc ccaattagct ggatcagtgg ctgaaatgtc ttcttatcat 2400
catggtgaga tgtcactaat gatgaccatt atcaatttgg ctcagaattt tgtgggtagc 2460
aataatctaa acctcttgca gcccattggt cagtttggta ccaggctaca tggtggcaag 2520
gattctgcta gtccacgata catctttaca atgctcagct ctttggctcg attgttattt 2580
ccaccaaaag atgatcacac gttgaagttt ttatatgatg acaaccagcg tgttgagcct 2640
gaatggtaca ttcctattat tcccatggtg ctgataaatg gtgctgaagg aatcggtact 2700
gggtggtcct gcaaaatccc caactttgat gtgcgtgaaa ttgtaaataa catcaggcgt 2760
ttgatggatg gagaagaacc tttgccaatg cttccaagtt acaagaactt caagggtact 2820
attgaagaac tggctccaaa tcaatatgtg attagtggtg aagtagctat tcttaattct 2880
acaaccattg aaatctcaga gcttcccgtc agaacatgga cccagacata caaagaacaa 2940
gttctagaac ccatgttgaa tggcaccgag aagacacctc ctctcataac agactatagg 3000
gaataccata cagataccac tgtgaaattt gttgtgaaga tgactgaaga aaaactggca 3060

gaggcagaga gagttggact acacaaagtc ttcaaactcc aaactagtct cacatgcaac 3120
tctatggtgc tttttgacca cgtaggctgt ttaaagaaat atgacacggt gttggatatt 3180
ctaagagact tttttgaact cagacttaaa tattatggat taagaaaaga atggctccta 3240
ggaatgcttg gtgctgaatc tgctaaactg aataatcagg ctcgctttat cttagagaaa 3300
atagatggca aaataatcat tgaaaataag cctaagaaag aattaattaa agttctgatt 3360
cagaggggat atgattcgga tcctgtgaag gcctggaaag aagcccagca aaaggttcca 3420
gatgaagaag aaaatgaaga gagtgacaac gaaaaggaaa ctgaaaagag tgactccgta 3480
acagattctg gaccaacctt caactatctt cttgatatgc ccctttggta tttaaccaag 3540
gaaaagaaag atgaactctg caggctaaga aatgaaaaag aacaagagct ggacacatta 3600
aaaagaaaga gtccatcaga tttgtggaaa gaagacttgg ctacatttat tgaagaattg 3660
gaggctgttg aagccaagga aaaacaagat gaacaagtcg gacttcctgg gaaagggggg 3720
aaggccaagg ggaaaaaaac acaaatggct gaagttttgc cttctccgcg tggtcaaaga 3780
gtcattccac gaataaccat agaaatgaaa gcagaggcag aaaagaaaaa taaaaagaaa 3840
attaagaatg aaaatactga aggaagccct caagaagatg gtgtggaact agaaggccta 3900
aaacaaagat tagaaaagaa acagaaaaga gaaccaggta caaagacaaa gaaacaaact 3960
acattggcat ttaagccaat caaaaaagga aagaagagaa atccctggtc tgattcagaa 4020
tcagatagga gcagtgacga aagtaatttt gatgtccctc cacgagaaac agagccacgg 4080
agagcagcaa caaaaacaaa attcacaatg gatttggatt cagatgaaga tttctcagat 4140
tttgatgaaa aaactgatga tgaagatttt gtcccatcag atgctagtcc acctaagacc 4200
aaaacttccc caaaacttag taacaaagaa ctgaaaccac agaaaagtgt cgtgtcagac 4260
cttgaagctg atgatgttaa gggcagtgta ccactgtctt caagccctcc tgctacacat 4320
ttcccagatg aaactgaaat tacaaaccca gttcctaaaa agaatgtgac agtgaagaag 4380
acagcagcaa aaagtcagtc ttccacctcc actaccggtg ccaaaaaaag ggctgcccca 4440
aaaggaacta aaagggatcc agctttgaat tctggtgtct ctcaaaagcc tgatcctgcc 4500
aaaaccaaga atcgccgcaa aaggaagcca tccacttctg atgattctga ctctaatttt 4560
gagaaaattg tttcgaaagc agtcacaagc aagaaatcca aggggagag tgatgacttc 4620
catatggact ttgactcagc tgtggctcct cgggcaaaat ctgtacgggc aaagaaacct 4680
ataaagtacc tggaagagtc agatgaagat gatctgtttt aaaatgtgag gcgattattt 4740
taagtaatta tcttaccaag cccaagactg gttttaaagt tacctgaagc tcttaacttc 4800
ctcccctctg aatttagttt ggggaaggtg tttttagtac aagacatcaa agtgaagtaa 4860
agcccaagtg ttctttagct ttttataata ctgtctaaat agtgaccatc tcatgggcat 4920
tgttttcttc tctgctttgt ctgtgttttg agtctgcttt cttttgtctt taaaacctga 4980

tttttaagtt cttctgaact gtagaaatag ctatctgatc acttcagcgt aaagcagtgt 5040

gtttattaac catccactaa gctaaaacta gagcagtttg atttaaaagt gtcactcttc 5100

ctccttttct actttcagta gatatgagat agagcataat tatctgtttt atcttagttt 5160

tatacataat ttaccatcag atagaacttt atggttctag tacagatact ctactacact 5220

cagcctctta tgtgccaagt ttttctttaa gcaatgagaa attgctcatg ttcttcatct 5280

tctcaaatca tcagaggcca aagaaaaaca ctttggctgt gtctataact tgacacagtc 5340

aatagaatga agaaaattag agtagttatg tgattatttc agctcttgac ctgtcccctc 5400

tggctgcctc tgagtctgaa tctcccaaag agagaaacca atttctaaga ggactggatt 5460

gcagaagact cggggacaac atttgatcca agatcttaaa tgttatattg ataaccatgc 5520

tcagcaatga gctattagat tcattttggg aaatctccat aatttcaatt tgtaaacttt 5580

gttaagacct gtctacattg ttatatgtgt gtgacttgag taatgttatc aacgtttttg 5640

taaatattta ctatgttttt ctattagcta aattccaaca attttgtact ttaataaa 5698

<210> 106
<211> 29375
<212> DNA
<213> homo sapiens

<400> 106
aggttcaagt ggagctctcc taaccgacgc gcgtctgtgg agaagcggct tggtcggggg 60

tggtctcgtg gggtcctgcc tgtttagtcg ctttcagggt tcttgagccc cttcacgacc 120

gtcaccatgg aagtgtcacc attgcaggta cggctcgcgg gggggactgg cggtggagcc 180

tccgcgcggc ccgggcatct ctctggccgc ccgtgacggg tgaagctctg gggctgcggt 240

caggccggcg accggcttgg gagcccatat tctccattcc cggttccggg gtgatcgtgg 300

agaggcggaa gccccttctg gtgctagtag tgaagtatga cccggcttcc agggtgtcgt 360

gcgttgttgc cctgtttcta ggggcaggag tccgttggtc ccgtagtgga tccatgttac 420

agcggcccgg gtgcgacgtt attgagtcgc gcgtacagat gctttcccct cctgcccgct 480

gcttgaaaac ggtcttgaat gtccccgatc ttggaaaggg cagagcctag cacagtgttt 540

ctctggagca gctggccagc ttacaaaagc gccccctcc tgagttcaca acgctcaggt 600

ggccaaggga ttgaacggat agccgcccaa actgctgctg ccgaactaga attatttacg 660

gtgtcgtgtg ctcgggacac cgtaaataat ttataataat aggcactcgg taaataatgt 720

atgcaggaac tgaaagaagt gggtgtattt tattgatgac atctaccatg ttgctcaaga 780

aaagaggttt ccagacagac ccacttggtg agataatatg tgtctgttta acctgacacc 840

tggggacctg gccgcagcga atttaaattg tcctatttgt acaaaactgg caactccacc 900

actgcctact aacagattgc aatgcatgtt gacatctcac tatggtaggc attttgttcc 960

tagtttaggg taaacttatg attaaaaatt cccttctttc tctctttttt tttaattgct 1020
agcctgtaaa tgaaaatatg caagtcaaca aaataaagaa aaatgaagat gctaagaaaa 1080
gactgtctgt tgaaagaatc tatcaaaaga aaacacaatt ggaacatatt ttgctccgcc 1140
cagacaccta cattggttct gtggaattag tgacccaggt aaaatcaagc tcatcaaagg 1200
cagttaacct agtagttgaa atggatactc ttctttctgt acctttcatg tactgagtgt 1260
gacctttctg tatctctcat gtactgagtg tcactggaac tggcctaaat atggtaatac 1320
ttaatatgaa cactttatgg taatcatgat aacagaacaa tgaatataga ataaaaataa 1380
cagtatttct ttgccttatt tctagcaaat gtgggtttac gatgaagatg ttggcattaa 1440
ctatagggaa gtcacttttg ttcctggttt gtacaaaatc tttgatgaga ttctaggtga 1500
gtaaatcctt taatataaag atctggtcaa atctttcata agaagtatca ttccaaataa 1560
aaatttaaaa aagataaaag taaaaacaaa aagtatcatt tctaccaaac ctgctgtagc 1620
cagggctcct gtcatttcaa tcagttgata aaggtgagct ggtttcctgg agcccaggag 1680
gttgagcttc tcgtagaaac agtactatgg acaaaaattg ctaaattaac accttgttaa 1740
tgccactcct gtgctgtttg ggaacttttt tttctatatc ttacttagag ttacagaatg 1800
tttcttaaaa cactaatttc ttacaaatgt atattttatt ttctttcagt taatgctgcg 1860
gacaacaaac aaagggaccc aaaaatgtct tgtattagag tcacaattga tccgtacgtc 1920
ttttgcttag tttttgttat gtagctcgtt gctattgtta gctgcatgta ttttgattac 1980
tggtgaagtt ggcttttcct aaacagaagt tgagaaaagt acacaaaagc agaatgctaa 2040
aatgaaaaac ctatggatac ccaccaccta gatccaacag ttgccaacgt tttccatat 2100
tttctccatc tttttttaa ttagttaatt tttttagagg aaggggtctt gctattttgc 2160
ctgggctggt cttagaactc ctggcctgaa gcaatcctcc agtgttggca ttatgggtgt 2220
gtgccatggc acgcggccta tttgctctgg atcatttcaa aataaattac aggacttatg 2280
acacctaatc cctaaatatt tgagcaagca ccttcagtaa ttgctttaag tattctggca 2340
tctggagttt taactttttc tcatgatgct attcaaacag taaaccagta ggtggtggta 2400
gagaataatt tgatctgaca tttctgctta taaatgcggg gtgtccctta gtgggtgatc 2460
agggtgctta tttcactttt ttgttagtct gattgattat gacaaagtat acctggattt 2520
tcctaaggac tcaatatcat agtctttaaa aaatgttgag ctagggccag gcacggtggc 2580
tcatgcctgt gatcccagca ctttgggagg ccgatgcggg cggatcacaa ggtcaggaga 2640
tcaagaccat cctggctaac acggtgaaac cctgtctcca ctaaaaatac aaaaaattag 2700
ccgggtgtag tggtgggcgc ctgtagtccc agctactcga gaggctgagg caggagaatg 2760
gcatgaaccc gggaggtgga gcttgcagtg agccgagatt gcaccactgc actccagcct 2820

gggtgacaca gcaagactct gtctcaaaaa aaaaaaaaaa gttgagctaa ttttaattag 2880
ttttttttat agataataag tactatgtag aacaagaacc ataatgtata tcgagttatt 2940
aactaaaata tagtaccatc caagaattaa cttaaaataa ctgaaataag ttttcagcat 3000
tttatttgag agcaaggcag tttatgattg atattggttt tccttttttt tttttttttt 3060
tttgatggag ttttgctctt attgcccagt ctggagtgca gtggcatgat ctcggctcac 3120
cgcaacatcc acctcctggg ttcaagcaat tctcctgcct cagcctccag agtagctggg 3180
attacaggcg cacaccacca cacctggcta attttgtatt ttttttagta gagatggggt 3240
ttctgcatgt tggtcaggct ggtctcgaac tcctgacctc aagtgatctg cctgccttag 3300
cctcccaaag tgctgggatt gctgggatta caggcgtgag ccactgcgcc cagcctgttt 3360
ttatgtactt tgttgagtgg gaacgagatg tatagatgat cctcttgaaa ggggtataat 3420
aggcatgatg actcactcct gtaatcccag cactttggga ggccaaggtg ggaggatcag 3480
ttgagcccag gagtagtttg agaccagcct gggccatata gtgagaccct catctctaca 3540
aaaaatttaa aaattagcca ggtgtggtga tggacacctg tagtcccagc tattcaggag 3600
gctgaagtgg gaggattgct tgagcccagg aggtcaagac tgcagtaagc tgtgatcctg 3660
ccattacact gcagcctggg tgacagagcg agatgctgtc tcaaaaaagg agtgggagtg 3720
gtgtataata atgtttccag attgtccttt tctttccatt gggcataaat catttccctc 3780
tgtttatcct aacattttaa aattttgtat tttttcaaca ttatcagtgt ctattcaggt 3840
gttggaaaag gttttttttt cctcttgact cctatttcag gaatgtcagg aactaagcat 3900
ggtgggaaat gttcctgctg aatttcagaa ggtatatatg aaaagtgcca aaaaaattga 3960
gggctgggta tggtggctca catctataac cccaacactt tgagcagttc tcctgcttca 4020
gcctcccaag tagctggaat cacaggcatg cgctaccatg cctggctaat ttttttttatt 4080
tttagtagag acggtttcaa catgttggcc agaactcctg gcctcaagtg atctgccctc 4140
ctcagcctcc caaagtgctg ggattacagg cacaggccac cgcgcccagc ctgaatatac 4200
atattttaga aataatagta tttaaatatt tttaaatgtt aagcattcgt ttaatgtgtt 4260
gtaaaataca attttcaatc tttttttctt agggaaaaca atttaattag tatatggaat 4320
aatggaaaag gtattcctgt tgttgaacac aaagttgaaa agatgtatgt cccagctctc 4380
atatttggac agctcctaac ttctagtaac tatgatgatg atgaaaagaa agtgacaggt 4440
agagtattga ggggaaataa catatttgtt gctaaaaata tatatattta aatgactgtc 4500
tgtggcatga gggttaaaga tatggaaata aatctctata attgaatagc tctgccagtg 4560
attaagaaat aaagctgtca atgagatagt aacaataaaa tagtgtttca tatttatttg 4620
cccaggtggt cgaaatggct atggagccaa attgtgtaac atattcagta ccaaatttac 4680
tgtggaaaca gccagtagag aatacaagaa aatgttcaaa caggcaagta aataagtgtc 4740

ttgtacctta atgataaatg gtagtagtat agccatttat aatggcatta atgattggtt 4800
taatttaaca taatttataa gctattgaag tatggaaaat tataagcata tatattaggt 4860
tattaggact cataaattta tgttatttac ttccagtttg tgagatgact tgaatttttc 4920
atgtttccta ttctttactt ccatagacat ggatggataa tatgggaaga gctggtgaga 4980
tggaactcaa gcccttcaat ggagaagatt atacatgtat caccttttcag cctgatttgt 5040
ctaagtttaa aatgcaaagc ctggacaaag atattgttgc actaatggtc agaagagcat 5100
atgatattgc tggatccacc aaagatgtca aagtctttct taatggaaat aaactgccag 5160
tgagtatttt cctggatgtt aaggataata agggattttg taatcattgt caagtgcaaa 5220
attgaatttt ttcccctccc atatgttttt gtttgtttgt ttgtttgttt gtttgagaca 5280
gagtctcaca ctgttgcccg ggctggagtg cagtggcacg atctcggctc accgcaacct 5340
ccacctccca ggttcacgca attctcctgc ctcagcctcc caagtagctg ggattacagg 5400
tgcctgccac cacacctggc taattttttg tatttttagt agagacaggt ttcactatgt 5460
tggccaggct ggtctcgaac accagacctc atgatccacc cgtcttggcc tcccaaagtg 5520
ctgggattac aggcatgagc cactgcacct ggcccaacca tatgtatttt cttaccactt 5580
ctcacatatg ttcttgaaaa gagaatggta tgccacattt tttaatcagc tcattttaaa 5640
cttaccgaag gaatttcttt ctcaaagaaa cacctaaaat aaatatttca tgtccttttt 5700
ttattttcct ttttctttct tttcttgata acctcgctgt gtcacccagg ctggagtaca 5760
gtgatgcaat cacggctcac tacagcctgg acctcccagg ctcaagcgat catcccacct 5820
cagcttctgg agtagctgga aatgcaggca gcaccaccat gcccagctaa tttttttttt 5880
tctttttaat agaggtgggg atctcactat gttgcccagg gtggtcttga actcctgggc 5940
tcaagtgatc cacccacctc ggcctgtgtc ctttaatgac cattccctta tgcctatcag 6000
tgaacatcat tgcattggtt ttggaaagtc ctcatagtct atcattgaac ctatttttta 6060
ataactttct taatactgtt acctttaatt cctgtacagg taaaaggatt tcgtagttat 6120
gtggacatgt atttgaagga caagttggat gaaactggta actccttgaa agtaatacat 6180
gaacaagtaa accacaggtg ggaagtgtgt ttaactatga gtgaaaaagg ctttcagcaa 6240
attagctttg tcaacagcat tgctacatcc aaggtaattt tattcttaaa ttattaatca 6300
tgatttatct ttacatatat gtgttcttat tgtttttaat atataaagtg gacttgaata 6360
ttgggctagc ttagtataaa ggaggttaaa ttagtttttta atgtttgatt attataattt 6420
tgaggatact gagttttaca gtttggtatt tttccttatt agggtggcag acatgttgat 6480
tatgtagctg atcagattgt gactaaactt gttgatgttg tgaagaagaa gaacaagggt 6540
ggtgttgcag taaaagcaca tcaggtatgt gcttttggca gttttctttt tctaaagtca 6600

aggaagaaga gaaaggctat aaataaagca tgagtacatt tttagtggct taatatcaac 6660
ttctattgca ggtgaaaaat cacatgtgga tttttgtaaa tgccttaatt gaaaacccaa 6720
cctttgactc tcagacaaaa gaaaacatga ctttacaacc caagagcttt ggatcaacat 6780
gccaattgag tgaaaaattt atcaaagctg tgagtactta gaggaaaata aaaatagaaa 6840
cacctgactt tattttccat tgcacttctt agctctgcag aaacaatgat tcttctcata 6900
gtgagcttct ccaagtcttc ccaatctgaa aaggaagtaa aaaagggctt tactttaact 6960
gatttaccaa agacttaatg accgtctata tttcagtatt tcccaattac attttaccat 7020
taagcttaga tcacttttga attaatctag ctgtttaaca aacaccctca cttaaatgcc 7080
taagacttgc tttcagtcaa cacatccaaa attgaatttg ttacctccat actcactgat 7140
ttgcccatac aagcagcccc ccactctcca acaaaaaaac aacttcctat cttagtaaaa 7200
agccccaacc aacctctagg ttgtataaac aagaaagctg ggagccttcc tttatttccc 7260
ctcctctcta atccggtcaa taagaatcat ctcttggatg ctgcagtagc ttctcaccat 7320
tatctctttt ttggtttact acaataggtt cttaaccttc atactggtta agtcctttcc 7380
ttggaatgct tttgagtgac ttttgtgtta aaacacccat ttttatcttc actctcattt 7440
gaaatctttc aatgacttcc actcagggaa agtccaaatt ccataatttg gccaacaaga 7500
aagatctgct gtaatctaat tacacctact tctccaactc atctcagtgc cagtttttcg 7560
tatattgtcc tgttgctttt aaattactga aaagcacagt gctcttcccc tcctcagagt 7620
ttattcacat gctaatccct ctgcatgaaa tacatccttt tcacctggct actttaggtc 7680
ttgtcctttc ctcaggaaag cctttacttt ctacccttcc ccccacctaa gttggttcca 7740
atataatatt gaacatacct tattagcaaa cttcttgctt atccataaca cttataacac 7800
tgtaacttat tttatttctg tctttttttt taggcagagt ctcgctctgt cgcccaggct 7860
ggaatgcagt ggcacgatct cggctcactg caacctccgc ctcctaggtt tcagtgattc 7920
tcttgcctca gcctcccgag tagctgagat tacaggcatg catcaccatg cctggctaat 7980
ttttttgttg ttgtattttc agtagagaca gcgtttcacc atgttggtca ggctggtctc 8040
aaactcctga cctcaaatga tccacccatc tcggccttcc aaagtgttgg gattacagcc 8100
atgagccact gtgcccagtc tttttttttt ttttttttga gacagagtct tgctgtgttg 8160
cccaggctac agtgcagtga cgtaatcctg gctcattgca atctctgcct cccaggttca 8220
agcgattctc cttgcctcct gagtagctgg gattctccag cctcctgagt agcggggatt 8280
acagacatgc accatcacat ccagctaatt tttgtatttt taatagagat ggggttttac 8340
catgttggcc aggctggtct tgaactcctg gcctcacttg atccaccatc ctcggcctcc 8400
taaagtgctg tgattacagg cgtaagccac tgcacccagc tatgtctgtc ttctatgttg 8460
tgtcttaaac ttgatgagga caagtgtctc aatttgtttt atttgtgtct aattttattt 8520

atttattttt aatttttttt tttggagaca gcttctcact gtttcccagg ctggagtgcc 8580
gtggcttgat ctcagttcac tgcaacccgc gcctcccagg ttaaagcaat tctcctgcct 8640
cagcctccca agcagctagg attacagcca tttcaccacc accatgcctg gctacccttt 8700
tttttttttt tttttttttt tttgagacgg agtttcactt ttgtcaccca ggctggagtg 8760
caatggtgcg atcttggctc gctgcaacct ctacctcctg ggttcaagcg attctcctgc 8820
ctcagcctcc cgagtagctg gaattacagg tgcccaccac cacgccagct aatttttgta 8880
tttttagtag agccggggtt tcgccatgtt ggccaggccg gtctcaaact cctgacctca 8940
ggtgttctgc ccaccttggc ctcctaaagt gctgggatta taggcgtgag ccaccgtgcc 9000
tggtctaatt tgttttaacc actatatctc caacaagtag ctcagtgcta gcacaatata 9060
attatatagt aaatatttat tgaacgaatg aaccaaaagg agcagctccc tcagtggtga 9120
taacctgaca tgggaagatg tgccaccctc tatccagaaa ttattgttct acatcttttt 9180
aatttttgaa tcatttttat ttgtattaag gctcatttgt attctagatt tctgatagat 9240
cccttcttcc ctaatatgat ccctaatatg aatcttctcg ttttcaggcc attggctgtg 9300
gtattgtaga aagcatacta aactgggtga agtttaaggc ccaagtccag ttaaacaaga 9360
agtgttcagc tgtaaaacat aatagaatca agggaattcc caaactcgat gatgccaatg 9420
atgcaggtat atatttaata atgtttccaa acttttaagt cttatagttg ttattttatt 9480
cattaatggc ataccacgga tatttatttt tcccttgaca gaataactat attcaacaga 9540
ataacttgtt aaaaatcggc ccgtttccta ttatggaaga tttaggtcat ttccatgtta 9600
taaataatat tgaggtgatt attttggagt ataaaacaag aatgtttata ttatgatcta 9660
ttacctaaca aataattttg ctcattatat agtaaattgt gttttatcac aaggctataa 9720
acagcatgtt caagttagta tatttgaggt tgaactaaat gtgctaatat taatatgtat 9780
atttttattt taggggggccg aaactccact gagtgtacgc ttatcctgac tgagggagat 9840
tcagccaaaa ctttggctgt ttcaggcctt ggtgtggttg ggagagacaa atatggggtt 9900
ttccctctta gaggaaaaat actcaatgtt cgagaagctt ctcataagca ggtagaatat 9960
aagacgatct tcagaatcta aatctaattt ataatacaag actttatgct tatatttaat 10020
tccctcatta ggcattttaa aatatatttt agacaatttg tgcttatttt gagaaattag 10080
gtacattgta gcctatttta acagaccttt ctgatgtagt aaattataag ctaatagctc 10140
aaaatactgg agctcaagaa aatccaagca acatatactg ttaaatttct ttgttctttt 10200
caaatttata aacgatgctt tttttggtat atgtccattt cagatcatgg aaaatgctga 10260
gattaacaat atcatcaaga ttgtgggtct tcagtacaag aaaaactatg aagatgaaga 10320
ttcattgaag acgcttcgtt atgggaagat aatgattatg acagatcagg tcagatttgt 10380

tattaaattt ttagattgtt caactaaatt aagcatgtct taatttaatt tcattgtttt 10440
ttgccatgaa aataaattag ttaaatagga gctttattca tcatctctaa tcaacatcta 10500
atcagatatg cttatatcat atgtatgttg caaatacagg ttaagtgagt ctggatttga 10560
acagaccttt tttgattccc atagaaaatt tgacaaattg ccagtaggtc agtcataata 10620
tttttttatt tctaaacaat tctttgtttg tttgagatgg agtttcgccc ttgtcgccca 10680
ggctggagtg caatggtgca atcttggctc actgcaacct ccgcctcatg ggttcaagcg 10740
attctcctgc ctcagcctcc cgagtagctg ggattgcagg cggatgccac cacacccaac 10800
taatttttgt atttttagtg gagacagggt ttcaccatgt tggccaggct ggtctcgaac 10860
gcctgacctc aggcgatccg cctgcctcgg cctcccaaag ttctgggatt acagatgtta 10920
gctaccacgc ccagcctaac agttcttttg aactttggct ttcaaatctt tctaggacca 10980
agatggttcc cacatcaaag gcttgctgat taattttatc catcacaact ggccctctct 11040
tctgcgacat cgttttctgg aggaatttat cactcccatt gtaaaggtac gctaatttct 11100
aagtaccatc atggatattt taagacccta ctcctcaaac ctggatatac atataagccc 11160
cgtcacatgt agtggtatac aggggacccc acagtgtaca aagccacagt cattgttttt 11220
tatcacaggt atctaaaaac aagcaagaaa tggcatttta cagccttcct gaatttgaag 11280
agtggaagag ttctactcca aatcataaaa aatggaaagt caaatattac aaaggtttgt 11340
aatgaaaccc atatagaact tctcatttta ttatacaccc tgtacaagac tatatgaagg 11400
aacttggtat ttttggtttt ataggtttgg gcaccagcac atcaaaggaa gctaaagaat 11460
actttgcaga tatgaaaaga catcgtatcc agttcaaata ttctggtcct gaagatgatg 11520
ctgctatcag cctggtaggt ttgagttgta ttttatatac attctaattt tagaaatcac 11580
tactttagcc agttgaaaca tttacatttt tgtataagac tccgtatcaa aaaaaaaaga 11640
atgttttata gaatagaatg tttccagtaa gcatatccca gagaaggaat cagatatttt 11700
agaaaatttt gatttttggc tgggcttggt ggctcatgcc tataattcta ggactttggg 11760
aggccaaggc aggtggatca cctgagatca agagttggag accaacctgt ccaacatggt 11820
gaaacccct ctctactaaa aatacgaaaa ttggccgagc gtagtggctc acgcctgtaa 11880
tcccagcact ttgggaggca gaggcgagca gatcacaagg tcaggagatc aagaccatcc 11940
tggctaacaa ggtgaaaccc catctctgct aaaaatacaa aaaattagcc ggccgtggtg 12000
gcacgcagct atagtcccag ctacttggga agctgaggca ggagaatcgc ttgaacccga 12060
gaggcagagg ttgcagtgag ccgagatcag gccactgctc tccagcctgg gcaacagagt 12120
gagactccat ctcaaaaaaa aaggaaaaag gccaggcgtg gtggctcacg cctgtaatgc 12180
cagtactttg gaaggctaag tcgggcagat cacaacgtca tgagatggag accatcctgg 12240
ctaacacggt gaaaccccgt ctctactaaa aataaaaaaa aataaaaaat aaaaaattag 12300

ccgggcttgg tgacacgcgc ctgtaatccc agctactccg gaggctgagg caggagaatg 12360
gcgtgaaccc aggaggcgga gcttgcagtg agctgagatg gtgccactgc actccagcct 12420
gggcgacaga gcgagactct gtctcaaaaa aaaaaaaaaa caaaaaaggt cgggcgcagt 12480
ggctcacacc agtaatcata gcactttggg aggccgaggc gggtggatca cctgagctcg 12540
ggagttcaag accagcctga ccaacatggt gaaaccccat ctctactaaa aatacaaaat 12600
attagctggg tgtggtggca cgtgcctgta atcccagcta cacgggaggc tgaggcagga 12660
gaatcacttg aacttgggaa gtggaggttg tgggagccga aatcgtgcca tcacattcca 12720
caagagtgaa actccatttc aaaaaaaaaa aaatacaaaa attagctggg cgtggtggcg 12780
ggcgccttta atcccagggg ctcaggaggc tgaggcagga gaatcgcttg aacctaggac 12840
gcagaggttg cagtgagcca agatgacacc attgcactcc aggctgggca agagaacagt 12900
aactcccgtc tcaaaaaaaa aaaaaaagga aaacagtatt tttgtttttc tgttgttcgt 12960
tctttgtaga aggatatttt ggaacctatt agcctattag taccagtgac atcttttcat 13020
cctttaattt ataggccttt agcaaaaaac agatagatga tcgaaaggaa tggttaacta 13080
atttcatgga ggatagaaga caacgaaagt tacttgggct tcctgaggta aaagttttaa 13140
atatatgcca caaaatggat tgttagactg accttttggt attgatacta tagcaaatta 13200
aacttactga atagttattt tagtaaaaaa tttgatatga tagagttgtg cagcagttaa 13260
atttgcttta tctttaaaac atataaaata tttctgtatt attcaatatt ttttaaagac 13320
ttaagaatta cagaaataat tcttagttct gaaaggtttc tgtttgattt ttgtgttgag 13380
cattgtttag accggtagac tcatgtaata ctatttggtc cttcaggatt acttgtatgg 13440
acaaactacc acatatctga catataatga cttcatcaac aaggaactta tcttgttctc 13500
aaattctgat aacgagagat ctatcccttc tatggtggat ggtgagttcc aatttgttag 13560
tctgttttca ttgtaagatg gaaatcaaat tccaaaattg gttaaattga ggatacttac 13620
gtttgctctt atttcatttt aaaggtttga aaccaggtca gagaaaggtt ttgtttactt 13680
gcttcaaacg gaatgacaag cgagaagtaa aggttgccca attagctgga tcagtggctg 13740
aaatgtcttc ttatcatcat ggtgaggtaa acacacaatc catgtttcca gaaagcatta 13800
tatcagaaat ccctgcagag tcattctcaa aacagatttg gtgaaagtat aagctcatat 13860
attcatagta aagaacaagt tggtaaaacc tgtgaaagaa taaaactgct tgccctttga 13920
tttctgaaag taatattcct gcagggatat tcataagtct gcaaaggtct acattaaaaa 13980
atgcttagta tgtttattat aactttgtga tgtcaaaatt tagacctcca ctgatttgaa 14040
aattgttata ataaattgtg acacatgtat gcatggaatc agtgctgtaa cttcagtaaa 14100
actgtgtgca ctgacactga ggaatatcca gggtctattc tcaggtgagg aaaaaaagag 14160

acagaacaat aactaaaaat gtctacaagg atatgttcca aattgttcac tttagttacc 14220
cctgagaact ggaaagggca actttcactt tttatatgtg tctgaattgt ttgactttac 14280
accaatgttt tgtgattact ttttgcatta attatgtaaa acagttttaa agaactagat 14340
tgcctacagg tttctagaat tttaaatata atggttgtct tgttgctttc aatcaaaata 14400
tagtagggat acaataaatt ttcacccaac gaacatttgt tgaatgccta ccatgtacca 14460
ggtatacaaa aataaataaa aatatgttcc ttatccccaa gatataataa caatttagcc 14520
agagggccaa tacacacaca gctatagaat aaagtgatac atgctagaat agaaatgaac 14580
acagcattgt aacacaaagg aggaaatatt tcattttgct gaggataagg agaagaacta 14640
tgccttcaaa gttgagtggg atttaataga aattgttgga cggcagccct aaacttggag 14700
ccagaagttg taggttcaag tactatttct gccatctatt aatctgagga aagtcactaa 14760
acaactagac ctcacaaaat gttacaggat tatagtgagg ttaagtcata atgtatttgt 14820
attagtaaaa gcctcagctt aatgaatctt tttttcccca cagatgtcac taatgatgac 14880
cattatcaat ttggctcaga attttgtggg tagcaataat ctaaacctct tgcagcccat 14940
tggtcagttt ggtaccaggc tacatggtgg caaggattct gctagtccac gatacatctt 15000
tacaatgctc aggtaggtat gctttcactt tctagtgact tgccatttcc agttttgaaa 15060
caatgtgaga tttagttcac caaaaataca aaaactcagt taatcagaag atactggcat 15120
ctagtggcag aggccagggg tgaggcttat acaggaaagc ccccccataa caattatcca 15180
gtgcaacatg tcaattatgc taaggttgag aaatcctgat gtagagcttg ggactgagta 15240
aaggagactt tgattcttcc taaatatcac aattgaaatt ttactccttt acaagcctat 15300
ggaaagtata ttatgaatgg tcaattttca gctgtcatcc cagtcaccct ttcagtggca 15360
ttcaaacagt tactaattgc tccatccttc ttgaaactct ttatttggcc tctatgactg 15420
tagactcttc tggttctctt ctaccttatt agctttctct gctgggtcct tctcaccttc 15480
ccaacctcta aatactggcc cattccaggg ctccttcatc agatctcttt ccaatgccta 15540
cactcaattc ccaaatgatc tcccattcat ccgttctata cctttaaatc tccagtcttg 15600
gtctctcccc tgaactccca acttttatgt ctgactgccc tcttaacatt tcccacttgg 15660
atatctagtt aagtatctca aacataagtc taaaaccaaa tccttagttt tcctgtctca 15720
gtaaatggaa tgccatgcca tccttctaat tgctcaggct aaaaaccttg tcacccttga 15780
ctcctttgct tgatacttaa catccagttc ctcagccaat cctataggat ctgcattgga 15840
aaaatactaa gccacccccca tctcatctaa actattgtaa catatcatct tttcttaaag 15900
cagccaatta ttttttaaaa aataagtcac tctgattcag aaccttcaac tgctcagagt 15960
aaaatctaaa gacctatttt agctaaccag acattatgtg atccgtgtcc ccaccttcca 16020
tgtcttttga ttccttctca taccatctcc tctccagcca cactgtcctt ggcctatact 16080

caaatatacc gagcacattt cctcttcagg gccttttact tactggttcc tttgcctgca 16140
acactctttc tctagatatc cacatggttc agtccctcag tttgtttttt gttgtttttt 16200
tttcttttga gatggagttt tgctcttttt gcccaggctg gagtgcagtg gcgcaatttc 16260
ggctcattgc aacctctgcc tcctgggttc aagtgattct cctgcctcag cctcccgtgt 16320
tgctgggatt acaggtacac gccaccatgc tcagctaatt ttatattttt agtagagatg 16380
aggtttcacc atgttggcca ggttggtctt gaactcctga cctcaggtga tctgcctccc 16440
tcagcctccc aaagtgctgg gattacaggc gcgagttact gcacctggcg ccctcagtgt 16500
cttttctctg ctcaaatgtt ttcctatcag aaacggctta actttcctta gcacctcttc 16560
agtcactttc ccactttttc ttctcttgtt tatcccacca cctaacaaaa tatgtgttaa 16620
ttgcaaactt ctactggact gtgagctcct tgaggattga gactttatgt ccatgtgcgt 16680
gacacatagc aagtgctcaa taaatattgt taaatgtata ttagtcagca agtaaaacca 16740
agataatttt catgtataat acatgtcctt tccataaaag ttaagtgtga aattcagtat 16800
ttttgtaaaa taacattctt tgtttctttt ctttagctct ttggctcgat tgttatttcc 16860
accaaaagat gatcacacgt tgaagttttt atatgatgac aaccagcgtg ttgagcctga 16920
atggtacatt cctattattc ccatggtgct gataaatggt gctgaaggaa tcggtactgg 16980
gtggtcctgc aaaatcccca actttgatgt gcgtgaaatt gtaaataaca tcaggcgttt 17040
gatggatgga gaagaacctt tgccaatggt aagtattctg tgtgtgttaa gagccttaac 17100
ttttccttgg gtttcagttg aatagattgt atgattaaag atgatgcaaa cataaaattt 17160
aatttcatac tttagattta ttgttaaacg ctgttgtgat ttccgtctag tatcacattt 17220
agaaaacata ctgttttttt aaatgccaat ttagcttcca agttacaaga acttcaaggg 17280
tactattgaa gaactggctc caaatcaata tgtgattagt ggtgaagtag ctattcttaa 17340
ttctacaacc attgaaatct cagagcttcc cgtcagaaca tggacccagg taaataatta 17400
tggatttctt ttttaggttt gtgatcaaaa gaaatacctt ttgtactatt gctgttgact 17460
attcagaagc ctatatttta gaaacttcag gattatttct ttctttagac atacaaagaa 17520
caagttctag aacccatgtt gaatggcacc gagaagacac ctcctctcat aacagactat 17580
agggaatacc atacagatac cactgtgaaa tttgttgtga agatgactga agaaaaactg 17640
gcagaggcag agagagttgg actacacaaa gtcttcaaac tccaaactag tctcacatgc 17700
aactctatgg tatgtatttg ttttgtgaga tgtacacgtt tcaatttata ttaaagatta 17760
atatcaattt taaaagtata tcttagtcta cctttataaa gtttagccaa tttgtctatt 17820
cttggctcta atcttgtttt ccttttttcag gtgctttttg accacgtagg ctgtttaaag 17880
aaatatgaca cggtgttgga tattctaaga gacttttttg aactcagact taaatattat 17940

ggattaagaa aagaatggct cctaggaatg cttggtgctg aatctgctaa actgaataat 18000
caggctcgct ttatcttaga gaaaatagat ggcaaaataa tcattggtat gttttgggaa 18060
taataactgc ttactaaaac tctagttacg ccaaactttc tttaaacatt tttggtgaga 18120
gtaatggtgg agaaagaggc aaaatccatt taattcacct ggtcacttat acacgcattt 18180
ctttaaaata tctgattagg tatttatagt ttgaaagaga tgatgtttcc ttgactgagc 18240
atcttgagaa atcaagattt agttgacaat tagacatgag gagaatagag agctagaaga 18300
ccttgcataa actgattgac caagagaata gatacactaa tcatgtctac aggaacagaa 18360
aataaaagag acagagaaga gataataaat ctgatggtaa aaaaaaaaaa aggcaggaag 18420
attacgaatg gcttctactc tctgggtgtg gtggcgcatg cctgtaatct cagcacttga 18480
gctggggagg tcaaggctgc agtgagccta ggtagtgcca ctgcactcca gcctggacac 18540
aagagtgaga gagaccctgt ctccaaaaaa aaatgatttg atcatatatg atttgactgc 18600
ccccttgtgg taatttacat ttgtcaatgg tttagggaga cttgcctgta taccgggata 18660
tacaaattta tgcaagcacg aagacagttt aatttccagt tttaaacttt tgacttgtgt 18720
aaaaccttat ccccttccta aaatattact caaaatcatt atctctgttt actttttttaa 18780
aaatagaaaa taagcctaag aaagaattaa ttaaagttct gattcagagg ggatatgatt 18840
cggatcctgt gaaggcctgg aaagaagccc agcaaaaggt aatcttgggt ggcacttttc 18900
tatattgtta aagactatgt attgtacaat gtttatgttt cacattttct aataaagcta 18960
gattttaatt aatcctttag gttccagatg aagaagaaaa tgaagagagt gacaacgaaa 19020
aggaaactga aaagagtgac tccgtaacag attctggacc aaccttcaac tatcttcttg 19080
atatgcccct ttggtattta accaaggaaa agaaagatga actctgcagg ctaagaaatg 19140
aaaaagtgag ttgatagtag gatggtacat gctgcttagt tttgttctat tctaatggta 19200
aaagtaaaat tgtgtgttgc atgaaaatgc tgcttgtata catatattaa ctcaattttg 19260
taattatctt atgaaggaac aagagctgga cacattaaaa agaaagagtc catcagattt 19320
gtggaaagaa gacttggcta catttattga agaattggag gtatgtagtt tataatgccc 19380
atgttagaat ttttattaat gaaataatat attccagcag tatacctttg tcaagatagt 19440
tcacaattgg caataaaaag aaaataagag gcataaggat aaattccaaa tagcgtataa 19500
aagaacagat tattggccag gtgcagtggc tcacgctggt aatcccagca ttttggaagg 19560
ctgagttggg tggatcactt gaggtcaggg gttcaagacc agcctggcca acatggtaaa 19620
accccatttc tactaaaaac acaaaaatta gccaggcatg atggtgcata ccagttactc 19680
aggaggctga ggcaggagaa ttgcttgaac cgagaggcag aggttgcagt gaaccgagat 19740
cacaccactg cactccagcc taggcaacac agcaagactc cgtctcaaaa aaaaaaaaaa 19800
aaaaaaaggg acagattatt aaatactact tagaatacaa ggccgggtgc ggtggctcac 19860

acctacaatc ccagcacttt gggaggccga agcaggcaga tcacctgagg ttgggagttc 19920

gagaccagcc tgaccaacat ggagaaaccc tgtctctact aaaattgcaa aattagccag 19980

gcgtagtggc acatgcctgt aatcccagct acttgggagg ctgaggcagg agaatcactt 20040

gaactcggga ggcagatgtt gctgtgagct aagatcacac cattgcactc cagcctgggc 20100

aacaagagtg gaactccgtc tcaaaaaaaa aaaaaaaaat actatttaga atacagtaaa 20160

tgataccagg agactgccca gacattcaga catttctgga caaaaaaaga aaagagcagg 20220

agttgatttt tgataaaggg aagaacatat taggactgag aagataaaaa gagcctaaat 20280

gtggaagaag accaccaacc cagtccccca agtcaactta aaaggacaaa gccacagggc 20340

atggtggctc atgcctgtaa tcccagcact ctgggaggct gaggtgggca gatcacttga 20400

ggtcaggagt tcgagaccag cctggacaac atggtgaaac cctgtctcta ctaaaaatac 20460

aaaaattagt tgggtgtggt ggcatccgcc tgtaatccca gctactcagg aggttgaggc 20520

aagaaaattg cttgaaccca gaaggcagaa gttgcagtga gccgagattg cgccactgta 20580

ctccagcctg ggtgacagag caacactcca tctaaaaaaa aaaaaaaaaa aaggagaaag 20640

cagggctatc cagatggttt cagaaggtta tttgttactt aaatcttcct aataacattt 20700

gtttctcaac attattttaa cataagaaaa aaggccagca ctgtggctca cacctgtaat 20760

cccaatgtgc tttgggaggc tgaggcagga ggatcacttg agattaggag ttcaaaacca 20820

gcttgggtaa cgtagcaaga cctcatctct acaaaatgtt ttttttaaat agccaggcat 20880

ggtggtgtgg gcctatagtc tcagctactc cggaggccaa ggtgggagga tcacttgaga 20940

ccaggagttc aaggctgcag tgaactatga tcatgtacca ttgtactcag cctgcgtgtc 21000

tcttaaaaaa aaaaaaacaa aaaaaaaacc accaaaagct ttatttctta aatttaacat 21060

gcaaatttgt attactgcac ttcagtatag atgcctagct cattgtaatc agttggttgg 21120

ataagtgtgc atcacttcct ttcatatttt gtatgtgagt ttacaaatat atatgtatat 21180

acatgtttta tttttagttt ctcccttgtt ttcctcaaaa aggttaagtg gtagaatcag 21240

gaataaaacc cagtccttct gtctccacac cttccttttc aaggtgtgtt ttaattacat 21300

cttcactatt gttgatagta tactaggtta tggagcaatt tatcactagt ttttaagatg 21360

taacaaaatg aaatcagttt aactgttggg tgtttagata ttgtaatgtt tttgttaaca 21420

tttaaacttg ctttcttata tcccttctgt aggctgttga agccaaggaa aaacaagatg 21480

aacaagtcgg acttcctggg aaagggggga aggccaaggg gaaaaaaaca caaatggctg 21540

aagttttgcc ttctccgcgt ggtcaaagag tcattccacg aataaccata gaaatgaaag 21600

cagaggcaga aaagaaaaat aaaaagaaaa ttaaggtaat actcttgtgg tggctcacac 21660

ctataatccc agcactttgg gggccaaggt gggtggatca cttgaggtca gtggttcaag 21720

accagcctcc caacatggtt gaaaccctgt ctctactaaa aattaaaaaa attaggcggg 21780
catggtggca ggcgcctgta atcccagcta ctcgggagac tgaggcagaa gaatcgcttg 21840
aacccgggag gcagaggttg cagtgagcca aggtcacacc actgcactcc agcctggcca 21900
acagagcaaa actccatctc aaaaaaaaaa aagaggtaat actcttaaaa atattatgca 21960
taaaatattg atgcatatgt ccacttaaaa ttgaaaaaaa aatgtataaa attttttttgt 22020
tgttgttgtt gagatggagt ctcgctgtgt tacccaggct ggagtgcaat ggcaggatct 22080
cagctcacag caacctccgc ctcccgtatt caagcgattc tcgtgcctca gcctcccaag 22140
tagttggggg cacatcacca cacccagcta atttttgtat ttttagtaga gatggggttt 22200
caccatcttg gccaggctgg tctcgaactc ctgacctcaa gagatctgcc caccacggcc 22260
tcccaaagtg ctgggattac aggcgtgagc caccgctccc agccaaaaat gtataaaatc 22320
tctaatccca tagttcagaa tttattctat atccaaccct aactttgcta tcttttagaa 22380
tgaaaatact gaaggaagcc ctcaagaaga tggtgtggaa ctagaaggcc taaaacaaag 22440
attagaaaag aaacagaaaa gagaaccagg tattacaaca tttttagaaa aatggtataa 22500
agtgaagatt aaattgtgtt tgttacctag tacattccac atagatgttc aaaacctttt 22560
tgttgaattg agaaacgtga aagaggactg tatctcaatt ctacttggac ctcttttttt 22620
tttttttttt ttttttgaga cagtttcact cttgttgccc aggctggagt gcaatagcac 22680
aatctcggct cactgcaatc ttcacctcct gggttcaagc gattctcctg cctcagcttc 22740
ctgagtagct gggattatag gtgcatgcca ccacgcctgg ctgctttttg tatttttagt 22800
agagacgggg tttcaccatg ttagtcaggc tggtctcaaa ctcctgacct tgtgatttgc 22860
ccgccttggc ctcccaaagt gctgggatta caggcgtgag ccactgcccc tggcctggac 22920
ctctttaata tcgtccttgg gatttgattc attatagaaa atattttgta atgaagtagt 22980
ttgttaattt gaattcctta attgggatct aagaaaatac ccagtttatt agatttcatt 23040
ttctagccac ttacgaggtt ctagtataca aagtttctta agaaaagtga ctacatatta 23100
gtaatgtttg tagtatctct agagttcaac atcatgcttc ggatataaat aatgtccact 23160
caatgaacta aatagaatta ataattctgt taatcatttg ataatgcctt tactgtctac 23220
ttttttgaa acatctttat tgagatattc acatgccaaa tttgcccatt taacgtatac 23280
catttggctg ggcgccatgg cttatgcctg taatcccagc actttgggag gctgaggctg 23340
gcggatcact tgaggtcagg agttcaagac caccctggcc aacatggtga aaccccatct 23400
ctactaaaaa tacaaaaatt agttgagtgt ggtggtgcgt gcctgtagtc ccagctactc 23460
gagaggctga ggcacgagtg tcgcttgaac ccaggaggca gaggttgcag agagccaaga 23520
ctgtgccatt gcactccagc ctgggtgaca gagcaagact ccaactcaaa aataaaaata 23580
aagtatgcca ttcagtggtt ttagtataga caaacagttt taatacaatt tattgccccc 23640

caagaaaact gaactcatta acaatcattt gccatttgcc accaactcct ctccactggg 23700
tgaccactaa aaaatctagg ggcatttcct ataaattaaa tcatataata tgtggtcttt 23760
tggagctgac ttctttactt aacacatttt caagggtaac ccatgtggca gcccatatat 23820
tagtacttca ttcttttttt tttttttttt ttgaggcaga gtttcgctct gtcgcccagg 23880
ctggcatgca gtatgcagta gtagtgagat ctcggctcac tgcaacctcc gcctgctagg 23940
ttcaggcaat tctcctgcct cagcctcccg aggagctggg attacaggca cgcaccacca 24000
tgcccaaata atttttgtat ttttagtaga aacagggttt caccatgttg gccaggatgg 24060
tctcaaactc ctgacctcaa gcgatctgcc cacctcggcc tcccattttg ctaagcccgt 24120
gaaggtaaac ctctgtgttt tcttctaagc attttatagt tttagctctt acatttaggt 24180
ctgtgatcca ctttgaatta gtttttgtat atggtgtaag gaaggtgtcc aacctgattc 24240
tttgcaggta gatatccaat tatttcagta tcatatgtta aaaagacttc tttctacaat 24300
gtttgtcttc acccttgtca aaaatcaatt gactataaat gttaaggttt atgtatggag 24360
tctcaattct gttccactga ttgagctata tatgtccttg agtatctact tttttttgaga 24420
cagggtttca ctcttgccca ggctggtctt gaaatcctgg ctcaagcaat ccttctatct 24480
cagcctccca agtagctggg actataggca ctcgccacca tgactagcta atttctaaaa 24540
gtttttttgta ttttgtagag acagtccttt tttcttttttt tttcttttttt tgagacaaga 24600
tttcactctg tctcccaggc tggagtacag tggcatgatc acagcccact gcagcctcga 24660
cctcccaggc tcaaacaatc ctcctgcctc tgcctccgcc tccctagtgg ctggaaccac 24720
aggcgcactc caccatgccc attaatttct gtatttttttg tagagatggg gttttgccat 24780
gttgaccagg ctggtcttta gctcctgagc tcaagcgatc cgcctacctc accctcccaa 24840
agtgctgaga ttacaggtgt gagccaccgc acccagccaa gacagggtct tatatcttac 24900
cccagctgat ctcaaacccc tgggctcaag taatcctccc gccccagcct ccctaagtgc 24960
tgggattaca ggcatgagcc actgtgcccg gccacattta tttatgagat taagtgacca 25020
agctatatgc accacaaaaa cagagaccat atctgtctct tttcacgatt gtattctcag 25080
aactatcaca ggagttaata aatttgaagg atggatggat gatagatgga gaaaagtggg 25140
tctaatatat aaaggtgatt aatttctatc tcccctctag gtacaaagac aaagaaacaa 25200
actacattgg catttaagcc aatcaaaaaa ggaaagaaga gaaatccctg gtctgattca 25260
gaatcagata ggagcagtga cgaaagtaat tttgatgtcc ctccacgaga aacagagcca 25320
cggagagcag caagtaagga aaactaaaga acattataga taaactgtaa gagtggaatg 25380
gccaatatac ttcagggtac tttgccccag aaattactgt gtcttaatag agcagtaaat 25440
tatgttaaag ctttgaaaca ctgaagcatc tttcaggaga tttaaaaact aaaatattca 25500

tgaaatgtta tgtcaaccta tgaattgttt ctcctactac cctctttgat ttttttgtat 25560
atggtttttt tactagcaaa aacaaaattc acaatggatt tggattcaga tgaagatttc 25620
tcagattttg atgaaaaaac tgatgatgaa gattttgtcc catcagatgc tagtccacct 25680
aagaccaaaa cttccccaaa gcaagtatct tatctaatat gggttttgtc atgattgttt 25740
ctaatatatt gtttttgct tgacacattt agaattggtt gtcaggattt ttttttttaa 25800
ttctagactt agtaacaaag aactgaaacc acagaaaagt gtcgtgtcag gtatgtattt 25860
aagtaaaagt agtgagacat ctgccttact gggacactca gatccaactg ggttctaatc 25920
ctggctacat tatccaagta cttcctttgg aaataaattt gagttcttat taacattatg 25980
ttgatactgt tcactgggtt tagtttcaca tgtaaatagg tactgcattc agtatactaa 26040
aagtaatcat agtgcttgcc aggtactttg ctaggtgcta gagatacaaa aaataaaaac 26100
tgttcttgct gataaaaaat tcagaaatta gaacaaagtt ttaagagtat gttttaaata 26160
ttccagaaaa gtcacatatt tggtgtttaa ataggaattc ataccaggga caaagcagaa 26220
aatggatttt catgtgttgc ttaaatgtac tattttcctt tccatttgaa ataatagacc 26280
ttgaagctga tgatgttaag ggcagtgtac cactgtcttc aagccctcct gctacacatt 26340
tcccagatga aactgaaatt acaaacccag ttcctaaaaa gaatgtgaca gtgaagaaga 26400
cagcagcaaa aagtaagcct aaatctttga gatgggttaa tgttgcaatt acctaactgg 26460
tttccacgtg tctatttcaa tttttttatt gccaaaactt actattgata ttacagatta 26520
aatgttttcc aattggaagc aatttctttt cgatctttat aatcaaaatt agtagtcaag 26580
gctgttccaa aacagtaagt tatctctatt gattgttcag ttacagatca aactccttgt 26640
tctactcttt tccctccttc tcactactgc acttgactag tcaaaaaaca aaacaaaagc 26700
caggcagagt ggctcatgcc tgtaatccca gcactttggg aggctaaaac aagaggattg 26760
cttgaggcca ggagttcaag gctgcagtga gctatatgat caagccactg cactccagcc 26820
tgagtgagag agcaagaccc tgtctccaaa aataaggtag tccaaatgtt taaaatcagt 26880
gagtttctct cagtaccagg cttcatctag ttcatttctg tgacaaggat aggatttata 26940
agcaattgca atgtttaacg taaaacgtat tcttgaaatt gaattaagtt taaggctggg 27000
tgcagtggct catgcctgta atcccagcac tttgggaggc caaggtgggt ggatcacctg 27060
aggtcaggag ttcgagacta gcctggccaa catagtgaaa cctcatctct actaaaaaca 27120
caaaattagc caggtgtggt ggcacatgcc tgtagtcagc tactcgggag gctgaaacag 27180
gagaatcact tgaacccagg aggcagaggc tacagtgagc cgagatcgtg ccactgcact 27240
ccagcctggg cgagatagag caagatgctg tctcaaaaaa aaaaaaaaaa aaatagaatg 27300
tttaagaata ctttgattct ctgttttcac ctctcttaga ttgtcttttc ctatgttaaa 27360
tatacagtca tcacattgct gaagaaagtt cgcaatgaga acaattcatc taagagtggc 27420

tgtgactagg tcaggcgcgg ttgctcatgc ctgtaatccc agcactttgg gaggccgagg 27480
cgagtggatc acctgaggtc aggagtttga gaccagcttg accaacatgg tggaatccca 27540
tctctactaa aaatacaaaa aattagccgg gtgtggtggc acacgcctgt aatcccagct 27600
actcaggagg ctgaggcagg agaatcgctt gaacccagga ggcagaggtt gcagtgagcc 27660
gagataacac cactgcactc cagcctggac gatagagtga gaccccatct caaaaaaaga 27720
gcagctgtga caaatgcctg tattgaattg caggtcagtc ttccacctcc actaccggtg 27780
ccaaaaaaag ggctgcccca aaaggaacta aaagggatcc agctttgaat tctggtgtct 27840
ctcaaaagcc tgatcctgcc aaaaccaaga atcgccgcaa aaggaagcca tccacttctg 27900
atgattctga ctctaatttt gagaaaattg tttcgaaagc agtcacaagc aaggtgagtg 27960
ttgatcctag tcagtccttt tgctgtagat gttctgaaac acgtaactaa gccattgctc 28020
ttaaaaattt ggcatatctt taagaaaatt aactctcata ttctgttagc ttttactgta 28080
catatttagt tttaacaaag ttaaatatgc cacttatttg ggcaatggaa gagttggcct 28140
tagatctgct tcttattact tggtagaaaa tagaaaactc cttgaatata gtgtcttgat 28200
acattttttt acattacaat tatgttgtca gatttacaat gtgcaagtta cctgggcttt 28260
tctcttttag aaatccaagg gggagagtga tgacttccat atggactttg actcagctgt 28320
ggctcctcgg gcaaaatctg tacgggcaaa gaaacctata aagtacctgg aagagtcaga 28380
tgaagatgat ctgttttaaa atgtgaggcg attattttaa gtaattatct taccaagccc 28440
aagactggtt ttaaagttac ctgaagctct taacttcctc ccctctgaat ttagtttggg 28500
gaaggtgttt ttagtacaag acatcaaagt gaagtaaagc ccaagtgttc tttagctttt 28560
tataatactg tctaaatagt gaccatctca tgggcattgt tttcttctct gctttgtctg 28620
tgttttgagt ctgctttctt ttgtctttaa aacctgattt ttaagttctt ctgaactgta 28680
gaaatagcta tctgatcact tcagcgtaaa gcagtgtgtt tattaaccat ccactaagct 28740
aaaactagag cagtttgatt taaaagtgtc actcttcctc cttttctact ttcagtagat 28800
atgagataga gcataattat ctgttttatc ttagttttat acataattta ccatcagata 28860
gaactttatg gttctagtac agatactcta ctacactcag cctcttatgt gccaagtttt 28920
tctttaagca atgagaaatt gctcatgttc ttcatcttct caaatcatca gaggccgaag 28980
aaaaacactt tggctgtgtc tataacttga cacagtcaat agaatgaaga aaattagagt 29040
agttatgtga ttatttcagc tcttgacctg tcccctctgg ctgcctctga gtctgaatct 29100
cccaaagaga gaaaccaatt tctaagagga ctggattgca gaagactcgg ggacaacatt 29160
tgatccaaga tcttaaatgt tatattgata accatgctca gcaatgagct attagattca 29220
ttttgggaaa tctccataat ttcaatttgt aaactttgtt aagacctgtc tacattgtta 29280

tatgtgtgtg acttgagtaa tgttatcaac gtttttgtaa atatttacta tgtttttcta 29340

ttagctaaat tccaacaatt ttgtacttta ataaa 29375

<210> 107
<211> 25
<212> DNA
<213> Artificial

<220>
<223> Synthesis

<220>
<221> misc_signal
<222> (1)..(25)

<400> 107
gattcattga agacgcttcg ttatg 25

<210> 108
<211> 22
<212> DNA
<213> Artificial

<220>
<223> Synthesis

<220>
<221> misc_signal
<222> (1)..(22)

<400> 108
gaagagaggg ccagttgtga tg 22

<210> 109
<211> 1536
<212> DNA
<213> homo sapiens

<400> 109
gggggggggg ggaccacttg gcctgcctcc gtcccgccgc gccacttggc ctgcctccgt 60

cccgccgcgc cacttcgcct gcctccgtcc cccgcccgcc gcgccatgcc tgtggccggc 120

tcggagctgc cgcgccggcc cttgcccccc gccgcacagg agcgggacgc cgagccgcgt 180

ccgccgcacg gggagctgca gtacctgggg cagatccaac acatcctccg ctgcggcgtc 240

aggaaggacg accgcacggg caccggcacc ctgtcggtat tcggcatgca ggcgcgctac 300

agcctgagag atgaattccc tctgctgaca accaaacgtg tgttctggaa gggtgttttg 360

gaggagttgc tgtggtttat caagggatcc acaaatgcta aagagctgtc ttccaaggga 420

gtgaaaatct gggatgccaa tggatcccga gactttttgg acagcctggg attctccacc 480

agagaagaag ggggacttggg cccagtttat ggcttccagt ggaggcattt tggggcagaa 540

tacagagata tggaatcaga ttattcagga cagggagttg accaactgca aagagtgatt 600

gacaccatca aaaccaaccc tgacgacaga agaatcatca tgtgcgcttg gaatccaaga 660

gatcttcctc tgatggcgct gcctccatgc catgccctct gccagttcta tgtggtgaac 720

agtgagctgt cctgccagct gtaccagaga tcgggagaca tgggcctcgg tgtgcctttc 780

aacatcgcca gctacgccct gctcacgtac atgattgcgc acatcacggg cctgaagcca 840

ggtgacttta tacacacttt gggagatgca catatttacc tgaatcacat cgagccactg 900

aaaattcagc ttcagcgaga acccagacct ttcccaaagc tcaggattct tcgaaaagtt 960

gagaaaattg atgacttcaa agctgaagac tttcagattg aagggtacaa tccgcatcca 1020

actattaaaa tggaaatggc tgtttagggt gctttcaaag gagcttgaag gatattgtca 1080

gtctttaggg gttgggctgg atgccgaggt aaaagttctt tttgctctaa aagaaaaagg 1140

aactaggtca aaaatctgtc cgtgacctat cagttattaa tttttaagga tgttgccact 1200

ggcaaatgta actgtgccag ttctttccat aataaaaggc tttgagttaa ctcactgagg 1260

gtatctgaca atgctgaggt tatgaacaaa gtgaggagaa tgaaatgtat gtgctcttag 1320

caaaaacatg tatgtgcatt tcaatcccac gtacttataa agaaggttgg tgaatttcac 1380

aagctatttt tggaatattt ttagaatatt ttaagaattt cacaagctat tccctcaaat 1440

ctgagggagc tgagtaacac catcgatcat gatgtagagt gtggttatga actttatagt 1500

tgttttatat gttgctataa taaagaagtg ttctgc 1536

<210> 110
<211> 15842
<212> DNA
<213> homo sapiens

<400> 110
ccacttggcc tgcctccgtc ccgccgcgcc acttggcctg cctccgtccc gccgcgccac 60

ttcgcctgcc tccgtccccc gcccgccgcg ccatgcctgt ggccggctcg gagctgccgc 120

gccggccctt gccccccgcc gcacaggagc gggacgccga gccgcgtccg ccgcacgggg 180

agctgcagta cctggggcag atccaacaca tcctccgctg cggcgtcagg aaggacgacc 240

gcacgggcac cggcaccctg tcggtattcg gcatgcaggc gcgctacagc ctgagaggtg 300

acgccgcggg cccctgcggg acgggtggcg ggaaggaggg aggcgcggct ggggagagcg 360

ctcgggagct gccgggcgct gcggaccccg tttagtccta acctcaatcc tgcgagggag 420

gggacgcatc gtcctcctcg ccttacagac gccgaaacgg agggtcccat tagggacgtg 480

actggcgcgg gcaacacaca cagcagcgac agccgggagg taagccgcgt cccagcggct 540

ccgcggccgg gctcgcagtc gccccagtga tgccgtggcc cccgaggcgg gcgtcatcgg 600

gcagcgtttg cccagtgctg gagggttagg gagagctgcc tgggcttgac cgcgcgccgg 660

tctcaaagtc ctggctttgg cccctcctcc gttttcccct gtggaccatt ccgcttcgca 720
gcgttttcaa aaactggagc gaaagtgatg tgggcggggc aaaggcggcg ggaagaggag 780
agcactgaag ctggcgcggg aacttggttt cctggtggcc tcccatccaa tccccacgaa 840
ccagctttcc tcttaaacct tgaaaagaga aattcgggag ttcgagtata agttcttagt 900
cgtcctttcc tctttccttt ccgacaggag caccccaggc aaaaaatgtc tcgcgggtca 960
ttggcgccag gctttcaggg gacagtgggg cggggcgggg tgggcacagg acgttaggca 1020
gccgttggcc ctccctaagg ccacaccgtc ctgccgtcct ggatcctgcg ccagctgcgc 1080
gggggagggg actcgaaggt gtgtgagcca ggggctgacc ttgaccgctc agataaatgg 1140
agcgcagcct tgacacaggg gtggaggtgg ttttgaatgg ggaaacccat tcgtggtgaa 1200
gcagattcac tgtagctagc ggaaaagccc tccggcccac ggacccatct agagacgaat 1260
acatagcagc tgctgtggct gattggcgtg ggacagcgtg gggagttttg tctgaggaga 1320
gggatccact tttctgcagc tccaagccca ggggcctttg atgagccata gacctcattt 1380
ttaacccacc tttctgctta gacattgagc aagttacttc tcatatagct tccctatatg 1440
ttaaaaatgg agaaaataat gcttagtagg caattctgat aaaagcaggt gcttgcaaaa 1500
atctctctgt tgtctgaata taaactgtac cacaagcgag tgcggatgaa cgaggactgc 1560
atttaaagat aagtttttac actttcattt ctctgtggct cgacacttct gatgcctccc 1620
tttttgttcc tgggacacat gcttggtgtt gtcttcacac ctttgtgaca ggattagcac 1680
tagtgggcag tggatgatag ctcctccctt ttgccacatg ttcatccctg ccctcgccac 1740
catctcactg tgtggaattc ctgtgtccac tcgtcaccgg ggcacagaag tgctgtctca 1800
gcctgaatcg ggccactgat gggacttgca gcctgggagc tccaccgtga tctctggccc 1860
actttgcggg agtctaggct ttctggatgc tccagggcct cacgtcccag ggcagttttc 1920
ttccctgaag aaagttggat ggcatgatct gtcttcccat cttgaaaccg tatggcaaat 1980
tgtttttcag atgaattccc tctgctgaca accaaacgtg tgttctggaa gggtgttttg 2040
gaggagttgc tgtggtttat caaggtaaag aagtcgctgc tattagaagt cagtagtctg 2100
ttctcaacac agcagccagt gagatccttt caaaactcaa agcagccagg tgtggtggct 2160
cacgcctgta atcccaccac tttgggaggc tgagtcagat cacctgaggt taggaatttg 2220
agaccagcct ggccaacatg gcgacacccc agtctctact aataacacaa aaaattagcc 2280
aggtgtgctg gtgcatgtct gtaatcctag ctactcagga ggctgaggca tgagaattgc 2340
tcacgaggcg gaggttgtag tgagctgaga tcgtggcact gtactccagc ctgggcgaca 2400
gagggagaac ccatgtcaaa aacaaaaaaa gacaccacca aaggtcaaag catatcattc 2460
ctcaccctca agcccttagt ggctccattt cactcagtaa gagccacggt ccttatggtg 2520
tccgtttttc agctctgacc ttagctgctg ctctctgcac cagccctgct gttcttgtga 2580

gtttttgagc acaccgggac atccccactc cctggaacct tcttccccca cacttggctt 2640
cttcctttga gtctctactc cactcgggca agccttccta gacctcctga tttaaaactg 2700
tgactctccc ccaacctcct tggtgtttct ccatagacga acatcaccat ctgatgtatg 2760
tcagcctttc ccttcccctg ttagaagggg gacagcaggt agtaaaagtg aaatgtgctg 2820
taagctttat gagggcagag gatttgtttc tcgtgttcac tgttgtatcg ccagggcctc 2880
aaacacagcc tgccacatag taggagtcaa catatattga tcactaaatg tagataccac 2940
ctgtgttccc atgttcatat aaattctaga agagtctctt cagtaacaag gtgaacccct 3000
tccagagggc tgagtaggta cctcaggccg gggccagagt gctgtgaaga caggcagcag 3060
cccagaccaa gcttctctgt gttccgtgtc ctggtctaga accagcgatg ttctttctga 3120
ccagtgcttt ttggaaggtg gctgaggtct gggctcaggt ctgggccata ctagaagctg 3180
ggatcccttc tatagagcac ttggtatggc ttgtatggtc ttggggcaag ccagacccaa 3240
gccctcttat cccattttag aaagggcttc aatttggatc cagccccagg tctgccttag 3300
ctctgtattc ttggggtatt ttgttctgta ttggcctatc ttgactaaca atgaaccttg 3360
gatttgaaac atatcatcag aaacctcaga agacaacatt cttaaactgg ctagagcctg 3420
gtctgaatgg atgaaaagga gagacttttg aagcaatatg taaaagattg agaaatgatt 3480
tgttggaaat ttctcaattg gagaaatttc tttgatttgt tggaaatttc tttgattctt 3540
tctcaatcaa agaaaatcgg gacaaactca acaatagaaa gggaggaagc aagatactca 3600
gaaataaaat gcattcccct gtttcaactt aatgcttcaa ttcaggattc taaggaatcc 3660
ttgccaggaa tgtcagactc accttgatag ttggagttac tccattggtg actcgatcaa 3720
atacaggagt tgaggcacct gcactgtaaa atactgatta gtctgatcat taggaatatc 3780
ctgtatgcca ggtagaagat acattgaaca gattgcatgt aggcattaaa ttcattttgg 3840
ggtattacat atagacaaca catttcatta agaaacataa aactgtcaga tcggtggaat 3900
acttaaaagc acttggaggt gtttagccta aaaagcttag ttgaggggaa tggaagaaaa 3960
gatctgggag ggtggttcca aagaagggat cagactgtcc taaagccctc aggaatctgg 4020
gctgggacca gcctacttaa agataggatg ggcagctggg tgtggtggct cacgcctgta 4080
atcccagcac ttcgggaggc cgaagtgggc ggatcacctg aggtcaggag ttcgaggcca 4140
gcctgaccaa catggagaaa cactgtctct actaaaaata caaaattagc tgggtgtagt 4200
ggcgcatgcc tgtaatccca gctactcggg aggctgaggc aggggaatcg cttgaacctg 4260
ggaggtggag ggtgccgtga gccacgatcg cgccattgca ctccagcctg ggcaacaaga 4320
gcgaaactct caaaaaacaa aaaaaaggat gggttccata tgggtggtgt caagtgccca 4380
cctcctagca agtcagcagg ggccagaggc ccttgtaagt ggtgtctcgg ggggatcaac 4440

tgagatggct taggatttac ctggatgcct gctctgctct ccccatctct tccagggatc 4500
cacaaatgct aaagagctgt cttccaaggg agtgaaaatc tgggatgcca atggatcccg 4560
agactttttg gacagcctgg gattctccac cagagaagaa ggggacttgg gcccagttta 4620
tggcttccag tggaggcatt ttggggcaga atacagagat atggaatcag gtgaggagat 4680
agaacaatgc cttccatttc ccgggtgccc ttcctagcat gtgtttgctc cgttgtttta 4740
gataaggtct gggggatgag tcaatgtcac aggagctgat gtatagcttt gaccttgtga 4800
ggggtggtgc caggttaagc cacaattaag cctcatgaag gccgtttcac actctttttt 4860
tttttttttt ttaattatta tactttaagt tttagggtac atgtgcacaa tgtgcaggtt 4920
agttacatat gtatacatgt gccatgctgg tgcgctgcac ccactaactc atgatctagc 4980
atcaggtata tctcccaatg ctatccctcc cccctcctcc caccccacaa cagtccccag 5040
agtgtgatgt tccccttcct gtgtccatat gttctcgttg ttcaattccc acctatgagt 5100
gagaatatgc agtgtttggt tttttgttct tgcgatagtt tactgagaat gatgatttcc 5160
aatttcatcc acgtccctac aaaggacatg aactcatcat tttttatggc tgcatagtat 5220
tccatggtgt atatgtgcca cattttctta atccagtcta tcattgttgg acatttgggt 5280
tggttccaag tctttgctat tgtgaatagt gccacaataa acatacgtgt gcatgtgtct 5340
ttatagcagc atgatttaat agtcctttgg gtatataccc agtaatggga gggctgggtc 5400
aaatggtatt tctagttcta gatccctgag gaatcgccac actgacttcc acaatggttg 5460
aactagttta cagtcccacc aacagtataa aagtgttcct atttctccac atcctctcca 5520
gcacctgttg tttcctgact ttttaatgat tgccattcta actggtgtga gatggtgtct 5580
cattgtggtt ttgatttgca tttctctgat ggccagtgat ggtgagcatt ttttcatgtg 5640
tttttggct gcataaatgt cttcttttga gaagtgtctg ttcatgtcct tcgcccactt 5700
tttgatgggg ttgtttgttt ttttcttata aatttgtttg agttcattgt agattctgga 5760
tattagctct ttgtcagatg agtaggttgc aaaaattttc tcccattttg tgggttgcct 5820
gttcactctg atggtagttt cttttgctgt gcagaagctc tttagtttaa ttagatccca 5880
tttgtcaatt ttgtcttttg ttgccattgc ttttggtgtt ttagacatga agtccttgcc 5940
catgcctatg tcctgaatgg taatgcctag gttttcttct agggttttta tggttttagg 6000
tctaacgttt aagtctttaa tccatcttga attgattttt gtataaggtg taaggaaggg 6060
atccagtttc agctttttac atatggctag ccagttttcc cagcaccatt tattaaatag 6120
ggaatccttt ccccattgct tgtttttctc aggtttgtca aagatcagat agttgtagat 6180
atgcggtgtt atttctgagg gctctgttct gttccattga tctatatgtc tgttttggta 6240
ccagtaccat actgttttgg ttactgtagc cttgtagtat agtttgaagt caggtagtgt 6300
gatgtctcca gctttgttct ttttggcttag gattgacttg gcgatgtggg ctcttttttg 6360

gttccatatg aactttaaag tagtttttc caattctgtg aagaaagtca ttggtagctt 6420
gatggggatg gcattgaatc tataaattac cttgggcagt atggccattt tcacgatatt 6480
gattcttcct acccatgagc atggaatggt cttccatttc tttgtatcct cttttatttc 6540
attgagcagt ggtttgtagt tctccttgaa gaggtccttc acatcccttt taaggtggat 6600
tcctaggtat tttattctct ttgaagcaat tgtgagtgga agttcactca tgatttggct 6660
ctctgtttgt ctgttattgg tgtataagaa tgcttgtgat ttttgcagat tgattttata 6720
tcctgagact ttgctgaagt tgcttatcag cttaaggaga ttttgggctg agacaatggg 6780
gttttctaga tatacaatca tgtcgtctgc aaacagggac aatttgactt cctcttttcc 6840
taattgaata ccctttattt ccttctcctg cctaattgcc ctggccagaa cttccaacac 6900
tatgttgaat aggagtggtg agagagggca tccctgtctt gtgccagttt tcaaagggaa 6960
tgcttccagt ttttgcccat tcagtatgat attggctgtg ggtttgtcat agatagctct 7020
tattattttg aaatatgtcc catcaatacc taatttattg agagttttta gcatgatgtg 7080
ttgttgaatt ttgtcaaagg ccttttctgc atctattgag ataatcatgt ggtttttgtc 7140
tttggatctg tttatatgct ggattacatt tattgatttg tgtatattga accagccttg 7200
catcccaggg atgaagccca catgatcatg gtggataagc tttttgatgt gctgctggat 7260
tcggtttgcc agtattttat tgaggaattt tgcatcaatg ttcatcaagg atattggtct 7320
aaaattctct tttttggttg tgtctctgcc cagctttggt atcaggatga tgctggcttc 7380
ataaaatgag ttagggagga ttccctcttt ttctattgat tggaatagtt tcagaaggaa 7440
tggtaccagt tcctctttgt acctctggta gaattcggct gtgaatccat ctggtcctgg 7500
actctttttg gttggtaagc tattgattat tgccacaatt tcagctcctg ttattggtct 7560
attcagagat tcaacttctt cctggtttag tcttgggaga gtgtatgtgt caaggaattt 7620
atccatttct tctagatttt ctagtttatt tgcgtagagg tgtttgtagt aatctctgat 7680
ggtagtttgt atttctgtgg gattggtggt gatatcccct ttatcatttt ttattgcgtc 7740
tatttgattc ttctcttttt ctttattagt cttgctagcg gtctataaat tttgttgatc 7800
ctttcaaaaa accagctcct ggattcatta atttttgaa gggtttttg tgtctctatt 7860
tccttcagtt ctgctctgat tttagttatt tcttgccttc tgctagcttt tgaatgtgtt 7920
tgctcttgct tttctagttc ttttaattgt gatgttaggg tgtcaatttt ggatctttcc 7980
tgctttctct tgtgggcatt tagtgctata aatttccctc tacacactgc tttgaatgtg 8040
tcccagagat tctggtatgt tgtgtctctg ttctcgttgg tttcaaagaa catctttatt 8100
tctgccttca tttcgttatg tacccagtag tcattccgga gcaggttgtt cagtttccat 8160
gtagttgagc agttttgagt gagtttctta gttctgagtt ctagtttgat tgcactgtgg 8220

tctgagagat agtttgttat aatttctgtt cttttacatt tgctgaggag agctttactt 8280
ccaactatgt ggtcaatttt ggaataggtg tggtgcggtg ctgaaaaaaa tgtatattct 8340
gttgatttgg ggtggagagt tctgtagatg tctattaggt ccgcttagtg cagagctgag 8400
ttcaattcct gggtatcctt gttaactttc tgtctcgttg atctgtctaa tgttgacagt 8460
ggggtgttaa agtctcccat tattaatgtg tgggagtcta agtctctttg taggtcagat 8520
gattggcact tactgggcgc ttggcacttt ccatactgtg tcatcggcag atagctgcat 8580
ggttggtgtt cgtgctgggg aatgggaagt tcatcggtgg gacaagggac aaaatgcccc 8640
cattgctttg ttgtggcttt aatctccctt tcgaggctga gccacagcgt gctgtaggtg 8700
gcgctgctgt gaagcgcagt gccagggtca cactccactc ccagctctgc agaggtggag 8760
aaagaatgaa acatctcact cctggacttc cactttcctg tcactgttgg tgtcacctct 8820
tactggatgt cacagagccc agcccctccc acctgtccct aggaaaagca gatgccacct 8880
tggaatgtgg ggtttgtgtg tgcaatttac tagctgggca gagaccagca acctggagag 8940
caggtgtctc atctaagggg acagtcacat ttcacctcca gccacctgga ggaatttggg 9000
cctggtgatg tcagaattct tcaataaaag cctaaaatct atattttatg tgcggtcatg 9060
agatctgtta aatgttagca acttcaggaa gtttaaaaat gctgtgtgga cctagaatag 9120
gcaagttctt aaaggcagaa agtggaatgc tagtttccag ggactgggga acagggagga 9180
atggggagtt catgtttaat gggcacagag gttttgttag ggatgacgaa aaagttcggg 9240
agatggtgat ggagatggtg atggtgatgg agatggtgat ggtgatggag atggtgatgg 9300
tgatggagat ggagatggtg atggagatgg agatggtgat ggtgatggag atggtgatgg 9360
tgatggagat ggagatggtg atggagatgg tgatggtgat ggagatggag atggtgatgg 9420
tgatggagat ggtgatggtg atggagatgg agatggagat ggtgatggtg atggtgatgg 9480
agatggtgat ggtgatggtg atggtgatgg agatggtgat ggtgatggtg atggtgatgg 9540
agatggtgat ggtgatggtg atggagatgg tgatggtgat ggagatggag atggtgatgg 9600
tgatggtgat ggagatggtg atggtgatgg gatggtgatg gagatggtga tggtgatggt 9660
gatggagatg gtgatggtga tggagatggt gatggagatg gtgatggtga tggagatggt 9720
gatggtgatg gtgatggtga tggagatggt gatggtgatg gtgatggtga tggtgatgga 9780
gatggtgatg gtgatggtga tggtgatgat ggagatggtg atggtgatgg tgatggtgat 9840
ggtgatggag atggtgatgg tgatggtgat ggtgatggag atggtgatgg tgatggtgat 9900
ggagatggtg atggtgatgg agatggtgat ggtgatggtg atggtgatgg tgatggtgat 9960
ggagatgggt gatggtgatg gttgcctaac atcaggaacg tgcttaatgc ttctgaattg 10020
cacacaaaaa tggcaagttt aatattatgt gtactttatc acaatgaaaa aagctgctgc 10080
gtgggccaag ttacttgtgc aggtaagtgt tctgcaggtc gttgcctgca cctcagttgt 10140

agggtgtccg taggatgtga ggccagtccc cgggcttaat gatgctttaa atcctgccta 10200
gtattcaatt atttcttgtc gcttaaaagg cctaataaaa ttatggtttt agtttacagt 10260
ggtatgaatg cttagctgtt ggattttagt aggaaagttt gtcccttttt gtttttaatt 10320
ttgttttaca gattcacagg aatttttttt tttttttttt taatgcacag aaagtttccc 10380
tggacacact acccagtttc ccccagtgat aatatcttgg gtaacatcct gtatacattc 10440
acattggtgc attcctcaga gttgtcagat tttgctagtt ttacgtgcac ttgtgtatgt 10500
gtgtatttgc aattttagca cgtgtagact cttgtaacca ctacaatcaa gttacagaac 10560
tacactacca aggttcatct ttttaaaatc tttgatgtta cctttttcgg aacagtgacc 10620
atgagaggac tttcctccca aaattttgag aactactgaa ccagaatata gtctgacact 10680
aataggtaga aatttaacca aaggagatta tgaagctctg cacttcagtt aacaaatcac 10740
ttctcagctt ccagttccat ctcagaagga aggaaaggga ttaaaatcca gagaccagaa 10800
atgggagcaa agtacaaggt ggtgtaatca ttacagaggt ttcctgatgt ttccaagtca 10860
gtcgtgtgtt gagctgctaa actctaaagt aattttaggt ggaagtgttg gaaacatgct 10920
gctgaggtga tagaaaggaa tccatggtcc tctgttagtt ggaaagtata tggaatacta 10980
tattctacat aagatacaac actctctgtg agacaaggat aaagtagatt ttgtcagtga 11040
aattgtgaca agaatcgctg atgggtttag agcctaagtt tgcgaggagc actggaagaa 11100
attaagattg ttgagattgg aaagggttag ctatgggggg aacaggagga ggtgactcca 11160
tgacagacca aatattcaaa ggactgtgta gaagaggaaa aagactttgt tagggctcca 11220
gaggacagag ccaggagtca gacagggcct tgaactcaac ccaccgagat ctgcaaactt 11280
tgcaggatgc accagatgtc ttgtagccat gggtcaaggg gggaccctgg gtaagagact 11340
gtaatagatg acctctaagg ccatctcatg acatgtgtga ttaatgtatg tacctgtcct 11400
ctctttttga caattctaca gattattcag gacagggagt tgaccaactg caaagagtga 11460
ttgacaccat caaaaccaac cctgacgaca gaagaatcat catgtgcgct tggaatccaa 11520
gaggttgaaa gaaccccgtc gtcttcattt atactaacca tactcttaga gggaagcaat 11580
ctggttttgt gcagaggcac ctgagggagg caggaccctg ggaacttccc ccagccacat 11640
ggttgattgt gtgacgttgg gcaagtcaca ttttgctgca ctttcacctt cagatcatga 11700
ggttgggccc agaggatttt tttttttttt tttttttttt gagacagagt tttgctctgt 11760
tgcccaggct ggaatgcaac ggcgtgatct tggctcactg taacctctgc ctcctgggtt 11820
cgagtgattc tcctgcctca gcctcccaag tagctgggat tacaggcatg tgccaccatg 11880
cccggctaat tttgtatttt tagtagagac ggggtttcac catgttggtc aggctggtct 11940
tgaactcctg acctcagatg atctgcctgc ctcagcctcc caaccagatg atcttaattt 12000

gtgtatttat actcattctt acaccaaaaa gggttttaaa ttgcctagaa actacatgta 12060

agatgttaac attttaaatg gaagcagatg aagttccagc tcgctgccac ctcactaaca 12120

tttttaacaa ttatattgta aaattcaact ctaccagggt gtagagccag gtgtggtggc 12180

tcacacctgt aattccaaca actccagagg ccaaggcgag aggatcattt gaacccagga 12240

atttgaggct gtagtgagtc atgatcacgc cattgcactc cagcctgggc aacagagtga 12300

gaccctgaat atttaaaaac aacaacaaca acaaaactct atcaggatat cataagtact 12360

tagagtgaaa tacttgcatc tgtaatagag acttattttt tttttttttt tgagacacag 12420

tctcaccctg ttgcccaggc tggagtgcag tggtgtgatc tccgctcacg gcaacctcca 12480

tctcccaggt tcaagtgagt tcccattcct cagccccaga gctgggacca caggcgcgcg 12540

aatttttgta tttttagcag agacggggtt tcactatgtt ggccaggcta gtctcgaact 12600

caagttggcc tcaagtgatc tgcccaccct ggcgtcccag tgttgggatt tcaggcatga 12660

gccactgtgc ctggccatgt aatagagact tttaatatag gagggtgtac cagaagcacc 12720

agtttcctgt ggcaaacaga attattcctg ctgtatttgt aatctggtgc cacgaggtag 12780

cccagatccc ttcagctctg atggaagagc attgcttcag ccgtaaatgg acacctgcag 12840

aaaccttgca ccgatggata gtctccctca gctccgtgcc atcgctgcag aggctgttat 12900

ggacatcact gcagcccagt ggctctctct cctggtctcc accatatgag ttggcttctg 12960

tttctctcct gttttacttt gcctttagct gtggtctttc aaaccaccat ccctccttat 13020

cttcctctgc tggttcctca gatcttcctc tgatggcgct gcctccatgc catgccctct 13080

gccagttcta tgtggtgaac agtgagctgt cctgccagct gtaccagaga tcgggagaca 13140

tgggcctcgg tgtgcctttc aacatcgcca gctacgccct gctcacgtac atgattgcgc 13200

acatcacggg cctgaaggtg ggctgtctcg ggaagggtga cttgccagcc taccacactg 13260

agctcttcag ttctttaata tgggaaaaca aattgcagag tttagtctct gattagcttt 13320

taaatttgat atgtgtaagt aagaaatgaa ccagctttta ctttgaaacc ttcctcttct 13380

ggaaggtttt ctggccctgt ggtatacgca ctaacagatc tatacaggtt gtttgtgata 13440

cagcttctat ggattttctc aaaagctatg ctgaggttgg gtatggtggc tcatgcctgt 13500

aatcccagca ctttgggaga ctgagacagg agcaattgct tgaggtctgg agttcaatac 13560

cagcctgggc aacataacaa gatgctgttg ctacaaaaaa atggaaaagc tacactaaat 13620

tatttttttta aaaaaagcct tgcggtgtct gcatattcta atgtttttaa atgatgtttt 13680

aaagaattga aactaacata ctgttctgct ttctcccccg ggtttatagc caggtgactt 13740

tatacacact ttgggagatg cacatattta cctgaatcac atcgagccac tgaaaattca 13800

ggtaagaatt agatgttata cttttgggtt tggtaccttc tcttgataaa aggttgactg 13860

tggaacaggc atctgctcaa tgctgtgtcc aagataaaga tgactgctcc aaatgtgggg 13920

cttcagttta gggagaagtg gtgggcaggt gggcaggaca aggcaggcat ctgcctcagc 13980
aaccatgggc acttaacatg tcaggtgctg tgaggtacta agcaccagta ccagagaggg 14040
aagagccaca ttcaagccag gggattgtcc aaaagggggc attttaactc attttaactt 14100
gaaggagaat tgaagtgcaa atgtttttcc ttttcttttt tttttgagat ggagtctttc 14160
tctgtcggcc aggctggagt gtgccgtggt gcgatctcag ctcactgcaa cctccacctc 14220
ccgggttcaa gcaattcttc tgcctcagcc tcccaggtag ctgggattac aggcacatgc 14280
caccacaccc agctaatttt tgtattatta gtagagatgg ggtttcatca tgttggccag 14340
gctagtctca aactcctgac ttcaagtgat ccacctgcct cagcctccga aaattctgga 14400
attacaggca taagccacca ccctggccat aaatattttt tgttaatttt acattaagta 14460
caatatttag gtccaaactt caaaagtctg ttgaaatccc taagttatag cagccaacaa 14520
ttgatatgaa atggcaataa aaatgtaagt tcatctgctt catgagcctt aaggaaaaaa 14580
actcagaacc agacactttt tagccccttc caggttagat ccaggtttta aaagttactc 14640
ctttgaggga gtttggctgc ttttgagtgg aggtgacttc aggcttattc tctctggctc 14700
tctgctctgg tcgtttttag acatagtaat aggttgtgac ctgtcttcac atcctaattg 14760
ccactgtctg ttcatcccag gaatcctggc tttcatccct ttctgttcac tgtccatgca 14820
tgtcatcttt ccttctttct gccagggaca gatgggttag ggattgtgga attcaagtaa 14880
acgtagagct actatgagtt acagattgac tgtgttcctg tctttaataa atttgccaag 14940
agtggttata agaacttaca cctgatgagg caccaggctc ctgatgctgt gtaatgtcac 15000
aaaatacccc tcactctcga tctgtgcaag agaacagctg gttgcgctcc aatcatgtta 15060
cataacctac ggcaaggtat cgacaggatc atactcctgt aaaatagaac tttgttgatc 15120
acatcctgtg tacttgtttc acggacatga ggagcaatta caacaggtcg tacaattatg 15180
gcaaaataat ggccttattt tgtttttagc ttcagcgaga acccagacct ttcccaaagc 15240
tcaggattct tcgaaaagtt gagaaaattg atgacttcaa agctgaagac tttcagattg 15300
aagggtacaa tccgcatcca actattaaaa tggaaatggc tgtttagggt gctttcaaag 15360
gagctcgaag gatattgtca gtctttaggg gttgggctgg atgccgaggt aaaagttctt 15420
tttgctctaa aagaaaaagg aactaggtca aaaatctgtc cgtgacctat cagttattaa 15480
tttttaagga tgttgccact ggcaaatgta actgtgccag ttctttccat aataaaaggc 15540
tttgagttaa ctcactgagg gtatctgaca atgctgaggt tatgaacaaa gtgaggagaa 15600
tgaaatgtat gtgctcttag caaaaacatg tatgtgcatt tcaatcccac gtacttataa 15660
agaaggttgg tgaatttcac aagctatttt tggaatattt ttagaatatt ttaagaattt 15720
cacaagctat tccctcaaat ctgagggagc tgagtaacac catcgatcat gatgtagagt 15780

gtggttatga actttaaagt tatagttgtt ttatatgttg ctataataaa gaagtgttct 15840

gc 15842

<210> 111
<211> 24
<212> DNA
<213> Artificial

<220>
<223> Synthesis

<220>
<221> misc_signal
<222> (1)..(24)

<400> 111
gagtgattga caccatcaaa acca 24

<210> 112
<211> 21
<212> DNA
<213> Artificial

<220>
<223> Synthesis

<220>
<221> misc_signal
<222> (1)..(21)

<400> 112
gcgccatcag aggaagatct c 21

<210> 113
<211> 3614
<212> DNA
<213> homo sapiens

<400> 113
ggcttggggc agccgggtag ctcggaggtc gtggcgctgg gggctagcac cagcgctctg 60

tcgggaggcg cagcggttag gtggaccggt cagcggactc accggccagg gcgctcggtg 120

ctggaatttg atattcattg atccgggttt tatccctctt ctttttcttt aaacattttt 180

ttttaaaact gtattgtttc tcgttttaat ttatttttgc ttgccattcc ccacttgaat 240

cgggccgacg gcttggggag attgctctac ttccccaaat cactgtggat tttggaaacc 300

agcagaaaga ggaaagaggt agcaagagct ccagagagaa gtcgaggaag agagagacgg 360

ggtcagagag agcgcgcggg cgtgcgagca gcgaaagcga caggggcaaa gtgagtgacc 420

tgcttttggg ggtgaccgcc ggagcgcggc gtgagccctc ccccttggga tcccgcagct 480

gaccagtcgc gctgacggac agacagacag acaccgcccc cagccccagc taccacctcc 540

tccccggccg gcggcggaca gtggacgcgg cggcgagccg cgggcagggg ccggagcccg 600
cgcccggagg cggggtggag ggggtcgggg ctcgcggcgt cgcactgaaa cttttcgtcc 660
aacttctggg ctgttctcgc ttcggaggag ccgtggtccg cgcgggggaa gccgagccga 720
gcggagccgc gagaagtgct agctcgggcc gggaggagcc gcagccggag gagggggagg 780
aggaagaaga gaaggaagag gagagggggc cgcagtggcg actcggcgct cggaagccgg 840
gctcatggac gggtgaggcg gcggtgtgcg cagacagtgc tccagccgcg cgcgctcccc 900
aggccctggc ccgggcctcg ggccggggag gaagagtagc tcgccgaggc gccgaggaga 960
gcgggccgcc ccacagcccg agccggagag ggagcgcgag ccgcgccggc cccggtcggg 1020
cctccgaaac catgaacttt ctgctgtctt gggtgcattg gagccttgcc ttgctgctct 1080
acctccacca tgccaagtgg tcccaggctg cacccatggc agaaggagga gggcagaatc 1140
atcacgaagt ggtgaagttc atggatgtct atcagcgcag ctactgccat ccaatcgaga 1200
ccctggtgga catcttccag gagtaccctg atgagatcga gtacatcttc aagccatcct 1260
gtgtgcccct gatgcgatgc gggggctgct gcaatgacga gggcctggag tgtgtgccca 1320
ctgaggagtc caacatcacc atgcagatta tgcggatcaa acctcaccaa ggccagcaca 1380
taggagagat gagcttccta cagcacaaca aatgtgaatg cagaccaaag aaagatagag 1440
caagacaaga aaaaaaatca gttcgaggaa agggaaaggg gcaaaaacga aagcgcaaga 1500
aatcccggta taagtcctgg agcgttccct gtgggccttg ctcagagcgg agaaagcatt 1560
tgtttgtaca agatccgcag acgtgtaaat gttcctgcaa aaacacagac tcgcgttgca 1620
aggcgaggca gcttgagtta aacgaacgta cttgcagatg tgacaagccg aggcggtgag 1680
ccgggcagga ggaaggagcc tccctcaggg tttcgggaac cagatctctc accaggaaag 1740
actgatacag aacgatcgat acagaaacca cgctgccgcc accacaccat caccatcgac 1800
agaacagtcc ttaatccaga aacctgaaat gaaggaagag gagactctgc gcagagcact 1860
ttgggtccgg agggcgagac tccggcggaa gcattcccgg gcgggtgacc cagcacggtc 1920
cctcttggaa ttggattcgc cattttattt ttcttgctgc taaatcaccg agcccggaag 1980
attagagagt tttatttctg ggattcctgt agacacaccc acccacatac atacatttat 2040
atatatatat attatatata tataaaaata aatatctcta ttttatatat ataaaatata 2100
tatattcttt ttttaaatta acagtgctaa tgttattggt gtcttcactg gatgtatttg 2160
actgctgtgg acttgagttg ggaggggaat gttcccactc agatcctgac agggaagagg 2220
aggagatgag agactctggc atgatctttt ttttgtccca cttggtgggg ccagggtcct 2280
ctcccctgcc caggaatgtg caaggccagg gcatgggggc aaatatgacc cagttttggg 2340
aacaccgaca aacccagccc tggcgctgag cctctctacc ccaggtcaga cggacagaaa 2400
gacagatcac aggtacaggg atgaggacac cggctctgac caggagtttg gggagcttca 2460

ggacattgct gtgctttggg gattccctcc acatgctgca cgcgcatctc gcccccaggg 2520

gcactgcctg gaagattcag gagcctgggc ggccttcgct tactctcacc tgcttctgag 2580

ttgcccagga gaccactggc agatgtcccg gcgaagagaa gagacacatt gttggaagaa 2640

gcagcccatg acagctcccc ttcctgggac tcgccctcat cctcttcctg ctccccttcc 2700

tggggtgcag cctaaaagga cctatgtcct cacaccattg aaaccactag ttctgtcccc 2760

ccaggagacc tggttgtgtg tgtgtgagtg gttgaccttc ctccatcccc tggtccttcc 2820

cttcccttcc cgaggcacag agagacaggg caggatccac gtgcccattg tggaggcaga 2880

gaaaagagaa agtgttttat atacggtact tatttaatat ccctttttaa ttagaaatta 2940

aaacagttaa tttaattaaa gagtagggtt ttttttcagt attcttggtt aatatttaat 3000

ttcaactatt tatgagatgt atcttttgct ctctcttgct ctcttatttg taccggtttt 3060

tgtatataaa attcatgttt ccaatctctc tctccctgat cggtgacagt cactagctta 3120

tcttgaacag atatttaatt ttgctaacac tcagctctgc cctccccgat cccctggctc 3180

cccagcacac attcctttga aataaggttt caatatacat ctacatacta tatatatatt 3240

tggcaacttg tatttgtgtg tatatatata tatatatgtt tatgtatata tgtgattctg 3300

ataaaataga cattgctatt ctgtttttta tatgtaaaaa caaaacaaga aaaaatagag 3360

aattctacat actaaatctc tctccttttt taattttaat atttgttatc atttatttat 3420

tggtgctact gtttatccgt aataattgtg gggaaaagat attaacatca cgtctttgtc 3480

tctagtgcag tttttcgaga tattccgtag tacatattta tttttaaaca acgacaaaga 3540

aatacagata tatcttaaaa aaaaaaaagc attttgtatt aaagaattta attctgatct 3600

caaaaaaaaa aaaa 3614

<210> 114
<211> 14391
<212> DNA
<213> homo sapiens

<400> 114
tcgcggaggc ttggggcagc cgggtagctc ggaggtcgtg gcgctggggg ctagcaccag 60

cgctctgtcg ggaggcgcag cggttaggtg gaccggtcag cggactcacc ggccagggcg 120

ctcggtgctg gaatttgata ttcattgatc cgggttttat ccctcttctt ttttcttaaa 180

catttttttt taaaactgta ttgtttctcg ttttaattta tttttgcttg ccattcccca 240

cttgaatcgg gccgacggct tggggagatt gctctacttc cccaaatcac tgtggatttt 300

ggaaaccagc agaaagagga aagaggtagc aagagctcca gagagaagtc gaggaagaga 360

gagacggggt cagagagagc gcgcgggcgt gcgagcagcg aaagcgacag gggcaaagtg 420

agtgacctgc ttttgggggt gaccgccgga gcgcggcgtg agccctcccc cttgggatcc 480

cgcagctgac cagtcgcgct gacggacaga cagacagaca ccgcccccag ccccagctac 540
cacctcctcc ccggccggcg gcggacagtg gacgcggcgg cgagccgcgg gcaggggccg 600
gagcccgcgc ccggaggcgg ggtggagggg gtcggggctc gcggcgtcgc actgaaactt 660
ttcgtccaac ttctgggctg ttctcgcttc ggaggagccg tggtccgcgc gggggaagcc 720
gagccgagcg gagccgcgag aagtgctagc tcgggccggg aggagccgca gccggaggag 780
ggggaggagg aagaagagaa ggaagaggag agggggccgc agtggcgact cggcgctcgg 840
aagccgggct catggacggg tgaggcggcg gtgtgcgcag acagtgctcc agccgcgcgc 900
gctccccagg ccctggcccg ggcctcgggc cggggaggaa gagtagctcg ccgaggcgcc 960
gaggagagcg ggccgcccca cagcccgagc cggagaggga gcgcgagccg cgccggcccc 1020
ggtcgggcct ccgaaaccat gaactttctg ctgtcttggg tgcattggag ccttgccttg 1080
ctgctctacc tccaccatgc caaggtaagc ggtcgtgccc tgctggcgcc gcgggccgct 1140
gcgagcgcct ctcccggctg ggacgtgcgg tgcgagcgcg cgcgtggggg ctccgtgccc 1200
cacgcgggtc catgggcacc aggcgtgcgg cgtcccccctc tgtcgtctta ggtgcagggg 1260
gagggggcgc gcgcgctagg tgggagggta cccggagaga ggctcaccgc ccacgcgggc 1320
cctgcccacc caccggagtc accgcacgta cgatctgggc cgaccagccg agggcgggag 1380
ccggaggagg aggccgaggg ggctgggctt gcgttgccgc tgccggctga agtttgctcc 1440
cggccgctgg tcccggacga actggaagtc tgagcagcgg gggcgggagc cagagaccag 1500
tgggcagggg gtgctcggac cttggaccgc gggagggcag agagcgtgga gggggcaggg 1560
cgcaggaggg agagggggct tgctgtcact gccactcggt ctcttcagcc ctcgccgcga 1620
gtttgggaaa agttttgggg tggattgctg cggggacccc ccctccctgc tgggccacct 1680
gcgccgcgcc aaccccgccc gtccccgctc gcgtcccgct cggtgcccgc cctcccccgc 1740
ccggccgggt gcgcgcggcg cggagccgat tacatcagcc cgggcctggc cggccgcgtg 1800
ttcccggagc ctcggctgcc cgaatgggga gcccagagtg gcgagcggca cccctccccc 1860
cgccagccct ccgcgggaag gtgacctctc gaggtagccc cagcccgggg atccagagaa 1920
ccatccctac cccttcctac tgtctccaga ccctacctct gcccagtgct aggaggaatt 1980
tcctgacgcc ccttctcttc acccatttcc tttttagcct ggagagaagc ccctgtcacc 2040
ccgcttattt tcatttctct ctgcggagaa gatccatcta accccttttct ggccccagag 2100
tccagggaaa ggatgatcac tgtcagaagt cgtggcgcgg gagcccactg ggcgctttgt 2160
cacattccac cgaaagtccc gacttggtga cagtgtgctt cccttccctc gccaacagtt 2220
ccgagtgagc tgtgctttag ctctcgtggg ggtgggtcaa gggaggattt gaagagtcat 2280
tgccccactt taccctttttg gagaaatggc ttgaaatttg ctgtgacacg ggcagcatgg 2340

gaatagtcct tcctgaaccc tggaaaggag ctcctgccag ccttgcacac actttgtcct 2400
ggtgaaaggc agccctggag caggtgtttt tttggaactc caaacctgcc cacccaactt 2460
gcttctgaaa gggactctaa agggtccctt tccgctcctc tctgacgcct tccctcagcc 2520
agaattccct tggagaggag gcaagaggaa agccatggac aggggtcgct gctaacaccg 2580
caagttcctc agaccctggc acaaaggcct tggctacagg cctccaagta gggaggaggg 2640
ggaggagtgg ctgcctggcc acagtgtgac cttcagaggc cccagagaa ggacacctgg 2700
cccctgcctg cctagaaccg cccctcctgt gctccctggc cttggaaggg gtatgaaatt 2760
tccgtcccct ttcctccttg gggcccagga ggagtggagg gtcccgggag aatattgtca 2820
gggggaaggc agggggtgtc atgggaatgg gtgagggggc tgaggtgcag aatccagggg 2880
gtccctgcag gagccgcagt ggtaagctgt ccagctggaa gcctggtaac tgttgttttc 2940
tcttgagagg ggcttcctgt gaccttggct gtctctggga gcagggctgg ggtacctgag 3000
tggggtgcat ttggggtgtg tgggaaggag agggaaagaa agatggacag tgggactctc 3060
ccctagcagg gtctggtgtt ccgtaggcta gagtgcccct ctgctctgcg agtgctgggc 3120
gggaggggag ttggtgagag ctggagaccc ccaggaaggg ctggcagaag cctttccttt 3180
tgggtgctgt caggtccgca tgtcttggcg tgttgacctt cacagcttct ggcgagggga 3240
ggaatgatct gatgcgggtg gggagggtta gaggaggcct caggcctaag gtggtgcagg 3300
gggcccccta ggggctgggc agtgccaagg cataaaagcc ttccctggtc cctggtggca 3360
tttgaaggtg cccaggtgag aggggcttgg cacctcctca ccctgggagg gagaagaaac 3420
cagggaacag gtaggagtgg gagacaggtg aggctttgga aatctattga ggctctggag 3480
agatttgtgt agagaggaaa atgtggttct cccccagggt ctcctcctgg gtttttaccc 3540
tctaagcaac ctgtgggcat gctgggttat tcctaaggac tagaagagct tggatggggg 3600
agggtggttg gtgcccttcg gtcctcggca cccccctccg tctccaacac cagctcaccc 3660
tggtatttgt catgtcagca ggagaaggtc accatgttgt ttttctcgcc cctagtcctt 3720
ccttcctgcc ccagtccaaa tttgtcctcc tatttgacct taatacttac catggctttg 3780
gaccagggaa ctagggggat agtgagagca gggagaggga agtgtgggga aggtacaggg 3840
gacctcgaca gtgaagcatt ctggggtttt cctcctgcat ttcgagctcc ccagccccca 3900
acatctggtt agtctttaac ttcctcgggt tcataaccat agcagtccag gagtggtggg 3960
catattctgt gcccgtgggg acccccggtt gtgtcctgtt cgactcagaa gacttggaga 4020
agccagaggc tgttggtggg agggaagtga ggagggagga ggggctgggt ggctgggcct 4080
gtgcacccca gcccctgccc atgcccatgc cttgctctct ttctgtcctc agtggtccca 4140
ggctgcaccc atggcagaag gaggagggca gaatcatcac gaaggtgagt ccccctggct 4200
gttggatggg gttccctgtc ctctcagggg atgggtggat ggcctaattc ctttttcttc 4260

agaactgtgg ggaggaaggg gaaggggcac aggaatataa ggatcaagaa agaaagagct 4320
gggcaccacg aggttcaccc tcagtttcgt gaggactctc cgctgttcag gtctctgcta 4380
gaagtaggac ttgttgcctt tttcttctgc tctttccagt aaaattttat ttggagaagg 4440
agtcgtgcgc acagagcagg aagacagtgt tcagggatcc taggtgttgg gggaagtgtc 4500
ccttgtttcc cctagctccc aggggagagt ggacatttag tgtcatttcc tatatagaca 4560
tgtcccattt gtgggaactg tgacccttcc tgtgtgagct ggaggcacag agggctcagc 4620
ctaatgggat ctctcctccc ttccctggtt tgcattcctt tgggggtgga gaaaacccca 4680
tttgactatg ttcgggtgct gtgaacttcc ctcccaggcc agcagagggc tggctgtagc 4740
tcccaggcgc cccgcccccc tgcccaaccc cgagtccgcc tgccttttgt tccgttgtgg 4800
tttggatcct cccatttctc tggggacacc ctggctctcc ccaccactga ctgtggcctg 4860
tgctctccac ctctggggag ggaaggccct ggggtcttcc ttcccgcgag tttccctgac 4920
ctaaatctgg cgtggctggg tagtggccag cagtggtgat gcccagcctg ttctgcctcc 4980
tccttcccca ccccaggagc cctttccttg gcctaggacc tggcttctca gccactgacc 5040
ggccccctgc ttccagtgcg ccacttaccc cttccagctt cccagtggtc tctggtctgg 5100
gagaggcagg acaaaggtct ttgtttgctg gagaaaaggt tgtctgcgat aaataaggaa 5160
aaccacgaaa gcctggttgt tggagtgtac gtgtgtgctc ccccaggcag tggaggccag 5220
ccctccttgg aggggcggct gcctgatgaa ggatgcgggt gaggttcccc gcctccacct 5280
cccatgggac ttggggattc attccaaggg gaagcttttt gggggaattc ctaccccagg 5340
tctttttacc ctcagttacc aaccccttgc ccaggccaga ccttcctgct atcccctcct 5400
gggccacaag cctggccctc ctctgtccca attgtgatga aggggcagtt caaaacttct 5460
tgattagtca tcttctcccc tatcgacttg gctttaaaaa atgacctttt cagacttcta 5520
gtctcgttca ctctttttga tgatgctttg ccgtaaccct tcgtgggtag agaaggattc 5580
tgtgcccatt ggtggtctgg ataaaagaaa tagagacctc acaggaagca gtggactggc 5640
ctgtttcccc actgttcttt ctgttttcac acctgtggcc ttctccccac cttcttccca 5700
atcaacctat tgtgtacata gccccccctca ttgtccttta ttcttctgga aagcagacct 5760
tggagggagg agtgaggggg aggctcagct gtggtctctg gggggtgggg gttgggagct 5820
ggggtggaag tccacgaagc atacacttaa gatgctttgg tgaagttcta aacttcatat 5880
tacccaggct gaaaaaagag cacttgttcc tagggctgga aatggaagcc aaaacaccac 5940
ctttttcagc ctgtttcagc atctttagag atcagcccaa cccacttaca cagttgagca 6000
gagttggagg cctagagagg ggagggactg gcccaaggtc ataccaactc atggccagag 6060
cctgggcctc ctcactggcc aggtgttatt tcttccctct gggtagggaa cctatttcag 6120

ggacaggatt gctatgtggt agtggtggtg gggtgcgata ggcgtggcag gctgggccac 6180

aatttggagt agtcatgcca gagtcctgca tttatttatt ctcaagggcc ccgcctctgt 6240

ggcccagaat taccccttca tgctccagtg caccccaggc ttcgtggcca gcctgggaaa 6300

ctgtctctac cctggtctcc cttcagatca gcttctagaa atgtttcgtg gctacagtgg 6360

cagcactgtt ttttccatga tgcaagcagt ttgccctctt gggcggggtt atcagtggct 6420

ggcagggctg gcacagcgtg tccgccccact gccacctgtg ggttccagga gggcccagcc 6480

cctgtgctga tgcccaccac cttctcagct catgtctggg gaagaggact ggcaggggga 6540

aaggtgcctc ctcctgaaag gtgcctcctc tgtttttgcc taatataggc ttgggaacac 6600

tttgatgtca gctaattctg actcctttac ttactagctg tgcggccttg gggcaactta 6660

cttagcctct ttgagcctcc tgttccccat ctgtaaaatg gaatctcaat agtgtctaat 6720

agtaccatgt ggagaaactt gtgtgaaatg atagctgtgg actactgtac acagtactca 6780

ggatgtagta agtgctcaat aaacagctgt tggtatggtt gacgttatgg tagtggttgt 6840

ggggaggacg taggaaactg gagactagct tggcaaagct ggctcttcct ccttttaggg 6900

aaagcttaga gcatccccat ggggtatacc catactcaga ctgtcctctg gcatcgaggt 6960

tggcccagga ttcagttcag ctgtcacagt gaggtggcgg gatcagatgt ggcaggccat 7020

gtcccttgga acttgagtac atcgtgtgat ctctggaatg aaaacaggcc ttcaccagtg 7080

ttgatggtgg aaagcttagg gaactgcttc aaacacagta ggagggactt acgttagatt 7140

ttggaaggac ttgcctgatt cggaagctcc aaagagtggc attacagagc tgggtggaga 7200

gaggggctag ccatcttttg tgtcgcccac cgggctcatg tgtcatcgcc tctcatgcag 7260

tggtgaagtt catggatgtc tatcagcgca gctactgcca tccaatcgag accctggtgg 7320

acatcttcca ggagtaccct gatgagatcg agtacatctt caagccatcc tgtgtgcccc 7380

tgatgcgatg cgggggctgc tgcaatgacg agggcctgga gtgtgtgccc actgaggagt 7440

ccaacatcac catgcaggtg ggcatctttg ggaagtgggg caaggggggg atagggaggg 7500

gggtaacact ttgggaacag gtggtcccag gtcgtttcct ggctagattt gccttgtctg 7560

gctcctgccc ctgagttgca caggggaggt atggtggggt cttgccttct gtggagaaga 7620

tgcttcattc ccagcccagg ttcccagcaa gccccaacca tctccttctc cctgatggtt 7680

gcccatgggc tcaggagggg acagatggat gcctgtgtca ggagcccctc tctccctctc 7740

ttggagagag tcctgagtgc ccccccttct tgggggcttt gtttgggaag ctggatgagc 7800

ctggtccatg gagagtttaa aaagtctttt ggtgttacct ggtaatgggg cacatctcag 7860

cccagatagg gtgggaggga gctgtgaaac acagggaggg ggttgctttc gggtatctac 7920

taggagtcag ggtgaagcct agagaggatg aaagaagggg aggggatggg gagtggtaag 7980

aacctaggat ttgaattccc agcctggcca acccttgcag ccatgtcttg gcctcaagtg 8040

gaacaagggc tccttgaggc cagcagggtt gggggagttg gggtgggcct gagcctcttt 8100
cctgctagag ctcttggtcc tccctgcctc caccacccat ccctgctctg cagaacccct 8160
gggtgctgag tggcaggagc cccagggttg tcccatctgg gtatggctgg ctgggtcact 8220
aacctctgtg atctgcttcc ttcctttcca gattatgcgg atcaaacctc accaaggcca 8280
gcacatagga gagatgagct tcctacagca caacaaatgt gaatgcaggt gaggatgtag 8340
tcacggattc attatcagca agtggctgca gggtgcctga tctgtgccag ggttaagcat 8400
gctgtacttt ttggcccccg tccagcttcc cgctatgtga cctttggcat tttacttcaa 8460
tgtgcctcag tttctacatc tgtaaaatgg gcacaatagt agtatacttc atagcattgt 8520
tataatgatt aaacaagtta tatatgaaaa gattaaaaca gtgttgctcc ataataaatg 8580
ctgtttttac tgtgattatt attgttgtta tccctatcat tatcatcacc atcttaaccc 8640
ttccctgttt tgctcttttc tctctcccta cccattgcag accaaagaaa gatagagcaa 8700
gacaagaaaa gtaagtggcc ctgactttag cacttctccc tctccatggc cggttgtctt 8760
ggtttggggc tcttggctac ctctgttggg ggctcccata gcctccctgg gtcagggact 8820
tggtcttgtg ggggacttgt ggtggcagca acaatgggat ggagccaact ccaggatgat 8880
ggctctaggg ctagtgagaa aacatagcca ggagcctggc acttcctttg gaagggacaa 8940
tgccttctgg gtctccagat cattcctgac caggacttgc tgtttcggtg tgtcaggggg 9000
cactgtggac actggctcac tggcttgctc taggacaccc acagtgggga gagggagtgg 9060
gtggcagaga ggccagcttt tgtgtgtcag aggaaatggc ctcttttggt ggctgctgtg 9120
acggtgcagt tggatgcgag gccggctgga gggtggtttc tcagtgcatg ccctcctgta 9180
ggcggcaggc ggcagacaca cagccctctt ggccagggag aaaaagttga atgttggtca 9240
ttttcagagg cttgtgagtg ctccgtgtta aggggcaggt aggatggggt gggggacaag 9300
gtctggcggc agtaaccctt caagacaggg tgggcggctg gcatcagcaa gagcttgcag 9360
ggaaagagag actgagagag agcacctgtg ccctgccctt tcccccacac catcttgtct 9420
gcctccagtg ctgtgcggac attgaagccc ccaccaggcc tcaacccctt gcctcttccc 9480
tcagctccca gcttccagag cgaggggatg cggaaacctt ccttccaccc tttggtgctt 9540
tctcctaagg gggacagact tgccctctct ggtcccttct ccccctcctt tcttccctgt 9600
gacagacatc ctgaggtgtg ttctcttggg cttggcaggc atggagagct ctggttctct 9660
tgaaggggac aggctacagc ctgccccccct tcctgtttcc ccaaatgact gctctgccat 9720
ggggagagta gggggctcgc ctgggctcgg aagagtgtct ggtgagatgg tgtagcaggc 9780
tttgacaggc tggggagaga actccctgcc aagtaccgcc caagcctctc ctccccagac 9840
ctccttaact cccaccccat cctgctgcct gcccagggct ccaggacacc cagccctgcc 9900

tcccagtcca ggtcgtgctg agcaggctgg tgttgctctt ggttccgtgc cagctcccaa 9960
ggtagccgct tcccccacac cgggattccc agaggttctg tcgcagttgc aaatgaaggc 10020
acaaggcctg atacacagcc ctccctccca ctcctgctcc ccatccaggc aggtctctga 10080
ccttctcccc aaagtctggc ctacctttta tcacccccgg accttcaggg tcagacttgg 10140
acagggctgc tgggcaaaga gccttccctc aggctttgcc ccctgccggg gactgggagc 10200
cactgtgagt gtggagacct ttgggtcctg tgccctccac ccagtctcgg cttcccacca 10260
aagccttgtc aggggctggg tttgccatcc catggtgggc agcgtgagga gaagaaagag 10320
ccatcgagtg cttgctgccc agacacgcct gtgtgcgccc gcgcatgcct ccccagagac 10380
cacctgcctc ctgacacttc ctccgggaag cggccctgtg tggctttgct ttggtcgttc 10440
ccccatccct gcccacctta ccacttcttt tactcccccc accgccccgg ctctctctct 10500
gtctctgttt ttttattttc cagaaaatca gttcgaggaa agggaaaggg gcaaaaacga 10560
aagcgcaaga aatcccggta taagtcctgg agcgtgtacg ttggtgcccg ctgctgtcta 10620
atgccctgga gcctccctgg cccccagtac aacctccgcc tgccattccc tgtaaccctg 10680
cctccctccc ctggtccttc cctggctctc atcctcctgg cccgtgtctc tctctcactc 10740
tctcactcca ctaattggca ccaacgggta gatttggtgg tggcattgct ggtccagggt 10800
tggggtgaat gggggtgccg acttggcctg gaggattaag ggaggggacc ctggcttggc 10860
tgggcaccga ttttctctca cccactgggc actggtggcg ggcccatgtt ggcacaggtg 10920
cctgctcacc caactggttt ccattgctct aggcttctgc actcgtctgg aagctgaggg 10980
tggtggggag ggcagacatg gcccaagaag ggctgtgaat gactggaggc agcttgctga 11040
atgactcctt ggctgaagga ggagcttggg tgggatcaga caccatgtgg cggcctccct 11100
tcatctggtg gaagtgccct ggctcctcac ggaggtgggg cctctggagg ggagcccccct 11160
attccggccc aacccatggc acccacagag gcctccttgc agggcagcct cttcctctgg 11220
gtcggaggct gtggtgggcc ctgccctggg ccctctggcc accagcggcc tggcctgggg 11280
acaccgcctc cgggcttagc ctcccatcac accctacttt agcccacctt ggtggaaggg 11340
cctggacatg agccttgcac ggggagaagg tggcccctga ttgccatccc cagcaggtga 11400
agagtcaagg cgtgctccga tgggggcaac agcagttggg tccctgtggc ctgagactca 11460
cccttgtctc ccagagacac agcattgccc cttatggcag cctctccctg cactctctgc 11520
ccgtctgtgc ccgcctcttc ctgcggcagg tgtcctagcc agtgctgcct ctttccgccg 11580
ctctctctgt cttttgctgt agcgctcgga tccttccagg gcctgggggc tgaccggctg 11640
ggtgggggtg cagctgcgga catgttaggg ggtgttgcat ggtgattttt tttctctctc 11700
tctgctgatg ctctagctta gatgtctttc cttttgcctt tttgcagtcc ctgtgggcct 11760
tgctcagagc ggagaaagca tttgtttgta caagatccgc agacgtgtaa atgttcctgc 11820

aaaaacacag actcgcgttg caaggcgagg cagcttgagt taaacgaacg tacttgcagg 11880
ttggttccca gagggcaagc aagtcagaga ggggcatcac acagagatgg ggagagagag 11940
agagaaagag agtgagcgag cgagcgagcg ggagagcgcc tgagaggggc cagctgcttg 12000
ctcagtttct agctgcctgc ctggtgactg ctgccttctc tgcttttaag gcccctgtgg 12060
tgggctgcag gcactggtcc agcctggcgg ggcctgttcc gaggttgccc tggttgcctg 12120
agtggtaggc tggtgtggct tagtgtagtg gtgtggacgc aagctgtgtg ttgtgtcctg 12180
tggtccttct gctcatagtg gctgttggtc ctgatgttat tactacctct ggtagtaatg 12240
ctgagaagct gaaagccgat tccaggtgtg gacaatgtca acaaagcaca gatgctctcg 12300
ctggggcctt gcctcggccc tttgaagtct gcatggctgg gcttctcact cactcagtgt 12360
ttcttgctgg gggaaggaat tgagtctccc acttcagact gggcctccct gaggaaaggg 12420
ttgtgtctcc ccactcagac tgaggttccc tgagggtagg gctgtgtctc tcccctccga 12480
cctgggctcc ctgatagggc tgtctccccg ctcagactga ggctccctca ggccagggct 12540
atgtctccct cctcagactg gggctctgag ggcaaggggt ctggctgttc gtttaggatg 12600
gggcactttt gcctacacac tgaaggagct gtagcatcca agaatactag atacctttaa 12660
tcctccacca gtcatggtga caaccccaag cagcccacac attttcaagt gcccccagga 12720
tgcgtggagg gaggggtctg tgcccattct cctgacatta gcctgtgagc tccgtaagcc 12780
cgggcctcgt ttacgtacct ttgtgagccc cgggcatctg tacctctttc ctttgcccat 12840
actggggacc aaggaagtgt caagtgcatg agtgaatgtg tgactcagtt cagagggtga 12900
ggtcaggagc acagggtcgg gacaggtggc tggcatcttt taatgcctta gcttatgttc 12960
tttataccaa cttggcctgt gctcagagtg agggaggccc tgggggtcag ggtaagcgtc 13020
agtcagggag gcaagacttt gtggggattt cctagacagg gccaaggcac ccccagctca 13080
ccccgaggct gtgttaggga agtccttgga gtgtctcccc tcccccagca atgttcttgt 13140
ggcttgtgtg tgctcagggg atgctgggaa ccaggcctgg gtagttggtg tggggtgctg 13200
tctgtcttgg ccctatgtga aaccaagagg gcgtatatta gtgctggggt gggggctctg 13260
cctaacttca gggctggatg aggggagtct cagttcccca ggggtccttg ggaaagataa 13320
gggacttgac attttagggt ttttaggtga ttattctgct gatgggggtt tgtgtgaagt 13380
gacctgggag ctaactgaag ttactctaac ctcccaatac ctttacccaa cccccaagct 13440
ggctgtatct gggaatatca gtttccaaaa ttggaggctt aggactccgt ttcggggctc 13500
cccagaaggg tagggcctgt tctgcctcct tctcacaatc acccaggggc aggggcatgc 13560
tgagaaagtt cttggaggcc ccctttgctt cagctggagt agtgaagccg ccgaattgtc 13620
tctccccatc ctaagtgaag cagcatattt gaaaggaaag acaacctgtt acctgggcct 13680

gcaacctcca ggcagctcaa gagagatgag gcctacagcc acagtgggag gggacatggg 13740

gaatggagat ggtccctcac cttcctgggg cctcctgctc tacgctaccc cctcgggagc 13800

ctcctgtccc cagggcaggc ccttgccatt gttggtcacc cggccaagcc tctctgcctc 13860

aggcgttctc ccagaagatc tgcccactct cttccccaca ccagccccta gagactgaac 13920

tgaaaaccct cctcagcagg gagcctcttc tgattaactt catccagctc tggtcaccca 13980

tcagctctta aaatgtcaag tggggactgt tctttggtat ccgttcattt gttgctttgt 14040

aaagtgttcc catgtccttg tcttgtctca agtagattgc aagctcagga gggtagactg 14100

ggagcccctg agtggagctg ctgctcaggc cggggctccc tgagggcagg gctggggctg 14160

ttctcatact ggggctttct gccccaggac cacaccttcc tgtcctctct gctcttatgg 14220

tgccggaggc tgcagtgacc caggggcccc caggaatggg gaggccgcct gcctcatcgc 14280

caggcctcct cacttggccc taaccccagc ctttgttttc catttccctc agatgtgaca 14340

agccgaggcg gtgagccggg caggaggaag gagcctccct cagggtttcg g 14391

<210> 115
<211> 21
<212> DNA
<213> Artificial

<220>
<223> Synthesis

<220>
<221> misc_signal
<222> (1)..(21)

<400> 115
gggcagaatc atcacgaagt g 21

<210> 116
<211> 21
<212> DNA
<213> Artificial

<220>
<223> Synthesis

<220>
<221> misc_signal
<222> (1)..(21)

<400> 116
ggtctcgatt ggatggcagt a 21

<210> 117
<211> 27
<212> DNA
<213> Artificial

<220>
<223> Synthesis

<220>
<221> misc_signal
<222> (1)..(27)

<400> 117
ggatggtagc agtctaggga ttaactt 27

<210> 118
<211> 22
<212> DNA
<213> Artificial

<220>
<223> Synthesis

<220>
<221> misc_signal
<222> (1)..(22)

<400> 118
ggaatgtccc atacccaaag aa 22

<210> 119
<211> 18
<212> DNA
<213> Artificial

<220>
<223> Synthesis

<220>
<221> misc_signal
<222> (1)..(18)

<400> 119
ccagcgccca gagagaca 18

<210> 120
<211> 21
<212> DNA
<213> Artificial

<220>
<223> Synthesis

<220>
<221> misc_signal
<222> (1)..(21)

<400> 120
agcaacaaca ggatgccaga a 21

<210> 121
<211> 21
<212> DNA
<213> Artificial

<220>
<223> Synthesis

<220>
<221> misc_signal
<222> (1)..(21)

<400> 121
cacccagaag aagagcctga a 21

<210> 122
<211> 17
<212> DNA
<213> Artificial

<220>
<223> Synthesis

<220>
<221> misc_signal
<222> (1)..(17)

<400> 122
ggcagcgcgt gatcttg 17

<210> 123
<211> 27
<212> DNA
<213> Artificial

<220>
<223> Synthesis

<220>
<221> misc_signal
<222> (1)..(27)

<400> 123
aaagtggtct tcagtttctc catagtg 27

<210> 124
<211> 24
<212> DNA
<213> Artificial

<220>
<223> Synthesis

<220>
<221> misc_signal

<222> (1)..(24)

<400> 124
cttcgatcac agtgaaatcc agat 24

<210> 125
<211> 17
<212> DNA
<213> Artificial

<220>
<223> Synthesis

<220>
<221> misc_signal
<222> (1)..(17)

<400> 125
cctaatgccg aacacat 17

<210> 126
<211> 20
<212> DNA
<213> Artificial

<220>
<223> Synthesis

<220>
<221> misc_signal
<222> (1)..(20)

<400> 126
tccaggctca gtccctgaag 20

<210> 127
<211> 22
<212> DNA
<213> Artificial

<220>
<223> Synthesis

<220>
<221> misc_signal
<222> (1)..(22)

<400> 127
cctggctgtc cttatcatca ca 22

<210> 128
<211> 16
<212> DNA
<213> Artificial

<220>

<223> Synthesis

<220>
<221> misc_signal
<222> (1)..(16)

<400> 128
gggcgctggg cttctc 16

<210> 129
<211> 19
<212> DNA
<213> Artificial

<220>
<223> Synthesis

<220>
<221> misc_signal
<222> (1)..(19)

<400> 129
tgtattgcgc acccctcaa 19

<210> 130
<211> 26
<212> DNA
<213> Artificial

<220>
<223> Synthesis

<220>
<221> misc_signal
<222> (1)..(26)

<400> 130
cctctacttg cgttcttcaa atgtac 26

<210> 131
<211> 19
<212> DNA
<213> Artificial

<220>
<223> Synthesis

<220>
<221> misc_signal
<222> (1)..(19)

<400> 131
ccgtgcttcc ggacaactt 19

<210> 132

<211> 21
<212> DNA
<213> Artificial

<220>
<223> Synthesis

<220>
<221> misc_signal
<222> (1)..(21)

<400> 132
gtggactgct tccaggtgtc a 21

<210> 133
<211> 21
<212> DNA
<213> Artificial

<220>
<223> Synthesis

<220>
<221> misc_signal
<222> (1)..(21)

<400> 133
aaattgaggg ctttcgcctt a 21

<210> 134
<211> 20
<212> DNA
<213> Artificial

<220>
<223> Synthesis

<220>
<221> misc_signal
<222> (1)..(20)

<400> 134
ccggtagtga acccgttgat 20

<210> 135
<211> 18
<212> DNA
<213> Artificial

<220>
<223> Synthesis

<220>
<221> misc_signal
<222> (1)..(18)

<400> 135
cggctcaggc cattatgc 18

<210> 136
<211> 21
<212> DNA
<213> Artificial

<220>
<223> Synthesis

<220>
<221> misc_signal
<222> (1)..(21)

<400> 136
ggtccccaga aaatggttca c 21

<210> 137
<211> 20
<212> DNA
<213> Artificial

<220>
<223> Synthesis

<220>
<221> misc_signal
<222> (1)..(20)

<400> 137
tgcagcttca ggaccacaac 20

<210> 138
<211> 25
<212> DNA
<213> Artificial

<220>
<223> Synthesis

<220>
<221> misc_signal
<222> (1)..(25)

<400> 138
ttccatagtc tgttccatca aaatg 25

<210> 139
<211> 20
<212> DNA
<213> Artificial

<220>
<223> Synthesis

<220>
<221> misc_signal
<222> (1)..(20)

<400> 139
gacgatggcc tggagtgtgt 20

<210> 140
<211> 22
<212> DNA
<213> Artificial

<220>
<223> Synthesis

<220>
<221> misc_signal
<222> (1)..(22)

<400> 140
ggtaccggat catgaggatc tg 22

<210> 141
<211> 25
<212> DNA
<213> Artificial

<220>
<223> Synthesis

<220>
<221> misc_signal
<222> (1)..(25)

<400> 141
tacctcagca agacgttatt tgaaa 25

<210> 142
<211> 25
<212> DNA
<213> Artificial

<220>
<223> Synthesis

<220>
<221> misc_signal
<222> (1)..(25)

<400> 142
aagtgtgatt ggcaaaactg attgt 25

<210> 143
<211> 22
<212> DNA

<213>  Artificial

<220>
<223>  Synthesis

<220>
<221>  misc_signal
<222>  (1)..(22)

<400>  143
gaggaaaatc cacttgctgg aa    22

<210>  144
<211>  21
<212>  DNA
<213>  Artificial

<220>
<223>  Synthesis

<220>
<221>  misc_signal
<222>  (1)..(21)

<400>  144
atcatgtgtg gcccacagag a    21

<210>  145
<211>  21
<212>  DNA
<213>  Artificial

<220>
<223>  Synthesis

<220>
<221>  misc_signal
<222>  (1)..(21)

<400>  145
ggactatgtc cgggaacaca a    21

<210>  146
<211>  22
<212>  DNA
<213>  Artificial

<220>
<223>  Synthesis

<220>
<221>  misc_signal
<222>  (1)..(22)

<400>  146
ccaagtagtt catgcccttt gc    22

<210> 147
<211> 27
<212> DNA
<213> Artificial

<220>
<223> Synthesis

<220>
<221> misc_signal
<222> (1)..(27)

<400> 147
gtccagatag ctaagggaat gatgtac 27

<210> 148
<211> 25
<212> DNA
<213> Artificial

<220>
<223> Synthesis

<220>
<221> misc_signal
<222> (1)..(25)

<400> 148
ctagcccaaa atctgtgatt ttcac 25

<210> 149
<211> 24
<212> DNA
<213> Artificial

<220>
<223> Synthesis

<220>
<221> misc_signal
<222> (1)..(24)

<400> 149
gcatacctca acgccaataa gttc 24

<210> 150
<211> 24
<212> DNA
<213> Artificial

<220>
<223> Synthesis

<220>

<221> misc_signal
<222> (1)..(24)

<400> 150
cgtcatacca aaatctccga tttt 24

<210> 151
<211> 22
<212> DNA
<213> Artificial

<220>
<223> Synthesis

<220>
<221> misc_signal
<222> (1)..(22)

<400> 151
gcagaagctg ggtgtcatag gt 22

<210> 152
<211> 21
<212> DNA
<213> Artificial

<220>
<223> Synthesis

<220>
<221> misc_signal
<222> (1)..(21)

<400> 152
cacggaggat gcggtcttat t 21

<210> 153
<211> 24
<212> DNA
<213> Artificial

<220>
<223> Synthesis

<220>
<221> misc_signal
<222> (1)..(24)

<400> 153
gctaaaaatc ttgacccaca ttgg 24

<210> 154
<211> 25
<212> DNA
<213> Artificial

<220>
<223> Synthesis

<220>
<221> misc_signal
<222> (1)..(25)

<400> 154
agtcacgttt gctcttgagg tagtt 25

<210> 155
<211> 17
<212> DNA
<213> Artificial

<220>
<223> Synthesis

<220>
<221> misc_signal
<222> (1)..(17)

<400> 155
ctgaaacggc gcttgga 17

<210> 156
<211> 24
<212> DNA
<213> Artificial

<220>
<223> Synthesis

<220>
<221> misc_signal
<222> (1)..(24)

<400> 156
cagatcttca ggagcttcct cttc 24

<210> 157
<211> 26
<212> DNA
<213> Artificial

<220>
<223> Synthesis

<220>
<221> misc_signal
<222> (1)..(26)

<400> 157
ggaaagaata agactgtgag caagct 26

<210> 158
<211> 25
<212> DNA
<213> Artificial

<220>
<223> Synthesis

<220>
<221> misc_signal
<222> (1)..(25)

<400> 158
catagaagta gatgagccgc tcatc 25

<210> 159
<211> 22
<212> DNA
<213> Artificial

<220>
<223> Synthesis

<220>
<221> misc_signal
<222> (1)..(22)

<400> 159
gaggctacaa ggtccgttat gc 22

<210> 160
<211> 22
<212> DNA
<213> Artificial

<220>
<223> Synthesis

<220>
<221> misc_signal
<222> (1)..(22)

<400> 160
ctgccgtact cattctccac aa 22

<210> 161
<211> 20
<212> DNA
<213> Artificial

<220>
<223> Synthesis

<220>
<221> misc_signal
<222> (1)..(20)

<400> 161
ccgctgaagg gaaacagaag 20

<210> 162
<211> 24
<212> DNA
<213> Artificial

<220>
<223> Synthesis

<220>
<221> misc_signal
<222> (1)..(24)

<400> 162
tcataacatt tccgaagacg acaa 24

<210> 163
<211> 23
<212> DNA
<213> Artificial

<220>
<223> Synthesis

<220>
<221> misc_signal
<222> (1)..(23)

<400> 163
tgcctttcaa tgacgtttat tgc 23

<210> 164
<211> 19
<212> DNA
<213> Artificial

<220>
<223> Synthesis

<220>
<221> misc_signal
<222> (1)..(19)

<400> 164
ggctcaagtg gaccccaaa 19

<210> 165
<211> 20
<212> DNA
<213> Artificial

<220>
<223> Synthesis

<220>
<221> misc_signal
<222> (1)..(20)

<400> 165
tgctcctcac tggccttttt 20

<210> 166
<211> 16
<212> DNA
<213> Artificial

<220>
<223> Synthesis

<220>
<221> misc_signal
<222> (1)..(16)

<400> 166
atgcagtagc caggac 16

<210> 167
<211> 22
<212> DNA
<213> Artificial

<220>
<223> Synthesis

<220>
<221> misc_signal
<222> (1)..(22)

<400> 167
gaatcattca ccaggcaaat tg 22

<210> 168
<211> 20
<212> DNA
<213> Artificial

<220>
<223> Synthesis

<220>
<221> misc_signal
<222> (1)..(20)

<400> 168
tctgtactgc gggtggaaca 20

<210> 169
<211> 31

<212> DNA
<213> Artificial

<220>
<223> Synthesis

<220>
<221> misc_signal
<222> (1)..(31)

<400> 169
accatttagg taaggtagta agtggataca g 31

<210> 170
<211> 21
<212> DNA
<213> Artificial

<220>
<223> Synthesis

<220>
<221> misc_signal
<222> (1)..(21)

<400> 170
gcagccagta ggtcaccaaa a 21

<210> 171
<211> 24
<212> DNA
<213> Artificial

<220>
<223> Synthesis

<220>
<221> misc_signal
<222> (1)..(24)

<400> 171
cctgtggatg actgagtacc tgaa 24

<210> 172
<211> 22
<212> DNA
<213> Artificial

<220>
<223> Synthesis

<220>
<221> misc_signal
<222> (1)..(22)

<400> 172

cagagacagc caggagaaat ca 22

<210> 173
<211> 20
<212> DNA
<213> Artificial

<220>
<223> Synthesis

<220>
<221> misc_signal
<222> (1)..(20)

<400> 173
tgcgggacaa gattcttggt 20

<210> 174
<211> 17
<212> DNA
<213> Artificial

<220>
<223> Synthesis

<220>
<221> misc_signal
<222> (1)..(17)

<400> 174
gcagccagac gggcatt 17

<210> 175
<211> 22
<212> DNA
<213> Artificial

<220>
<223> Synthesis

<220>
<221> misc_signal
<222> (1)..(22)

<400> 175
atgctgccta catgagcaag gt 22

<210> 176
<211> 23
<212> DNA
<213> Artificial

<220>
<223> Synthesis

<220>
<221> misc_signal
<222> (1)..(23)

<400> 176
gctctgtcaa ctccgtctca ttg 23

<210> 177
<211> 21
<212> DNA
<213> Artificial

<220>
<223> Synthesis

<220>
<221> misc_signal
<222> (1)..(21)

<400> 177
gagaccctgc tgtcccagaa c 21

<210> 178
<211> 25
<212> DNA
<213> Artificial

<220>
<223> Synthesis

<220>
<221> misc_signal
<222> (1)..(25)

<400> 178
aggttgtagt cagcgaagga gatct 25

<210> 179
<211> 22
<212> DNA
<213> Artificial

<220>
<223> Synthesis

<220>
<221> misc_signal
<222> (1)..(22)

<400> 179
gcagaccagc atgacagatt tc 22

<210> 180
<211> 20
<212> DNA
<213> Artificial

<220>
<223> Synthesis

<220>
<221> misc_signal
<222> (1)..(20)

<400> 180
gcggattagg gcttcctctt 20

<210> 181
<211> 20
<212> DNA
<213> Artificial

<220>
<223> Synthesis

<220>
<221> misc_signal
<222> (1)..(20)

<400> 181
cctgcaaccg acgattcttc 20

<210> 182
<211> 18
<212> DNA
<213> Artificial

<220>
<223> Synthesis

<220>
<221> misc_signal
<222> (1)..(18)

<400> 182
gggcgtctgc tccacaga 18

<210> 183
<211> 28
<212> DNA
<213> Artificial

<220>
<223> Synthesis

<220>
<221> misc_signal
<222> (1)..(28)

<400> 183
aaagagcaca ggaaaatata taccatga 28

<210> 184
<211> 23
<212> DNA
<213> Artificial

<220>
<223> Synthesis

<220>
<221> misc_signal
<222> (1)..(23)

<400> 184
ggtgacacct gttctcactc aca 23

<210> 185
<211> 19
<212> DNA
<213> Artificial

<220>
<223> Synthesis

<220>
<221> misc_signal
<222> (1)..(19)

<400> 185
cggattcctg gagggaaca 19

<210> 186
<211> 29
<212> DNA
<213> Artificial

<220>
<223> Synthesis

<220>
<221> misc_signal
<222> (1)..(29)

<400> 186
caattgatac taagattctt gatcttgga 29

<210> 187
<211> 20
<212> DNA
<213> Artificial

<220>
<223> Synthesis

<220>
<221> misc_signal

<222> (1)..(20)

<400> 187
tgccccagat tcaggatcag 20

<210> 188
<211> 24
<212> DNA
<213> Artificial

<220>
<223> Synthesis

<220>
<221> misc_signal
<222> (1)..(24)

<400> 188
tgggactatt aggctcaggt gaac 24

<210> 189
<211> 22
<212> DNA
<213> Artificial

<220>
<223> Synthesis

<220>
<221> misc_signal
<222> (1)..(22)

<400> 189
ctccaggttt gccatcttca gt 22

<210> 190
<211> 19
<212> DNA
<213> Artificial

<220>
<223> Synthesis

<220>
<221> misc_signal
<222> (1)..(19)

<400> 190
gcctggcgag tccctattg 19

<210> 191
<211> 18
<212> DNA
<213> Artificial

<220>

<223> Synthesis

<220>
<221> misc_signal
<222> (1)..(18)

<400> 191
cagccccagc agggtttt 18

<210> 192
<211> 21
<212> DNA
<213> Artificial

<220>
<223> Synthesis

<220>
<221> misc_signal
<222> (1)..(21)

<400> 192
caccctctgt tgtcggaagt g 21

<210> 193
<211> 24
<212> DNA
<213> Artificial

<220>
<223> Synthesis

<220>
<221> misc_signal
<222> (1)..(24)

<400> 193
tgaaacacct agcgatgcta ttga 24

<210> 194
<211> 22
<212> DNA
<213> Artificial

<220>
<223> Synthesis

<220>
<221> misc_signal
<222> (1)..(22)

<400> 194
ggtaggatca ttttccgctt ga 22

<210> 195

<211> 26
<212> DNA
<213> Artificial

<220>
<223> Synthesis

<220>
<221> misc_signal
<222> (1)..(26)

<400> 195
gatggagaat atttcaccct tcagat 26

<210> 196
<211> 21
<212> DNA
<213> Artificial

<220>
<223> Synthesis

<220>
<221> misc_signal
<222> (1)..(21)

<400> 196
cctcattcag ctctcggaac a 21

<210> 197
<211> 22
<212> DNA
<213> Artificial

<220>
<223> Synthesis

<220>
<221> misc_signal
<222> (1)..(22)

<400> 197
cagctgctta gacgctggat tt 22

<210> 198
<211> 23
<212> DNA
<213> Artificial

<220>
<223> Synthesis

<220>
<221> misc_signal
<222> (1)..(23)

<400> 198
ttcctgttgg tgaagctaac gtt 23

<210> 199
<211> 31
<212> DNA
<213> Artificial

<220>
<223> Synthesis

<220>
<221> misc_signal
<222> (1)..(31)

<400> 199
tatacctcgg attacttcat tagctatggt a 31

<210> 200
<211> 18
<212> DNA
<213> Artificial

<220>
<223> Synthesis

<220>
<221> misc_signal
<222> (1)..(18)

<400> 200
ccgagctgat gggatgga 18

<210> 201
<211> 19
<212> DNA
<213> Artificial

<220>
<223> Synthesis

<220>
<221> misc_signal
<222> (1)..(19)

<400> 201
acgtctgcat cgccatgac 19

<210> 202
<211> 23
<212> DNA
<213> Artificial

<220>
<223> Synthesis

<220>
<221> misc_signal
<222> (1)..(23)

<400> 202
atggcctcag atttcaactc gtt 23

<210> 203
<211> 25
<212> DNA
<213> Artificial

<220>
<223> Synthesis

<220>
<221> misc_signal
<222> (1)..(25)

<400> 203
ttcttaacca aacggatgaa actct 25

<210> 204
<211> 25
<212> DNA
<213> Artificial

<220>
<223> Synthesis

<220>
<221> misc_signal
<222> (1)..(25)

<400> 204
ccaaaggtct tgcagaaagt taaaa 25

<210> 205
<211> 22
<212> DNA
<213> Artificial

<220>
<223> Synthesis

<220>
<221> misc_signal
<222> (1)..(22)

<400> 205
gggaaattca agcagtgttt cc 22

<210> 206
<211> 36
<212> DNA

<213> Artificial

<220>
<223> Synthesis

<220>
<221> misc_signal
<222> (1)..(36)

<400> 206
ccagtcacat agaactcgaa aaactttgga ctgatg 36

<210> 207
<211> 21
<212> DNA
<213> Artificial

<220>
<223> Synthesis

<220>
<221> misc_signal
<222> (1)..(21)

<400> 207
ctaccacatc caaggaaggc a 21

<210> 208
<211> 21
<212> DNA
<213> Artificial

<220>
<223> Synthesis

<220>
<221> misc_signal
<222> (1)..(21)

<400> 208
tttttcgtca ctacctcccc g 21